# EUROPEAN PATENT SPECIFICATION

(11) **EP 1 648 886 B1**
(45) Date of publication and mention of the grant of the patent: **09.12.2009**
(21) Application number: 04741318.2
(22) Date of filing: 29.07.2004
(51) Int. Cl.: C07D 413/04, A61K 31/535, A61P 25/00

(54) **BENZOXAZINONE-DERIVED SULFONAMIDE COMPOUNDS, THEIR PREPARATION AND USE AS MEDICAMENTS**
SULPHONAMID BENZOXAZINONDERIVATE. IHRE HERSTELLUNG UND VERWENDUNG ALS MEDIKAMENTE
COMPOSES SULFONAMIDE DERIVES DE BENZOXAZINONE, LEUR PREPARATION ET LEUR UTILISATION COMME MEDICAMENTS

(30) Priority: 30.07.2003 ES 200301812
(43) Date of publication of application: 26.04.2006
(73) Proprietor: LABORATORIOS DEL DR. ESTEVE, S.A., 08041 Barcelona (ES)
(72) Inventor: TORRENS JOVER, Antoni, E-08221 Terrase (ES); MAS PRIO , Josep, E-08191 Rubi (ES); DORDAL ZUERAS, Alberto, E-08016 Barcelona (ES); FISAS ESCASANY, Maria Angeles, E-08025 Barcelona (ES)
(74) Representative: ABG Patentes, S.L.
(86) International application number: PCT/EP2004/008507
(87) International publication number: WO 2005/014589

(56) References cited:
- EP-A- 1 242 396
- WO-A-01/85725
- WO-A-97/45419
- US-A- 5 665 719
- US-B1- 6 225 312

## Description

The present invention relates to benzoxazinone-derived sulphonamide compounds of general formula (I), 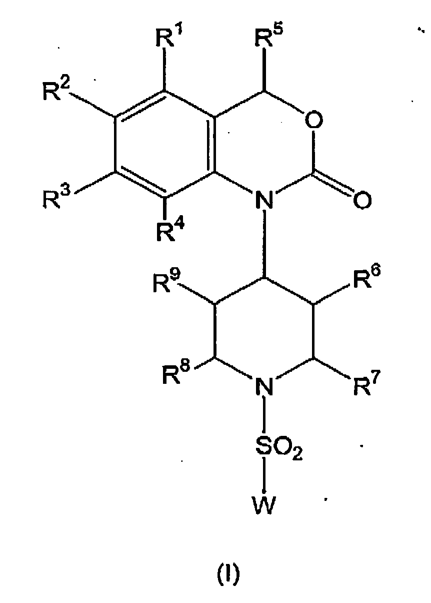 a process for their preparation, a medicament comprising these compounds and the use of benzoxazinone-derived sulphonamide compounds for the preparation of medicaments for the treatment of disorders related to the 5 -HT₆, receptor.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both In rats [F.J. Monsma, et al., Mol. Pharmacol., 1993, 43, 320; M. Ruat, set al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther. , 1995, 274, 173; A.J. Sleight, et al., Behav. Brain Res., 1996, 73, 245; T.A. Branchek, et al., Annu. Rev. Pharmacol. Toxicol., 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J, Mosma, et al., Mol. Pharmacol. , 1993, 43, 320; T. Shinkai, et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol. , 2000, 130, 1597; C. Gérard, et al., Brain Research , 1997, 746, 207; M.R. Pranzatelli, Drugs of Today , 1997, 33, 379].

Moreover, it has been shown that the 5-HT₆ receptor also plays a role in food ingestion [Neuropharmacology, 41, 2001, 210-219].
Food ingestion disorders, particularly obesity, are a serious, fast growing threat to the health of humans of all age groups, since they increase the risk of developing other serious, even life-threatening diseases such as diabetes or coronary diseases.

WO 01/85725 discloses *inter alia* 3*H-benzooxazol*-2-one piperidine derivative compounds displaying partial agonism activity towards members of the dopamine D₂ receptor subfamily and affinity for relevant serotonin and/or noradrenergic receptors *in vitro* and high efficacy in a therapeutic model for anxiolytic/antidepressant activity *in vivo.*

WO 97145419 discloses benxoxazinone 4-substituted piperazin-1-ylmcthyl derivative compounds with antagonistic activity at dopamine D₄ receptors. They are therefore suspected to be useful in the treatment of psychosis and schizophrenia.

US 6,225,312 discloses *inter alia* substituted 5-(1-benzyl-piperidin-4-yl)-4*H-*benzo[1,4]oxazin-3-one compounds devoid of sulfonamide functionality showing activities as either partial agonists or antagonists at dopamine D₂, D₃ and D₄ receptors and agonists of the serotonin 5-HT_{1A} receptor, and activity in, therapeutic models sensitive to antipsychotics, antidepresants and anxiolytics.

Thus, it was an object of the present invention to provide novel compounds that are suitable in particular as active substances in medicaments, preferably in medicaments for cognitive enhancement, for the prophylaxis and/or treatment of food-intake related disorders, disorders of the central nervous system, disorders of the gastrointestinal tract, such as irritable intestine syndrom, anxiety, panic, depression, cognitive disorders such as memory disorders, senile dementia disorders, such as Morbus Alzheimer, Morbus Parkinson and Morbus Huntington, schizophrenia, psychosis, infantile hyperkinesia, ADHC (attention deficit, hyperactivity disorders) particularly in mammals, including humans.

It has been found that the benzoxazinone-derived sulphonamide compounds of general formulas (I), (Ia) and (Ib) given below show affinity for the 5-HT₆-receptor. These compounds are therefore also suitable for the manufacture of a medicament for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Nan-Insulln Dependent Diabetes Mellitus), preferably type II diabetes, which is caused by obesity, disorders of the central nervous system, disorders of the gastrointestinal tract, such as irritable intestine syndrom, anxiety, panic, depression, cognitive memory disorders, senile dementia disorders, such as Morbus Alzheimer, Morbus Parkinson and Morbus Huntington, schizophrenia, psychosis, infantile hyperkinesia, ADHC.(attention deficit, hyperactivity disorders) particularly in mammals, including man.

Thus, one aspect of the present invention are benzoxazinone-derived sulfonamide compounds of general formula (I), 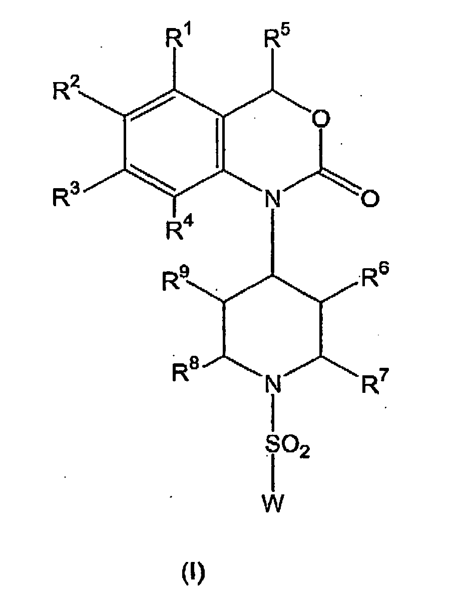 wherein
R¹, R², R³, R⁴ are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, - OR¹⁰, -OC(=O)R¹¹, -(C=O)-O-R¹¹, -SR¹², -SOR¹², -SO₂R¹², -NH-SO₂R¹², -SO₂NH₂ and a -NR¹³R¹⁴ moiety,
R⁵ represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R⁵, R⁷, R⁸, R⁹ are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a -COOR¹⁵ moiety,
W represents an unbranched or branched, saturated or unsaturated, optionally at least mono-substituted aliphatic radical,
a saturated or unsaturated, optionally at least mono-substituted, optionally at least one heteroatom as ring member containing cycloaliphatic radical, which may be bonded via an optionally mono-substituted alkylene group and/or may be condensed with an optionally at least mono-substituted aromatic mono- or polycyclic ring-system,
an optionally at least mono-substituted heteroaryl radical, which may be bonded via an optionally mono-substituted alkylene group and/or may be condensed with an optionally at least mono-substituted mono- or polycyclic ring-system,
an optionally at least mono-substituted, monocyclic aryl- radical, which is condensed with an optionally at least mono-substituted mono- or polycyclic ring-system and which may be bonded via an optionally at least mono-substituted alkylene group,
a NR¹⁶R¹⁷-moiety,
a COR¹⁸-moiety,
or a phenyl radical, which is at least mono-substituted with one of the substituents selected from the group consisting of:
2,2,2,-Trifluoroethoxy-, C₂₋₆-Alkenyl-, 1,3-Dihydro-1-oxo-2H-isoindol-2-yl-, N-Phthalimidinyl-, [(2-chloro-1,3-thiazolyl-5-yl)-methoxy, Ethyl-5-yl-2-methyl-3-furoate, C₁₁₋₂₀-alkyl-, 1,3-Dioxo-2-azaspiro[4,4]non-2-yl-, pyrazolyl-, (1,3-oxazol-5-yl)-, (5-Methyl-1,3,4-oxadiazol-2-yl)-, difluoromethoxy, dichloromethoxy, 1-pyrrolidinylsulfonyl, morpholinosulfonyl, 2-methyl-4-pyrimidinyl-, a phenoxy group, which is at least mono-substituted with C₁₋₅-alkoxy, a phenyl group, which is at least mono-substituted with one of the substituents selected from the group consisting of nitro, C₁₋₅-alkoxy, F, Cl, Br, at least partially fluorinated C₁₋₅-alkyl, at least partially chlorinated C₁₋₅-alkyl, [(2-Chloro-1,3-thiazol-5-yl)-methoxy]-, -(C=O)-H and -(C=O)-C₁₋₅-alkyl, a pyridinyl group, which is at least mono-substituted with C₁₋₅-alkoxy, a pyridinyloxy group, which is at least mono-substituted with C₁₋₅-alkoxy, a phenoxy group, which is at least di-substituted and a pyridinyloxy group, which is at least di-subsituted,
with the proviso that W does not represent unsubstituted furyl-, unsubstituted thienyl- or thienyl substituted with a substituent selected from the group consisting of C₁₋₅-alkoxycarbonyl, C₁₋₅-alkylcarbonyl, carboxyl and pyridyl, unsubstituted pyrrolyl-, unsubstituted naphthyl, unsubstituted indolyl, unsubstituted tetrahydronaphthyl, substituted or unsubstituted pyridyl, unsubstituted pyrazinyl, unsubstituted quinolinyl-, d₁₋₅-afkyfsubstituted pyrrolyl-, and unsubstituted cyclohexyl or cyclohexyl substituted with one or two members selected from the group consisting of oxo, hydroxyl, C₁₋₅-alkoxyl, C₁₋₅-alkoxy-carbonylamino-C₁₋₅ alkyl and amino-C₁₋₅ alkyl,
R¹⁰ represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹¹ represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹² represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹³ and R¹⁴ each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R¹³ and R¹⁴ together with the bridging nitrogen atom form a saturated, unsaturated or aromatic heterocyclic ring, which may be at least mono-substituted and/or contain at least one further heteroatom as a ring member,
R¹⁵ represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹⁶ represents an unbranched or branched, saturated or unsaturated, optionally at least mono-substituted aliphatic radical,
R¹⁷ represents an unbranched or branched, saturated or unsaturated, optionally at least mono-substituted aliphatic radical, and
R¹⁸ represents an optionally at least mono-substituted aryl radical,
optionally in form of one of its stereoisomers, preferably enantiomers or diastereomers, its racemate or in form of a mixture of at least two of its stereoisomers, preferably enantiomers or diastereomers, in any mixing ratio, or a corresponding salt thereof, or a corresponding solvate.

A mono- or polycyclic ring-system according to the present invention means a mono-or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-system are prefarably 5- or 6-membered.

If one or more of the residues R¹-R¹⁷ and W represents an aliphatic radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and an unsubstituted phenyl radical. If any one of the above mentioned substitutents itself is at least mono-substituted, said substituents may preferably be selected from the group consisting of F, Cl, methyl and methoxy.

If one or more of the residues R¹-R¹⁵ represents or comprises a cycloaliphatic radical, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, phenoxy, benzoyl, cyclohexyl, branched or unbranched C₁₋₄-perfluoroalkyl, -NR^{A}R^{B} wherein R^{A}, R^{B} are each independently selected from the group consisting of H, a branched or unbranched C₁₋₄-alkyl-radical, -CH₂-CH₂-OH and phenyl, carboxy, amido, cyano, nitro, -SO₂NH₂, - CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may in each case be branched or unbranched, unsubstituted or at least mono-substituted phenyl or naphthyl and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, methoxy, ethoxy, benzoyl, phenoxy, cyclohexyl, -CF₃, -CO-CH₃, -CO-OCH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any one of the above mentioned substitutents itself is at least mono-substituted, said substituents may preferably be selected from the group consisting of F, Cl, methyl and methoxy.

If one or more of the residues R¹-R⁴, R¹⁰-R¹⁵ and W comprises an alkylene group, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and unsubstituted phenyl. If any one of the above mentioned substitutents itself is at least mono-substituted, said substituents may preferably be selected from the group consisting of F, Cl, methyl and methoxy.

If one or more of the residues R¹-R⁴ and R¹⁰-R¹⁵ comprises a mono- or polycyclic ringsystem, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, branched or unbranched C₁₋₄-perfluoroalkyl, amino, carboxy, amido, cyano, keto, nitro, -SO₂NH₂, -CO-C₁₋₄-alkyl, -SO-C₁₋₄-alkyl, -SO₂-C₁-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl, more preferably from the group consisting of hydroxy, F, Cl, Br, methyl, ethyl, methoxy, ethoxy, CF₃, keto, cyano and an unsubstituted phenyl radical. If any one of the above mentioned substitutents itself is at least mono-substituted, said substituents may preferably be selected from the group consisting of F, Cl, methyl and methoxy.

If one or more of the residues R¹-R⁴, R¹⁰-R¹⁵ and R¹⁸ represents or comprises an aryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{A}R^{B}wherein R^{A}, R^{B} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, -CH(OH)(phenyl), methoxy, ethoxy, unsubstituted or at least mono-substituted benzoyl, unsubstituted or at least mono-substituted phenoxy, cyclohexyl, CF₃, -CO-CH₃, -CO-OCH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any of the above mentioned substitutents itself is at least mono-substituted, said substituents may preferably be selected from the group consisting of F, Cl, methyl and methoxy.

If one or more of the residues R¹-R⁴ and R¹⁰-R¹⁵ represents or comprises a heteroaryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{A}R^{B} wherein R^{A}, R^{B} are each independently selected from the group consisting of H, a branched or unbranched C₁₋₄-alkyl-radical, -CH₂-CH₂-OH and phenyl, carboxy, amido, cyano, nitro, -CH(OH)(phenyl), -SO₂NH₂, -CO-C₁₋₄-alkyl, -CO-OC₁₋₄-alkyl, SO-C₁₋₄-alkyl, SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, CI, Br, methyl, ethyl, cyano, methoxy, ethoxy, unsubstituted or at least mono-substituted benzoyl, unsubstituted or at least mono-substituted phenoxy, cyclohexyl, CF₃, - CH(OH)(phenyl), -CO-CH₃, -CO-OCH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any one of the above mentioned substitutents itself is at least mono-substituted, said substituents may preferably be selected from the group consisting of F, Cl, methyl and methoxy.

If R¹³ and R¹⁴ form a heterocyclic ring, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, CI, Br, methoxy, ethoxy, methyl, CF₃ and an unsubstituted phenyl radical. If any of the above mentioned substitutents itself is at least mono-substituted, said substituents may preferably be selected from the group consisting of F, CI, methyl and methoxy.

If R¹³ and R¹⁴ form a heterocyclic ring, which contains one or more further heteroatoms as ring members, unless defined otherwise, each of these heteroatoms may preferably be selected from the group consisting of N, O and S, more preferably from the group consisting of N and O.

If one or more of the residues R¹-R¹⁵ and W represents or comprises a cycloaliphatic radical, which contains one or more heteroatoms as ring members, unless defined otherwise, each of these heteroatoms may preferably be selected from the group consisting of N, O, S and P, more preferably from the group consisting of N, O and S.

If one or more of the residues R¹-R⁴, R¹⁰-R¹⁵ and W represents or comprises an heteroaryl radical, which contains one or more heteroatoms as ring members, unless defined otherwise, each of these heteroatoms may preferably be selected from the group consisting of N, O S and P, more preferably from the group consisting of N, O and S.

If W represents or comprises a cycloaliphatic radical, a heteroaryl radical, an aryl radical and/or a mono- or polycyclic ring system, which is substituted by one or more substituents, unless defined otherwise, each of these substituents may preferably be selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆-alkenyl, SO₂-C₁₋₄-alkyl, - (C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅-alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -C₁₋₄-Alkylen-NH-(C=O)-phenyl, more preferably from the group consisting of hydroxy, nitro, carboxy, cyano, keto, F, Cl, Br, I, C₁₋₁₂-alkyl, CH₂F, CHF₂, CF₃, CH₂Cl, CH₂Cl₂, CCl₃, CH₂Br, CHBr₂, CBr₃, OCF₃, OCHF₂, OCH₂F, O-CH₂-CF₃, vinyl, SO₂-CH₃, - (C=O)-CH₃, -(C=O)-C₂H₅, -(C=O)-O-CH₃, -(C=O)-O-C₂CH₅, -(C=O)-Cl, -S-CH₃-, - (C=O)-H, -NH-(C=O)-NH-CH₃, -(C=OrCF₃, dimethylamino, diethylamino, di-n-propylamino, di-iso-propylamino, di-n-butylamino, di-tert-butyamino, NH-(C=O)-CH₃, - CH₂-(C=O)-CH₃, -CH₂-(C=O)-C₂H₅, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -CH₂-NH-(C=O)-phenyl.

If any of the afore mentioned substituents itself is substituted by one or more substituents, said substituents may preferably be selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, -C(=O)-O-C₁₋₅-alkyl, -(C=O)-CH₂-F, -(C=O)-CH₂-CI, -(C=O)-CH₂-Br, preferably from the group consisting of F, Cl, Br, CH₂F, CHF₂, CF₃, CH₂Cl, CHCl₂, CCl₃, CH₂Br, CHBr₂, CBr₃, nitro, cyano, hydroxy, -(C=O)-CH₃, CH₃, C₂H₅, -S-CH₃, -C(=O)-O-CH₃, -C(=O)-O-C₂H₅, -(C=O)-CH₂-F, -(C=O)-CH₂-Cl and -(C=O)-CH₂-Br.

Preferred compounds of general formula (I) are those, wherein R¹, R², R³, R⁴ are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano group, -OR¹⁰, -OC(=O)R¹¹, -SR¹², -SOR¹², -SO₂R¹², -NH-SO₂R¹², -SO₂NH₂ and a -NR¹³R¹⁴ moiety,
preferably selected from the group consisting of H, F, Cl, Br, a saturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro group, a cyano group, -OR¹⁰, -OC(=O)R¹¹, -SR¹² and -NR¹³R¹⁴ moiety,
more preferably selected from the group consisting of H, F, Cl, -CH₃, -CH₂CH₃, -CF₃, -CF₂CF₃, cyclopentyl, cyclohexyl, a nitro group, a cyano group and -OR¹⁰,
and R⁵-R¹⁸ and W have the meaning as defined above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of generals formula (I), wherein R⁵ represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₈-cycloaliphatic radical,
preferably represents H or a branched or unbranched C₁₋₃-alkyl radical,
more preferably H, CH₃ or CH₂CH₃,
and R¹-R⁴, R⁶-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Preferred compounds of general formula (I) are also those, wherein R⁶, R⁷, R⁸, R⁹ are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano group and a -COOR¹⁵ moiety,
preferably selected from the group consisting of H, a branched or unbranched C₁₋₃-alkyl radical, a cyano group and a -COOR¹⁵ group,
more preferably from the group consisting of H, -CH₃, -CH₂CH₃ and a cyano moiety,
and R¹-R⁵, R¹⁰-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein W represents an unbranched or branched, optionally at least mono-substituted C₁₁₋₂₀-alkyl radical, a napthyl group, which is at least mono substituted, a quinolinyl group, which is at least mono-substituted, a pyrrolyl group, which is at least mono-substituted by a substituent other than C₁₋₅-alkyl, an optionally at least mono-substituted thiazolyl-, benzo[b]-thiophenyl-, benzo[b]-furanyl-, isoquinolinyl-, tetrahydroisoquinolinyl-, pyrazolyl-, isoazolyl-, chromanyl-, benzothiadiazolyl-, imidazolyl-, benzofurazanyl-, dibenzo[b,d]-furanyl-, benzoxadiazolyl-, imidazo[2,1-b]-thiazolyl-, anthracenyl-, coumarinyl-, 2,3-Dihydro-1,4-benzodioxinyl-, 2,3-Dihydrobenzo[b]furanyl-, 3,4-Dihydro-2H-1,4-Benzoxazinyl-, 3,4-Dihydro-2H-1,5-Benzodioxepinyl-, Benzothiazolyl-, Imidazo[1,2-a]-pyridinyl-, a chromonyl- group, an isatinyl group, a pentamethyldihydrobenzofuranyl group, an optionally at least mono-substituted cyclopropyl- or cyclopentyl-group, a 2-(1,3-Dioxo-1,3-dihydro-isoindol-2-yl-)-ethyl, a thienyl group, which is at least mono-substituted by one or more substituents independently selected from the group consisting of F, Cl, Br, C₁₋₅-alkoxy-, CF₃, -SO₂-C₁₋₅-alkyl and optionally at least mono substituted benzoylaminomethyl-, phenylsulfonyl-, isoxazolyl-, benzamidomethyl-, pyrimidyl-, thiazolyl-, pyrazolyl-, phenyl-, 1,2,4-thiadiazolyl-, 1,3-oxazolyl- or 1,2,4-oxadiazolyl-, a furyl group, which is at least mono-substituted by one or more subsitutents independently selected from the group consisting of a C₁₋₅-alkyl radical, which may be at least partially fluorinated or chlorinated, an optionally at least mono-substituted phenyl and a -(C=O)-O-C₁₋₅-alkyl group,
a NR¹⁶R¹⁷-moiety,
a COR¹⁸-moiety,
or a phenyl radical, which is at least mono-substituted with one of the substituents selected from the group consisting of:
2,2,2,-Trifluoroethoxy-, C₂₋₆-Alkenyl-, 1,3-Dihydro-1-oxo-2H-isoindol-2-yl-, N-Phthalimidinyl-, [(2-chloro-1,3-thiazolyl-5-yl)-methoxy, Ethyl-5-yl-2-methyl-3-furoate, C₁₁₋₂₀-alkyl-, 1,3-Dioxo-2-azaspiro[4,4]non-2-yl-, pyrazolyl-, (1,3-oxazol-5-yl)-, (5-Methyl-1,3,4-oxadiazol-2-yl)-, difluoromethoxy, dichloromethoxy, 1-pyrrolidinylsulfonyl, morpholinosulfonyl, 2-methyl-4-pyrimidinyl-, a phenoxy group, which is at least mono-substituted with C₁₋₅-alkoxy, a phenyl group, which is at least mono-substituted with one of the substituents selected from the group consisting of nitro, C₁₋₅-alkoxy, F, Cl, Br, at least partially fluorinated C₁₋₅-alkyl, at least partially chlorinated C₁₋₅-alkyl, [(2-Chloro-1,3-thiazol-5-yl)-methoxy]-, -(C=O)-H and -(C=O)-C₁₋₅-alkyl, a pyridinyl group, which is at least mono-substituted with C₁₋₅-alkoxy, a pyridinyloxy group, which is at least mono-substituted with C₁₋₅-alkoxy, a phenoxy group, which is at least di-substituted and a pyridinyloxy group, which is at least di-subsituted,
more preferably W represents a moiety selected from the group consisting of 5-Dimethylamino-napth-1-yl, 2-Acetamido-4-methyl-5-thiazolyl-, Trifluoromethyl-, Trichloromethyl-, lsopropyl-, Methyl-, 2,2,2-Trifluoroethyl-, Ethyl-, Hexadecyl-, 2-Chloroethyl-, n-Propyl-, 3-Chloro-propyl-, n-Butyl-, Dichloromethyl-, Chloromethyl-, Dodecyl-, 1-Octyl-, 6-(p-toluidino)-naphth-2-yl-, 4,5-Dibromo-thiophene-2-yl-, Benzoylchloride-2-yl-, 1-Octadecyl-, 4-Bromo-2,5-dichloro-thiophene-3-yl-, 2,5-Dichloro-thiophene-3-yl-, 5-Chloro-thiophene-2-yl-, 1-Decyl-, 3,5-Dichloro-4-(2-chloro-4-nitrophenoxy)-phenyl-, 2,3-Dichlorothiophene-5-yl-, 3-Bromo-2-chloro-thiophene-5-yl-, 3-Bromo-5-chloro-thiophene-2-yl-, 2-(Benzoylaminomethyl)-thiophene-5-yl-, 4-(Phenyl-sulphonyl)-thiophene-2-yl-, 2-Phenyl-sulphonyl-thiophene-5-yl-, 2-[1-Methyl-5-(trifluoromethyl)pyrazol-3-yl]-thiophene-5-yl-, 5-Chloro-1,3-dimethylpyrazole-4-yl-, 3,5-Dimethylisoxazole-4-yl-, 2-(2,4-Dichlorophenoxy)-phenyl, 4-(2-Chloro-6-nitrophenoxy)-phenyl-, 4-(3-chloro-2-cyanophenoxy)-phenyl, 2,4-Dimethyl-1,3-thiazole-5-yl-, Methyl-methane-sulfonyl-, 2,5-Bis-(2,2,2-Trifluoroethoxyrphenyl-, 5-(Di-n-propylamino)-naphth-1-yl-, 2,2,5,7,8-Pentamethyl-chroman-6-yl-, 5-Chloro-4-nitrothiophene-2-yl-, 2,1,3-Benzothiadiazole-4-yl-, 1-Methyl-imidazole-4-yl-, Benzofurazan-4-yl-, 5-(lsoxazol-3-yl)-thiophene-2-yl-, Vinyl-phenyl-4-yl-, 5-Dichloromethyl-furan-2-yl-, 5-Bromo-thiophene-2-yl-, 5-(4-Chlorobenzamidomethyl)-thiophene-2-yl-, Dibenzo[b,d]-furan-2-yl-, 5-Chloro-3-methylbenzo[b]-thiophene-2-yl-, 3-Methoxy-4-(methoxycarbonyl)-thiophene-2-yl-, 5-[2-(Methylthio)-pyrimidin-4-yl-]-thiophene-2-yl-, 4-Chloro-2,1,3-Benzoxadiazole-7-yl-, 5-Chloro-2,1,3-Benzoxadiazole-4-yl-, 6-Chloro-imidazo(2,1-B)-thiazole-5-yl-, 3-Methyl-benzo[b]-thiophene-2-yl-, 4-[[3-Chloro-5-(Trifluoromethyl)-2-pyridyl]oxy-phenyl-, 5-Chloronaphth-1-yl-, 5-Chloro-naphth-2-yl-, 9,10-Dibromoanthracene-2-yl-, Isoquinoline-5-yl-, 4-Methoxy-2,3,6-trimethylbenzoyl-, 4'-Nitro-biphenyl-4-yl-, (1,3-Dihydro-1-oxo-2H-isoindol-2-yl-)-4-phenyl-, 5-(2-Methyl-1,3-thiazole-4-yl)-thiophene-2-yl-, 5-(1-Methyl-3-(trifluoromethyl)pyrazol-5-yl-]-thiophene-2-yl-, 5-[5-Trifluoromethyl)-isoxazol-3-yl]-thiophene-2-yl-, p-Dodecyl-phenyl-, 4-[(3-Cyano-4-methoxy-2-pyridinyl)oxy]-phenyl-, 4-(N-phthalimidinyl)-phenyl-, 1,2,3,4-Tetrahydro-2-(trifluoroacetyl)-isoquinoline-7-yl-, 1,2-Dimethylimidazole-4-yl-, 2,2,4,6,7-Pentamethyldihydrobenzofuran-5-yl-, 4-Chloronaphth-1-yl-, 2,5-Dichloro-4-nitro-thiophene-3-yl-, 4-(4-Methoxy-phenoxy)-phenyl-, [4-(3,5-Dichlorophenoxy)phenyl]-, [4-(3,4-Dichlorophenoxy)phenyl]-, [4-(3,5)-Bis(trifluoromethylphenoxy)phenyl]-, 3-(2-Methoxy-phenoxy)-phenyl, 3-(4-Methoxy-phenyl)-phenyl-, 3-(4-Chloro-phenyl)-phenyl-, 3-(3,5-Dichloro-phenyl)-phenyl-, 3-(3,4-Dichloro-phenyl)-phenyl-, 3-(4-Fluorophenyl)-phenyl-, 3-[4-(Trifluoromethyl)-phenyl]-phenyl-, 3-[3,5-Bis-(Trifluoromethyl)-phenyl]-phenyl-, 4-(2-Methoxy-phenoxy)-phenyl-, 4-(2-Methyl-phenoxy)-phenyl-, 4-(4-Methoxy-phenoxy)-phenyl-, 4-(4-Chlorophenyl)-phenyl-, 4-(3,5-Dichlorophenyl)-phenyl-, 4-(3,4-Dichlorophenyl)-phenyl-, 4-(4-Fluorophenyl)-phenyl-, 4-[4-(Trifluormethyl)-phenyl]-phenyl-, 4-[3,5-Bis-(Trifluoromethyl)-phenyl]-phenyl-, Cyclopropyl-, 2-(2-Chlorophenyl)-2-Phenylethyl-, 2-(2-Trifluoromethylphenyl)-2-phenylethyl-, 5-[4-Cyano-1-methyl-5-(methylthio)-1H-pyrazol-3-yl-thiophene-2-yl-, 3-Cyano-2,4-bis-(2,2,2-Trifluorothoxy)-phenyl-, 4-[(2-Chloro-1,3-Thiazol-5-yl)-methoxy]-phenyl-, 2-(1,3-Dioxo-1,3-dihydro-isoindol-2-yl)-ethyl-, 5-lodo-naphth-1-yl-, Ethyl-2,5-dimethyl-1-phenylpyrrole-4-carboxylate-3-yl-, Ethyl-2-methyl-1,5-diphenyl-1 H-pyrrole-3-carboxylate-4-yl-, Ethyl-5-(4-chlorophenyl)-2-methyl-3-furoate-4-yl, Ethyl-5-(4-chlorophenyl)-2-methyl-1-phenyl-3-carboxylate-4-yl-, Ethyl-2,5-dimethyl-3-furoate-4-yl-, 3-Chloro-4-(1,3-dioxo-2-Azaspiro[4,4]non-2-yl)-phenyl-, Coumarin-6-yl, 3-(4-Methoxy-phenoxy)-phenyl-, [3-(3,5-Dichlorophenoxy)]-phenyl-, [3-(3,4 Dichlorophenoxy)]-phenyl-, 3,5-Bis(Trifluoromethyl)phenoxyphenyl-, 2,2-Diphenylethyl-, 4-Phenyl-5-(trifluoromethyl)-thiophene-3-yl-, Methyl-4-Phenyl-5-(Trifluoromethyl)-thiophene-2-carboxylate-3-yl-, Methyl-1,2,5-trimethylpyrrole-3-Carboxylate-4-yl-, 4-Fluoro-naphth-1-yl-, 5-Fluoro-3-methylbenzo[b]-thiophene-2-yl-, Methyl-2,5-dimethyl-3-furoate-4-yl-, Methyl-2-furoate-5-yl-, Methyl-2-methyl-3-furoate-5-yl-, Methyl-1-methyl-1H-pyrrole-2-Carboxylate-5-yl-, 2-(5-Chloro-1,2,4-Thiadiazol-3-yl)-thiophene-5-yl-, 1,3,5-Trimethyl-1H-pyrazole-4-yl-, Pentafluoroethoxytetrafluoroethyl-, 5-(5-Isoxazyl)-thiophene-2-yl-, 5-(5-lsoxazol-yl)-2-furyl-, 5-Methyl-2,1,3-benzothiadiazole-4-yl-, 2,3-Dihydro-1,4-benzodioxine-6-yl-, 4-Methyl-Naphth-1-yl-, 5-Methyl-2-(Trifluormethyl)-3-Furyl-, 2,3-Dihydrobenzo[b]furan-5-yl-, 1-Benzothiophene-3-yl-, 4-Methyl-3,4-dihydro-2H-1,4-Benzoxazine-7-yl-, 5-Methyl-1-phenyl-1H-pyrazole-4-yl-, 6-Morpholino-3-Pyridinyl-, 4-(1H-Pyrazol-1-yl)-phenyl-, 6-Phenoxy-3-Pyridyl-, 3,4-Dihydro-2H-1,5-benzodioxepine-7-yl-, 5-(1,3-Oxazol-5-yl)-2-thienyl-, 4-(1,3-Oxazol-5-yl)-phenyl-, 5-Methyl-4-isoxazolyl, 2,1,3-Benzothiadiazole-5-yl-, 5-Acetamido-naphth-1-yl-, 3-Methyl-8-Quinolinyl-, 1,3-Benzothiazole-6-yl-, 2-Morpholino-3-Pyridyl-, 2,5-Dimethyl-3-thienyl-, 5-[5-(Chloromethyl)-1,2,4-oxadiazol-3-yl]-2-thienyl-, Ethyl-3-[5-yl-2-thienyl-]1,2,4-oxadiazole-5-carboxylate-, 3-(5-Methyl-1,3,4-oxadiazol-2-ylrphenyl-, 4-(Difluoromethoxy)-phenyl-, 3-(Difluoromethoxy)-phenyl-, 2,2-Dimethyl-6-Chromanyl-, Ethyl-3,5-dimethyl-1H-pyrrole-2-carboxylate-4-yl-, lmidazo[1,2-A]pyridine-3-yl-, 3-(1,3-Oxazol)-5-yl)-phenyl)-, Ethyl-5-[4-yl)-phenyl]-2-methyl-3-furoate, 1-Pyrrolidinylphenylsulfonyl-, Methyl-5-yl-4-methyl-2-thiophene-carboxylate, Methyl-3-yl-4-(isopropylsulfonyl)-2-thiophene, 7-Chlorochromone-3-yl-, 4'-Bromobiphenyl-4-yl-, 4'-Acetyl-biphenyl-4-yl-, 4'-Bromo-2'-fluoro-biphenyl-4-yl-, 1-Methyl-5-isatinyl-, 2-Chloro-3-thiophenecarboxylic-acid-5-yl-, 2-Methoxy-5-(N-phthalimidinyl)-phenyl-, 1-Benzothiophene-2-yl-, Morpholinophenylsulfonyl- and 3-(2-Methyl-4-pyrimidinyl)-phenyl- and R¹-R¹⁸ have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Furthermore, compounds of general formula (I) are preferred, wherein R¹⁰ represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical,
more preferably H, -CH₃, -C₂H₅ or phenyl,
and R¹-R⁹, R¹²-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Moreover, compounds of general formula (I) are preferred, wherein R¹¹ represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably H, a linear or branched C₁₋₄-alkyl radical, a cyclohexyl radical or a phenyl radical, more preferably H, -CH₃, -C₂H₅ or phenyl,
and R¹-R¹⁰, R¹²-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Preference is also given to compounds of general formula (I), wherein R¹² represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system,
preferably represents H, a linear or branched C₁₋₄-alkyl radical, a cyclohexyl radical or a phenyl radical,
more preferably H, -CH₃, -C₂H₅ or phenyl,
and R¹-R¹¹, R¹³-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein R¹³ and R¹⁴ are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably are each independently selected from the group consisting of H, a linear or branched C₁₋₄-alkyl radical, a cyclohexyl radical and a phenyl radical,
more preferably are each independently selected from the group consisting of H, CH₃, C₂H₅ and phenyl,
and R¹-R¹², R¹⁵-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Furthermore, compounds of general formula I are preferred, wherein R¹³ and R¹⁴ together with the bridging nitrogen atom form a saturated, unsaturated or aromatic, 5- or 6-membered heterocyclic ring, which may be at least mono-substituted and/or contain at least one further heteroatom as a ring member,
preferably form an unsubstituted piperidin or morpholine group,
and R¹-R¹², R¹⁵-R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein R¹⁵ represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical,
more preferably represents H, -CH₃, -C₂H₅ or phenyl,
and R¹-R¹⁴, R¹⁶, R¹⁷, R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein R¹⁶ represents an unbranched or branched, saturated or unsaturated, optionally at least mono-substituted C₁₋₆ aliphatic radical,
preferably an unbranched or branched, saturated, unsubstituted C₁₋₃ alkyl radical,
more preferably a methyl radical,
and R¹-R¹⁵ , R¹⁷ , R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein R¹⁷ represents an unbranched or branched, saturated or unsaturated, optionally at least mono-substituted C₁₋₆ aliphatic radical,
preferably an unbranched or branched, saturated, unsubstituted C₁₋₃ alkyl radical,
more preferably a methyl radical,
and R¹-R¹⁶ , R¹⁸ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein R¹⁸ represents a phenyl radical, which is optionally at least mono-substituted by a C₁₋₆ aliphatic radical, more preferably a phenyl radical, which is optionally at least mono-substituted by a methyl group,
and R¹-R¹⁷ and W have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Particularly preferred are compounds of general formula (I) selected from the following list A:
List A:

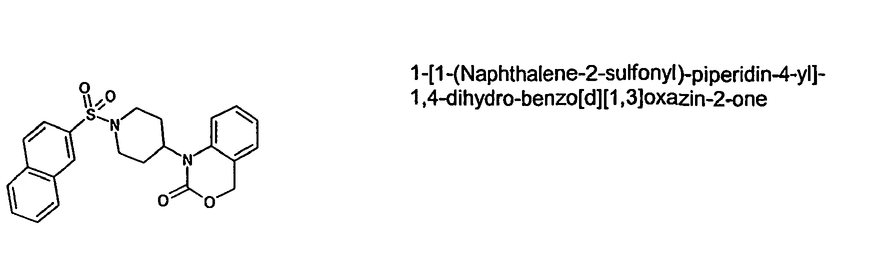

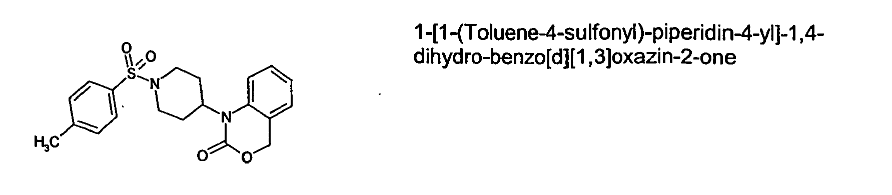

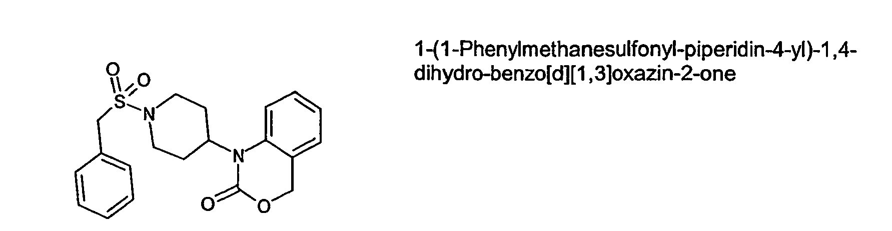

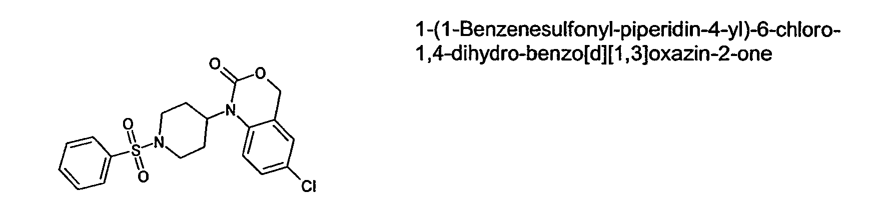

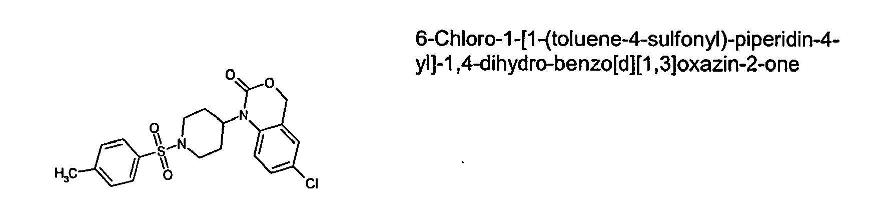

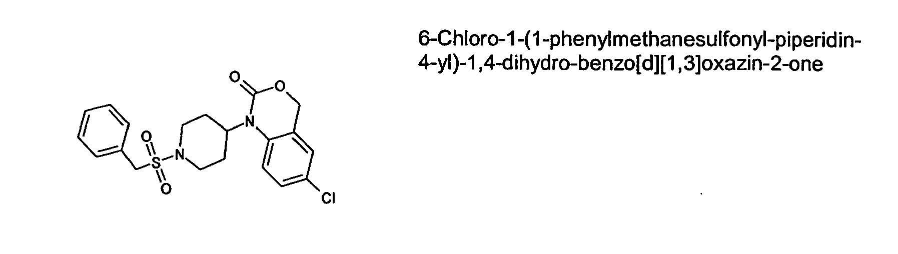

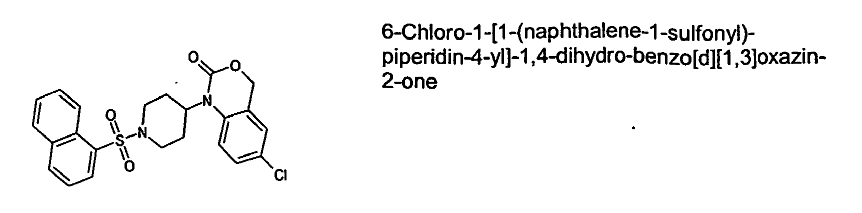

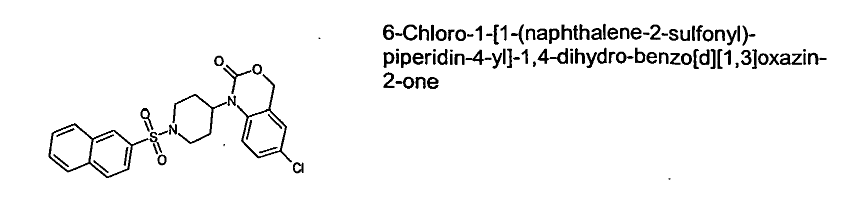

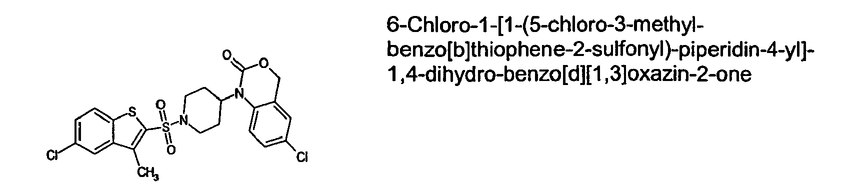

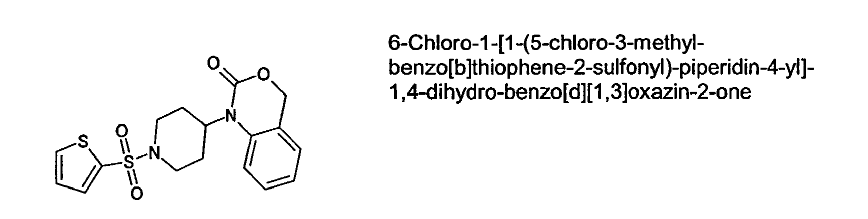

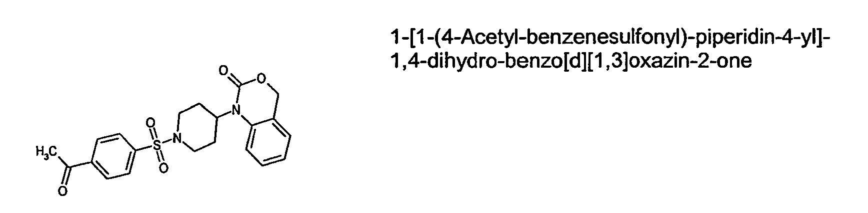

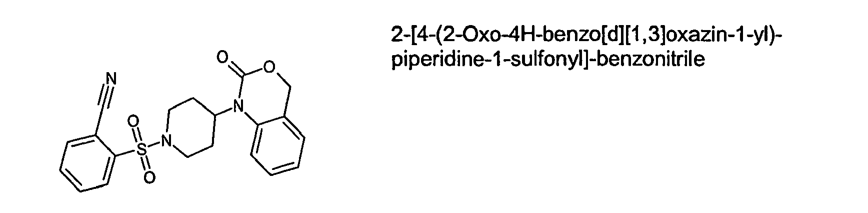

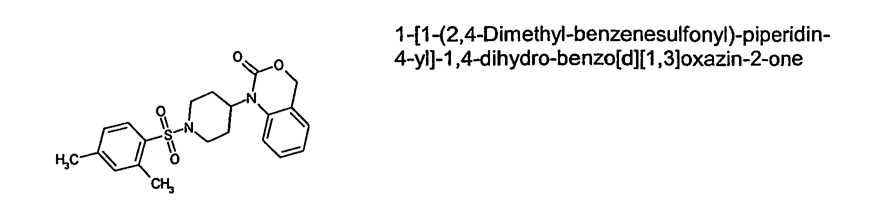

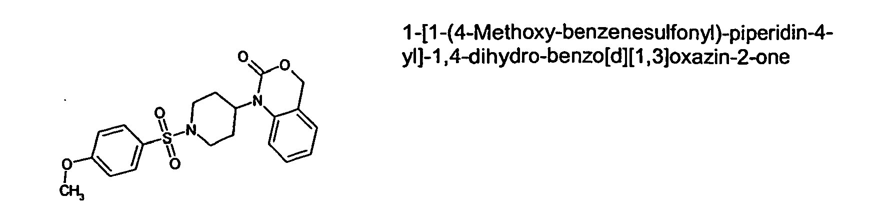

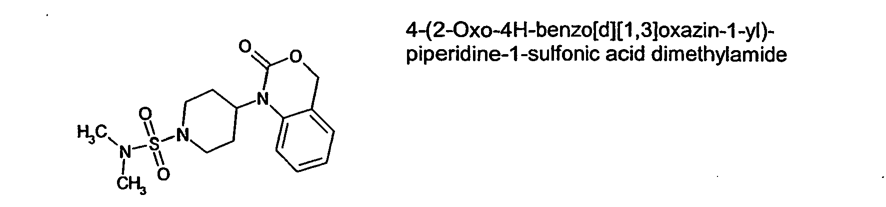

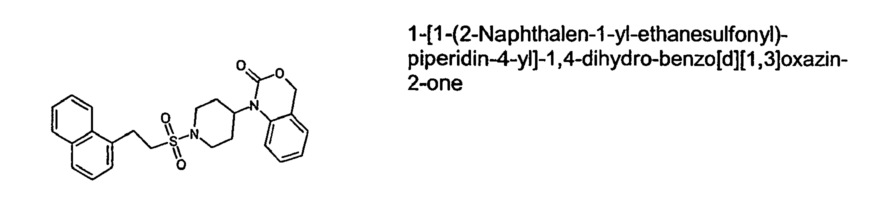

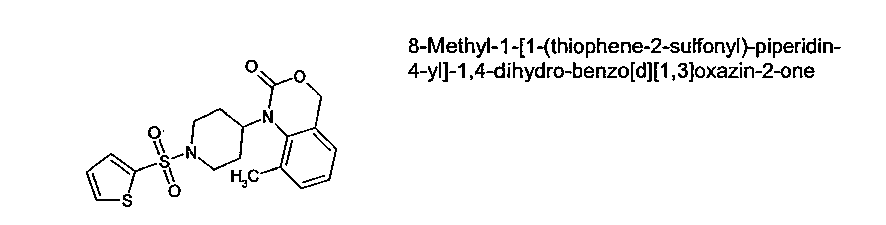

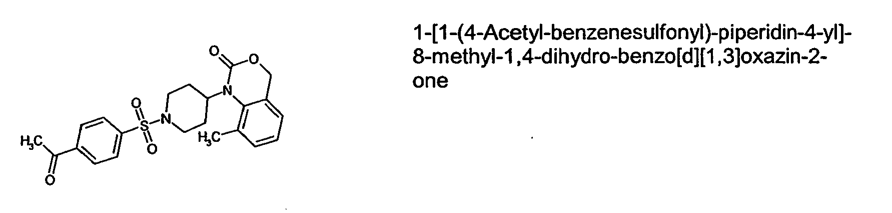

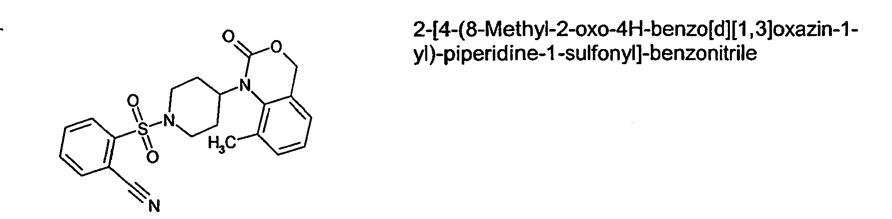

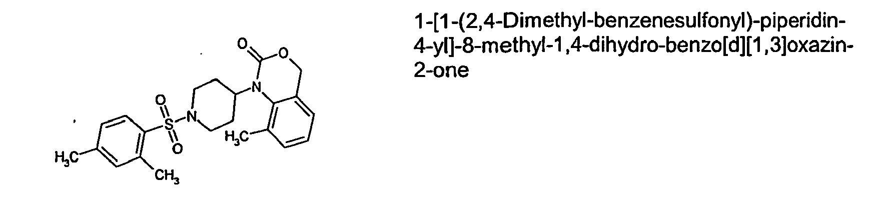

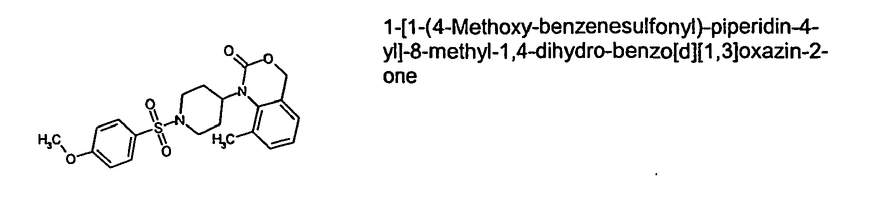

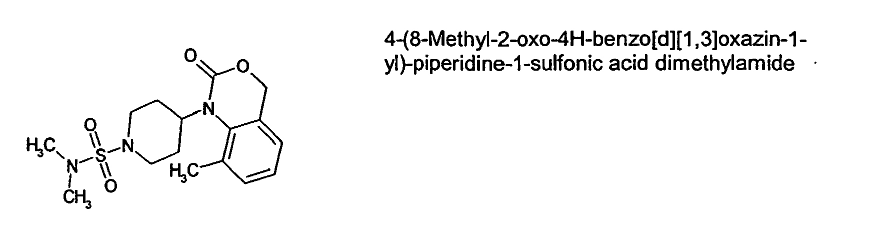

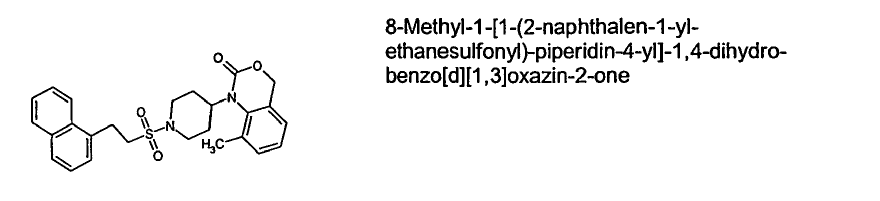

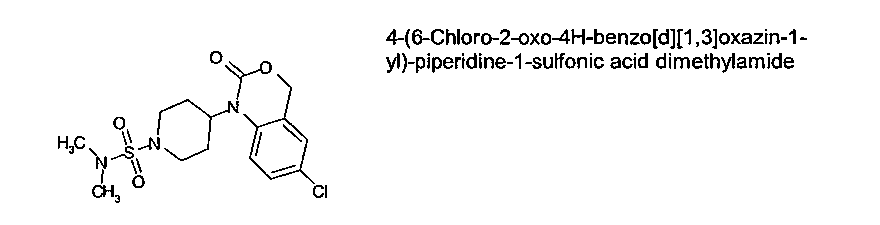

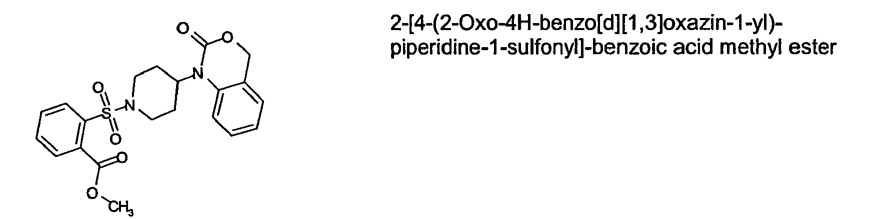

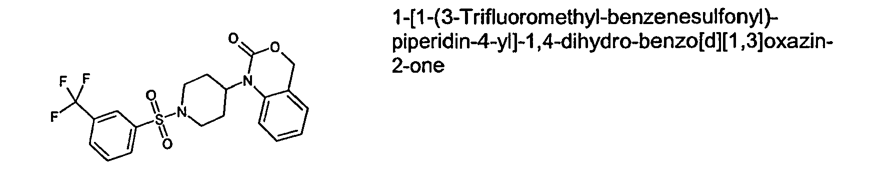

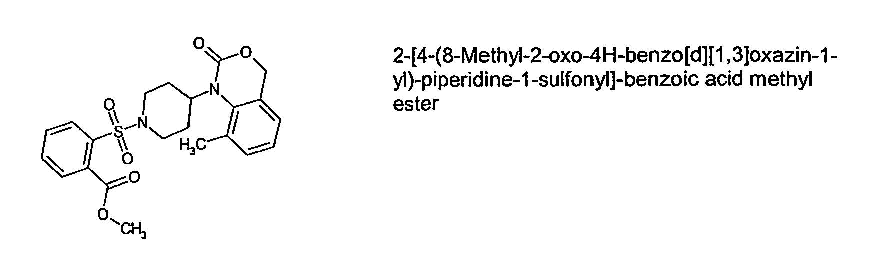

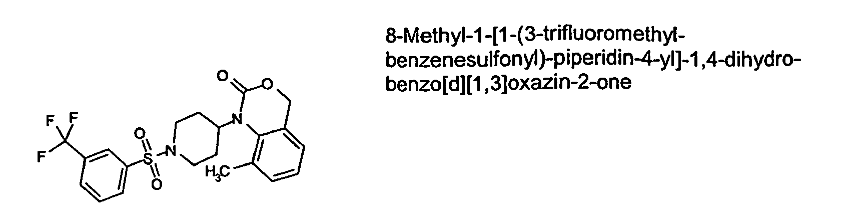

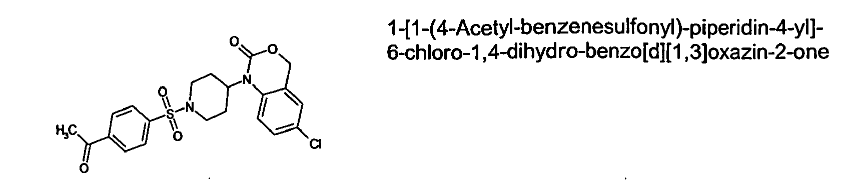

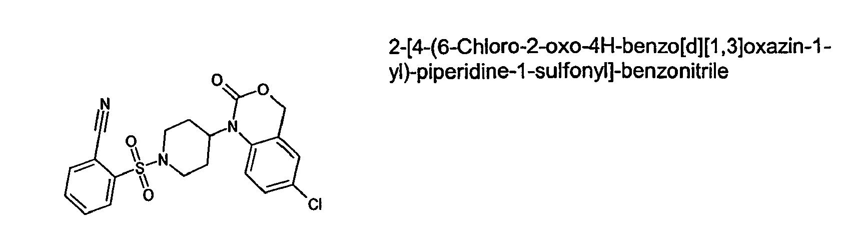

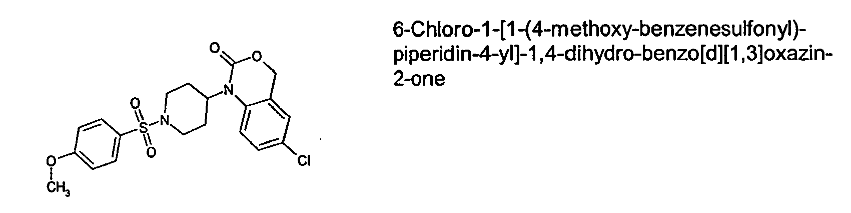

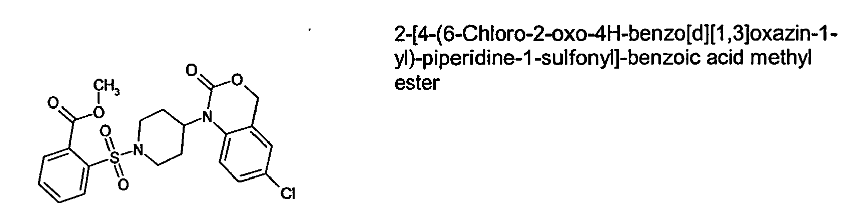

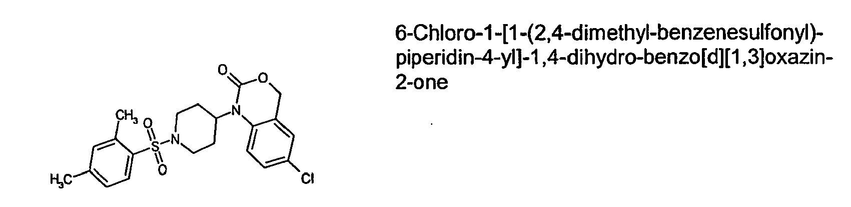

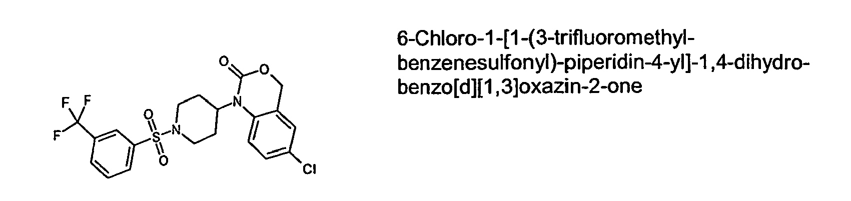

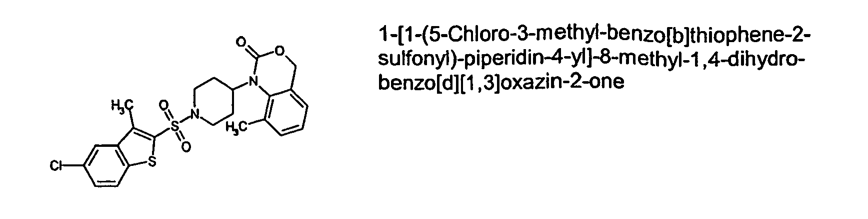

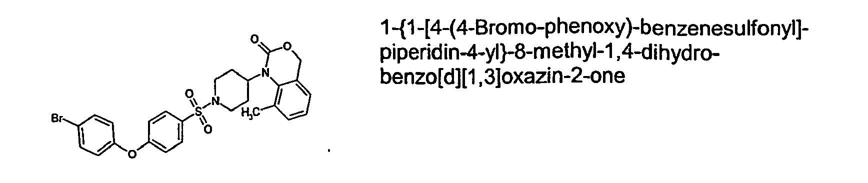

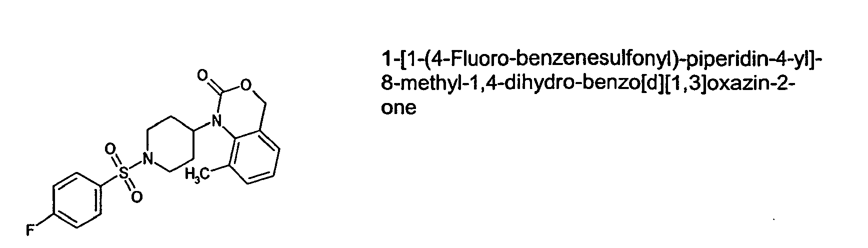

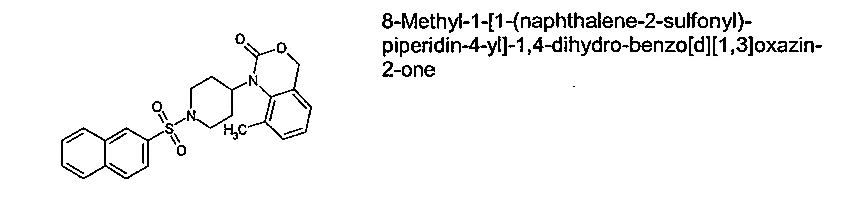

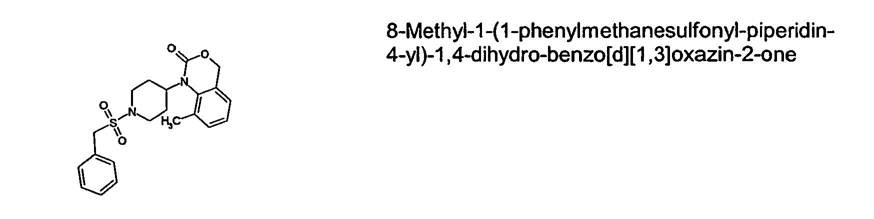

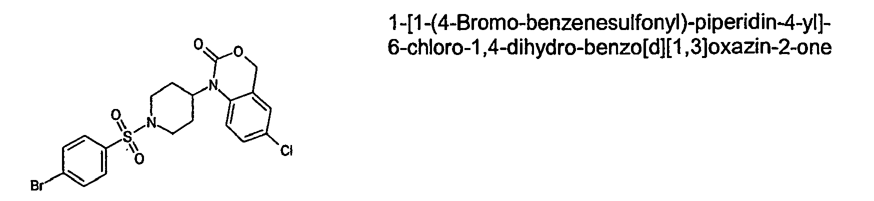

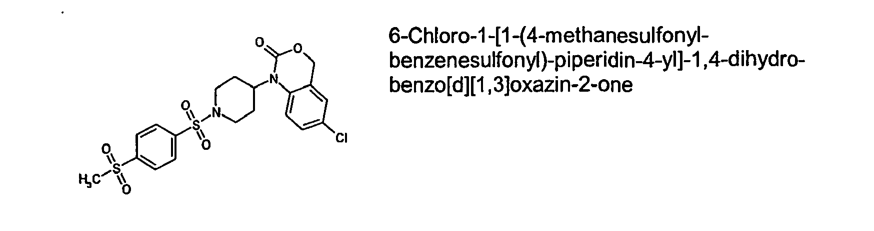

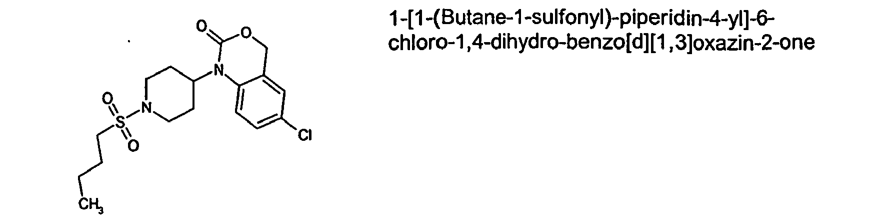

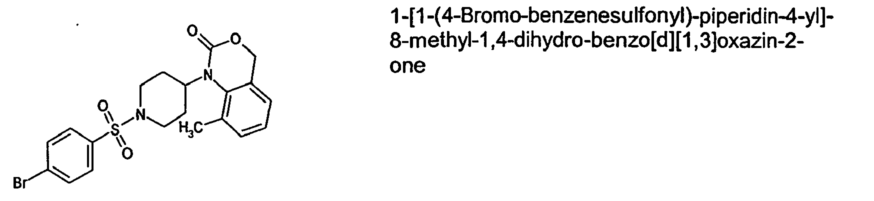

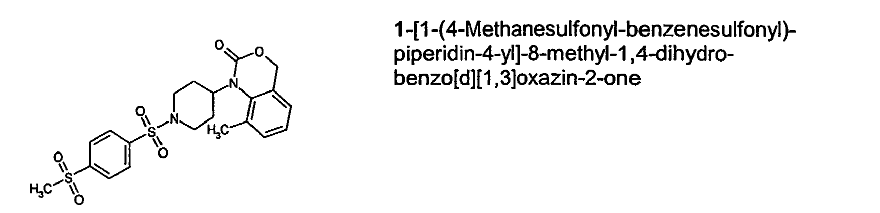

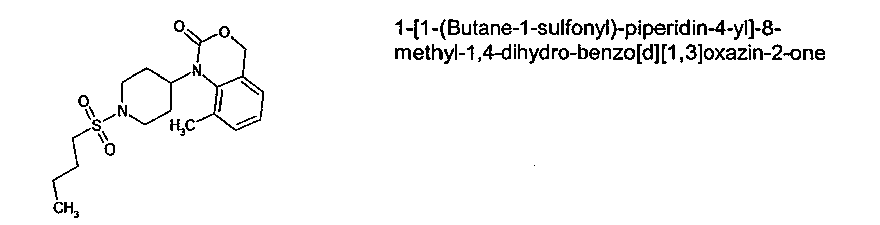

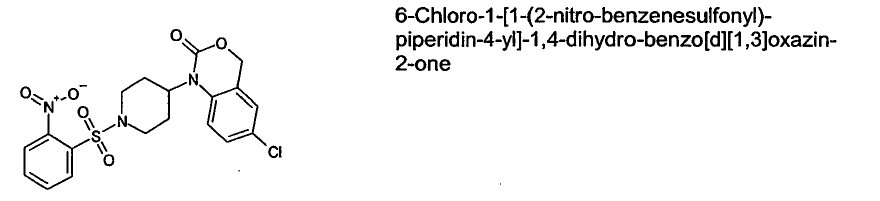

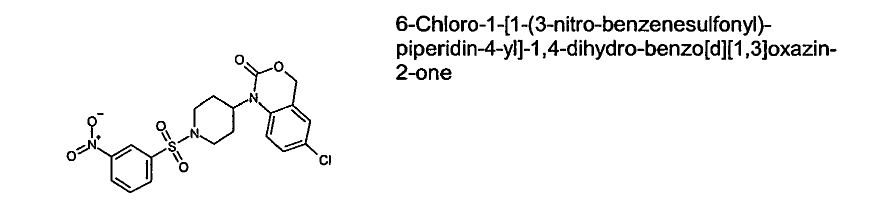

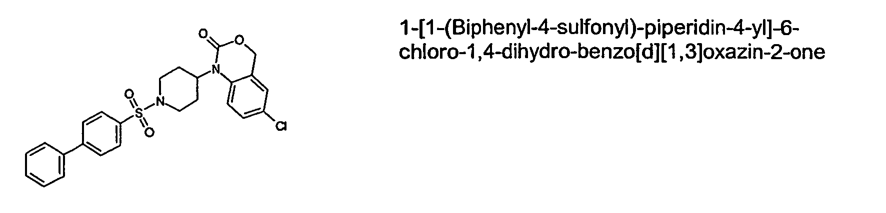

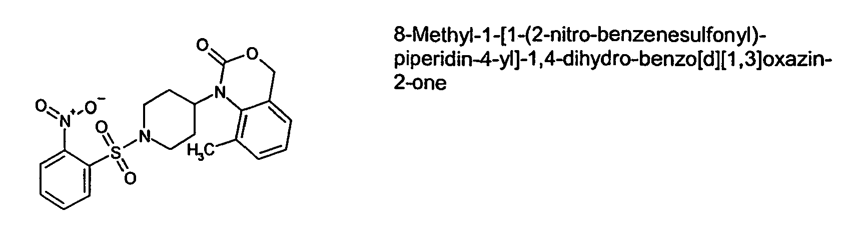

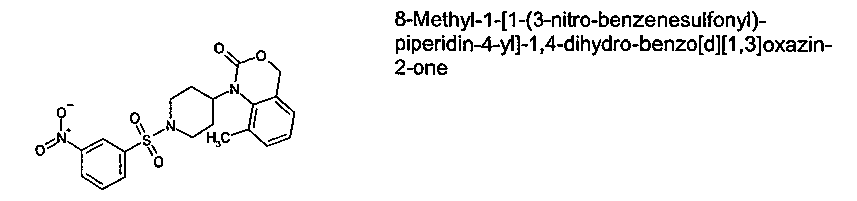

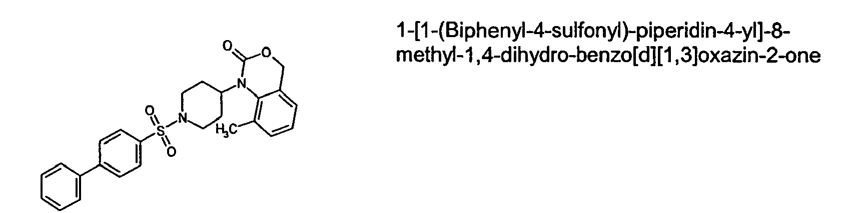

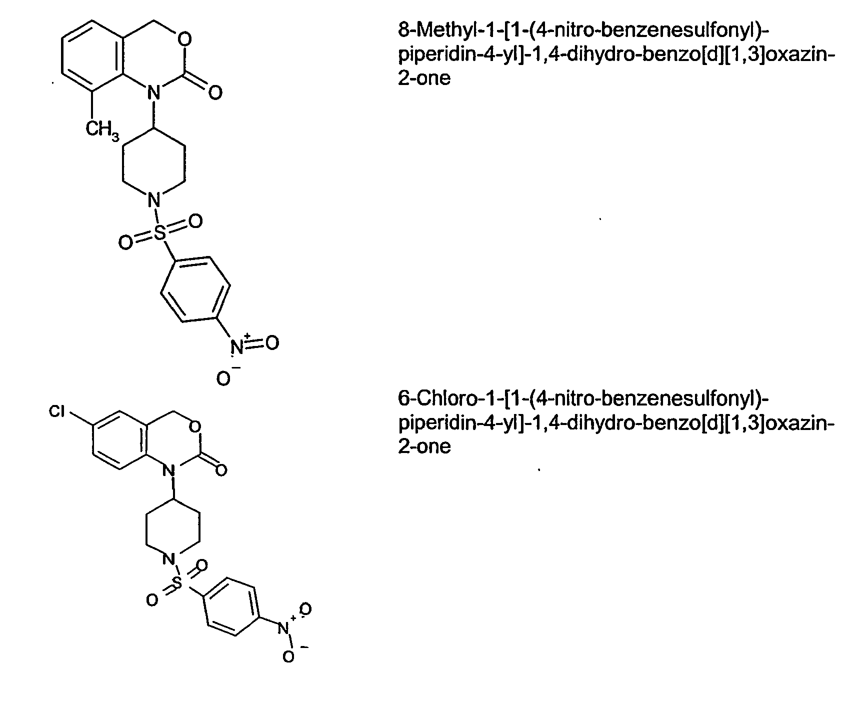

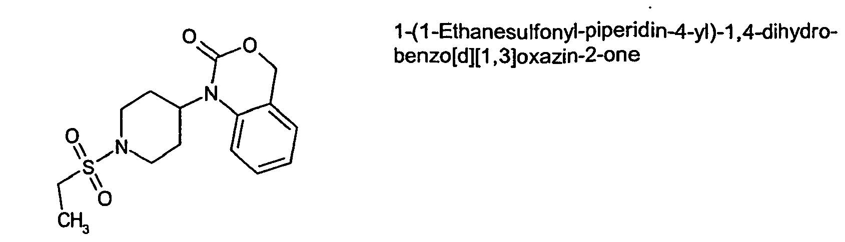

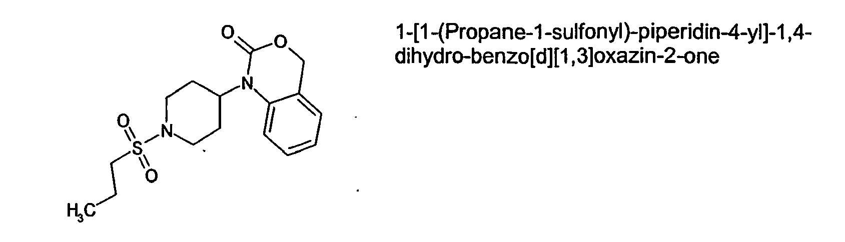

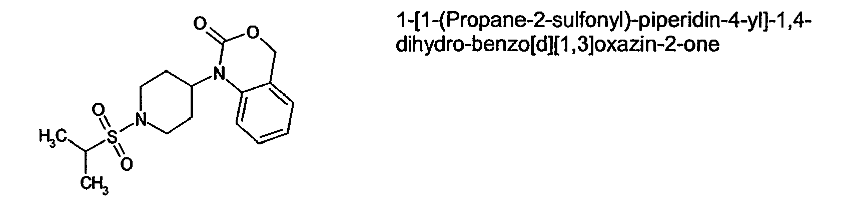

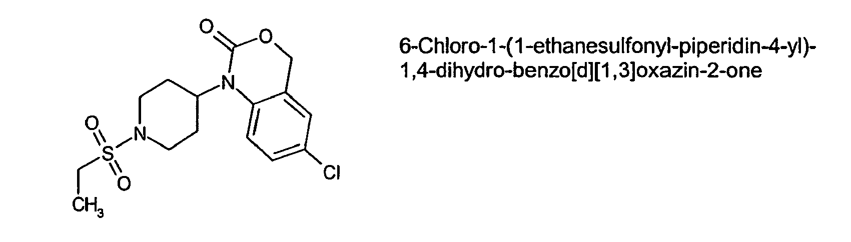

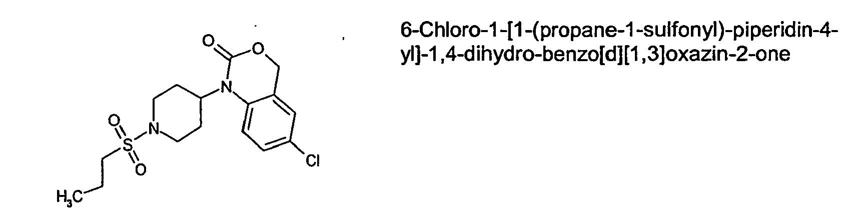

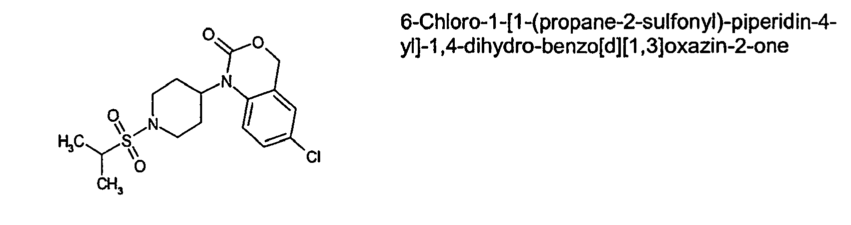

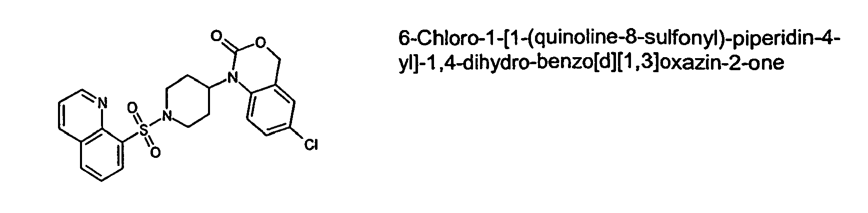

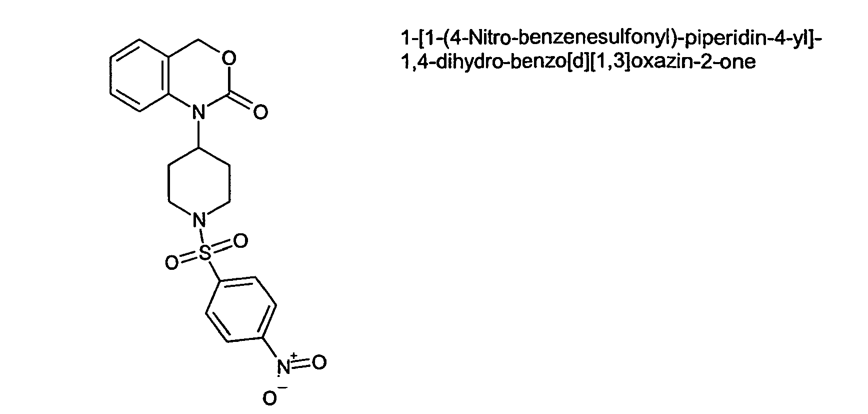

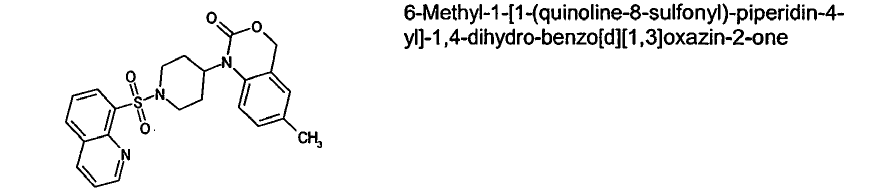

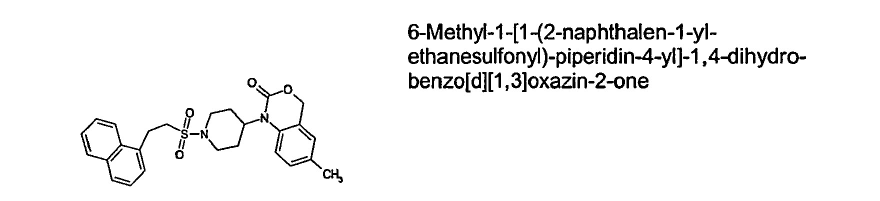

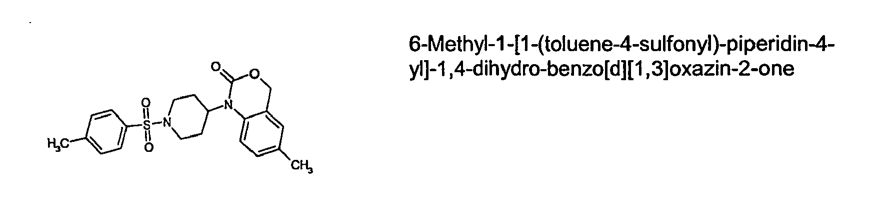

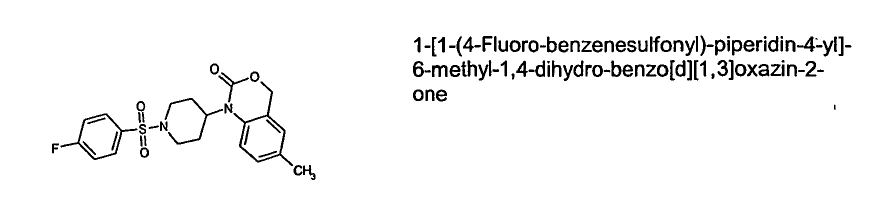

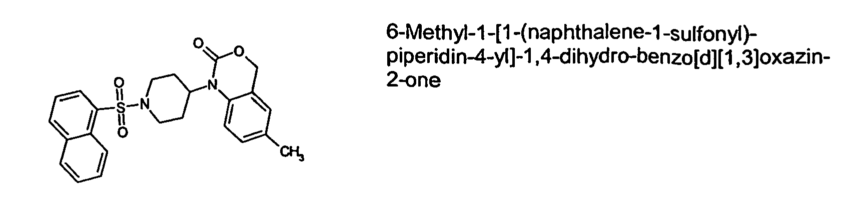

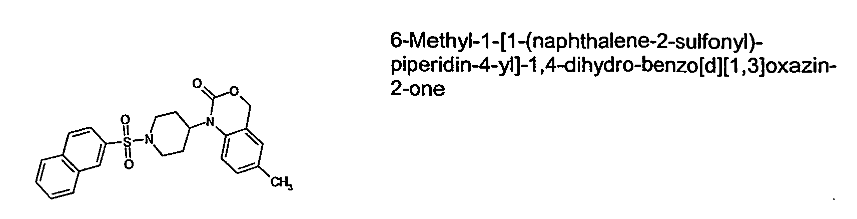

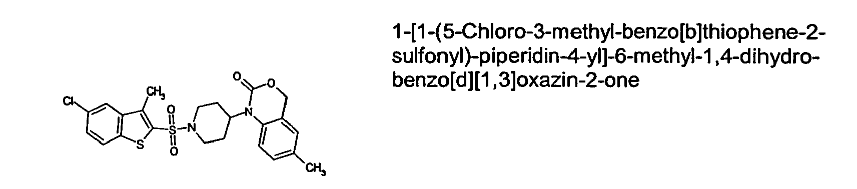

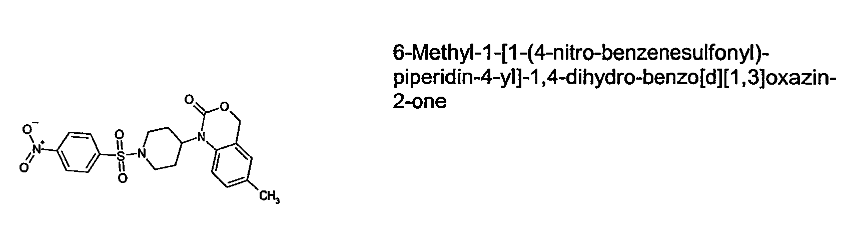

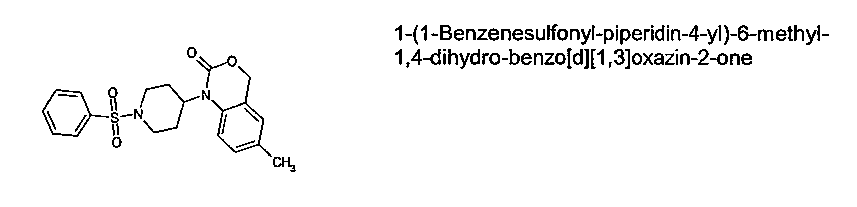

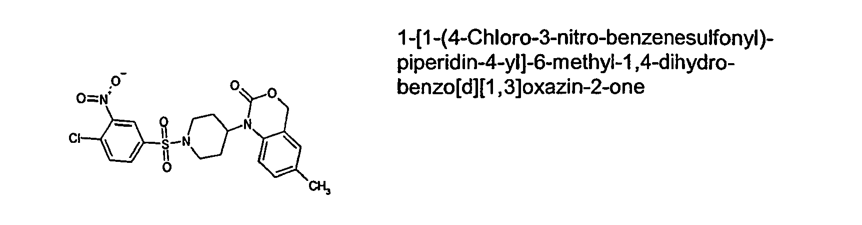

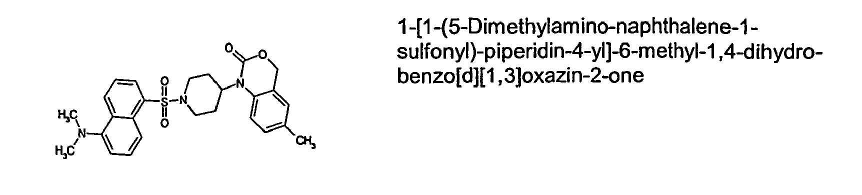

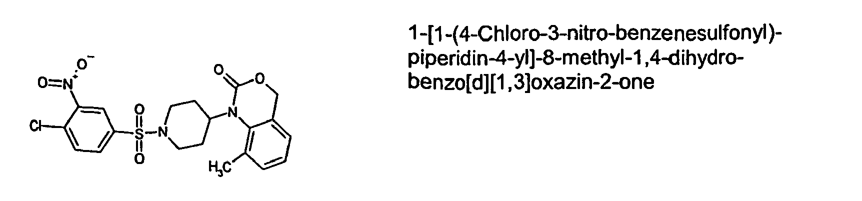

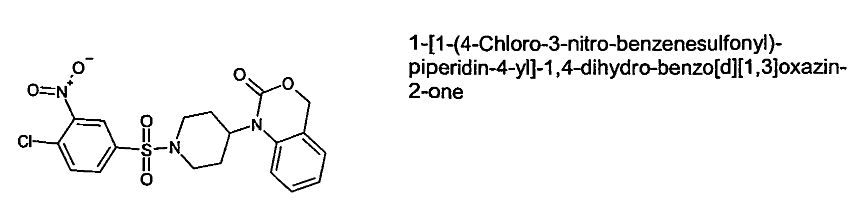

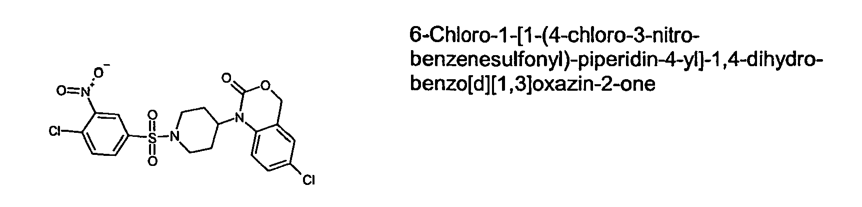

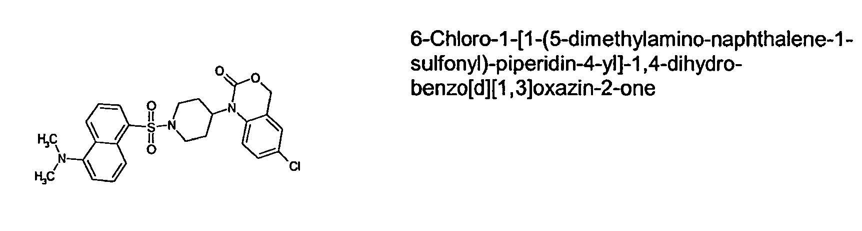

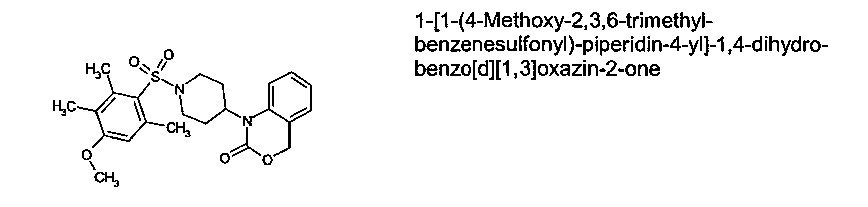

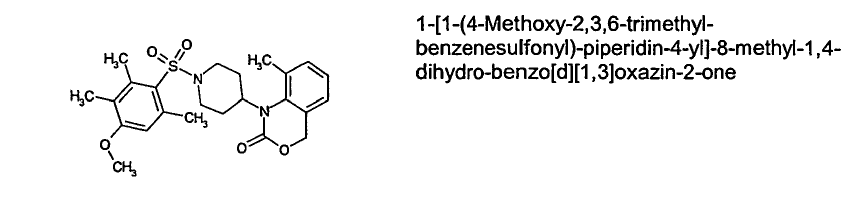

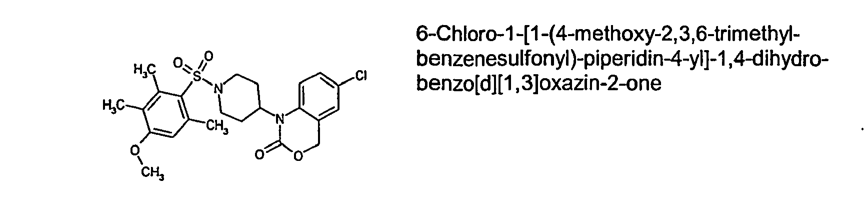

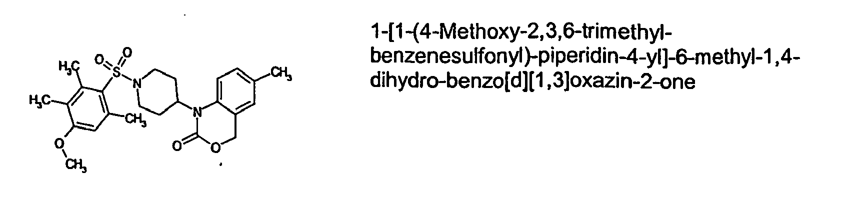

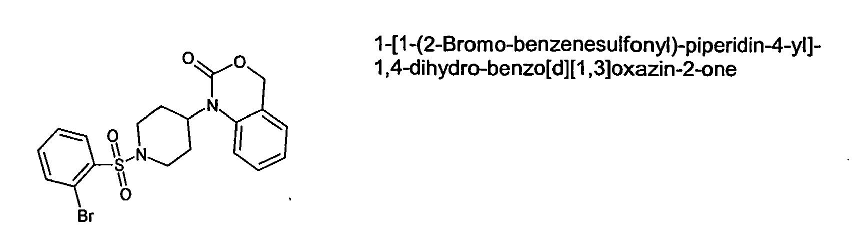

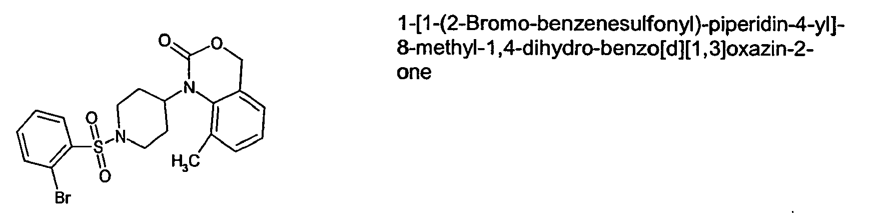

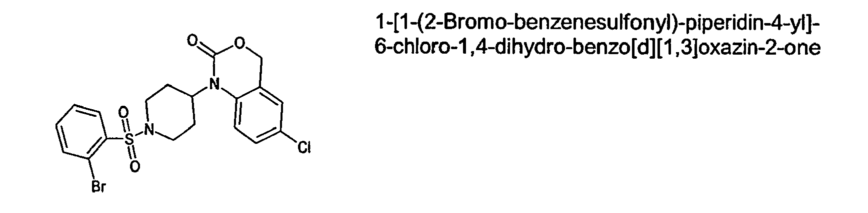

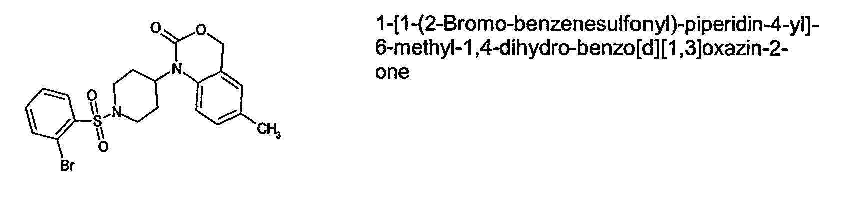

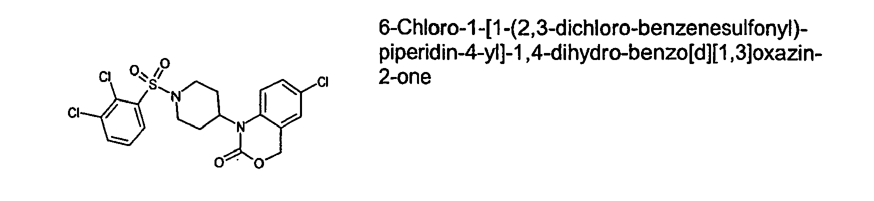

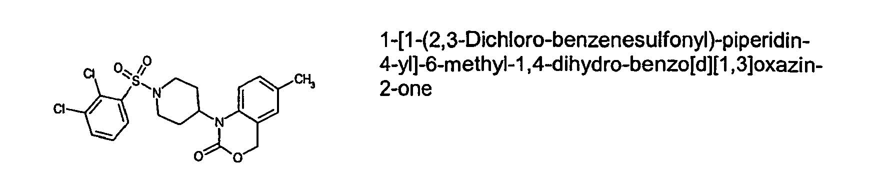

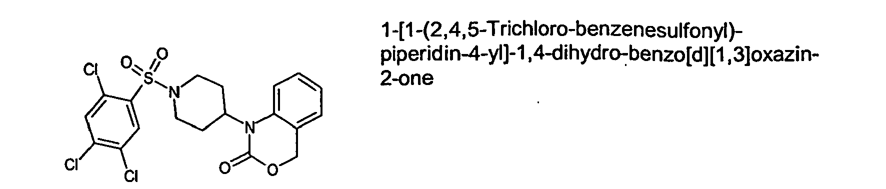

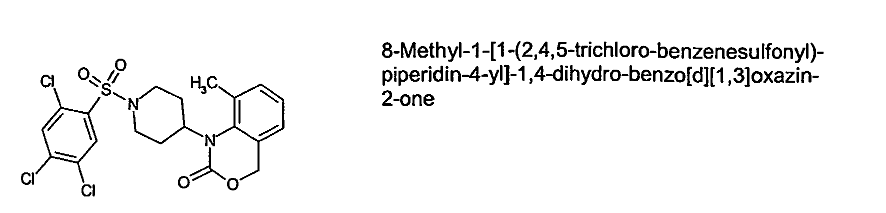

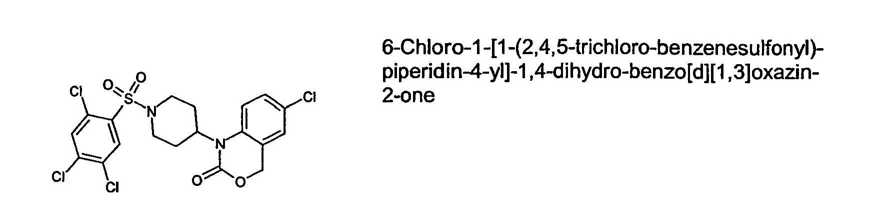

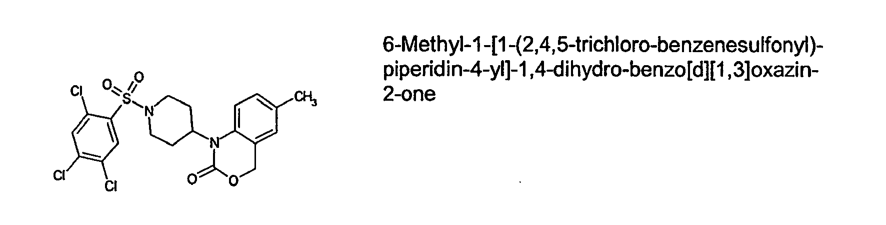

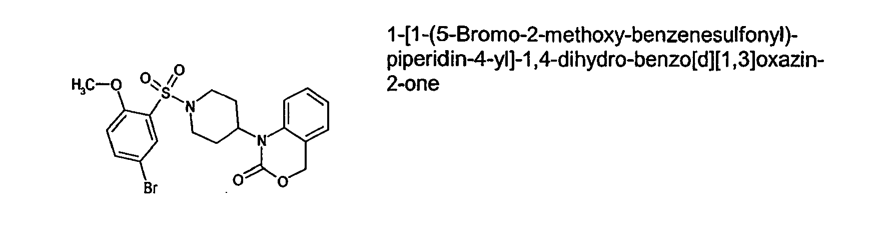

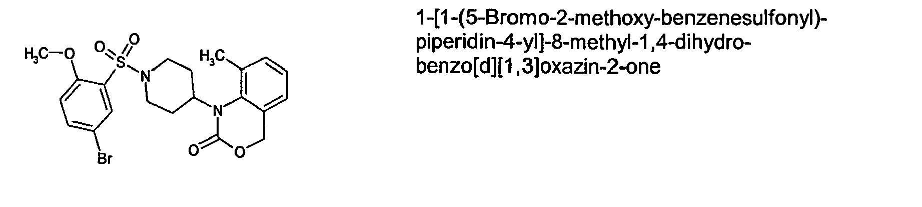

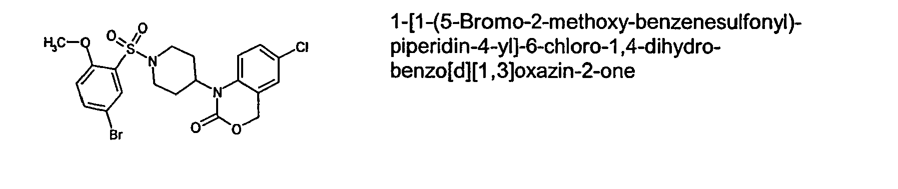

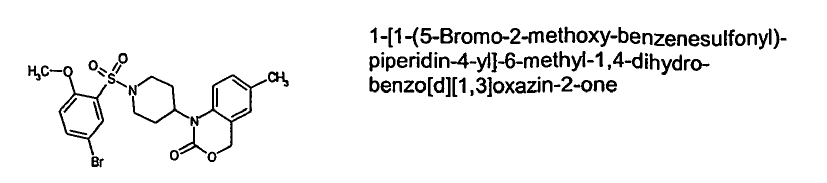

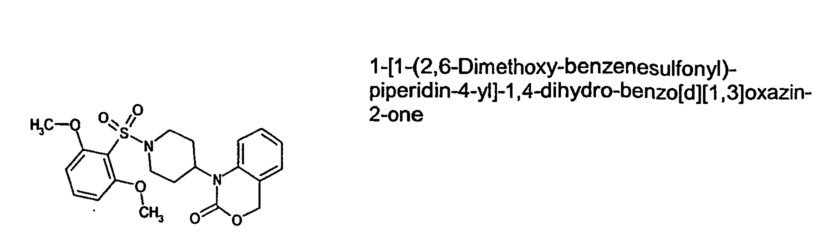

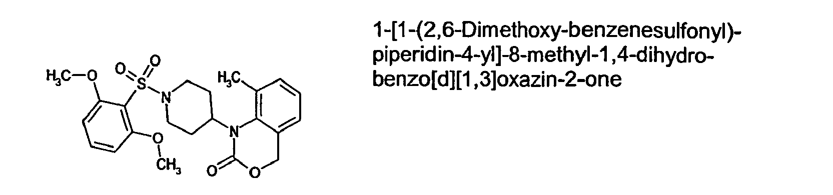

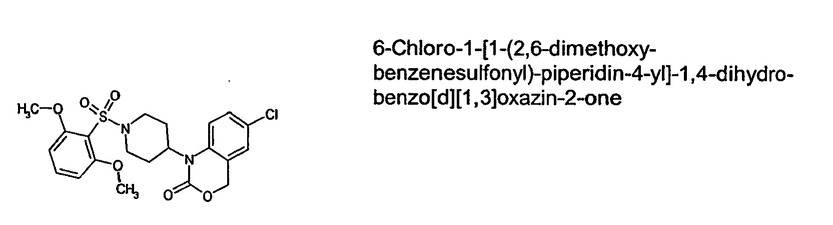

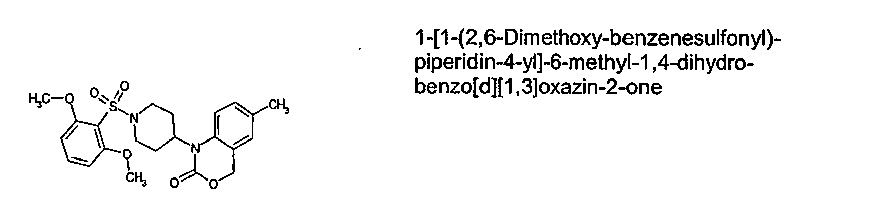

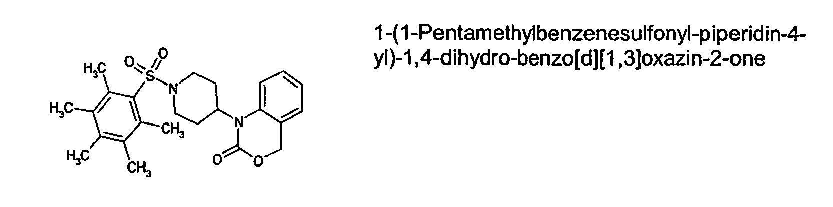

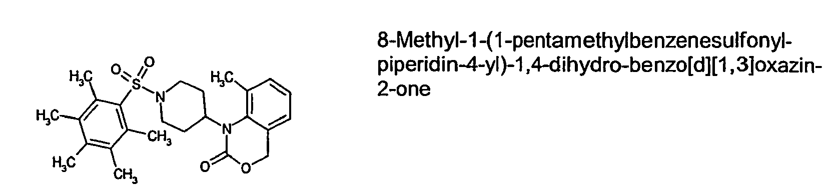

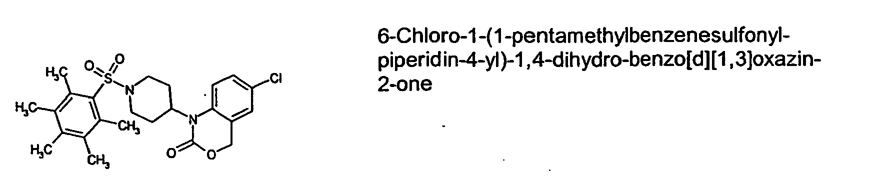

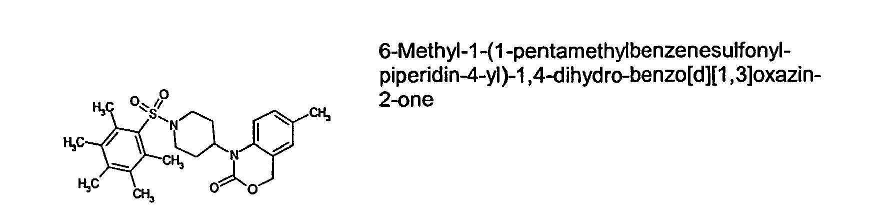

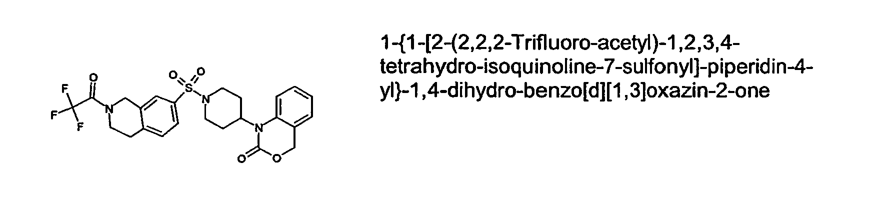

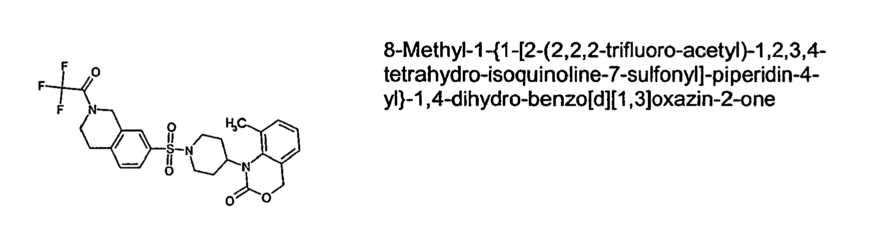

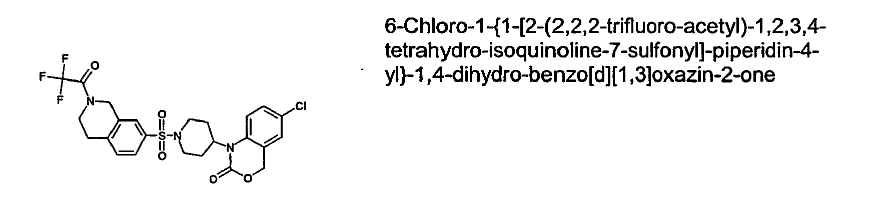

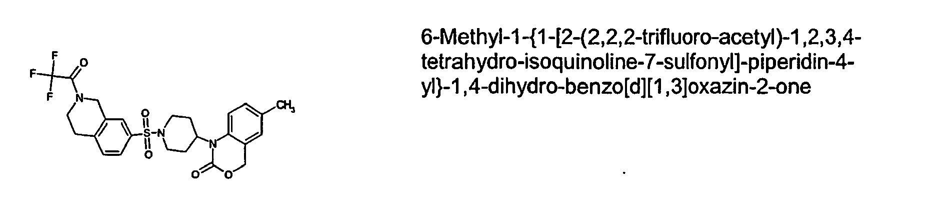

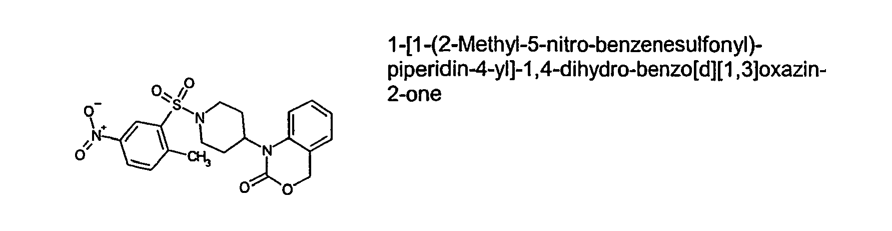

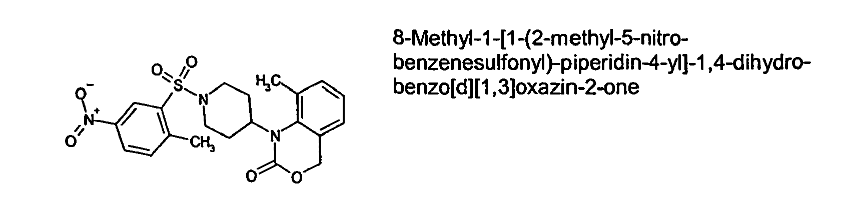

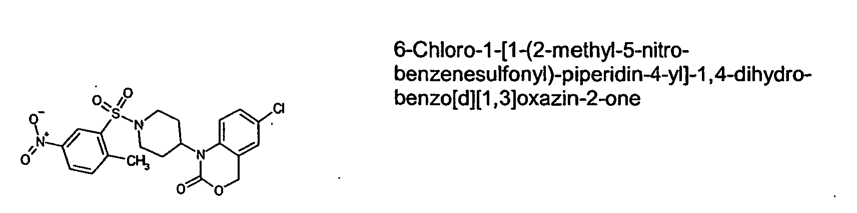

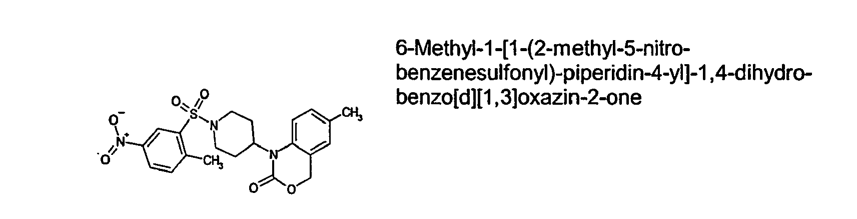

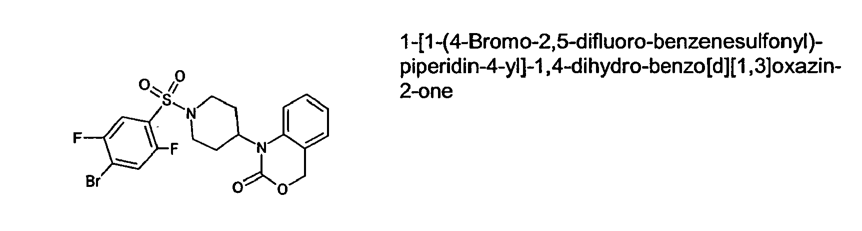

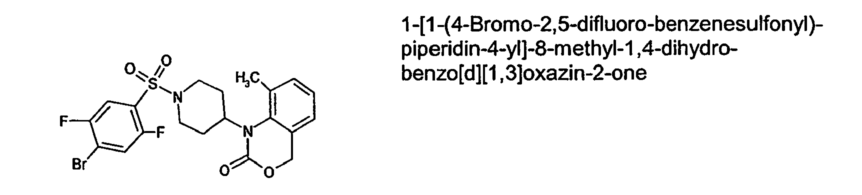

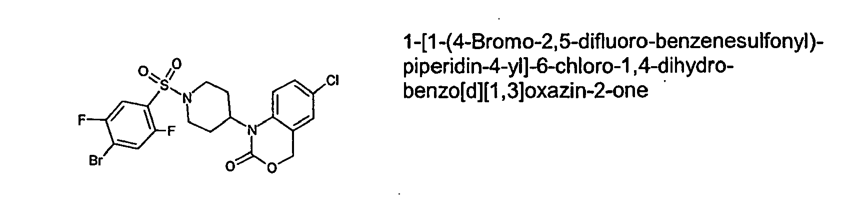

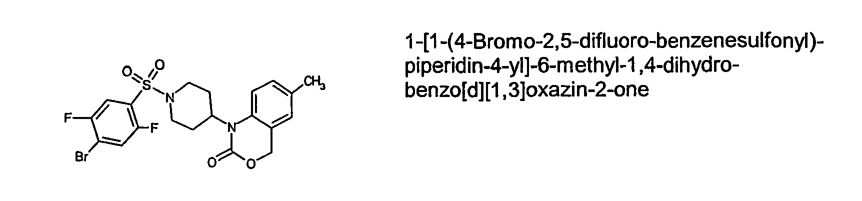

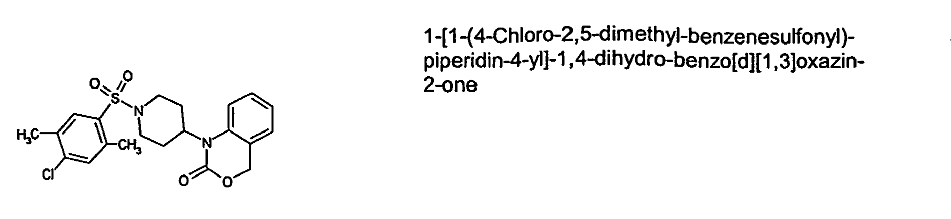

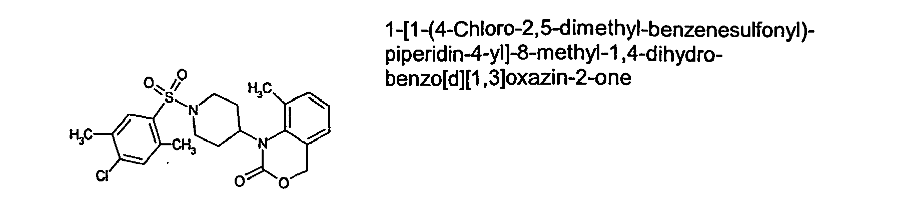

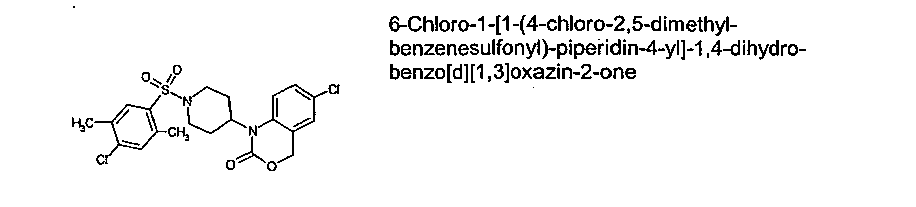

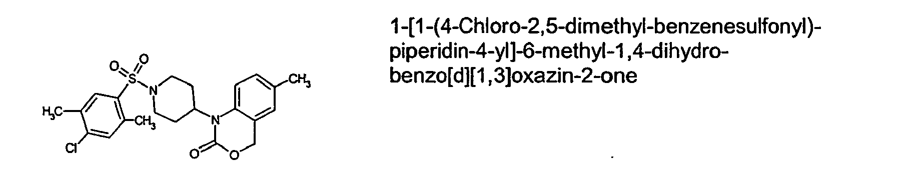

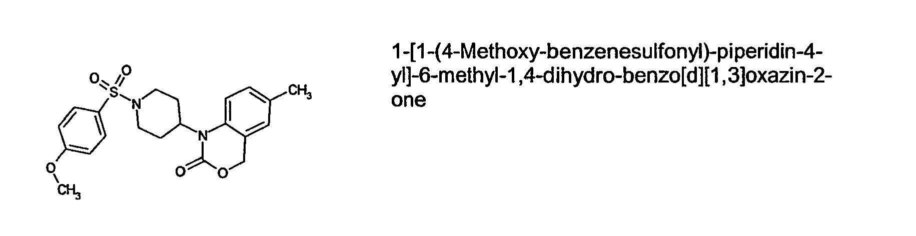

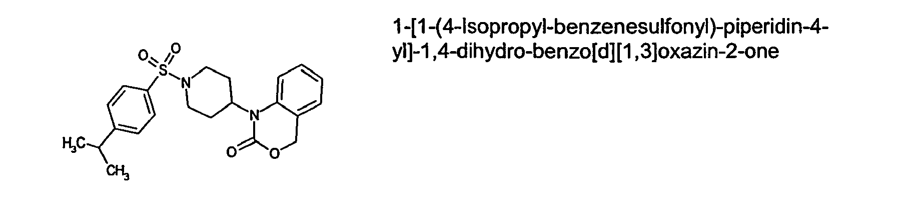

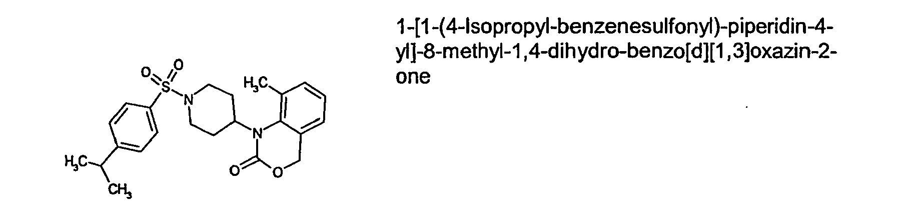

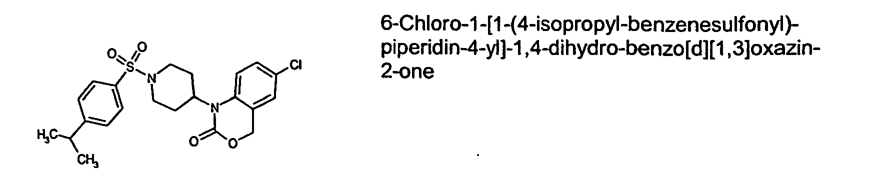

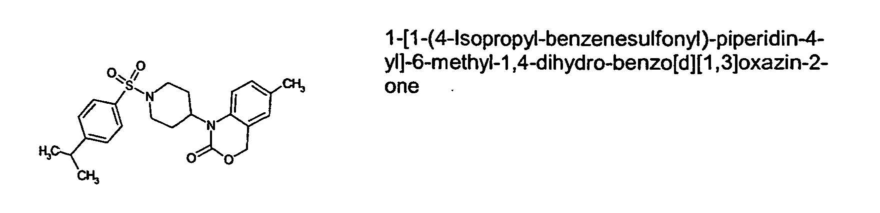

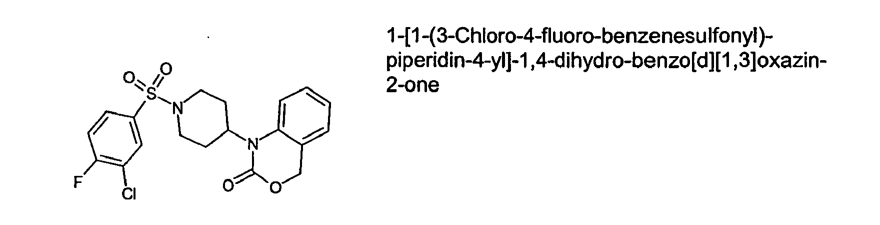

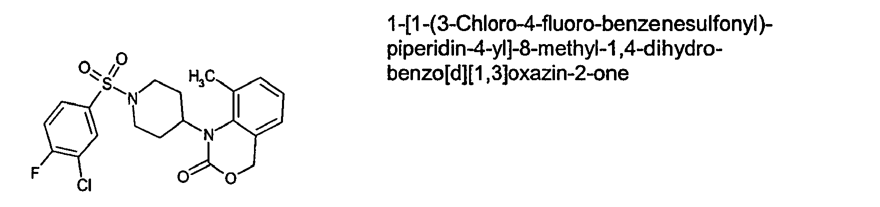

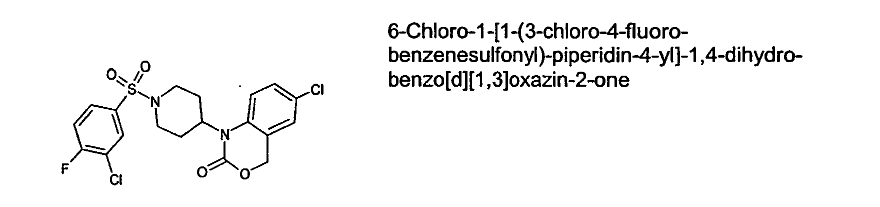

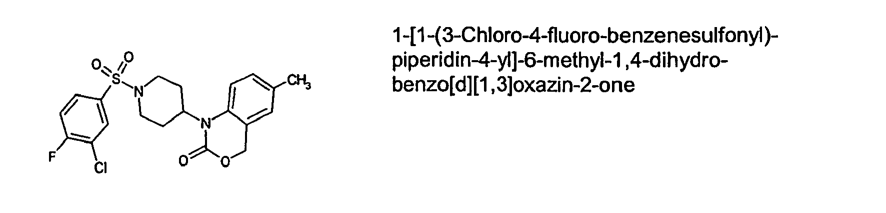

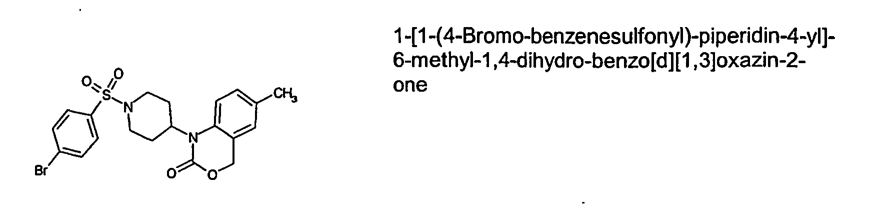

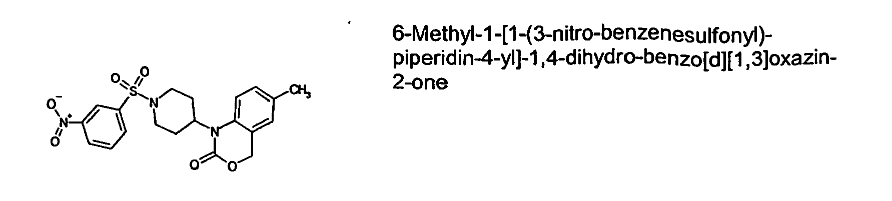

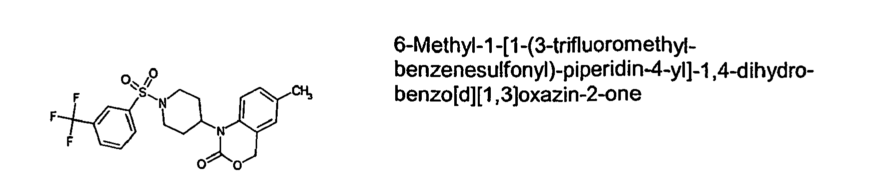

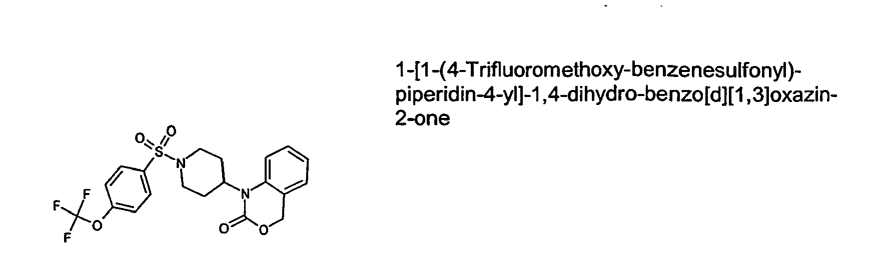

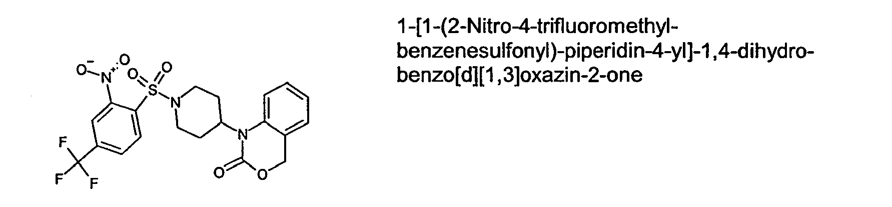

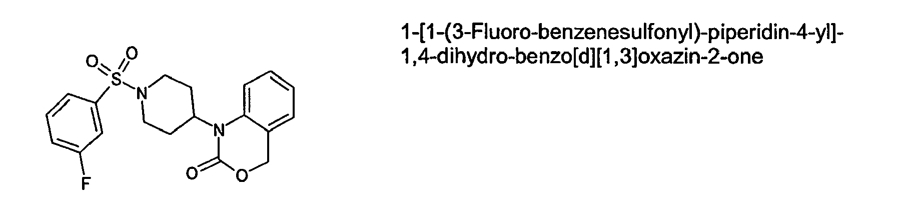

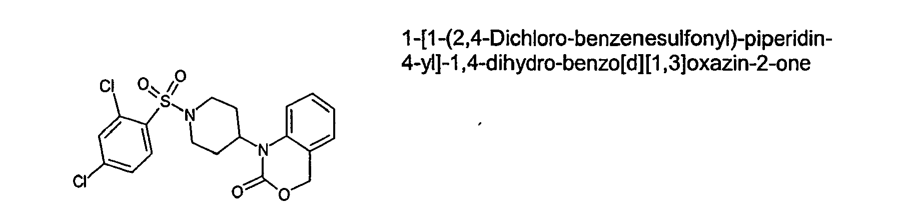

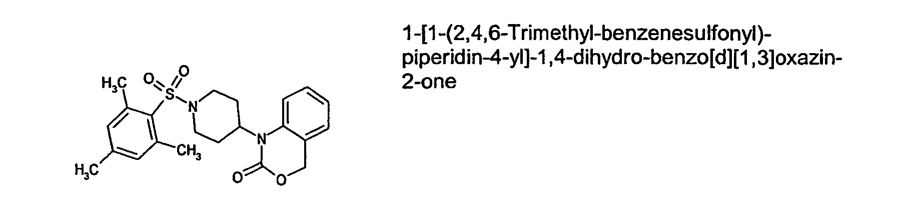

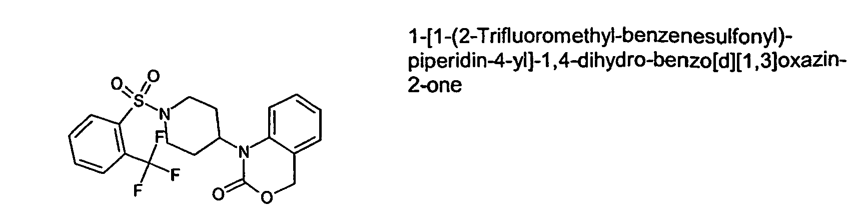

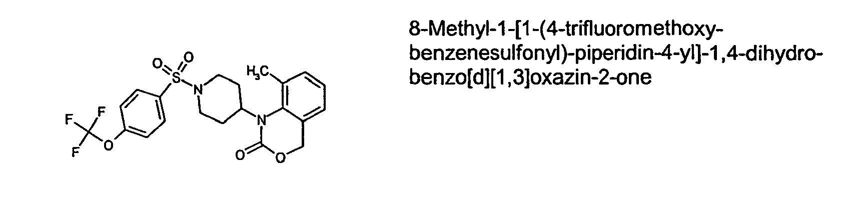

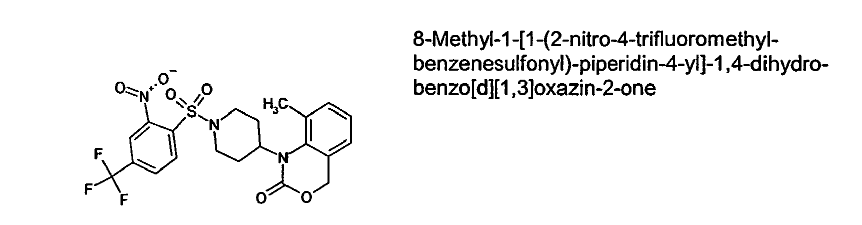

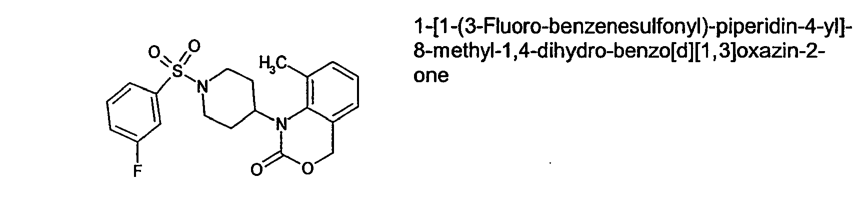

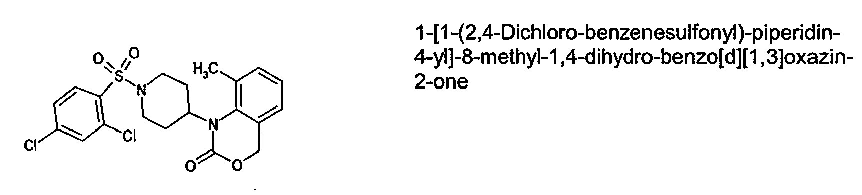

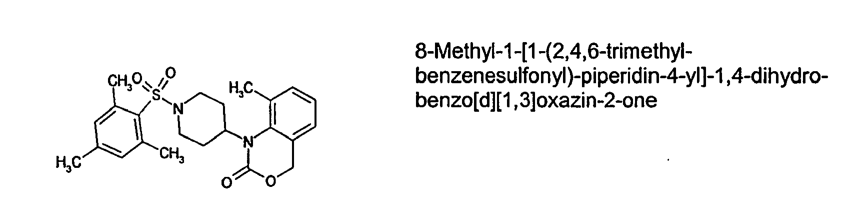

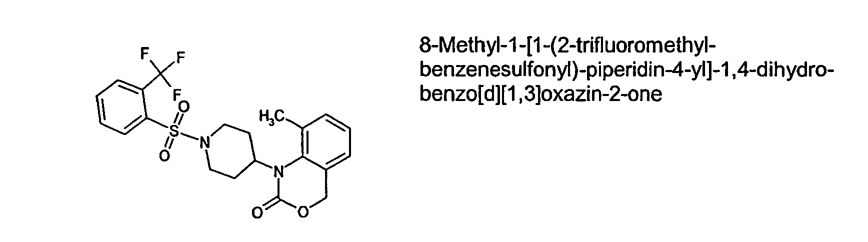

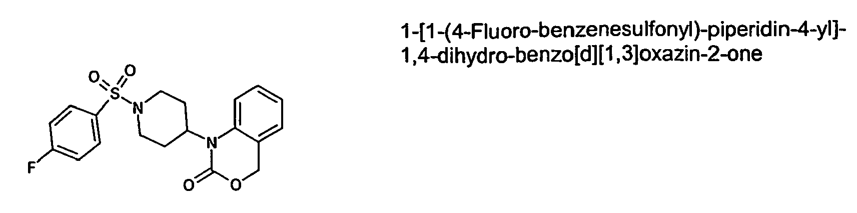

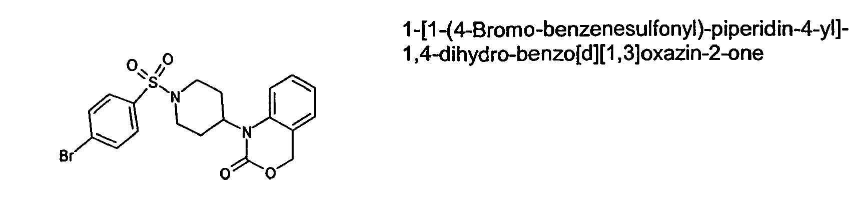

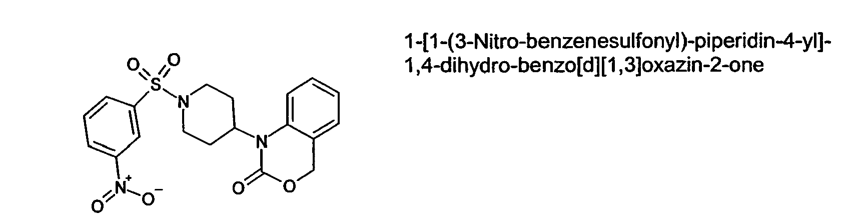

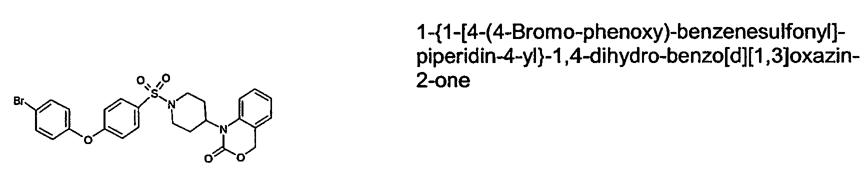

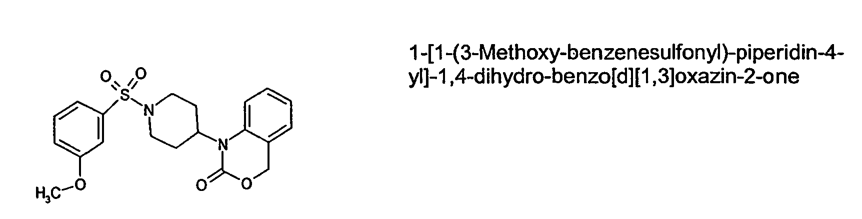

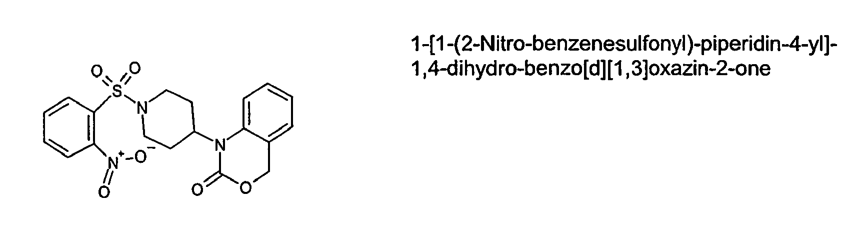

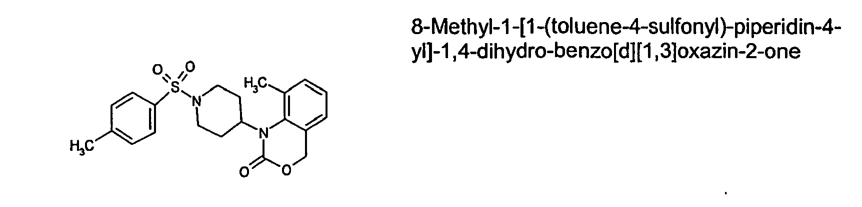

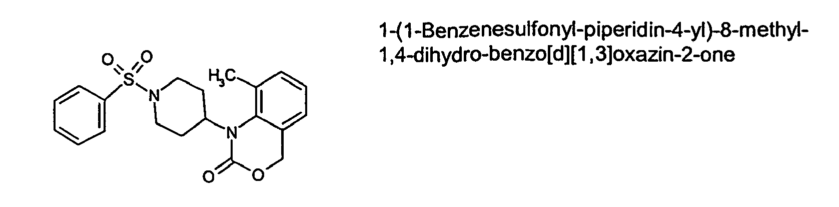

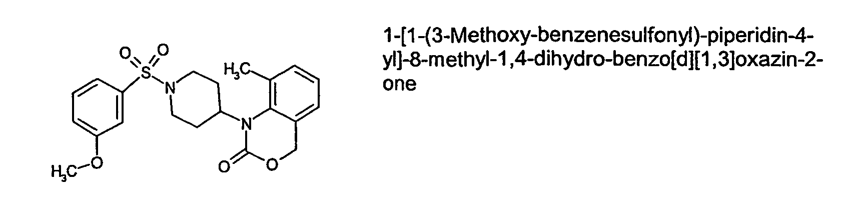

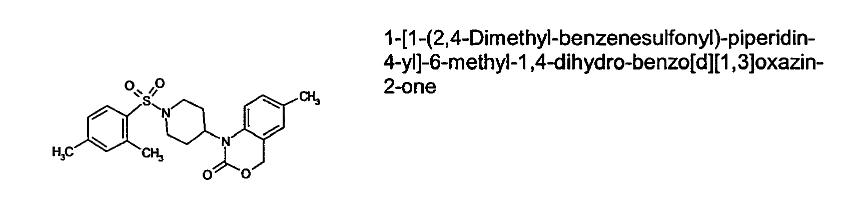

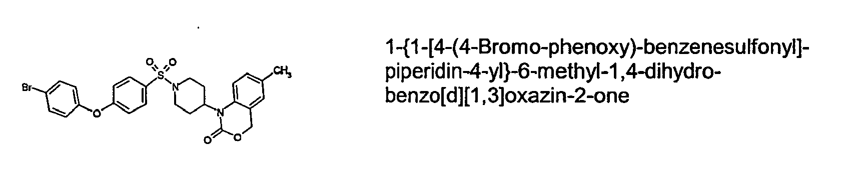

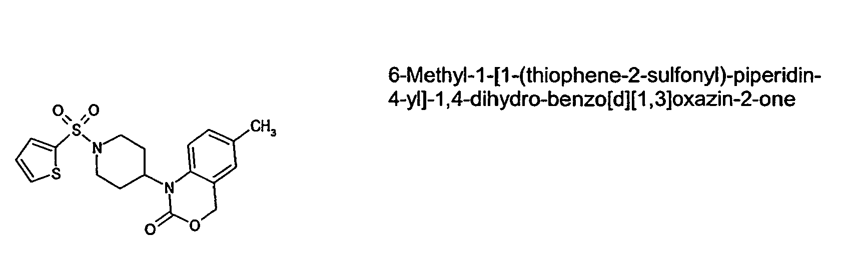

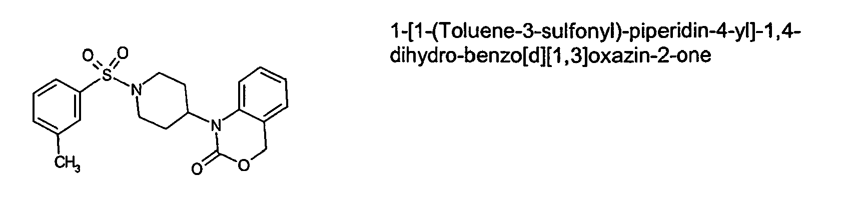

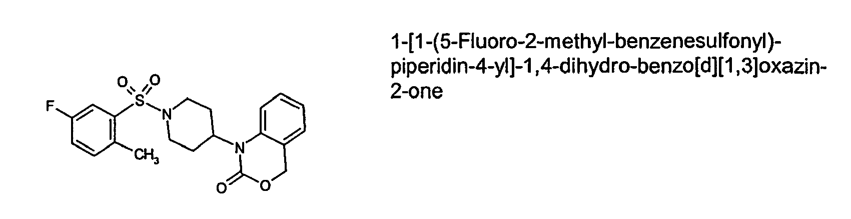

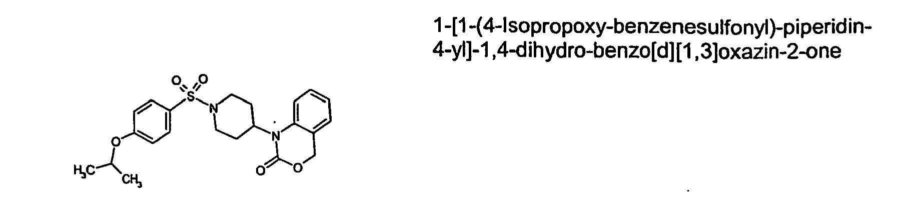

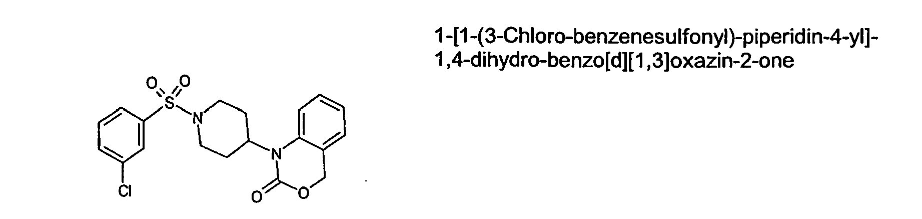

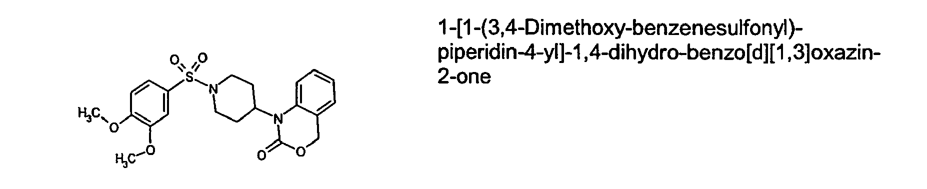

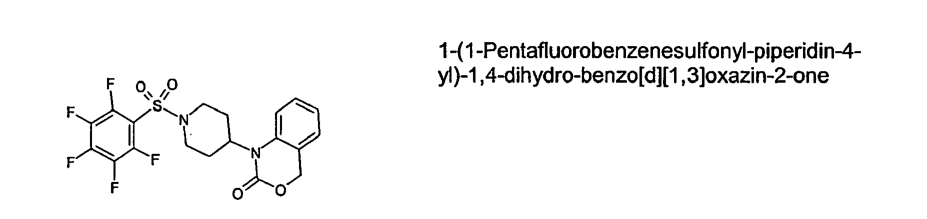

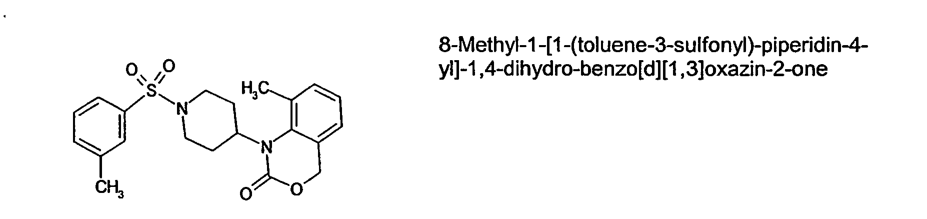

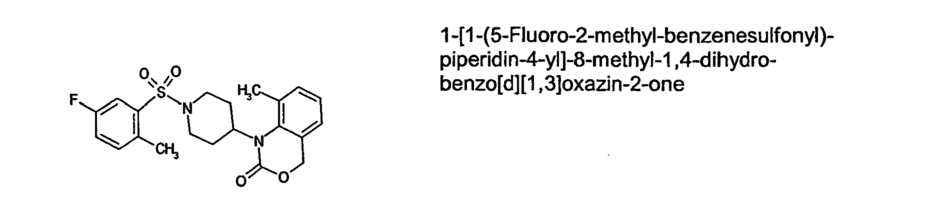

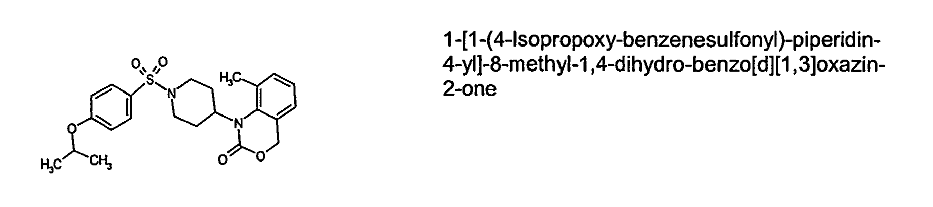

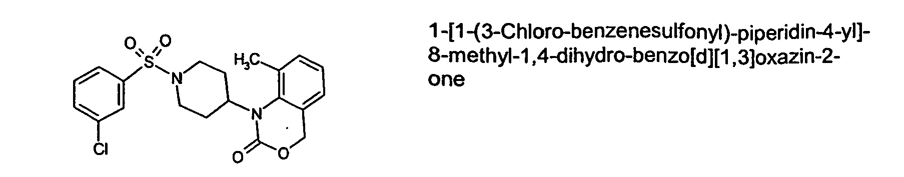

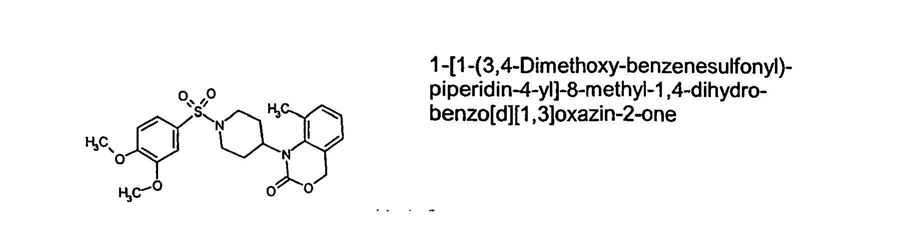

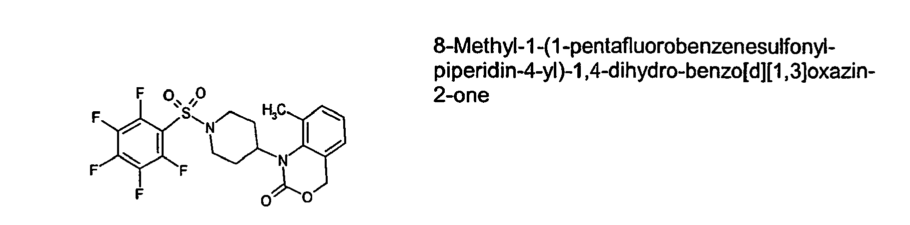

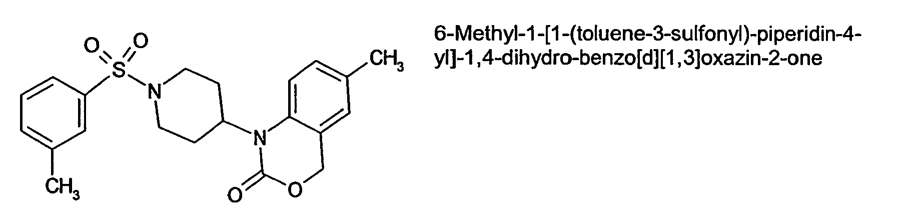

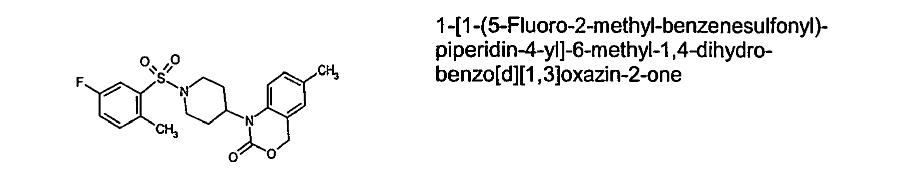

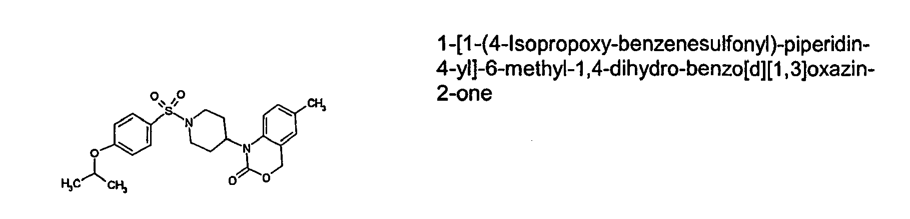

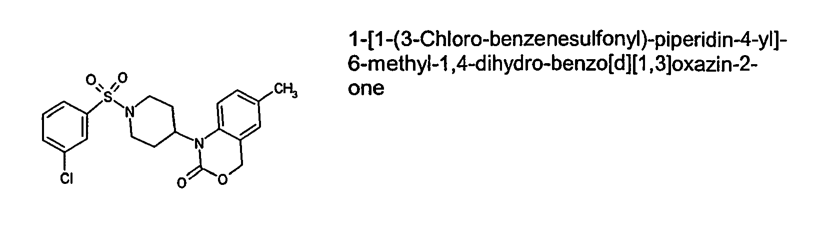

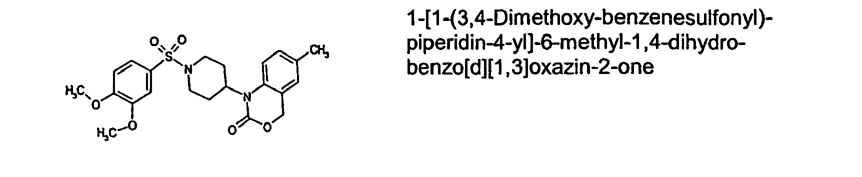

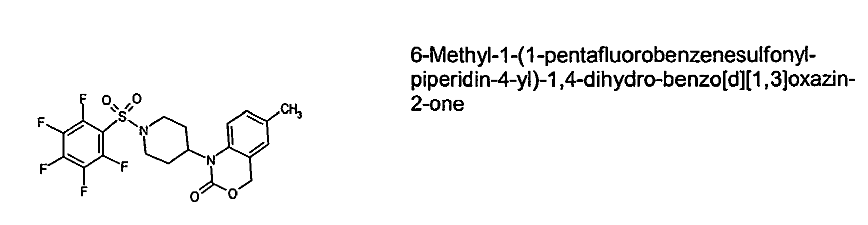

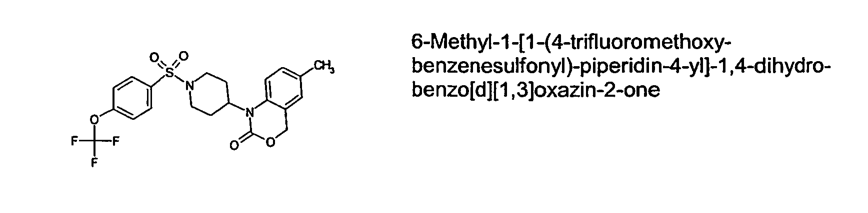

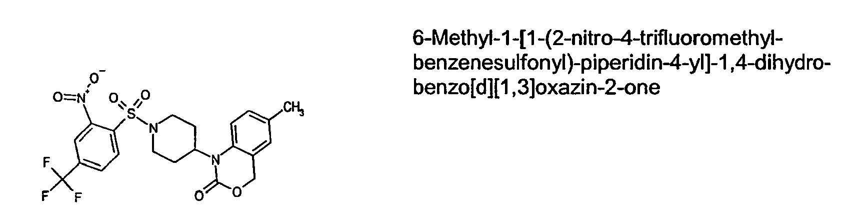

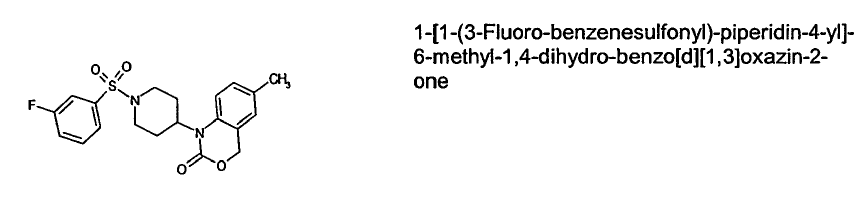

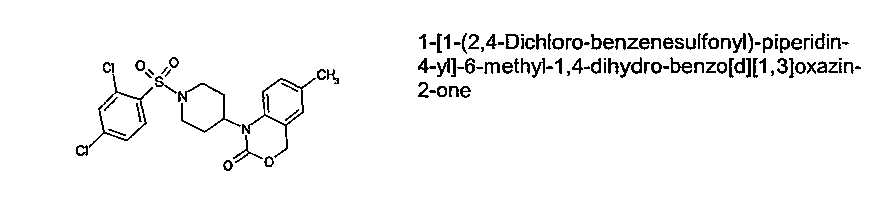

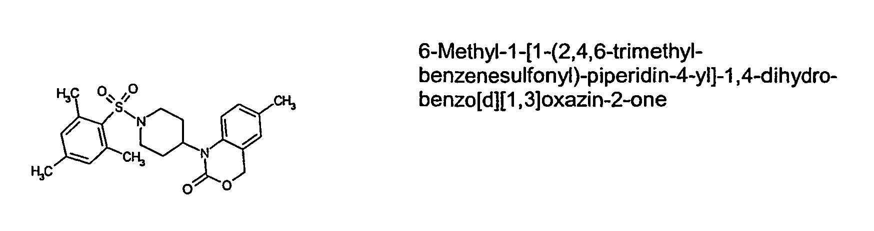

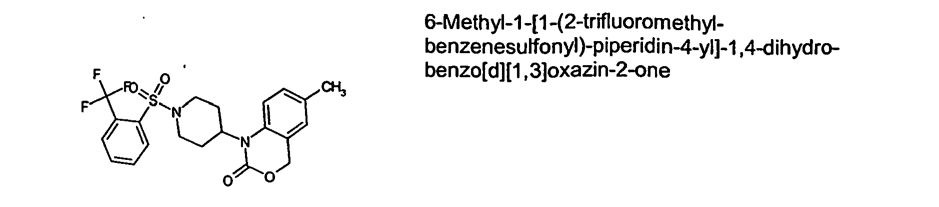

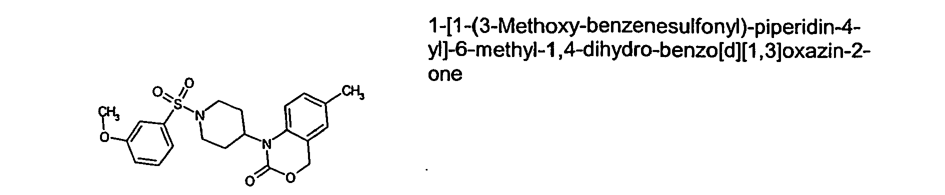

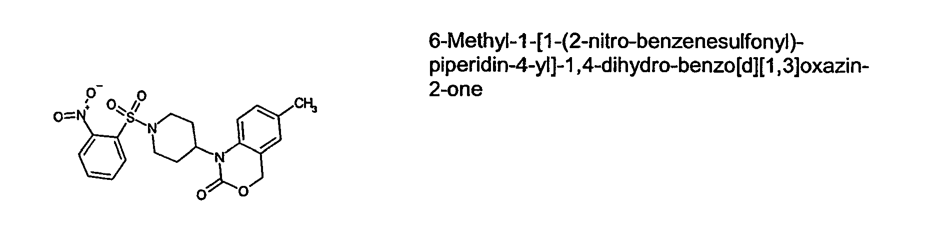

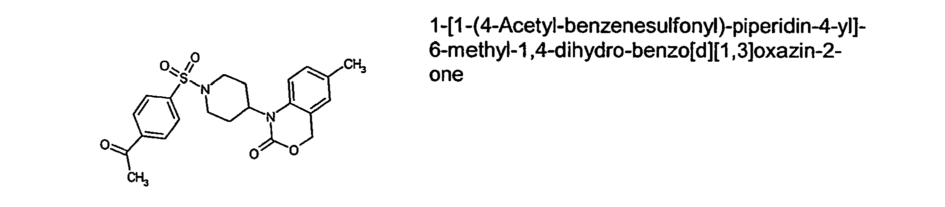

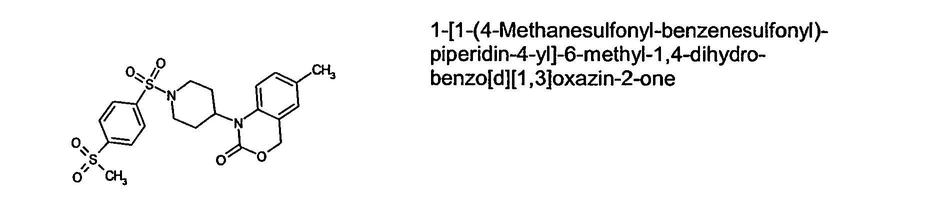

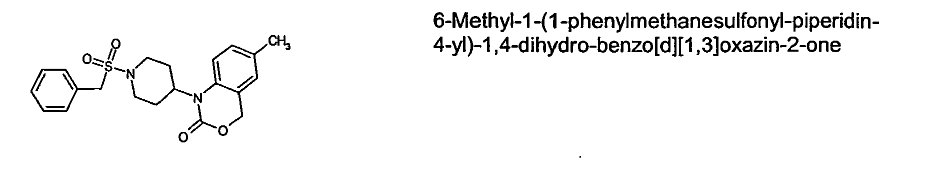

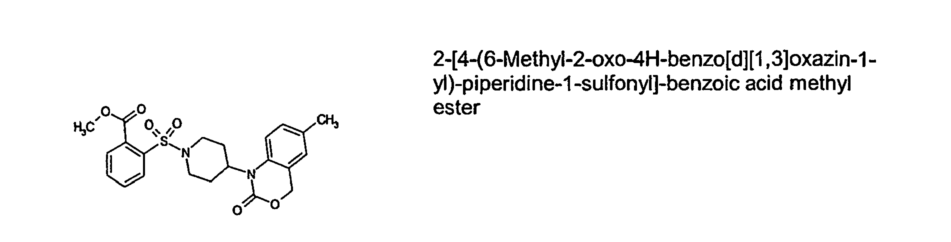

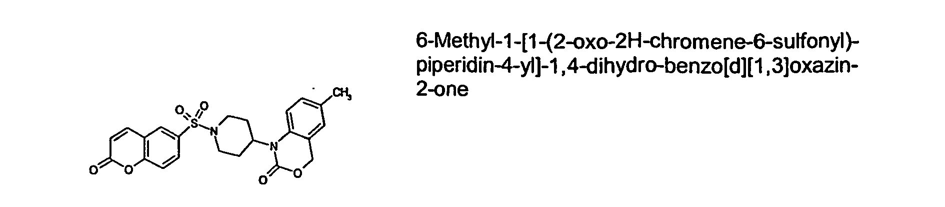

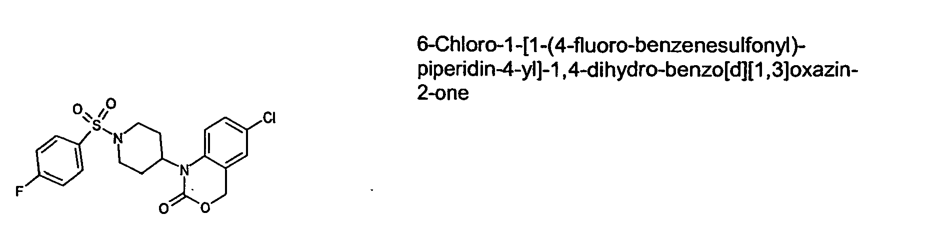

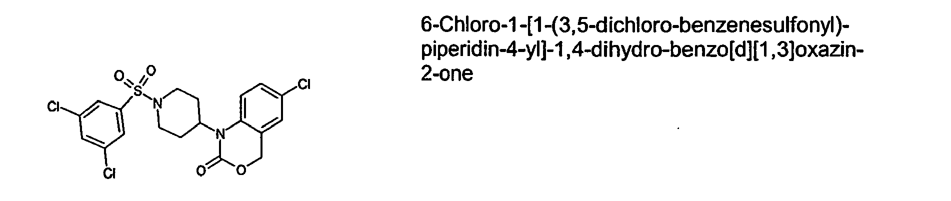

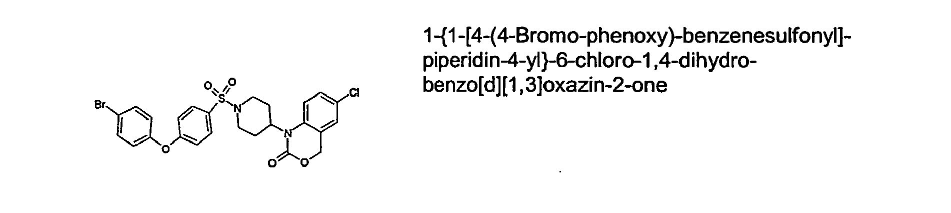

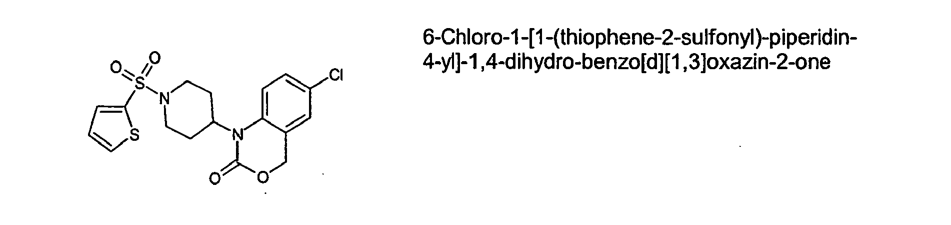

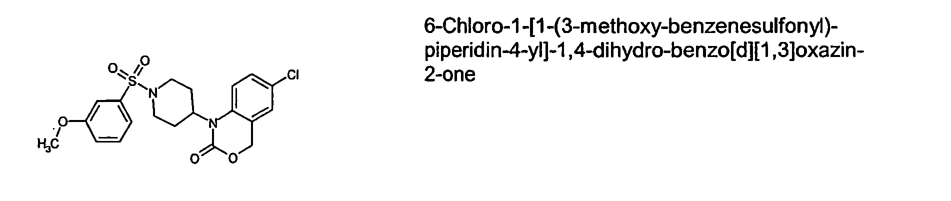

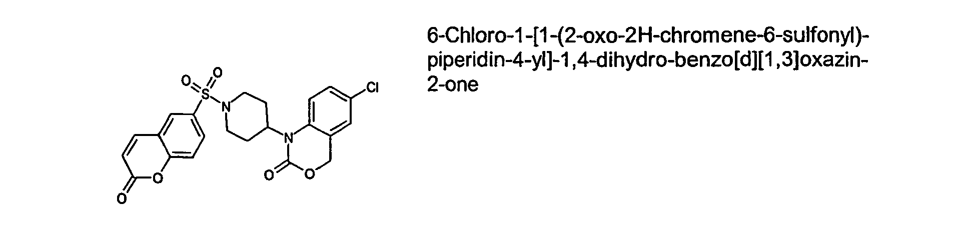

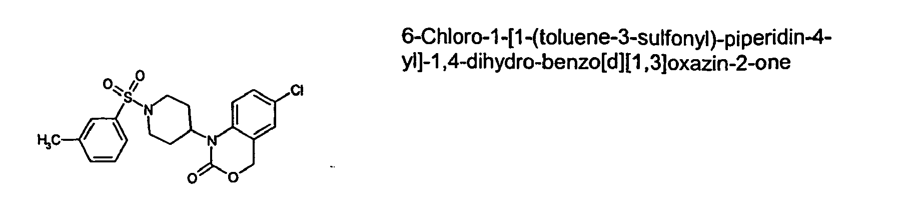

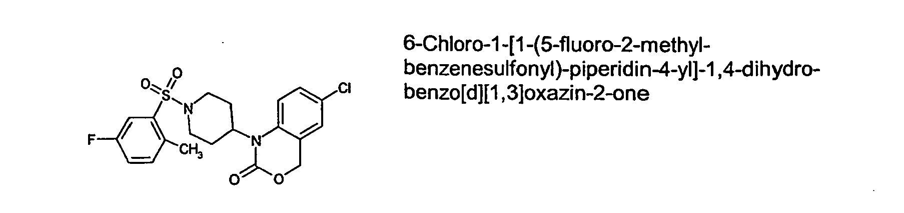

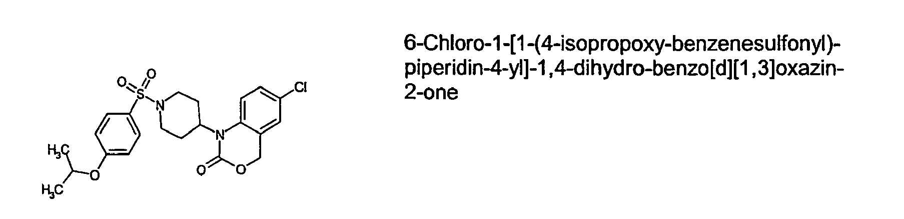

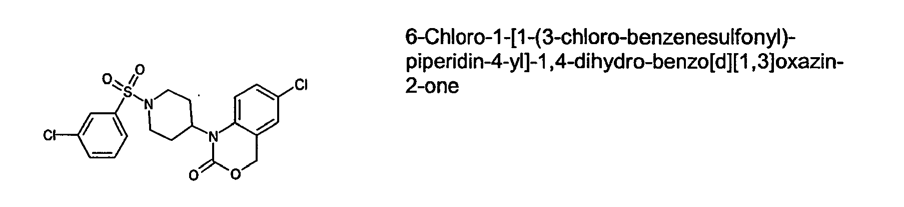

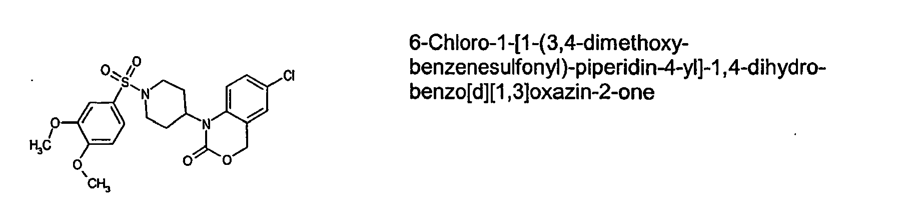

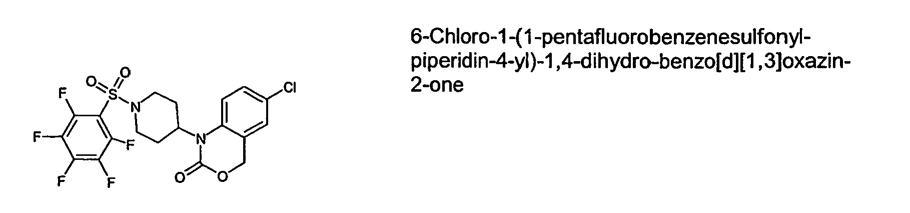

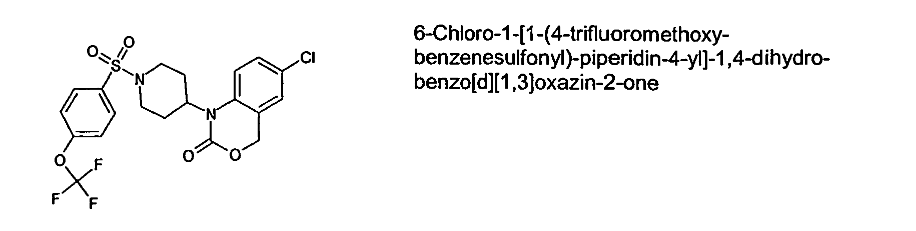

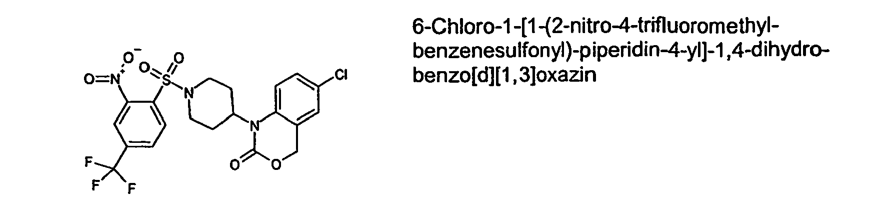

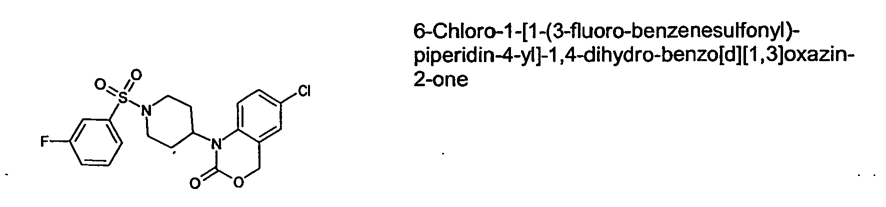

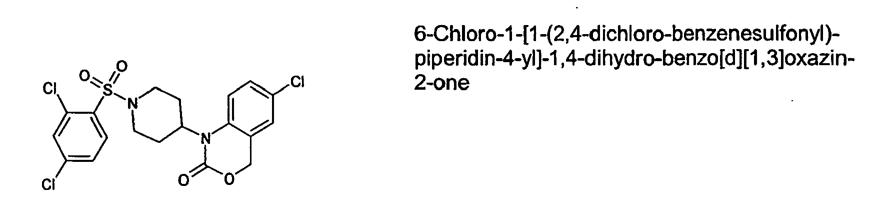

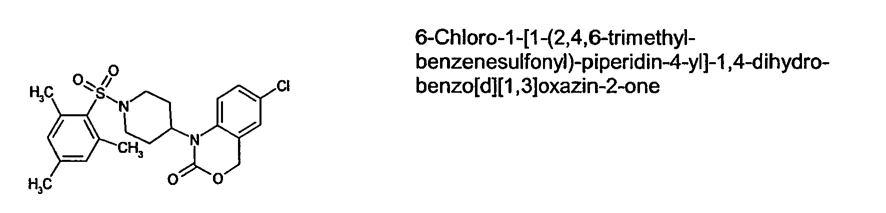

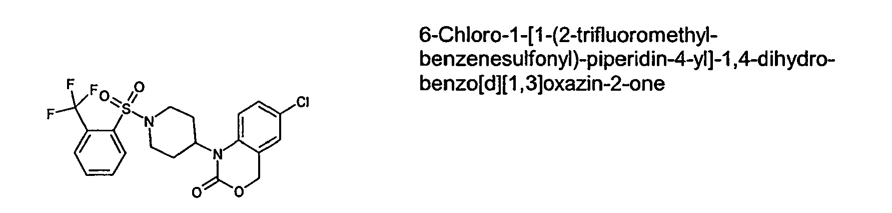

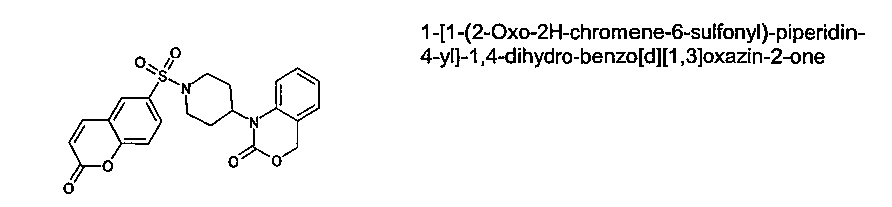

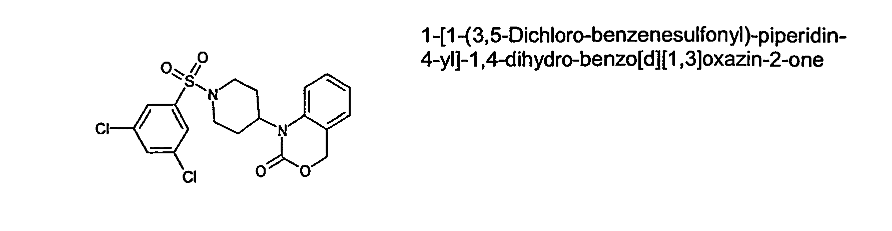

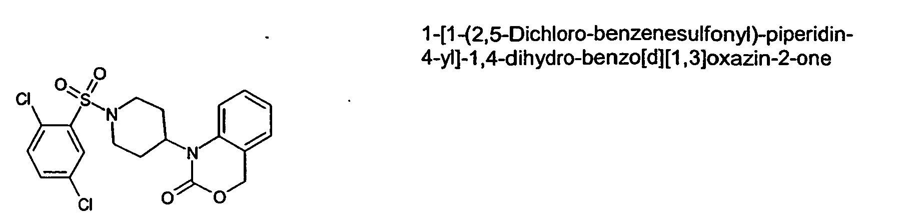

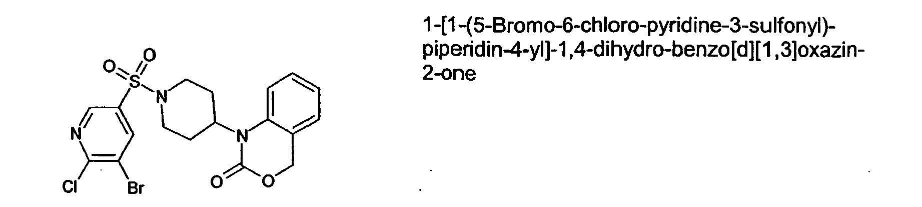

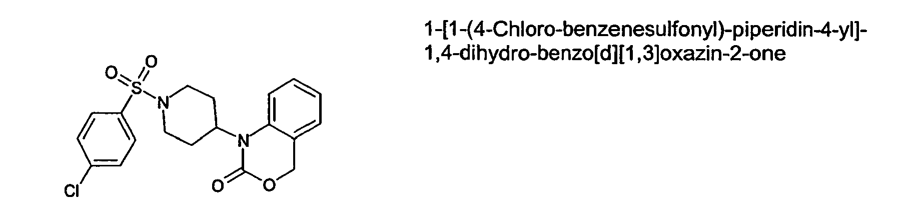

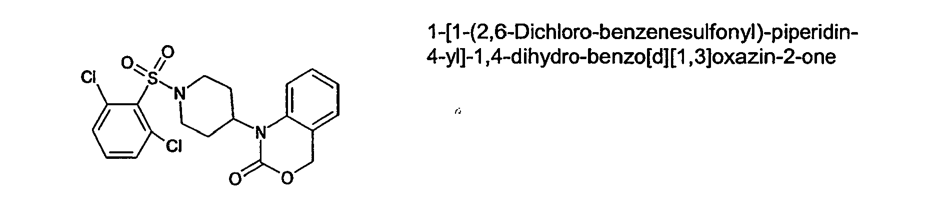

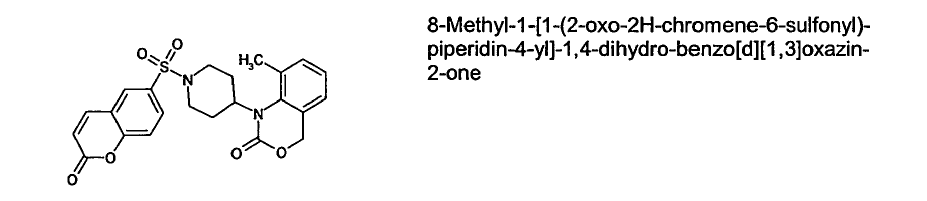

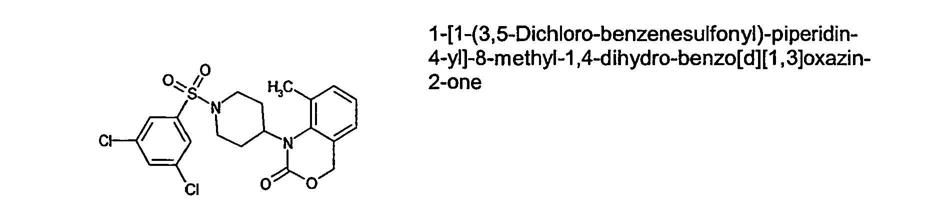

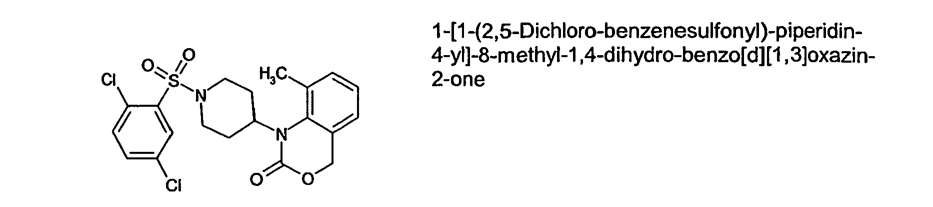

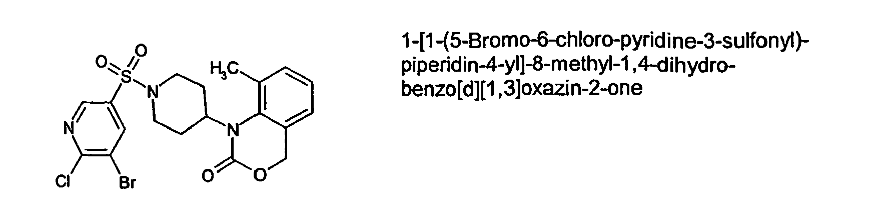

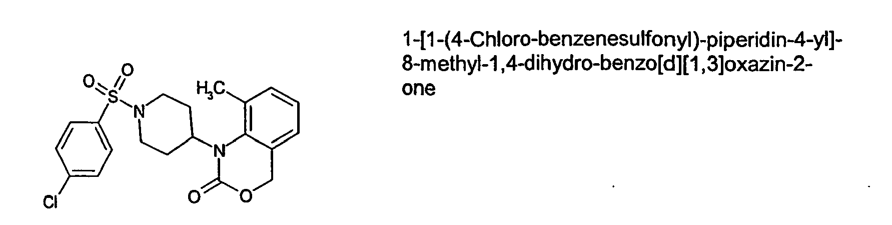

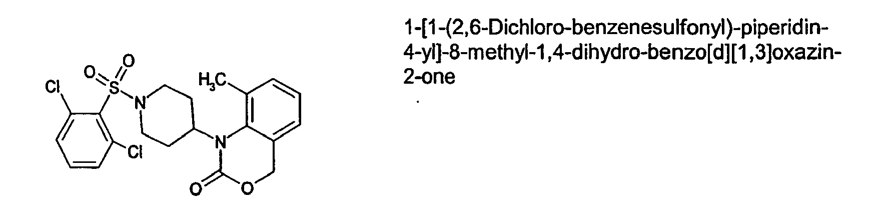

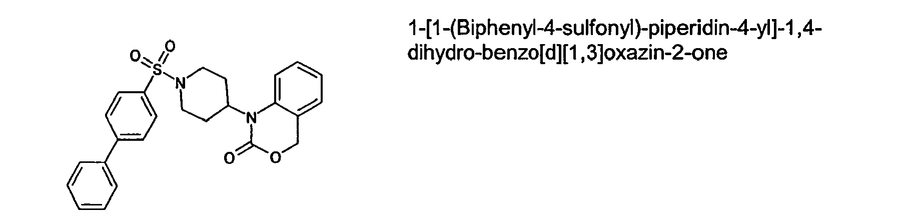

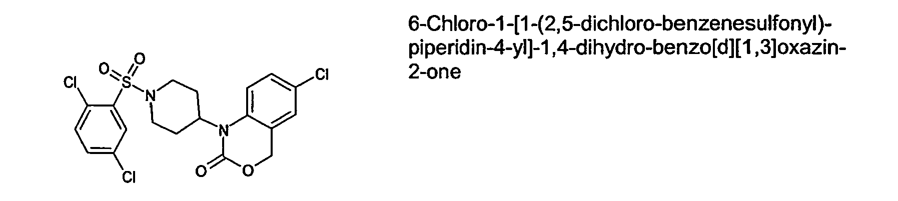

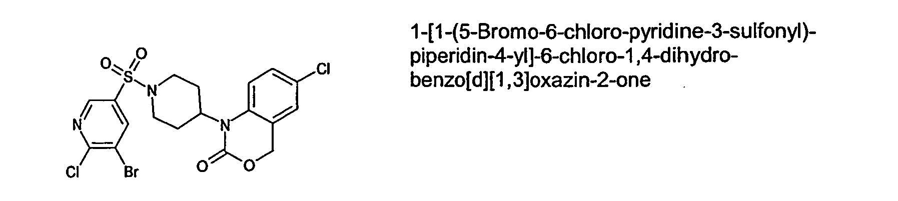

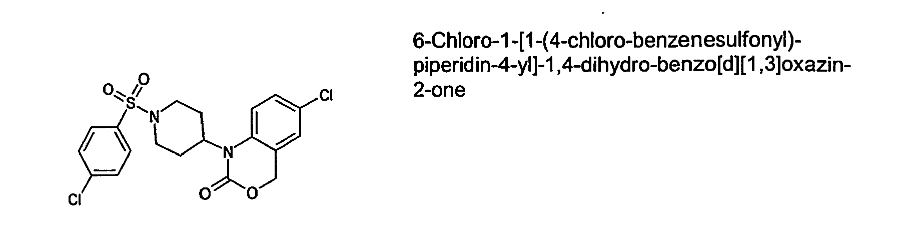

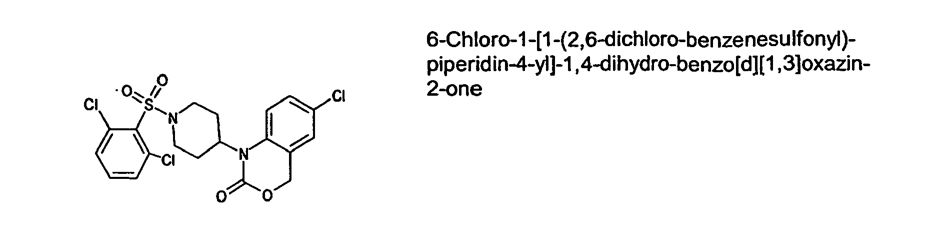

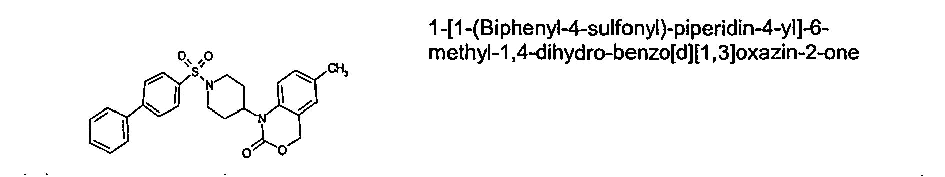

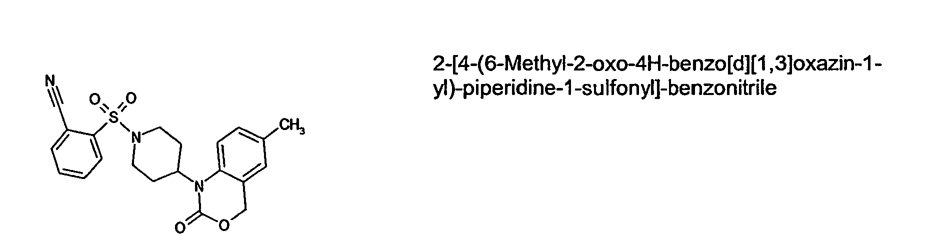

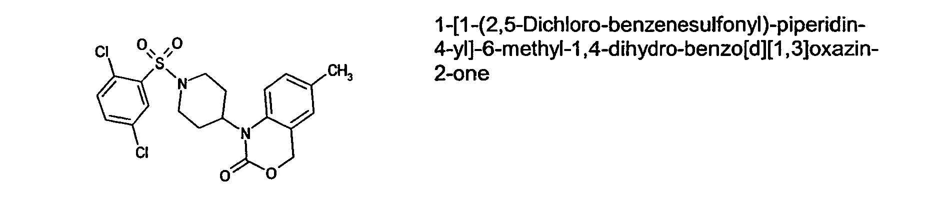

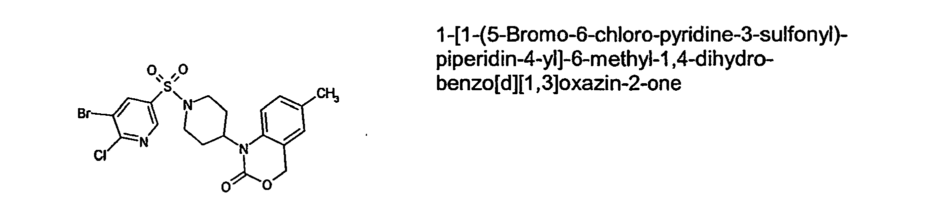

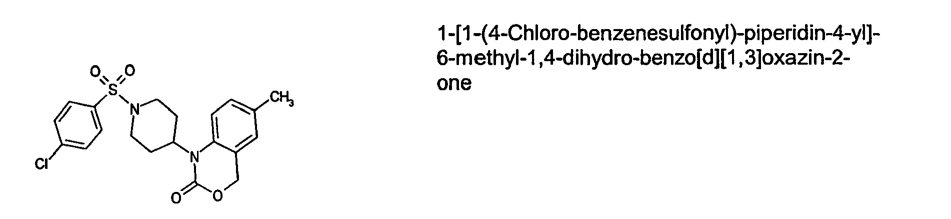

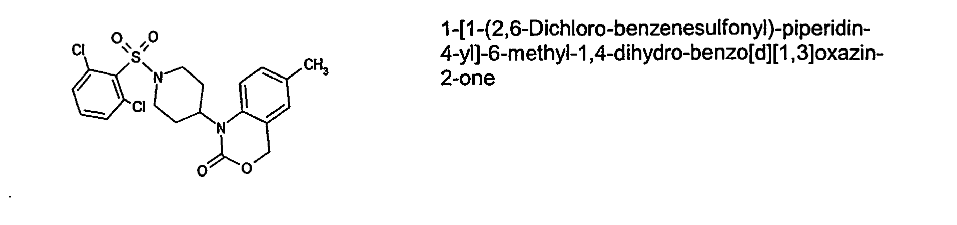

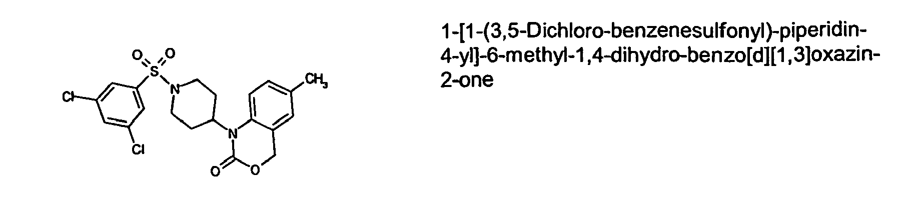

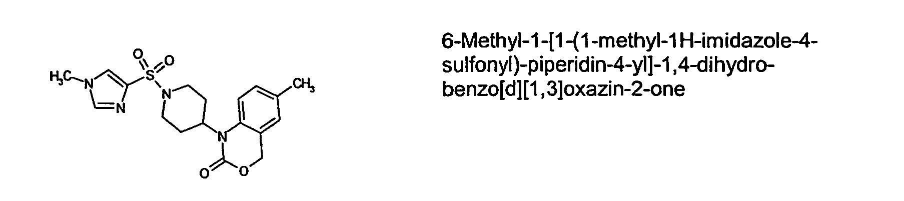

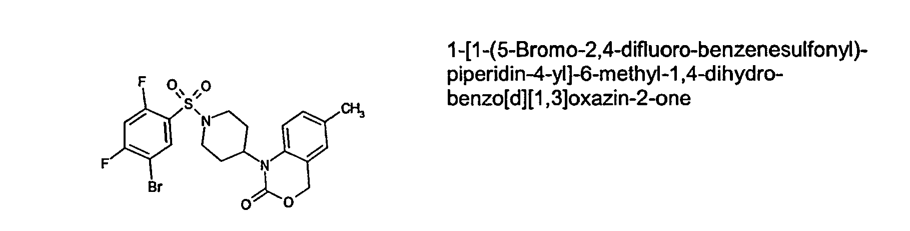

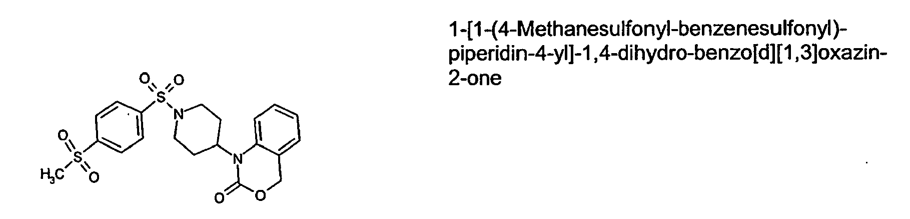

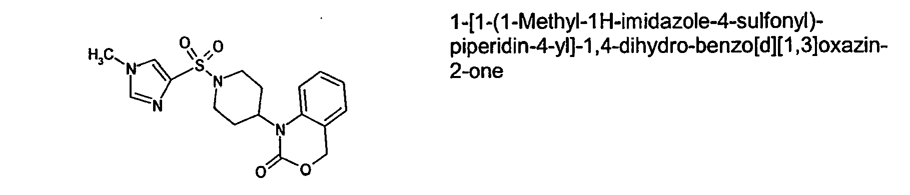

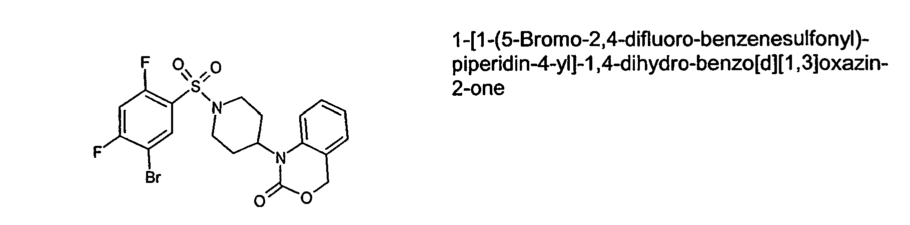

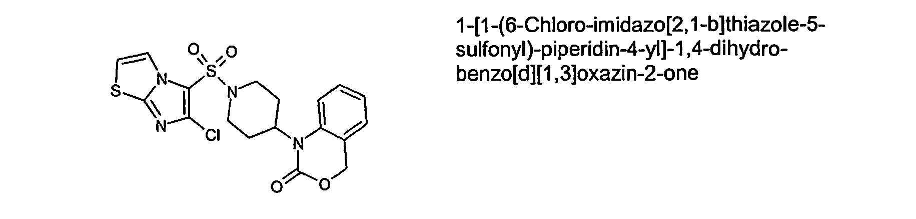

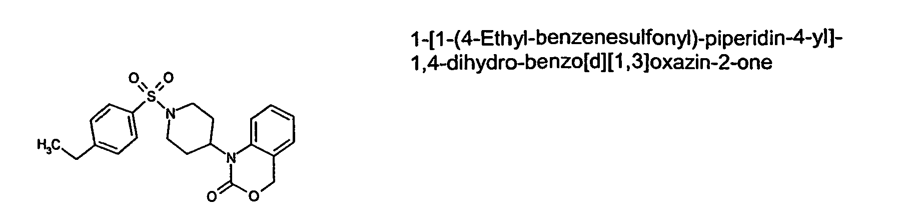

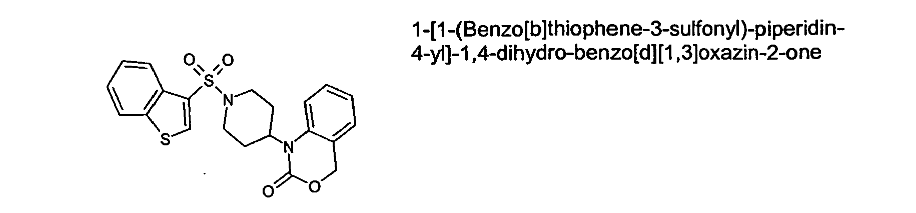

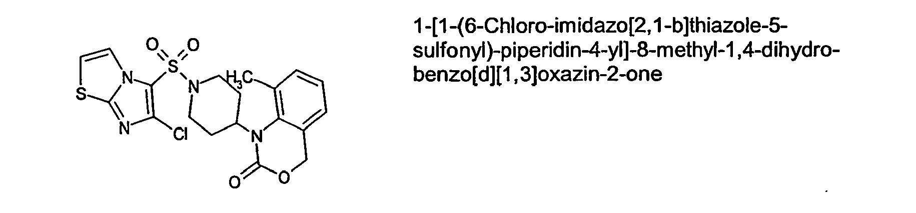

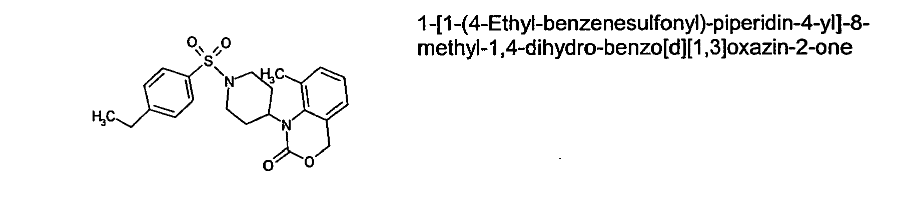

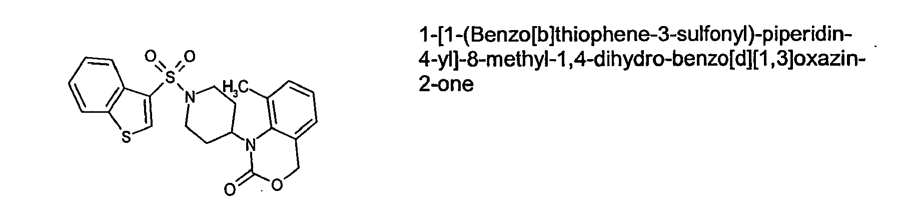

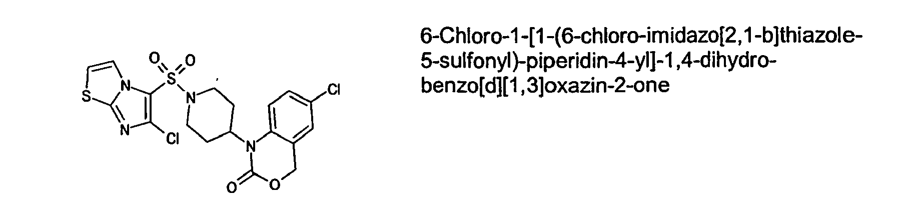

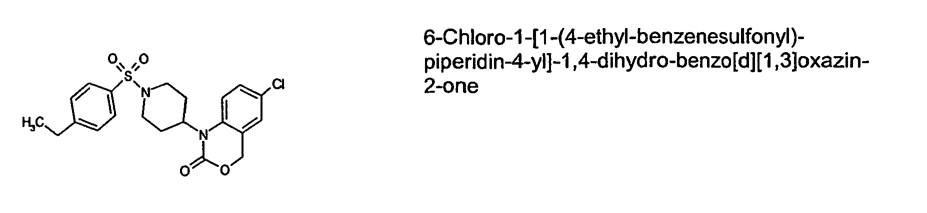

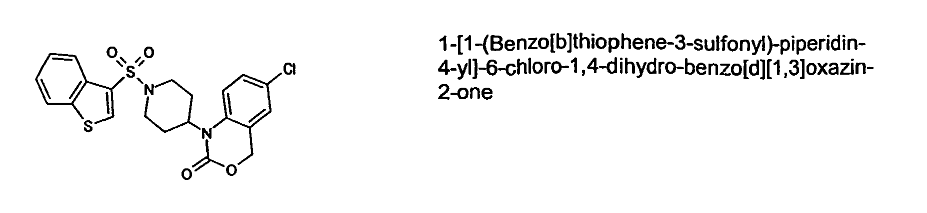

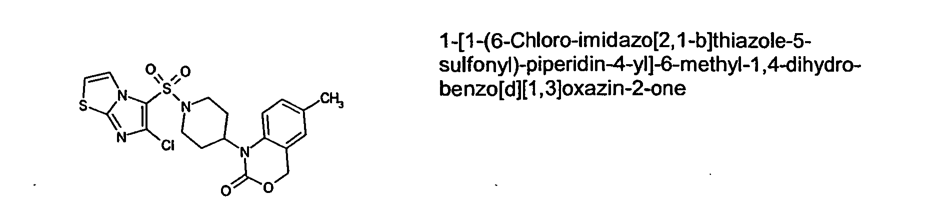

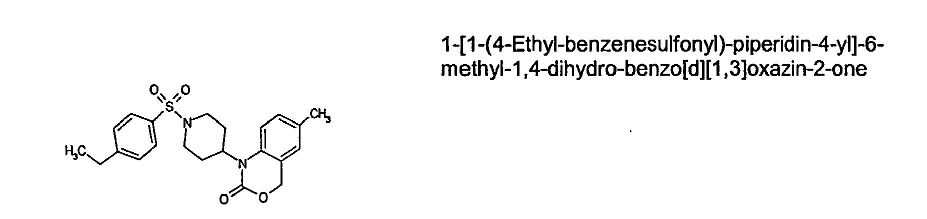

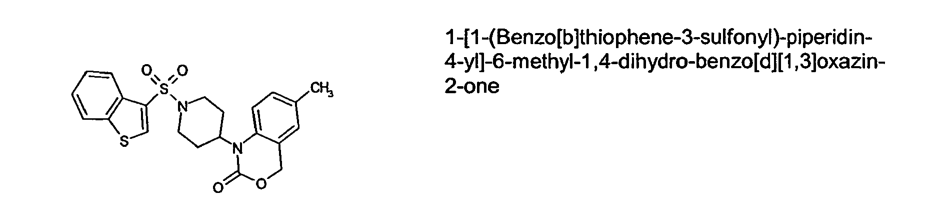

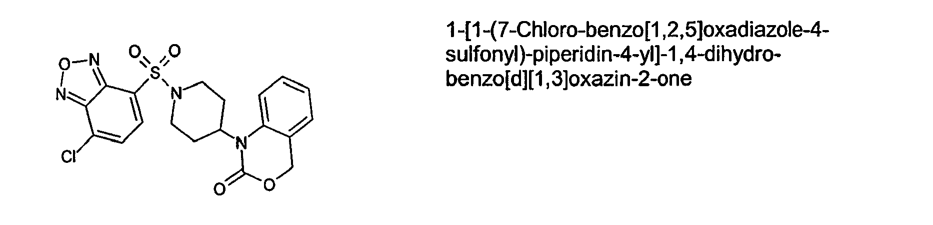

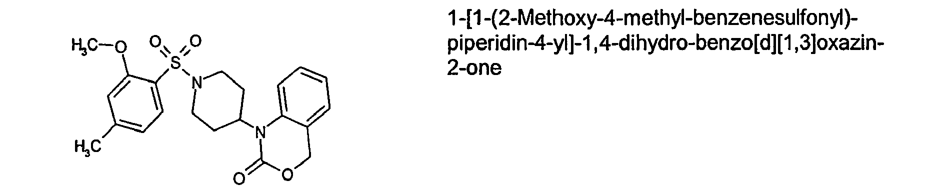

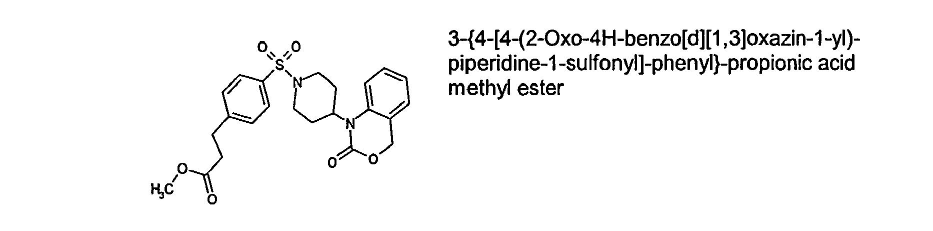

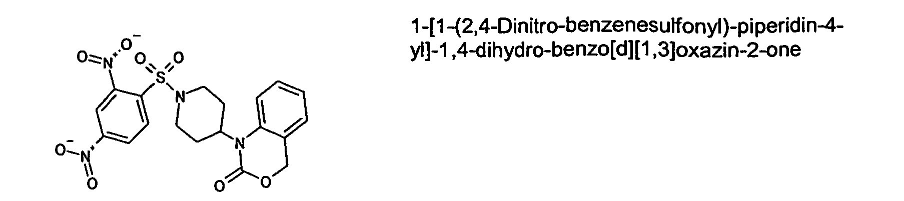

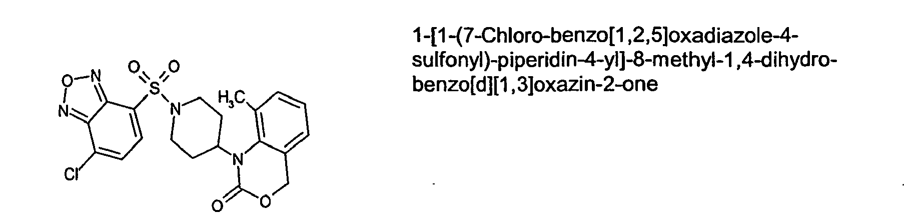

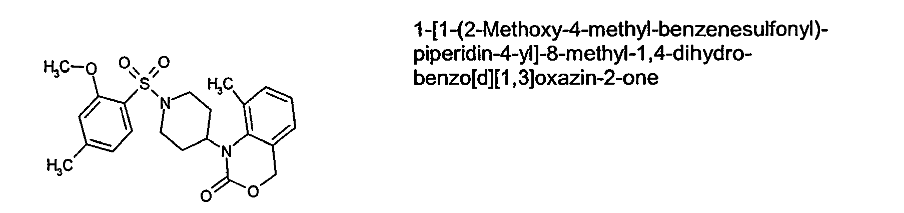

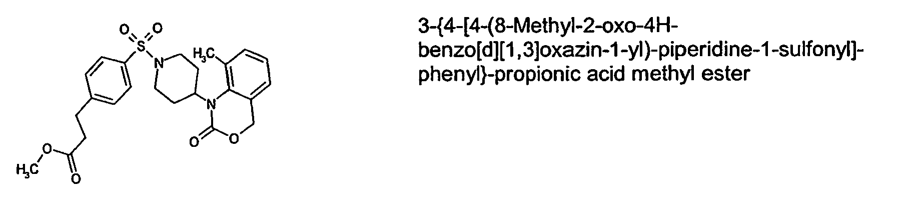

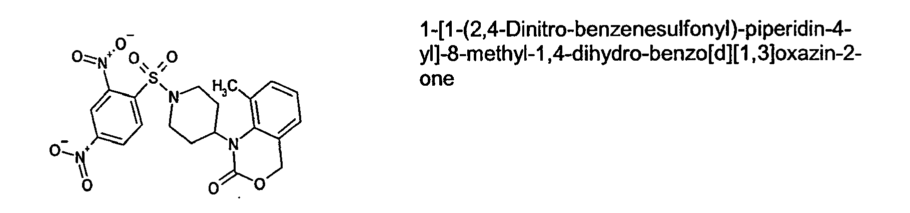

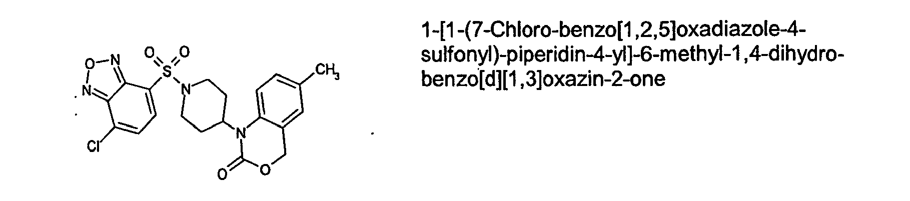

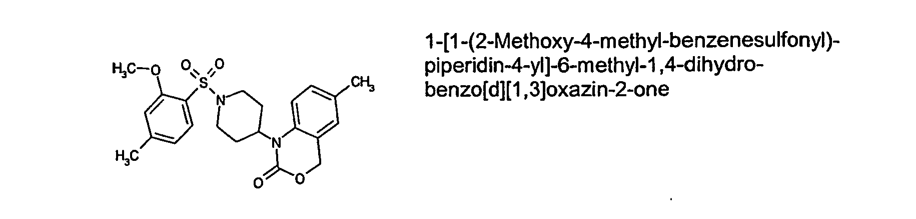

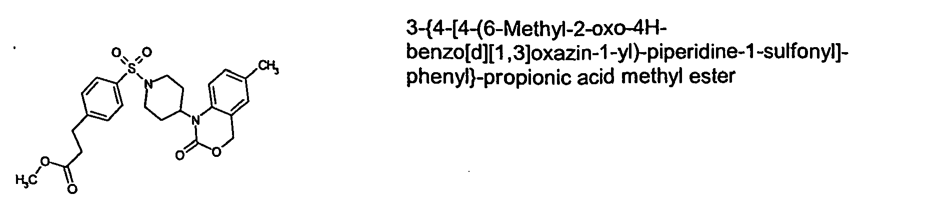

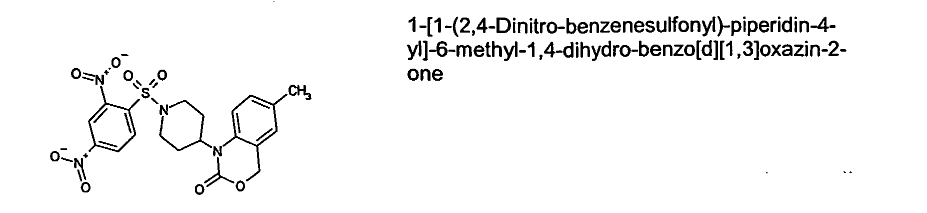

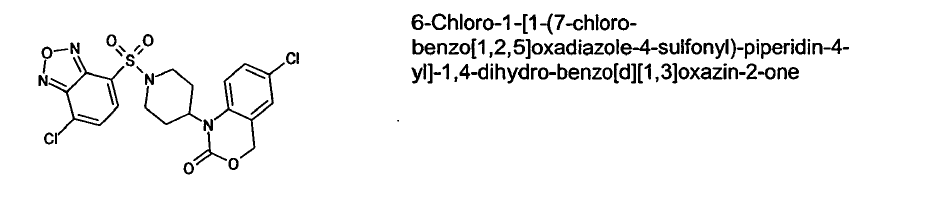

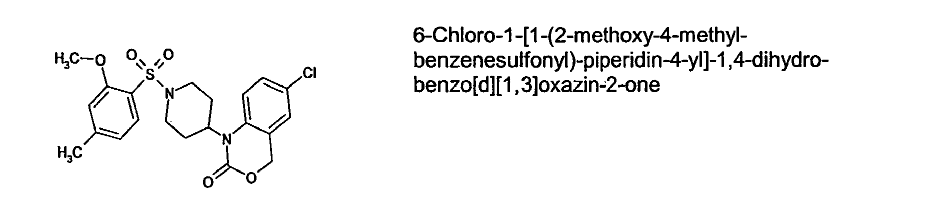

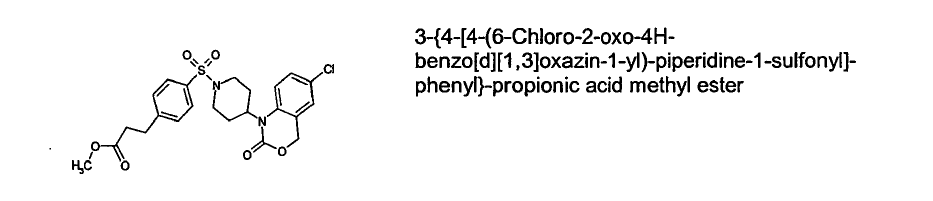

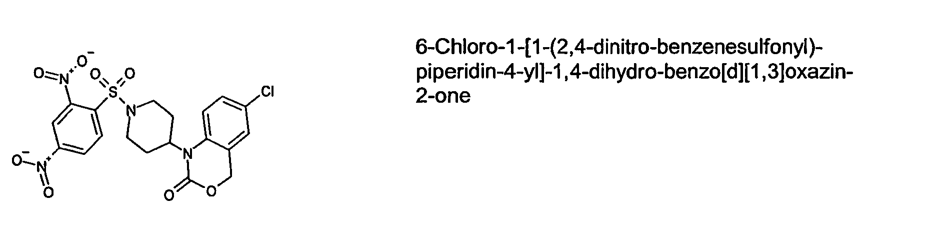

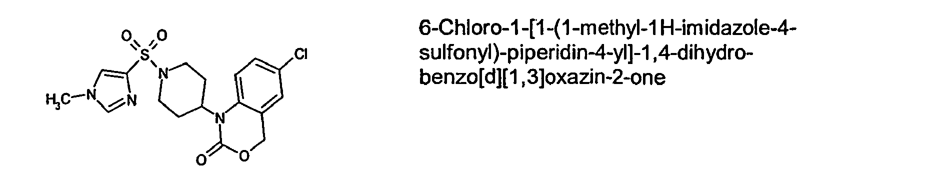

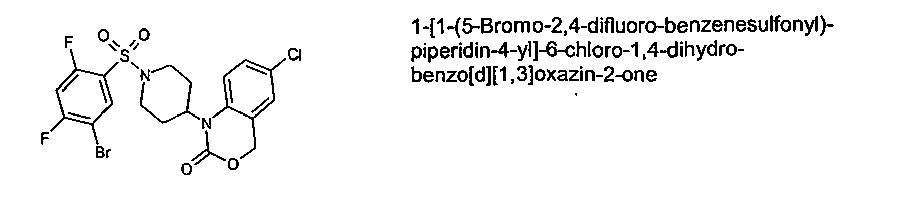

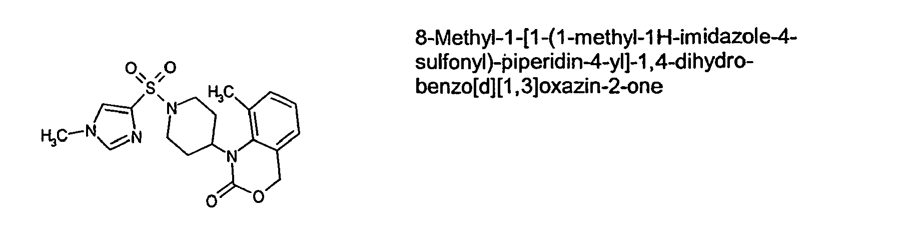

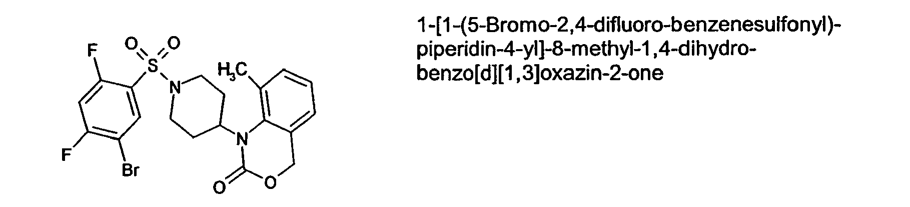

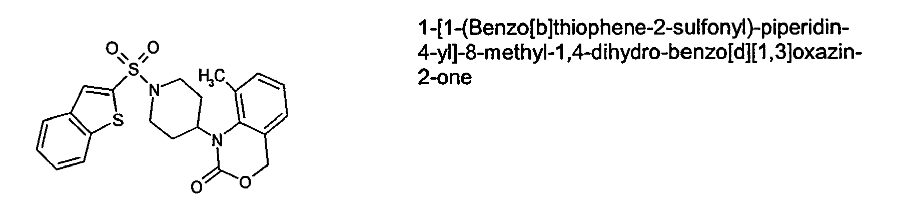

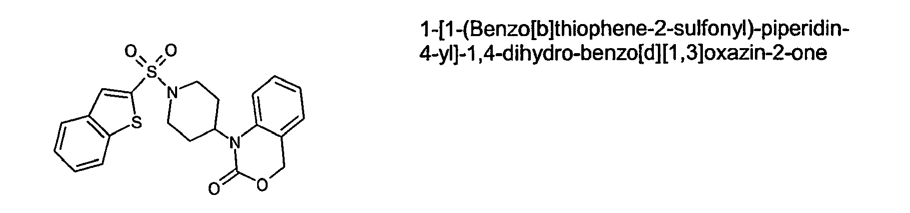

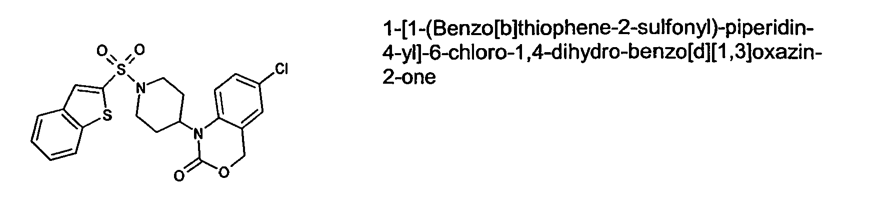

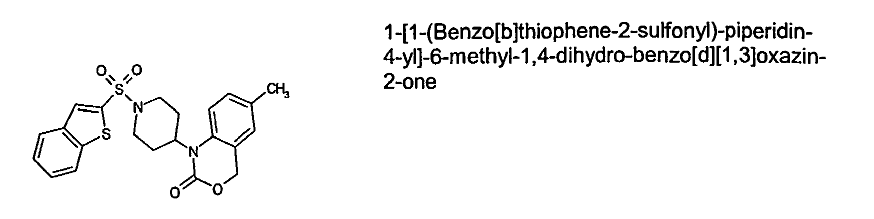

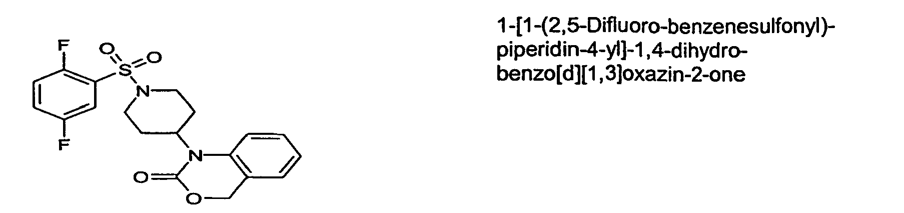

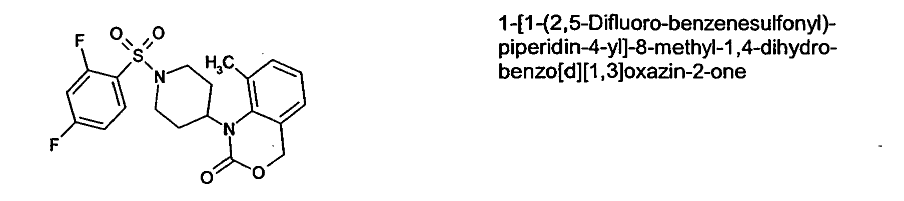

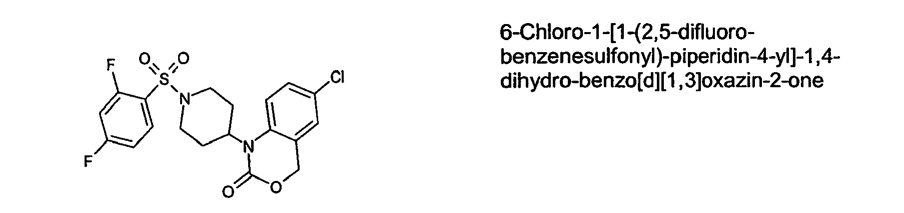

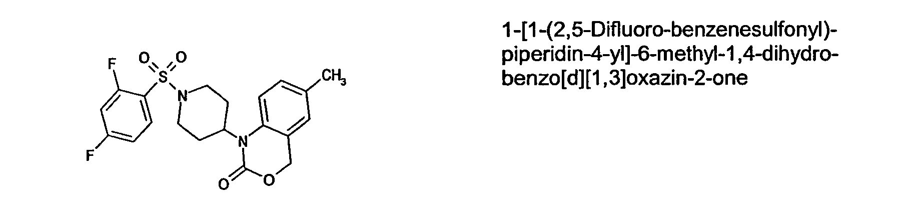

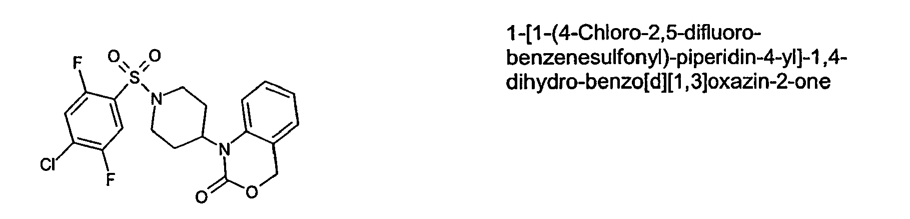

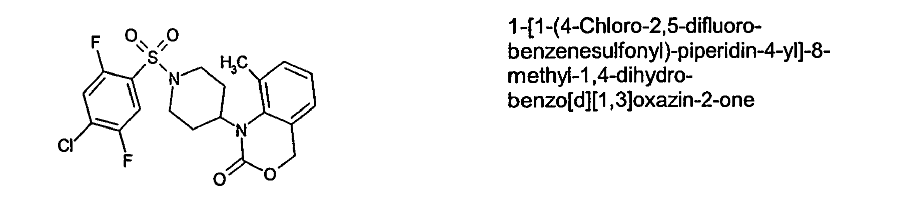

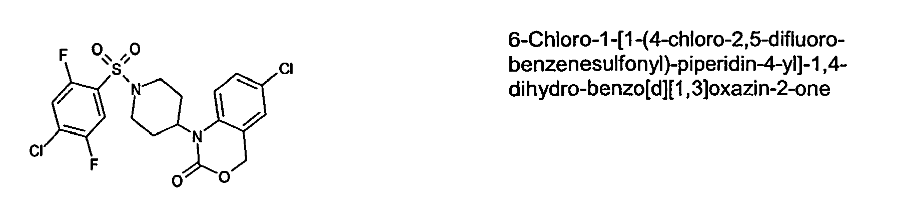

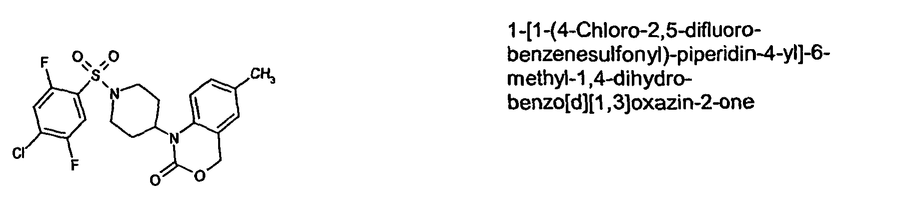

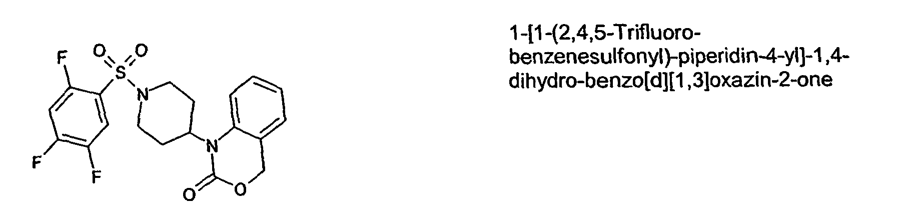

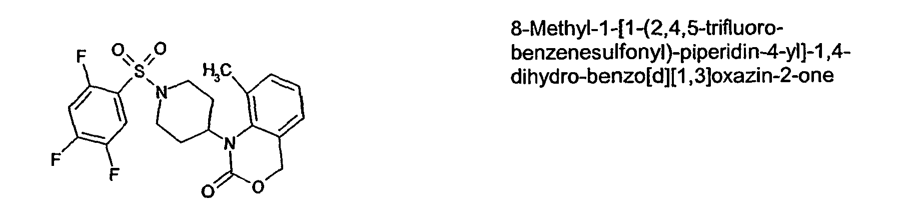

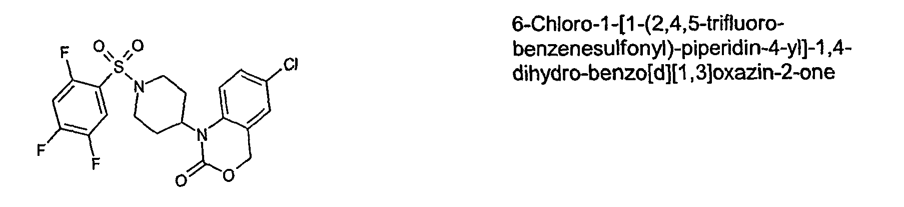

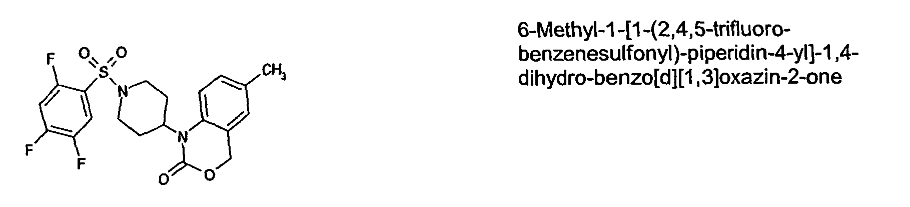

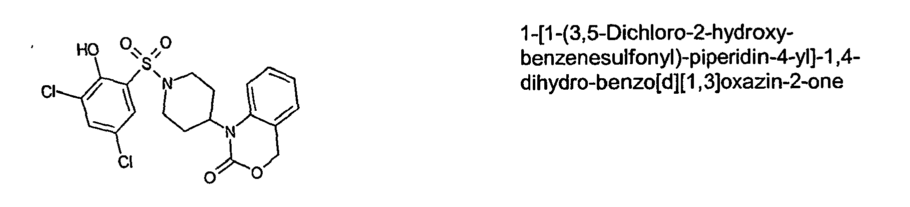

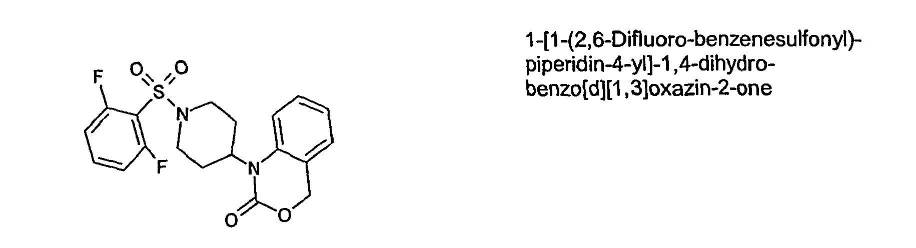

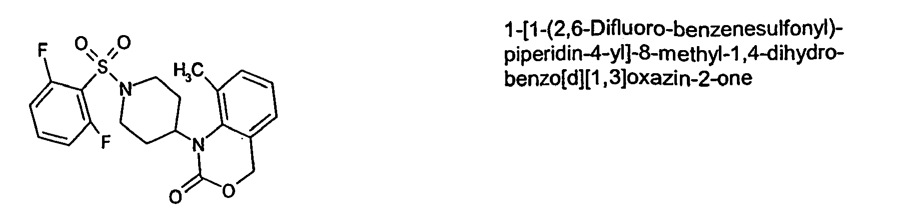

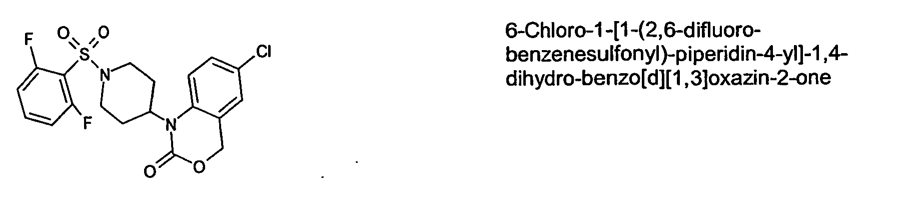

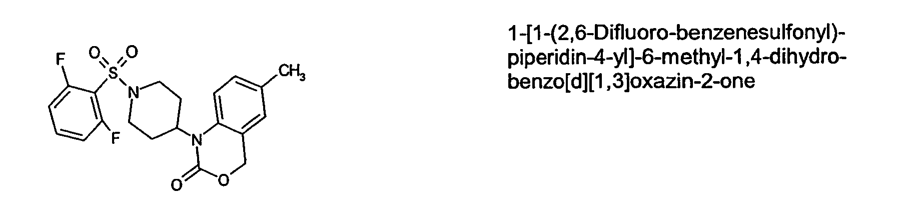

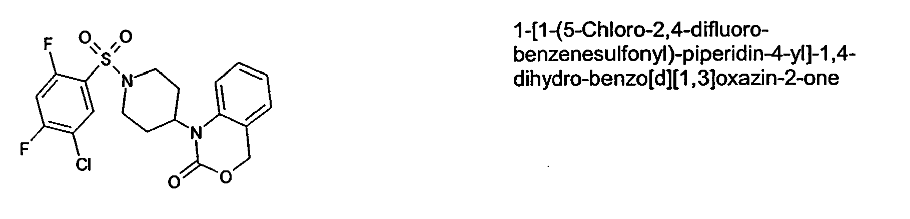

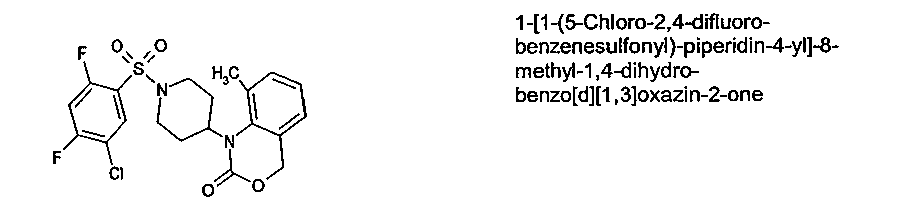

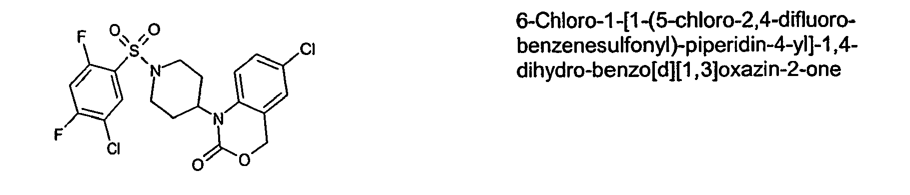

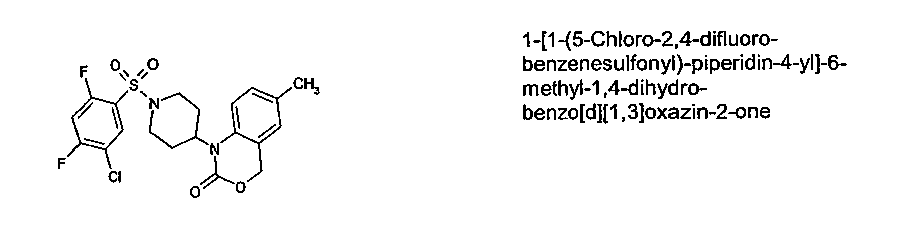

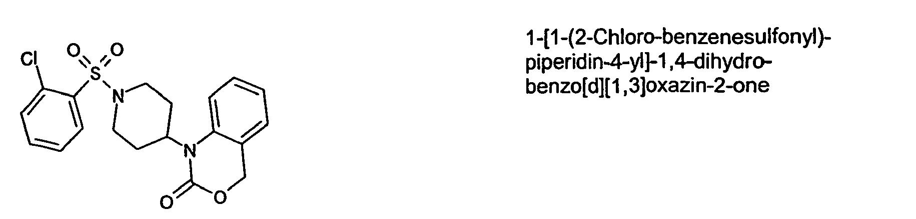

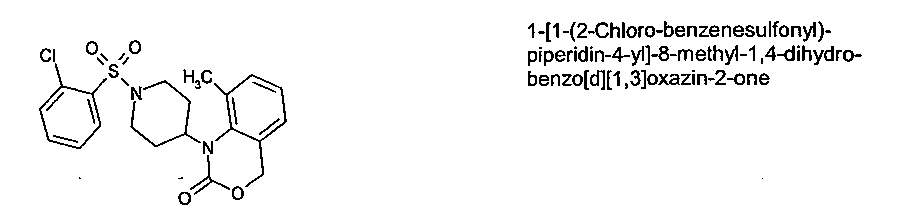

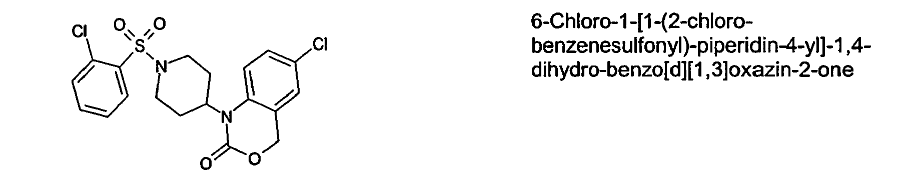

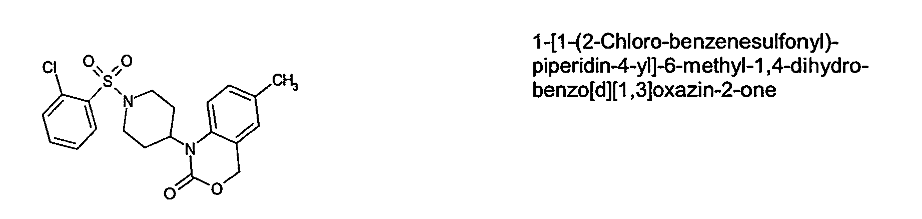

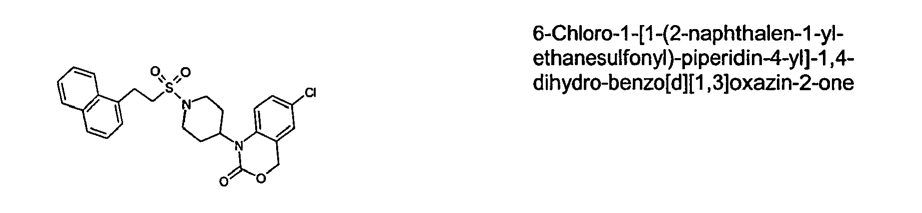

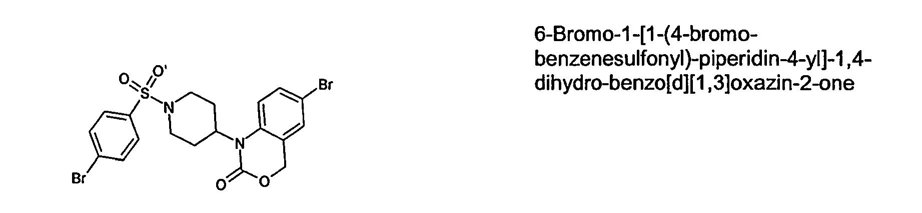

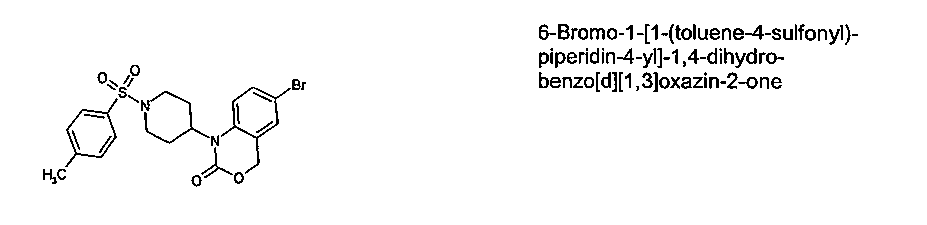

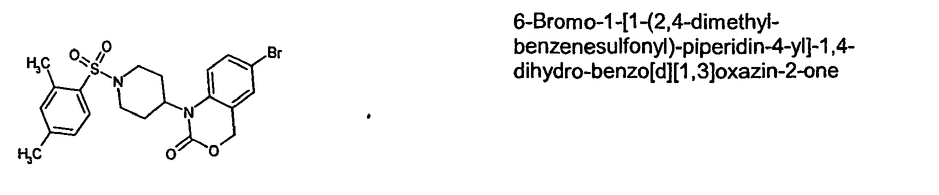

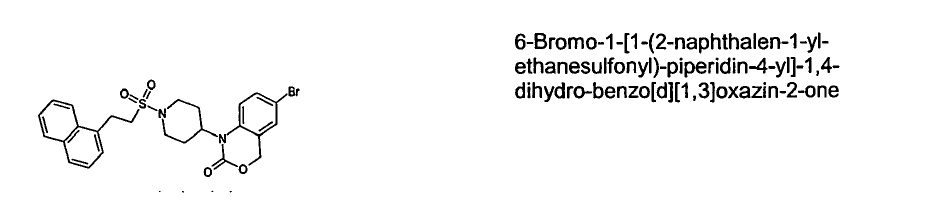

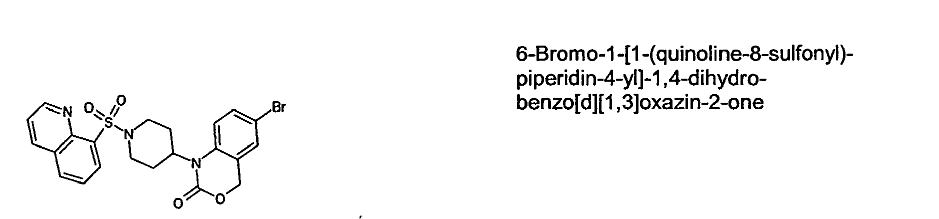

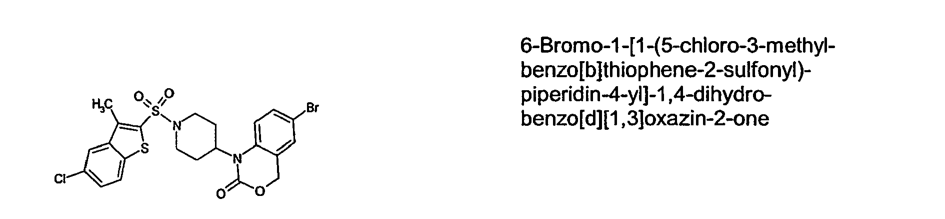

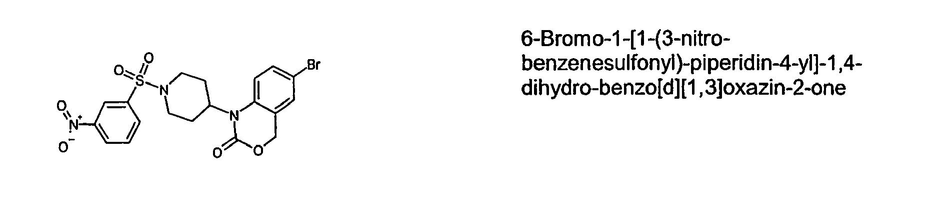

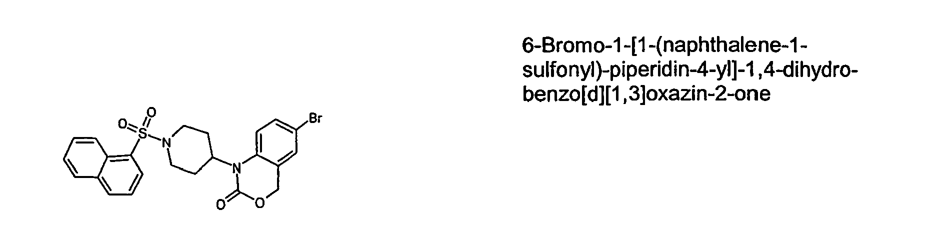

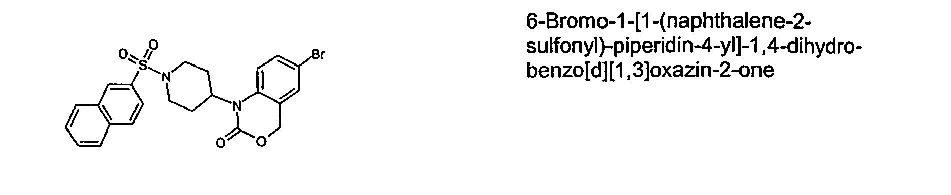

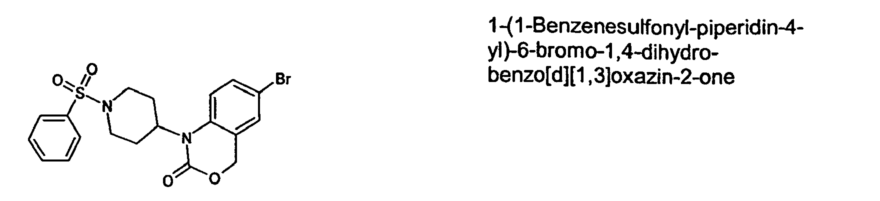

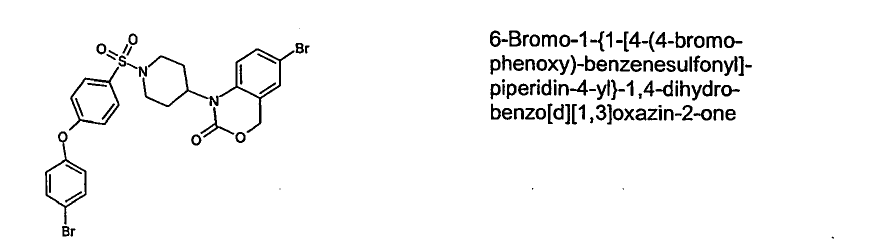

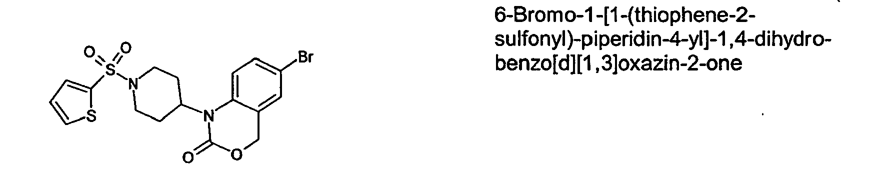

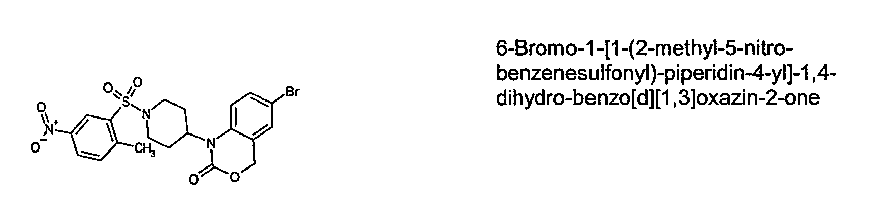

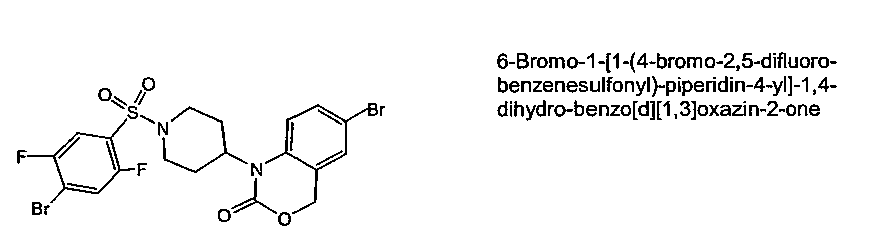

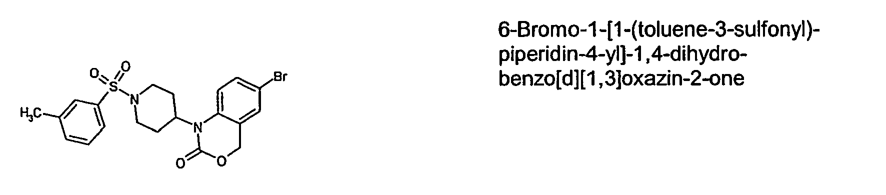

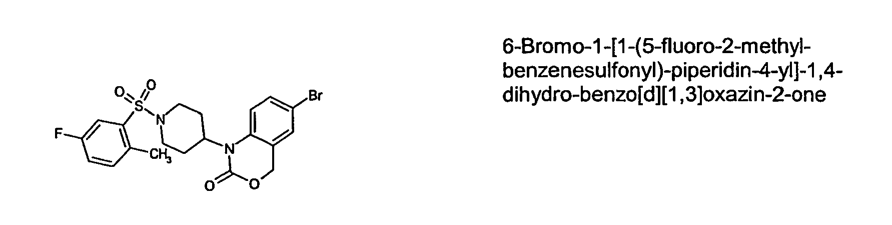

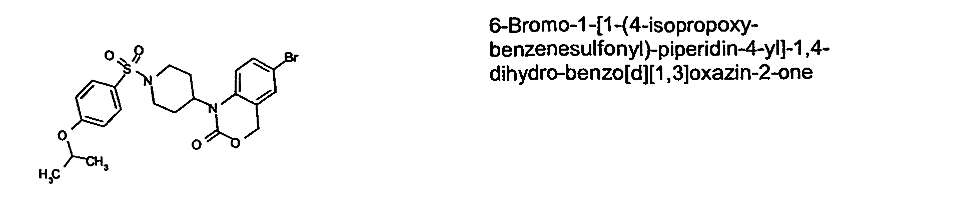

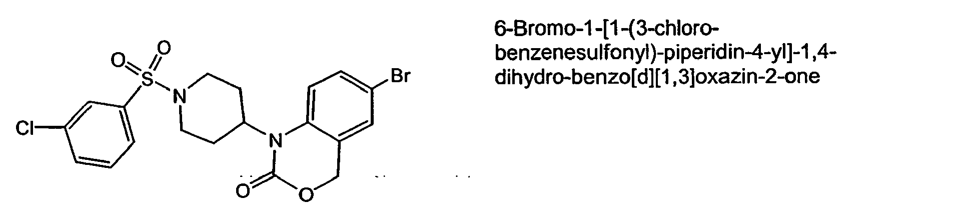

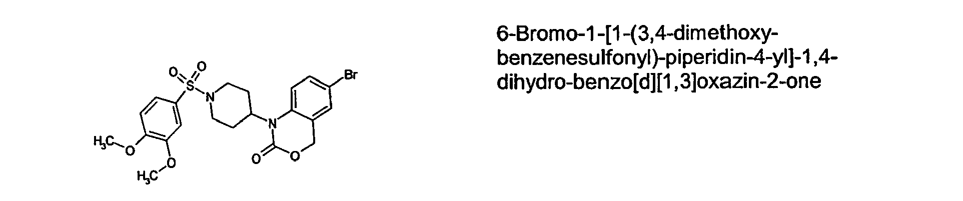

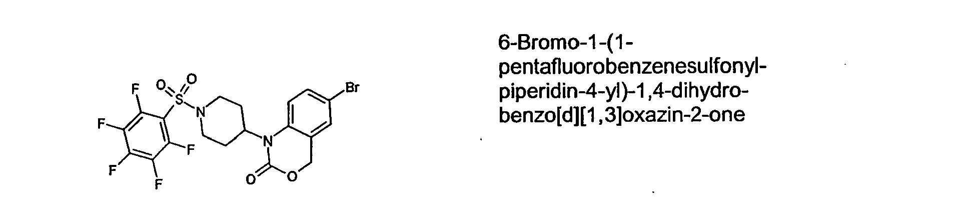

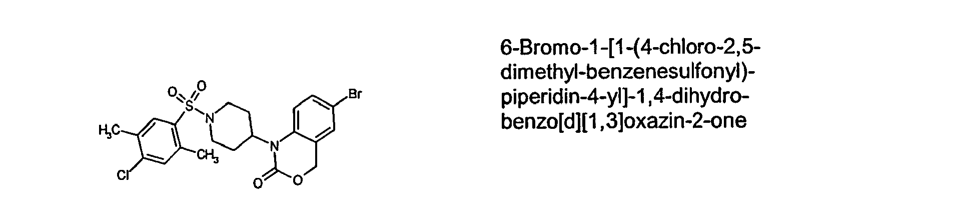

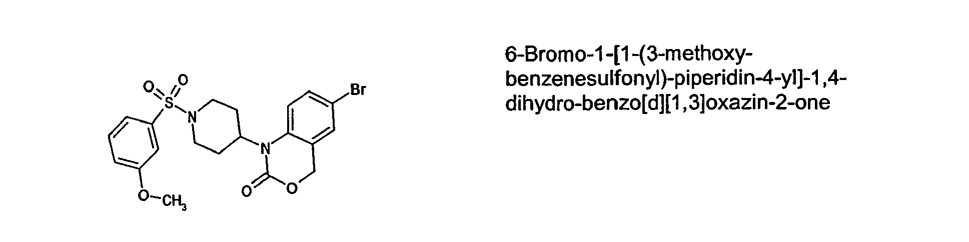

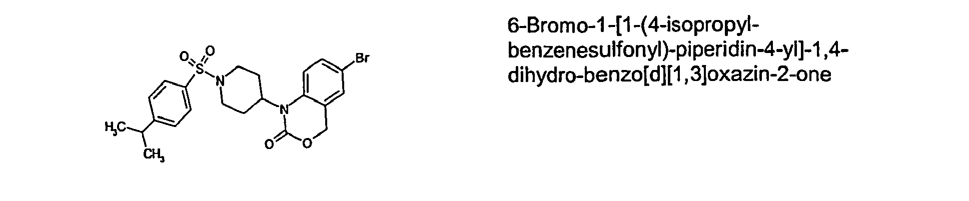

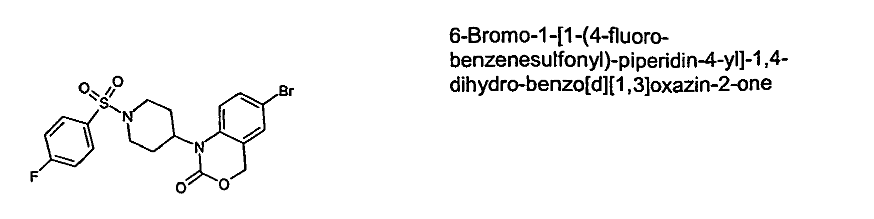

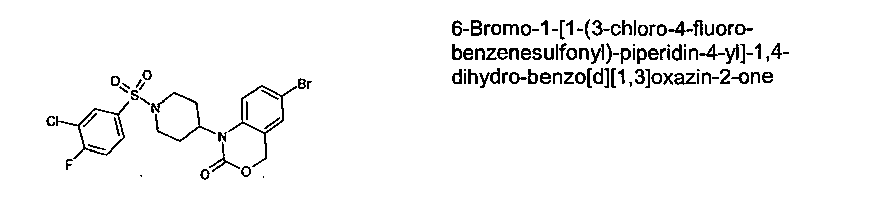

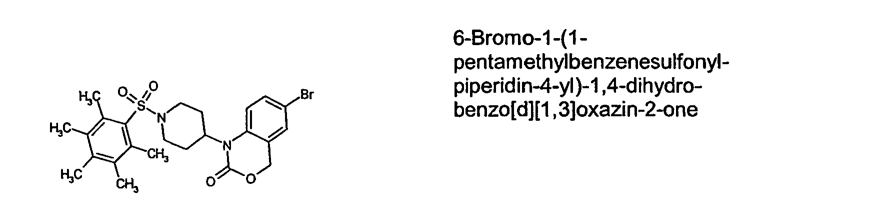

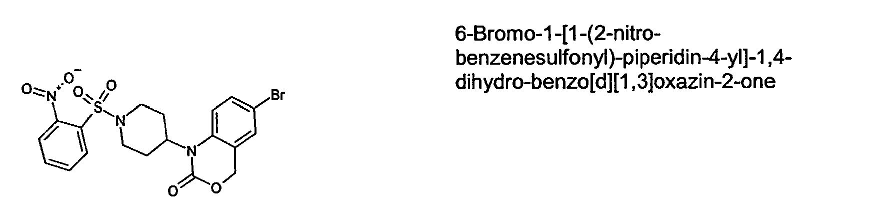

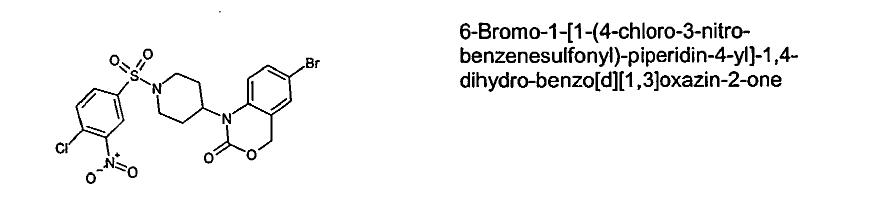

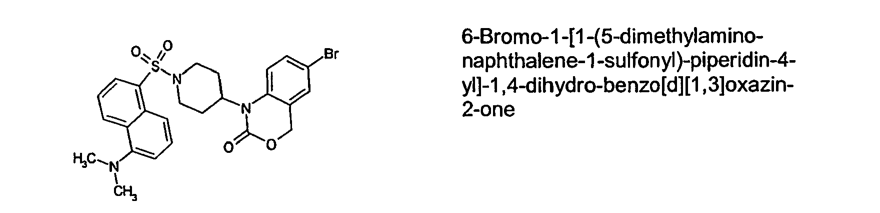

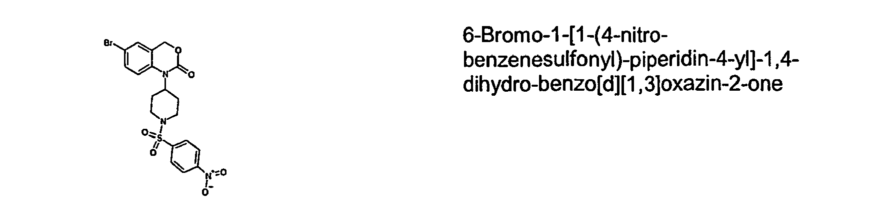

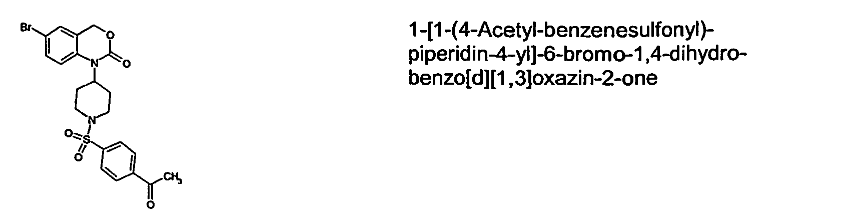

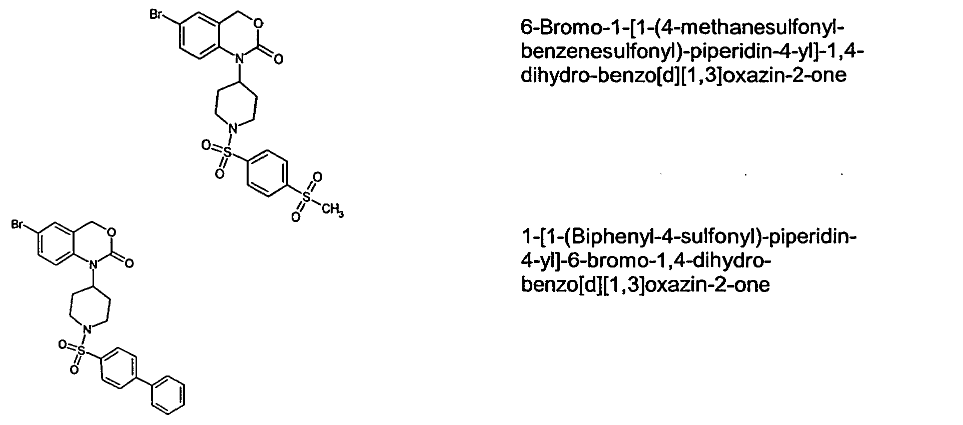

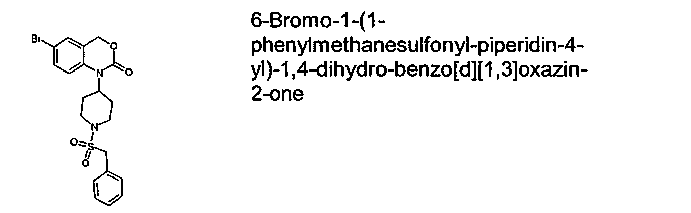

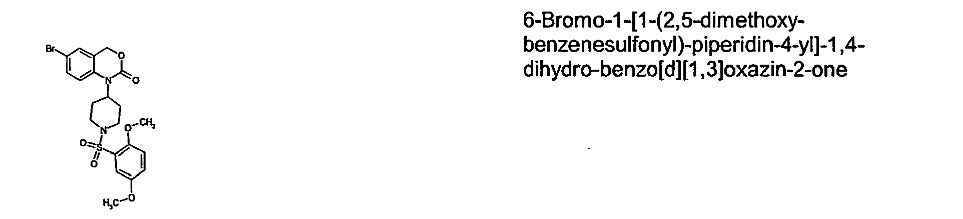

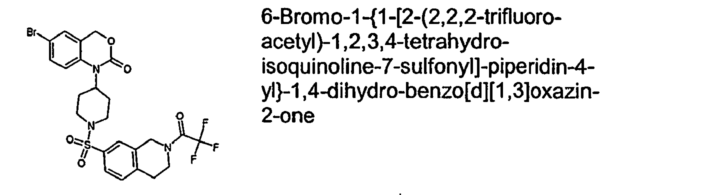

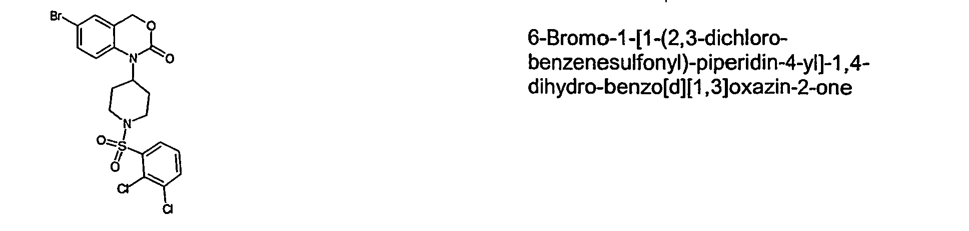

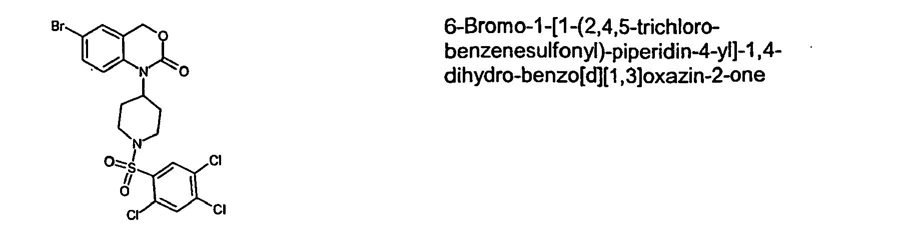

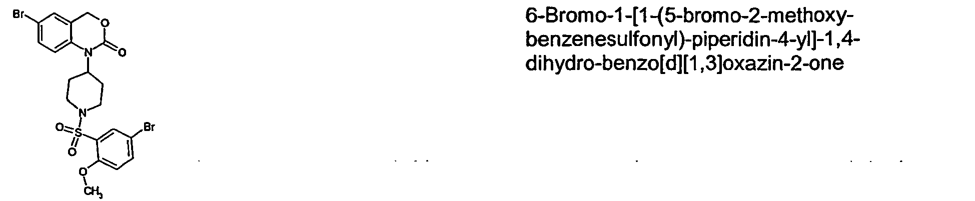

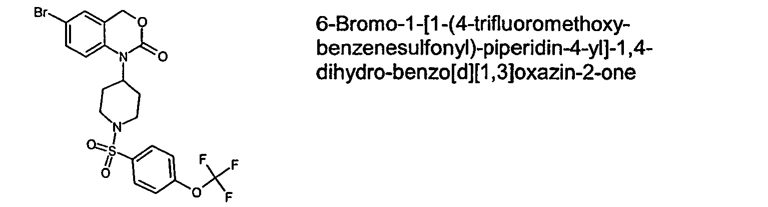

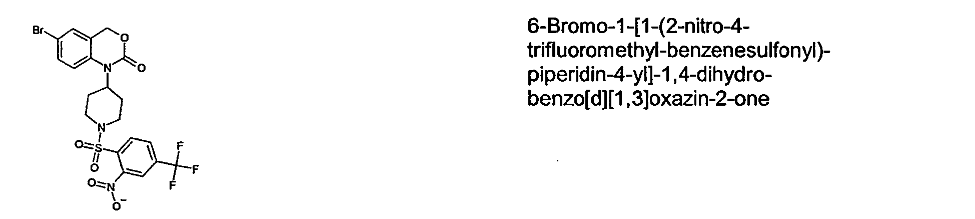

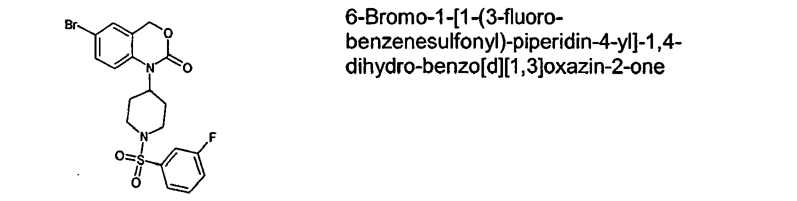

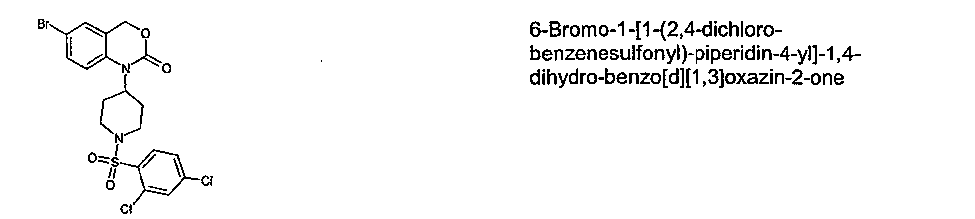

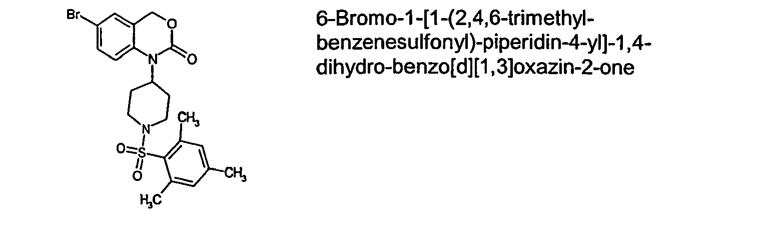

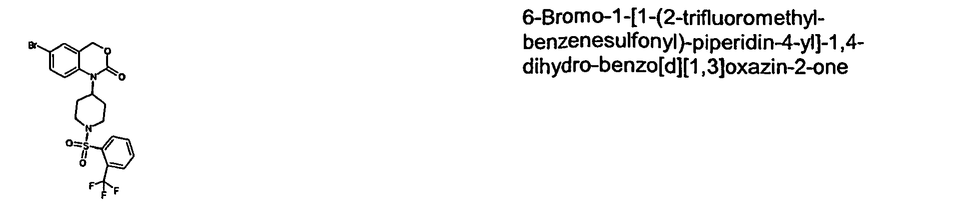

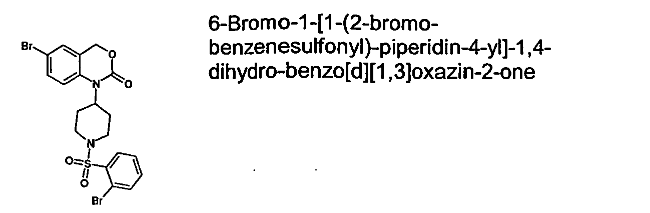

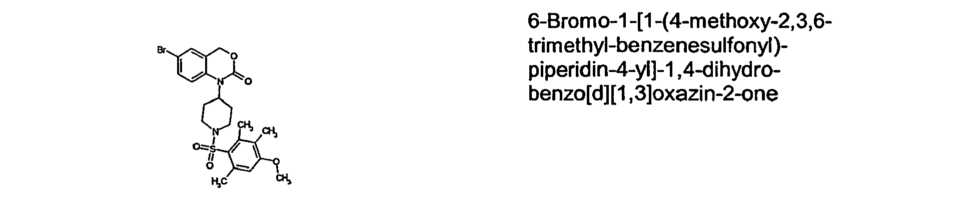

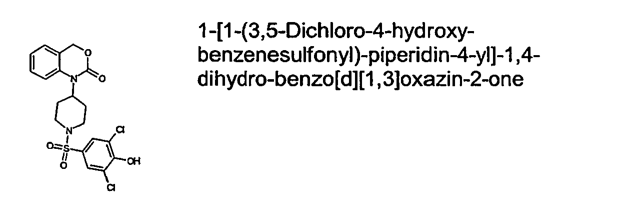

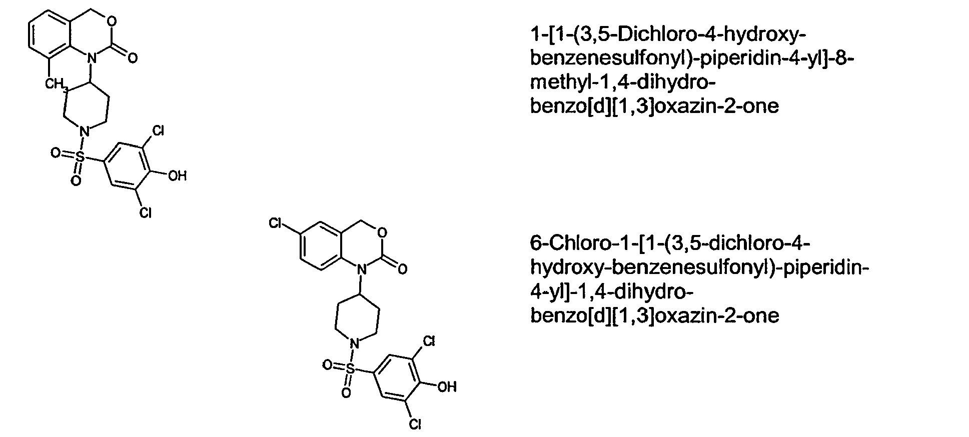

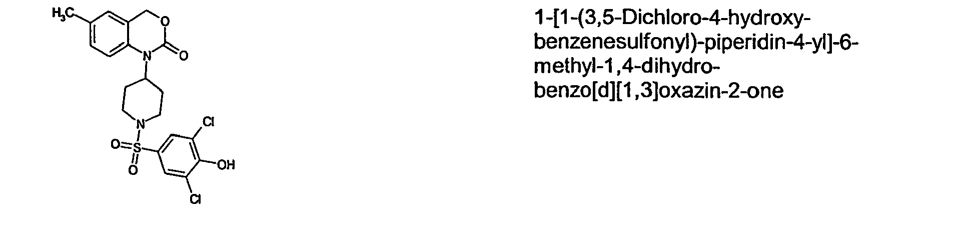

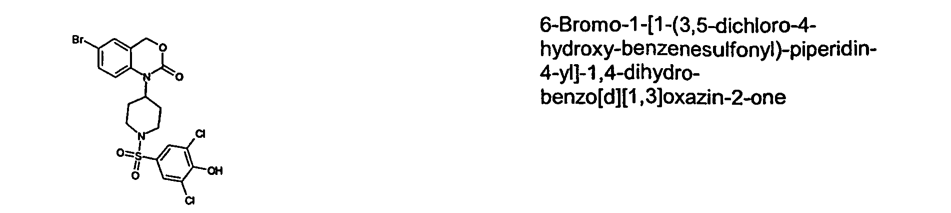

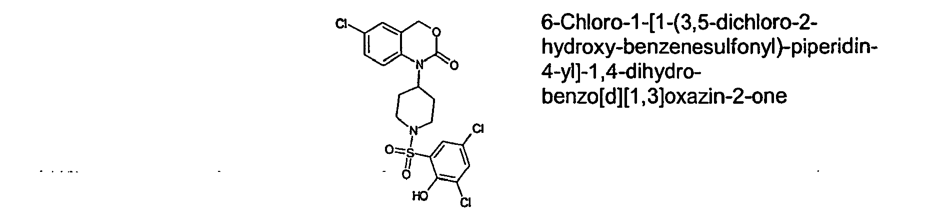

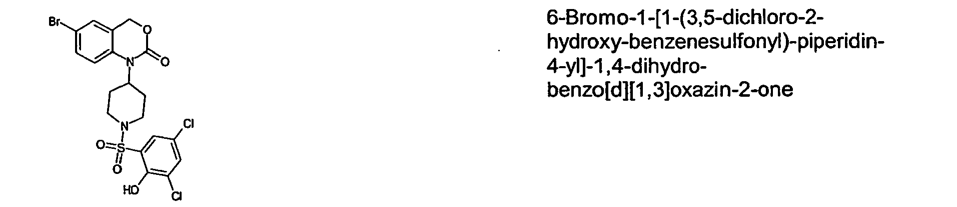

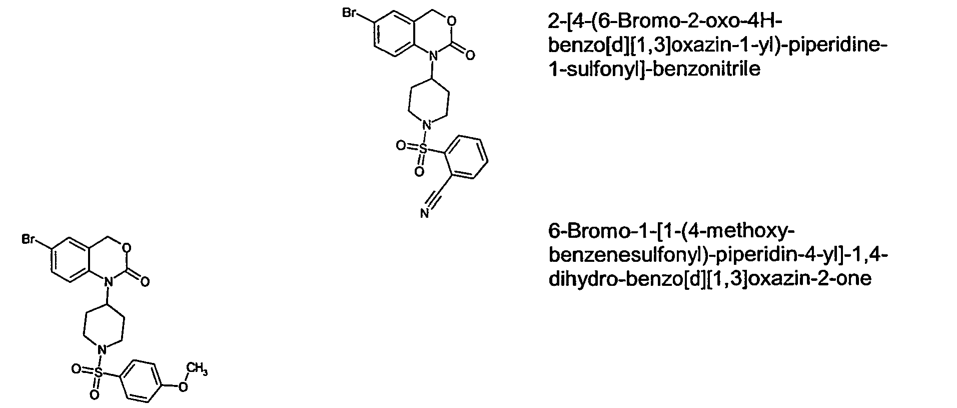

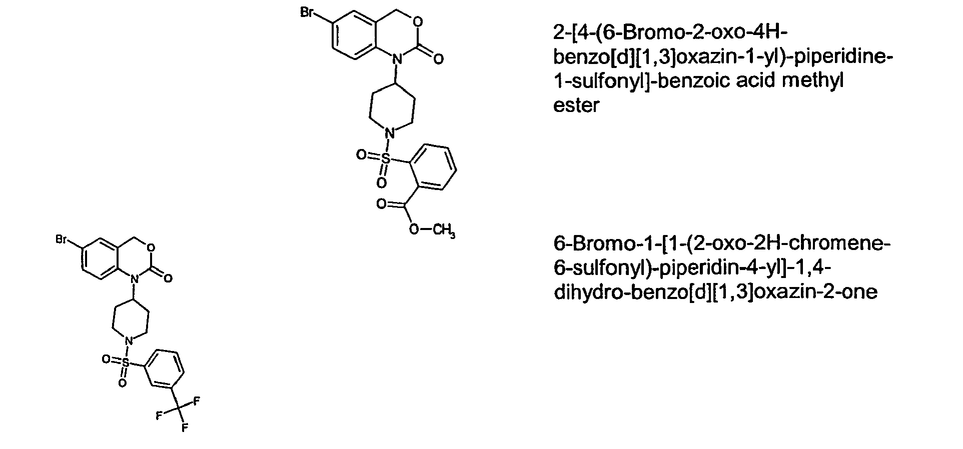

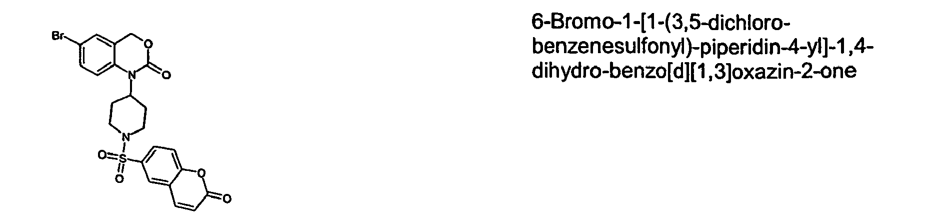

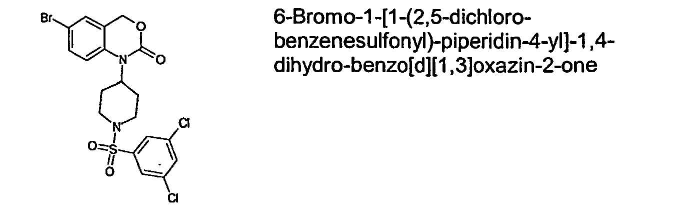

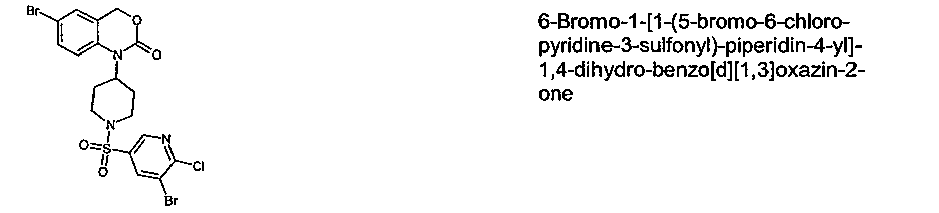

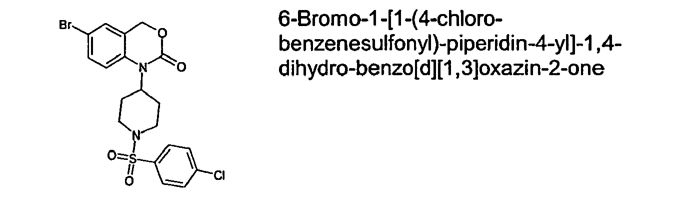

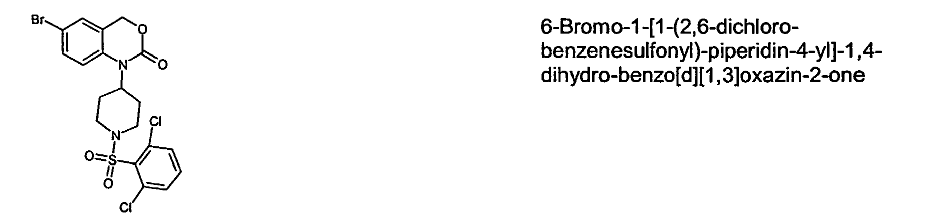

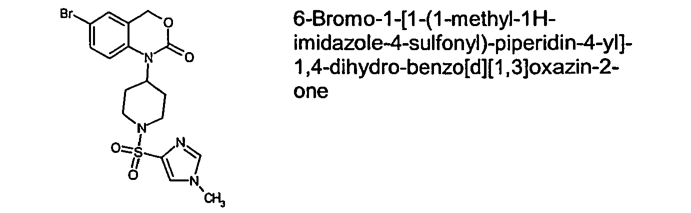

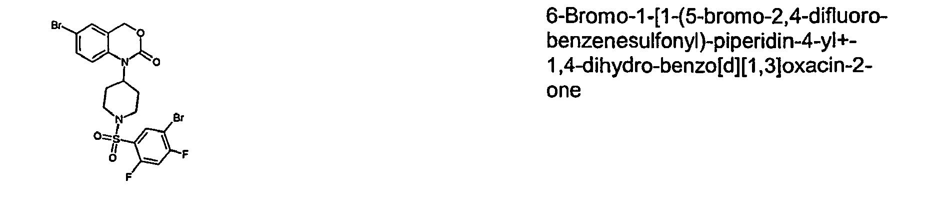

More particularly preferred are compounds of general formula (I) selected from the group consisting of
1-[1-(5-Chloro-3-methyl-benzo[b]thiophenyl-2-sulfonyl)-piperidine-4-yl]-1,4-dihydro-benzo[d][1,3]oxazin-2-one,
1-[1-(5-Dimethylamino-naphthyl-1-sulfonyl)-piperidine-4-yl]-8-methyl-1,4-dihydro-benzo[d][1,3]oxazin-2-one,
1-[1-(5-Dimethylamino-naphthyl-1-sulfonyl)-piperidine-4-yl]-1,4-dihydro-benzo[d][1,3]oxazin-2-one,
and corresponding salts thereof, and corresponding solvates.

In a further aspect the present invention also provides a process for the preparation of benzoxazinone-derived sulphonamide compounds of general formula (I), wherein R¹-R⁹ and W have the meaning given above, comprising reacting at least one piperidine compound of general formula (II) and/or a corresponding salt thereof, preferably a hydrochloride salt, 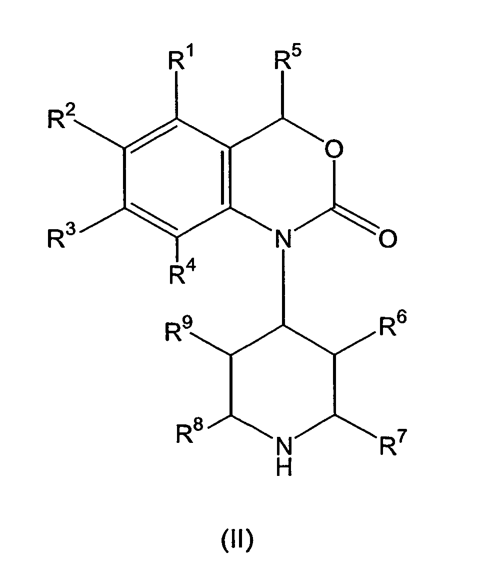 wherein R¹ to R⁹ have the meaning given above, with at least one compound of general formula (III), 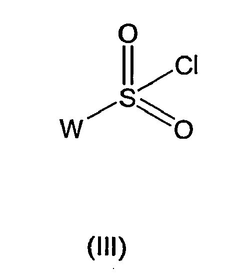 wherein W has the meaning given above, in a suitable reaction medium, optionally in the presence of at least one base and/or at least one auxiliary agent, to yield a compound of general formula (I).

Suitable reaction media include e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents, preferably ethyl acetate, triethylamine, pyridine, dimethylsulfoxide, diemthylformamide, hexamethylphosphoramide, acetonitril, acetone or nitromethane, are included. Mixtures based one or more of the afore mentioned solvents may also be used.

Bases that may be used in the processes according to the present invention are generally organic or inorganic bases, preferably alkali metal hydroxides, e.g. sodium hydroxyde or potassium hydroxyde, or obtained from other metals such as barium hydroxyde or different carbonates, preferably potassium carbonate, sodium carbonate, calcium carbonate, or alkoxides, e.g. sodium methoxide, potassium methoxide, sodium ethoxide, potassium methoxide, potassium ethoxide or potassium tert-butoxide, or organic amines, preferably triethylamine, diisopropyethylamine or heterocycles, e.g. 1,4-diazabicyclo[2.2.2] octane, 1,8-diazabicyclo[5.4.0]undec-7-ene pyridine, diamino pyridine, dimethylaminopyridine, methylpiperidine or morpholine.

Alkali metals such as sodium or ist hydrides, e.g. sodium hydride, may also be used. Mixtures based one or more of the afore mentioned bases may also be used.

During the synthetic reactions described above or while preparing the compounds of general formulas (II) or (III) the protection of sensitive groups or of reagents may be necessary and/or desirable. This can be performed by using conventional protective groups like those described in the literature [Protective groups in Organic Chemistry, ed. J. F.W. McOmie, Plenum Press, 1973; T.W. Greene & P.G.M.Wuts, Protective Groups in Organic Chemistry, John Wiley & sons, 1991. Said literature description is hereby incorporated by reference as part of the disclosure. The protective groups may also be eliminated as convenient by means well-known to those skilled in the art.

The compounds of general formulas (II) and (III) are either commercially available or can be produced according to methods known to those skilled in the art. The reaction of compounds of general formulas (II) and (III) to yield benzoxazinone-derived sulphonamide compounds of general formula (I) may also be facilitated by conventional methods known to those skilled in the art.

The substituted benzoxazinone compounds of general formula (II), wherein R⁵ represents H, are preferably synthesized from substituted anthranilic acid or a corresponding ester via the corresponding substituted benzylalcohol (see scheme 1, method A). By reductive amination with 1-Boc-(tert.-Butylcarbonyloxy)-4-piperidone the Boc-piperidin-moiety is introduced into the substituted benzylalcohol. The benzoxazinone-ring is formed by cyclisation with triphosgene. The elimination of the Boc-protecting group is carried out by treatment in acidic media according to the method described in Williams et al., J. Med. Chem. 1995 38, 4634 and later by Bell et al., J. Med. Chem., 1998, 41, 2146 which are hereby incorporated by reference and form part of the disclosure. By reacting such a substituted benzoxazinone compound of general formula (II) with a substituted sulfuryl chloride of general formula (III) compounds of general formula (I) are obtained.
By reduction of the corresponding ketones via conventional methods known to those skilled in the art, e.g. by reduction with sodium borohydride (see scheme 1, method B, R5=Z) benzoxazinone derived sulphonamide compounds of general formula (I), wherein R⁵ represents an unbranched or branched, saturated or unsaturated, optionally at least mono-substituted aliphatic radical or a saturated or unsaturated, optionally at least mono-substituted, optionally at least one heteroatom as ring member containing cycloaliphatic radical (denoted by Z in method B) can be obtained.
The respective reagents used in said process for the preparation of benzoxazinone derived sulphonamide compounds of general formula (I) are either commercially available or can be obtained by methods well known to those skilled in the art.

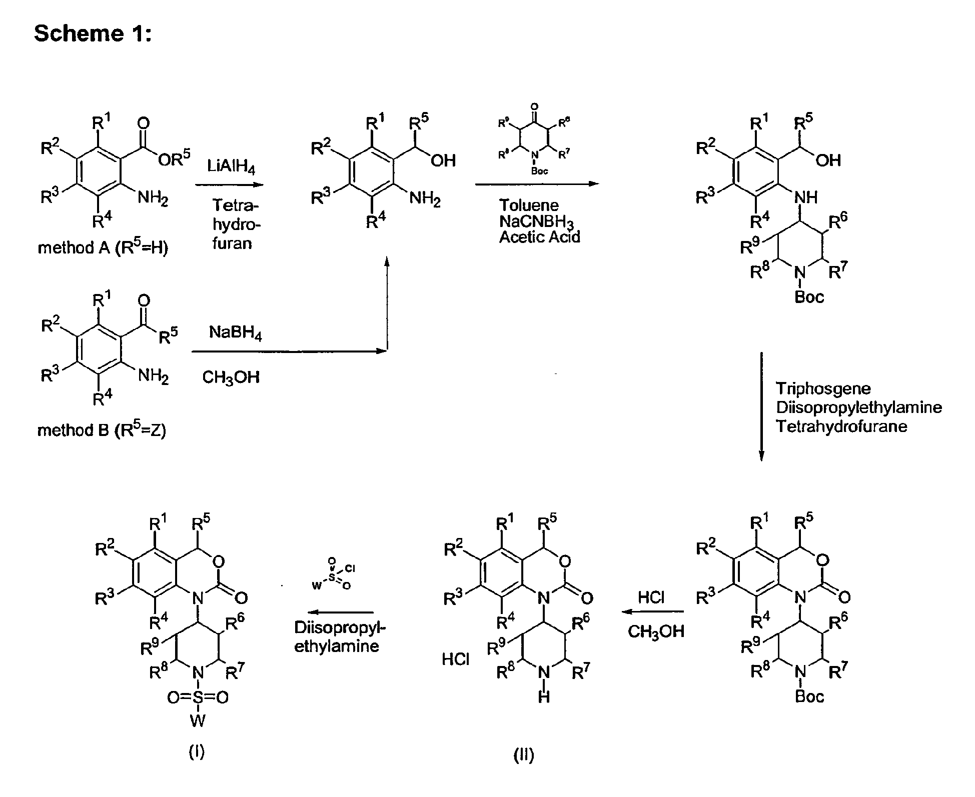

In a further aspect the present invention also provides a process for the preparation of salts of benzoxazinone-derived sulphonamide compounds of general formula (I), wherein at least one compound of general formula (I) having at least one basic group is reacted with at least one inorganic and/or organic acid, preferably in the presence of a suitable reaction medium. Suitable reaction media are, for example, the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of benzoxazinone-derived sulphonamide compounds of general formula (I), wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, the ones given above.

Solvates, preferably hydrates, of the Benzoxazinone-derived sulphonamide compounds of general formula (I) or of the salts thereof may also be obtained by standard procedures known to those skilled in the art.

If the Benzoxazinone-derived compounds of general formula (I) are obtained in form of a mixture of stereoisomers, particularly enantiomers or diastereomers, said mixtures may be separated by standard procedures known to those skilled in the art, e.g. chromatographic methods or crystallization with chiral reagents.

The purification and isolation of the Benzoxazinone-derived sulphonamide compounds of general formula (I) or a corresponding stereoisomer, or salt, or solvate respectively, if required, may be carried out by conventional methods known to those skilled in the art, e.g. chromatographic methods or recrystallization.

The Benzoxazinone-derived sulphonamide compounds of general formula (I), their stereoisomers, the corresponding salts and the corresponding solvates are toxicologically acceptable and are therefore suitable as pharmaceutical active substances for the preparation of medicaments.

The present invention therefore also provides for a medicament comprising at least one benzoxazinone-derived sulphonamide compound of general formula (I), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a corresponding solvate, and optionally one or more pharmaceutically acceptable adjuvants.

Furthermore, the present invention also provides for a pharmaceutical composition comprising at least one benzoxazinone-derived sulphonamide compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a corresponding solvate and optionally one or more pharmaceutically acceptable adjuvants, which is not yet formulated into a medicament.

Preferably the medicament is suitable for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity, disorders of the central nervous system, disorders of the gastrointestinal tract, such as irritable intestine syndrom, anxiety, panic, depression, cognitive memory disorders, senile dementia disorders, such as Morbus Alzheimer, Morbus Parkinson and Morbus Huntington, schizophrenia, psychosis, infantile hyperkinesia, ADHC (attention deficit, hyperactivity disorders) particularly in mammals, including humans.

A further aspect of the present invention is the use of at least one benzoxazinone-derived compound of general formula (I) for the manufacture of a medicament for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity, disorders of the central nervous system, disorders of the gastrointestinal tract, such as irritable intestine syndrom, anxiety, panic, depression, cognitive memory disorders, senile dementia disorders, such as Morbus Alzheimer, Morbus Parkinson and Morbus Huntington, schizophrenia, psychosis, infantile hyperkinesia, ADHC (attention deficit, hyperactivity disorders) particularly in mammals, including humans.

In yet a further aspect, the present invention also provides for the use of at least one benzoxazinone-derived sulphonamide compound of general formula (Ia) 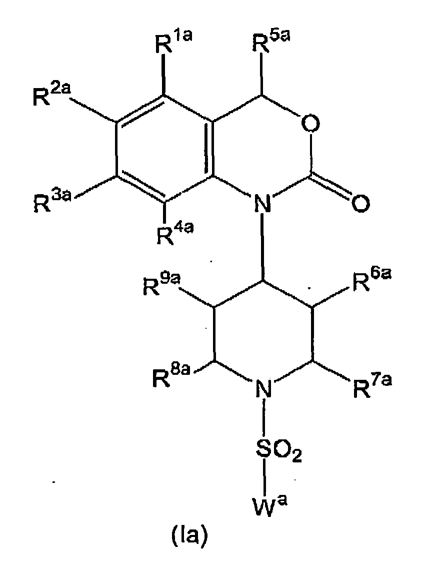 wherein
R^{1a}, R^{2a}, R^{4a} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, - OR^{10a}, -OC(=O)R^{11a}, -C(=O)-OR^{11a}, -SR^{12a}, SOR^{12a}, -SO₂R^{12a}, -NH-SO₂R^{12a}, - SO₂NH₂ and a -NR^{13a}R^{14a} moiety,
R^{5a} represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R^{6a} , R^{7a}, R^{8a}, R^{9a} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a -COOR ^{15a} moiety,
W^{a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ring-system, a NR^{16a}R^{17a}-moiety or a -COR^{18a}-moiety,
R^{10a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{12a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13a} and R^{14a} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{13a} and R^{14a} together with the bridging nitrogen atom form a saturated, unsaturated or aromatic heterocyclic ring, which may be at least mono-substituted and/or contain at least one further heteroatom as a ring member,
R^{15a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{16a} represents an unbranched or branched, saturated or unsaturated, optionally at least mono-substituted aliphatic radical,
R^{17a} represents an unbranched or branched, saturated or unsaturated, optionally at least mono-substituted aliphatic radical,
R^{18a} represents an optionally at least mono-substituted aryl radical,
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively,
for the manufacture of a medicament for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity, disorders of the gastrointestinal tract, such as irritable intestine syndrom, anxiety, panic, depression, cognitive memory disorders, senile dementia disorders, such as Morbus Alzheimer, Morbus Parkinson and Morbus Huntington, schizophrenia, psychosis, infantile hyperkinesia, ADHC (attention deficit, hyperactivity disorders) particularly in mammals, preferably humans.

If one or more of the residues R^{1a}-R^{17a} and W^{a} represents an aliphatic radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl. -SQ-C₁₋₄-alkyl, -SO₂-C₁₋₄-alkyl, -NH-SO₂-C₁₋₄-alkyl, wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and an unsubstituted phenyl radical. If any one of the above mentioned substitutents itself is at least mono-substituted, said substituents may preferably be selected from the group consisting of F, Cl, methyl and methoxy.

If one or more of the residues R^{1a}-R^{15a} represents or comprises a cycloaliphatic radical, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, phenoxy, benzoyl, cyclohexyl, branched or unbranched C₁₋₄-perfluoroalkyl, -NR^{A}R^{B} wherein R^{A}, R^{B} are each independently selected from the group consisting of H, a branched or unbranched C₁₋₄-alkyl-radical, -CH₂-CH₂-OH and phenyl, carboxy, keto, amido, cyano, nitro, - SO₂NH₂, -CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may in each case be branched or unbranched, unsubstituted or at least mono-substituted phenyl or naphthyl and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, methoxy, ethoxy, keto, benzoyl, phenoxy, cyclohexyl, -CF₃, -CO-CH₃, -CO-OCH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any one of the above mentioned substitutents itself is at least mono-substituted, said substituents may preferably be selected from the group consisting of F, Cl, methyl and methoxy.

If one or more of the residues R^{1a}-R^{4a} and R^{10a}-R^{15a} and W^{a} comprises an alkylene group, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SQ-C₁₋₄-alkyl, -SO₂-C₁₋₄-alkyl, -NH-SO2-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, methoxy, ethoxy, CF₃ and unsubstituted phenyl. If any one of the above mentioned substitutents itself is at least mono-substituted, said substituents may preferably be selected from the group consisting of F, Cl, methyl and methoxy.

If one or more of the residues R^{1a}-R^{4a} and R^{10a}-R^{15a} comprises a mono- or polycyclic ringsystem, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, branched or unbranched C₁₋₄-perfluorocarbonyl, branched or unbranched C₁₋₄-perfluoroalkyl, amino, carboxy, amido, cyano, keto, nitro, -SO₂NH₂, -CO-C₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl, more preferably from the group consisting of hydroxy, F, Cl, Br, methyl, ethyl, methoxy, ethoxy, CF₃, -(C=O)-CF₃, keto, cyano and an unsubstituted phenyl radical. If any one of the above mentioned substitutents itself is at least mono-substituted, said substituents may preferably be selected from the group consisting of F, Cl, methyl and methoxy.

If one or more of the residues R^{1a}-R^{4a}, R^{10a}-R^{15a} and R^{18a} represents or comprises an aryl radical, which is substituted by one or more substituents, unless defined otherwise, each of these substituents may preferably be selected from the group-consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{A}R^{B} wherein R^{A}, R^{B} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, -SO-C₁₋₄-alkyl, - SO₂-C₁₋₄-alkyl, -NH-SO2-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, nitro, -CH(OH)(phenyl), methoxy, ethoxy, unsubstituted or at least mono-substituted benzoyl, unsubstituted or at least mono-substituted phenoxy, cyclohexyl, CF₃, OCF₃,-CO-CH₃, -CO-OCH₃, SO₂-CH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any of the above mentioned substitutents itself is at least mono-substituted, said substituents may preferably be selected from the group consisting of F, Cl, Br, CF₃, OCF₃, methyl and methoxy.

If one or more of the residues R^{1a}-R^{4a} and R^{10a}-R^{15a} represents or comprises a heteroaryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{A}R^{B} wherein R^{A}, R^{B} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, nitro, -CH(OH)(phenyl), methoxy, ethoxy, unsubstituted or at least mono-substituted benzoyl, unsubstituted or at least mono-substituted phenoxy, cyclohexyl, CF₃, OCF₃,-CO-CH₃, -CO-OCH₃, SO₂-CH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any of the above mentioned substitutents itself is at least mono-substituted, said substituents may preferably be selected from the group consisting of F, Cl, Br, CF₃, OCF₃, methyl and methoxy.

If R^{13a} and R^{14a} form a heterocyclic ring, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO2-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, methyl, CF₃ and an unsubstituted phenyl radical. If any of the above mentioned substitutents itself is at least mono-substituted, said substituents may preferably be selected from the group consisting of F, Cl, methyl and methoxy.

If R^{13a} and R^{14a} form a heterocyclic ring, which contains one or more further heteroatoms as ring members, unless defined otherwise, each of these heteroatoms may preferably be selected from the group consisting of N, O and S, more preferably from the group consisting of N and O.

If one or more of the residues R^{1a}-R^{15a} and W^{a} represents or comprises a cycloaliphatic radical, which contains one or more heteroatoms as ring members, unless defined otherwise, each of these heteroatoms may preferably be selected from the group consisting of N, O, S and P, more preferably from the group consisting of N, O and S.

If one or more of the residues R^{1a}-R^{4a}, R^{10a}-R^{15a} and W^{a} represents or comprises an heteroaryl radical, which contains one or more heteroatoms as ring members, unless defined otherwise, each of these heteroatoms may preferably be selected from the group consisting of N, O, S and P, more preferably from the group consisting of N, O and S.

If W^{a} represents or comprises a cycloaliphatic radical, a heteroaryl radical, an aryl radical and/or a mono- or polycyclic ring system, which is substituted by one or more substituents, unless defined otherwise, each of these substituents may preferably be selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆-alkenyl, SO₂-C₁₋₄-alkyl, - (C=O)-C₁₋₅-alky), -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅-alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, S0O₂-morpholinyl-, thiadiazolyl, oxadiazolyl, oxazolyl, thiazolyl, isoxazolyl, O-CH₂-thiazolyl,-, NH-phenyl, and -C₁₋₄-Alkylen-NH-(C=O)-phenyl, more preferably from the group consisting of hydroxy, nitro, carboxy, cyano, keto, F, Cl, Br, I, C₁₋₁₂-alkyl, CH₂F, CHF₂, CF₃, CH₂Cl, CH₂Cl₂, CCl₃, CH₂Br, CHBr₂, CBr₃, OCF₃, OCHF₂, OCH₂F, O-CH₂-CF₃, vinyl, SO₂-CH₃, - (C=O)-CH₃, -(C=O)-C₂H₅. -(C=O)-O-CH₃, -(C=O)-O-C₂CH₅, -(C=O)-Cl, -S-CH₃-, - (C=O)-H, -NH-(C=O)NH-CH₃, -(C=O)-CF₃, dimethylamino, diethylamino, di-n-propylamino, di-iso-propylamino, di-n-butylamino, di-tert-butyamino, NH-(C=O)-CH₃, - CH₂-(C=O)-CH₃, -CH₂-(C=O)-C₂H₅, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -CH₂-NH-(C=O)-phenyl.

If any of the afore mentioned substituents itself is substituted by one or more substituents, said substituents may preferably be selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, -C(=O)-O-C₁₋₅-alkyl, -(C=O)-CH₂-F, -(C=O)-CH₂-Cl, -(C=O)-CH₂-Br, preferably from the group consisting of F, Cl, Br, CH₂F, CHF₂, CF₃, CH₂Cl, CHCl₂, CCl₃, CH₂Br, CHBr₂, CBr₃, nitro, cyano, hydroxy, -(C=O)-CH₃, CH₃, C₂H₅, -S-CH₃, -C(=O)-O-CH₃, -C(=O)-O-C₂H₅, -(C=O)-CH₂-F, -(C=O)-CH₂-Cl and -(C=O)-CH₂-Br.

Preferred is the use of compounds of general formula (Ia), wherein R^{1a}, R^{2a}, R^{3a}, R^{4a} are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano group, -OR^{10a}, -OC(=O)R^{11a}, -SR^{12a}, -SOR^{12a}, -SO₂R^{12a}, -NH-SO₂R^{12a}, -SO₂NH₂ and a -NR^{13a}R^{14a} moiety,
preferably selected from the group consisting of H, F, Cl, Br, a saturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro, cyano, -OR^{10a}, - OC(=O)R^{11a}, -SR^{12a} and -NR^{13a}R^{14a} moiety,
more preferably selected from the group consisting of H, F, Cl, CH₃, CH₂CH₃, CF₃, CF₂CF₃, cyclopentyl, cyclohexyl, a nitro group, a cyano group and -OR^{10a}, and R^{5a}-R^{18a} and W^{a} have the meaning as defined above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (Ia), wherein R^{5a} represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₈-cycloaliphatic radical,
preferably represents H or a branched or unbranched C₁₋₃-alkyl radical,
more preferably H, -CH₃ or -CH₂CH₃,
and R^{1a}-R^{4a}, R^{6a} -R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Preference is also given to the use of compounds of general formula (Ia), wherein R^{6a}, R^{7a}, R^{8a}, R^{9a} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano group and a -COOR^{15a} moiety,
preferably selected from the group consisting of H, a branched or unbranched C₁₋₃-alkyl radical, a cyano group and a -COOR^{15a} group,
more preferably from the group consisting of H, -CH₃, -CH₂CH₃ and a cyano moiety, and R^{1a}-R^{5a}, R^{10a}-R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (Ia), wherein W^{a} represents an unbranched or branched, saturated or unsaturated, optionally at least mono-substituted C₁₋₂₀ aliphatic radical, a saturated or unsaturated, optionally at least mono-substituted, optionally at least one heteroatom as ring member containing C₃₋₈ cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆- alkylene group and/or may be condensed with an optionally at least mono-substituted mono- or polycyclic ring-system, a NR^{16a}R^{17a}-moiety or a COR^{18a}-moiety,
preferably is selected from the group consisting of 1-Naphthyl-, 5-Dimethylaminonapth-1-yl, 2-Naphthyl-, 2-Acetamido-4-methyl-5-thiazolyl-, 2-Thienyl-, 8-Quinolinyl-, Phenyl-, Pentafluorophenyl-, 2,4,5-Trichloro-phenyl-, 2,5-Dichloro-phenyl-, 2-Nitrophenyl-, 2,4-Dinitro-phenyl-, 3,5-Dichloro-2-hydroxy-phenyl-, 2,4,6-Trisisopropyl-phenyl-, 2-Mesityl-, 3-Nitro-phenyl-, 4-Bromo-phenyl-, 4-Fluoro-phenyl-, 4-Chlorophenyl-, 4-Chloro-3-nitro-phenyl-, 4-lodo-phenyl-, N-Acetyl-sulfanilyl-, 4-Nitrophenyl-, 4-Methoxy-phenyl-, Benzoic-acid-4-yl-, 4-tert-Butyl-phenyl-, p-Tolyl-, Trifluoromethyl-, Trichloromethyl-, Isopropyl-, Methyl-, Benzyl-, trans-styryl-, 2,2,2-Trifluoroethyl-, Ethyl-, Hexadecyl-, 2-Chloroethyl-, n-Propyl-, 3-Chloro-propyl-, n-Butyl-, Methyl-benzoate-2-yl-, 2-Nitro-4-(trifluoromethyl)-phenyl-, Pentamethyl-phenyl-, 2,3,5,6-Tetramethyl-phenyl-, 3-(Trifluoromethyl)phenyl-, 3,5-Bis-(Trifluoromethyl)-phenyl-, Dichloromethyl-, Chloromethyl-, Dodecyl-, 1-Octyl-, 2,3,4-Trichloro-phenyl-, 2,5-Dimethoxy-phenyl-, o-Tolyl-, p-xylyl-2-yl-, Benzoic-acid-3-yl-, 4-Chloro-3-(trifluoromethyl)-phenyl-, 4-Chloro-5-nitro-benzoic acid-3-yl-, 6-(p-toluidino)-naphth-2-yl-, 4-Methoxy-2,3,6-trimethylphenyl-, 3,4-Dichlorophenyl-, 4,5-Dibromo-thiophene-2-yl-, 3-Chloro-4-fluoro-phenyl-, 4-Ethylphenyl-, 4-n-Propyl-phenyl-, 4-(1,1-Dimethylpropyl)-phenyl-, 4-Isopropyl-phenyl-, 4-Bromo-2,5-difluoro-phenyl-, 2-Fluoro-phenyl-, 3-Fluoro-phenyl-, 4-(Trifluoromethoxy)-phenyl-, 4-(Trifluoromethyl)-phenyl-, 2,4-Difluoro-phenyl-, 2,4-Dichloro-5-methyl-phenyl-, 4-Chloro-2,5-dimethyl-phenyl-, 5-Diethylamino-napth-2-yl-, Benzoyl chloride-3-yl-, 2-Chloro-phenyl-, 1-Octadecyl-, 4-Bromo-2,5-dichloro-thiophene-3-yl-, 2,5-Dichloro-thiophene-3-yl-, 5-Chloro-thiophene-2-yl-, 2-Methyl-5-nitro-phenyl-, 2-(Trifluoromethyl)-phenyl-, 3-Chloro-phenyl-, 3,5-Dichloro-phenyl-, 1-Decyl-, 3-Methyl-phenyl-, 2-Chloro-6-methyl-, 5-Bromo-2-methoxy-phenyl-, 3,4-Dimethoxy-phenyl-, 2-3-Dichloro-phenyl-, 2-Bromo-phenyl-, 3,5-Dichloro-4-(2-chloro-4-nitrophenoxy)-phenyl-, 2,3-Dichloro-thiophene-5-yl-, 3-Bromo-2-chloro-thiophene-5-yl-, 3-Bromo-5-chloro-thiophene-2-yl-, 2-(Benzoylaminomethyl)-thiophene-5-yl-, 4-(Phenylsulphonyl)-thiophene-2-yl-, 2-Phenyl-sulphonyl-thiophene-5-yl-, 3-Chloro-2-methyl-phenyl-, 2-[1-Methyl-5-(trifluoromethyl)pyrazol-3-yl]-thiophene-5-yl-, 5-Pyrid-2-yl-thiophene-2-yl-, 2-Chloro-5-(trifluoromethyl)-phenyl-, 2,6-Dichloro-phenyl-, 3-Bromo-phenyl-, 2-(Trifluoromethoxy)-phenyl-, 4-Cyano-phenyl-, 2-Cyano-phenyl-, 4-n-Butoxy-phenyl-, 4-Acetamido-3-chloro-phenyl, 2,5-Dibromo-3,6-difluoro-phenyl-, 5-Chloro-1,3-dimethylpyrazole-4-yl-, 3,5-Dimethylisoxazole-4-yl-, 2-(2,4-Dichlorophenoxy)-phenyl-, 4-(2-Chloro-6-nitro-phenoxy)-phenyl-, 4-(3-Chloro-2-cyano-phenoxy)-phenyl-, 2,4-Dichloro-phenyl-, 2,4-Dimethyl-1,3-thiazole-5-yl-, Methyl-methane-sulfonyl-, 2,5-Bis-(2,2,2-Trifluoroethoxy)-phenyl-, 2-Chloro-4-(trifluoromethylrphenyl-, 2-Chloro-4-fluoro-phenyl-, 5-Fluoro-2-methyl-phenyl-, 5-Chloro-2-methoxy-phenyl-, 2,4,6-Trichloro-phenyl-, 2-Hydroxy-benzoic acid-5-yl-, 5-. (Di-n-propylamino)-naphth-1-yl-, 6-Methoxy-m-tolyl-, 2,5-Difluoro-phenyl-, 2,4-Dimethoxy-phenyl-, 2,5-Dibromo-phenyl-, 3,4-Dibromo-phenyl-, 2,2,5,7,8-Pentamethyl-chroman-6-yl-, 2-Methoxy-benzoic-acid-5-yl-, 5-Chloro-4-nitrothiophene-2-yl-, 2,1,3-Benzothiadiazole-4-yl-, 1-Methyl-imidazole-4-yl-, Benzofurazan-4-yl-, 2-(Methoxycarbonyl)-thiophene-3-yl-, 5-(Isoxazol-3-yl)-thiophene-2-yl-, 2,4,5-Trifluoro-phenyl-, Biphenyl-4-yl-, Vinyl-phenyl-4-yl-, 2-Nitro-benzyl-, 5-Dichloro-methyl-furan-2-yl-; 5-Bromo-thiophene-2-yl-, 5-(4-Chlorobenzamidomethyl)-thiophene-2-yl-, 2,6-Difluoro-phenyl-, 2,5-Dimethoxy-4-nitro-phenyl-, Dibenzo[b,d]-furan-2-yl-, 2,3,4-Trifluoro-phenyl-, 3-Nitro-p-tolyl-, 4-Methoxy-2-nitro-phenyl-, 3,4-Difluoro-phenyl-, 4-(Bromoethyl)-phenyl-, 3,5-Dichloro-4-hydroxy-phenyl-, 4-n-Amyl-phenyl-, 5-Chloro-3-methylbenzo[b]-thiophene-2-yl-, 3-Methoxy-4-(methoxycarbonyl)-thiophene-2-yl-, 4-n-Butyl-phenyl-, 2-Chloro-4-cyano-phenyl-, 5-[2-(Methylthio)-pyrimidin-4-yl-]-thiophene-2-yl-, 3,5-Dinitro-4-methoxy-phenyl-, 4-Bromo-2-(trifluoromethoxy)-phenyl-, 4-Chloro-2,1,3-Benzoxadiazole-7-yl-, 2-(1-Naphthyl)-ethyl-, 3-Cyano-phenyl-, 5-Chloro-2,1,3-Benzoxadiazole-4-yl-, 3-Chloro-4-methyl-phenyl-, 4-Bromo-2-ethyl-phenyl-, 2,4-Dichloro-6-methyl-phenyl-, 6-Chloro-imidazo(2,1-B)-thiazole-5-yl-, 3-Methyl-benzo[b]-thiophene-2-yl-, 4-Methylsulphonyl-phenyl-, 2-Methyl-sulphonyl-phenyl-, 4-Bromo-2-methyl-phenyl-, 2,6-Dichloro-4-(trifluoromethylrphenyl-, 4-[[3-Chloro-5-(trifluoromethyl)-2 -pyridinyl]oxy]-phenyl-, 5-Chloro-naphth-1-yl-, 5-Chloro-naphth-2-yl-, 9,10-Dibromoanthracene-2-yl-, Isoquinoline-5-yl-, 4-Methoxy-2,3,6-trimethyl-phenyl-, 4'-Nitro-biphenyl-4-yl-, [(4-Phenoxy)-phenyl-, (1,3-Dihydro-1-oxo-2H-isoindol-2-yl-)-4-phenyl-, 4-Acetyl-phenyl-, 5-(2-Methyl-1,3-thiazole-4-yl)-thiophene-2-yl-, 5-(1-Methyl-3-(trifluoromethyl)pyrazol-5-yl-]-thiophene-2-yl-, 5-[5- Trifluoromethyl)-isoxazol-3-yl]-thio phene-2-yl-, 2-lodo-phenyl-, p-Dodecyl-phenyl-, 4-[(3-Cyano-4-methoxy-2-pyridinyl)oxy]-phenyl-, 4-(N-phthalimidinyl)-phenyl-, 1,2,3,4-Tetrahydro-2-(trifluoroacetyl)-isoquinoline-7-yl-, 4-Bromo-2-fluoro-phenyl-, 2-Fluoro-5-(trifluoromethyl)-phenyl-, 4-Fluoro-2-(trifluoromethyl)-phenyl-, 4-Fluoro-3-(trifluoromethyl)-phenyl-, 2,4,6-Trifluoro-phenyl-, 3-(Trifluoromethoxy)-phenyl-, 1,2-Dimethylimidazole-4-yl-, Ethyl-4-Carboxylate-3-yl-, 2,2,4,6,7-Pentamethyldihydrobenzofuran-5-yl-, 3-Bromo-2-chloropyridine-5-yl-, 3-Methoxy-phenyl-, 2-Methoxy-4-methyl-phenyl-, 2-Chloro-4-fluorobenzoic-acid-5-yl-, 4-Chloro-naphth-1-yl-, 2,5-Dichloro-4-nitro-thiophene-3-yl-, 4-(4-Methoxy-phenoxy)-phenyl-, 4-(4-Chloro-phenoxy)-phenyl-, 4-(3,5-Dichloro-phenoxy)-phenyl-, 4-(3,4-Dichloro-phenoxy)-phenyl-, 4-(4-Fluoro-phenoxy)-phenyl-, 4-(4-Methylphenoxy)-phenyl-, 4-[4-(Trifluormethyl)-phenoxy-phenyl-, 4-[3,5-Bis-(trifluoromethyl)-phenoxy]-phenyl-, 3-(2-Methoxy-phenoxy)-phenyl-, [3-(2-Chloro-phenoxy)-phenyl-, 3-(2-Methyl-phenoxy)-phenyl-, 4-[2-(Trifluoromethyl)-phenoxy]-phenyl-, 3-Phenyl-phenyl-, 3-(4-Methoxy-phenyl)-phenyl-, 3-(4-Chloro-phenyl)-phenyl-, 3-(3,5-Dichlorophenyl)-phenyl-, 3-(3,4-Dichloro-phenyl)-phenyl-, 3-(4-Fluorophenyl)-phenyl-, 3-(4-Methylphenyl)-phenyl-, 3-[4-(Trifluoromethyl)-phenyl]-phenyl-, 3-[3,5-Bis-(Trifluoromethyl)-phenyl]-phenyl-, 4-(4-Pyridyloxy)-phenyl)-, 4-(2-Methoxy-phenoxy)-phenyl-, 4-(2-Chloro-phenoxy)-phenyl-, 4-(2-Methyl-phenoxy)-phenyl-, 4-(4-Methoxyphenoxy)-phenyl-, 4-(4-Chlorophenyl)-phenyl-, 4-(3,5-Dichlorophenyl)-phenyl-, 4-(3,4-Dichlorophenyl)-phenyl-, 4-(4-Fluorophenyl)-phenyl-, 4-(4-Methylphenyl)-phenyl-, 4-[4-(Trifluormethyl)-phenyl]-phenyl-, 4-[3,5-Bis-(Trifluoromethyl)-phenyl]-phenyl-, [3-(Trifluoromethyl)-phenyl]-methyl-, (4-Chlorophenyl)-methyl-, (3,5-Dichlorophenyl)-methyl-, (3,5-Dichlorophenyl)-methyl-, (4-Fluorophenyl)-methyl-, 4-Methylphenylmethyl-, [4-(Trifluoromethyl)-phenyl]-methyl-, Cyclopropyl-, 2-(2-Chlorophenyl)-2-Phenylethyl-, 2-(2-Trifluoromethylphenyl)-2-phenylethyl-, 5-[4-Cyano-1-methyl-5-(methylthio)-1H-pyrazol-3-yl-thiophene-2-yl-, 3-Cyano-2,4-bis-(2,2,2-Trifluorothoxy)-phenyl-, 4-[(2-Chloro-1,3-Thiazol-5-yl)-methoxy]-phenyl-, 3-Nitro-phenylmethyl-, 4-Formylphenyl-, 2-(1,3-Dioxo-1,3-dihydro-isoindol-2-yl)-ethyl-, [3,5-Bis-(Trifluoromethyl)-phenyl]-methyl-, (4-(2-Pyridyloxy)-phenyl)-, (4-(3-Pyridyloxyrphenyl)-, 5-lodo-naphth-1-yl-, Ethyl-2,5-dimethyl-1-phenylpyrrole-4-carboxylate-3-yl-, Ethyl-2-methyl-1,5-diphenyl-1H-pyrrole-3-carboxylate-4-yl-, Ethyl-5-(4-chlorophenyl)-2-methyl-3-furoate-4-yl, Ethyl-5-(4-chlorophenyl)-2-methyl-1-phenyl-3-carboxylate-4-yl-, Ethyl-2,5-dimethyl-3-furoate-4-yl-, 3-Chloro-4-(1,3-dioxo-2-Azaspiro[4,4]non-2-yl)-phenyl-, 5-Bromo-2,4-difluoro-phenyl-, 5-Chloro-2,4-difluorophenyl-, Coumarin-6-yl, 2-Methoxy-phenyl, (3-Phenoxy)-phenyl-, 3-(4-Methoxy-phenoxy)-phenyl-, 3-(4-Chlorophenoxy)-phenyl-, 3-(3,5-Dichlorophenoxy)-phenyl-, 3-(3,4-Dichlorophenoxy)-phenyl-, 3-(4-Fluorophenoxy)-phenyl-, 3-(4-Methylphenoxy)-phenyl-, 3-[4-(Trifluoromethyl)-phenoxy]-phenyl-, 3-[3,5-(Trifluoromethyl)-phenoxy]-phenyl-, 3-[2-(Trifluoromethyl)-phenoxy]-phenyl-, 2,2-Diphenylethyl-, 4-Phenyl-5-(trifluoromethyl)-thiophene-3-yl-, Methyl-4-Phenyl-5-(Trifluoromethyl)-thiophene-2-carboxylate-3-yl-, Methyl-1,2,5-trimethylpyrrole-3-Carboxylate-4-yl-, 4-Fluoro-naphth-1-yl-, 3,5-Difluorophenyl-, 3-Fluoro-4-methoxy-phenyl-, 4-Chloro-2,5-difluorophenyl-, 2-Chloro-4,5-difluoro-phenyl-, 5-Fluoro-3-methylbenzo[b]-thiophene-2-yl-, Methyl-3-phenylpropionate-4-yl, Dihydrocinnamic Acid-4-yl-, Methyl-2,5-dimethyl-3-furoate-4-yl-, Methyl-2-furoate-5-yl-, Methyl-2-methyl-3-furoate-5-yl-, Methyl-1-methyl-1H-pyrrole-2-Carboxylate-5-yl-, 2-(5-Chloro-1,2,4-Thiadiazol-3-yl)-thiophene-5-yl-, 1,3,5-Trimethyl-1H-pyrazole-4-yl-, 3-Chloro-5-fluoro-2-methylphenyl-, Pentafluoroethoxytetrafluoroethyl-, 5-(5-Isoxazyl)-thiophene-2-yl-, 5-(5-Isoxazol-yl)-2-furyl-, 5-Methyl-2,1,3-benzothiadiazole-4-yl-, Biphenyl-2-yl-, 2,3-Dihydro-1,4-benzodioxine-6-yl-, 4-Methyl-Naphth-1-yl-; 5-Methyl-2-(Trifluormethyl)-3-Furyl-, 2,3-Dihydrobenzo[b]furan-5-yl-, 1-Benzothiophene-3-yl-, 4-Methyl-3,4-dihydro-2H-1,4-Benzoxazine-7-yl-, 5-Methyl-1-phenyl-1H-pyrazole-4-yl-, 6-Morpholino-3-Pyridinyl-, 4-(1H-Pyrazol-1-yl)-phenyl-, 6-Phenoxy-3-Pyridyl-, 3,4-Dihydro-2H-1,5-benzodioxepine-7-yl-, 5-(1,3-Oxazol-5-yl)-2-thienyl-, 4-(1,3-Oxazol-5-yl)-phenyl-, 5-Methyl-4-isoxazolyl, 2,1,3-Benzothiadiazole-5-yl-, 3-Thienyl-, 2-Methyl-benzyl-, 3-Chloro-benzyl-, 5-Acetamido-naphth-1-yl-, 3-Methyl-8-Quinolinyl-, 4-Chloro-2-nitrophenyl-, 6-Quinolinyl-, 1,3-Benzothiazole-6-yl-, 2-Morpholino-3-Pyridyl-, 2,5-Dimethyl-3-thienyl-, 5-[5-(Chloromethyl)-1,2,4-oxadiazol-3-yl]-2-thienyl-, Ethyl-3-[5-yl-2-thienyl-]1,2,4-oxadiazole-5-carboxylate-, 3-(5-Methyl-1,3,4-oxadiazol-2-yl)-phenyl-, 4-Isopropoxyphenyl-, 2,4-Dibromophenyl-, 3-Cyano-4-fluorophenyl-, 2,5-Bis-(Trifluoromethyl)-phenyl, 2-Bromo-4-fluorophenyl-, 4-Bromo-3-fluorophenyl-, 4-(Difluoromethoxy)-phenyl-, 3-(Difluoromethoxy)-phenyl-, 5-Chloro-2-fluoro-phenyl-, 3-Chloro-2-fluorophenyl-, 2-Fluoro-4-methylphenyl-, 4 Nitro-3-(trifluoromethyl)-phenyl-, 3-Fluoro-4-methylphenyl-, 4-Fluoro-2-methylphenyl-, 4-Bromo-3-(tifluoromethyl)-phenyl-, 4-Bromo-2-(trifluoromethyl)-phenyl-, 3-Bromo-5-(trifluoromethyl)-phenyl-, 2-Bromo-4-(trifluoromethyl)-phenyl-, 2-Bromo-5-(trifluoromethyl)-phenyl-, 2,4-Dichloro-5-fluorophenyl-, 4,5-Dichloro-2-fluorophenyl-, 3,4,5-Trifluorophenyl-, 4-Chloro-2-fluorophenyl-, 2-Bromo-4,6-Difluorophenyl-, 2-Ethylphenyl-, 4-Bromo-2-chlorophenyl-, 4-Bromo-2,6-dichlorophenyl-, 2-Bromo-4,6-dichloro-phenyl-, 4-Bromo-2,6-dimethylphenyl-, 3,5-Dimethylphenyl-, 4-Bromo-3-methylphenyl-, 2-Methoxy-4-nitrophenyl-, 2,2-Dimethyl-6-Chromanyl-, Ethyl-3,5-dimethyl-1H-pyrrole-2-carboxylate-4-yl-, Imidazo[1,2-A]pyridine-3-yl-, 3-(1,3-Oxazol-5-yl)-phenyl-, Ethyl-5-[4-yl)-phenyl]-2-methyl-3-furoate, Methyl-3-(yl)-4-methoxybenzoate, 1-Pyrrolidinylphenylsulfonyl-, Methyl-5-yl-4-methyl-2-thiophenecarboxylate, Methyl-3-yl-4-(isopropylsulfonyl)-2-thiophene, 2-Pyridyl-, 3-Fluoro-4-nitrophenyl-, 7-Chlorochromone-3-yl-, 4'-Bromobiphenyl-4-yl-, 4'-Acetyl-biphenyl-4-yl-, 4'-Bromo-2'-fluoro-biphenyl-4-yl-, 2-Chloro-4-(3-propyl-Ureido)-phenyl-, 3-(-Bromoacetyl)-phenyl-, 2-Bromo-3-(trifluoromethyl)-phenyl-, 1-Methyl-5-isatinyl-, 4-Isopropyl-benzoic-acid-3-yl-, 2-Chloro-3-thiophenecarboxylic-acid-5-yl-, 3-Pyridyl-, Cyclohexylmethyl-, 2-Methoxy-5-(N-phthalimidinyl)-phenyl-, 1-Benzothiophene-2-yl-, Morpholinophenylsulfonyl-, 3-(2-Methyl-4-pyrimidinyl)-phenyl-, and 2-Cyano-5-methylphenyl-, and R^{1a}-R^{18a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Furthermore, the use of compounds of general formula (Ia) is preferred, wherein R^{10a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably H, a linear or branched C₁₋₄-alkyl radical, a cyclohexyl radical or a phenyl radical,
more preferably H, -CH₃, -C₂H₅ or phenyl,
and R^{1a}-R^{9a}, R^{12a}-R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Moreover, the use of compounds of general formula (Ia) is preferred, wherein R^{11a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably H, a linear or branched C₁₋₄-alkyl radical, a cyclohexyl radical or a phenyl radical, more preferably H, -CH₃, -C₂H₅ or phenyl,
and R^{1a}-R^{10a}, R^{12a}-R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Preference is also given to the use of compounds of general formula (Ia), wherein R^{12a} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical,
more preferably H, -CH₃, -C₂H₅ or phenyl,
and R^{1a}-R^{11a}, R^{13a}-R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (Ia), wherein R^{13a} and R^{14a} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably are each independently selected from the group consisting of H, a linear or branched C₁₋₄-alkyl radical, a cyclohexyl radical and a phenyl radical,
more preferably are each independently selected from the group consisting of H, - CH₃, -C₂H₅ and phenyl,
and R^{1a}-R^{12a}, R^{15a}-R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Furthermore, the use of compounds of general formula (Ia) is preferred, wherein R^{13a} and R^{14a} together with the bridging nitrogen atom form a saturated, unsaturated or aromatic, 5- or 6-membered heterocyclic ring, which may be at least mono-substituted and/or contain at least one further heteroatom as a ring member,
preferably form an unsubstituted piperidin or morpholine group,
and R^{1a}-R^{12a}, R^{15a}-R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ia), wherein R^{15a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system,
preferably represents H, a linear or branched C₁₋₄-alkyl radical, a cyclohexyl radical or a phenyl radical,
more preferably represents H, -CH₃, -C₂H₅ or phenyl,
and R^{1a}-R^{14a}, R^{16a} - R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (Ia), wherein R^{16a} represents an unbranched or branched, saturated or unsaturated, optionally at least mono-substituted C₁₋₆ aliphatic radical,
preferably an unbranched or branched, saturated, unsubstituted C₁₋₃ alkyl radical,
more preferably a methyl radical,
and R^{1a}-R^{15a}, R^{17a}, R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (Ia), wherein R^{17a} represents an unbranched or branched, saturated or unsaturated, optionally at least mono-substituted C₁₋₆ aliphatic radical,
preferably an unbranched or branched, saturated, unsubstituted C₁₋₃ alkyl radical,
more preferably a methyl radical,
and R^{1a}-R^{16a}, R^{18a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ia), wherein R^{18a} represents a phenyl radical, which is optionally at least mono-substituted by a C₁₋₆ aliphatic radical, more preferably a phenyl radical, which is optionally at least mono-substituted by a methyl group and R^{1a}-R^{17a} and W^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Particularly preferred is the use of one or more benzoxazinone-derived sulfonamide compound of general-formula (Ia) selected from the list A given above.

More particularly preferred is the use of one or more benzoxazinone-derived sulfonamide compound of general formula (Ia) selected from the group consisting of
1-[1-(Naphthyl-1-sulfonyl)-piperidine-4-yl]-1 ,4-dihydro-benzo[d][1,3]oxazin-2-one,
1-(1-Phenylsulfonyl-piperidine-4-yl]-1,4-dihydro-benzo[d][1,3]-oxazin-2-one,
1 -[1 -(5-Chloro-3-methyl-benzo[b]thiophenyl-2-sulfonyl)-piperidine-4-yl]-1,4-dihydro-benzo[d][1,3]oxazin-2-one,
8-Methyl-1-[1-naphthyl-1-sulfonyl)-piperidine-4-yl]-1,4-dihydro-benzo[d][1,3]oxazin-2-one,
1-[1-(Quinoline-8-sulfonyl)-piperidin-4-yl]-1 ,4-dihydro-benzo[d][1,3]oxazin-2-one,
8-Methyl-1-[1-(quinoline-8-sulfonyl)-piperidin-4-yl]-1,4-dihydro-benzo[d][1,3]oxazin-2-one,
1-[1-(5-Dimethylamino-naphthyl-1-sulfonyl)-piperidine-4-yl]-8-methyl-1 ,4-dihydrobenzo[d][1,3]oxazin-2-one,
1-[1-(5-Dimethylamino-naphthyl-1-sulfonyl)-piperidine-4-yl]-1,4-dihydro-benzo[d][1,3]oxazin-2-one,
1-[1-(2,3-Dichloro-phenylsulfonyl)-piperidine-4-yl]-1 ,4-dihydro-benzo[d][1,3]oxazin-2-one,
1-[1-(2,3-Dichloro-phenylsulfonyl)-piperidin-4-yl]-8-methyl-1,4-dihydro-benzo[d][1,3]oxazin-2-one, and
corresponding salts thereof, or corresponding solvates thereof.

The compounds of general formula (Ia), their stereoisomers, corresponding salts and corresponding solvates may be obtained analogously by the methods described above for compounds of general formula (I).

In another aspect the present invention relates to benzoxazinone-derived sulfonamide compounds of general formula (Ib), 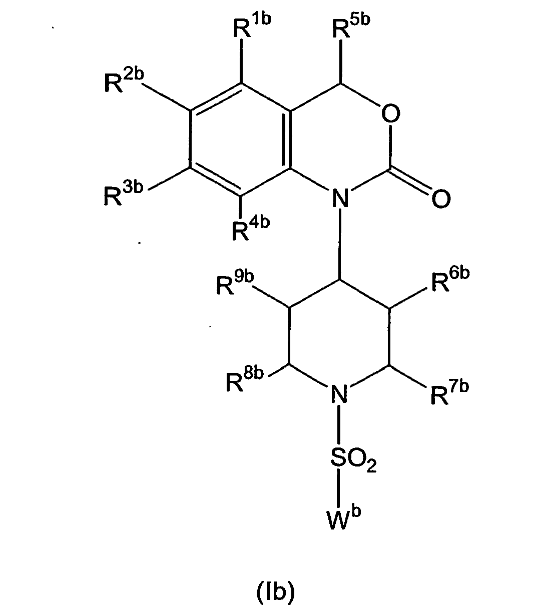 wherein
R^{1b}, R^{2b}, R^{3b}, R^{4b} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, - OR^{10b}, -OC(=O)R^{11b}, -(C=O)-OR^{11b}, -SR^{12b}, -SOR^{12b}, -SO₂R^{12b}, -NH-SO₂R^{12b}, -SO₂NH₂ and a -NR^{13b}R^{14b} moiety,
R^{5b} represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R^{6b}, R^{7b}, R^{8b}, R^{9b} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a -COOR^{15b} moiety,
W^{b} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted aromatic mono-or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ring-system, a NR^{16b}R^{17b}-moiety or a COR^{18b}-moiety,
R^{10b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{12b} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13b} and R^{14b} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{13b} and R^{14b} together with the bridging nitrogen atom form a saturated, unsaturated or aromatic heterocyclic ring, which may be at least mono-substituted and/or contain at least one further heteroatom as a ring member,
R^{15b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{16b} represents an unbranched or branched, saturated or unsaturated, optionally at least mono-substituted aliphatic radical,
R^{17b} represents an unbranched or branched, saturated or unsaturated, optionally at least mono-substituted aliphatic radical,
R^{18b} represents an optionally at least mono-subsituted aryl radical,
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

If one or more of the residues R^{1b}-R^{17b} and W^{b} represents an aliphatic radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl , wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and an unsubstituted phenyl radical. If any one of the above mentioned substitutents itself is at least mono-substituted, said substituents may preferably be selected from the group consisting of F, Cl, methyl and methoxy.

If one or more of the residues R^{1b}-R^{15b} represents or comprises a cycloaliphatic radical, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, phenoxy, benzoyl, cyclohexyl, branched or unbranched C₁₋₄-perfluoroalkyl, -NR^{A}R^{B} wherein R^{A}, R^{B} are each independently selected from the group consisting of H, a branched or unbranched C₁₋₄-alkyl-radical, -CH₂-CH₂-OH and phenyl, carboxy, keto, amido, cyano, nitro, - SO₂NH₂, -CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may in each case be branched or unbranched, unsubstituted or at least mono-substituted phenyl or naphthyl and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, methoxy, ethoxy, keto, benzoyl, phenoxy, cyclohexyl, -CF₃, -CO-CH₃, -CO-OCH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any one of the above mentioned substitutents itself is at least mono-substituted, said substituents may preferably be selected from the group consisting of F, Cl, methyl and methoxy.

If one or more of the residues R^{1b}-R^{4b} and R^{10b}-R^{15b} and W^{b} comprises an alkylene group, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, methoxy, ethoxy, CF₃ and unsubstituted phenyl. If any one of the above mentioned substitutents itself is at least mono-substituted, said substituents may preferably be selected from the group consisting of F, Cl, methyl and methoxy.

If one or more of the residues R^{1b} -R^{4b} and R^{10b}-R^{15b} comprises a mono- or polycyclic ringsystem, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, branched or unbranched C₁₋₄-perfluorocarbonyl, branched or unbranched C₁₋₄-perfluoroalkyl, amino, carboxy, amido, cyano, keto, nitro, -SO₂NH₂, -CO-C₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl, more preferably from the group consisting of hydroxy, F, Cl, Br, methyl, ethyl, methoxy, ethoxy, CF₃, -(C=O)-CF₃, keto, cyano and an unsubstituted phenyl radical. If any one of the above mentioned substitutents itself is at least mono-substituted, said substituents may preferably be selected from the group consisting of F, CI, methyl and methoxy. If one or more of the residues R^{1b}-R^{4b}, R^{10b}-R^{15b} and R^{18b} represents or comprises an aryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{A}R^{B} wherein R^{A}, R^{B} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, nitro, -CH(OH)(phenyl), methoxy, ethoxy, unsubstituted or at least mono-substituted benzoyl, unsubstituted or at least mono-substituted phenoxy, cyclohexyl, CF₃, OCF₃,-CO-CH₃, -CO-OCH₃, SO₂-CH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any of the above mentioned substitutents itself is at least mono-substituted, said substituents may preferably be selected from the group consisting of F, Cl, Br, CF₃, OCF₃, methyl and methoxy.

If one or more of the residues R^{1b}-R^{4b} and R^{10b}-R^{15b} represents or comprises a heteroaryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{A}R^{B} wherein R^{A}, R^{B} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, nitro, -CH(OH)(phenyl), methoxy, ethoxy, unsubstituted or at least mono-substituted benzoyl, unsubstituted or at least mono-substituted phenoxy, cyclohexyl, CF₃, OCF₃,-.CO-CH₃, -CO-OCH₃, SO₂-CH₃, -NR^{A}R^{B} wherein R^{A}, R^{B} are each independently selected from the group consisting of H, a branched or unbranched C₁₋₄-alkyl-radical, -CH₂-CH₂-OH and phenyl, and an unsubstituted phenyl radical. If any of the above mentioned substitutents itself is at least mono-substituted, said substituents may preferably be selected from the group consisting of F, Cl, Br, CF₃, OCF₃, methyl and methoxy.

If R^{13b} and R^{14b} form a heterocyclic ring, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, methyl, CF₃ and an unsubstituted phenyl radical. If any of the above mentioned substitutents itself is at least mono-substituted, said substituents may preferably be selected from the group consisting of F, Cl, methyl and methoxy.

If R^{13b} and R^{14b} form a heterocyclic ring, which contains one or more further heteroatoms as ring members, unless defined otherwise, each of these heteroatoms may preferably be selected from the group consisting of N, O and S, more preferably from the group consisting of N and O.

If one or more of the residues R^{1b}-R^{15b} and W^{b} represents or comprises a cycloaliphatic radical, which contains one or more heteroatoms as ring members, unless defined otherwise, each of these heteroatoms may preferably be selected from the group consisting of N, O, S and P, more preferably from the group consisting of N, O and S.

If one or more of the residues R^{1b}-R^{4b} , R^{10b}-R^{15b} and W^{b} represents or comprises an heteroaryl radical, which contains one or more heteroatoms as ring members, unless defined otherwise, each of these heteroatoms may preferably be selected from the group consisting of N, O, S and P, more preferably from the group consisting of N, O and S.

If W^{b} represents or comprises a cycloaliphatic radical, a heteroaryl radical, an aryl radical and/or a mono- or polycyclic ring system, which is substituted by one or more substituents, unless defined otherwise, each of these substituents may preferably be selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated 6₁₋₄ alkoxy, C₂₋₆-alkenyl, SO₂-C₁₋₄-alkyl, - (C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅-alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrroiidiny), morpholinyl, SO₂-morpholinyl-, thiadiazolyl, oxadiazolyl, oxazolyl, thiazolyl, isoxazolyl, O-CH₂-thiazolyl,-, NH-phenyl, and -C₁₋₄-Alkylen-NH-(C=O)-phenyl, more preferably from the group consisting of hydroxy, nitro, carboxy, cyano, keto, F, Cl, Br, I, C₁₋₁₂-alkyl, CH₂F, CHF₂, CF₃, CH₂Cl, CH₂Cl₂, CCl₃, CH₂Br, CHBr₂, CBr₃, OCF₃, OCHF₂, OCH₂F, O-CH₂-CF₃, vinyl, SO₂-CH₃, - (C=O)-CH₃, -(C=O)-C₂H₅, -(C=O)-O-CH₃, -(C=O)-O-C₂CH₅, -(C=O)-Cl, -S-CH₃-, - (C=O)-H, -NH-(C=O)-NH-CH₃, -(C=O)-CF₃, dimethylamino, diethylamino, di-n-propylamino, di-iso-propylamino, di-n-butylamino, di-tert-butyamino, NH-(C=O)-CH₃, - CH₂-(C=O)-CH₃, -CH₂-(C=O)-C₂H₅, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -CH₂-NH-(C=O)-phenyl.

If any of the afore mentioned substituents itself is substituted by one or more substituents, said substituents may preferably be selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, -C(=O)-O-C₁₋₅-alkyl, -C=O)-CH₂-F, -(C=O)-CH₂-Cl, -(C=O)-CH₂-Br, preferably from the group consisting of F, Cl, Br, CH₂F, CHF₂, CF₃, CH₂Cl, CHCl₂, CCl₃, CH₂Br, CHBr₂, CBr₃, nitro, cyano, hydroxy, -(C=O)-CH₃, CH₃, C₂H₅, -S-CH₃, -C(=O)-O-CH₃, -C(=O)-O-C₂H₅, -(C=O)-CH₂-F, -(C=O)-CH₂-Cl and -(C=O)-CH₂-Br.

Preferred are compounds of general formula (Ib) given above, wherein R^{1b}, R^{2b}, R^{3b}, R^{4b} are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano group, -OR^{10b}, -OC(=O)R^{11b}, -SR^{12b}, -SOR^{12b}, -SO₂R^{12b}, -NH-SO₂R^{12b}, -SO₂NH₂ and a -NR^{13b}R^{14b} moiety, preferably selected from the group consisting of H, F, Cl, Br, a saturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro group, a cyano group, -OR^{10b}, - OC(=O)R^{11b}, -SR^{12b} and -NR^{13b}R^{14b} moiety, more preferably selected from the group consisting of H, F, Cl, Br, -CH₃, -CH₂CH₃, -CF₃, -CF₂CF₃, cyclopentyl, cyclohexyl, nitro, cyano and -OR^{10b}, and R^{5b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{5b} represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₈-cycloaliphatic radical, preferably represents H or a branched or unbranched C₁₋₃-alkyl radical, more preferably H, -CH₃ or -CH₂CH₃, most preferably a hydrogen atom, and R^{1b}-R^{4b}, R^{6b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{6b}, R^{7b}, R^{8b}, R^{9b} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano group and a -COOR^{15b} moiety, preferably selected from the group consisting of H, a branched or unbranched C₁₋₃-alkyl radical, a cyano group and a COOR^{15b} group, more preferably from the group consisting of H, -CH₃, - CH₂CH₃ and a cyano moiety, most preferably each of R^{6b}, R^{7b}, R^{8b} and R^{9b} represent a hydrogen atom, and R^{1b}-R^{5b}, R^{10b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein W^{b} represents an unbranched or branched, saturated or unsaturated, optionally at least mono-substituted C₁₋₂₀ aliphatic radical, a saturated or unsaturated, optionally at least mono-substituted, optionally at least one heteroatom as ring member containing C₃₋₈ cycloaliphatic radical, which may be bonded via an optionally at least mono-substituted C₁₋₆-alkylene group and/or may be condensed with an optionally at least mono-substituted aromatic mono- or polycyclic ring-system, an optionally at least mono-substituted, 5- or 6-membered aryl- or heteroaryl radical, which may be bonded via an optionally at least mono-substituted C₁₋₆ alkylene group and/or may be condensed with an optionally at least mono-substituted mono- or polycyclic ring-system, a NR^{16b}R^{17b}-moiety or a COR^{18b}-moiety,
preferably W^{b} represents
a linear or branched C₁₋₂₀-alkyl radical, preferably an alkyl radical selected from the group consisting of methyl, ethyl, n-propyl, iso-propyl, n-butyl, iso-butyl, sec-butyl, tert-butyl and 1,1-dimethyl-propyl; a linear or branched C₂₋₂₀-alkenyl radical; preferably a vinyl radical; -CF₃; -CHF₂; -CH₂F; -CCl₃; -CHFl₂; -CH₂Cl; -CH₂-CF₃; - CH₂-CH₂-Cl; -CH₂-CH₂-CH₂-Cl; -CH₂-S(=O)₂-CH₃; a cyclopropyl radical; a cyclobutyl radical a cyclopentyl radical; a cyclohexyl radical; -CH₂-cyclopropyl; -CH₂-cyclobutyl; -CH₂-cyclopentyl; -CH₂-cyclohexyl; -N(CH₃)₂; -N(C₂H₅)₂; -N(n-CH₂-CH₂-CH₃)₂; phenyl; benzyl; naphthyl; -CH=CH-phenyl; -(CF₂)-(CF₂)-O-phenyl; -(CH₂)-naphtyl; - (CH₂)-(CH₂)-naphthyl; anthracenyl; -(C=4)-phenyl; thiophenyl; benzo[b]thiophenyl; furanyl; 2-oxo-2H-chromenyl; dibenzofuranyl; 2,3-dihydrobenzofuranyl; chromanyl; 2,3-dihydro-benzo[1,4]dioxinyl; 3,4-dihydro-2H-1,5-benzo-dioxepinyl; chromonyl; 1H-imidazolyl; pyridinyl; pyrrolidine-2,5-dionyl; pyrrolyl; 1H-pyrazolyl; 1H-pyrimidine-2,4-dionyl; quinolinyl; isoquinolinyl; 1H-Benzoimidazolyl; 1,4-dihydro-quinoxaline-2,3-dionyl; 1,2,3,4-tetrahydro-isoquinolinyl; 1,4-dihydro-benzo[b][1,4]diazepine-2,4-dionyl; 1,3-dihydro-1-oxo-2H-isoindolyi; phthalimidinyl; 2-(1,3-dioxo-1,3-dihydro-isoindol-2-yl)-ethyl; imidazo[1,2-a]pyridine; isatinyl; thiazolyl; 1,3-thiazolyl; 1,2,4-thiadiazolyl; imidazo[2,1-b]thiazolyl; 1,3-benzothiazolyl; benzo[1,2,5]thiadiazolyl; 2-oxo-2,3-dihydro-benzothiazolyl; 2,1,3-benzothiadiazolyl; imidazo[2,1-b]thiazolyl; isoxazolyl; benzo[1,2,5]oxadiazolyl; benzo[d]isoxazolyl; benzofurazanyl; 2-oxo-2,3-dihydro-benzooxazolyl; 3,4-dihydro-2H-benzo[1,4]oxazinyl; or 2,1,3-benzoxadiazolyl;
whereby each of these afore mentioned cyclic moieties may optionally be substituted with 1, 2, 3, 4 or 5 substituents independently selected from the group consisting of methyl; ethyl; n-propyl; iso-propyl; n-butyl; iso-butyl; sec-butyl; tert-butyl; 1,1-dimethyl-propyl; n-pentyl; vinyl; cyclopropyl; cyclobutyl; cyclopentyl; cyclohexyl; morpholino; methoxy; ethoxy; n-propoxy; iso-propoxy; n-propoxy; F; Cl; Br; I; -CN; - OH; -CF₃; -CF₂H; -CH₂F; -CCl₃; -CClH₂; -CHCl₂; -CH₂-F; -CH₂-Cl;-CH₂-Br; -(C=O)-CH₂-Br; -OCF₃; -O-CH₂-CF₃; -O-CHF₂; -NO₂; -NH₂; -N(CH₃)₂; -N(C₂H₅)₂; -N(n-CH₂-CH₂-CH₃)₂; -N(n-CH₂-CH₂-CH₂-CH₃)₂;-NH-(C=O)-CH₃; -NH-phenyl; -(C=O)-CF₃; - (C=O)-OH; =O (oxo); -(C=O)-H; -S(=O)₂-CH₃; -S(=O)₂-isopropyl; -S(=O)₂-phenyl; - S(=O)₂-pyrrolidinyl; -S(=O)₂-morpholino; -(CH₂)-(CH₂)-(C=O)-O-CH₃; -NH-(C=O)-NH-CH₂-CH₂-CH₃; -(C=O)-CH₃; -(C=O)-O-CH₃; -(C=O)-O-C₂H₅; -(CH₂)-NH-(C=O)-phenyl; -CH₂-C(H)(phenyl)(phenyl); -O-CH₂-thiazolyl; 1,3-dioxo-2-azaspiro[4.4]non-2-yl; phenyl; phenoxy; isoxazolyl; 1,3-oxazolyl; 1,2,4-oxadiazolyl; 1,3,4-oxadiazolyl; pyridinyl; pyridinyloxy; pyrazolyl; pyrimidinyl and phthalimidinyl; and
whereby each of the cyclic moieties of these afore mentioned substituents may optionally be substituted with 1, 2, 3, 4 or 5 substituents that are independently selected from the group consisting of methyl; ethyl; n-propyl; iso-propyl; F; Cl; Br; I; CN; -CH₂-F; -CH₂-Cl; -CH₂-Br; -CF₃ and -S-CH₃,
more preferably W^{b} represents
an alkyl radical selected from the group consisting of methyl; ethyl; n-propyl; iso-propyl; n-butyl; sec.butyl; iso-butyl and tert-butyl; vinyl (CH₂=CH-); -N(CH₃)₂; 1-naphthyl; benzyl; 2-naphtyl; phenyl; 2-methyl-phenyl; 3-methyl-phenyl; 4-methyl-phenyl; 2-ethyl-phenyl; 3-ethyl-phenyl; 4-ethyl-phenyl; 2-n-propyl-phenyl; 3-n-propyl-phenyl; 4-n-propyl-phenyl; 2-isopropyl-phenyl; 3-isopropyl-phenyl; 4-isopropyl-phenyl; 2-n-butyl-phenyl; 3-n-butyl-phenyl; 4-n-butyl-phenyl; 2-iso-butyl-phenyl; 3-iso-butyl-phenyl; 4-iso-butyl-phenyl; 2-tert-butyl-phenyl; 3-tert-butyl-phenyl; 4-tert-butyl-phenyl; 1,1-dimethylpropyl-phenyl; 2-cyclopentyl-phenyl; 3-cyclopentyl-phenyl; 4-cyclopentyl-phenyl 2-cyclohexyl-phenyl; 3-cyclohexyl-phenyl; 4-cyclohexyl-phenyl; 2-methoxy-phenyl; 3-methoxy-phenyl; 4-methoxy-phenyl; 2-ethoxy-phenyl; 3-ethoxy-phenyl; 4-ethoxy-phenyl; 2-n-propoxy-phenyl; 3-n-propoxy-phenyl; 4-n-propoxy-phenyl; 2-iso-propoxy-phenyl; 3-iso-propoxy-phenyl; 4-isopropoxy-phenyl;2-fluoro-phenyl; 3-fluoro-phenyl; 4-fluoro-phenyl; 2-chloro-phenyl; 3-chloro-phenyl; 4-chloro-phenyl; 2-bromo-phenyl; 3-bromo-phenyl; 4-bromo-phenyl; 2-trifluoromethyl-phenyl; 3-trifluoromethyl-phenyl; 4-trifluoromethyl-phenyl; 2-trifluoromethoxy-phenyl; 3-trifluoromethoxy-phenyl; 4-trifluoromethoxy-phenyl; 2-carboxy-phenyl; 3-carboxy-phenyl; 4-carboxy-phenyl; 2-acetyl-phenyl; 3-acetyl-phenyl; 4-acetyl-phenyl; 2-(C=O)-O-CH₃-phenyl; 3-(C=O)-O-CH₃₋phenyl; 4-(C=O)-O-CH₃-phenyl; 2-(CH2₎-(CH2)₋(C=O)-O-CH₃; 3-(CH₂)-(CH₂)-(C=O)-O-CH₃; 4-(CH₂)-(CH₂)-(C=O)-O-CH₃; 2-cyano-phenyl; 3-cyano-phenyl; 4-cyano-phenyl; 2-nitro-phenyl; 3-nitro-phenyl; 4-nitro-phenyl; 4-(4-bromophenoxy)-phenyl; 2-methylsulfonyl-phenyl; 3-methylsulfonyl-phenyl; 4-methylsulfonyl-phenyl; 2-phenyl-phenyl (biphenyl-2-yl); 3-phenyl-phenyl (biphenyl-3-yl); 4-phenyl-phenyl (biphenyl-4-yl); 2-phenoxy-phenyl; 3-phenoxy-phenyl; 4-phenoxy-phenyl; 2,4-dimethyl-phenyl; 3,4-dimethyl-phenyl; 2,4,6-trimethyl-phenyl; 2,3,5,6-tetramethyl-phenyl; pentamethyl-phenyl; 2,5-dimethoxy-phenyl; 3,4-dimethoxy-phenyl; 2,3-dichloro-phenyl; 2,4-dichloro-phenyl; 2,5-dichloro-phenyl; 3,4-dichloro-phenyl; 3,5-dichloro-phenyl; 2,6-dichloro-phenyl; 2,4-difluoro-phenyl; 3,4-difluoro-phenyl; 2,5-difluoro-phenyl; 2,6-difluoro-phenyl; 3-chloro-2-fluoro-phenyl; 3-chloro-4-fluoro-phenyl; 5-chloro-2-fluoro-phenyl; 2,3,4-trichloro-phenyl; 2,4,5-trichloro-phenyl; 2,4,6-trichloro-phenyl; 2,4,5-trifluoro-phenyl; 2,3,4-trifluoro-phenyl-; 2-chloro-4,5-difluorophenyl; 2-bromo-4-fluoro-phenyl; 2-bromo-4,6-difluoro-phenyl; 4-chloro-2,5-difluorophenyl; 5-chloro-2,4-difluoro-phenyl; 4-bromo-2,5-difluoro-phenyl; 5-bromo-2,4-difluoro-phenyl; pentafluoro-phenyl; 2,4-dinitro-phenyl; 4-chloro-3-nitro-phenyl; 2-methyl-5-nitro-phenyl; 5-bromo-2-methoxy-phenyl; 3-chloro-2-methyl-phenyl; 4-bromo-3-methyl-phenyl; 4-chloro-2,5-dimethyl-phenyl; 4-fluoro-3-methyl-phenyl; 5-fluoro-2-methyl-phenyl; 2-nitro-4-trifluoromethyl-phenyl; 2-methoxy-4-methyl-phenyl; 3,5-dichloro-2-hydroxy-phenyl; 3,5-dichloro-4-hydroxy-phenyl; 5-chloro-2,4-difluorophenyl; 3-chloro-4-(NH)-(C=O)-CH₃-phenyl; 2-chloro-6-methyl-phenyl; 2-chloro-5-trifluoromethyl-phenyl; 2-chloro-5-trifluoromethoxy-phenyl; 4-bromo-2-trifluoromethoxy-phenyl; 4-bromo-2-trifluoromethyl-phenyl; 4-bromo-3-trifluoromethyl-phenyl; 3-carboxy-4-fluoro-phenyl; 3-carboxy-4-chloro-6-fluoro-phenyl; 4-methoxy-2,3,6-trimethyl-phenyl-; or one of the following groups: 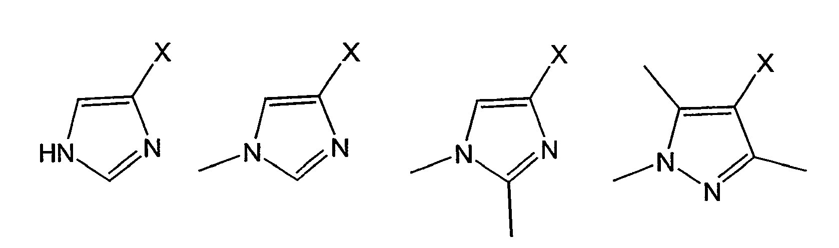 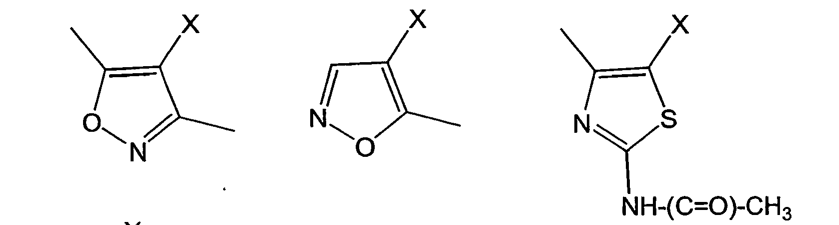 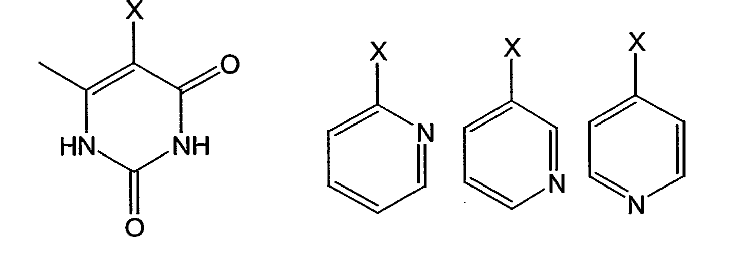 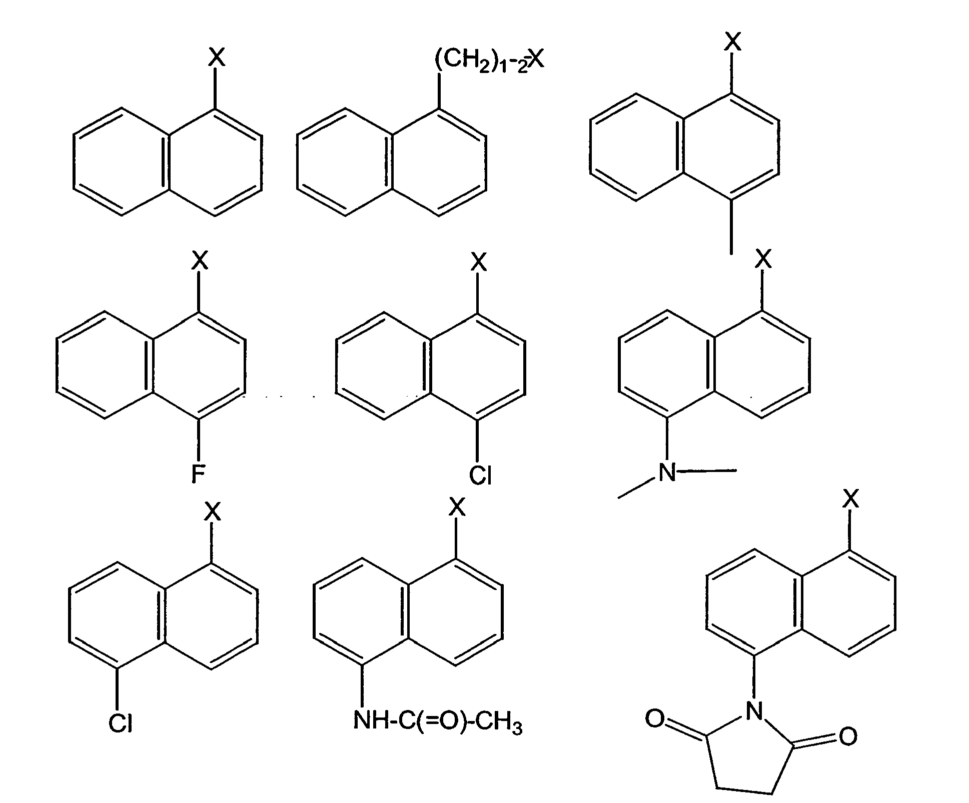 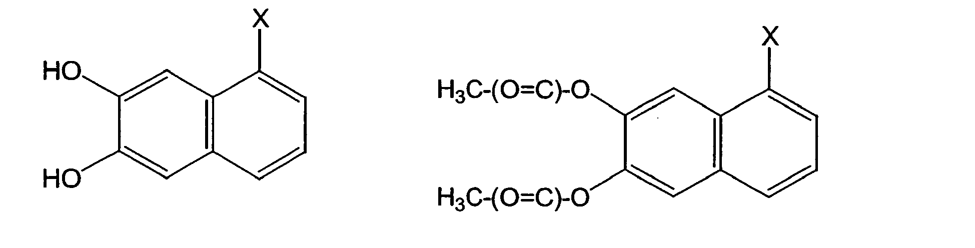 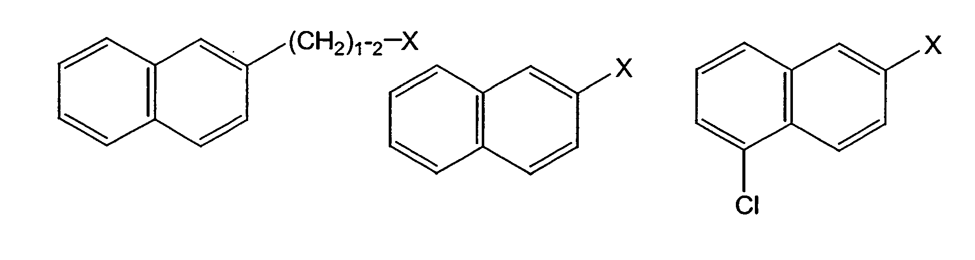 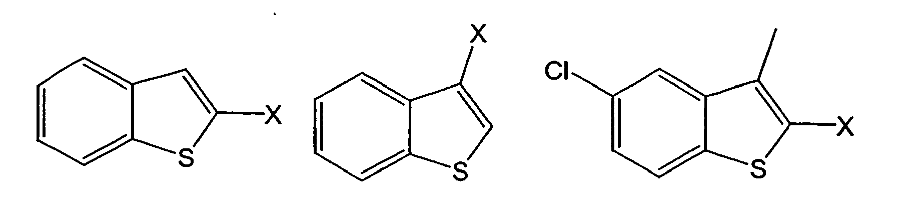 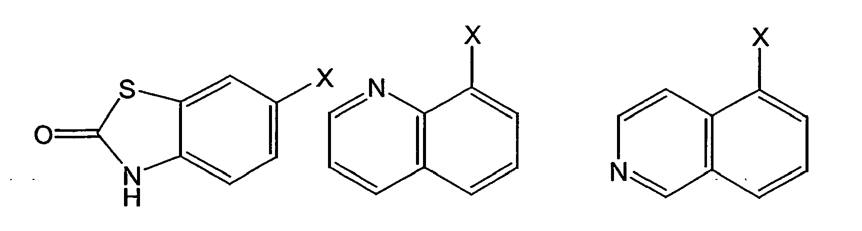 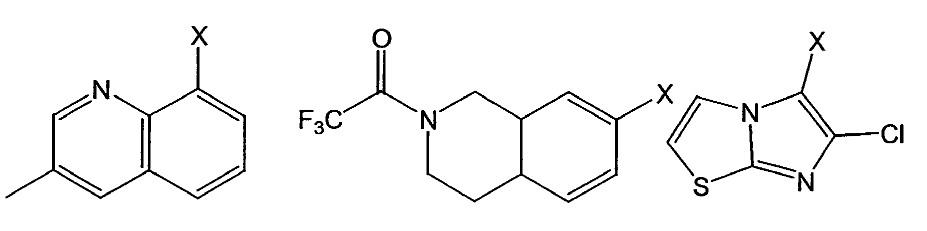 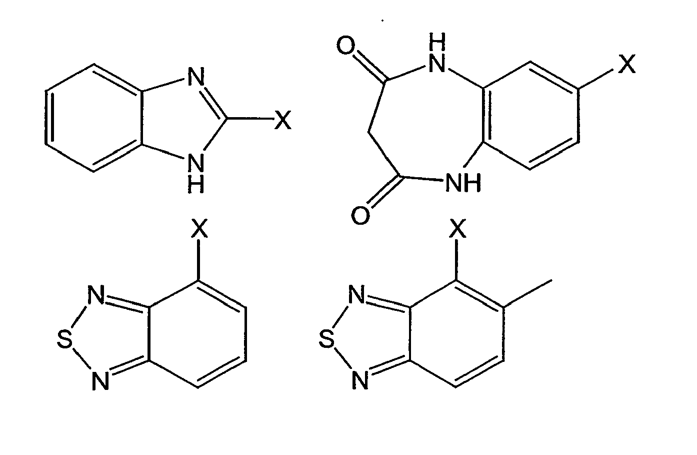 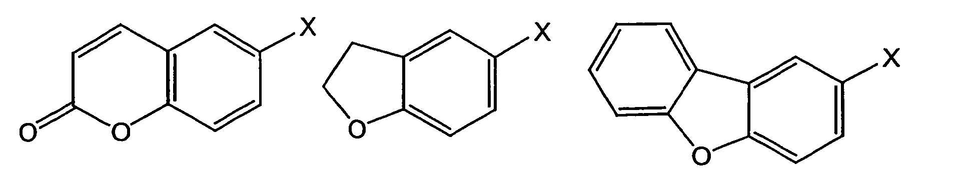 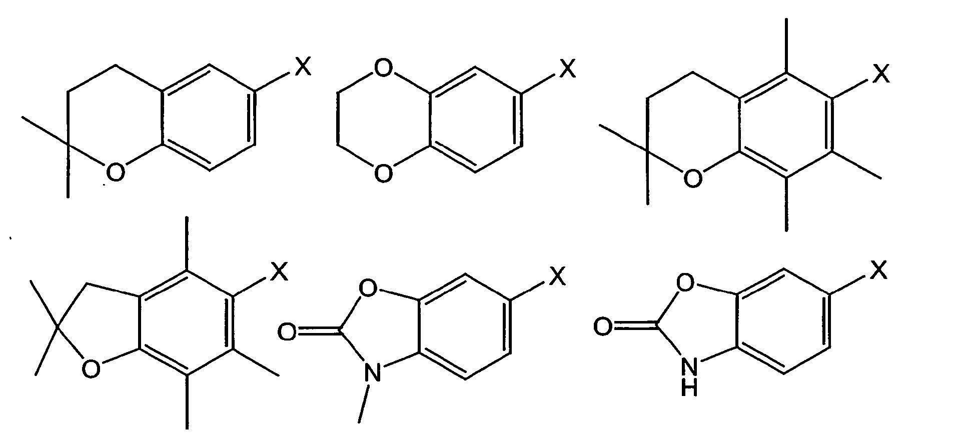 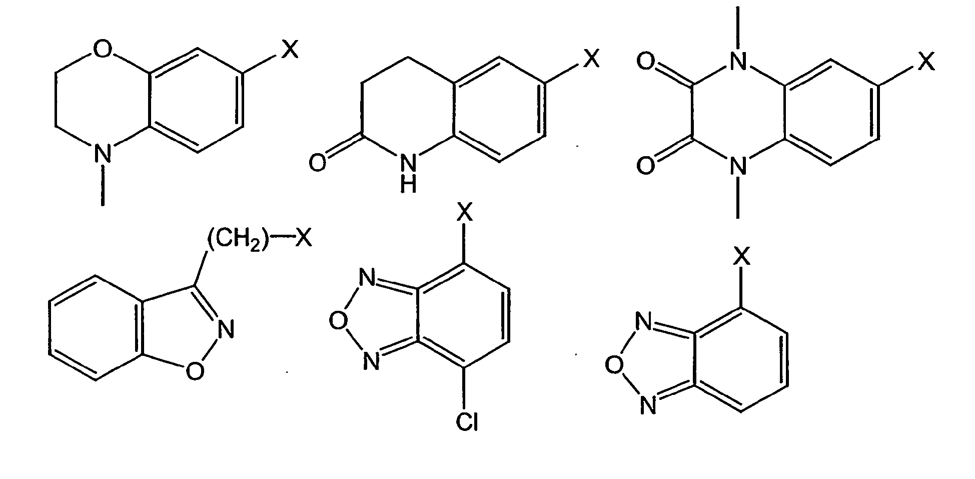 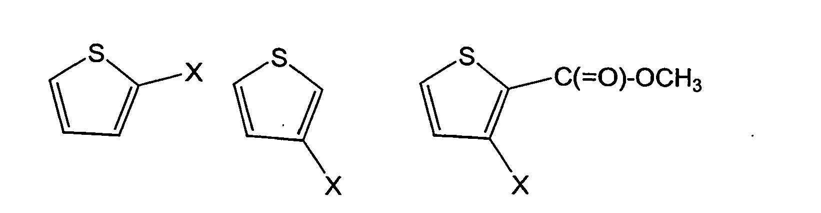 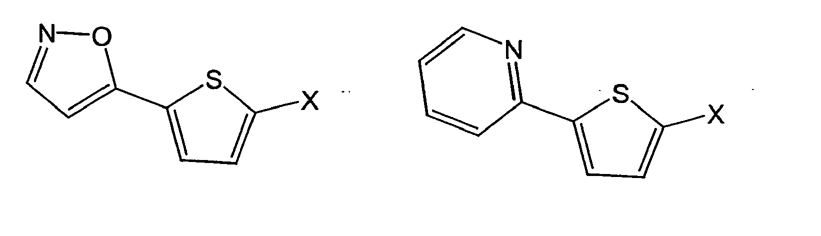 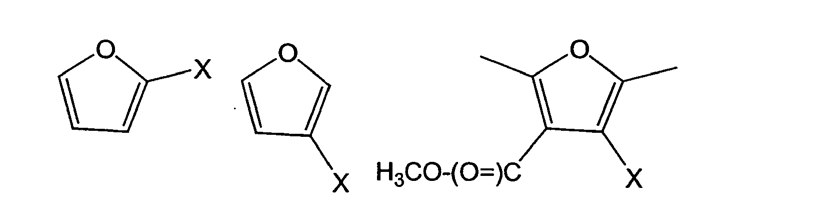 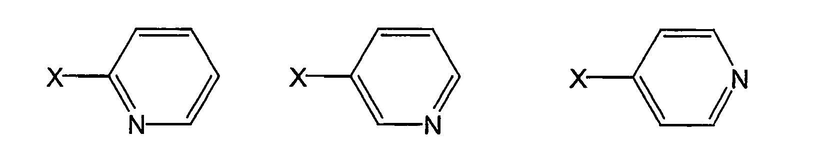 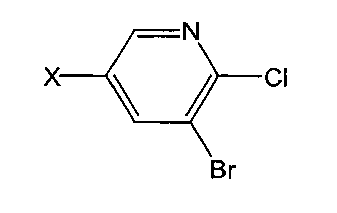 whereby in each case X denotes the position by which the respective substituent W^{b} is bonded to the -SO₂ group of formula (Ib),
and R^{1b}-R^{18b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{10b} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl, and R^{1b}-R^{9b}, R^{11b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{11b} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl, and R^{1b}-R^{10b}, R^{12b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{12b} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably H, -CH₃, -C₂H₅ or phenyl, and R^{1b}-R^{11b}, R^{13b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{13b} and R^{14b} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably are each independently selected from the group consisting of H, a linear or branched C₁-₄-alkyl radical, cyclohexyl and a phenyl radical, more preferably are each independently selected from the group consisting of H, -CH₃, -C₂H₅ and phenyl, and R^{1b}-R¹²b, R^{15b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{13b} and R^{14b} together with the bridging nitrogen atom form a saturated, unsaturated or aromatic, 5- or 6-membered heterocyclic ring, which may be at least mono-substituted and/or contain at least one further heteroatom as a ring member, preferably form an unsubstituted piperidin or morpholine group, and R^{1b}-R^{12b}, R^{15b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{15b} represents hydrogen, an unbranched or branched, saturated or unsaturated, optionally at least mono-substituted C₁-₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably represents H, -CH₃, -C₂H₅ or phenyl, and R^{1b}-R^{14b}, R^{16b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{16b} represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical, and R^{1b}-R^{15b}, R^{17b} and R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{17b} represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical, and R^{1b}-R^{16b}, R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (Ib) given above, wherein R^{18b} represents a phenyl radical, which is optionally at least mono-substituted by a C₁₋₆ aliphatic radical, more preferably a phenyl radical, which is optionally at least mono-substituted by a methyl group, and R^{1b}-R^{17b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

More preferred are compounds of general formula (Ib) given above, wherein
R^{1b}, R^{2b}, R^{3b}, R^{4b} are each independently selected from the group consisting of a hydrogen atom; a fluorine atom; a chlorine atom; a bromine atom; a methyl group and a methoxy group;
R^{5b} represents a hydrogen atom;
R^{6b}, R^{7b}, R^{8b}, R^{9b} each represent a hydrogen atom;
W^{b} represents
an alkyl radical selected from the group consisting of methyl; ethyl; n-propyl; iso-propyl; n-butyl; sec.butyl; iso-butyl and tert-butyl; vinyl (CH₂=CH-); -N(CH₃)₂; 1-naphthyl; benzyl; 2-naphtyl; phenyl; 2-methyl-phenyl; 3-methyl-phenyl; 4-methyl-phenyl; 2-ethyl-phenyl; 3-ethyl-phenyl; 4-ethyl-phenyl; 2-n-propyl-phenyl; 3-n-propyl-phenyl; 4-n-propyl-phenyl; 2-isopropyl-phenyl; 3-isopropyl-phenyl; 4-isopropyl-phenyl; 2-n-butyl-phenyl; 3-n-butyl-phenyl; 4-n-butyl-phenyl; 2-iso-butyl-phenyl; 3-iso-butyl-phenyl; 4-iso-butyl-phenyl; 2-tert-butyl-phenyl; 3-tert-butyl-phenyl; 4-tert-butyl-phenyl; 1,1-dimethylpropyl-phenyl; 2-cyclopentyl-phenyl; 3-cyclopentyl-phenyl; 4-cyclopentyl-phenyl 2-cyclohexyl-phenyl; 3-cyclohexyl-phenyl; 4-cyclohexyl-phenyl; 2-methoxy-phenyl; 3-methoxy-phenyl; 4-methoxy-phenyl; 2-ethoxy-phenyl; 3-ethoxy-phenyl; 4-ethoxy-phenyl; 2-n-propoxy-phenyl; 3-n-propoxy-phenyl; 4-n-propoxy-phenyl; 2-iso-propoxy-phenyl; 3-iso-propoxy-phenyl; 4-isopropoxy-phenyl;2-fluoro-phenyl; 3-fluoro-phenyl; 4-fluoro-phenyl; 2-chloro-phenyl; 3-chloro-phenyl; 4-chloro-phenyl; 2-bromo-phenyl; 3-bromo-phenyl; 4-bromo-phenyl; 2-trifluoromethyl-phenyl; 3-trifluoromethyl-phenyl; 4-trifluoromethyl-phenyl; 2-trifluoromethoxy-phenyl; 3-trifluoromethoxy-phenyl; 4-trifluoromethoxy-phenyl; 2-carboxy-phenyl; 3-carboxy-phenyl; 4-carboxy-phenyl; 2-acetyl-phenyl; 3-acetyl-phenyl; 4-acetyl-phenyl; 2-(C=O)-O-CH₃-phenyl; 3-(C=O)-O-CH₃-phenyl; 4-(C=O)-O-CH₃-phenyl; 2-(CH₂)-(CH₂)-(C=O)-O-CH₃; 3-(CH₂)-(CH₂)-(C=O)-O-CH₃; 4-(CH₂)-(CH₂)-(C=O)-O-CH₃; 2-cyano-phenyl; 3-cyano-phenyl; 4-cyano-pheny.l; 2-nitro-phenyl; 3-nitro-phenyl; 4-nitro-phenyl; 4-(4-bromophenoxy)-phenyl; 2-methylsulfonyl-phenyl; 3-methylsulfonyl-phenyl; 4-methylsulfonyl-phenyl; 2-phenyl-phenyl (biphenyl-2-yl); 3-phenyl-phenyl (biphenyl-3-yl); 4-phenyl-phenyl (biphenyl-4-yl); 2-phenoxy-phenyl; 3-phenoxy-phenyl; 4-phenoxy-phenyl; 2,4-dimethyl-phenyl; 3,4-dimethyl-phenyl; 2,4,6-trimethyl-phenyl; 2,3,5,6-tetramethyl-phenyl; pentamethyl-phenyl; 2,5-dimethoxy-phenyl; 3,4-dimethoxy-phenyl; 2,3-dichloro-phenyl; 2,4-dichloro-phenyl; 2,5-dichloro-phenyl; 3,4-dichloro-phenyl; 3,5-dichloro-phenyl; 2,6-dichloro-phenyl; 2,4-difluoro-phenyl; 3,4-difluoro-phenyl; 2,5-difluoro-phenyl; 2,6-difluoro-phenyl; 3-chloro-2-fluoro-phenyl; 3-chloro-4-fluoro-phenyl; 5-chloro-2-fluoro-phenyl; 2,3,4-trichloro-phenyl; 2,4,5-trichloro-phenyl; 2,4,6-trichloro-phenyl; 2,4,5-trifluoro-phenyl; 2,3,4-trifluoro-phenyl-; 2-chloro-4,5-difluorophenyl; 2-bromo-4-fluoro-phenyl; 2-bromo-4,6-difluoro-phenyl; 4-chloro-2,5-difluorophenyl; 5-chloro-2,4-difluoro-phenyl; 4-bromo-2,5-difluoro-phenyl; 5-bromo-2,4-difluoro-phenyl; pentafluoro-phenyl; 2,4-dinitro-phenyl; 4-chloro-3-nitro-phenyl; 2-methyl-5-nitro-phenyl; 5-bromo-2-methoxy-phenyl; 3-chloro-2-methyl-phenyl; 4-bromo-3-methyl-phenyl; 4-chloro-2,5-dimethyl-phenyl; 4-fluoro-3-methyl-phenyl; 5-fluoro-2-methyl-phenyl; 2-nitro-4-trifluoromethyl-phenyl; 2-methoxy-4-methyl-phenyl; 3,5-dichloro-2-hydroxy-phenyl; 3,5-dichloro-4-hydroxy-phenyl; 5-chloro-2,4-difluorophenyl; 3-chloro-4-(NH)-(C=O)-CH₃-phenyl; 2-chloro-6-methyl-phenyl; 2-chloro-5-trifluoromethyl-phenyl; 2-chloro-5-trifluoromethoxy-phenyl; 4-bromo-2-trifluoromethoxy-phenyl; 4-bromo-2-trifluoromethyl-phenyl; 4-bromo-3-trifluoromethyl-phenyl; 3-carboxy-4-fluoro-phenyl; 3-carboxy-4-chloro-6-fluoro-phenyl; 4-methoxy-2,3,6-trimethyl-phenyl-; or one of the following groups: 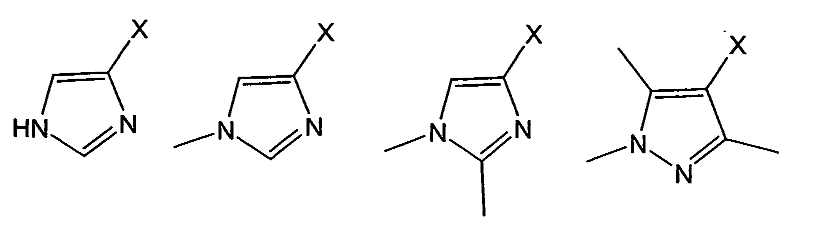 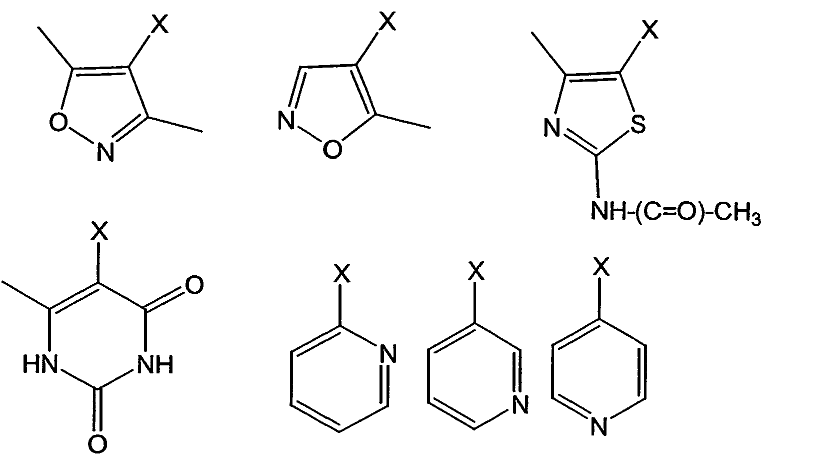 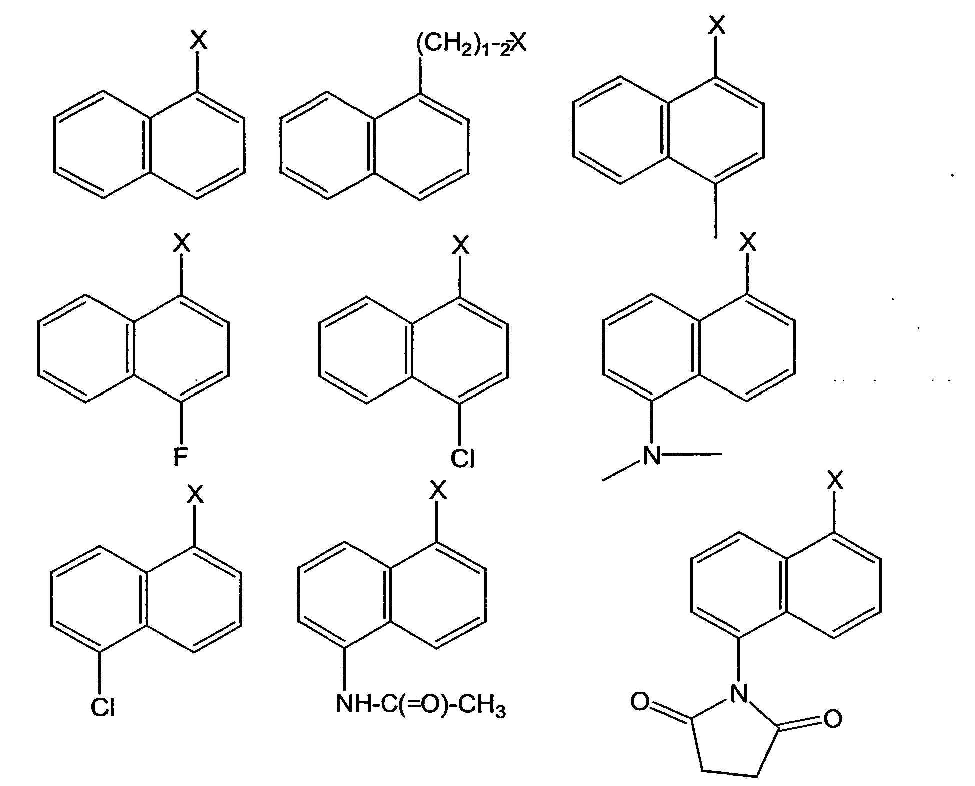 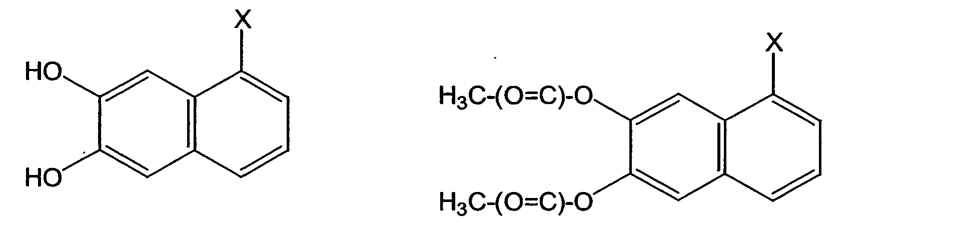 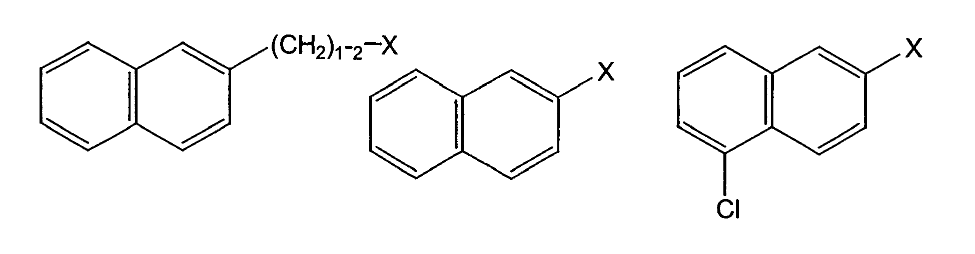 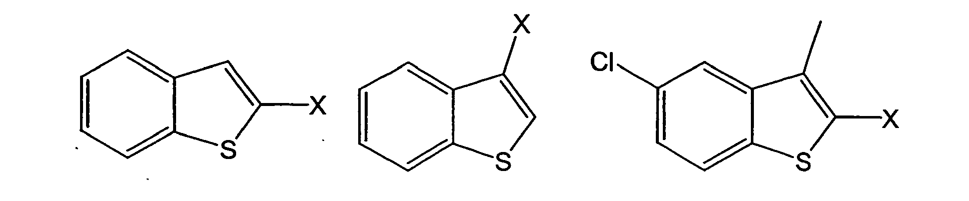 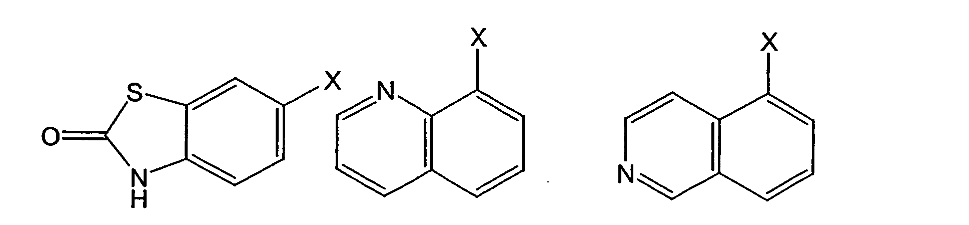 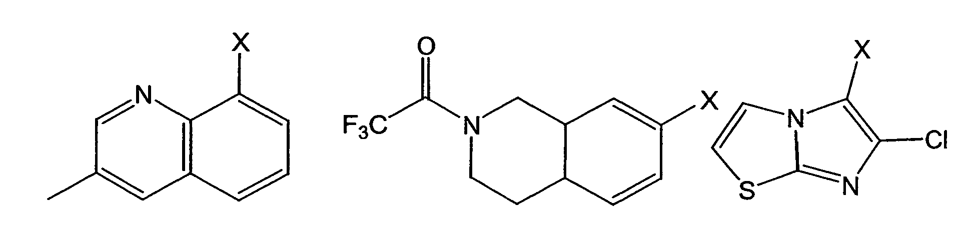 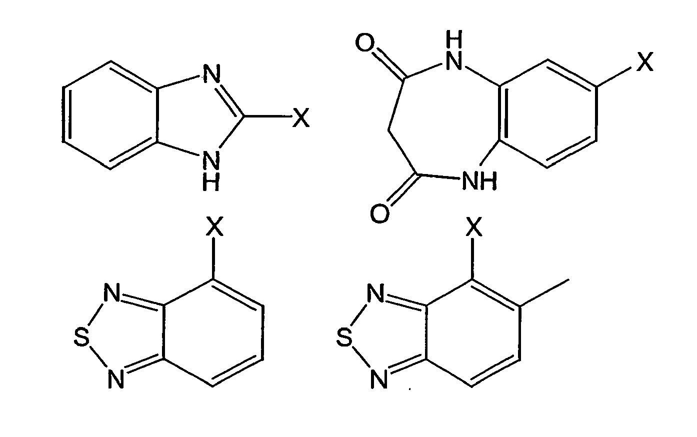 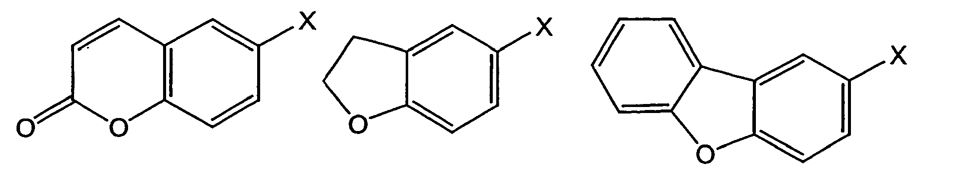 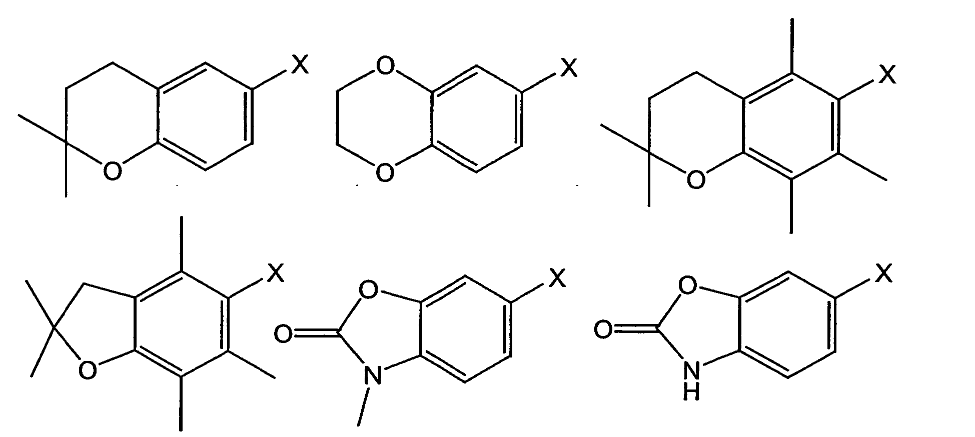 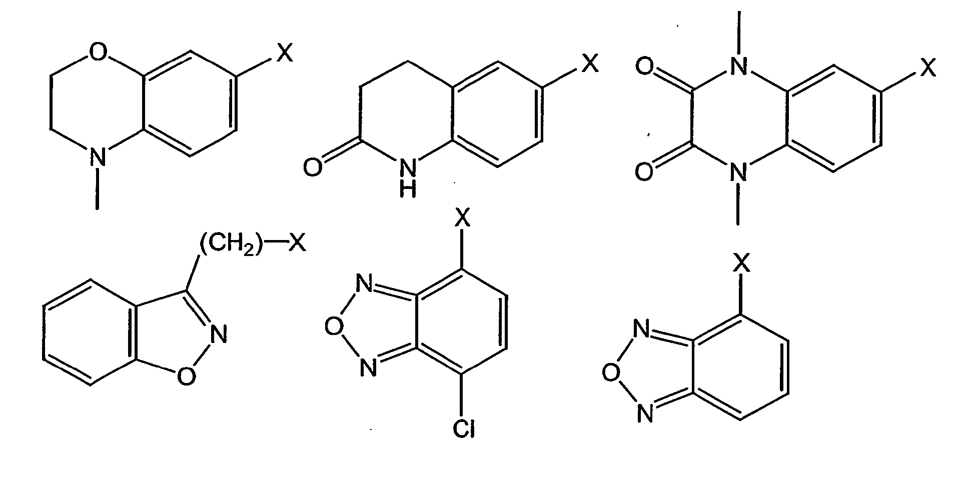 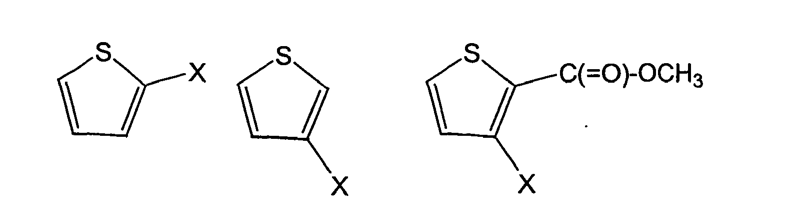 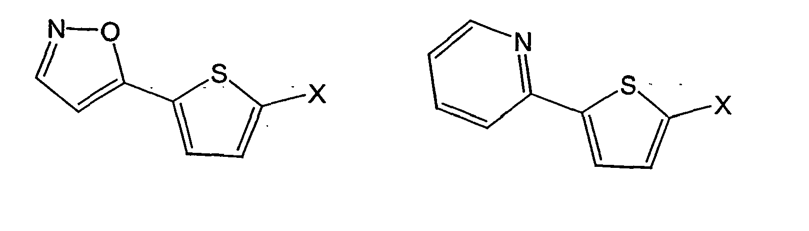 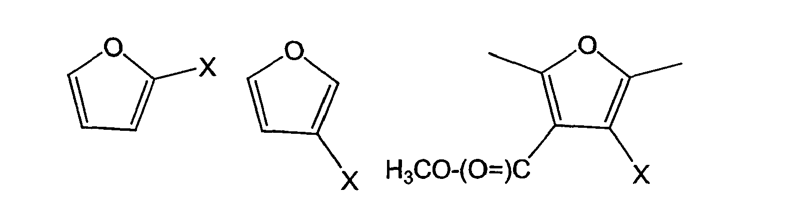 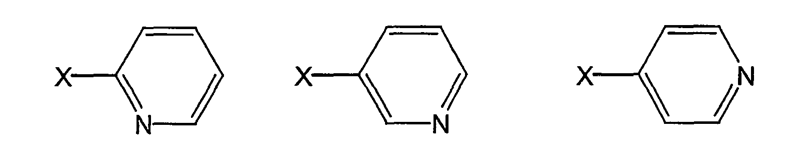 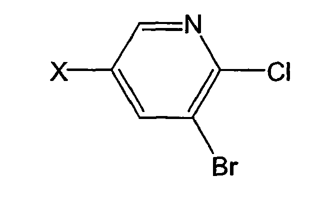 whereby in each case X denotes the position by which the respective substituent W^{b} is bonded to the -SO₂ group of formula (Ib).
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

Most particularly preferred are compounds of general formula (Ib) selected from the following group given in list B:
List B:

| **N°** | **Compound** |
|---|---|
| 1 | 1-[1-(Naphthalene-2-sulfonyl)-piperidin-4-yl]-1,4-dihydro-benzo[d][1,3]oxazin-2-one |
| 2 | 1-[1-(Toluene-4-sulfonyl)-piperidin-4-yl]-1,4-dihydro-benzo[d][1,3]oxazin-2-one |
| 3 | 1-(1-Phenylmethanesulfonyl-piperidin-4-yl)-1,4-dihydro-benzo[d][1,3]oxazin-2-one |
| 4 | 1-(1-Benzenesuffonyl-piperidin-4-yl)-6-chloro-1,4-dihydro-benzo[d][1,3]oxazin-2-one |
| 5 | 6-Chloro-1-[1-(toluene-4-sulfonyl)-piperidin-4-yl]-1,4-dihydro-benzo[d][1,3]oxazin-2-one |
| 6 | 6-Chloro-1-(1-phenylmethanesulfonyl-piperidin-4-yl)-1,4-dihydro-benzo[d][1,3]oxazin-2-one |
| 7 | 6-Chloro-1-[1-(naphthalene-1-sulfonyl)-piperidin-4-yl]-1,4-dihydro-benzo[d][1,3]oxazin-2-one |
| 8 | 6-Chloro-1-[1-(naphthalene-2-sulfonyl)-piperidin-4-yl]-1,4-dihydro-benzo[d][1,3]oxazin-2-one |
| 9 | 6-Chloro-1-[1-(5-chloro-3-methyl-benzo[b]thiophene-2-sulfonyl)-piperidin-4-yl]-1,4-dihydro-benzo[d][1,3]oxazin-2-one |
| 10 | 1-[1-(Thiophene-2-sulfonylfpiperidin-4-yl]-1,4-dihydro-benzo[d][1,3]oxazin-2-one |
| 11 | 1-[1-(4-Acetyl-benzenesulfonyl)-piperidin-4-yl]-1,4-dihydro-benzo[d][1,3]oxazin-2-one |
| 12 | 2-[4-(2-Oxo-4H-benzo[d][1,3]oxazin-1-yl)-piperidine-1-sulfonyl]-benzonitrile |
| 13 | 1-[1-(2,4-Dimethyl-benzenesulfonyl)-piperidin-4-yl]-1,4-dihydro-benzo[d][1,3]oxazin-2-one |
| 14 | 1-[1-(4-Methoxy-benzenesulfonyl)-piperidin-4-yl]-1,4-dihydro-benzo[d][1,3]oxazin-2-one |
| 15 | 1-[1-(2-Naphthalen-1-yl-ethanesulfonyl)-piperidin-4-yl]-1,4-dihydro-benzo[d][1,3]oxazin-2-one |
| 16 | 8-Methyl-1-[1-(thiophene-2-sulfonyl)-piperidin-4-yl]-1,4-dihydro-benzo[d][1,3]oxazin-2-one |
| 17 | 1-[1-(4-Acetyl-benzenesulfonyl)-piperidin-4-yl]-8-methyl-1,4-dihydro-benzo[d][1,3]oxazin-2-one |
| 18 | 2-[4-(8-Methyl-2-oxo-4H-benzo[d][1,3]oxazin-1-yl)-piperidine-1-sulfonyl]-benzonitrile |
| 19 | 1-[1-(2,4-Dimethyl-benzenesulfonyl)-piperidin-4-yl]-8-methyl-1,4-dihydro-benzo[d][1,3]oxazin-2-one |
| 20 | 1-[1-(4-Methoxy-benzenesulfonyl)-piperidin-4-yl]-8-methyl-1,4-dihydro-benzo[d][1,3]oxazin-2-one |
| 21 | 8-Methyl-1-[1-(2-naphthalen-1-yl-ethanesulfonyl)-piperidin-4-yl]-1,4-dihydro-benzo[d][1,3]oxazin-2-one |
| 22 | 4-(8-Methy)-2-oxo-4H-benzo[d][1,3]oxazin-1-yl)-piperidine-1-sulfonic acid dimethylamide |
| 23 | 2-[4-(2-Oxo-4H-benzo[d][1,3]oxazin-1-yl)-piperidine-1-sulfonyl]-benzoic acid methyl ester |
| 24 | 1-[1-(3-Tifluoromethyl-benzenesulfonyl)-piperidin-4-yl]-1,4-dihydro-benzo[d][1,3]oxazin-2-one |
| 25 | 2-[4-(8-Methyl-2-oxo-4H-benzo[d][1,3]oxazin-1-yl)-piperidine-1-sutfonyl]-benzoic acid methyl ester |
| 26 | 8-Methyl-1-[1-(3-trifluoromethyl-benzenesulfonyl)-piperidin-4-yl]-1,4-dihydro-benzo[d][1,3]oxazin-2-one |
| 27 | 1-[1-(4-Acetyl-benzenesulfonyl)-piperidin-4-yl]-6-chloro-1,4-dihydro-benzo[d][1,3]oxazin-2-one |
| 28 | 2-[4-(6-Chloro-2-oxo-4H-benzo[d][1,3]oxazin-1-yl)-piperidine-1-sulfonyl]-benzonitrile |
| 29 | 6-Chloro-1-[1-(4-methoxy-benzenesulfonyl)-piperidin-4-yl]-1,4-dihydro-benzo[d][1,3]oxazin-2-one |
| 30 | 2-[4-(6-Chloro-2-oxo-4H-benzo[d][1,3]oxazin-1-yl)-piperidine-1-sulfonyl]-benzoic acid methyl ester |
| 31 | 6-Chloro-1-[1-(2,4-dimethyl-benzenesulfonyl)-piperidin-4-yl]-1,4-dihydro-benzo[d][1,3]oxazin-2-one |
| 32 | 6-Chloro-1-[1-(3-trifluoromethyl-benzenesulfonyl)-piperidin-4-yl]-1,4-dihydro-benzo[d][1,3]oxazin-2-one |
| 33 | 1-[1-(5-Chloro-3-methyl-benzo[b]thiophene-2-sulfonyl)-piperidin-4-yl]-8-methyl-1,4-dihydro-benzo[d][1,3]oxazin-2-one |
| 34 | 1-{1-[4-(4-Bromo-phenoxy)-benzenesulfonyl]-piperidin-4-yl}-8-methyl-1,4-dihydro-benzo[d][1,3]oxazin-2-one |
| 35 | 1-[1-(4-Fluoro-benzenesulfonyl)-piperidin-4-yl]-8-methyl-1,4-dihydro-benzo[d][1,3]oxazin-2-one |
| 36 | 8-Methyl-1-[1-(naphthalene-2-sulfonyl)-piperidin-4-yl]-1,4-dihydro-benzo[d][1,3]oxazin-2-one |
| 37 | 8-Methyl-1-(1-phenylmethanesulfonyl-piperidin-4-yl)-1,4-dihydro-benzo[d][1,3]oxazin-2-one |
| 38 | 1-[1-(4-Bromo-benzenesulfonyl)-piperidin-4-yl]-6-chloro-1,4-dihydro-benzo[d][1,3]oxazin-2-one |
| 39 | 6-Chloro-1-[1-(4-methanesuffonyl-benzenesulfonyl)-piperidin-4-yl]-1,4-dihydro-benzo[d][1,3]oxazin-2-one |
| 40 | 1-[1-(Butane-1-sulfonyl)-piperidin-4-yl]-6-chloro-1,4-dihydro-benzo[d][1,3]oxazin-2-one |
| 41 | 1-[1-(4-Bromo-benzenesulfonyl)-piperidin-4-yl]-8-methyl-1,4-dihydro-benzo[d][1,3]oxazin-2-one |
| 42 | 1-[1-(4-Methanesulfonyl-benzenesulfonyl)-piperidin-4-yl]-8-methyl-1,4-dihydro-benzo[d][1,3]oxazin-2-one |
| 43 | 1-[1-(Butane-1-sulfonyl)-piperidin-4-yl]-8-methyl-1,4-dihydro-benzo[d][1,3]oxazin-2-one |
| 44 | 6-Chloro-1-[1-(2-nitro-benzenesulfonyl)-piperidin-4-yl]-1,4-dihydro-benzo[d][1,3]oxazin-2-one |
| 45 | 6-Chloro-1-[1-(3-nitro-benzenesulfonyl)-piperidin-4-yl]-1,4-dihydro-benzo[d][1,3]oxazin-2-one |
| 46 | 1-[1-(Biphenyl-4-sulfonyl)-piperidin-4-yl]-6-chloro-1,4-dihydro-benzo[d][1,3]oxazin-2-one |
| 47 | 8-Methyl-1-[1-(2-nitro-benzenesulfonyl)-piperidin-4-yl]-1,4-dihydro-benzo[d][1,3]oxazin-2-one |
| 48 | 8-Methyl-1-[1-(3-nitro-benzenesulfonyl)-piperidin-4-yl]-1,4-dihydro-benzo[d][1,3]oxazin-2-one |
| 49 | 1-[1-(Biphenyl-4-sulfonyl)-piperidin-4-yl]-8-methyl-1,4-dihydro-benzo[d][1,3]oxazin-2-one |
| 50 | 8-Methyl-1-[1-(4-nitro-benzenesulfonyl)-piperidin-4-yl]-1,4-dihydro-benzo[d][1,3]oxazin-2-one |
| 51 | 6-Chloro-1-[1-(4-nitro-benzenesulfonyl)-piperidin-4-yl]-1,4-dihydro-benzo[d][1,3]oxazin-2-one |
| 52 | 1-(1-Ethanesulfonyl-piperidin-4-yl)-1,4-dihydro-benzo[d][1,3]oxazin-2-one |
| 53 | 1-[1-(Propane-1-sulfonyl)-piperidin-4-yl]-1,4-dihydro-benzo[d][1,3]oxazin-2-one |
| 54 | 1-[1-(Propane-2-sulfonyl)-piperidin-4-yl]-1,4-dihydro-benzo[d][1,3]oxazin-2-one |
| 55 | 6-Chloro-1-(1-ethanesulfonyl-piperidin-4-yl)-1,4-dihydro-benzo[d][1,3]oxazin-2-one |
| 56 | 6-Chloro-1-[1-(propane-1-sulfonyl)-piperidin-4-yl]-1,4-dihydro-benzo[d][1,3]oxazin-2-one |
| 57 | 6-Chloro-1-[1-(propane-2-sulfonyl)-piperidin-4-yl]-1,4-dihydro-benzo[d][1,3]oxazin-2-one |
| 58 | 6-Chloro-1-[1-(quinoline-8-sulfonyl)-piperidin-4-yl]-1,4-dihydro-benzo[d][1,3]oxazin-2-one |
| 59 | 1-[1-(4-Nitro-benzenesulfonyl)-piperidin-4-yl]-1,4-dihydro-benzo[d][1,3]oxazin-2-one |
| 60 | 6-Methyl-1-[1-(quinoline-8-sulfonyl)-piperidin-4-yl]-1,4-dihydro-benzo[d][1,3]oxazin-2-one |
| 61 | 6-Methyl-1-[1-(2-naphthalen-1-yl-ethanesulfonyl)-piperidin-4-yl]-1,4-dihydro-benzo[d][1,3]oxazin-2-one |
| 62 | 6-Methyl-1-[1-(toluene-4-sulfonyl)-piperldin-4-yi]-1,4-dihydro-benzo[d][1,3]xazin-2-one |
| 63 | 1-[1-(4-Fluoro-benzenesulfonyl)-piperidin-4-yl]-6-methyl-1,4-dihydro-benzo[d][1,3]oxazin-2-one |
| 64 | 6-Methyl-1-[1-(naphthalene-1-sulfonyl)-piperidin-4-yl]-1,4-dihydro-benzo[d][1,3]oxazin-2-one |
| 65 | 6-Methyl-1-[1-(naphthalene-2-sulfonyl)-piperidin-4-yl]-1,4-dihydro-benzo[d][1,3]oxazin-2-one |
| 66 | 1-(1-(5-Chloro-3-methyl-benzo[b]thiophene-2-sulfonyl)-piperidin-4-yl]-6-methyl-1,4-dihydro-benzo[d][1,3]oxazin-2-one |
| 67 | 6-Methyl-1-[1-(4-nitro-benzenesulfonyl)-piperidin-4-yl]-1,4-dihydro-benzo[d][1,3]oxazin-2-one |
| 68 | 1-(1-Benzenesulfonyi-piperidin-4-yl)-6-methyl-1,4-dihydro-benzo[d][1,3]oxazin-2-one |
| 69 | 1-[1-(4-Chloro-3-nitro-benzenesulfonyl)-piperidin-4-yl]-6-methyl-1,4-dihydro-benzo[d][1,3]oxazin-2-one |
| 70 | 1-[1-(5-Dimethylamino-naphthalene-1-sulfonyl)-piperidin-4-yl]-6-methyl-1,4-dihydro-benzo[d][1,3]oxazin-2-one |
| 71 | 1-[1-(4-Chloro-3-nitro-benzenesulfonyl)-piperidin-4-yl]-8-methyl-1,4-dihydro-benzo[d][1,3]oxazin-2-one |
| 72 | 1-[1-(4-Chloro-3-nitro-benzenesulfonyl)-piperidin-4-yl]-1,4-dihydro-benzo[d][1,3]oxazin-2-one |
| 73 | 6-Chloro-1-[1-(4-chloro-3-nitro-benzenesulfonyl)-piperidin-4-yl]-1,4-dihydro-benzo[d][1,3]oxazin-2-one |
| 74 | 6-Chloro-1-[1-(5-dimethylamino-naphthalene-1-sulfonyl)-piperidin-4-yl]-1,4-dihydro-benzo[d][1,3]oxazin-2-one |
| | |
| 75 | 1-[1-(4-Methoxy-2,3,6-trimethyl-benzenesulfonyl)-piperidin-4-yl]-1,4-dihydro-benzo[d][1,3]oxazin-2-one |
| 76 | 1-[1-(4-Methoxy-2,3,6-trimethyl-benzenesulfonyl)-piperidin-4-yl]-8-methyl-1,4-dihydro-benzo[d][1,3]oxazin-2-one |
| 77 | 6-Chloro-1-[1-(4-methoxy-2,3,6-trimethyl-benzenesulfonyl)-piperidin-4-yl]-1,4-dihydro-benzo[d][1,3]oxazin-2-one |
| 78 | 1-[1-(4-Methoxy-2,3,6-trimethyl-benzenesulfonyl)-piperidin-4-yl]-6-methyl-1,4-dihydro-benzo[d][1,3]oxazin-2-one |
| 79 | 1-[1-(2-Bromo-benzenesulfonyl)-piperidin-4-yl]-1,4-dihydro-benzo[d][1,3]oxazin-2-one |
| 80 | 1-[1-(2-Bromo-benzenesulfonylfpiperidin-4-yl]-8-methyl-1,4-dihydro-benzo[d][1,3]oxazin-2-one |
| 81 | 1-[1-(2-Bromo-benzenesulfonyl)-piperidin-4-yl]-6-chloro-1,4-dihydro-benzo[d][1,3]oxazin-2-one |
| 82 | 1-[1-(2-Bromo-benzenesulfonyl)-piperidin-4-yl]-6-methyl-1,4-dihydro-benzo[d][1,3]oxazin-2-one |
| 83 | 6-Chloro-1-[1-(2,3-dichloro-benzenesulfonyl)-piperidin-4-yl]-1,4-dihydro-benzo[d][1,3]oxazin-2-one |
| 84 | 1-[1-(2,3-Dichloro-benzenesulfonyl)-piperidin-4-yl]-6-methyl-1,4-dihydro-benzo[d][1,3]oxazin-2-one |
| 85 | 1-[1-(2,4,5-Trichloro-benzenesulfonyl)-piperidin-4-yl]-1,4-dihydro-benzo[d][1,3]oxazin-2-one |
| 86 | 8-Methyl-1-[1-(2,4,5-trichloro-benzenesulfonyl)-piperidin-4-yl]-1,4-dihydro-benzo[d][1,3]oxazin-2-one |
| 87 | 6-Chloro-1-[1-(2,4,5-trichloro-benzenesulfonyl)-piperidin-4-yl]-1,4-dihydro-benzo[d][1,3]oxazin-2-one |
| 88 | 6-Methyl-1-[1-(2,4,5-trichloro-benzenesulfonyl)-piperidin-4-yl]-1,4-dihydro-benzo[d][1,3]oxazin-2-one |
| 89 | 1-[1-(5-Bromo-2-methoxy-benzenesulfonyl)-piperidin-4-yl]-1,4-dihydro-benzo[d][1,3]oxazin-2-one |
| 90 | 1-[1-(5-Bromo-2-methoxy-benzenesulfonyl)-piperidin-4-yl]-8-methyl-1,4-dihydro-benzo[d][1,3]oxazin-2-one |
| 91 | 1-[1-(5-Bromo-2-methoxy-benzenesulfonyl)-piperidin-4-yl]-6-chloro-1,4-dihydro-benzo[d][1,3]oxazin-2-one |
| 92 | 1-[1-(5-Bromo-2-methoxy-benzenesulfonyl)-piperidin-4-yl]-6-methyl-1,4-dihydro-benzo[d][1,3]oxazin-2-one |
| 93 | 1-[1-(2,5-Dimethoxy-benzenesulfonyl)-piperidin-4-yl]-1,4-dihydro-benzo[d][1,3]oxazin-2-one |
| 94 | 1-[1-(2,5-Dimethoxy-benzenesulfonyl)-piperidin-4-yl]-8-methyl-1,4-dihydro-benzo[d][1,3]oxazin-2-one |
| 95 | 6-Chloro-1-[1-(2,5-dimethoxy-benzenesulfonyl)-piperidin-4-yl]-1,4-dihydro-benzo[d][1,3]oxazin-2-one |
| 96 | 1-[1-(2,5-Dimethoxy-benzenesulfonyl)-piperidin-4-yl]-6-methyl-1,4-dihydro-benzo[d][1,3]oxazin-2-one |
| 97 | 1-(1-Pentamethylbenzenesulfonyl-piperidin-4-yl)-1,4-dihydro-benzo[d][1,3]oxazin-2-one |
| 98 | 8-Methyl-1-(1-pentamethylbenzenesulfonyl-piperidin-4-yl)-1,4-dihydro-benzo[d][1,3]oxazin-2-one |
| 99 | 6-Chloro-1-(1-pentamethylbenzenesulfony)-piperidin-4-yl)-1,4-dihydro-benzo[d][1,3]oxazin-2-one |
| 100 | 6-Methyl-1-(1-pentamethylbenzenesulfonyl-piperidin-4-yl)-1,4-dihydro-benzo[d][1,3]oxazin-2-one |
| 101 | 1-{1-[2-(2,2,2-Trifluoro-acetyl)-1,2,3,4-tetrahydro-isoquinoline-7-sulfonyl]-piperidin-4-yl}-1,4-dihydro-benzo[d][1,3]oxazin-2-one |
| 102 | 8-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 103 | 6-Chloro-1-{1-[2-(2,2,2-trifiuoro-acetyl)-1,2,3,4-tetrahydro-isoquinoline-7-sulfonyl]-piperidin-4-yl}-1,4-dihydro-benzo[d][1,3]oxazin-2-one |
| 104 | 6-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 105 | 1-[1-(2-Methyl-5-nitro-benzenesulfonyl)-piperidin-4-yl]-1,4-dihydro-benzo[d][1,3]oxazin-2-one |
| 106 | 8-Methyl-1-[1-(2-methyl-5-nitro-benzenesulfonyl)-piperidin-4-yl]-1,4-dihydro-benzo[d][1,3]oxazin-2-one |
| 107 | 6-Chloro-1-[1-(2-methyl-5-nitro-benzenesulfonyl)-piperidin-4-yl]-1,4-dihydro-benzo[d][1,3]oxazin-2-one |
| 108 | 6-Methyl-1-[1-(2-methyl-5-nitro-benzenesulfonyl)-piperidin-4-yl]-1,4-dihydro-benzo[d][1,3]oxazin-2-one |
| 109 | 1-[1-(4-Bromo-2,5-difluoro-benzenesulfonyl)-piperidin-4-yl]-1,4-dihydro-benzo[d][1,3]oxazin-2-one |
| 110 | 1-[1-(4-Bromo-2,5-difluoro-benzenesulfonyl)-piperidin-4-yl]-8-methyl-1,4-dihydro-benzo[d][1,3]oxazin-2-one |
| 111 | 1-[1-(4-Bromo-2,5-difluoro-benzenesulfonyl)-piperidin-4-yl]-6-chloro-1,4-dihydro-benzo[d][1,3]oxazin-2-one |
| 112 | 1-[1-(4-Bromo-2,5-difluoro-benzenesulfonyl)-piperidin-4-yl]-6-methyl-1,4-dihydro-benzo[d][1,3]oxazin-2-one |
| 113 | 1-[1-(4-Chloro-2,5-dimethyl-benzenesulfonyl)-piperidin-4-yl]-1,4-dihydro-benzo[d][1,3]oxazin-2-one |
| 114 | 1-[1-(4-Chloro-2,5-dimethyl-benzenesulfonyl)-piperidin-4-yl]-8-methyl-1,4-dihydro-benzo[d][1,3]oxazin-2-one |
| 115 | 6-Chloro-1-[1-(4-chloro-2,5-dimethyl-benzenesulfonyl)-piperidin-4-yl]-1,4-dihydro-benzo[d][1,3]oxazin-2-one |
| 116 | 1-[1-(4-Chloro-2,5-dimethyl-benzenesulfonyl)-piperidin-4-yl]-6-methyl-1,4-dihydro-benzo[d][1,3]oxazin-2-one |
| 117 | 1-[1-(4-Methoxy-benzenesulfonyl)-piperidin-4-yl]-6-methyl-1,4-dihydro-benzo[d][1,3]oxazin-2-one |
| 118 | 1-[1-{4-lsopropyl-benzenesulfonyl)-piperidin-4-yl]-1,4-dihydro-benzo[d][1,3]oxazin-2-one |
| 119 | 1-[1-(4-Isopropyl-benzenesulfonyl)-piperidin-4-yl]-8-methyl-1,4-dihydro-benzo[d][1,3]oxazin-2-one |
| 120 | 6-Chloro-1-[1-(4-isopropyl-benzenesulfonyl)-piperidin-4-yl]-1,4-dihydro-benzo[d][1,3]oxazin-2-one |
| 121 | 1-[1-(4-Isopropyl-benzenesulfonyl)-piperidin-4-yl]-6-methyl-1,4-dihydro-benzo[d][1,3]oxazin-2-one |
| 122 | 1-[1-(3-Chloro-4-fluoro-benzenesulfonyl)-piperidin-4-yl]-1,4-dihydro-benzo[d][1,3]oxazin-2-one |
| 123 | 1-[1-(3-Chloro-4-fluoro-benzenesulfonyl)-piperidin-4-yl]-8-methyl-1,4-dihydro-benzo[d][1,3]oxazin-2-one |
| 124 | 6-Chloro-1-[1-(3-chloro-4-fluoro-benzenesulfonyl)-piperidin-4-yl]-1,4-dihydro-benzo[d][1,3]oxazin-2-one |
| 125 | 1-[1-(3-Chloro-4-fluoro-benzenesulfonyl)-piperidin-4-yl]-6-methyl-1,4-dihydro-benzo[d][1,3]oxazin-2-one |
| 126 | 1-[1-(4-Bromo-benzenesulfonyl)-piperidin-4-yl]-6-methyl-1,4-dihydro-benzo[d][1,3]oxazin-2-one |
| 127 | 6-Methyl-1-[1-(3-nitro-benzenesulfonyl)-piperidin-4-yl]-1,4-dihydro-benzo[d][1,3]oxazin-2-one |
| 128 | 6-Methyl-1-[1-(3-trifluoromethyl-benzenesulfonyl)-piperidin-4-yl]-1,4-dihydro-benzo[d][1,3]oxazin-2-one |
| 129 | 1-[1-(4-Trifluoromethoxy-benzenesulfonyl)-piperidin-4-yl]-1,4-dihydro-benzo[d][1,3]oxazin-2-one |
| 130 | 1-[1-(2-Nitro-4-trifluoromethyl-benzenesulfonyl)-piperidin-4-yl]-1,4-dihydro-benzo[d][1,3]oxazin-2-one |
| 131 | 1-[1-(3-Fluoro-benzenesulfonyl)-piperidin-4-yl]-1,4-dihydro-benzo[d][1,3]oxazin-2-one |
| 132 | 1-[1-(2,4-Dichloro-benzenesulfonyl)-piperidin-4-yl]-1,4-dihydro-benzo[d][1,3]oxazin-2-one |
| 133 | 1-[1-(2,4,6-Trimethyl-benzenesulfonyl)-piperidin-4-yl]-1,4-dihydro-benzo[d][1,3]oxazin-2-one |
| 134 | 1-[1-(2-Trifluoromethyl-benzenesulfonyl)-piperidin-4-yl]-1,4-dihydro-benzo[d][1,3]oxazin-2-one |
| 135 | 8-Methyl-1-[1-(4-trifluoromethoxy-benzenesulfonyl)-piperidin-4-yl]-1,4-dihydro-benzo[d][1,3]oxazin-2-one |
| 136 | 8-Methyl-1-[1-(2-nitro-4-trifluoromethyl-benzenesulfonyl)-piperidin-4-yl]-1,4-dihydro-benzo[d][1,3]oxazin-2-one |
| 137 | 1-[1-(3-Fluoro-benzenesulfonyl)-piperidin-4-yl]-8-methyl-1,4-dihydro-benzo[d][1,3]oxazin-2-one |
| 138 | 1-[1-(2,4-Dichloro-benzenesulfonyl)-piperidin-4-yl]-8-methyl-1,4-dihydro-benzo[d][1,3]oxazin-2-one |
| 139 | 8-Methyl-1-[1-(2,4,6-trimethyl-benzenesulfonyl)-piperidin-4-yl]-1,4-dihydro-benzo[d][1,3]oxazin-2-one |
| 140 | 8-Methyl-1-[1-(2-trifluoromethyl-benzenesulfonyl)-piperidin-4-yl]-1,4-dihydro-benzo[d][1,3]oxazin-2-one |
| 141 | 1-[1-(4-Fluoro-benzenesulfonyl)-piperidin-4-yl]-1,4-dihydro-benzo[d][1,3]oxazin-2-one |
| 142 | 1-[1-(4-Bromo-benzenesulfonyl)-piperidin-4-yl]-1,4-dihydro-benzo[d][1,3]oxazin-2-one |
| 143 | 1-[1-(3-Nitro-benzenesutfonyl)-piperidin-4-yl]-1,4-dihydro-benzo[d][1,3]oxazin-2-one |
| 144 | 1-{1-[4-(4-Bromo-phenoxy)-benzenesulfonyl]-piperidin-4-yl}-1,4-dihydro-benzo[d][1,3]oxazin-2-one |
| 145 | 1-[1-(3-Methoxy-benzenesulfonyl)-piperidin-4-yl]-1,4-dihydro-benzo[d][1,3]oxazin-2-one |
| 146 | 1-[1-(2-Nitro-benzenesulfonyl)-piperidin-4-yl]-1,4-dihydro-benzo[d][1,3]oxazin-2-one |
| 147 | 8-Methyl-1-[1-(toluene-4-sulfonyl)-piperidin-4-yl]-1,4-dihydro-benzo[d][1,3]oxazin-2-one |
| 148 | 1-(1-Benzenesulfonyl-piperid in-4-yl)-8-methyl-1,4-dihydro-benzo[d][1,3]oxazin-2-one |
| 149 | 1-[1-(3-Methoxy-benzenesulfonyl)-piperidin-4-yl]-8-methyl-1,4-dihydro-benzo[d][1,3]oxazin-2-one |
| 150 | 1-[1-(2,4-Dimethyl-benzenesulfonyl)-piperidin-4-yl]-6-methyl-1,4-dihydro-benzo[d][1,3]oxazin-2-one |
| 151 | 1-{1-[4-(4-Bromo-phenoxy)-benzenesulfonyl]-piperidin-4-yl}-6-methyl-1,4-dihydro-benzo[d][1,3]oxazin-2-one |
| 152 | 6-Methyl-1-[1-(thiophene-2-sulfonyl)-piperidin-4-yl]-1,4-dihydro-benzo[d][1,3]oxazin-2-one |
| 153 | 1-[1-(Toluene-3-sulfonyl)-piperidin-4-yl]-1,4-dihydro-benzo[d][1,3]oxazin-2-one |
| 154 | 1-[1-(5-Fluoro-2-methyl-benzenesulfonyl)-piperidin-4-yl]-1,4-dihydro-benzo[d][1,3]oxazin-2-one |
| 155 | 1-[1-(4-lsopropoxy-benzenesulfonyl)-piperidin-4-yl]-1,4-dihydro-benzo[d][1,3]oxazin-2-one |
| 156 | 1-[1-(3-Chloro-benzenesulfonyl)-piperidin-4-yl]-1,4-dihydro-benzo[d][1,3]oxazin-2-one |
| 157 | 1-[1-(3,4-Dimethoxy-benzenesulfonyl)-piperidin-4-yl]-1,4-dihydro-benzo[d][1,3]oxazin-2-one |
| 158 | 1-(1-Pentafluorobenzenesulfonyl-piperidin-4-yl)-1,4-dihydro-benzo[d][1,3]oxazin-2-one |
| 159 | 8-Methyl-1-[1-(toluene-3-sulfonyl)-piperidin-4-yl]-1,4-dihydro-benzo[d][1,3]oxazin-2-one |
| 160 | 1-[1-(5-Fluoro-2-methyt-benzenesulfonyl)-piperidin-4-yl]-8-methyl-1,4-dihydrobenzo[d][1,3] oxazin-2-one |
| 161 | 1-[1-(4-Isopropoxy-benzenesulfonyl)-piperidin-4-yl]-8-methyl-1,4-dihydro-benzo[d][1,3] oxazin-2-one |
| 162 | 1-[1-(3-Chloro-benzenesulfonyl)-piperidin-4-yl]-8-methyl-1,4-dihydro-benzo[d][1,3]oxazin-2-one |
| 163 | 1-[1-(3,4-Dimethoxy-benzenesulfonyl)-pipendin-4-yl]-8-methyl-1,4-dihydro-benzo[d][1,3] oxazin-2-one |
| 164 | 8-Methyl-1-(1-pentafluorobenzenesulfonyl-piperidin-4-yl)-1,4-dihydro-benzo[d][1,3]oxazin-2-one |
| 165 | 6-Methyl-1-[1-(toluene-3-sulfonyl)-piperidin-4-yl]-1,4-dihydro-benzo[d][1,3]oxazin-2-one |
| 166 | 1-[1-(5-Fluoro-2-methyl-benzenesulfonyl)-piperidin-4-yl]-6-methyl-1,4-dihydro-benzo[d][1,3]oxazin-2-one |
| 167 | 1-[1-(4-Isopropoxy-benzenesulfonyl)-piperidin-4-yl]-6-methyl-1,4-dihydro-benzo[d][1,3]oxazin-2-one |
| 168 | 1-[1-(3-Chloro-benzenesulfonyl)-piperidin-4-yl]-6-methyl-1,4-dihydro-benzo[d][1,3]oxazin-2-one |
| 169 | 1-[1-(3,4-Dimethoxy-benzenesulfonyl)-piperidin-4-yl]-6-methyl-1,4-dihydro-benzo[d][1,3]oxazin-2-one |
| 170 | 6-Methyl-1-(1-pentafluorobenzenesulfonyl-piperidin-4-yl)-1,4-dihydro-benzo[d][1,3]oxazin-2-one |
| 171 | 6-Methyl-1-[1-(4-trifluoromethoxy-benzenesulfonyl)-piperidin-4-yl]-1,4-dihydro-benzo[d][1,3]oxazin-2-one |
| 172 | 6-Methyl-1-[1-(2-nitro-4-trifluoromethyl-benzenesulfonyl)-piperidin-4-yl]-1,4-dihydro-benzo[d][1,3]oxazin-2-one |
| 173 | 1-[1-(3-Fluoro-benzenesulfonyl)-piperidin-4-yl]-6-methyl-1,4-dihydro-benzo[d][1,3]oxazin-2-one |
| 174 | 1-[1-(2,4-Dichloro-benzenesulfonyl)-piperidin-4-yl]-6-methyl-1,4-dihydro-benzo[d][1,3]oxazin-2-one |
| 175 | 6-Methyl-1-[1-(2,4,6-trimethyl-benzenesulfonyl)-piperidin-4-yl]-1,4-dihydro-benzo[d][1,3]oxazin-2-one |
| 176 | 6-Methyl-1-[1-(2-trifluoromethyl-benzenesulfonyl)-piperidin-4-yi]-1,4-dihydro-benzo[d][1,3]oxazin-2-one |
| 177 | 1-[1-(3-Methoxy-benzenesulfonyl)-piperidin-4-yl]-6-methyl-1,4-dihydro-benzo[d][1,3]oxain-2-one |
| 178 | 6-Methyl-1-[1-(2-nitro-benzenesulfonyl)-piperidin-4-yl]-1,4-dihydro-benzo[d][1,3]oxazin-2-one |
| 179 | 1-[1-(4-Acetyl-benzenesulfonyl)-piperidin-4-yl]-6-methyl-1,4-dihydro-benzo[d][1,3]oxazin-2-one |
| 180 | 1-[1-(4-Methanesulfonyl-benzenesulfonyl)-piperidin-4-yl]-6-methyl-1,4-dihydro-benzo[d][1,3]oxazin-2-one |
| 181 | 6-Methyl-1-(1-phenylmethanesulfonyl-piperidin-4-yl)-1,4-dihydro-benzo[d][1,3]oxazin-2-one |
| 182 | 2-[4-(6-Methyl-2-oxo-4H-benzo[d][1,3]oxazin-1-yl)-piperidine-1-sulfonyl]benzoic acid methyl ester |
| 183 | 6-Methyl-1-[1-(2-oxo-2H-chromene-6-sulfonyl)-piperidin-4-yl]-1,4-dihydro-benzo[d][1,3]oxazin-2-one |
| 184 | 6-Chloro-1-[1-(4-fluoro-benzenesulfonyl)-piperidin-4-yl]-1,4-dihydro-benzo[d][1,3]oxazin-2-one |
| 185 | 6-Chloro-1-[1-(3,5-dichloro-benzenesulfonyl)-piperidin-4-yl]-1,4-dihydro-benzo[d][1,3]oxazin-2-one |
| 186 | 1-(1-[4-(4-Bromo-phenoxy)-benzenesulfonyl]-piperidin-4-yl)-6-chloro-1,4-dihydro-benzo[d][1,3]oxazin-2-one |
| 187 | 6-Chloro-1-[1-(thiophene-2-sulfonyl)-piperidin-4-yi]-1,4-dihydro-benzo[d][1,3]oxazin-2-one |
| 188 | 6-Chloro-1-[1-(3-methoxy-benzenesulfonyl)-piperidin-4-yl]-1,4-dihydro-benzo[d][1,3]oxazin-2-one |
| 189 | 6-Chloro-1-[1-(2-oxo-2H-chromene-6-sulfonyl)-piperidin-4-yl]-1,4-dihydro-benzo[d][1,3]oxazin-2-one |
| 190 | 6-Chloro-1-[1-(toluene-3-sulfonyl)-piperidin-4-yl]-1,4-dihydro-benzo[d][1,3]oxazin-2-one |
| 191 | 6-Chloro-1-[1-(5-fluoro-2-methyl-benzenesulfonyl)-piperidin-4-yl]-1,4-dihydro-benzo[d][1,3]oxazin-2-one |
| 192 | 6-Chloro-1-[1-(4-isopropoxy-benzenesulfonyl)-piperidin-4-yl]-1,4-dihydro-benzo[d][1,3] oxazin-2-one |
| 193 | 6-Chloro-1-[1-(3-chloro-benzenesulfonyl)-piperidin-4-yl]-1,4-dihydro-benzo[d][1,3]oxazin-2-one |
| 194 | 6-Chloro-1-[1-(3,4-dimethoxy-benzenesulfonyl)-piperidin-4-yl]-1,4-dihydro-benzo[d][1,3]oxazin-2-one |
| 195 | 6-Chloro-1-(1-pentafluorobenzenesulfonyl-piperidin-4-yl)-1,4-dihydro-benzo[d][1,3]oxazin-2-one |
| 196 | 6-Chloro-1-[1-(4-trifluoromethoxy-benzenesulfonyl)-piperidin-4-yl]-1,4-dihydro-benzo[d][1,3]oxazin-2-one |
| 197 | 6-Chloro-1-[1-(2-nitro-4-trifluoromethyl-benzenesulfonyl)-piperidin-4-yl]-1,4-dihydro-benzo[d][1,3]oxazin-2-one |
| 188 | 6-Chloro-1-[1-(3-fluoro-benzenesulfonyl)-piperidin-4-yl]-1,4-dihydro-benzo[d][1,3]oxazin-2-one |
| 199 | 6-Chloro-1-[1-(2,4-dichloro-benzenesulfonyl)-piperidin-4-yl]-1,4-dihydro-benzo[d][1,3]oxazin-2-one |
| 200 | 6-Chloro-1-[1-(2,4,6-trimethyl-benzenesulfonyl)-piperidin-4-yl]-1,4-dihydro-benzo[d][1,3]oxazin-2-one |
| 201 | 6-Chloro-1-[1-(2-trifluoromethyl-benzenesulfonyl)-piperidin-4-yl]-1,4-dihydro-benzo[d][1,3]oxazin-2-one |
| 202 | 1-[1-(2-Oxo-2H-chromene-6-sulfonyl)-piperidin-4-yl]-1,4-dihydro-benzo[d][1,3]oxain-2-one |
| 203 | 1-[1-(3,5-Dichloro-benzenesulfonyl)-piperidin-4-yl]-1,4-dihydro-benzo[d][1,3]oxazin-2-one |
| 204 | 1-[1-(2,5-Dichloro-benzenesulfonyl)-piperidin-4-yl]-1,4-dihydro-benzo[d][1,3]oxazin-2-one |
| 205 | 1-[1-(5-Bromo-6-chloro-pyridine-3-sulfonyl)-piperidin-4-yl]-1,4-dihydro-benzo[d][1,3]oxazin-2-one |
| 206 | 1-[1-(4-Chloro-benzenesulfonyl)-piperidin-4-yl]-1,4-dihydro-benzo[d][1,3]oxazin-2-one |
| 207 | 1-[1-(2,6-Dichloro-benzenesulfonyl)-piperidin-4-yl]-1,4-dihydro-benzo[d][1,3]oxazin-2-one |
| 208 | 8-Methyl-1-[1-(2-oxo-2H-chromene-6-sulfonylppiperidin-4-yl]-1,4-dihydro-benzo[d][1,3]oxazin-2-one |
| 209 | 1-[1-(3,5-Dichloro-benzenesulfonyl)-piperidin-4-yl]-8-methyl-1,4-dihydro-benzo[d][1,3]oxazin-2-one |
| 210 | 1-[1-(2,5-Dichloro-benzenesulfonyl)-piperidin-4-yl]-8-methyl-1,4-dihydro-benzo[d][1,3]oxazin-2-one |
| 211 | 1-[1-(5-Bromo-6-chloro-pyridine-3-sulfonyl)-piperidin-4-yl]-8-methyl-1,4-dihydro-benzo[d][1,3]oxazin-2-one |
| 212 | 1-[1-(4-Chloro-benzenesulfonyl)-piperidin-4-yl]-8-methyl-1,4-dihydro-benzo[d][1,3]oxazin-2-one |
| 213 | 1-[1-(2,6-Dichloro-benzenesulfonyl)piperidin-4-yl]-8-methyl-1,4-dihydro-benzo[d][1,3]oxazin-2-one |
| 214 | 1-[1-(Biphenyl-4-sulfonyl)-piperidin-4-yl]-1 ,4-dihydro-benzo[d][1,3]oxazin-2-one |
| 215 | 6-Chloro-1-[1-(2,5-dichloro-benzenesulfonyl)-piperidin-4-yl]-1,4-dihydro-benzo[d][1,3]oxazin-2-one |
| 216 | 1-[1-(5-Bromo-6-chloro-pyridine-3-sulfonyl)-piperidin-4-yl]-6-chloro-1,4-dihydro-benzo[d][1,3]oxazin-2-one |
| 217 | 6-Chloro-1-[1-(4-chloro-benzenesulfonyl)-piperidin-4-yl]-1,4-dihydro-benzo[d][1,3]oxazin-2-one |
| 218 | Chloro-1-[1-(2,6-dichloro-benzenesulfonyl)-piperidin-4-yl]-1,4-dihydro-benzo[d][1,3]oxazin-2-one |
| 219 | 1-[1-(Biphenyl-4-sulfonyl)-piperidin-4-yl]-6-methyl-1,4-dihydro-benzo[d][1,3]oxazin-2-one |
| 220 | 2-[4-(6-Methyl-2-oxo-4H-benzo[d][1,3]oxazin-1-yl)-piperidine-1-sulfonyl]-benzonitrile |
| 221 | 1-[1-(2,5-Dichloro-benzenesulfonyl)-piperidin-4-yl]-6-methyl-1,4-dihydro-benzo[d][1,3]oxazin-2-one |
| 222 | 1-[1-(5-Bromo-6-chloro-pyridine-3-sulfonyl)-piperidin-4-yl]-6-methyl-1,4-dihydro-benzo[d][1,3]oxazin-2-one |
| 223 | 1-[1-(4-Chloro-benzenesulfonyl)-piperidin-4-yl]-6-methyl-1,4-dihydro-benzo[d][1,3]oxazin-2-one |
| 224 | 1-[1-(2,6-Dichloro-benzenesulfonyl)-piperidin-4-yl]-6-methyl-1,4-dihydro-benzo[d][1,3]oxazin-2-one |
| 225 | 1-[1-(3.5-Dichloro-benzenesulfonyl)-piperidin-4-yl]-6-methyl-1,4-dihydro-benzo[d][1,3]oxazin-2-one |
| 226 | 6-Methyl-1-[1-(1-methyl-1H-imidazole-4-sulfonyl)-piperidin-4-yl]-1,4-dihydro-benzo[d][1,3]oxazin-2-one |
| 227 | 1-[1-(5-Bromo-2,4-difluoro-benzenesulfonyl)-piperidin-4-yl]-6-methyl-1,4-dihydro-benzo[d][1,3]oxazin-2-one |
| 228 | 1-[1-(4-Methanesulfonyl-benzenesulfonyl)-piperidin-4-yl]-1,4-dihydro-benzo[d][1,3]oxazin-2-one |
| 229 | 1-[1-(1-Methyl-1H-imidazole-4-sulfonyl)-piperidin-4-yl]-1,4-dihydro-benzo[d][1,3]oxazin-2-one |
| 230 | 1-[1-(5-Bromo-2,4-difluoro-benzenesulfonyl)-piperidin-4-yl]-1,4-dihydro-benzo[d][1,3]oxazin-2-one |
| 231 | 1-[1-(6-Chloro-imidazo[2,1-b]thiazole-5-sulfonyl)-piperidin-4-yl]-1,4-dihydro-benzo[d][1,3]oxazin-2-one |
| 232 | 1-[1-(4-Ethyl-benzenesulfonyl)-piperidin-4-yl]-1,4-dihydro-benzo[d][1,3]oxazin-2-one |
| 233 | 1-[1-(Benzo[b]thiophene-3-sulfonyl)-piperidin-4-yl]-1,4-dihydro-benzo[d][1,3]oxazin-2-one |
| 234 | 1-[1-(6-Chloro-imidazo[2,1-b]thiazole-5-sulfonyl)-piperidin-4-yl]-8-methyl-1,4-dihydro-benzo[d][1,3]oxazin-2-one |
| 235 | 1-[1-(4-Ethyl-benzenesulfonyl)-piperidin-4-yl]-8-methyl-1,4-dihydro-benzo[d][1,3]oxazin-2-one |
| 236 | 1-[1-(Benzo[b]thiophene-3-sulfonyl)-piperidin-4-yl]-8-methyl-1,4-dihydro-benzo[d][1,3]oxazin-2-one |
| 237 | 6-Chloro-1-[1-(6-chloro-imidazo[2,1-b]thiazole-5-sulfonyl)-piperidin-4-yl]-1,4-dihydro-benzo[d][1,3]oxazin-2-one |
| 238 | 6-Chloro-1-[1-(4-ethyl-benzenesulfonyl)-piperidin-4-yl]-1,4-dihydro-benzo[d][1,3]oxazin-2-one |
| 239 | 1-[1-(Benzo[b]thiophene-3-sulfonyl)-piperidin-4-yl]-6-chloro-1,4-dihydro-benzo[d][1,3]oxazin-2-one |
| 240 | 1-[1-(6-Chloro-imidazo[2,1-b]thiazole-5-sulfonyl)-piperidin-4-yl]-6-methyl-1,4-dihydro-benzo[d][1,3]oxazin-2-one |
| 241 | 1-[1-(4-Ethyl-benzenesulfonyl)-piperidin-4-yl]-6-methyl-1,4-dihydro-benzo[d][1,3]oxazin-2-one |
| 242 | 1-[1-(Benzo[b]thiophene-3-sulfonyl)-piperidin-4-yl]-6-methyl-1,4-dihydro-benzo[d][1,3]oxazin-2-one |
| 243 | 1-[1-(7-Chloro-benzo[1,2,5]oxadiazole-4-sutfonyl)-piperidin-4-yl]-1,4-dihydro-benzo[d][1,3]oxazin-2-one |
| 244 | 1-[1-(2-Methoxy-4-methyl-benzenesulfonyl)-piperidin-4-yl]-1,4-dihydro-benzo[d][1,3]oxazin-2-one |
| 245 | 3-{4-[4-{2-Oxo-4H-benzo[d][1,3]oxazin-1-yl}-piperidine-1-sulfonyl]-phenyl}-propionic acid methyl ester |
| 246 | 1-[1-(2,4-Dinitro-benzenesulfonyl)-piperidin-4-yl]-1,4-dihydro-benzo[d][1,3]oxazin-2-one |
| 247 | 1-[1-(7-Chloro-benzo[1,2,5]oxadiazole-4-sulfonyl)-piperidin-4-yl]-8-methyl-1,4-dihydro-benzo[d][1,3]oxazin-2-one |
| 248 | 1-[1-(2-Methoxy-4-methyl-benzenesulfonyl)-piperidin-4-yl]-8-methyl-1,4-dihydro-benzo[d][1,3]oxazin-2-one |
| 249 | 3-{4-[4-(8-Methyl-2-oxo-4H-benzo[d][1,3]oxazin-1-yl)-piperidine-1-sulfonyl]-phenyl}-propionic acid methyl ester |
| 250 | 1-[1-(2,4-Dinitro-benzenesulfonyl)-piperidin-4-yl]-8-methyl-1,4-dihydro-benzo[d][1,3]oxazin-2-one |
| 251 | 1-[1-(7-Chloro-benzo[1,2,5]oxadiazole-4-sulfonylppiperidin-4-yl]-6-methyl-1,4-dihydro-benzo[d][1,3]oxazin-2-one |
| 252 | 1-[1-(2-Methoxy-4-methyl-benzenesulfonyl)-piperidin-4-yl]-6-methyl-1,4-dihydro-benzo[d][1,3]oxazin-2-one |
| 253 | 3-{4-[4-(6-Methyl-2-oxo-4H-benzo[d][1,3]oxazin-1-yl)-piperidine-1-sulfonyl]-phenyl}-propionic acid methyl ester |
| 254 | 1-[1-(2,4-Dinitro-benzenesulfonyl)-piperidin-4-yl]-6-methyl-1,4-dihydro-benzo[d][1,3]oxazin-2-one |
| 255 | 6-Chloro-1-[1-(7-chloro-benzo[1,2,5]oxadiazole-4-sulfonyl)-piperidin-4-yl]-1,4-dihydro-benzo[d][1,3]oxazin-2-one |
| 256 | 6-Chloro-1-[1-(2-methoxy-4-methyl-benzenesulfonyl)-piperidin-4-yl]-1,4-dihydro-benzo[d][1,3]oxazin-2-one |
| 257 | 3-{4-[4-(6-Chloro-2-oxo-4H-benzo[d][1,3]oxazin-1-yl)-piperidine-1-sulfonyl]-phenyl}-propionic acid methyl ester |
| 258 | 6-Chloro-1-[1-(2,4-dinitro-benzenesulfonyl)-piperidin-4-yl]-1,4-dihydro-benzo[d][1,3]oxazin-2-one |
| 259 | 6-Chloro-1-[1-(1-methyl-1H-imidazole-4-sulfonyl)-piperidin-4-yl]-1,4-dihydro-benzo[d][1,3]oxazin-2-one |
| 260 | 1-[1-(5-Bromo-2,4-difluoro-benzenesulfonyl)-piperidin4-yl]-6-chloro-1,4-dihydro-benzo[d][1,3]oxazin-2-one |
| 261 | 8-Methyl-1-[1-(1-methyl-1H-imidazole-4-sulfonyl)-piperidin-4-yl]-1,4-dihydro-benzo[d][1,3]oxazin-2-one |
| 262 | 1-[1-(5-Bromo-2,4-difluoro-benzenesulfonyl)-piperidin-4-yl]-8-methyl-1,4-dihydro-benzo[d][1,3]oxazin-2-one |
| 263 | 1-[1-(Benzo[b]thiophene-2-sulfonyl)-piperidin-4-yl]-8-methyl-1,4-dihydro-benzo[d][1,3]oxazin-2-one |
| 264 | 1-[1-(Benzo[b]thiophene-2-sulfonyl)-piperidin-4-yl]-1,4-dihydro-benzo[d][1,3]oxazin-2-one |
| 265 | 1-[1-(Benzo[b]thiophene-2-sulfonyl)-piperidin-4-yl]-6-chloro-1,4-dihydro-benzo[d][1,3]oxazin-2-one |
| 266 | 1-[1-(Benzo[b]thiophene-2-sulfonyl)-piperidin-4-yl]-6-methyl-1,4-dihydro-benzo[d][1,3]oxazin-2-one |
| 267 | 1-[1-(2,5-Difluoro-benzenesulfonyl)-piperidin-4-yl]-1,4-dihydro-benzo[d][1,3]oxazin-2-one e |
| 268 | 1-[1-(2,5-Difluoro-benzenesulfonyl)-piperidin-4-yl]-8-methyl-1,4-dihydro-benzo[d][1,3]oxazin-2-on |
| 269 | 6-Chloro-1-[1-(2,5-difluoro-benzenesulfonyl)-piperidin-4-yl]-1,4-dihydro-benzo[d][1,3]oxazin-2-one |
| 270 | 1-[1-(2,5-Difluoro-benzenesulfonyl)-piperidin-4-yl]-6-methyl-1,4-dihydro-benzo[d][1,3]oxazin-2-one |
| 271 | 1-[1-(4-Chloro-2,5-difluoro-benzenesulfonyl)-piperidin-4-yl]-1,4-dihydro-benzo[d][1,3]oxazin-2-one |
| 272 | 1-[1-(4-Chloro-2,5-difluoro-benzenesulfonyl)-piperidin-4-yl]-8-methyl-1,4-dihydro-benzo[d][1,3]oxazin-2-one |
| 273 | 6-Chloro-1-[1-(4-chloro-2,5-difluoro-benzenesulfonyl)-piperidin-4-yl]-1,4-dihydro-benzo[d][1,3]oxazin-2-one |
| 274 | 1-[1-(4-Chloro-2,5-difluoro-benzenesulfonyl)-piperidin-4-yl]-6-methyl-1,4-dihydro-benzo[d][1,3]oxazin-2-one |
| 275 | 1-[1-(2,4,5-Trifluoro-benzenesulfonyl)-piperidin-4-yl]-1,4-dihydro-benzo[d][1,3]oxazin-2-one |
| 276 | 8-Methyl-1-[1-(2,4,5-trifluoro-benzenesulfonyl)-piperidin-4-yl]-1,4-dihydro-benzo[d][1,3]oxazin-2-one |
| 277 | 6-Chloro-1-[1-(2,4,5-trifluoro-benzenesulfonyl)-piperidin-4-yl]-1,4-dihydro-benzo[d][1,3]oxazin-2-one |
| 278 | 6-Methyl-1-[1-(2,4,5-trifluoro-benzenesulfonyl)-piperidin-4-yl]-1,4-dihydro-benzo[d][1,3]oxazin-2-one |
| 279 | 1-[1-(3,5-Dichloro-2-hydroxy-benzenesulfonyl)-piperidin-4-yl]-1,4-dihydro-benzo[d][1,3]oxazin-2-one |
| 280 | 1-[1-(2,6-Difluoro-benzenesulfonyl)-piperidin-4-yl]-1,4-dihydro-benzo[d][1,3]oxazin-2-one |
| 281 | 1-[1-(2,6-Difluoro-benzenesulfonyl)-piperidin-4-yl]-8-methyl-1,4-dihydro-benzo[d][1,3]oxazin-2-one |
| 282 | 6-Chloro-1-[1-(2,6-difluoro-benzenesulfonyl)-piperidin-4-yl]-1,4-dihydro-benzo[d][1,3]oxazin-2-one |
| 283 | 1-[1-(2,6-Difluoro-benzenesulfonyl)-piperidin-4-yl]-6-methyl-1,4-dihydro-benzo[d][1,3]oxazin-2-one |
| 284 | 1-[1-(5-Chloro-2,4-difluoro-benzenesulfonyl)-piperidin-4-yl]-1,4-dihydro-benzo[d][1,3]oxazin-2-one |
| 285 | 1-[1-(5-Chloro-2,4-difluoro-benzenesulfonyl)-piperidin-4-yl]-8-methyl-1,4-dihydro-benzo[d][1,3]oxazin-2-one |
| 286 | 6-Chloro-1-[1-(5-chloro-2,4-difluoro-benzenesulfonyl)-piperidin-4-yl]-1,4-dihydro-benzo[d][1,3]oxazin-2-one |
| 287 | 1-[1-(5-Chloro-2,4-difluoro-benzenesulfonyl)-piperidin-4-yl]-6-methyl-1,4-dihydro-benzo[d][1,3]oxazin-2-one |
| 288 | 1-[1-(2-Chloro-benzenesulfonyl)-piperidin-4-yl]-1,4-dihydro-benzo[d][1,3]oxazin-2-one |
| 289 | 1-[1-(2-Chloro-benzenesulfonyl)-piperidin-4-yl]-8-methyl-1,4-dihydro-benzo[d][1,3]oxazin-2-one |
| 290 | 6-Chloro-1-[1-(2-chloro-benzenesulfonyl)-piperidin-4-yl]-1,4-dihydro-benzo[d][1,3]oxazin-2-one |
| 291 | 1-[1-(2-Chloro-benzenesulfonyl)-piperidin-4-yl]-6-methyl-1,4-dihydro-benzo[d][1,3]oxazin-2-one |
| 292 | 6-Chloro-1-[1-(2-naphthalen-1-yl-ethanesulfonyl)-piperidin-4-yl]-1,4-dihydro-benzo[d][1,3]oxazin-2-one |
| 293 | 6-Bromo-1-[1-(4-bromo-benzenesulfonyl)-piperidin-4-yl]-1,4-dihydro-benzo[d][1,3]oxazin-2-one |
| 294 | 6-Bromo-1-[1-(toluene-4-sulfonyl)-piperidin-4-yl]-1,4-dihydro-benzo[d][1,3]oxazin-2-one |
| 295 | 6-Bromo-1-[1-(2,4-dimethyl-benzenesulfonyl)-piperidin-4-yl]-1,4-dihydro-benzo[d][1,3]oxazin-2-one |
| 296 | 6-Bromo-1-[1-(2-naphthalen-1-yl-ethanesulfonyl)-piperidin-4-yl]-1,4-dihydro-benzo[d][1,3]oxazin-2-one |
| 297 | 6-Bromo-1[1-(quinoline-8-sulfonyl)-piperidin-4-yl]-1,4-dihydro-benzo[d][1,3]oxazin-2-one |
| 298 | 6-Bromo-1-[1-(5-chloro-3-methyl-benzo[b]thiophene-2-sulfonyl)-piperidin-4-yl]-1,4-dihydro-benzo[d][1,3]oxazin-2-one |
| 299 | 6-Bromo-1-[1-(3-nitro-benzenesulfonyl)-piperidin-4-yl)]-1,4-dihydro-benzo[d][1,3]oxazin-2-one |
| 300 | 6-Bromo-1-[1-(naphthalene-1-sulfonyl)-piperidin-4-yl]-1,4-dihydro-benzo[d][1,3]oxazin-2-one |
| 301 | 6-Bromo-1-[1-(naphthalene-2-sulfonyl)-piperidin-4-yl]-1,4-dihydro-benzo[d][1,3]oxazin-2-one |
| 302 | 1-(1-Benzenesulfonyl-piperidin-4-yl)-6-bromo-1,4-dihydro-benzo[d][1,3]oxazin-2-one |
| 303 | 6-Bromo-1-{1-[4-(4-bromo-phenoxy)-benzenesulfonyl]-piperidin-4-yl}-1,4-dihydro-benzo[d][1,3]oxazin-2-one |
| 304 | 6-Bromo-1-[1-(thiophene-2-suffonyl)-piperidin-4-yl]-1,4-dihydro-benzo[d][1,3]oxazin-2-one |
| 305 | 6-Bromo-1-[1-(2-methyl-5-nitro-benzenesulfonyl)-piperidin-4-yl]-1,4-dihydro-benzo[d][1,3]oxazin-2-one |
| 306 | 6-Bromo-1-[1-(4-bromo-2,5-difluoro-benzenesulfonyl)-piperidin-4-yl]-1,4-dihydro-benzo[d][1,3]oxazin-2-one |
| 307 | 6-Bromo-1-[1-(toluene-3-sulfonyl)-piperidin-4-yl]-1 ,4-dihydro-benzo[d][1,3]oxazin-2-one |
| 308 | 6-Bromo-1-1-(5-fluoro-2-methyl-benzenesulfonyl)-piperidin-4-yl]-1,4-dihydro-benzo[d][1,3]oxazin-2-one |
| 309 | 6-Bromo-1-[1-(4-isopropoxy-benzenesulfonyl)-piperidin-4-yl]-1,4-dihydro-benzo[d][1,3]oxazin-2-one |
| 310 | 6-Bromo-1-[1-(3-chloro-benzenesulfonyl)-piperidin-4-yl]-1,4-dihydro-benzo[d][1,3]oxazin-2-one |
| 311 | 6-Bromo-1-[1-(3,4-dimethoxy-benzenesulfonyl)-piperidin-4-yl]-1,4-dihydro-benzo[d][1,3]oxazin-2-one |
| 312 | 6-Bromo-1-(1-pentafluorobenzenesulfonyl-piperidin-4-yl)-1,4-dihydro-benzo[d][1,3]oxazin-2-one |
| 313 | 6-Bromo-1-[1-(4-chloro-2,5-dimethyl-benzenesulfonyl)-piperidin-4-yl]-1,4-dihydro-benzo[d][1,3]oxazin-2-one |
| 314 | 6-Bromo-1-[1-(3-methoxy-benzenesulfonyl)-piperidin-4-yl]-1,4-dihydro-benzo[d][1,3]oxazin-2-one |
| 315 | 6-Bromo-1-[1-(4-isopropyl-benzenesulfonyl)-piperidin-4-yl]-1,4-dihydro-benzo[d][1,3]oxazin-2-one |
| 316 | 6-Bromo-1-[1-(4-fluoro-benzenesulfonyl)-piperidin-4-yl]-1,4-dihydro-benzo[d][1,3]oxazin-2-one |
| 317 | 6-Bromo-1-[1-(3-chloro-4-fluoro-benzenesulfonyl)-piperidin-4-yl]-1,4-dihydro-benzo[d][1,3]oxazin-2-one |
| 318 | 6-Bromo-1-(1-pentamethylbenzenesulfonyl-piperidin-4-yl)-1,4-dihydro-benzo[d][1,3]oxazin-2-one |
| 319 | 6-Bromo-1-[1-(2-nitro-benzenesulfonyl)-piperidin-4-yl]-1,4-dihydro-benzo[d][1,3]oxazin-2-one |
| 320 | 6-Bromo-1-[1-(4-chloro-3-nitro-benzenesulfonyl)-piperidin-4-yl]-1,4-dihydro-benzo[d][1,3]oxazin-2-one |
| 321 | 6-Bromo-1-[1-(5-dimethylamino-naphthalene-1-sulfonyl)-piperidin-4-yl]-1,4-dihydro-benzo[d][1,3]oxazin-2-one |
| 322 | 6-Bromo-1-[1-(4-nitro-benzenesulfonyl)-piperidin-4-yl]-1,4-dihydro-benzo[d][1,3]oxazin-2-one |
| 323 | 1-[1-(4-Acetyl-benzenesulfonyl)-piperidin-4-yl]-6-bromo-1,4-dihydro-benzo[d][1,3]oxazin-2-one |
| 324 | 6-Bromo-1-[1-(4-methanesulfonyl-benzenesulfonyl)-piperidin-4-yl]-1,4-dihydro-benzo[d][1,3]oxazin-2-one |
| 325 | 1-[1-(Biphenyl-4-sulfonyl)-piperidin-4-yl]-6-bromo-1,4-dihydro-benzo[d][1,3]oxazin-2-one |
| 326 | 6-Bromo-1-(1-phenylmethanesulfonyl-piperidin-4-yl)-1,4-dihydro-benzo[d][1,3]oxazin-2-one |
| 327 | 6-Bromo-1-[1-(2,5-dimethoxy-benzenesulfonyl)-piperidin-4-yl]-1,4-dihydro-benzo[d][1,3]oxazin-2-one |
| 328 | 6-Bromo-1-{1-[2-(2,2,2trifluoro-acetyl)-1,2,3,4-tetrahydro-isoquinoline-7-sulfonyl]-piperidin-4-yl}-1,4-dihydro-benzo[d][1,3]oxazin-2-one |
| 329 | 6-Bromo-1-[1-(2,3-dichloro-benzenesulfonyl)-piperidin-4-yl]-1,4-dihydro-benzo[d][1,3]oxazin-2-one |
| 330 | 6-Bromo-1-[1-(2,4,5-trichloro-benzenesulfonyl)-piperidin-4-yl]-1,4-dihydro-benzo[d][1,3]oxazin-2-one |
| 331 | 6-Bromo-1-[1-(5-bromo-2-methoxy-benzenesulfonyl)-piperidin-4-yl]-1,4-dihydro-benzo[d][1,3]oxazin-2-one |
| 332 | 6-Bromo-1-[1-(4-trifluoromethoxy-benzenesulfonyl)-piperidin-4-yl]-1,4-dihydro-benzo[d][1,3]oxazin-2-one |
| 333 | 6-Bromo-1-[1-(2-nitro-4-trifluoromethyl-benzenesulfonyl)-piperidin-4-yl]-1,4-dihydro-benzo[d][1,3]oxazin-2-one |
| 334 | 6-Bromo-1-[1-(3-fluoro-benzenesulfonyl)-piperidin-4-yl]-1,4-dihydro-benzo[d][1,3]oxazin-2-one |
| 335 | 6-Bromo-1-[1-(2,4-dichloro-benzenesulfonyl)-piperidin-4-yl]-1,4-dihydro-benzo[d][1,3]oxazin-2-one |
| 336 | 6-Bromo-1-[1-(2,4,6-trimethyl-benzenesulfonyl)-piperidin-4-yl]-1,4-dihydro-benzo[d][1,3]oxazin-2-one |
| 337 | 6-Bromo-1-[1-(2-trifluoromethyl-benzenesulfonyl)-piperidin-4-yl]-1,4-dihydro-benzo[d][1,3]oxazin-2-one |
| 338 | 6-Bromo-1-[1-(2-bromo-benzenesulfonyl)-piperidin-4-yl]-1,4-dihydro-benzo[d][1,3]oxazin-2-one |
| 339 | 6-Bromo-1-[1-(4-methoxy-2,3,6-trimethyl-benzenesulfonyl)-piperidin-4-yl]-1,4-dihydro-benzo[d][1,3]oxazin-2-one |
| 340 | 1-[1-(3,5-Dichloro-4-hydroxy-benzenesulfonyl)-piperidin-4-yl]-1,4-dihydro-benzo[d][1,3]oxazin-2-one |
| 341 | 1-[1-(3,5-Dichloro-4-hydroxy-benzenesulfonylppiperidin-4-yl]-8-methyl-1,4-dihydro-benzo[d][1,3]oxazin-2-one |
| 342 | 6-Chloro-1-[1-(3,5-dichloro-4-hydroxy-benzenesulfonyl)-piperidin-4-yl]-1,4-dihydro-benzo[d][1,3]oxazin-2-one |
| 343 | 1-[1-(3,5-Dichloro-4-hydroxy-benzenesulfonyl)-piperidin-4-yl]-6-methyl-1,4-dihydro-benzo[d][1,3]oxazin-2-one |
| 344 | 6-Bromo-1-[1-(3,5-dichloro-4-hydroxy-benzenesulfonyl)-piperidin-4-yl]-1,4-dihydro-benzo[d][1,3]oxazin-2-one |
| 345 | 6-Chloro-1-[1-(3,5-dichloro-2-hydroxy benzenesulfonyl)-piperidin-4-yl]-1,4-dihydro-benzo[d][1,3]oxazin-2-one |
| 346 | 6-Bromo-1-[1-(3,5-dichloro-2-hydroxy-benzenesulfonyl)-piperidin-4-yl]-1,4-dihydro-benzo[d][1,3]oxazin-2-one |
| 347 | 2-[4-(6-Bromo-2-oxo-4H-benzo[d][1,3]oxazin-1-yl)-piperidine-1-sulfonyl]-benzonitrile |
| 348 | 6-Bromo-1-[1-(4-methoxy-benzenesulfonyl)-piperidin-4-yl]-1,4-dihydro-benzo[d][1,3]oxazin-2-one |
| 349 | 2-[4-(6-Bromo-2-oxo-4H-benzo[d][1,3]oxazin-1-yl)-piperidine-1-sulfonyl]-benzoic acid methyl ester |
| 350 | 6-Bromo-1-[1-(3-trifluoromethyl-benzenesulfonylppiperidin-4-yl]-1,4-dihydro-benzo[d][1,3]oxazin-2-one |
| 351 | 6-Bromo-1-[1-(2-oxo-2H-chromene-6-sulfonyl)-piperidin-4-yl]-1,4-dihydro-benzo[d][1,3]oxazin-2-one |
| 352 | 6-Bromo-1-[1-(3,5-dichloro-benzenesulfonyl)-piperidin-4-yl]-1,4-dihydro-benzo[d][1,3]oxazin-2-one |
| 353 | 6-Bromo-1-[1-(2,5-dichloro-benzenesulfonyl)-piperidin-4-yl]-1,4-dihydro-benzo[d][1,3]oxazin-2-one |
| 354 | 6-Bromo-1-[1-(5-bromo-6-chloro-pyridine-3-sulfonyl)-piperidin-4-yl]-1,4-dihydro-benzo[d][1,3]oxazin-2-one |
| 355 | 6-Bromo-1-[1-(4-chloro-benzenesulfonyl)-piperidin-4-yl]-1,4-dihydro-benzo[d][1,3]oxazin-2-one |
| 356 | 6-Bromo-1-[1-(2,6-dichloro-benzenesulfonyl)-piperidin-4-yl]-1,4-dihydro-benzo[d][1,3]oxazin-2-one |
| 357 | 6-Bromo-1-[1-(1-methyl-1H-imidazole-4-sulfonyl)-piperidin-4-yl]-1,4-dihydro-benzo[d][1,3]oxazin-2-one |
| 358 | 6-Bromo-1-[1-(5-bromo-2,4-difluoro-benzenesulfonyl)-piperidin-4-yl]-1,4-dihydro-benzo[d][1,3]oxazin-2-one |
| 359 | 6-Bromo-1-[1-(4-ethyl-benzenesulfonyl)-piperidin-4-yl]-1,4-dihydro-benzo[d][1,3]oxazin-2-one |
| 360 | 6-Bromo-1-[1-(6-chloro-imidazo[2,1-b]thiazole-5-sulfonyl)-piperidin-4-yl]-1,4-dihydro-benzo[d][1,3]oxazin-2-one |
| 361 | 1-[1-(Benzo[b]thiophene-3-sulfonyl)-piperidin-4-yl]-6-bromo-1,4-dihydro-benzo[d][1,3]oxazin-2-one |
| 362 | 6-Bromo-1-[1-(7-chloro-benzo[1,2,5]oxadiazole-4-sulfonyl)-piperidin-4-yl]-1,4-dihydro-benzo[d][1,3]oxazin-2-one |
| 363 | 6-Bromo-1-[1-(2-methoxy-4-methyl-benzenesulfonyl)-piperidin-4-yl]-1,4-dihydro-benzo[d][1,3]oxazin-2-one |
| 364 | 3-{4-[4-(6-Bromo-2-oxo-4H-benzo[d][1,3]oxazin-1-yl)-piperidine-1-sulfonyl]-phenyl}-propionic acid methyl ester |
| 365 | 6-Bromo-1-[1-(2,4-dinitro-benzenesulfonyl)-piperidin-4-yl]-1,4-dihydro-benzo[d][1,3]oxazin-2-one |
| 366 | 1-[1-(Benzo[b]thiophene-2-sulfonyl)-piperidin-4-yl]-6-bromo-1,4-dihydro-benzo[d][1,3]oxazin-2-one |
| 367 | 6-Bromo-1-[1-(2,5-difluoro-benzenesulfonyl)-piperidin-4-yl]-1,4-dihydro-benzo[d][1,3]oxazin-2-one |
| 368 | 6-Bromo-1-[1-(4-chloro-2,5-difluoro-benzenesulfonyl)-piperidin-4-yl]-1,4-dihydro-benzo[d][1,3]oxazin-2-one |
| 369 | 6-Bromo-1-[1-(2,4,5-trifluoro-benzenesulfonyl)-piperidin-4-yl]-1,4-dihydro-benzo[d][1,3]oxazin-2-one |
| 370 | 6-Bromo-1-[1-(2,6-difluoro-benzenesulfonyl)-piperidin-4-yl]-1,4-dihydro-benzo[d][1,3]oxazin-2-one |
| 371 | 6-Bromo-1-[1-(5-chloro-2,4-difluoro-benzenesulfonyl)-piperidin-4-yl]-1,4-dihydro-benzo[d][1,3]oxazin-2-one |
| 372 | 6-Bromo-1-[1-(2-chloro-benzenesulfonyl)-piperidin-4-yl]-1,4-dihydro-benzo[d][1,3]oxazin-2-one |
| 373 | 6-Bromo-1-[1-(2,3,4-trifluoro-benzenesufonyl)-piperidin-4-yl]-1,4-dihydro-benzo[d][1,3]oxazin-2-one |
| 374 | N-{4-[4-(6-Bromo-2-oxo-4H-benzo[d][1,3]oxazin-1-yl)-piperidine-1-sulfonyl]-2-chloro-phenyl}-acetamide |
| 375 | 1-[1-(2,3,4-Trifluoro-benzenesulfonyl)-piperidin-4-yl)]-1,4-dihydro-benzo[d][1,3]oxazin-2-one |
| 376 | 8-Methyl-1-[1-(2,3,4-trifluoro-benzenesulfonyl)-piperidin-4-yl]-1,4-dihydro-benzo[d][1,3]oxazin-2-one |
| 377 | 6-Chloro-1-[1-(2,3,4-trifluoro-benzenesulfonyl)-piperidin-4-yl]-1,4-dihydro-benzo[d][1,3]oxazin-2-one |
| 378 | 6-Methyl-1-[1-(2,3,4-trifluoro-benzenesulfonyl)-piperidin-4-yl]-1,4-dihydro-benzo[d][1,3]oxazin-2-one |
| 379 | N-{2-Chloro-4-[4-(6-methyl-2-oxo-4H-benzo[d][1,3]oxazin-1-yl)-piperidine-1-sulfonyl]-phenyl}-acetamide |
| 380 | 1-[1-(3,4-Difluoro-benzenesulfonyl)-piperidin-4-yl]-1,4-dihydro-benzo[d][1,3]oxazin-2-one |
| 381 | 1-[1-(3,4-Difluoro-benzenesulfonyl)-piperidin-4-yl]-8-methyl-1,4-dihydro-benzo[d][1,3]oxazin-2-one |
| 382 | 6-Chloro-1-[1-(3,4-difluoro-benzenesulfonyl)-piperidin-4-yl]-1 ,4-dihydro-benzo[d][1,3]oxazin-2-one |
| 383 | 1-[1-(3,4-Difluoro-benzenesulfonyl)-piperidin-4-yl]-6-methyl-1,4-dihydro-benzo[d][1,3]oxazin-2-one |
| 384 | 6-Bromo-1-[1-(3,4-difluoro-benzenesulfonyl)-piperidin-4-yl]-1,4-dihydro-benzo[d][1,3]oxazin-2-one |
| 385 | N-{2-Chloro-4-[4-(8-methyl-2-oxo-4H-benzo[d][1,3]oxazin-1-yl)-piperidine-1-sulfonyl]-phenyl}-acetamide |
| 386 | 1-[1-(2-Chloro-4,5-difluoro-benzenesulfonyl)-piperidin-4-yl]-1,4-dihydro-benzo[d][1,3]oxazin-2-one |
| 387 | 1-[1-(2-Chloro-4,5-difluoro-benzenesulfonyl)-piperidin-4-yl]-8-methyl-1,4-dihydro-benzo[d][1,3]oxazin-2-one |
| 388 | 6-Chloro-1-[1-(2-chloro-4,5-difluoro-benzenesulfonyl)-piperidin-4-yl]-1,4-dihydro-benzo[d][1,3]oxazin-2-one |
| 389 | 1-[1-(2-Chloro-4,5-difluoro-benzenesulfonyl)-piperidin-4-yl]-6-methyl-1,4-dihydro-benzo[d][1,3]oxazin-2-one |
| 390 | 6-Bromo-1-[1-(2-chloro-4,5-difluoro-benzenesulfonyl)-piperidin-4-yl]-1,4-dihydro-benzo[d][1,3]oxazin-2-one |
| 391 | N-{2-Chloro-4-[4-(2-oxo-4H-benzo[d][1,3]oxazin-1-yl)-piperidine-1-sulfonyl]-phenyl}-acetamide |
| 392 | 1-[1-(Benzo[1,2,5]oxadiazole-4-sulfonyl)-piperidin-4-yl]-1,4-dihydro-benzo[d][1,3]oxazin-2-one |
| 393 | 1-[1-(Benzo[1,2,5]oxadiazole-4-sulfonyl)-piperidin-4-yl]-8-methyl-1,4-dihydro-benzo[d][1,3]oxazin-2-one |
| 394 | 1-[1-(Benzo[1,2,5]oxadiazole-4-sulfonyl)-piperidin-4-yl]-6-chloro-1,4-dihydro-benzo[d][1,3]oxazin-2-one |
| 395 | 1-[1-(Benzo[1,2,5]oxadiazole-4-sulfonyl)-piperidin-4-yl]-6-methyl-1,4-dihydro-benzo[d][1,3]oxazin-2-one |
| 396 | 1-[1-(Benzo[1,2,5]oxadiazole-4-sulfonyl)-piperidin-4-yl]-6-bromo-1,4-dihydro-benzo[d][1,3]oxazin-2-one |
| 397 | N-{2-Chloro-4-[4-(6-chloro-2-oxo-4H-benzo[d][1,3]oxazin-1-yl)-piperidine-1-sulfonyl]-phenyl}-acetamide |
| 398 | 1-[1-(Benzo[1,2,5]thiadiazole-4-sulfonyl)-piperidin-4-yl]-1,4-dihydro-benzo[d][1,3]oxazin-2-one |
| 399 | 1-[1-(Benzo[1,2, 5]thiadiazole-4-sulfonyl)-piperidin-4-yl]-8-methyl-1,4-dihydro-benzo[d][1,3]oxazin-2-one |
| 400 | 1-[1-(Benzo[1,2,5]thiadiazole-4-sulfonyl)-piperidin-4-yl]-6-chloro-1,4-dihydro-benzo[d][1,3]oxazin-2-one |
| 401 | 1-[1-(Benzo[1,2,5]thiadiazole-4-sulfonyl)-piperidin-4-yl]-6-methyl-1,4-dihydro-benzo[d][1,3]oxazin-2-one |
| 402 | 1-[1-(Benzo[1,2,5]thiadiazole-4-sulfonyl)-piperidin-4-yl]-6-bromo-1,4-dihydro-benzo[d][1,3]oxazin-2-one |
| 403 | 1-(1-Ethanesulfonyl-piperidin-4-yl)-8-methyl-1,4-dihydro-benzo[d][1,3]oxazin-2-one |
| 404 | 1-[1-(2,4-Difluoro-benzenesulfonyl)-piperidin-4-yl]-1,4-dihydro-benzo[d][1,3]oxazin-2-one |
| 405 | 1-[1-(2,4-Difluoro-benzenesulfonyl)-piperidin-4-yl]-8-methyl-1,4-dihydro-benzo[d][1,3]oxazin-2-one |
| 406 | 6-Chloro-1-[1-(2,4-difluoro-benzenesulfonyl)-piperidin-4-yl]-1,4-dihydro-benzo[d][1,3]oxazin-2-one |
| 407 | 1-[1-(2,4-Difluoro-benzenesulfonyl)-piperidin-4-yl]-6-methyl-1,4-dihydro-benzo[d][1,3]oxazin-2-one |
| 408 | 6-Bromo-1-[1-(2,4-difluoro-benzenesulfonyl)-piperidin-4-yl]-1,4-dihydro-benzo[d][1,3]oxazin-2-one |
| 409 | 8-Methyl-1-[1-(propane-2-sulfonyl)-piperidin-4-yl]-1,4-dihydro-benzo[d][1,3]oxazin-2-one |
| 410 | 1-[1-(3,4-Dichloro-benzenesulfonyl)-piperidin-4-yl]-1,4-dihydro-benzo[d][1,3]oxazin-2-one |
| 411 | 1-[1-(3,4-Dichloro-benzenesulfonyl)-piperidin-4-yl]-8-methyl-1,4-dihydro-benzo[d][1,3]oxazin-2-one |
| 412 | 6-Chloro-1-[1-(3,4-dichloro-benzenesulfonyl)-piperidin-4-yl]-1,4-dihydro-benzo[d][1,3]oxazin-2-one |
| 413 | 1-[1-(3,4-Dichloro-benzenesulfonyl)-piperidin-4-yl]-6-methyl-1,4-dihydro-benzo[d][1,3]oxazin-2-one |
| 414 | 6-Bromo-1-[1-(3,4-dichloro-benzenesulfonyl)-piperidin-4-yl]-1,4-dihydro-benzo[d][1,3]oxazin-2-one |
| 415 | 8-Methyl-1-[1-propane-1-sulfonylfpiperidin-4-yl]-1,4-dihydro-benzo[d][1,3]oxazin-2-one |
| 416 | 1-[1-(2-Chloro-6-methyl-benzenesulfonyl)-piperidin-4-yl]-1,4-dihydro-benzo[d][1,3]oxazin-2-one |
| 417 | 1-[1-(2-Chloro-6-methyl-benzenesulfonyl)-piperidin-4-yl]-8-methyl-1,4-dihydro-benzo[d][1,3]oxazin-2-one |
| 418 | 6-Chloro-1-[1-(2-chloro-6-methyl-benzenesulfonyl)-piperidin-4-yl]-1,4-dihydro-benzo[d][1,3]oxazin-2-one |
| 419 | 1-[1-(2-Chloro-6-methyl-benzenesulfonyl)-piperidin-4-yl]-6-methyl-1,4-dihydro-benzo[d][1,3]oxazin-2-one |
| 420 | 1-[1-(2-Chloro-6-methyl-benzenesulfonyl)-piperidin-4-yl]-6-methyl-1,4-dihydro-benzo[d][1,3]oxazin-2-one |
| 421 | 8-Methyl-1-[1-(2,3,5,6-tetramethyl-benzenesulfonylypiperidin-4-yl]-1,4-dihydro-benzo[d][1,3]oxazin-2-one |
| 422 | 1-[1-(2,3,4-Trichloro-benzenesulfonyl)-piperidin-4-yl]-1,4-dihydro-benzo[d][1,3]oxazin-2-one |
| 423 | 8-Methyl-1-[1-(2,3,4-trichloro-benzenesulfonyl)-piperidin-4-yl]-1,4-dihydro-benzo[d][1,3]oxazin-2-one |
| 424 | 6-Chloro-1-[1-(2,3,4-trichloro-benzenesulfonyl)-piperidin-4-yl]-1,4-dihydro-benzo[d][1,3]oxazin-2-one |
| 425 | 6-Methyl-1-[1-(2,3,4-trichloro-benzenesulfonyl)-piperidin-4-yl]-1,4-dihydro-benzo[d][1,3]oxazin-2-one |
| 426 | 6-Bromo-1-[1-(2,3,4-trichloro-benzenesulfonyl)-piperidin-4-yl]-1,4-dihydro-benzo[d][1,3]oxazin-2-one |
| 427 | 1-[1-(2, 3,5,6-Tetramethyl-benzenesulfonyl)-piperidin-4-yl]-1,4-dihydro-benzo[d][1,3]oxazin-2-one |
| 428 | 1-[1-(Thiophene-3-sulfonyl)-piperidin-4-yl]-1,4-dihydro-benzo[d][1,3]oxazin-2-one |
| 429 | 8-Methyl-1-[1-(thiophene-3-sulfonyl)-piperidin-4-yl]-1,4-dihydro-benzo[d][1,3]oxazin-2-one |
| 430 | 6-Chloro-1-[1-(thiophene-3-sulfonyl)-piperidin-4-yl]-1,4-dihydro-benzo[d][1,3]oxazin-2-one |
| 431 | 6-Methyl-1-[1-(thiophene-3-sulfonyl)-piperidin-4-yl]-1,4-dihydro-benzo[d][1,3]oxazin-2-one |
| 432 | 6-Bromo-1-[1-(thiophene-3-sulfonyl)-piperidin-4-yl]-1,4-dihydro-benzo[d][1,3]oxazin-2-one |
| 433 | 6-Chloro-1-[1-(2,3,5,6-tetramethyl)-benzenesulfonyl-piperidin-4-yl]-1,4-dihydro-benzo[d][1,3]oxazin-2-one |
| 434 | 1-[1-(2,4,6-Trichloro-benzenesulfonyl)-piperidin-4-yl]-1,4-dihydro-benzo[d][1,3]oxazin-2-one |
| 435 | 8-Methyl-1-[1-(2,4,6-trichloro-benzenesulfonyl)-piperidin-4-yl]-1,4-dihydro-benzo[d][1,3]oxazin-2-one |
| 436 | 6-Chloro-1-[1-(2,4,6-trichloro-benzenesulfonyl)-piperidin-4-yl]-1,4-dihydro-benzo[d][1,3]oxazin-2-one |
| 437 | 6-Methyl-1-[1-(2,4,6-trichloro-benzenesulfonyl)-piperidin-4-yl]-1,4-dihydro-benzo[d][1,3]oxazin-2-one |
| 438 | 6-Bromo-1-[1-(2,4,6-trichloro-benzenesulfonyl)-piperidin-4-yl]-1,4-dihydro-benzo[d][1,3]oxazin-2-one |
| 439 | 6-Methyl-1-[1-(2,3,5,6-tetramethyl-benzenesulfonyl)-piperidin-4-yl]-1,4-dihydro-benzo[d][1,3]oxazin-2-one |
| 440 | 1-[1-(2-Bromo-4,6-difluoro-benzenesulfonyl)-piperidin-4-yl]-8-methyl-1,4-dihydro-benzo[d][1,3]oxazin-2-one |
| 441 | 1-[1-(2-Bromo-4,6-difluoro-benzenesulfonyl)-piperidin-4-yl]-6-chloro-1,4-dihydro-benzo[d][1,3]oxazin-2-one |
| 442 | 1-[1-(2-Bromo-4,6-difluoro-benzenesulfonyl)-piperidin-4-yl]-6-methyl-1,4-dihydro-benzo[d][1,3]oxazin-2-one |
| 443 | 6-Bromo-1-[1-(2-bromo-4,6-difluoro-benzenesulfonyl)-piperidin-4-yl]-1,4-dihydro-benzo[d][1,3]oxazin-2-one |
| 444 | 6-Bromo-1-[1-(2,3,5,6-tetramethyl-benzenesulfonyl)-piperidin-4-yl]-1,4-dihydro-benzo[d][1,3]oxazin-2-one |
| 445 | 1-[1-(4-Bromo-2-trifluoromethoxy-benzenesulfonyl)-piperidin-4-yl]-1,4-dihydro-benzo[d][1,3]oxazin-2-one |
| 446 | 1-[1-(4-Bromo-2-trifluoromethoxy-benzenesulfonyl)-piperidin-4-yl]-8-methyl-1,4-dihydro-benzo[d][1,3]oxazin-2-one |
| 447 | 1-[1-(4-Bromo-2-trifluoromethoxy-benzenesulfonyl)-piperidin-4-yl]-6-chloro-1,4-dihydro-benzo[d][1,3]oxazin-2-one |
| 448 | 1-[1-(4-Bromo-2-trifluoromethoxy-benzenesulfonyl)-piperidin-4-yl]-6-methyl-1,4-dihydro-benzo[d][1,3]oxazin-2-one |
| 449 | 6-Bromo-1-[1-(4-bromo-2-trifluoromethoxy-benzenesulfonyl)-piperidin-4-yl]-1,4-dihydro-benzo[d][1,3]oxazin-2-one |
| 450 | 1-[1-(4-Phenoxy-benzenesulfonyl)-piperidin-4-yl]-1,4-dihydro-benzo[d][1,3]oxazin-2-one |
| 451 | 1-[1-(3-Bromo-benzenesulfonyl)-piperidin-4-yl]-1 ,4-dihydro-benzo[d][1,3]oxazin-2-one |
| 452 | 1-[1-(3-Bromo-benzenesulfonyl)-piperidin-4-yl]-8-methyl-1,4-dihydro-benzo[d][1,3]oxazin-2-one |
| 453 | 1-[1-(3-Bromo-benzenesulfonyl)-piperidin-4-yl]-6-chloro-1,4-dihydro-benzo[d][1,3]oxazin-2-one |
| 454 | 1-[1-(3-Bromo-benzenesulfonyl)-piperidin-4-yl]-6-methyl-1,4-dihydro-benzo[d][1,3]oxazin-2-one |
| 455 | 6-Bromo-1-[1-(3-bromo-benzenesulfonyl)-piperidin-4-yl]-1,4-dihydro-benzo[d][1,3]oxazin-2-one |
| 456 | 8-Methyl-1-[1-(4-phenoxy-benzenesulfonyl)-piperidin-4-yl]-1,4-dihydro-benzo[d][1,3]oxazin-2-one |
| 457 | 1-[1-(4-tert-Butyl-benzenesulfonyl)-piperidin-4-yl]-1,4-dihydro-benzo[d][1,3]oxazin-2-one |
| 458 | 1-[1-(4-tert-Butyl-benzenesulfonyl)-piperidin-4-yl]-8-methyl-1,4-dihydro-benzo[d][1,3]oxazin-2-one |
| 459 | 1-[1-(4-tert-Butyl-benzenesulfonyl)-piperidin-4-yl]-6-chloro-1,4-dihydro-benzo[d][1,3]oxazin-2-one |
| 460 | 1-[1-(4-tert-Butyl-benzenesulfonyl)-piperidin-4-yl]-6-methyl-1,4-dihydro-benzo[d][1,3]oxazin-2-one |
| 461 | 6-Bromo-1-[1-(4-tert-butyl-benzenesulfonylrpiperidin-4-yl]-1,4-dihydro-benzo[d][1,3]oxazin-2-one |
| 462 | 6-Chloro-1-[1-(4-phenoxy-benzenesulfonyl)-piperidin-4-yl]-1,4-dihydro-benzo[d][1,3]oxazin-2-one |
| 463 | 1-[1-(2-Bromo-4,6-difluoro-benzenesulfonyl)-piperidin-4-yl]-1,4-dihydro-benzo[d][1,3]oxazin-2-one |
| 464 | 1-[1-(2-Methanesulfonyl-benzenesulfonyl)-piperidin-4-yl]-8-methyl-1,4-dihydro-benzo[d][1,3]oxazin-2-one |
| 465 | 6-Chloro-1-[1-(2-methanesulfonyl-benzenesulfonyl)-piperidin-4-yl]-1,4-dihydro-benzo[d][1,3]oxazin-2-one |
| 466 | 1-[1-(2-Methanesulfonyl-benzenesulfonyl)-piperidin-4-yl]-6-methyl-1,4-dihydro-benzo[d][1,3]oxazin-2-one |
| 467 | 6-Bromo-1-[1-(2-methanesulfonyl-benzenesulfonyl)-piperidin-4-yl]-1,4-dihydro-benzo[d][1,3]oxazin-2-one |
| 468 | 8-Methyl-1-[1-(4-propyl-benzenesulfonyl)-piperidin-4-yl]-1,4-dihydro-benzo[d][1,3]oxazin-2-one |
| 469 | 6-Chloro-1-[1-(4-propyl-benzenesulfonyl)-piperidin-4-yl]-1,4-dihydro-benzo[d][1,3]oxazin-2-one |
| 470 | 6-Methyl-1-[1-(4-propyl-benzenesulfonyl)-piperidin-4-yl]-1,4-dihydro-benzo[d][1,3]oxazin-2-one |
| 471 | 6-Bromo-1[1-(4-propyl)-benzenesulfonyl)-piperidin-4-yl]-1,4-dihydro-benzo[d][1,3]oxazin-2-one |
| 472 | 1-[1-(3-Chloro-2-methyl-benzenesulfonyl)-piperidin-4-yl]-8-methyl-1,4-dihydro-benzo[d][1,3]oxazin-2-one |
| 473 | 6-Chloro-1-[1-(3-chloro-2-methyl-benzenesulfonyl)-piperidin-4-yl]-1,4-dihydro-benzo[d][1,3]oxazin-2-one |
| 474 | 1-[1-(3-Chloro-2-methyl-benzenesulfonyl)-piperidin-4-yl]-6-methyl-1,4-dihydro-benzo[d][1,3]oxazin-2-one |
| 475 | 6-Bromo-1-[1-(3-chloro-2-methyl-benzenesulfonyl)-piperidin-4-yl]-1,4-dihydro-benzo[d][1,3]oxazin-2-one |
| 476 | 1-[1-(4-Butyl-benzenesulfonyl)-piperidin-4-yl]-8-methyl-1,4-dihydro-benzo[d][1,3]oxazin-2-one |
| 477 | 1-[1-(4-Butyl-benzenesulfonyl)-piperidin-4-yl]-6-chloro-1,4-dihydro-benzo[d][1,3]oxazin-2-one |
| 478 | 1-[1-(4-Butyl-benzenesulfonyl)-piperidin-4-yl]-6-methyl-1,4-dihydro-benzo[d][1,3]oxazin-2-one |
| 479 | 6-Bromo-1-[1-(4-butyl-benzenesulfonyl)-piperidin-4-yl]-1,4-dihydro-benzo[d][1,3]oxazin-2-one |
| 480 | 1-[1-(4-Bromo-3-methyl-benzenesulfonyl)-piperidin-4-yl]-8-methyl-1,4-dihydro-benzo[d][1,3]oxazin-2-one |
| 481 | 1-[1-(4-Bromo-3-methyl-benzenesulfonyl)-piperidin-4-yl]-6-chloro-1,4-dihydro-benzo[d][1,3]oxazin-2-one |
| 482 | 1-[1-(4-Bromo-3-methyl-benzenesulfonyl)-piperidin-4-yl]-6-methyl-1,4-dihydro-benzo[d][1,3]oxazin-2-one |
| 483 | 6-Bromo-1-[1-(4-bromo-3-methyl-benzenesulfonyl)-piperidin-4-yl]-1,4-dihydro-benzo[d][1,3]oxazin-2-one |
| 484 | 1-{1-[4-(1,1-Dimethyl-propyl)-benzenesulfonyl]-piperidin-4-yl}-8-methyl-1,4-dihydro-benzo[d][1,3]oxazin-2-one |
| 485 | 6-Chloro-1-{1-[4-(1,1-dimethyl-propyl)-benzenesulfonyl]-piperidin-4-yl}-1,4-dihydro-benzo[d][1,3]oxazin-2-one |
| 486 | 1-{1-[4-(1,1-Dimethyl-propyl)-benzenesulfonyl]-piperidin-4-yl}-6-methyl-1,4-dihydro-benzo[d][1,3]oxazin-2-one |
| 487 | 6-Bromo-1-{1-[4-(1,1-dimethyl-propyl)-benzenesulfonyl]-piperidin-4-yl}-1,4-dihydro-benzo[d][1,3]oxazin-2-one |
| 488 | 1-(1-Ethenesulfonyl-piperidin-4-yl)-8-methyl-1,4-dihydro-benzo[d][1,3]oxazin-2-one |
| 489 | 3-[4-(8-Methyl-2-oxo-4H-benzo[d][1,3]oxazin-1-yl)-piperidine-1-sulfonyl]-benzoic acid |
| 490 | 3-[4-(6-Methyl-2-oxo-4H-benzo[d][1,3]oxazin-1-yl)-piperidine-1-sulfonyl]-benzoic acid |
| 491 | 3-[4-(6-Bromo-2-oxo-4H-benzo[d][1,3]oxazin-1-yl)-piperidine-1-sulfonyl]-benzoic acid |
| 492 | 1-[1-(3-Chloro-2-fluoro-benzenesulfonyl)-piperidin-4-yl]-8-methyl-1,4-dihydro-benzo[d][1,3]oxazin-2-one |
| 493 | 6-Chloro-1-[1-(3-chloro-2-fluoro-benzenesulfonyl)-piperidin-4-yl]-1,4-dihydro-benzo[d][1,3]oxazin-2-one |
| 494 | 1-[1-(3-Chloro-2-fluoro-benzenesulfonyl)-piperidin-4-yl]-6-methyl-1,4-dihydro-benzo[d][1,3]oxazin-2-one |
| 495 | 6-Bromo-1-[1-(3-chloro-2-fluoro-benzenesulfonyl)-piperidin-4-yl]-1,4-dihydro-benzo[d][1,3]oxazin-2-one |
| 496 | N-{4-Methyl-5-[4-(8-methyl-2-oxo-4H-benzo[d][1,3]oxazin-1-yl)-piperidine-1-sulfonyl]-thiazol-2-yl}-acetamide |
| 497 | N-{5-[4-(6-Chloro-2-oxo-4H-benzo[d][1,3]oxazin-1-yl)-piperidine-1-sulfonyl]-4-methyl-thiazol-2-yl}-acetamide |
| 498 | N-{4-Methyl-5-[4-(6-methyl-2-oxo-4H-benzo[d][1,3]oxazin-1-yl)-piperidine-1-sulfonyl]-thiazol-2-yl}-acetamide |
| 499 | N-{5-[4-(6-Bromo-2-oxo-4H-benzo[d][1,3]oxazin-1-yl)-piperidine-1-sulfonyl]-4-methyl-thiazol-2-yl}-acetamide |
| 500 | 1-[1-(2-Bromo-4-fluoro-benzenesulfonyl)-piperidin-4-yl]-8-methyl-1,4-dihydro-benzo[d][1,3]oxazin-2-one |
| 501 | 1-[1-(2-Bromo-4-fluoro-benzenesulfonyl)-piperidin-4-yl]-6-chloro-1,4-dihydro-benzo[d][1,3]oxazin-2-one |
| 502 | 1-[1-(2-Bromo-4-fluoro-benzenesulfonyl)-piperidin-4-yl]-6-methyl-1,4-dihydro-benzo[d][1,3]oxazin-2-one |
| 503 | 6-Bromo-1-[1-(2-bromo-4-fluoro-benzenesulfonyl)-piperidin-4-yl]-1,4-dihydro-benzo[d][1,3]oxazin-2-one |
| 504 | 1-[1-(5-Chloro-2-fluoro-benzenesulfonyl)-piperidin-4-yl]-8-methyl-1,4-dihydro-benzo[d][1,3]oxazin-2-one |
| 505 | 6-Chloro-1-[1-(5-chloro-2-fluoro-benzenesulfonyl)-piperidin-4-yl]-1,4-dihydro-benzo[d][1, 3]oxazin-2-one |
| 506 | 1-[1-(5-Chloro-2-fluoro-benzenesulfonyl)-piperidin-4-yl]-6-methyl-1,4-dihydro-benzo[d][1,3]oxazin-2-one |
| 507 | 6-Bromo-1-[1-(5-chloro-2-fluoro-benzenesulfonyl)-piperidin-4-yl]-1,4-dihydro-benzo[d][1,3]oxazin-2-one |
| 508 | 1-[1-(4-Bromo-3-trifluoromethyl-benzenesulfonyl)-piperidin-4-yl]-8-methyl-1,4-dihydro-benzo[d][1,3]oxazin-2-one |
| 509 | 1-[1-(4-Bromo-3-trifluoromethyl-benzenesulfonyl)-piperidin-4-yl]-6-chloro-1,4-dihydro-benzo[d][1,3]oxazin-2-one |
| 510 | 1-[1-(4-Bromo-3-trifluoromethyl-benzenesulfonyl)-piperidin-4-yl]-6-methyl-1,4-dihydro-benzo[d][1,3]oxazin-2-one |
| 511 | 6-Bromo-1-[1-(4-bromo-3-trifluoromethyl-benzenesulfonyl)-piperidin-4-yl]-1,4-dihydro-benzo[d][1,3]oxazin-2-one |
| 512 | 1-[1-(2-Methanesulfonyl-benzenesulfonyl)-piperidin-4-yl]-1,4-dihydro-benzo[d][1,3]oxazin-2-one |
| 513 | 1-[1-(4-Propyl-benzenesulfonyl)-piperidin-4-yl]-1,4-dihydro-benzo[d][1,3]oxazin-2-one |
| 514 | 1-[1-(3-Chloro-2-methyl-benzenesulfonyl)-piperidin-4-yl]-1,4-dihydro-benzo[d][1,3]oxazin-2-one |
| 515 | 1-[1-(4-Butyl-benzenesulfonyl)-piperidin-4-yl]-1,4-dihydro-benzo[d][1,3]oxazin-2-one |
| 516 | 1-[1-(4-Bromo-3-methyl-benzenesulfonyl)-piperidin-4-yl]-1,4-dihydro-benzo[d][1,3]oxazin-2-one |
| 517 | 1-{1-[4-(1,1-Dimethyl-propyl)-benzenesulfonyl]-piperidin-4-yl}-1,4-dihydro-benzo[d][1,3]oxazin-2-one |
| 518 | N-{4-Methyl-5-[4-(2-oxo-4H-benzo[d][1,3]oxazin-1-yl)-piperidine-1-sulfonyl]-thiazol-2-yl}-acetamide |
| 519 | 1-[1-(3-Chloro-2-fluoro-benzenesulfonyl)-piperidin-4-yl]-1,4-dihydro-benzo[d][1,3]oxazin-2-one |
| 520 | 1-[1-(2-Bromo-4-fluoro-benzenesulfonyl)-piperidin-4-yl]-1,4-dihydro-benzo[d][1,3]oxazin-2-one |
| 521 | 1-[1-(4-Bromo-3-trifluoromethyl-benzenesulfonyl)-piperidin-4-yl]-1,4-dihydro-benzo[d][1,3]oxazin-2-one |
| 522 | 1-[1-(5-Chloro-2-fluoro-benzenesulfonyl)-piperidin-4-yl]-1,4-dihydro-benzo[d][1,3]oxazin-2-one |
| 523 | 1-[1-(Isoquinoline-5-sulfonyl)-piperidin-4-yl]-1,4-dihydro-benzo[d][1,3]oxazin-2-one |
| 524 | 6-Fluoro-1-[1-(2-methanesulfonyl-benzenesulfonyl)-piperidin-4-yl]-1,4-dihydro-benzo[d][1,3]oxazin-2-one |
| 525 | 6-Fluoro-1-[1-(4-propyl-benzenesulfonyl)-piperidin-4-yl]-1,4-dihydro-benzo[d][1,3]oxazin-2-one |
| 526 | 1-[1-(3-Chloro-2-methyl-benzenesulfonyl)-piperidin-4-yl]-6-fluoro-1,4-dihydro-benzo[d][1,3]oxazin-2-one |
| 527 | 1-[1-(4-Butyl-benzenesulfonyl)-piperidin-4-yl]-6-fluoro-1,4-dihydro-benzo[d][1,3]oxazin-2-one |
| 528 | 1-[1-(4-Bromo-3-methyl-benzenesulfonyl)-piperidin-4-yl]-6-fluoro-1,4-dihydro-benzo[d][1,3]oxazin-2-one |
| 529 | 1-{1-[4-(1,1-Dimethyl-propyl)-benzenesulfonyl]-piperidin-4-yl}-6-fluoro-1,4-dihydro-benzo[d][1,3]oxazin-2-one |
| 530 | N-{5-[4-(6-Fluoro-2-oxo-4H-benzo[d][1,3]oxazin-1-yl)-piperidine-1-sulfonyl]-4-methyl-thiazol-2-yl}-acetamide |
| 531 | 1-[1-(3-Chloro-2-fluoro-benzenesulfonyl)-piperidin-4-yl)-6-fluoro-1,4-dihydro-benzo[d][1,3]oxazin-2-one |
| 532 | 1-[1-(2-Bromo-4-fluoro-benzenesulfonyl)-piperidin-4-yl]-6-fluoro-1,4-dihydro-benzo[d][1,3]oxazin-2-one |
| 533 | 1-[1-(4-Bromo-3-trifluoromethyl-benzenesulfonyl)-piperidin-4-yl]-6-fluoro-1,4-dihydro-benzo[d][1,3]oxazin-2-one |
| 534 | 1-[1-(5-Chloro-2-fluoro-benzenesulfonyl)-piperidin-4-yl]-6-fluoro-1,4-dihydro-benzo[d][1,3]oxazin-2-one |
| 535 | 6-Fluoro-1-[1-(isoquinoline-5-sulfonyl)-piperidin-4-yl]-1,4-dihydro-benzo[d][1,3]oxazin-2-one |
| 536 | 6-Fluoro-1-[1-(quinoline-8-suffonyl)-piperidin-4-yl]-1,4-dihydro-benzo[d][1,3]oxazin-2-one |
| 537 | 1-[1-(5-Chloro-3-methyl-benzo[b]thiophene-2-sulfonyl)-piperidin-4-yl]-6-fluoro-1,4-dihydro-benzo[d][1,3]oxazin-2-one |
| 538 | 6-Fluoro-1-[1-(naphthalene-1-sulfonyl)-piperidin-4-yl]-1,4-dihydro-benzo[d][1,3]oxazin-2-one |
| 539 | 6-Fluoro-1-[1-(naphthalene-2-sulfonyl)-piperidin-4-yl]-1,4-dihydro-benzo[d][1,3]oxazin-2-one |
| 540 | 1-[1-(Benzo[b]thiophene-2-sulfonyl)-piperidin-4-yl]-6-fluoro-1,4-dihydro-benzo[d][1,3]oxazin-2-one |
| 541 | 1-[1-(Benzo[b]thiophene-3-sulfonyl)-piperidin-4-yl]-6-fluoro-1,4-dihydro-benzo[d][1,3]oxazin-2-one |
| 542 | 8-Methoxy-1-[1-(quinoline-8-sulfonyl)-piperidin-4-yl]-1,4-dihydro-benzo[d][1,3]oxazin-2-one |
| 543 | 1-[1-(5-Chloro-3-methyl-benzo[b]thiophene-2-sulfonyl)-piperidin-4-yl]-8-methoxy-1,4-dihydro-benzo[d][1,3]oxazin-2-one |
| 544 | 8-Methoxy-1-[1-(naphthalene-1-sulfonyl)-piperidin-4-yl]-1,4-dihydro-benzo[d][1,3]oxazin-2-one |
| 545 | 8-Methoxy-1-[1-(naphthalene-2-sulfonyl)-piperidin-4-yl]-1,4-dihydro-benzo[d][1,3]oxazin-2-one |
| 546 | 1-[1-(Benzo[b]thiophene-2-sulfonyl)-piperidin-4-yl]-8-methoxy-1,4-dihydro-benzo[d][1,3]oxazin-2-one |
| 547 | 1-[1-(Benzo[b]thiophene-3-sulfonyl)-piperidin-4-yl]-8-methoxy-1,4-dihydro-benzo[d][1,3]oxazin-2-one |
| 548 | 5-Chloro-1-[1-(2-methanesulfonyl-benzenesulfonyl)-piperidin-4-yl]-1,4-dihydro-benzo[d][1,3]oxazin-2-one |
| 549 | 5-Chloro-1-[1-(4-propyl-benzenesulfonyl)-piperidin-4-yl]-1,4-dihydro-benzo[d][1,3]oxazin-2-one |
| 550 | 5-Chloro-1-[1-(3-chloro-2-methyl-benzenesulfonyl)-piperidin-4-yl]-1,4-dihydro-benzo[d][1,3]oxazin-2-one |
| 551 | 1-[1-(4-Butyl-benzenesulfonyl)-piperidin-4-yl]-5-chloro-1,4-dihyd ro-benzo[d][1,3]oxazin-2-one |
| 552 | 1-[1-(4-Bromo-3-methyl-benzenesulfonyl)-piperidin-4-yl]-5-chloro-1,4-dihydro-benzo[d][1,3]oxazin-2-one |
| 553 | 5-Chloro-1-{1-[4-(1,1-dimethyl-propyl)-benzenesulfonyl]-piperidin-4-yl}-1,4-dihydro-benzo[d][1,3]oxazin-2-one |
| 554 | N-{5-[4-(5-Chloro-2-oxo-4H-benzo[d][1,3]oxazin-1-yl)-piperidine-1-sulfonyl]-4-methyl-thiazol-2-yl}-acetamide |
| 555 | 5-Chloro-1-[1-(3-chloro-2-fluoro-benzenesulfonyl)-piperidin-4-yl]-1,4-dihydro-benzo[d][1,3]oxazin-2-one |
| 556 | 1-[1-(2-Bromo-4-fluoro-benzenesulfonyl)-piperidin-4-yl]-5-chloro-1,4-dihydro-benzo[d][1,3]oxazin-2-one |
| 557 | 1-[1-(4-Bromo-3-trifluoromethyl-benzenesulfonyl)-piperidin-4-yl]-5-chloro-1,4-dihydro-benzo[d][1,3]oxazin-2-one |
| 558 | 5-Chloro-1-[1-(5-chloro-2-fluoro-benzenesulfonyl)-piperidin-4-yl]-1,4-dihydro-benzo[d][1,3]oxazin-2-one |
| 559 | 5-Chloro-1-[1-(isoquinoline-5-sulfonyl)-piperidin-4-yl]-1,4-dihydro-benzo[d][1,3]oxazin-2-one |
| 560 | 1-[1-(2-Methanesulfonyl-benzenesulfonyl)-piperidin-4-yl]-8-methoxy-1,4-dihydro-benzo[d][1,3]oxazin-2-one |
| 561 | 1-[1-(2-Methanesulfonyl-benzenesutfonyl)-piperidin-4-yl]-8-methoxy-1,4-dihydro-benzo[d][1,3]oxazin-2-one |
| 562 | 1-[1-(3-Chloro-2-methyl-benzenesulfonyl)-piperidin-4-yl]-8-methoxy-1,4-dihydro-benzo[d][1,3]oxazin-2-one |
| 563 | 1-[1-(4-Butyl-benzenesulfonyl)-piperidin-4-yl]-8-methoxy-1,4-dihydro-benzo[d][1,3]oxazin-2-one |
| 564 | 1-[1-(4-Bromo-3-methyl-benzenesulfonyl)-piperidin-4-yl]-8-methoxy-1,4-dihydro-benzo[d][1,3]oxazin-2-one |
| 565 | 1-{1-[4-(1,1-Dimethyl-propylrbenzenesulfonyl]-piperidin-4-yl}-8-methoxy-1,4-dihydro-benzo[d][1,3]oxazin-2-one |
| 566 | N-{5-[4-(8-Methoxy-2-oxo-4H-benzo[d][1,3]oxazin-1-yl)-piperidine-1-sulfonyl]-4-methyl-thiazol-2-yl}-acetamide |
| 567 | 1-[1-(3-Chloro-2-fluoro-benzenesulfonyl)-piperidin-4-yl]-8-methoxy-1,4-dihydro-benzo[d][1,3]oxazin-2-one |
| 568 | 1-[1-(2-Bromo-4-fluoro-benzenesulfonyl)-piperidin-4-yl]-8-methoxy-1,4-dihydro-benzo[d][1,3]oxazin-2-one |
| 569 | 1-[1-(4-Bromo-3-trifluoromethyl-benzenesulfonyl)-piperidin-4-yl]-8-methoxy-1,4-dihydro-benzo[d][1,3]oxazin-2-one |
| 570 | 1-[1-(5-Chloro-2-fluoro-benzenesulfonyl)-piperidin-4-yl]-8-methoxy-1,4-dihydro-benzo[d][1,3]oxazin-2-one |
| 571 | 1-[1-(Isoquinoline-5-sulfonyl)-piperidin-4-yl]-8-methoxy-1,4-dihydro-benzo[d][1,3]oxain-2-one; hydrochloride |
| 572 | 1-[1-(4-Methyl-naphthalene-1-sulfonyl)-piperidin-4-yl]-1,4-dihydro-benzo[d][1,3]oxazin-2-one |
| 573 | 6-Chloro-1-[1-(4-methyl-naphthalene-1-sulfonyl)-piperidin-4-yl]-1,4-dihydro-benzo[d][1,3] oxazin-2-one |
| 574 | 6-Methyl-1-[1-(4-methyl-naphthalene-1-sulfonyl)-piperidin-4-yl]-1,4-dihydro-benzo[d][1,3]oxazin-2-one |
| 575 | 8-Methyl-1-[1-(4-methyl-naphthalene-1-sulfonyl)-piperidin-4-yl]-1,4-dihydro-benzo[d][1,3]oxazin-2-one |
| 576 | 6-Fluoro-1-[1-(4-methyl-naphthalene-1-sulfonyl)-piperidin-4-yl]-1,4-dihydro-benzo[d][1,3]oxazin-2-one |
| 577 | 8-Methoxy-1-[1-(4-methyl-naphthalene-1-sulfonyl)-piperidin-4-yl]-1,4-dihydro-benzo[d][1,3]oxazin-2-one |
| 578 | 5-Chloro-1-[1-(4-methyl-naphthalene-1-sulfonylypiperidin-4-yl]-1,4-dihydro-benzo[d][1,3]oxazin-2-one |
| 579 | 5-Chloro-1-[1-(naphthalene-1-sulfonyl)-piperidin-4-yl]-1,4-dihydro-benzo[d][1,3]oxazin-2-one |
| 580 | 5-Chloro-1-[1-(naphthalene-2-sulfonyl)-piperidin-4-yl]-1,4-dihydro-benzo[d][1,3]oxazin-2-one |
| 581 | 5-Chloro-1-[1-(quinoline-8-sulfonyl)-piperidin-4-yl]-1,4-dihydro-benzo[d][1,3]oxazin-2-one |
| 582 | 5-Chloro-1-[1-(5-chloro-3-methyl-benzo[b]thiophene-2-sulfonyl)-piperidin-4-yl]-1,4-dihydro-benzo[d][1,3]oxazin-2-one |
| 583 | 1-[1-(Benzo[b]thiophene-2-sulfonyl)-piperidin-4-yl]-5-chloro-1,4-dihydro-benzo[d][1,3]oxazin-2-one |
| 584 | 1-[1-(Benzo[b]thiophene-3-sulfonyl)-piperidin-4-yl]-5-chloro-1,4-dihydro-benzo[d][1,3]oxazin-2-one |
| 585 | 6-Bromo-1-[1-(4-methyl-naphthalene-1-sulfonyl)-piperidin-4-yl]-1,4-dihydro-benzo[d][1,3]oxazin-2-one |
| 586 | 2-Chloro-4-fluoro-5-[4-(8-methyl-2-oxo-4H-benzo[d][1,3]oxazin-1-yl)-piperidine-1-sulfonyl]-benzoic acid |
| 587 | 2-Chloro-5-[4-(6-chloro-2-oxo-4H-benzo[d][1,3]oxazin-1-yl)-piperidine-1-sulfonyl]-4-fluoro-benzoic acid |
| 588 | 2-Chloro-4-fluoro-5-[4-(6-methyl-2-oxo-4H-benzo[d][1,3]oxazin-1-yl)-piperidine-1-sulfonyl]-benzoic acid |
| 589 | 2-Chloro-4-fluoro-5-[4-(2-oxo-4H-benzo[d][1,3]oxazin-1-yl)-piperidine-1-sulfonyl]-benzoic acid |
| 590 | 2-Chloro-4-fluoro-5-[4-(8-methoxy-2-oxo-4H-benzo[d][1,3]oxazin-1-yl)-piperidine-1-sulfonyl]-benzoic acid |
| 591 | 2-Chloro-5-[4-(5-chloro-2-oxo-4H-benzo[d][1,3]oxazin-1-yl)-piperidine-1-sulfonyl]-4-fluoro-benzoic acid |
| 592 | 3-[4-(2-Oxo-4H-benzo[d][1,3]oxazin-1-yl)-piperidine-1-sulfonyl]-benzoic acid |
| 593 | 3-[4-(8-Methoxy-2-oxo-4H-benzo[d][1,3]oxazin-1-yl)-piperidine-1-sulfonyl]-benzoic acid |
| 594 | 3-[4-(5-Chloro-2-oxo-4H-benzo[d] [1,3]oxazin-1-yl)-piperidine-1-sulfonyl]-benzoic acid |
| 595 | 1-[1-(Isoquinoline-5-sulfonyl)-piperidin-4-yl]-8-methyl-1,4-dihydro-benzo[d][1,3]oxazin-2-one; hydrochloride |
| 596 | 6-Chloro-1-[1-(isoquinoline-5-sulfonyl)-piperidin-4-yl]-1,4-dihydro-benzo[d][1,3]oxazin-2-one; hydrochloride |
| 597 | -[1-(Isoquinoline-5-sulfonyl)-piperidin-4-yl]-6-methyl-1,4-dihydro-benzo[d][1,3]oxazin-2-one;hydrochloride |
| 598 | 6,7-Difluoro-1-[1-(quinoline-8-sulfonyl)-piperidin-4-yl]-1,4-dihydro-benzo[d][1,3]oxazin-2-one |
| 599 | 1-[1-(5-Chloro-3-methyl-benzo[b]thiophene-2-sulfonyl)-piperidin-4-yl]-6,7-difluoro-1,4-dihydro-benzo[d][1,3]oxazin-2-one |
| 600 | 6,7-Difluoro-1-[1-(naphthalene-1-sulfonyl)-piperidin-4-yl]-1,4-dihydro-benzo[d][1,3]oxazin-2-one |
| 601 | 6,7-Difluoro-1-[1-(naphthalene-2-sulfonyl)-piperidin-4-yl]-1,4-dihydro-benzo[d][1,3]oxazin-2-one |
| 602 | 1-[1-(Benzo[b]thiophene-2-sulfonyl)-piperidin-4-yl]-6,7-difluoro-1,4-dihydro-benzo[d][1,3]oxazin-2-one |
| 603 | 1-[1-(Benzo[b]thiophene-3-sulfonyl)-piperidin-4-yl]-6,7-difluoro-1,4-dihydro-benzo[d][1,3]oxazin-2-one |
| 604 | 1-[1-(5-Dimethylamino-naphthalene-1-sulfonyl)-piperidin-4-yl]-6,7-difluoro-1,4-dihydro-benzo[d][1,3]oxazin-2-one |
| 605 | 1-[1-(Biphenyl-4-sulfonyl)-piperidin-4-yl]-6,7-difluoro-1,4-dihydro-benzo[d][1,3]oxazin-2-one |
| 606 | 1-[1-(Benzo[1,2,5]thiadiazole-4-sulfonyl)-piperidin-4-yl]-6,7-difluoro-1,4-dihydro-benzo[d][1,3]oxazin-2-one |
| 607 | 1-[1-(Benzo[1,2,5]oxadiazole-4-sulfonyl)-piperidin-4-yl]-6,7-difluoro-1,4-dihydro-benzo[d][1,3]oxazin-2-one |
| 608 | 1-[1-(7-Chloro-benzo[1,2,5]oxadiazole-4-sulfonyl)-piperidin-4-yl]-6,7-difluoro-1,4-dihydro-benzo[d][1,3]oxazin-2-one |
| 609 | 6,7-Difluoro-1-[1-(4-methyl-naphthalene-1-sulfonyl)-piperidin-4-yl]-1,4-dihydro-benzo[d][1,3]oxazin-2-one |
| 610 | 1-[1-(4-Chloro-naphthalene-1-sulfonyl)-piperidin-4-yl]-1,4-dihydro-benzo[d][1,3]oxazin-2-one |
| 611 | 1-[1-(4-Fluoro-naphthalene-1-sulfonyl)-piperidin-4-yl]-1,4-dihydro-benzo[d][1,3]oxazin-2-one |
| 612 | 1-[1-(Dibenzofuran-2-sulfonyl)-piperidin-4-yl]-1,4-dihydro-benzo[d][1,3]oxazin-2-one |
| 613 | 1-[1-(2,3-Dihydro-benzofuran-5-sulfonyl)-piperidin-4-yl]-1,4-dihydro-benzo[d][1,3]oxazin-2-one |
| 614 | 1-[1-(Biphenyl-2-sulfonyl)-piperidin-4-yl]-1,4-dihydro-benzo[d][1,3]oxazin-2-one |
| 615 | 1-[1-(5-Isoxazol-5-yl-thiophene-2-sulfonyl)-piperidin-4-yl]-1,4-dihydro-benzo[d][1,3]oxazin-2-one |
| 616 | 1-[1-(4-Chloro-naphthalene-1-sulfonyl)-piperidin-4-yl]-8-methyl-1,4-dihydro-benzo[d][1,3]oxazin-2-one |
| 617 | 1-[1-(4-Fluoro-naphthalene-1-sulfonyl)-piperidin-4-yl]-8-methyl-1,4-dihydro-benzo[d][1,3]oxazin-2-one |
| 618 | 1-[1-(Dibenzofuran-2-sulfonyl)-piperidin-4-yl]-8-methy1-1,4-dihydro-benzo[d][1,3]oxazin-2-one |
| 619 | 1-[1-(2,3-Dihydro-benzofuran-5-sulfonyl)-piperidin-4-yl]-8-methyl-1,4-dihydro-benzo[d][1,3]oxazin-2-one |
| 620 | 1-[1-(Biphenyl-2-sulfonyl)-piperidin-4-yl]-8-methyl-1,4-dihydro-benzo[d][1,3]oxazin-2-one |
| 621 | 1-[1-(5-Isoxazol-5-yl-thiophene-2-sulfonyl)-piperidin-4-yl]-8-methyl-1,4-dihydro-benzo[d][1,3]oxazin-2-one |
| 622 | 5-Chloro-1-[1-(4-chloro-naphthalene-1-sulfonyl)-piperidin-4-yl]-1,4-dihydro-benzo[d][1,3]oxazin-2-one |
| 623 | 5-Chloro-1-[1-(4-fluoro-naphthalene-1-sulfonyl)-piperidin-4-yl]-1,4-dihydro-benzo[d][1,3]oxazin-2-one |
| 624 | 5-Chloro-1-[1-(dibenzofuran-2-sulfonylrpiperidin-4-yl]-1,4-dihydro-benzo[d][1,3]oxazin-2-one |
| 625 | 5-Chloro-1-[1-(2,3-dihydro-benzofuran-5-sulfonyl)-piperidin-4-yl]-1,4-dihydro-benzo[d][1,3]oxazin-2-one |
| 626 | 1-[1-(Biphenyl-2-sulfonyl)-piperidin-4-yl]-5-chloro-1,4-dihydro-benzo[d][1,3]oxazin-2-one |
| 627 | 5-Chloro-1-[1-(5-isoxazol-5-yl-thiophene-2-sulfonyl)-piperidin-4-yl]-1,4-dihydro-benzo[d][1,3]oxazin-2-one |
| 628 | 1-[1-(4-Chloro-naphthalene-1-sulfonyl)-piperidin-4-yl]-8-methoxy-1,4-dihydro-benzo[d][1,3]oxazin-2-one |
| 629 | 1-[1-(4-Fluoro-naphthalene-1-sulfonyl)-piperidin-4-yl]-8-methoxy-1,4-dihydro-benzo[d][1,3]oxazin-2-one |
| 630 | 1-[1-(Dibenzofuran-2-sulfonyl)-piperidin-4-yl]-8-methoxy-1,4-dihydro-benzo[d][1,3]oxazin-2-one |
| 631 | 1-[1-(2,3-Dihydro-benzofuran-5-sulfonyl)-piperidin-4-yl]-8-methoxy-1,4-dihydro-benzo[d][1,3]oxazin-2-one |
| 632 | 1-[1-(Biphenyl-2-sulfonyl)-piperidin-4-yl]-8-methoxy-1,4-dihydro-benzo[d][1,3]oxazin-2-one |
| 633 | 1-[1-(5-Isoxazol-5-yl-thiophene-2-sulfonyl)-piperidin-4-yl]-8-methoxy-1,4-dihydro-benzo[d][1,3]oxazin-2-one |
| 634 | 6-Chloro-1-[1-(4-chloro-naphthalene-1-sulfonyl)-piperidin-4-yl]-1,4-dihydro-benzo[d][1,3]oxazin-2-one |
| 635 | 6-Chloro-1-[1-(4-fluoro-naphthalene-1-sulfonyl)-piperidin-4-yl]-1,4-dihydro-benzo[d][1,3]oxazin-2-one |
| 636 | 6-Chloro-1-[1-(dibenzofuran-2-sulfonyl)-piperidin-4-yl]-1,4-dihydro-benzo[d][1,3]oxazin-2-one |
| 637 | 6-Chloro-1-[1-(2,3-dihydro-benzofuran-5-sulfonyl)-piperidin-4-yl]-1,4-dihydro-benzo[d][1,3]oxazin-2-one |
| 638 | 1-[1-(Biphenyl-2-sutfonyl)-piperidin-4-yl]-6-chloro-1,4-dihydro-benzo[d][1,3]oxazin-2-one |
| 639 | 6-Chloro-1-[1-(5-isoxazol-5-yl-thiophene-2-sulfonyl)-piperidin-4-yl]-1,4-dihydro-benzo[d][1,3]oxazin-2-one |
| 640 | 1-[1-(4-Chloro-naphthalene-1-sulfonyl)-piperidin-4-yl]-6-methyl-1,4-dihydro-benzo[d][1,3]oxazin-2-one |
| 641 | 1-[1-(4-Fluoro-naphthalene-1-sulfonyl)-piperidin-4-yl]-6-methyl-1,4-dihydro-benzo[d][1,3]oxazin-2-one |
| 642 | 1-[1-(Dibenzofuran-2-sulfonyl)-piperidin-4-yl]-6-methyl-1,4-dihydro-benzo[d][1,3]oxain-2-one |
| 643 | 1-[1-(2,3-Dihydro-benzofuran-5-sulfonyl)-piperidin-4-yl]-6-methyl-1,4-dihydro-benzo[d][1,3]oxazin-2-one |
| 644 | 1-[1-(Biphenyl-2-sulfonyl)-piperidin-4-yl]-6-methyl-1,4-dihydro-benzo[d][1,3]oxazin-2-one |
| 645 | 1-[1-(5-Isoxazol-5-yl-thiophene-2-sulfonyl)-piperidin-4-yl]-6-methyl-1,4-dihydro-benzo[d][1,3]oxazin-2-one |
| 646 | 1-[1-(4-Chloro-naphthalene-1-sulfonyl)-piperidin-4-yl]-6,7-difluoro-1,4-dihydro-benzo[d][1,3]oxazin-2-one |
| 647 | 6,7-Difluoro-1-[1-(4-fluoro-naphthalene-1-sulfonyl)-piperidin-4-yl]-1,4-dihydro-benzo[d](1,3]oxazin-2-one |
| 648 | 1-[1-(Dibenzofuran-2-sulfonyl)-piperidin-4-yl]-6,7-difluoro-1,4-dihydro-benzo[d][1,3]oxazin-2-one |
| 649 | 1-[1-(2,3-Dihydro-benzofuran-5-sulfonyl)-piperidin-4-yl]-6,7-difluoro-1,4-dihydro-benzo[d][1,3]oxazin-2-one |
| 650 | 1-[1-(Biphenyl-2-sulfonyl)-piperidin-4-yl]-6,7-difluoro-1,4-dihydro-benzo[d][1,3]oxazin-2-one |
| 651 | 6,7-Difluoro-1-[1-(5-isoxazol-5-yl-thiophene-2-sulfonyl)-piperidin-4-yl]-1,4-dihydro-benzo[d][1,3]oxazin-2-one |
| 652 | 1-[1-(1,2-Dimethyl-1H-imidazole-4-sulfonyl)-piperidin-4-yl]-1,4-dihydro-benzo[d][1,3]oxazin-2-one |
| 653 | 1-[1-(5-Methyl-benzo[1,2,5]thiadiazole-4-sulfonyl)-piperidin-4-yl]-1,4-dihydro-benzo[d][1,3]oxazin-2-one |
| 654 | 1-[1-(3,5-Dimethyl-isoxazole-4-sulfonyl)-piperidin-4-yl]-1,4-dihydro-benzo[d][1,3]oxazin-2-one |
| 655 | 1-[1-(1,2-Dimethyl-1H-imidazole-4-sulfonyl)-piperidin-4-yl]-8-methyl-1,4-dihydro-benzo[d][1,3]oxazin-2-one |
| 656 | 8-Methyl-1-[1-(5-methyl-benzo[1,2,5]thiadiazole-4-sulfonyl)-piperidin-4-yl]-1,4-dihydro-benzo[d][1,3]oxazin-2-one |
| 657 | 1-[1-(3,5-Dimethyl-isoxazole-4-sulfonyl)-piperidin-4-yl]-8-methyl-1,4-dihydro-benzo[d][1,3]oxazin-2-one |
| 658 | 6-Chloro-1-[1-(1,2-dimethyl-1H-imidazole-4-sulfonyl)-piperidin-4-yl]-1,4-dihydro-benzo[d][1,3]oxazin-2-one |
| 659 | 6-Chloro-1-[1-(5-methyl-benzo[1,2,5]thiadiazole-4-sulfonyl)-piperidin-4-yl]-1,4-dihydro-benzo[d][1,3]oxazin-2-one |
| 660 | 6-Chloro-1-[1-(3,5-dimethyl-isoxazole-4-sulfonyl)-piperidin-4-yl]-1,4-dihydro-benzo[d][1,3]oxazin-2-one |
| 661 | 1-[1-(1,2-Dimethyl-1H-imidazole-4-sulfonyl)-piperidin-4-yl]-8-methoxy-1,4-dihydro-benzo[d][1,3]oxazin-2-one |
| 662 | 8-Methoxy-1-[1-(5-methyl-benzo[1,2,5]thiadiazole-4-sulfonyl)-piperidin-4-yl]-1,4-dihydro-benzo[d][1,3]oxazin-2-one |
| 663 | 1-[1-(3,5-Dimethyl-isoxazole-4-sulfonyl)-piperidin-4-yl]-8-methoxy-1,4-dihydro-benzo[d][1,3]oxazin-2-one |
| 664 | 5-Chloro-1-[1-(1,2-dimethyl-1H-imidazole-4-sulfonyl)-piperidin-4-yl]-1,4-dihydro-benzo[d][1,3]oxazin-2-one |
| 665 | 5-Chloro-1-[1-(5-methyl-benzo[1,2,5]thiadiazole-4-sulfonyl)-piperidin-4-yl]-1,4-dihydro-benzo[d][1,3]oxazin-2-one |
| 666 | 5-Chloro-1-[1-(3,5-dimethyl-isoxazole-4-sulfonyl)-piperidin-4-yl]-1,4-dihydro-benzo[d][1,3]oxazin-2-one |
| 667 | 1-[1-(1,2-Dimethyl-1H-imidazole-4-sulfonyl)-piperidin-4-yl]-6-methyl-1,4-dihydro-benzo[d][1,3]oxazin-2-one |
| 668 | 6-Methyl-1-[1-(5-methyl-benzo[1,2,5]thiadiazole-4-sulfonyl)-piperidin-4-yl]-1,4-dihydro-benzo[d][1,3]oxazin-2-one |
| 669 | 1-[1-(3,5-Dimethyl-isoxazole-4-sulfonyl)-piperidin-4-yl]-6-methyl-1,4-dihydro-benzo[d][1,3]oxazin-2-one |
| 670 | 1-[1-(1,2-Dimethyl-1H-imidazole-4-sulfonyl)-piperidin-4-yl]-6-fluoro-1,4-dihydro-benzo[d][1,3]oxazin-2-one |
| 671 | 6-Fluoro-1-[1-(5-methyl-benzo[1,2,5]thiadiazole-4-sulfonyl)-piperidin-4-yl]-1,4-dihydro-benzo[d][1,3]oxazin-2-one |
| 672 | 1-[1-(3,5-Dimethyl-isoxazole-4-sulfonyl)-piperidin-4-yl]-6-fluoro-1,4-dihydro-benzo[d][1,3]oxazin-2-one |
| 673 | 1-[1-(1,2-Dimethyl-1H-imidazole-4-sulfonyl)-piperidin-4-yl]-6,7-difluoro-1,4-dihydro-benzo[d][1,3]oxazin-2-one |
| 674 | 6,7-Difluoro-1-[1-(5-methyl-benzo[1,2,5]thiadiazole-4-sulfonyl)-piperidin-4-yl]-1,4-dihydro-benzo[d][1,3]oxazin-2-one |
| 675 | 1-[1-(3,5-Dimethyl-isoxazole-4-sulfonyl)-piperidin-4-yl]-6,7-difluoro-1,4-dihydro-benzo[d][1,3]oxazin-2-one |
| 676 | 1-[1-(5-Chloro-naphthalene-1-sulfonyl)-piperidin-4-yl]-1,4-dihydro-benzo[d][1,3]oxazin-2-one |
| 677 | 1-[1-(5-Chloro-naphthalene-2-sulfonyl)-piperidin-4-yl]-1,4-dihydro-benzo[d][1,3]oxazin-2-one |
| 678 | N-{5-[4-(2-Oxo-4H-benzo[d][1,3]oxazin-1-yl)-piperidine-1-sulfonyl]-naphthalen-1-yl}-acetamide |
| 679 | 1-[1-(5-Chloro-naphthalene-1-sulfonyl)-piperidin-4-yl]-8-methyl-1,4-dihydro-benzo[d][1,3]oxazin-2-one |
| 680 | 1-[1-(5-Chloro-naphthalene-2-sulfonyl)-piperidin-4-yl]-8-methyl-1,4-dihydro-benzo[d][1,3]oxazin-2-one |
| 681 | N-{5-[4-(8-Methyl-2-oxo-4H-benzo[d][1,3]oxazin-1-yl)-piperidine-1-sulfonyl]-naphthalen-1-yl}-acetamide |
| 682 | 5-Chloro-1-[1-(5-chloro-naphthalene-1-sulfonyl)-piperidin-4-yl]-1,4-dihydro-benzo[d][1,3]oxazin-2-one |
| 683 | 5-Chloro-1-[1-(5-chloro-naphthalene-2-sulfonyl)-piperidin-4-yl]-1,4-dihydro-benzo[d][1,3]oxazin-2-one |
| 684 | N-{5-[4-(5-Chloro-2-oxo-4H-benzo[d][1,3]oxazin-1-yl)-piperidine-1-sulfonyl]-naphthalen-1-yl}-acetam ide |
| 685 | 1-[1-(5-Chloro-naphthalene-1-sulfonyl)-piperidin-4-yl]-8-methoxy-1,4-dihydro-benzo[d][1,3]oxazin-2-one |
| 686 | 1-[1-(5-Chloro-naphthalene-2-sulfonyl)-piperidin-4-yl]-8-methoxy-1,4-dihydro-benzo[d][1,3]oxazin-2-one |
| 687 | N-{5-[4-(8-Methoxy-2-oxo-4H-benzo[d][1,3]oxazin-1-yl)-piperidine-1-sulfonyl]-naphthalen-1yl}-acetamide |
| 688 | 2,5-Dimethyl-4-[4-(8-methyl-2-oxo-4H-benzo[d][1,3]oxazin-1-yl)-piperidine-1-sulfonyl]-furan-3-carboxylic acid methyl ester |
| 689 | 8-Methyl-1-[1-(2-oxo-2,3-dihydro-benzothiazole-6-sulfonyl)-piperidin-4-yl]-1,4-dihydro-benzo[d][1,3]oxazin-2-one |
| 690 | 1-[1-(4-Fluoro-3-methyl-benzenesulfonyl)-piperidin-4-yl]-8-methyl-1,4-dihydro-benzo[d][1,3]oxazin-2-one |
| 691 | 8-Methyl-1-[1-(2-oxo-2,3-dihydro-benzooxazole-6-sulfonyl)-piperidin-4-yl]-1,4-dihydro-benzo[d][1,3]oxazin-2-one |
| 692 | 1-[1-(4-Cyclohexyl-benzenesulfonyl)-piperidin-4-yl]-8-methyl-1,4-dihydro-benzo[d][1,3]oxazin-2-one |
| 693 | 2,5-Dimethyl-4-[4-(2-oxo-4H-benzo[d][1,3]oxazin-1-yl)-piperidine-1-sulfonyl]-furan-3-carboxylic acid methyl ester |
| 694 | 1-[1-(4-Fluoro-3-methyl-benzenesulfonyl)-piperidin-4-yl]-1,4-dihydro-benzo[d][1,3]oxazin-2-one |
| 695 | 1-[1-(2-Oxo-2,3-dihydro-benzooxazole-6-sulfonyl)-piperidin-4-yl]-1,4-dihydro-benzo[d][1,3]oxazin-2-one |
| 696 | 1-[1-(4-Cyclohexyl-benzenesulfonyl)-piperidin-4-yl]-1,4-dihydro-benzo[d][1,3]oxazin-2-one |
| 697 | 2-Fluoro-5-[4-(8-methyl-2-oxo-4H-benzo[d][1,3]oxazin-1-yl)-piperidine-1-sulfonyl]-benzoic acid |
| 698 | 2-Fluoro-5-[4-(2-oxo-4H-benzo[d][1,3]oxazin-1-yl)-piperidine-1-sulfonyl]-benzoic acid |
| 699 | 1-[1-(2-Oxo-2,3-dihydro-benzothiazole-6-sulfonyl)-piperidin-4-yl]-1,4-dihydro-benzo[d][1,3]oxazin-2-one |
| 700 | 1-[1-(5-Pyridin-2-yl-thiophene-2-sulfonyl)-piperidin-4-yl]-1,4-dihydro -benzo[d][1,3]oxazin-2-one |
| 701 | 3-[4-(2-Oxo-4H-benzo[d][1,3]oxazin-1-yl)-piperidine-1-sulfonyl]-benzonitrile |
| 702 | 3-[4-(2-Oxo-4H-benzo[d][1,3]oxazin-1-yl)-piperidine-1-sulfonyl]-thiophene-2-carboxylic acid methyl ester |
| 703 | 1-{5-[4-(2-Oxo-4H-benzo[d][1,3]oxazin-1-yl)-piperidine-1-sulfonyl]-naphthalen-1-yl}-pyrrolidine-2,5-dione |
| 704 | 1-[1-(2-Chloro-5-trifluoromethyl-benzenesulfonyl)-piperidin-4-yl]-1,4-dihydro-benzo[d][1,3]oxazin-2-one |
| 705 | 1-[1-(3,4-Dimethyl-benzenesulfonyl)-piperidin-4-yl]-1,4-dihydro-benzo[d][1,3]oxazin-2-one |
| 706 | 8-Methyl-1-[1-(5-pyridin-2-yl-thiophene-2-sulfonyl)-piperidin-4-yl]-1,4-dihydro-benzo[d][1,3]oxazin-2-one |
| 707 | 3-[4-(8-Methyl-2-oxo-4H-benzo[d][1,3]oxazin-1-yl)-piperidine-1-sulfonyl]-benzonitrile |
| 708 | 3-[4-(8-Methyl-2-oxo-4H-benzo[d][1,3]oxazin-1-yl)-piperidine-1-sulfonyl]-thiophene-2-carboxylic acid methyl ester |
| 709 | 1-{5-[4-(8-Methyl-2-oxo-4H-benzo[d][1-3]oxazin-1-yl)-piperidine-1-sulfonyl]-naphthalen-1-yl} pyrrolidine-2,5-dione |
| 710 | 1-[1-(2-Chloro-5-trifluoromethyl-benzenesulfonyl)-piperidin-4-yl]-8-methyl-1,4-dihydro-benzo[d][1,3]oxazin-2-one |
| 711 | 1-[1-(3,4-Dimethyl-benzenesulfonyl)-piperidin-4-yl]-8-methyl-1,4-dihydro-benzo[d][1,3]oxazin-2-one |
| 712 | 5-Chloro-1-[1-(5-pyridin-2-yl-thiophene-2-sulfonyl)-piperidin-4-yl]-1,4-dihydro-benzo[d][1,3]oxazin-2-one |
| 713 | 3-[4-(5-Chloro-2-oxo-4H-benzo[d][1,3]oxazin-1-yl)-piperidine-1-sulfonyl]-benzonitrile |
| 714 | 3-[4-(5-Chloro-2-oxo-4H-benzo[d][1,3]oxazin-1-yl)-piperidine-1-sulfonyl]-thiophene-2-carboxylic acid methyl ester |
| 715 | 1-{5-[4-(5-Chloro-2-oxo-4H-benzo[d][1,3]oxazin-1-yl)-piperidine-1-sulfonyl]-naphthalen-1-yl}-pyrrolidine-2,5-dione |
| 716 | 5-Chloro-1-[1-(2-chloro-5-trifluoromethyl-benzenesulfonyl)-piperidin-4-yl]-1,4-dihydro-benzo[d][1,3]oxazin-2-one |
| 717 | 5-Chloro-1-[1-(3,4-dimethyl-benzenesulfonyl)-piperidin-4-yl]-1,4-dihydro-benzo[d][1,3]oxazin-2-one |
| 718 | 6-Methyl-1-[1-(5-pyridin-2-yl-thiophene-2-sulfonyl)-piperidin-4-yl]-1,4-dihydro-benzo[d][1,3]oxazin-2-one |
| 719 | 3-[4-(6-Methyl-2-oxo-4H-benzo[d][1,3]oxazin-1-yl)-piperidine-1-sulfonyl]-benzonitrile |
| 720 | 3-[4-(6-Methy)-2-oxo-4H-benzo[d][1,3]oxazin-1-yl)-piperidine-1-sulfonyl]-thiophene-2-carboxylic acid methyl ester |
| 721 | 1-{5-[4-(6-Methyl-2-oxo-4H-benzo[d][1,3]oxazin-1-yl)-piperidine-1-sulfonyl]-naphthalen-1-yl}-pyrrolidine-2,5-dione |
| 722 | 1-[1-(2-Chloro-5-trifluoromethyl-benzenesulfonyl)-piperidin-4-yl]-6-methyl-1,4-dihydro-benzo[d][1,3]oxazin-2-one |
| 723 | 1-[1-(3,4-Dimethyl-benzenesulfonyl)-piperidin-4-yl]-6-methyl-1,4-dihydro-benzo[d][1,3]oxazin-2-one |
| 724 | 6-Chloro-1-[1-(5-pyridin-2-yl-thiophene-2-sulfonyl)-piperidin-4-yl]-1,4-dihydro-benzo[d][1,3]oxazin-2-one |
| 725 | 3-[4-(6-Chloro-2-oxo-4H-benzo[d][1,3]oxazin-1-yl)-piperidine-1-sulfonyl]-benzonitrile |
| 726 | 3-[4-(6-Chloro-2-oxo-4H-benzo[d][1,3]oxazin-1-yl)-piperidine-1-sulfonyll-thiophene-2-carboxylic acid methyl ester |
| 727 | 1-{5-[4-(6-Chloro-2-oxo-4H-benzo[d][1,3]oxazin-1-yl)-piperid ine-1-sulfonyl]-naphthalen-1-yl}-pyrrolidine-2,5-dione |
| 728 | 6-Chloro-1-[1-(2-chloro-5-trifluoromethyl-benzenesulfonyl)-piperidin-4-yl]-1,4-dihydro-benzo[d][1, 3]oxazin-2-one |
| 729 | 6-Chloro-1-[1-(3,4-dimethyl-benzenesulfonyl)-piperidin-4-yl]-1,4-dihydro-benzo[d][1,3]oxazin-2-one |
| 730 | 1-[1-(5-Methyl-isoxazole-4-sulfonyl)-piperidin-4-yl]-1,4-dihydro-benzo[d][1,3]oxazin-2-one |
| 731 | 1-[1-(2,2-Dimethyl-chroman-6-sulfonyl)-piperidin-4-yl]-1,4-dihydro-benzo[d][1,3]oxazin-2-one |
| 732 | 1-[1-(4-Methyl-3,4-dihydro-2H-benzo[1,4]oxazine-7-sulfonyl)-piperidin-4-yl]-1,4-dihydro-benzo[d][1,3]oxazin-2-one |
| 733 | 1-[1-(2,3-Dihydro-benzo[1,4]dioxine-6-sulfonyl)-piperidin-4-yl]-1,4-dihydro-benzo[d][1,3]oxazin-2-one |
| 734 | 1-[1-(1,3,5-Trimethyl-1H-pyrazole-4-sulfonyl)-piperidin-4-yl]-1,4-dihydro-benzo[d][1,3]oxazin-2-one |
| 735 | 1-[1-(3-Methyl-2-oxo-2,3-dihydro-benzooxazole-6-sulfonyl)-piperidin-4-yl]-1,4-dihydro-benzo[d][1,3]oxazin-2-one |
| 736 | 8-Methyl-1-[1-(5-methyl-isoxazole-4-sulfonyl)-piperidin-4-yl]-1,4-dihydro-benzo[d][1,3]oxazin-2-one |
| 737 | 1-[1-(2,2-Dimethyl-chroman-6-sulfonyl)-piperidin-4-yl]-8-methyl-1,4-dihydro-benzo[d][1,3]oxazin-2-one |
| 738 | 8-Methyl-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 739 | 1-[1-(2,3-Dihydro-benzo[1,4]dioxine-6-sulfonyl)-piperidin-4-yl]-8-methyl-1,4-dihydro-benzo[d][1,3]oxazin-2-one |
| 740 | 8-Methyl-1-[1-(1,3,5-trimethyl-1H-pyrazole-4-sulfonyl)-piperidin-4-yl]-1,4-dihydro-benzo[d][1,3]oxazin-2-one |
| 741 | 8-Methyl-1-[1-(3-methyl-2-oxo-2,3-dihydro-benzooxazole-6-sulfonyl)-piperidin-4-yl]-1,4-dihydro-benzo[d][1,3]oxazin-2-one |
| 742 | 8-Methoxy-1-[1-(1,3,5-trimethyl-1H-pyrazole-4-sulfonyl)-piperidin-4-yl]-1,4-dihydro-benzo[d][1,3]oxazin-2-one. |
| 743 | 8-Methoxy-1-[1-(3-methyl-2-oxo-2,3-dihydro-benzooxazole-6-sulfonyl)-piperidin-4-yl]-1,4-dihydro-benzo[d][1,3]oxazin-2-one |
| 744 | 1-[1-(Benzo[d]isoxazol-3-ylmethanesulfonyl)-piperidin-4-yl]-1,4-dihydro-benzo[d][1,3]oxazin-2-one |
| 745 | 1-[1-(2,2,4,6,7-Pentamethyl-2,3-dihydro-benzofuran-5-sulfonyl)-piperidin-4-yl]-1,4-dihydro-benzo[d][1,3]oxazin-2-one |
| 746 | 6-Methyl-5-[4-(2-oxo-4H-benzo[d][1,3]oxazin-1-yl)-piperidine-1-sulfonyl]-1H-pyrimidine-2,4-dione |
| 747 | 1-[1-(3-Methyl-quinoline-8-sulfonyl)-piperidin-4-yl]-1,4-dihydro-benzo[d][1,3]oxazin-2-one |
| 748 | 1-[1-(2,2,5,7,8-Pentamethyl-chroman-6-sulfonyl)-piperidin-4-yl]-1,4-dihydro-benzo[d][1,3]oxazin-2-one |
| 749 | 1,4-Dimethyl-6-[4-(2-oxo-4H-benzo[d][1,3]oxazin-1-yl)piperidine-1-sulfonyl]-1,4-dihydro-quinoxaline-2,3-dione |
| 750 | 1-[1-(1H-Imidazole-4-sulfonyl)-piperidin-4-yl]-1,4-dihydro-benzo[d][1,3]oxazin-2-one |
| 751 | 1-[1-(2-Oxo-1,2,3,4-tetrahydro-quinoline-6-sulfonyl)-piperidin-4-yl]-1,4-dihydro-benzo[d][1,3]oxazin-2-one |
| 752 | 7-[4-(2-Oxo-4H-benzo[d][1,3]oxazin-1-yl)-piperidine-1-sulfonyl]-1,5-dihydro-benzo[b][1,4]diazepine-2,4-dione |
| 753 | 8-Methyl-1-[1-(3-methyl-quinoline-8-sulfonyl)-piperidin-4-yl]-1,4-dihydro-benzo[d][1,3]oxazin-2-one |
| 754 | 6-Chloro-1-[1-(3-methyl-quinoline-8-sulfonyl)-piperidin-4-yl]-1,4-dihydro-benzo[d][1,3]oxazin-2-one |
| 755 | 5-Chloro-1-[1-(3-methyl-quinoline-8-sulfonyl)-piperidin-4-yl]-1,4-dihydro-benzo[d][1,3]oxazin-2-one |
| 756 | 8-Methoxy-1-[1-(3-methyl-quinoline-8-sulfonyl)-piperidin-4-yl]-1,4-dihydro-benzo[d][1,3]oxazin-2-one |
| 757 | 1-[1-(Pyridine-2-sulfonyl)-piperidin-4-yl]-1,4-dihydro-benzo[d][1,3]oxazin-2-one |
| 758 | 1-[1-(6,7-Dihydroxy-naphthalene-1-sulfonyl)-piperidin-4-yl]-1,4-dihydro-benzo[d][1,3]oxazin-2-one |
| 759 | Acetic acid 3-acetoxy-5-[4-(2-oxo-4H-benzo[d][1,3]oxazin-1-yl)-piperidine-1-sulfonyl]-naphthalen-2-yl ester |
| 760 | 1-[1-(1H-Benzoimidazole-2-sulfonyl)-piperidin-4-yl]-1,4-dihydro-benzo[d][1,3]oxazin-2-one |
| 761 | 1-[1-(1H-Benzoimidazole-2-sulfonyl)-piperidin-4-yl]-8-methoxy-1,4-dihydro-benzo[d][1,3]oxazin-2-one |
| 762 | 1-[1-(1H-Benzoimidazole-2-sulfonyl)-piperidin-4-yl]-5-chloro-1,4-dihydro-benzo[d][1,3]oxazin-2-one |
| 763 | 1-[1-(2,5-Dimethoxy-benzenesulfonyl)-piperidin-4-yl]-6-fluoro-1,4-dihydro-benzo[d][1,3]oxazin-2-one |
| 764 | 1-[1-(2,5-Dimethoxy-benzenesulfonyl)-piperidin-4-yl]-8-methoxy-1,4-dihydro-benzo[d][1,3]oxazin-2-one |
| 765 | 1-[1-(2,5-Dimethoxy-benzenesulfonyl)-piperidin-4-yl]-6,7-difluoro-1,4-dihydro-benzo[d][1,3]oxazin-2-one |
| 766 | 5-Chloro-1-[1-(2,5-dimethoxy-benzenesulfonyl)-piperidin-4-yl]-1,4-dihydro-benzo[d][1,3]oxazin-2-one |
| 767 | 1-[1-(5-Dimethylamino-naphthalene-1-sulfonyl)-piperidin-4-yl]-8-methoxy-1,4-dihydro-benzo[d][1,3]oxazin-2-one |
| 768 | 5-Chloro-1-[1-(5-dimethylamino-naphthalene-1-sulfonyl)-piperidin-4-yl]-1,4-dihydro-benzo[d][1,3]oxazin-2-one |
| 769 | 6-Chloro-1-[1-(5-chloro-naphthalene-1-sulfonyl)-piperidin-4-yl]-1,4-dihydro-benzo[d][1,3]oxazin-2-one |
| 770 | 1-[1-(5-Chloro-naphthalene-1-sulfonyl)-piperidin-4-yl]-6-methyl-1,4-dihydro-benzo[d][1,3]oxazin-2-one |
| 771 | 1-[1-(5-Chloro-naphthalene-1-sulfonyl)-piperidin-4-yl]-6-fluoro-1,4-dihydro-benzo[d][1,3]oxazin-2-one |
| 772 | 6-Chloro-1-[1-(5-chloro-naphthalene-2-sulfonyl)-piperidin-4-yl]-1,4-dihydro-benzo[d][1,3]oxazin-2-one |
| 773 | 1-[1-(5-Chloro-naphthalene-2-sulfonyl)-piperidin-4-yl]-6-methyl-1,4-dihydro-benzo[d][1,3]oxazin-2-one |
| 774 | 1-[1-(5-Chloro-naphthalene-2-sulfonyl)-piperidin-4-yl]-6-fluoro-1,4-dihydro-benzo[d][1,3]oxazin-2-one |
| 775 | 6-Methyl-1-[1-(3-methyl-quinoline-8-sulfonyl)-piperidin-4-yl]-1,4-dihydro-benzo[d][1,3]oxazin-2-one |
| 776 | 6-Fluoro-1-[1-(3-methyl-quinoline-8-sulfonyl)-piperidin-4-yi]-1,4-dihydro-benzo[d][1,3]oxazin-2-one |
| 777 | 6,7-Difluoro-1-[1-(3-methyl-quinoline-8-sulfonyl)-piperidin-4-yl]-1,4-dihydro-benzo[d][1,3]oxazin-2-one |
| 778 | 6-Chloro-1-[1-(3-methyl-2-oxo-2,3-dihydro-benzooxazole-6-sulfonyl)-piperidin-4-yl]-1,4-dihydro-benzo[d][1,3]oxazin-2-one |
| 779 | 6-Methyl-1-[1-(3-methyl-2-oxo-2,3-dihydro-benzooxazole-6-sulfonyl)-piperidin-4-yl]-1,4-dihydro-benzo[d][1,3]oxazin-2-one |
| 780 | 6-Fluoro-1-[1-(3-methyl-2-oxo-2,3-dihydro-benzooxazole-6-sulfonyl)-piperidin-4-yl]-1,4-dihydro-benzo[d][1,3]oxazin-2-one |
| 781 | 6,7-Difluoro-1-[1-(3-methyl-2-oxo-2,3-dihydro-benzooxazole-6-sulfonyl)-piperidin-4-yl]-1,4-dihydro-benzo[d][1,3]oxazin-2-one |
| 782 | 5-Chloro-1-[1-(3-methyl-2-oxo-2,3-dihydro-benzooxazole-6-sulfonyl)-piperidin-4-yl]-1,4-dihydro-benzo[d][1,3]oxazin-2-one |
| 783 | 6-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 784 | 6-Methyl-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 785 | 6-Fluoro-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 786 | 8-Methoxy-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 787 | 5-Chloro-1-[1-(4-methyl-3,4-dihydro-2H-benzo[1,4]oxazine-7-sulfonylppiperidin-4-yl]-1,4-dihydro-benzo[d][1,3]oxazin-2-one. |

Preferably, the compound 1-[[(7,7-dimethyl-2-oxobicyclo[2.2.1]hept-1-yl)methyl]sulfonyl)-4-(2-oxo-2H-3,1-benzoxazin-1(4H)-yl-piperidine may be excluded. More preferably, the compound 1-[[(7,7-dimethyl-2-oxobicyclo[2.2.1]hept-1-yl)methyl]sulfonyl]-4-(2-oxo-2H-3,1-benzoxazin-1(4H)-yl-piperidine, salts thereof and solvates thereof may be excluded.

The compounds of general formula (Ib), their stereoisomers, corresponding salts and corresponding solvates may be obtained analogously by the methods described above for compounds of general.formula (I). For details of the process reference is made to the description given above for compounds of general formula (I).

Thus, in another aspect the present invention relates to a process for the preparation of benzoxazinone-derived sulfonamide compounds of general formula (Ib) given above, which comprises reacting at least one piperidine compound of general formula (IIb), wherein R^{1b} to R^{9b} have the meaning given above and/or a salt, preferably a hydrochloride salt, thereof, 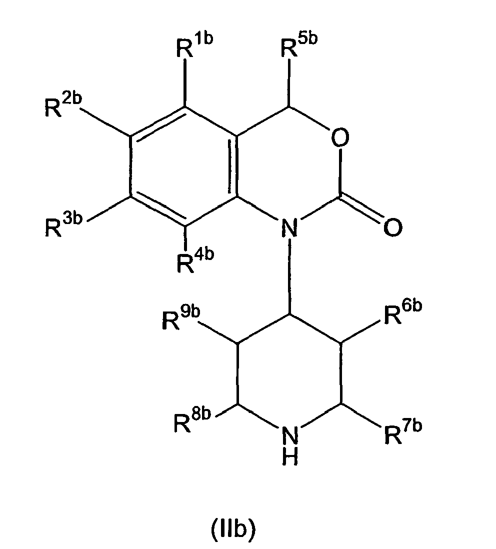 with at least one compound of general formula (IIIb), 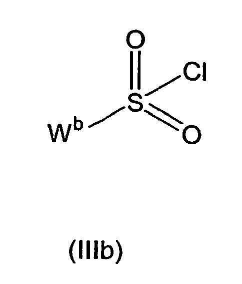 wherein W^{b} has the meaning given above, in a suitable reaction medium, optionally in the presence of at least one base and/or at least one auxiliary agent.

In a further aspect the present invention relates to a process for the preparation of a physiologically acceptable salt of the benzoxazinone-derived sulphonamide compounds of general formula (Ib) given above or a corresponding stereoisomer thereof, characterized in that at least one compound of general formula (Ib) having at least one basic group is reacted with at least one acid, preferably an inorganic or organic acid, preferably in the presence of a suitable reaction medium.

In a further aspect the present invention relates to a process for the preparation of a physiologically acceptable salt of the benzoxazinone-derived sulphonamide compounds of general formula (Ib) given above or a corresponding stereoisomer thereof, characterized in that at least one compound of general formula (Ib) having at least one acidic group is reacted with at least one base, preferably in the presence of a suitable reaction medium.

In yet another aspect the present invention relates to a pharmaceutical composition comprising at least one benzoxazinone-derived sulphonamide compound of general formula (Ib) given above, optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and optionally one or more pharmaceutically acceptable adjuvants.

In yet another aspect the present invention provides for a medicament comprising at least one benzoxazinone-derived sulphonamide compound of general formula (Ib) given above, optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and optionally one or more pharmaceutically acceptable adjuvants.

The medicament of the present invention is suitable for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment type II diabetes (non-insulin dependent diabetes mellitus).

Also in particular, the medicament of the present invention is suitable for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment cognitive disorders, preferably memory disorders; for improvement of cognition (for cognitive enhancement); for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

Another aspect of the present invention

is the use of at least one benzoxazinone-denved sulphonamide compound of general formula (Ib) given above, optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, for the manufacture of a medicament for the prophylaxis and/or treatment of food intake disorders, preferably for the regulation of appetite, for the reduction, increase or maintenance of body weight; for the prophylaxis and/or treatment of obesity, for the prophylaxis and/or treatment of bulimia, for the prophylaxis and/or treatment of anorexia; for the prophylaxis and/or treatment of cachexia; or for the prophylaxis and/or treatment of type II diabetes.

Preferred is the use of at least one benzoxazinone-derived sulphonamide compound of general formula (Ib) given above, optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, for the manufacture of a medicament for the prophylaxis and/or treatment of of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment cognitive disorders, preferably memory disorders; for improvement of cognition (for cognitive enhancement); for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia; or for the prophylaxis and/or treatment hyperactivity disorder (ADHD, attention deficit, hyperactivity disorder).

The medicament according to the present invention may be in any form suitable for the application to humans and/or animals, particularly mammals, including humans, and can be produced by standard procedures known to those skilled in the art. The composition of the medicament may vary depending on the route of administration.

The preparation of corresponding pharmaceutical compositions as well as of the formulated medicaments may be carried out by conventional methods known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective literature descriptions are incorporated by reference and are part of the disclosure.

The pharmaceutical compositions as well as the formulated medicaments prepared according to the present invention may in addition to at least one compound of general formula (I), (Ia) or (Ib), optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate, comprise further conventional auxiliary substances known to those skilled in the art, such as carriers, fillers, solvents, diluents, colouring agents, coating agents, matrix agents and/or binders. As is also known to those skilled in the art, the choice of the auxiliary substances and the amounts thereof to be used are dependent on the intended route of administration, e.g. oral, rectal, intravenous, intraperitoneal, intramuscular, intranasal, buccal or topical route.

Medicaments suitable for oral administration are for example, tablets, sugar-coated pills, capsules or multiparticulates, such as granules or pellets, optionally compressed into tablets, filled into capsules or suspended in a suitable liquid, solutions or suspensions.

Medicaments suitable for parenteral, topical or inhalatory administration may preferably be selected from the group consisting of solutions, suspensions, readily reconstitutable dry preparations and also sprays.

Suitable medicaments, e.g. medicaments for oral or percutaneous use may release the compounds of general formula (I), (Ia) or (Ib) and/or in each case stereoisomers thereof in a delayed manner, whereby the preparation of these delayed release medicaments is generally known to those skilled in the art.

Suitable delayed-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

The medicament of the present invention may also have at least one enteric coating which dissolves as a function of pH. Because of this coating, the medicament can pass through the stomach undissolved and the compounds of general formula (I), (Ia) or (Ib) are only released in the intestinal tract. The enteric coating preferably dissolves at a pH of between 5 and 7.5. Suitable materials and methods for the preparation of enteric coatings are also known to those skilled in the art

The compositions of the present invention may also be administered topically or via a suppository.

The above mentioned compositions include preferably 1 to 60 % by weight of one or more of the benzoxazinone-derived sulphonamide compounds of general formulas (I), (Ia) or (Ib), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, sex, weight or degree of illness and so forth. The daily dosage for humans usally ranges from 1 to 2000, preferably 1 to 1500, more preferably 1 to 1000 milligrams of substace to be administered during one or several intakes.

### Pharmacological Methods:

### BINDING TO SEROTONIN RECEPTOR 5HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytriptamine-7 Receptors. The Journal of Pharmacology and Experimental Therapeutics, 1994, 268, 1403] with the following slight changes. The respective part of the literature description is hereby incorporated by reference and forms part of the disclosure.
The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, which is hereby incorporated by reference and forms part of the disclosure.

### FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the inventively used sulphonamide derivatives of general formula (I) on food intake in fasted rats is then determined as follows:

The rats were fasted for 23 hours in their single homecages. After this period, the rats are orally or intraperitoneally dosed with a composition comprising a sulphonamide derivative of general formula (I) or a corresponding composition (vehicle) without said sulphonamide derivative. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and of Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples:

The intermediates of general formulas (II) and (III) were prepared by means of conventional organic chemistry methods known to those skilled in the art. The following example A shows the preparation of an intermediate of general formula (II):

### Example A:

### Synthesis of an intermediate compound of general formula (II)

### Preparation of 6-Chloro-1-(piperidine-4-yl)-1,4-dihydro-2H-3,1-benzoxazin-2-one hydrochloride

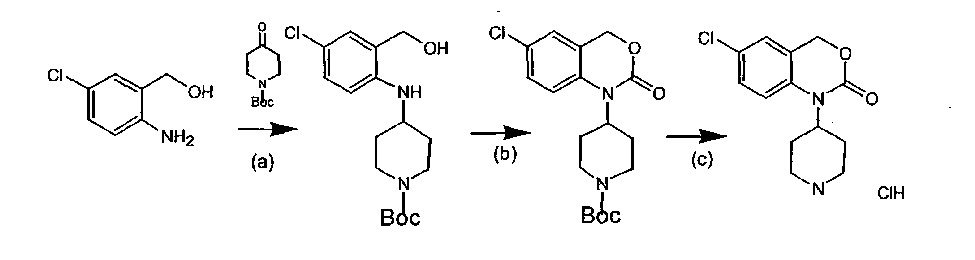

### a) 1-(tert-Butyloxycarbonyl)-4-[4-chloro-(2-hydroxymethylphenylamine)] piperidine

A solution of 1-(terl-butyloxycarbonyl)-4-piperidinone (20 g, 0.10 mol), 2-amino-5-chlorobenzylic alcohol (17.34 g, 0.11 mol) and acetic acid (14 mL, 0.22 mol) in dry toluene (500 mL) was heated at reflux temperature, with water elimination by means of azeotrope distillation with Dean-Stark, for 6 hours. The mixture was then cooled and vacuum concentrated up to half volume. NaBH₃CN (20 g, 0.32 mol) and dry THF (300 mL) were added to the resulting solution. Acetic acid (10 mL, 0.17 mol) was then dripped for one hour. The reaction was stirred at room temperature for 24 hours. The mixture was vacuum concentrated and the residue was dissolved in ethyl acetate (750 mL), washed with a NaHCO₃-saturated solution (4 x 250 mL) and a NaCl-saturated solution (250 mL), dried and evaporated to dryness. The residue was purified by means of flash chromatography eluting with a mixture of ethyl acetate: petroleum ether (1:3). The desired product was thus obtained as an oil (32.7 g, 96%). ¹H NMR (CDCl₃): 1.32 (d, *J=*11.2 Hz, 2H), 1.41 (s, 9H), 1.92 (d, *J*=11.2 Hz, 2H), 2.92 (t, *J*=12.0 Hz, 1H), 3.10 (s, 1H), 3.37 (m, 1H), 3.88 (d, *J*= 13.7 Hz, 2H), 4.49 (s, 2H), 4.75 (s, 1H), 6.52 (d, *J*= 8.6 Hz, 1H), 6.96 (s, 1H), 7.07 (d, *J*= 8.6 Hz, 1H).

### b.) 1-(1-tert-Butyloxycarbonyl-4-piperidinyl)-6-chloro-1,4-dihydro-2H-3,1-benzoxazin-2-one

N, N-diisopropylethylamine (DIEA) (43 mL, 0.25 mol) and triphosgene (8.65 g, 29.2 mmol) were added to a solution of 1-(*tert*-Butyloxycarbonyl)-4-[(4-chloro-(2-hydroxymethyl) phenyl-amino)]piperidine (27.0 g, 79 mmol) in dry THF (250 mL) cooled at 0°C. The reaction was stirred at 0°C for 1 h and at room temperature for 72 h. Ethyl ether was added and the mixture was cooled at 0°C for 3 h and the DIEA hydrochloride was then filtered. The filtered solution was evaporated to dryness and the residue was dissolved in ethyl acetate (750 mL), washed with 5% solution of critic acid (2 x 500 mL), water (250 mL) and NaHCO₃ saturated solution (2 x 500 mL). The ethyl acetate solution was dried (MgSO₄), filtered and evaporated under reduced pressure. The residue was brought to the boil with ethyl ether until the whole solid was dissolved and then cooled overnight to yield the desired compound in crystalline form (28.9 g, 67%). Melting point: 177-179 °C
¹H NMR (CDCl₃): 1.46 (s, 9H), 1.79 (d, *J*= 10.1 Hz, 1H), 2.54 (m, 2H), 2.78 (m, 2H), 3.96 (m, 1H), 4.28 (m, 2H), 5.02 (s, 2H), 6.98 (d, *J*= 8.7 Hz, 1H) 7.13 (d, *J*= 2.4 Hz, 1H), 7.28 (dd, *J*= 8.7 Hz, *J*= 2.4 Hz, 1H).

### c.) 6-chloro-1-(piperidin-4-yl)-1,4-dihydro-2H-3,1-benzoxazin-2-one hydrochloride

A solution of 1-[(1-*tert*-Butyloxycarbonyl)-4-piperidinyl]-6-chloro-1,4-dihydro-2H-3,1-benzoxazin-2-one (24 g, 65 mmol) in ethyl acetate (500 mL) was cooled at 0°C. A 5 M solution of hydrogen chloride in ethyl ether (500 mL) was then added and the resulting mixture was stirred at 0°C for 4 h. The precipitate formed was collected by filtration, washed with ether and vacuum dried to yield the desired product as a solid (16.95 g, 97%).
Melting point: 254-257 °C
¹H NMR (CD₃OD) 2.13 (d, *J*= 12.2 Hz, 2H), 2.88 (m, 2H), 3.20 (m, 2H), 3.53 (d, *J*= 12.8 Hz, 2H), 4.24 (m, 1H), 5.16 (s, 2H), 7.31 (m, 2H), 7.41 (dd, *J*= 8.8 Hz, *J*= 2.6 Hz, 1H).

Several substituted 3,1-benzoxazin-2-one compounds were prepared via the respectively substituted benzyl alcohols by reducing the respectively substituted anthranilic acids with lithium aluminium hydride and other known reducing agents by methods well known to those skilled in the art (see scheme 1), e.g. por ejemplo 6-methyl-1-(piperidin-4-yl)-1,4-dihydro-2H-3,1-benzoxazin-2-one, 7-methyl-1-(piperidin-4-yl)-1,4-dihydro-2H-3,1-benzoxazin-2-one, 8-methyl-1-(piperidin-4-yl)-1,4-dihydro-2H-3,1-benzoxazin-2-one, 5-methoxy-1-(piperidin-4-yl)-1,4-dihydro-2H-3,1-benzoxazin-2-one, 6-fluoro-1-(piperidin-4-yl)-1,4-dihydro-2H-3,1-benzoxazin-2-one, 8-methoxy-1-(piperidinyl)-1,4-dihydro-2H-3,1-benzoxazin-2-one, 5-methyl-1-(piperidin-4-yl)-1,4-dihydro-2H-3,1-benzoxazin-2-one, 7-fluoro-1-(piperidin-4-yl)-1,4-dihydro-2H-3,1-benzoxazin-2-one, 5-fluoro-1-(piperidin-4-yl)-1,4-dihydro-2H-3,1-benzoxazin-2-one, 6-methoxy-1-(piperidinyl)-1,4-dihydro-2H-3,1-benzoxazin-2-one, 5-chloro-1-(piperidinyl)-1,4-dihydro-2H-3,1-benzoxazin-2-one, 7-chloro-1-(piperidinyl)-1,4-dihydro-2H-3,1-benzoxazin-2-one, 8-chloro-1-(piperidinyl)-1,4-dihydro-2H-3,1-benzoxazin-2-one and others. The reaction of the respective 5-methoxy-1-(piperidinyl)-1,4-dihydro-2H-3,1-benzoxazin-2-one, 8-methoxy-1-(piperidinyl)-1,4-dihydro-2H-3,1-benzoxazin-2-one and 6-methoxy-1-(piperidinyl)-1,4-dihydro-2H-3,1-benzoxazin-2-one compounds according to conventional methods, e.g. BBr₃ in an inert organic solvent yields the respective 5-hydroxy-1-(piperidinyl)-1,4-dihydro-2H-3,1-benzoxazin-2-one, 8-hydroxy-1-(piperidinyl)-1,4-dihydro-2H-3,1-benzoxazin-2-one and 6-hydroxy-1-(piperidinyl)-1,4-dihydro-2H-3,1-benzoxazin-2-one compounds. The unsubstituted benzoxazin-2-one 1-(piperidin-4-yl)-1,4-dihydro-2H-3,1-benzoxazin-2-one is prepared according the method described in J. Med. Chem. 1995, 38, 4634 and J.Med.Chem. 1998, 41, 2146, which are hereby incorporated by reference and form part of the disclosure.

The substituted anthranilic acids were reduced by conventional methods known to those skilled in the art, e.g. by the use of LiAlH₄ as reducing agent in anhydrous THF under an inert-gas atmosphere, e.g. argon or nitrogen. This process is very efficient and in most cases the respective 2-aminobenzylalcohols are obtained in very good yields.

General instruction for the reduction of substituted anthranilic acids:

To a three neck flask, equipped with a mechanical stirrer and an inlet for gaseous nitrogen, 100 mL anhydrous THF and 116,6 mmoles of LiAlH₄ were given and the resulting suspension cooled to 0 °C. After the addition of 58,3 mmoles of the respective substituted anthranilic acid in 150 mL anhydrous THF, the resulting reaction mixture is warmed to room temperature and stirred or about an hour. Under cooling to 0° C 4,7 mL water, 4,7 mL NaOH 15 wt.%, and finally 14 mL water are carefully added to the mixture. The resulting suspension is filtered and washed with ethylacetate.

The organic phase is washed with water, dried and the solvent evaporated. In most cases the resulting product may be used without further purification.

### Example 5:

### Preparation of 1-[1-quinoline-8-sulfonyl)-piperidine-4-yl]-1,4-dihydro-benzo[d][1,3]oxazin-2-one

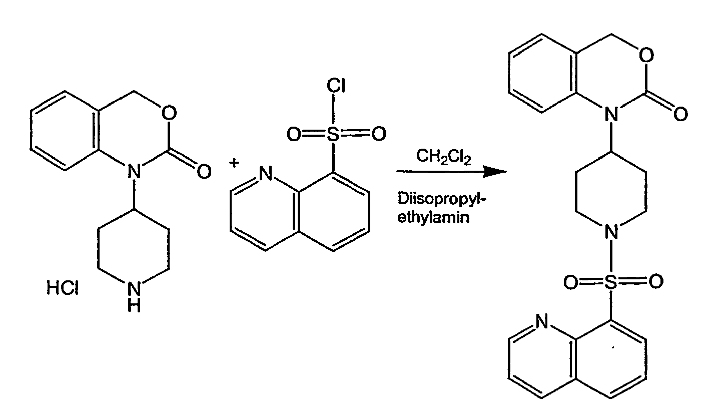

150 mg (0,66 mmol) quinoline-8-sulfonyl chloride are added to a mixture of 1-(4-piperidinyl)-1,4-dihydro-2H-3,1-benzoxazinone hydrochloride (161 mg, 0,60 mmol) and diisopropylethylamin (230 mg, 1,80 mmol) in dichloromethane (10 ml) and the resulting reaction mixture was stirred overnight at room temperature. The reaction mixture was then washed with water (3 x 15 mL) and the organic phase was separated, dryed and evaporated to dryness. A solid was obtained, which was recrystallized from ethanol. 182 mg of 1-[1-quinoline-8-sulfonyl)-piperidine-4-yl]-1,4-dihydro-benzo[d][1,3]oxazin-2-one were obtained as a white solid (yield 69 %).
IR (cm⁻¹) KBr: 1712, 1337, 1291, 1205, 1162, 1144, 1034, 717, 583
¹H-NMR(δ in ppm): 1.8 (d, *J*=9.5 Hz, 2 H) 2.6 (qd, *J*=12.6, 4.4 Hz, 2 H) 3.0 (td, *J*=12.8, 2.5 Hz, 2 H) 4.1 (tt, *J*=12.5, 3.8 Hz, 1 H) 4.3 (ddd, *J*=13.0, 2.3 Hz, 2 H) 5.0 (s, 2 H) 7.1 (m, 3 H) 7.3 (m, 1 H) 7.6 (dd, *J*=8.4, 4.2 Hz, 1 H) 7.6 (m, 1 H) 8.1 (dd, *J*=8.2, 1.3 Hz, 1 H) 8.3 (dd, *J*=8.3, 1.7 Hz, 1 H) 8.5 (dd, *J*=7.3, 1.5 Hz, 1 H) 9.1 (dd, *J*=4.2, 1.8 Hz, 1 H) (CDCI3-d).
Melting point: 170-172 °C.

The compounds according to examples 1-4 and 6-10 given in the following table I as well as the compounds according to lists A and B given above were prepared analogously to the methods described above.

**Table I:**

| | | |
|---|---|---|
| Ex. 1 | 1-[1-(Naphthyl-1-sulfonyl)-piperidine-4-yl]-1,4-dihydro-benzo[d][1,3]oxazine-2-one | |
| | 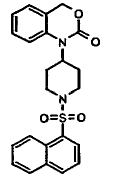 | 1H-NMR: 1.8 (d, *J*=10.5 Hz, 2 H) 2.4 (m, 2 H) 2.7 (t, *J*=11.6 Hz, 2 H) 3.9 (m,3H)5.1 (s,2H)7.1 (m, 2 H) 7.2 (m, 2 H) 7.7 (m, 3 H) 8.1 (d, *J*=8.1 Hz, 1 H) 8.2 (d, *J*=7.8 Hz, 1 H) 8.3 (d, *J*=8.1 Hz, 1 H) 8.7 (d, *J*=8.3 Hz, 1 H) (DMSO-d6) |
| | | IR (KBr) 1709, 1498, 1353, 1162, 1034, 770, 718, 579 |
| | | Melting point: 147-149°C |
| Ex. 2 | 1-(1-Phenylsulfonyl-piperidine-4-yl]-1,4-dihydro-benzo[d][1,3]oxazine-2-one | |
| | 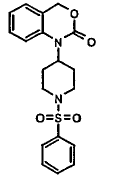 | 1H-NMR: 1.9 (dd, *J*=12.1, 2.1 Hz, 2 H) 2.4 (td, *J*=12.2, 2.4 Hz, 2 H) 2.7 (qd, *J*=12.6, 4.3 Hz, 2 H) 3.9 (tt, *J*=12.3, 3.9 Hz, 1 H) 4.0 (dt, *J*=11.9, 2.1 Hz, 2 H) 5.0 (s, 2 H) 7.0 (d, *J*=8.3 Hz, 1 H) 7.0 (t, *J*=7.3 Hz, 1 H) 7.1 (m, 1 H) 7.3 (m, 1 H) 7.6 (m, 3 H) 7.8 (m, 2 H) (CDCl₃-d) |
| | | IR (KBr) 1705, 1497, 1340, 1293, 1205, 1160, 736, 691, 576 |
| | | Melting point: 172-174°C |
| Ex. 3 | 1-[1-(5-chloro-3-methyl-benzo[b]tiophene-2-sulfonyl)-piperidine-4-yl]-1,4-dihydro-benzo[d][1,3]oxazine-2-one | |
| | 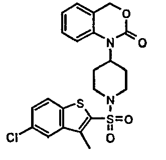 | 1H-NMR: 1.8 (d, *J*=10.8 Hz, 2 H) 2.5 (m, 2 H) 2.7 (s, 3 H) 2.8 (t, *J*=11.4 Hz, 2 H) 3.8 (d, *J*=11.4 Hz, 2 H) 3.9 (m, 1 H) 5.1 (s, 2 H) 7.0 (t, *J*=7.2 Hz, 1 H) 7.2 (d, *J*=8.1 Hz, 1 H) 7.2 (m, 2 H) 7.6 (dd, *J*=8.6, 2.0 Hz, 1 H) 8.1 (d, *J*=2.0 Hz, 1 H) 8.2 (d, *J*=8.6 Hz, 1 H) (DMSO-d6) |
| | | IR (KBr) 1717, 1358, 1248, 1201, 1160, 1035, 712, 554 |
| | | Melting point: 204-206°C |
| Ex. 4 | 8-Methyl-1-[1-(naphthyl-1-sulfonyl)-piperidine-4-yl]-1,4-dihydro-benzo[d][1,3]oxazine-2-one | |
| | 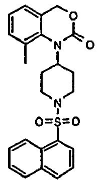 | 1H-NMR: 1.9 (d, *J*=12.5 Hz, 2 H) 2.3 (s, 3 H) 2.7 (m, 4 H) 3.3 (m, 1 H) 4.0 (d, *J*=11.2 Hz, 2 H) 4.9 (s, 2 H) 7.0 (m, 2 H) 7.1 (d, *J*=7.0 Hz, 1 H) 7.6 (m, 3 H) 7.9 (m, 1 H) 8.1 (d, *J*=8.2 Hz, 1 H) 8.2 (dd, *J*=7.3, 1.1 Hz, 1 H) 8.7 (d, *J*=8.8 Hz, 1 H) (CDCl3-d) |
| | | IR (KBr) 1712, 1316, 1279, 1222, 1160, 1135, 1025, 768, 607 |
| | | Melting point: 203-204°C |
| Ex. 5 | 1-[1-(Quinolinyl-8-sulfonyl)-piperidine-4-yl]-1,4-dihydro-benzo[d][1,3]oxazine-2-one | |
| | 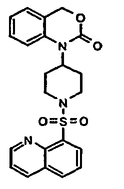 | 1H-NMR: 1.8 (d, *J*=9.5 Hz, 2 H) 2.6 (qd, *J*=12.6, 4.4 Hz, 2 H) 3.0 (td, *J*=12.8, 2.5 Hz, 2 H) 4.1 (tt, *J*=12.5, 3.8 Hz, 1 H) 4.3 (ddd, *J*=13.0, 2.3 Hz, 2 H) 5.0 (s, 2 H) 7.1 (m, 3 H) 7.3 (m, 1 H) 7.6 (dd, *J*=8.4, 4.2 Hz, 1 H) 7.6 (m, 1 H) 8.1 (dd, *J*=8.2, 1.3 Hz, 1 H) 8.3 (dd, *J*=8.3, 1.7 Hz, 1 H) 8.5 (dd, *J*=7.3, 1.5 Hz, 1 H) 9.1 (dd, *J*=4.2, 1.8 Hz, 1H) (CDCl3-d) |
| | | IR (KBr) 1712, 1337, 1291, 1205, 1162, 1144, 1034, 717, 583 |
| | | Melting point: 170-172°C |
| Ex. 6 | 8-Methyl-1-[1-(quinolinyl-8-sulfonyl)-piperidine-4-yl]-1,4-dihydro-benzo[d][1,3]oxazine-2-one | |
| | 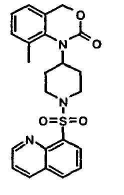 | 1H-NMR: 1.9 (d, *J*=12.6 Hz, 2 H) 2.3 (s, 3 H) 2.7 (qd, *J*=12.2, 3.9 Hz, 2 H) 2.9 (m, 2 H) 3.3 (tt, *J*=11.7, 3.4 Hz, 1 H) 4.3 (d, *J*=12.8 Hz, 2 H) 4.9 (s, 2 H) 7.0 (m, 2 H) 7.1 (d, *J*=7.3 Hz, 1 H) 7.5 (dd, *J*=8.3, 4.1 Hz, 1 H) 7.6 (m, 1 H) 8.0 (dd, *J*=8.2, 1.3 Hz, 1 H) 8.2 (dd, *J*=8.3, 1.7 Hz, 1 H) 8.5 (dd, *J*=7.3, 1.5 Hz, 1 H) 9.1 (dd, *J*=4.2,1.8 Hz, 1 H) (CDCl3-d) |
| | | IR (KBr) 1702, 1329, 1284, 1218, 1024, 785, 701, 582 |
| | | Melting point: 202-206°C |
| Ex. 7 | 1-[1-(5-Dimethylamino-naphthyl-1-sulfonyl)-piperidine-4-yl]-8-Methyl-1,4-dihydro-benzo[d][1,3]oxazine-2-one | |
| | 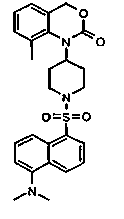 | 1H-NMR: 1.9 (d, *J*=11.9 Hz, 2 H) 2.3 (s, 3 H) 2.7 (m, 4 H) 2.9 (s, 6 H) 3.3 (m, 1 H) 4.0 (d, *J*=9.9 Hz, 2 H) 4.9 (s, 2 H) 7.0 (m, 2 H) 7.2 (m, *J*=7.3 Hz, 2 H) 7.5 (m, 2 H) 8.2 (dd, *J*=7.3, 1.1 Hz, 1 H) 8.4 (d, *J*=8.6 Hz, 1 H) 8.6 (d, *J*=8.4 Hz, 1 H) (CDCI3-d) |
| | | IR (KBr) 2981, 1711, 1336, 1221, 1149, 1025, 794, 709, 571 |
| | | Melting point: 202-203°C |
| Ex. 8 | 1-[1-(5-Dimethylamino-naphthyl-l-sulfonyl)-piperidine-4-yl]-1,4-dihydro-benzo[d][1,3]oxazine-2-one | |
| | 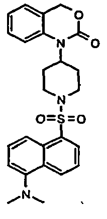 | 1H-NMR 1.8 (dd, *J*=12.3, 3.5 Hz, 2 H) 2.7 (m, 4 H) 2.9 (s, 6 H) 4.0 (m, 3 H) 5.0 (s, 2 H) 6.9 (d, *J*=8.2 Hz, 1 H) 7.1 (m, 2 H) 7.3 (m, 2 H) 7.6 (td, *J*=8.9, 7.4 Hz, 2 H) 8.3 (dd, *J*=7.3, 1.3 Hz, 1 H) 8.4 (d, *J*=8.8 Hz, 1 H) 8.6 (d, *J*=8.2 Hz, 1 H) (CDCI3-d) |
| | | IR (KBr) 2935, 1720, 1319, 1242, 1144, 920, 791, 755, 642 |
| | | Melting point: 182-186°C |
| Ex. 9 | 1-[1-(2,3-Dichloro-phenylsulfonyl)-piperidine-4-yl]-1,4-dihydro-benzo[d][1,3]oxazine-2-one | |
| | 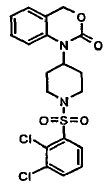 | 1H-NMR 1.9 (d, *J*=10.1 Hz, 2 H) 2.7 (qd, *J*=12.6, 4.2 Hz, 2 H) 3.0 (td, *J*=12.7, 2.3 Hz, 2 H) 4.1 (m, 3 H) 5.1 (s, 2 H) 7.1 (m, 3 H) 7.3 (m, 2 H) 7.7 (dd, *J*=8.0, 1.6 Hz, 1 H) 8.0 (dd, *J*=8.0, 1.6 Hz, 1H) (CDCl3-d) |
| | | IR (KBr) 1697,1395,1244,1165,1045,942,710,582 |
| | | Melting point: 185-187 °C |
| Ex. 10 | 1-[1-(2,3-Dichloro-phenylsulfonyl)-piperidine-4-yl]-8-Methyl-1,4-dihydro-benzo[d][1,3]oxazine-2-one | |
| | 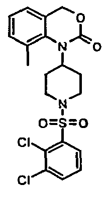 | 1H-NMR: 2.0 (d, *J*=11.5 Hz, 2 H) 2.4 (s, 3 H) 2.8 (m, 4 H) 3.4 (m, 1 H) 4.0 (d, *J*=9.9 Hz, 2 H) 5.0 (s, 2 H) 7.0 (m, 2 H) 7.2 (d, *J*=7.7 Hz, 1 H) 7.3 (t, *J*=8.0 Hz, 1 H) 7.7 (dd, *J*=8.1, 1.5 Hz, 1 H) 8.0 (dd, *J*=8.0, 1.6 Hz, 1 H) (CDCl3-d) |
| | | IR (KBr) 1705, 1404, 1339, 1224, 1149, 939 |
| | | Melting point: 184-185°C |

### Pharmacological data:

The binding of the benzoxazinone derived sulphonamide compounds of general formulas (I), (Ia) and (Ib) was determined as described above.

The binding results of some these compounds are given in the following table 2:

| **Table 2:** | | |
|---|---|---|
| | | |
| | | |
| **Compound according to example:** | **% Inhibition 10⁻⁶ M** | **Kᵢ (nM)** |
| 1 | 98,1 ± 4.0 | 51.7 |
| 3 | | 107.4 |
| 4 | | 246 |
| 5 | | 152 |
| 6 | | 165.9 |
| 7 | 88 | |
| 8 | 68 | |

## Claims

1. Benzoxazinone-derived sulfonamide compounds of general formula (I) 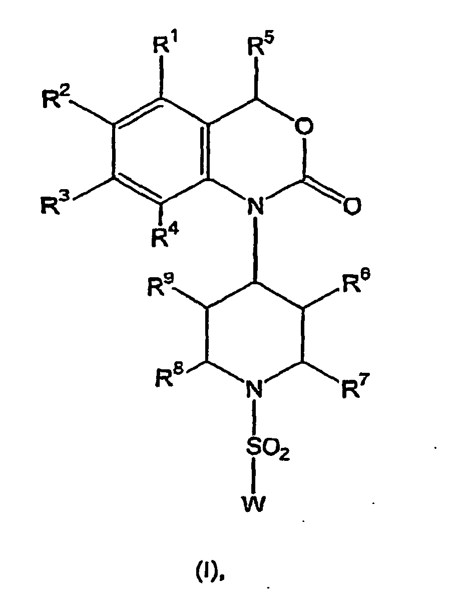 wherein
R¹, R², R³, R⁴ are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, -OR¹⁰, - OC(=O)R¹¹, -(C=O)-OR¹¹, -SR¹², -SOR¹², -SO₂R¹², -NH-SO₂R¹², -SO₂NH₂ and a -NR¹³R¹⁴ moiety,
R⁵ represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R⁶, R⁷, R⁸, R⁹ are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a -COOR¹⁵ moiety,
W represents an unbranched or branched, saturated or unsaturated aliphatic radical, which may be substituted by one or more substituents selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, - SO-C₁₋₄-alkyl, -SO₂-C₁₋₄-alkyl, -NH-SO₂-C₁₋₄-alkyl, wherein the C₁₋₄-alkyl may in each case be branched or unbranched, an unsubstituted or at least mono-substituted phenyl or naphthyl radical and an unsubstituted or at least mono-substituted furanyl, thienyl, pyrrolyl, imidazolyl, pyrazolyl, pyridinyl, pyrimidinyl, quinolinyl and isoquinolinyl radical,
whereby said substituents may be at least mono-substituted with F, Cl, methyl and methoxy,
a saturated or unsaturated, optionally at least one heteroatom as ring member containing cycloaliphatic radical, whereby said cycloaliphatic radical may be substituted by one or more substituents selected from the group consisting of hydroxy, nitro, carboy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆-alkenyl, SO₂-C₁₋₄-alkyl, -(C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅alkyl, -C₁₋₅-alkylen-(C=O)-C₁₋₆-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl, -NH-phenyl, and -C₁₋₄-Alkylen-NH-(C=O)-phenyl. whereby said substituents may be substituted by one or more substituents selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, - C(=O)-O-C₁₋₅-alkyl, -(C=O)-CH₂-F, -(C=O)-CH₂-Cl and -(C=O)-CH₂-Br, and whereby said cycloaliphatic radical may be bonded via an optionally mono-substituted alkylene group and/or may be condensed with an optionally at least mono-substituted aromatic mono- or polycyclic ring-system,
an optionally at least mono-substituted heteroaryl radical, which may be bonded via an optionally mono-substituted alkylene group and/or may be condensed with an optionally at least mono-substituted mono- or polycyclic ring-system,
an optionally at least mono-substituted, monocyclic aryl radical, which is condensed with an optionally at least mono-substituted mono- or polycyclic ring-system and which may be bonded via an optionally at least mono-substituted alkylene group,
a -NR¹⁶R¹⁷-moiety,
a -COR¹⁸-moiety,
or a phenyl radical, which is at least mono-substituted with one of the substituents selected from the group consisting of:
2,2,2, Trifluoroethoxy-, C₂₋₆Alkenyl-, 1,3-Dihydro-1-oxo-2H-isoindol-2-yl-. N-Phthalimidinyl-, [(2-chloro-1,3-thiazolyl-5-yl)-methoxy, Ethyl-5-yl-2-methyl-3-furvate, C₁₁₋₂₀-alkyl-, 1,3-Dioxo-2-azaspiro[4,4]non-2-yl-, pyrazolyl-, (1,3-oxazol-5-yl)-, (5-Methyl-1,3,4-oxadiazol-2-yl)-, difluoromethoxy, dichlommethoxy, 1-pyrrolidinytsulfonyl, morpholinosulfonyl, 2-methyl-4-pyrimidinyl-, a phenoxy group, which is at least mono-substituted with C₁₋₅-alkoxy, a phenyl group, which is at least mono-substituted with one of the substituents selected from the group consisting of nitro, C₁₋₅-alkoxy, F, Cl, Br, at least partially fluorinated C₁₋₅-alkyl, at least partially chlorinated C₁₋₅-alkyl, [(2-Chloro-1,3-thiazol-5-yl)-methoxy]-, -(C=O)-H and -(C=O)-C₁₋₅-alkyl, a pyridinyl group, which is at least mono-substituted with C₁₋₅-alkoxy, a pyridinyloxy group, which is at least mono-substituted with C₁₋₅-alkoxy, a phenoxy group, which is at least di-substituted and a pyridinyloxy group, which is at least di-subsituted,
with the proviso that W does not represent unsubstituted furyl-, unsubstituted thienyl- or thienyl substituted with a substituent selected from the group consisting of C₁₋₅-alkoxycarbonyl, C₁₋₅-alkylcarbonyl, carboxyl and pyridyl, unsubstituted pyrrolyl-, unsubstituted naphthyl, unsubstituted indolyl, unsubstituted tetrahydronaphthyl, substituted or unsubstituted pyridyl, unsubstituted pyrazinyl, unsubstituted quinolinyl-, C₁₋₅-alkylsubstituted pyrrolyl-, and unsubstituted cyclohexyl or cyclohexyl substituted with one or two members selected from the group consisting of oxo, hydroxyl, C₁₋₅-alkoxyl, C₁₋₅-alkoxy-carbonylamino-C₁₋₅ alkyl and amino-C₁₋₅ alkyl,
R¹⁰ represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹¹ represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹² represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹³ and R¹⁴ each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R¹³ and R¹⁴ together with the bridging nitrogen atom form a saturated, unsaturated or aromatic heterocyclic ring, which may be at least mono-substituted and/or contain at least one further heteroatom as a ring member,
R¹⁵ represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R¹⁶ represents an unbranched or branched, saturated or unsaturated, optionally at least mono-substituted aliphatic radical,
R¹⁷ represents an unbranched or branched, saturated or unsaturated, optionally at least mono-substituted aliphatic radical, and
R¹⁸ represents an optionally at least mono-substituted aryl radical,
optionally in form of one of its stereoisomers, preferably enantiomers or diastereomers, its racemate or in form of a mixture of at least two of its stereoisomers, preferably enantiomers or diastereomers, in any mixing ratio, or a corresponding salt thereof, or a corresponding solvate.

2. Compounds according to claim 1, **characterized in that** R¹, R², R³, R⁴ are each independently selected from the group consisting of H. F, Cl. Br, an unbranched or branched, saturated or unsaturated, optionally at least mono-substituted C₁₋₆aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₈-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano, -OR¹⁰, - OC(=O)R¹¹, -SR¹², -SOR¹², -SO₂R¹², -NH-SO₂R¹², -SO₂NH₂ and a -NR¹³R¹⁴ moiety, preferably selected from the group consisting of H, F, Cl. Br, a saturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro, cyano. -OR¹⁰, -OC(=O)R¹¹, -SR¹² and -NF¹³R¹⁴ moiety, more preferably selected from the group consisting of H, F, Cl, -CH₃,-CH₂CH₃, -CF₃, -CF₂CF₃, cyclopentyl, cyclohexyl, a nitro group, a cyano group and -OR¹⁰.

3. Compounds according to claim 1 or 2, **characterized in that** R⁵ represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₆-cycloaliphatic radical, preferably represents H or a branched or unbranched C₁₋₃-alkyl radical, more preferably represents H, -CH₃ or -CH₂CH₃.

4. Compounds according to any one of claims 1 to 3, **characterized in that** R⁶, R⁷, R⁸, R⁹ are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano group and a -COOR¹⁵ moiety, preferably selected from the group consisting of H, a branched or unbranched C₁₋₃-alkyl radical, a cyano group and a -COOR¹⁵ group, more preferably from the group consisting of H, -CH₃, -CH₂CH₃ and a cyano moiety.

5. Compounds according to any one of claims 1 to 4, **characterized in that** W represents an unbranched or branched C₁₁₋₂₀-alkyl radical, which may be substituted by one or more substitutents selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, - SO₂-C₁₋₄-alkyl, -NH-SO₂-C₁₋₄-alkyl , wherein the C₁₋₄-alkyl may in each case be branched or unbranched, an unsubstituted or at least mono-substituted phenyl or naphthyl radical and an unsubstituted or at least mono-substituted furanyl, thienyl, pyrrolyl, imidazolyl, pyrazolyl, pyridinyl, pyrimidinyl, quinolinyl and isoquinolinyl radical, whereby said substituents may be at least mono-substituted with F, Cl, methyl and methoxy; a napthyl group, which is at least mono substituted, a quinolinyl group, which is at least mono-substituted, a pyrrolyl group, which is at least mono-substituted by a substituent other than C₁₋₅-alkyl, an optionally at least mono-substituted thiazolyl-, benzo[b]-thiophenyl-, benzo[b]-furanyl-, isoquinolinyl-, tetrahydroisoquinolinyl-, pyrazolyl-, isoazolyl-, chromanyl-, benzothiadiazolyl-, imidazolyl-, benzofurazanyl-, dibenzo[b,d]-furanyl-, benzoxadiazolyl-, imidazo[2,1-b]-thiazolyl-, anthracenyl-, coumarinyl-, 2,3-Dihydro-1,4-benzodioxinyl-, 2,3-Dihydrobenzo[b]furanyl-, 3,4-Dihydro-2H-1,4-Benzoxazinyl-, 3,4-Dihydro-2H-1,5-Benzodioxepinyl-, Benzothiazolyl-, Imidazo[1,2-a]-pyridinyl-, a chromonylgroup, an isatinyl group, a pentamethyldihydrobenzofuranyl group, a cyclopropyl- or cyclopentyl-group whereby said cyclopropyl or cyclopentyl group may be substituted by one or more substituents selected selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₈-alkenyl, SO₂-C₁₋₄-alkyl, -(C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl, -NH-phenyl, and -C₁₋₄-Alkylen-NH-(C=O)-phenyl, whereby said substituents may be substituted by one or more substituents selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, - C(=O)-O-C₁₋₅-alkyl, -(C=O)-CH₂-F, -(C=O)-CH₂-Cl and -(C=O)-CH₂-Br, a 2-(1,3-Dioxo-1,3-dihydro-isoindol-2-yl-)-ethyl, a thienyl group, which is at least mono-substituted by one or more substituents independently selected from the group consisting of F, Cl, Br, C₁₋₅-alkoxy-, CF₃, -SO₂-C₁₋₅-alkyl and optionally at least mono substituted benzoylaminomethyl-, phenylsulfonyl-, isoxazolyl-, benzamidomethyl-, pyrimidyl-, thiazolyl-, pyrazolyl-, phenyl-, 1,2,4-thiadiazolyl-. 1,3-oxazolyl,- or 1,2,4-oxadiazolyl-, a furyl group, which is at least mono-substituted by one or more subsitutents independently selected from the group consisting of a C₁₋₅-alkyl radical, which may be at least partially fluorinated or chlorinated, an optionally at least mono-substituted phenyl and a -(C=O)-O-C₁₋₅-alkyl group,
a NR¹⁶R¹⁷-moiety,
a COR¹⁸-moiety,
or a phenyl radical, which is at least mono-substituted with one of the substituents selected from the group consisting of:
2,2,2,-Trifluoroethoxy-, C₂₋₆-Alkenyl-; 1,3-Dihydro-1-oxo-2H-isoindol-2-yl-, N-Phthalimidinyl-, [(2-chloro-1,3-thiazolyl-5-yl)-methoxy, Ethyl-5-yl-2-methyl-3-furoate, C₁₁₋₂₀-alkyl-, 1,3-Dioxo-2-azaspiro[4,4]non-2-yl-, pyrazolyl-, (1,3-oxazol-5-yl)-, (5-Methyl-1,3,4-oxadiaz-ol-2-yl)-, difluoromethoxy, dichloromethoxy, 1-pyrrolidinylsulfonyl, morpholinosulfonyl, 2-methyl-4-pyrimidinyl-, a phenoxy group, which is at least mono-substituted with C₁₋₅-alkoxy, a phenyl group, which is at least mono-substituted with one of the substituents selected from the group consisting of nitro, C₁₋₅-alkoxy, F, Cl, Br, at least partially fluorinated C₁₋₅-alkyl, at least partially chlorinated C₁₋₅-alkyl, [(2-Chloro-1,3-thiazol-5-yl)-methoxyl-, -(C=O)-H and -(C=O)-C₁₋₅-alkyl, a pyridinyl group, which is at least mono-substituted with C₁₋₅-alkoxy, a pyridinyloxy group, which is at least mono-substituted with C₁₋₅-alkoxy, a phenoxy group, which is at least di-substituted and a pyridinyloxy group, which is at least di-subsituted,
more preferably W represents a moiety selected from the group consisting of 5-Dimethylamino-napth-1-yl, 2-Acetamido-4-methyl-5-thiazolyl-, Trifluoromethyl-, Trichloromethyl-, Isopropyl-, Methyl-, 2,2,2-Trifluoroethyl-, Ethyl-, Hexadecyl-, 2-Chloroethyl-, n-Propyl-, 3-Chloro-propyl-, n-Butyl-, Dichloromethyl-, Chloromethyl-, Dodecyl-, 1-Octyl-, 6-(p-toluidino)-naphth-2-yl-, , 4,5-Dibromo-thiophene-2-yl-, Benzoylchloride-3-yl-, 1-Octadecyl-, 4-Bromo-2,5-dichloro-thiophene-3-yl-, 2,5-Dichloro-thiophene-3-yl-, 5-Chloro-thiophene-2-yl-, 1-Decyl-, 3,5-Dichloro-4-(2-chloro-4-nitrophenoxy)-phenyl-, 2,3-Dichlorothiophene-5-yl-, 3-Bromo-2-chloro-thiophene-5-yl-, 3-Bromo-5-chlorothiophene-2-yl-, 2-(Benzoylaminomethyl)-thiophene-5-yl-, 4-(Phenylsulphonyl)-thiophene-2-yl-, 2-Phenyl-sulphonyl-thiophene-5-yl-, 2-[1-Methyl-5-(trifluoromethyl)pyrazol-3-yl]-thiophene-5-yl-, 5-Chloro-1,3-dimethylpyrazole-4-yl-, 3,5-Dimethylisoxazole-4-yl-, 2-(2,4-Dichlorophenoxy)-phenyl, 4-(2-Chloro-6-nitro-phenoxy)-phenyl-, 4-(3-chloro-2-cyanophenoxy)-phenyl, 2,4-Dimethyl-1,3-thiazole-5-yl-, Methyl-methane-sulfonyl-, 2,5-Bis-(2,2,2-Trifluoroethoxy)-phenyl-, 5-(Di-n-pmpylamino)-naphth-1-yl-, 2,2,5,7,8-Pentamethyl-chroman-6-yl-, 5-Chloro-4-nitro-thiophene-2-yl-, 2,1,3-Benzothiadiazole-4-yl-, 1-Methyl-imidazole-4-yl-, Benzofurazan-4-yl-, 5-(Isoxazol-3-yl)thiophene-2-yl-, Vinyl-phenyl-4-yl-, 5-Dichloro-methyl-furan-2-yl-, 5-Bromo-thiophene-2-yl-, 5-(4-Chlorobenzamidomethyl)-thiophene-2-yl-, Dibenzo[b,d]-furan-2-yl-, 5-Chloro-3-methylbenzo[b]-thiophens-2-yl-, 3-Methoxy-4-(methoxycarbonyl)-thiophene-2-yl-, 5-[2-(Methylthio)-pyrimidin-4-yl]-thiophene-2-yl-, 4-Chloro-2,1,3-Benzoxadiazole-7-yl-, 5-Chloro-2,1,3-Benzoxadiazole-4-yl-, 6-Chloro-imidazo(2,1-b)-thiazole-5-yl-, 3-Methylbenzo[b]-thiophene-2-yl-, 4-[[3-Chloro-5-(Trifluoromethyl)-2-pyridyl]oxy-phenyl-, 5-Chloro-naphth-1-yl-, 5-Chloro-naphth-2-yl-, 9,10-Dibromoanthracene-2-yl-, Isoquinoline-5-yl-,4-Methoxy-2,3,6-trimethylbenzoyl-, 4'-Nitro-biphenyl-4-yl-, (1,3-Dihydro-1-oxo-2H-isoindol-2-yl-)-4-phenyl-, 5-(2-Methyl-1,3-thiazole-4-yl)-thiophene-2-yl-, 5-(1-Methyl-3-(trifluoromethyl)pyrazol-5-yl-]-thiophene-2-yl-, 5-[5-Trifluoromethyl)-isoxazol-3-yl]-thiophene-2-yl-, p-Dodecyl-phenyl-, 4-[(3-Cyano-4-methoxy-2-pyridinyl)oxy]-phenyl-, 4-(N-phthalimidinyl)-phenyl-, 1,2,3,4-Tetrahydro-2-(trifluoroacetyl)-isoquinoline-7-yl-, 1,2-Dimethylimidazole-4-yl-, 2,2,4,6,7-Pentamethyldihydrobenzofuran-5-yl-, 4-Chloro-naphth-1-yl-, 2,5-Dichloro-4-nitro-thiophene-3-yl-, 4-(4-Methoxy-phenoxy)-phenyl-, [4-(3,5-Dichlorophenoxy)phenyl]-,[4-(3,4-Dichlorophenoxy)phenyl]-, [4-(3,5)-Bis(trifluoromethylphenoxy)phenyl]-, 3-(2-Methoxy-phenoxy)-phenyl, 3-(4-Methoxy-phenyl)-phenyl-, 3-(4-Chloro-phenyl)-phenyl-, 3-(3,5-Dichlorophenyl-phenyl-, 3-(3,4-Dichloro-phenyl)-phenyl-, 3-(4-Fluorophenyl)-phenyl-, 3-[4-(Trifluoromethyl)-phenyl]-phenyl-, 3-[3,5-Bis-(Trifluoromethyl)-phenyl]-phenyl-, 4-(2-Methoxy-phenoxy)-phenyl-, 4-(2-Methyl-phenoxy)-phenyl-, 4-(4-Methoxy-phenoxy)-phenyl-, 4-(4-Chlorophenyl)-phenyl-, 4-(3,5-Dichlorophenyl)-phenyl-, 4'-(3,4-Dichlorophenyl)-phenyl, 4-(4-Fluorophenyl)-phenyl-, 4-[4-(Trifluormethyl)-phenyl]-phenyl-, 4-[3,5-Bis-(Trifluomethyl)-phenyl]-phenyl-, Cyclopropyl-, 2-(2-Chlorophenyl)-2-Phenylethyl-, 2-(2-Trifluoromethylphenyl)-2-phenylethyl-, 5-[4-Cyano-1-methyl-5-(methylthio)-1H-pyrazol-3-yl-thiophene-2-yl-, 3-Cyano-2,4-bis-(2,2,2-Trifluorothoxy)-phenyl-, 4-[(2-chloro-1,3-Thiazol-5-yl)-methoxy]-phenyl, 2-(1,3-Dioxo-1,3-dihydroisoindol-2-yl)-ethyl-, 5-Iodo-naphth-1-yl-, Ethyl-2,5-dimethyl-1-phenylpyrrole-4-carboxylate-3-yl-, Ethyl-2-methyl-1,5-diphenyl-1H-pyrole-3-carboxylate-4-yl-, Ethyl-5-(4-chlorophenyl)-2-methyl-3-furoate-4-yl, Ethyl-5-(4-chlorophenyl)-2-methyl-1-pheny)-3-carboxylate-4-yl-, Ethyl-2,5-dimethyl-3-furoate-4-yl-, 3-Chloro-4-(1,3-dioxo-2-Azaspiro[4,4]non-2-yl)-phenyl-, Coumarin-6-yl, 3-(4-Methoxy-phenoxy)-phenyl-, [3-(3,5-Dichlomphenoxy)]-phenyl-, 13-(3,4 Dichlorophenoxy)]-phenyl-, 3,5-Bis(Trifluoromethyl)phenoxyphenyl-, 2,2-Diphenylethyl-, 4-Phenyl-5-(trifluoromethyl)-thiophene-3-yl-, Methyl-4-Phenyl-5-(Trifluoromethyl)-thiophene-2-carboxylate-3-yl-, Methyl-1,2,5-trimethylpyrrole-3-carboxylate-4-yl-, 4-Fluoro-naphth-1-yl-, 5-Fluoro-3-methylbenzo[b]-thiophene-2-yl-, Methyl-2,5-dimethyl-3-furoate-4-yl-, Methyl-2-furoate-5-yl-, Methyl-2-methyl-3-furoate-5-yl, Methyl-1-methyl-1H-pyrrole-2-Carboxylate-5-yl-, 2-(5-Chloro-1,2,4-Thiadiazol-3-yl)-thiophene-5-yl-, 1,3,5-Trimethyl-1H-pyrazole-4-yl-, Pentafluoroethoxytetrafluoroethyl-, 5-(5-Isoxazyl)-thiophene-2-yl-, 5-(5-Isoxazol-yl)-2-furyl-, 5-Methyl-2,1,3-benzothiadiazole-4-yl-, 2,3-Dihydro-1,4-benzodioxine-6-yl-, 4-Methyl-Naphth-1-yl-, 5-Methyl-2-(Trifluormethyl)-3-Furyl-, 2,3-Dihydrobenzo[b]furan-5-yl-, 1-Benzothiophene-3-yl-, 4-Methyl-3,4-dihydro-2H-1,4-Benzoxazine-7-yl-, 5-Methyl-1-phenyl-1H-pyrazole-4-yl-, 6-Morpholino-3-Pyridinyl-, 4-(1H-Pyrazol-1-yl)-phenyl-, 6-Phenoxy-3-Pyridyl-, 3,4-Dihydro-2H-1,5-benzodioxepine-7-yl-,5-(1,3-Oxazol-5-yl)-2-thienyl-, 4-(1,3-Oxazol-5-yl)-phenyl-, 5-Methyl-4-isoxazolyl, 2,1,3-Benzothiadiazole-5-yl-, 5-Acetamido-naphth-1-yl-, 3-Methyl-8-Quinolinyl-, 1,3-Benzothiazole-6-yl-, 2-Morpholino-3-Pyridyl-, 2,5-Dimethyl-3-thienyl-, 5-[5-(Chloromethyl)-1,2,4-oxadiazol-3-yl]-2-thienyl-, Ethyl-3-[5-yl-2-thienyl-]1,2,4-oxadiazole-5-carboxylate-, 3-(5-Methyl-1,3,4-oxadiazol-2-yl)-phenyl-, 4-(Difluoromethoxy)phenyl-, 3-(Difluoromethoxy)-phenyl-, 2,2-Dimethyl-6-Chromanyl-, Ethyl-3,5-dimethyl-1H-pyrrole-2-carboxylate-4-yl-, Imidazo[1,2-A]pyridine-3-yl-, 3-(1,3-Oxazol-5-yl)-phenyl-, Ethyl-5-[4-yl)-phenyl]-2-methyl-3-furoate, 1-Pyrrolidinylphenylsulfonyl-, Methyl-5-yl-4-methyl-2-thiophenecarboxylate, Methyl-3-yl-4-(isopropylsulfonyl)-2-thiophene, 7-Chlorochromone-3-yl-, 4'-Bromobiphenyl-4-yl-, 4'-Acetyl-biphenyl-4-yl-, 4'-Bromo-2-fluorobiphenyl-4-yl-, 1-Methyl-5-isatinyl-, 2-Chloro-3-thiophenecarboxylic-acid-5-yl-, 2-Methoxy-5-(N-phthalimidinyl)-phenyl-, 1-Benzothiophene-2-yl-, Morpholinophenylsulfonyl- and 3-(2-Methyl-4-pyrimidinyl)-phenyl-

6. Compounds according to any one of claims 1 to 5, **characterized in that** R¹⁰ represents hydrogen, an unbranched or branched, saturated or unsaturated, optionally at least mono-substituted C₁₋₈aliphatic r-adical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono-or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl.

7. Compounds according to any one of claims 1 to 6, **characterized in that** R¹¹ represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono-or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl.

8. Compounds according to any one of claims 1 to 7, **characterized in that** R¹² represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloatiphatic radical, which may be bonded via an optionally at least mono-substituted C₁₋₆-alkylone group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably H, -CH₃, -CzHs or phenyl.

9. Compounds according to any one of claims 1 to 8, **characterized in that** R¹³ and R¹⁴ are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₈-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably are each independently selected from the group consisting of H, a linear or branched C₁₋₄-alkyl radical, a cyclohexyl radical and a phenyl radical, more preferably are each independently selected from the group consisting of H, -CH₃, -C₂H₅ and phenyl.

10. Compounds according to any one of claims 1 to 8, **characterized in that** R¹³ and R¹⁴ together with the bridging nitrogen atom form a saturated, unsaturated or aromatic, 5- or 6-membered heterocyclic ring, which may be at least mono-substituted and/or contain at least one further heteroatom as a ring member, preferably form an unsubstituted piperidin or morpholine group.

11. Compounds according to any one of claims 1 to 10, **characterized in that** R¹⁵ represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloallphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably represents H, -CH₃, -C₂H₅ or phenyl.

12. Compounds according to any one of claims 1 to 11, **characterized in that** R¹⁶ represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical.

13. Compounds according to any one of claims 1 to 12, **characterized in that** R¹⁷ represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical.

14. Compounds according to one or more of claims 1 to 13:
1-[1-(5-Chloro-3-methyl-benzo[b]thiophenyl-2-sulfonyl)-piperidine-4-yl]-1,4-dihydro-benzo[d][1,3]oxazin-2-one,
1-[1-(5-Dimethylamino-naphthyl-1-sulfonyl)-piperidine-4-yl]-8-methyl-1,4-dihydro-benzo[d][1,3]oxazin-2-one,
1-[1-(5-Dimethylamino-naphthyl-1-sulfonyl)-piperidine-4-yl]-1,4-dihydro-benzo[d][1,3]oxazin-2-one,
and corresponding salts thereof, and corresponding solvates.

15. Process for the preparation of benzoxazinone-derived sulfonamide compounds of general formula (I) according to one or more of claims 1 to 14, **characterized in that** it comprises reacting at least one piperidine compound of general formula (II), wherein R¹ to R⁹ have the meaning according to claim 1, and/or a salt, preferably a hydrochloride salt, thereof, 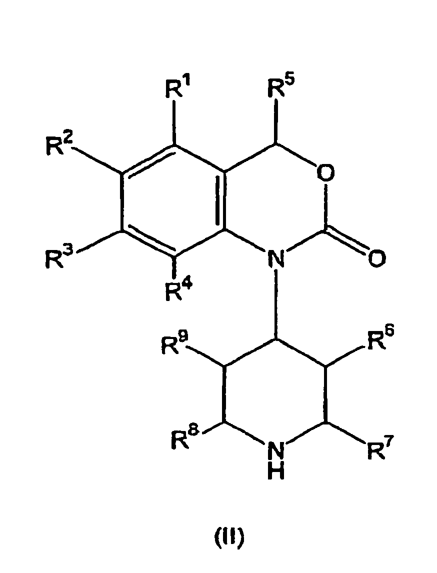 with at least one compound of general formula (III), 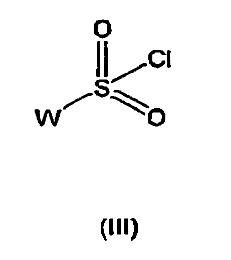 wherein W has the meaning according to claim 1, in a suitable reaction medium, optionally in the presence of at least one base and/or at least one auxiliary agent

16. Process for the preparation of a physiologically acceptable salt of the benzoxazinone-derived sulphonamide compounds according to claims 1-14, **characterized in that** at least one compound of general formula (I) having at least one basic group is reacted with at least one acid, preferably an inorganic or organic acid, preferably in the presence of a suitable reaction medium.

17. Process for the preparation of a physiologically acceptable salt of the benzoxazinone-derived sulphonamide compounds according to claims 1-14, **characterized in that** at least one compound of general formula (I) having at least one acidic group is reacted with at least one base, preferably in the presence of a suitable reaction medium.

18. Medicament comprising at least one benzoxazinone-derived sulphonamide compound according to any one of claims 1-14, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

19. Medicament according to claim 18 for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity, disorders of the central nervous system, disorders of the gastrointestinal tract, such as irritable intestine syndrom, anxiety, panic, depression, cognitive memory disorders, senile dementia disorders, such as Morbus Alzheimer, Morbus Parkinson and Morbus Huntington, schizophrenia, psychosis, infantile hyperkinesia or ADHC (attention deficit, hyperactivity disorders).

20. Use of at least one benzoxazinone-derived sulphonamide compound according to any one of claims 1-14, optionally in form of one of its stereoisomers, preferably enantiomers or diastereomers, its racemate or in form of a mixture of at least two of its stereoisomers in any mixing ratio, or a physiologically acceptable salt thereof, or a solvate, for the manufacture of a medicament for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity, disorders of the central nervous system, disorders of the gastrointestinal tract, such as irritable intestine syndrom, anxiety, panic, depression, cognitive memory disorders, senile dementia disorders, such as Morbus Alzheimer, Morbus Parkinson, and Morbus Huntington, schizophrenia, psychosis, infantile hyperkinesia or ADHC (attention deficit, hyperactivity disorders).

21. Use of at least one benzoxazinone-derived sulfonamide compound of general formula (Ia), 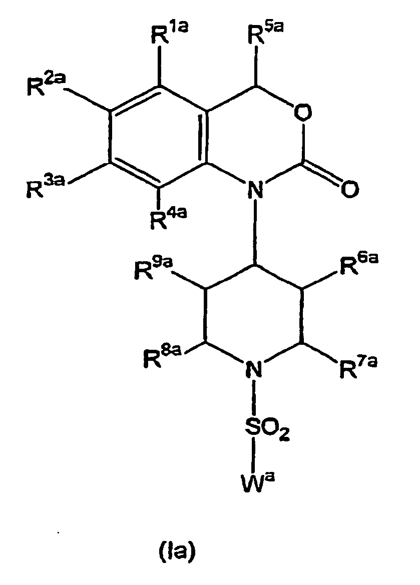 wherein
R^{1a}, R^{2a}, R^{3a}, R^{4a} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, -OR^{10a}, - OC(=O)R^{11a}, -(C=O)-OR^{11a}, -SR^{12a}, -SOR^{12a}, -SO₂R^{12a}, -NH-SO₂R^{12a},-SO₂NH₂ and a -NR^{13a}R^{14a} moiety,
R^{5a} represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R^{6a}, R^{7a}, R^{8a}, R^{9a} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a -COOR^{15a} moiety,
W^{a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ring-system, a NR^{16a}R^{17a}-moiety or a COR^{18a} moiety,
R^{10a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{12a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13a} and R^{14a} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{13a} and R^{14a} together with the bridging nitrogen atom form a saturated, unsaturated or aromatic heterocyclic ring, which may be at least mono-substituted and/or contain at least one further heteroatom as a ring member,
R^{15a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{16a} represents an unbranched or branched, saturated or unsaturated, optionally at least mono-substituted aliphatic radical,
R^{17a} represents an unbranched or branched, saturated or unsaturated, optionally at least mono-substituted aliphatic radical,
R^{18a} represents an optionally at least mono-subsituted aryl radical,
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively,
for the manufacture of a medicament for the prophylaxis and/or treatment of food intake disorders; anxiety; panic; depression; cognitive disorders: preferably memory disorders; senile dementia processes, preferably selected from the group consisiting of Morbus Alzheimer, Morbus Parkinson, Morbus Huntington; psychosis; infantile hyperkinesia,; ADHC (attention deficit/hyperacitivity disorder); disorders of the gastrointestinal tract, preferably intestine syndrom; schizophrenia or for cognitive enhancement

22. Use according to claim 21, **characterized in that** R^{1a}, R^{2a}, R^{3a}, R^{4a} are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano group, - OR^{10a}, -OC(=O)R^{11a}, -SR^{12a}, -SOR^{12a}, -SO₂R^{12a}, -NH-SO₂R^{12a}, -SO₂NH₂ and a -NR^{13a}R^{14a} moiety, preferably selected from the group consisting of H, F, Cl, Br, a saturated, branched or unbranched, optionally at least mono-substituted C₁₋₃-aliphatic radical, a saturated, optionally at least mono-substituted, optionally at least one heteroatom as ring member containing C₅- or C₈- cycloaliphatic radical, which may be bonded via an optionally at least mono-substituted C₁- or C₂-alkylene group, a nitro group, a cyano group, -OR^{10a}, - OC(=O)R^{11a}, -SR^{12a} and -NR^{13a}R^{14a} moiety, more preferably selected from the group consisting of H, F, Cl, -CH₃,-CH₂CH₃, -CF₃, -CF₂CF₃, cyclopentyl, cyclohexyl, nitro, cyano and -OR^{10a}.

23. Use according to claim 21 or 22, **characterized in that** R^{5a} represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₈-cycloaliphatic radical, preferably represents H or a branched or unbranched C₁₋₃-alkyl radical, more preferably H, -CH₃ or-CH₂CH₃.

24. Use according to any one of claims 21 to 23, **characterized in that** R^{6a}, R^{7a}, R^{8a}, R^{9a} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano group and a -COOR^{15a} moiety, preferably selected from the group consisting of H, a branched or unbranched C₁-₃-alkyl radical, a cyano group and a COOR^{15a} group, more preferably from the group consisting of H, -CH₃, -CH₂CH₃ and a cyano moiety.

25. Use according to any one of claims 21 to 24, **characterized in that** W^{a} represents an unbranched or branched, saturated or unsaturated, optionally at least mono-substituted C₁₋₂₀ aliphatic radical, a saturated or unsaturated, optionally at least mono-substituted, optionally at least one heteroatom as ring member containing C₃₋₈ cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆- alkylene group and/or may be condensed with an optionally at least mono-substituted mono- or polycyclic ring-system, a NR^{16a}R^{17a}-moiety or a COR^{18a}-moiety,
preferably is selected from the group consisting of 1-Naphthyl-, 5-Dimethylamino-napth-1-yl, 2-Naphthyl-, 2-Acetamido-4-methyl-5-thiazolyl-, 2-Thienyl-, 8-Quinolinyl-, Phenyl, Pentafluorophenyl-, 2,4,5-Trichloro-phenyl-, 2,5-Dichloro-phenyl-, 2-Nitrophenyl-, 2,4-Dinitro-phenyl-, 3,5-Dichloro-2-hydroxy-phenyl-, 2,4,6-Trisisopropyl-phenyl-, 2-Mesityl-, 3-Nitro-phenyl-, 4-Bromo-phenyl-, 4-Fluoro-phenyl-, 4-Chlorophenyl-, 4-Chloro-3-nitro-phenyl-, 4-lodo-phenyl-, N-Acetyl-sulfanilyl-, 4-Nitro-phenyl-, 4-Methoxy-phenyl-, Benzoic-acid-4-yl-, 4-tert-Butyl-phenyl-, p-Tolyl-, Trifluoromethyl-, Trichloromethyl-, Isopropyl-, Methyl-, Benzyl-, trans-styryl-, 2,2,2-Trifluoroethyl-, Ethyl-, Hexadecyl-, 2-Chloroethyl-, n-Propyl-, 3-Chloro-propyl-, n-Butyl-, Methyl-benzoate-2-yl-, 2-Nitro-4-(trifluoromethyl)-phenyl-, Pentamethyl-phenyl-, 2,3,5,6-Tetramethyl-phenyl-, 3-(Trifluoromethyl)-phenyl-, 3,5-Bis-(Trifluoromethyl)-phenyl-, Dichloromethyl-, Chloromethyl-, Dodecyl-, 1-Octyl-, 2,3,4-Trichloro-phenyl-, 2,5-Dimethoxy-phenyl-, o-Tolyl-, p-xylyl-2-yl-, Benzoic-acid-3-yl-, 4-Chloro-3-(trifluoromethyl)-phenyl-, 4-Chloro-5-nitrobenzoic acid-3-yl-, 6-(p-toluidino)-naphth-2-yl-, 4-Methoxy-2,3,6-trimethylphenyl-, 3,4-Dichlorophenyl-, 4,5-Dibromo-thiophene-2-yl-, 3-Chloro-4-fluoro-phenyl-, 4-Ethyl-phenyl-, 4-n-Propyl-phenyl-, 4-(1,1-Dimethylpropyl)-phenyl-, 4-Isopropyl-phenyl-, 4-Bromo-2,5-difluoro-phenyl-, 2-Fluoro-phenyl-, 3-Fluoro-phenyl-, 4-(Trifluoromethoxy)-phenyl-, 4-(Trifluoromethyl)-phenyl-, 2,4-Difluoro-phenyl-, 2,4-Dichloro-5-methyl-phenyl-, 4-Chloro-2,5-dimethylphenyl-, 5-Diethylamino-napth-2-yl-, Benzoyl chloride-3-yl-, 2-Chloro-phenyl-, 1-Octadecyl-, 4-Bromo-2,5-dichloro-thiophene-3-yl-, 2-5-Dichloro-thiophene-3-yl-, 5-Chloro-thiophene-2-yl-, 2-Methyl-5-nitro-phenyl-, 2-(Trifluoromethyl)-phenyl-, 3-Chloro-phenyl-, 3,5-Dichloro-phenyl-, 1-Decyl-, 3-Methyl-phenyl-, 2-Chloro-6-methyl-, 5-Bromo-2-methoxy-phenyl-, 3,4-Dimethoxy-phenyl-, 2-3-Dichloro-phenyl-, 2-Bromo-phenyl-, 3,5-Dichloro-4-(2-chloro-4-nitrophenoxy)-phenyl-, 2,3-Dichloro-thiophene-5-yl-, 3-Bromo-2-chloro-thiophene-5-yl-, 3-Bromo-5-chloro-thiophene-2-yl-, 2-(Benzoylaminomethyl)-thiophen-5-yl-, 4-(Phenyl-sulphonyl)-thiophene-2-yl-, 2-Phenyl-sulphonyl-thiophene-5-yl-, 3-Chloro-2-methyl-phenyl-, 2-[1-Methyl-5-(trifluoromethyl)pyrazol-3-yl]-thiophene-5-yl-, 5-Pyrid-2-yl-thiophene-2-yl-, 2-Chloro-5-(trifluoromethyl)-phenyl-, 2,6-Dichloro-phenyl-, 3-Bromo-phenyl-, 2-(Trifluoromethoxy)-phenyl-, 4-Cyano-phenyl-, 2-Cyano-phenyl-, 4-n-Butoxy-phenyl-, 4-Acetamido-3-chlorophenyl, 2,5-Dibromo-3,6-difluoro-phenyl-, 5-Chloro-1,3-dimethylpyrazole-4-yl-, 3,5-Dimethylisoxazole-4-yl-, 2-(2,4-Dichlorophenoxy)-phenyl-, 4-(2-Chloro-6-nitro-phenoxy)-phenyl-, 4-(3-Chloro-2-cyano-phenoxy)-phenyl-, 2,4-Dichlorophenyl-, 2,4-Dimethyl-1,3-thiazole-5-yl-, Methyl-methane-sulfonyl-, 2,5-Bis-(2,2,2-Trifluoroethoxy)-phenyl-, 2-Chloro-4-(trifluoromethyl)-phenyl-, 2-Chloro-4-fluoro-phenyl-, 5-Fluoro-2-methyl-phenyl-, 5-Chloro-2-methoxy-phenyl-, 2,4,6-Trichloro-phenyl-, 2-Hydroxy-benzoic acid-5-yl-, 5-(Di-n-propylamino)-naphth-1-yl-,6-Methoxy-m-tolyl-, 2,5-Difluoro-phenyl-, 2,4-Dimethoxy-phenyl-, 2,5-Dibromo-phenyl-, 3,4-Dibromo-phenyl-, 2,2,5,7,8-Pentamethyl-chroman-6-yl-, 2-Methoxy-benzoic-acid-5-yl-, 5-Chloro-4-nitro-thiophene-2-yl-, 2,1,3-Benzothiadiazole-4-yl-, 1-Methyl-imidazole-4-yl-, Benzofurazan-4-yl-,2-(Methoxycarbonyly-thiophene-3-yl-,5-(Isoxazol-3-yl)-thiophene-2-yl-, 2,4,5-Trifluoro-phenyl-, Biphenyl-4-yl-, Vinyl-phenyl-4-yl-, 2-Nitro-benzyl-, 5-Dichloromethyl-furan-2-yl-, 5-Bromo-thiophene-2-yl-, 5-(4-Chlorobenzamidomethyl)-thiophene-2-yl-, 2,6-Difluro-phenyl-, 2,5-Dimethoxy-4-nitro-phenyl-, Dibenzo[b,d]-furan-2-yl-, 2,3,4-Trifluoro-phenyl-, 3-Nitro-p-tolyl-, 4-Methoxy-2-nitro-phenyl-, 3,4-Difluro-phenyl-, 4-(Bromoethyl)-phenyl-, 3,5-Dichloro-4-hydroxy-phenyl-, 4-n-Amyl-phenyl-, 5-Chloro-3-methylbenzo[b]-thiophene-2-yl-, 3-Methoxy-4-(methoxycarbonyl)-thiophene-2-yl-, 4-n-Butyl-phenyl-, 2-Chloro-4-cyano-phenyl-, 5-[2-(Methylthio)-pyrimidin-4-yl-]-thiophene-2-yl-, 3,5-Dinitro-4-methoxy-phenyl-, 4-Bromo-2-(trifluoromethoxy)-phenyl-, 4-Chloro-2,1,3-Benzoxadiazole-7-yl-, 2-(1-Naphthyl)-ethyl-, 3-Cyano-phenyl-, 5-Chloro-2,1,3-Benzoxadiazole-4-yl-, 3-Chloro-4-methyl-phenyl-, 4-Bromo-2-ethyl-phenyl-, 2,4-Dichloro-6-methyl-phenyl-, 6-Chloro-imidazo(2,1-B)-thiazole-5-yl-, 3-Methyl-benzo[b]-thiophene-2-yl-, 4-Methyl-sulphonyl-phenyl-, 2-Methyl-sulphonyl-phenyl-, 4-Bromo-2-methyl-phenyl-, 2,6-Dichloro-4-(trifluoromethyl)-phenyl-, 4-[[3-Chloro-5-(trifluoromethyl)-2-pyridinyl]oxy]-phenyl-, 5-Chloronaphth-1-yl-, 5-Chloro-naphth-2-yl-, 9,10-Dibromoanthracene-2-yl-, Isoquinofine-5-yl-, 4-Methoxy-2,3,6-trimethyl-phenyl-, 4'-Nitro-biphenyl-4-yl-, [(4-Phenoxy)-phenyl-, (1,3-Dihydro-1-oxo-2H-isoindol-2-yl-)-4-phenyl-, 4-Acetyl-phenyl-, 5-(2-Methyf-1,3-thiazole-4-yl)-thiophene-2-yl-, 5-(1-Methyl-3-(trifluoromethyl)pyrazol-5-yl-]-thiophene-2-yl-,5-[5-Trifluoromethyl)-isoxazol-3-yl]-thiophene-2-yl-, 2-iodo-phenyl-, p-Dodecyl-phenyl-, 4-[(3-Cyano-4-methoxy-2-pyridinyl)oxy]-phenyl-, 4-(N-phthalimidinyl)-phenyl-, 1,2,3,4-Tetrahydro-2-(trifluoroacetyl)-isoquinoline-7-yl-, 4-Bromo-2-fluoro-phenyl-, 2-Fluoro-5-(trifluoromethyl)-phenyl-, 4-Fluoro-2-(trifluoromethyl)-phenyl-, 4-Fluoro-3-(trifluoromethyl)-phenyl-, 2,4,6-Trifluoro-phenyl-, 3-(Trifluoromethoxy)-phenyl-, 1,2-Dimethylimidazole-4-yl-, Ethyl-4-Carboxylate-3-yl-, 2,2,4,6,7-Pentamethyldihydrobenzofuran-5-yl-, 3-Bromo-2-chloropyridine-5-yl-, 3-Methoxy-phenyl-, 2-Methoxy-4-methyl-phenyl-, 2-Chloro-4-fluorobenzoic-acid-5-yl-, 4-Chloro-naphth-1-yl-, 2,5-Dichloro-4-nitrothiophene-3-yl-, 4-(4-Methoxy-phenoxy)-phenyl-, 4-(4-Chloro-phenoxy)-phenyl-, 4-(3,5-Dichloro-phenoxy)-phenyl-, 4-(3,4-Dichloro-phenoxy)phenyl-, 4-(4-Fluoro-phenoxy)-phenyl-, 4-(4-Methyl-phenoxy)-phenyl-, 4-[4-(Trifluormethyl)-phenoxy-phenyl-, 4-[3,5-Bis-(trifluoromethyl)-phenoxy]-phenyl-, 3-(2-Methoxy-phenoxy)-phenyl-, [3-(2-Chloro-phenoxy)-phenyl-, 3-(2-Methylphenoxy)-phenyl-, 4-[2-(Trifluoromethyl)-phenoxy]-phenyl-, 3-Phenyl-phenyl-, 3-(4-Methoxy-phenyl)-phenyl-, 3-(4-Chloro-phenyl)-phenyl-, 3-(3,5-Dichlorophenyl)-phenyl-, 3-(3,4-Dichloro-phenyl)-phenyl-, 3-(4-Fluorophenyl)-phenyl-, 3-(4-Methylphenyl)-phenyl-, 3-[4-(Trifluoromethyl)-phenyl-phenyl-, 3-[3,5-Bis-(Trifluoromethyl)-phenyl]-phenyl-, 4-(4-Pyridyloxy)-phenyl)-, 4-(2-Methoxy phenoxy)-phenyl-, 4-(2-Chloro-phenoxy)-phenyl-, 4-(2-Methyl-phenoxy)-phenyl-, 4-(4-Methoxy-phenoxy)-phenyl-, 4-(4-Chlorophenyl)-phenyl-, 4-(3,5-Dichlorophenyl)-phenyl-, 4-(3,4-Dichlorophenyl)-phenyl-, 4-(4-Fluorophenyl)-phenyl-, 4-(4-Methylphenyl)-phenyl-, 4-[4-(Trifluoromethyl)-phenyl]-phenyl-, 4-[3,5-Bis-(Trifluoromethyl)-phenyl]-phenyl-, [3-(Trifluoromethyl)-phenyl]-methyl-, (4-Chlorophenyl)-methyl-, (3,5-Dichlorophenyl)-methyl-, (3,5-Dichlorophenyl)-methyl-, (4-Fluorophenyl)-methyl-, 4-Methylphenylmethyl-, [4-(Trifluaromethyl)-phenyl]-methyl-, Cyclopropyl-, 2-(2-Chlorophenyl)-2-Phenylethyl-, 2-(2-Trifluoromethylphenyl)-2-phenylethyl-, 5-[4-Cyano-1-methyl-5-(methylthio)-1 H-pyrazol-3-yl-thiophene-2-yl-, 3-Cyano-2,4-bis-(2,2,2-Trifluorothoxy)-phenyl-, 4-[(2-Chloro-1,3-Thiazol-5-yl)-methoxy]-phenyl-, 3-Nitro-phenylmethyl-, 4-Formylphenyl-, 2-(1,3-Dioxo-1,3-dihydro-isoindol-2-yl)-ethyl-, [3,5-Bis-(Trifluoromethyl)-phenyl]-methyl-, (4-(2-Pyridyloxy)-phenyl)-, (4-(3-Pyridyloxy)-phenyl, 5-iodo-naphth-1-yl-, Ethyl-2,5-dimethyl-1-phenylpyrrole-4-carboxylate-3-yl-, Ethyl-2-methyl-1,5-diphenyl-1H-pyrrole-3-carboxylate-4-yl-, Ethyl-5-(4-chlorophenyl)-2-methyl-3-furoate-4-yl, Ethyl-5-(4-chlorophenyl)-2-methyl-1-phenyl-3-carboxylate-4-yl-, Ethyl-2,5-dimethyl-3-furoate-4-yl-, 3-Chloro-4-(1,3-dioxo-2-Azaspiro[4,4]non-2-yl)-phenyl-, 5-Bromo-2,4-difluorophenyl-, 5-Chloro-2,4-difluorophenyl-, Coumarin-6-yl, 2-Methoxy-phenyl, (3-Phenoxy)-phenyl-, 3-(4-Methoxy-phenoxy)-phenyl-, 3-(4-Chlorophenoxy)-phenyl-, 3-(3,5-Dichlorophenoxy)-phenyl-, 3-(3,4-Dichlorophenoxy)-phenyl-, 3-(4-Fluorophenoxy)-phenyl-, 3-(4-Methylphenoxy)-phenyl-, 3-[4-(Trifluoromethyl)-phenoxy]-phenyl-, 3-[3,5-(Trifluoromethyl)-phenoxyl-phenyl-, 3-[2-(Trifluoromethyl)-phenoxy]-phenyl-, 2,2-Diphenylethyl-, 4-Phenyl-5-(trifluoromethyl)-thiophene-3-yl-, Methyl-4-Phenyl-5-(Trifluoromethyl)-thiophene-2-carboxylate-3-yl-, Methyl-1,2,5-trimethylpyrrole-3-Carboxylate-4-yl-, 4-Fluoro-naphth-1-yl-, 3,5-Difluorophenyl-, 3-Fluoro-4-methoxy-phenyl-, 4-Chloro-2,5-difluorophenyl-, 2-Chloro-4,5-difluoro-phenyl-, 5-Fluoro-3-methylbenzo[b]-thiophene-2-yl-, Methyl-3-phenylpropionate-4-yl, Dihydrocinnamic Acid-4-yl-, Methyl-2,5-dimethyl-3-furoate-4-yl-, Methyl-2-furoate-5-yl-, Methyl-2-methyl-3-furoate-5-yl-, Methyl-1-methyl-1 H-pyrrole-2-Carboxylate-5-yl-, 2-(5-Chloro-1,2,4-Thiadiazol-3-yl)-thiophene-5-yl-, 1,3,5-Trimethyl-1 H-pyrazole-4-yl-, 3-Chloro-5-fluoro-2-methylphenyl-, Pentafluoroethoxytetrafluoroethyl-, 5-(5-Isoxazyl)-thiophene-2-yl-, 5-(5-Isoxazol-yl)-2-furyl-, 5-Methyl-2,1,3-benzothiadiazole-4-yl-, Biphenyl-2-yl-, 2,3-Dihydro-1,4-benzodioxine-6-yl-, 4-Methyl-Naphth-1-yl-, 5-Methyl-2-(Trifluormethyl)-3-Furyl-, 2,3-Dihydrobenzo[b]furan-5-yl-, 1-Benzothiophene-3-yl-, 4-Methyl-3,4-dihydro-2H-1,4-Benzoxazine-7-yl-, 5-Methyl-1-phenyl-1H-pyrazole-4-yl-, 6-Morpholino-3-Pyridinyl-, 4-(1H-Pyrazol-1-yl)-phenyl-, 6-Phenoxy-3-Pyridyl-, 3,4-Dihydro-2H-1,5-benzodioxepine-7-yl-, 5-(1,3-Oxazol-5-yl)-2-thienyl-, 4-(1,3-Oxazol-5-yl)-phenyl-, 5-Methyl-4-isoxazolyl, 2,1,3-Benzothiadiazole-5-yl-, 3-Thienyl-, 2-Methyl-benzyl-, 3-Chloro-benzyl-, 5-Acetamido-naphth-1-yl-, 3-Methyl-8-Quinolinyl-, 4-Chloro-2-nitrophenyl-, 6-Quinolinyl-, 1,3-Benzothiazole-6-yl-, 2-Morpholino-3-Pyridyl-, 2,5-Dimethyl-3-thienyl-, 5-[5-(Chloromethyl)-1,2,4-oxadiazol-3-yl]-2-thienyl-, Ethyl-3-[5-yl-2-thienyl-]1,2,4-oxadiazole-5-carboxylate-, 3-(5-Methyl-1,3,4-oxadiazol-2-yl)-phenyl-, 4-Isopropoxyphenyl-, 2,4-Dibromophenyl-, 3-Cyano-4-fluorophenyl-, 2.5-Bis-(Trifluoromethyl)-phenyl, 2-Bromo4-fluorophenyl-, 4-Bromo-3-fluorophenyl-, 4-(Difluoromethoxy)-phenyl-, 3-(Difluoromethoxy)-phenyl-, 5-Chloro-2 fluoro-phenyl-, 3-Chloro-2-fluorophenyl-, 2-Fluoro-4-methylphenyl-, 4 Nitro-3-(trifluoromethyl)-phenyl-, 3-Fluoro-4-methylphenyl-, 4-fluoro-2-methylphenyl-, 4-Bromo-3-(tifluoromethyl)-phenyl-, 4-Bromo-2-(trifluoromethyl)-phenyl-, 3-Bromo-5-(trifluoromethyl)-phenyl-, 2-Bromo-4-(trifluoromethyl)-phenyl-,2-Bromo-5-(trifluoromethyl)-phenyl-, 2,4-Dichloro-5-fluorophenyl-, 4,5-Dichloro-2-fluorophenyl-, 3,4,5-Trifluorophenyl-, 4-Chloro-2-fluorophenyl-, 2-Bromo-4,6-Difluorophenyl-, 2-Ethylphenyl-, 4-Bromo-2-chlorophenyl-, 4-Bromo-2,6-dichlorophenyl-, 2-Bromo-4,6-dichloro-phenyl-, 4-Bromo-2,6-dimethylphenyl-, 3,5-Dimethylphenyl-, 4-Bromo-3-methylphenyl-, 2-Methoxy-4-nitrophenyl-, 2,2-Dimethyl-6-Chromanyl-, Ethyl-3,5-dimethyl-1 H-pyrrole-2-carboxylate-4-yl-, Imidazo[1,2-A]pyridine-3-yl-, 3-(1,3-Oxazol-5-yl)-phenyl-, Ethyl-5-[4-yl]-phenyl]-2-methyl-3-furoate, Methyl-3-(yl)-4-methoxybenzoate, 1-Pyrrolidinylphenylsulfonyl-, Methyl-5-yl-4-methyl-2-thiophene-carboxylate, Methyl-3-yl-4-(isopropylsulfonyl)-2-thiophene, 2-Pyridyl-, 3-Fluoro-4-nitrophenyl-, 7-Chlorochromone-3-yl-, 4'-Bromobiphenyl-4-yl-, 4'-Acetyl-biphenyl-4-yl-, 4'-Bromo-2'-fluoro-biphenyl-4-yl-, 2-chloro4-(3-propyl-Ureido)-phenyl-, 3-(-Bromoacetyl)-phenyl-, 2-Bromo-3-(trifluoromethyl)-phenyl-, 1-Methyl-5-isatinyl-, 4-Isopropyl-benzoic-acid-3-yl-, 2-Chloro-3-thiophenecarboxylic-acid-5-yl-, 3-Pyridyl-, Cyclohexylmethyl-, 2-Methoxy-5-(N-phthalimidinyl)-phenyl-, 1-Benzothiophene-2-yl-, Morpholinophenylsulfonyl-, 3-(2-Methyl-4-pyrimidinyl)-phenyl-, and 2-Cyano-5-methylphenyl-.

26. Use according to any one of claims 21 to 25, **characterized in that** R^{10a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono-or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl.

27. Use according to any one of claims 21 to 26, **characterized in that** R^{11a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono-or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁-₄-alkyl radical, a cyclohexyl radical or a phenyl radical, more preferably H, -CH₃, -C₂H₅ or phenyl.

28. Use according to any one of claims 21 to 27, **characterized in that** R^{12a} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₅-alkylene group and/or may be condensed with an optionally at least mono-substituted mono- or polycyclic ring-system, preferably represents H, a linear or branched C₁-₄-alkyl radical, a cyclohexyl radical or a phenyl radical, more preferably H, -CH₃, -C₂H₅ or phenyl.

29. Use according to any one of claims 21 to 28, **characterized in that** R^{13a} and R^{14a} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably are each independently selected from the group consisting of H, a linear or branched C₁-₄-alkyl radical, cyclohexyl and a phenyl radical, more preferably are each independently selected from the group consisting of H, -CH₃, -C₂H₅ and phenyl.

30. Use according to any one of claims 21 to 29, **characterized in that** R^{13a} and R^{14a} together with the bridging nitrogen atom form a saturated, unsaturated or aromatic, 5- or 6-membered heterocyclic ring, which may be at least mono-substituted and/or contain at least one further heteroatom as a ring member, preferably form an unsubstituted piperidin or morpholine group.

31. Use according to any one of claims 21 to 30, **characterized in that** R^{15a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably represents H, -CH₃, -C₂H₅ or phenyl.

32. Use according to any one of claims 21 to 31, **characterized in that** R^{16a} represents an unbranched or branched, saturated or unsaturated, optionally at least mono-substituted C₁₋₅ aliphatic radical, preferably an unbranched or branched, saturated, unsubstituted C₁₋₃ alkyl radical, more preferably a methyl radical.

33. Use according to any one of claims 21 to 32 **characterized in that** R^{17a} represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical.

34. Use according to any one of claims 21 to 33, **characterized in that** one or more benzoxazinone-derived sulfonamide compounds of general formula (Ia) are selected from the group consisting of:
1-[1-(Naphthyl-1-sulfonyl)-piperidine-4-yl]-1,4-dihydro-benzo[d][1,3]oxazin-2-one,
1-(1-Phenylsulfonyl-piperidine-4-yl]-1,4-dihydro-benzo[d][1,3]-oxazin-2-one,
1-[1-(5-Chloro-3-methyl-benzo[b]thiophenyl-2-sulfonyl)-piperidine-4-yl]-1,4-dihydro-benzo[d][1,3]oxazin-2-one,
8-Methyl-1-[1-naphthyl-1-sulfonyl)-piperidine-4-yl]-1,4-dihydrobenzo[d][1,3]oxazin-2-one,
1-[1-(quinoline-8-sulfonyl)-piperidin-4-yl]-1,4-dihydro-benzo[d][1,3]oxazin-2-one,
8-Methyl-1-[1-(quinoline-8-sulfonyl)-piperidin-4-yl]-1,4-dihydro-benzo[d][1,3]oxazin-2-one,
1-[1-(5-Dimethylamino-naphthyl-1-sulfonyl)-piperidine-4-yl]-8-methyl-1,4-dihydrobenzo[d][1,3]oxazin-2-one,
1-[1-(5-Dimethylamino-naphthyl-1-sulfonyl)-piperidine-4-yl]-1,4-dihydro-benzo[d][1,3]oxazin-2-one,
1-[1-(2,3-Dichloro-phenylsulfonyl)-piperidine-4-yl]-1,4-dihydro-benzo[d][1,3]oxazin-2-one,
1-[1-(2,3-Dichloro-phenylsufonyl)-piperidin-4-yl]-8-methyl-1,4-dihydro-benzo[d][1,3]oxazin-2-one, and
corresponding salts thereof, or corresponding solvates thereof.

35. Use according to any one of claims 21-34 for the regulation of appetite.

36. Use according to any one of claims 21-34 for the reduction, increase or maintenance of body weight.

37. Use according to any one of claims 21-34 for the prophylaxis and/or treatment of obesity.

38. Use according to any one of claims 21-34 for the prophylaxis and/or treatment of bulimia.

39. Use according to any one of claims 21-34 for the prophylaxis and/or treatment of anorexia.

40. Use according to any one of claims 21-34 for the prophylaxis and/or treatment of cachexia.

41. Use according to any one of claims 21-34 for the prophylaxis and/or treatment of type II diabetes.

42. Benzoxazinone-derived sulfonamide compounds of general formula (Ib), 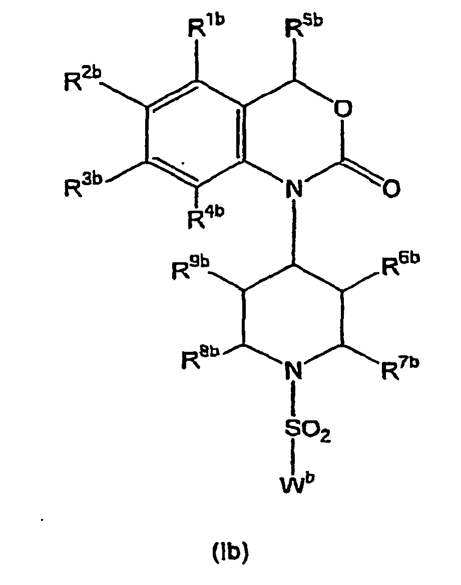 wherein
R^{1b}, R^{2b},R^{3b}, R^{4b} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, -OR^{10b},-OC(=O)R^{11b}, -(C=O)-OR^{11b}, -SR^{12b}, -SOR^{12b}, -SO₂R^{12b} -NH-SO₂R^{12b},-SO₂NH₂ and a -NR^{13b}R^{14b} moiety,
R^{5b} represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R^{6b}, R^{7b}, R^{8b}, R^{9b} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a -COOR^{15b} moiety,
W^{b} represents an unbranched or branched, saturated or unsaturated, aliphatic radical, which may be substituted by one or more substituents selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl,-SO-C₁₋₄-alkyl, -SO₂-C₁₋₄-alkyl, -NH-SO₂-C₁₋₄-alkyl , wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical,
whereby said substituents may be at least mono-substituted with F, Cl, methyl and methoxy,
a saturated or unsaturated, optionally at least one heteroatom as ring member containing cycloaliphatic radical,
whereby said cycloaliphatic radical may be substituted by one or more substituents selected from the group selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆-alkenyl, SO₂-C₁₋₄-alkyl, -(C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl,-NR^{A}R^{B}, wherein R^{A} and R^{B} are independently selected from the group consisting of H, C₁₋₄-alkyl and phenyl, NH-(C=O)-C₁₋₅-alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, 0-CH₂-thiazolyl, -NH-phenyl, and -C₁₋₄-Alkylen-NH-(C=O)-pheny), whereby said substituents may be substituted by one or more substituents selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl,-C(=O)-O-C₁₋₅-alkyl, -(C=O)-CH₂-F, -(C=O)-CH₂-Cl and -(C=O)-CH₂-Br, and whereby said cycloaliphatic radical may be bonded via an optionally at least mono-substituted alkylene group and/or may be condensed with an optionally at least mono-substituted aromatic mono- or polycyclic ring-system,
an optionally at least mono-substituted aryl- or heteroaryl radical, which may be bonded via an optionally at least mono-substituted alkylene group and/or may be condensed with an optionally at least mono-substituted mono- or polycyclic ring-system, a NR^{16b}R^{17b}-moiety or a COR^{18b}-moiety,
R^{10b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{12b} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13b} and R^{14b} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{13b} and R^{14b} together with the bridging nitrogen atom form a saturated, unsaturated or aromatic heterocyclic ring, which may be at least mono-substituted and/or contain at least one further heteroatom as a ring member,
R^{15b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{16b} represents an unbranched or branched, saturated or unsaturated, optionally at least mono-substituted aliphatic radical,
R^{17b} represents an unbranched or branched, saturated or unsaturated, optionally at least mono-substituted aliphatic radical,
R^{18b} represents an optionally at least mono-subsituted aryl radical,
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

43. Compounds according to claim 42, **characterized in that** R^{1b}, R^{2b}, R^{3b}, R^{4b} are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano group, - OR^{10b}, -OC(=O)R^{11b}, -SR^{12b}, -SOR^{12b}, -SO₂R^{12b}, -NH-SO₂R^{12b}, -SO₂NH₂ and a -NR^{13b}R^{14b} moiety, preferably selected from the group consisting of H,F, Cl, Br, a saturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro group, a cyano group, -OR^{10b},-OC(=O)R^{11b}, -SR^{12b} and -NR^{13b}R^{14b} moiety, more preferably selected from the group consisting of H, F, Cl, Br, -CH₃, -CH₂CH₃, -CF₃, -CF₂CF₃, cyclopentyl, cyclohexyl, nitro, cyano and -OR^{10b}.

44. Compounds according to claim 42 or 43, **characterized in that** R^{5b} represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₈-cycloaliphatic radical, preferably represents H or a branched or unbranched C₁₋₃-alkyl radical, more preferably H, -CH₃ or - CH₂CH₃, most preferably a hydrogen atom.

45. Compounds according to any one of claims 42 to 44, **characterized in that** R^{6b,} R^{7b}, R^{8b}, R^{9b} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano group and a -COOR^{15b} moiety, preferably selected from the group consisting of H, a branched or unbranched C₁₋₃-alkyl radical, a cyano group and a COOR^{15b} group, more preferably from the group consisting of H, -CH₃, -CH₂CH₃ and a cyano moiety, most preferably each of R^{6b}, R^{7b}, R^{8b} and R^{9b} represent a hydrogen atom.

46. Compounds according to any one of claims 42 to 45, **characterized in that** W^{b} represents an unbranched or branched, saturated or unsaturated, C₁₋₂₀ aliphatic radical, which may be substituted by one or more substituents selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂C₁₋₄. alkyl, wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, whereby said substituents may be at least mono-substituted with F, Cl, methyl and methoxy; a saturated or unsaturated, optionally at least one heteroatom as ring member containing C₃₋₈ cycloaliphatic radical, whereby said C₃₋₈ cycloaliphatic radical may be substituted by one or more substituents selected from the group selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆alkenyl, SO₂-C₁₋₄-alkyl, -(C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅-alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂morpholinyl-, thiadiazolyl, oxadiazolyl, oxazolyl, thiazolyl, isoxazolyl, O-CH₂-thiazolyl, -NH-phenyl, and -C₁₋₄-Alkylen-NH-(C=O)-phenyl, whereby said substituents may be substituted by one or more substituents selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, - C(=O)-O-C₁₋₅-alkyl, -(C=O)-CH₂-F, -(C=O)-CH₂-Cl and -(C=O)-CH₂-Br, and whereby said C₃₋₈ cycloaliphatic radical may be bonded via an optionally at least mono-substituted C₁₋₆alkylene group and/or may be condensed with an optionally at least mono-substituted aromatic mono- or polycyclic ring-system,
an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, a NR^{16b}R^{17b}-moiety or a COR^{18b}-moiety,
preferably W^{b} represents
a linear or branched C₁₋₂₀-alkyl radical, preferably an alkyl radical selected from the group consisting of methyl, ethyl, n-propyl, iso-propyl, n-butyl, iso-butyl, sec-butyl, tert-butyl and 1,1-dimethyl-propyl; a linear or branched C₂₋₂₀-alkenyl radical; preferably a vinyl radical; -CF₃; -CHF₂; -CH₂F; -CCl₃; -CHCl₂; - CH₂Cl; -CH₂-CF₃; -CH₂-CH₂-Cl; -CH₂-CH₂-CH₂-Cl; -CH₂-S(=O)₂-CH₃; a cyclopropyl radical; a cyclobutyl radical; a cyclopentyl radical; a cyclohexyl radical; -CH₂-cyclopropyl; -CH₂-cyclobutyl; -CH₂-cyclopentyl: -CH₂-cyclohexyl; -N(CH₃)₂; -N(C₂H₅)_{z}; -N(n-CH₂-CH₂-CH₃)₂; phenyl; benzyl; naphthyl; -CH=CH-phenyl; -(CF₂)-(CF₂)-O-phenyl; -(CH₂)-naphtyl; -(CH₂)-(CH₂)-naphthyl; anthracenyl; -(C=O)-phenyl; thiophenyl; benzo[b]thiophenyl; furanyl; 2-oxo-2H-chromenyl; dibenzofuranyl; 2,3-dihydrobenzofuranyl; chromanyl; 2,3-dihydro-benzo[1,4]dioxinyl; 3,4-dihydro-2H-1,5-benzo-dioxepinyl; chromonyl; 1H-imidazolyl; pyridinyl; pyrrolidine-2,5-dionyl; pyrrolyl; 1 H-pyrazolyl; 1 H-pyrimidine-2,4-dionyl; quinolinyl; isoquinolinyl; ¹H-Benzoimidazolyl; 1,4-dihydro-quinoxaline-2,3-dionyl; 1,2,3,4-tetrahydro-isoquinolinyl; 1,4-dihydro-benzo[b][1,4]diazepine-2,4-dionyl; 1,3-dihydro-1-oxy-2H-isoindolyl; phthalimidinyl; 2-(1,3-dioxo-1,3-dihydro-isoindol-2-yl)-ethyl; imidazo[1,2-a]pyridine; isatinyl; thiazolyl; 1,3-thiazolyl; 1,2,4-thiadiazolyl; imidazo[2,1-b]thiazolyl; 1,3-benzothiazolyl; benzo[1,2,5]thiadiazolyl; 2-oxo-2,3-dihydrobenzothiazolyl; 2,1,3-benzothiadiazolyl; imidazo[2,1-b]thiazolyl; isoxazolyl; benzo[1,2,5]oxadiazolyl; benzo[d]isoxazolyl; benzofurazanyl;
2-oxo-2,3-dihydro-benzooxazolyl; 3,4-dihydro-2H-benzo[1,4]oxazinyl; or 2,1,3-benzoxadiazolyl;
whereby each of these afore mentioned cyclic moieties may optionally be substituted with 1, 2, 3, 4 or 5 substituents independently selected from the group consisting of methyl; ethyl; n-propyl; iso-propyl; n-butyl; iso-butyl; sec-butyl; tert-butyl; 1,1-dimethyl-propyl; n-pentyl; vinyl; cyclopropyl; cyclobutyl; cyclopentyl; cyclohexyl; morpholino; methoxy; ethoxy; n-propoxy; iso-propoxy; n-propoxy; F; Cl; Br; I; -CN; -OH; -CF₃; - CF₂H; -CH₂F; -CCl₃; -CClH₂; -CHCl₂: -CH₂-F; -CH₂-Cl;-CH₂-Br; -(C=O)-CH₂-Br-, -OCF₃; -O-CH₂-CF₃; -O-CHF₂; -NO₂; -NH₂; -N(CH₃)₂; -N(C₂H₅)₂; - N(n-CH₂-CH₂-CH₃)₂; -N(n-CH₂-CH₂-CH₂-CH₃)₂;-NH-(C=O)-CH₃; -NH-phenyl; -(C=O)-CF₃; -(C=O)-OH; =O (oxo); -(C=O)-H; -S(=O)₂-CH₃; - S(=O)₂-isopropyl; -5(=O)₂-phenyl; -S(=O)₂-pyrrolidinyl; -S(=O)₂-morpholino; -(CH₂)-(CH₂)-(C=O)-O-CH₃; -NH-(C=O)-NH-CH₂-CH₂-CH₃; - (C=O)-CH₃; -(C=O)-O-CH₃; -(C=O)-O-C₂H₅; -(CH₂)-NH-(C=O)-phenyl; - CH₂-C(H)(phenyl)(phenyl); -O-CH₂-thiazolyl; 1,3-dioxo-2-azaspiro[4.4]non-2-yl; phenyl; phenoxy; isoxazolyl; 1,3-oxazolyl; 1,2,4-oxadiazolyl; 1,3,4-oxadiazolyl; pyridinyl; pyridinyloxy; pyrazolyl; pyrimidinyl and phthalimidinyl; and
whereby each of the cyclic moieties of these afore mentioned substituents may optionally be substituted with 1, 2, 3, 4 or 5 substituents independently selected from the group consisting of methyl; ethyl; n-propyl; iso-propyl; F; Cl; Br; l; CN; -CH₂-F; -CH₂-Cl; -CH₂-Br; -CF₃ and -S-CH₃,
more preferably W^{b} represents
an alkyl radical selected from the group consisting of methyl; ethyl; n-propyl; iso-propyl; n-butyl: sec.butyl; iso-butyl and tert-butyl: vinyl (CH₂=CH-); - N(CH₃)₂; 1-naphthyl; benzyl; 2-naphtyl; phenyl; 2-methyl-phenyl; 3-methyl-phenyl; 4-methyl-phenyl; 2-ethyl-phenyl; 3-ethyl-phenyl; 4-ethyl-phenyl; 2-n-propyl-phenyl; 3-n-propyl-phenyl; 4-n-propyl-phenyl; 2-isopropyl-phenyl; 3-isopropyl-phenyl; 4-isopropyl-phenyl; 2-n-butyl-phenyl; 3-n-butyl-phenyl; 4-n-butyl-phenyl; 2-iso-butyl-phenyl; 3-iso-butyl-phenyl; 4-iso-butyl-phenyl; 2-tert-butyl-phenyl; 3-tert-butyl-phenyl; 4-tert-butyl-phenyl; 1,1-dimethylpropyl-phenyl; 2-cyclopentyl-phenyl; 3-cyclopentyl-phenyl; 4-cyclopentyl-phenyf 2-cyclohexyl-phenyl; 3-cyclohexyl-phenyl; 4-cyclohexyl-phenyl; 2-methoxy-phenyl; 3-methoxy-phenyl; 4-methoxy-phenyl; 2-ethoxy-phenyl; 3-ethoxy-phenyl; 4-ethoxy-phenyl; 2-n-propoxy-phenyl; 3-n-propoxy-phenyl; 4-n-propoxy-phenyl; 2-iso-propoxy-phenyl; 3-iso-propoxy-phenyl; 4-isopropoxyphenyl;2-fluoro-phenyl; 3-fluoro-phenyl; 4-fluoro-phenyl; 2-chloro-phenyl; 3-chloro-phenyl; 4-chloro-phenyl; 2-bromo-phenyl; 3-bromo-phenyl; 4-bromo-phenyl; 2-trifluoromethyl-phenyl; 3-trifluoromethyl-phenyl; 4-trifluoromethyl-phenyl; 2-trifluoromethoxy-phenyl; 3-trifluoromethoxy-phenyl; 4-trifluoromethoxy-phenyl; 2-carboxy-phenyl; 3-carboxy-phenyl; 4-carboxy-phenyl; 2-acetyl-phenyl; 3-acetyl-phenyl; 4-acetyl-phenyl; 2-(C=O)-O-CH₃-phenyl; 3-(C=O)-O-CH₃-phenyl; 4-(C=O)-O-CH₃-phenyl; 2-(CH₂)-(CH₂)-(C=O)-O-CH₃: 3-(CH₂)-(CH₂)-(C=O)-O-CH₃; 4-(CH₂)-(CH₂)-(C=O)-O-CH₃; 2-cyano-phenyl; 3-cyano-phenyl; 4-cyano-phenyl; 2-nitro-phenyl; 3-nitro-phenyl; 4-nitro-phenyl; 4-(4-bromophenoxy)-phenyl; 2-methylsulfonyl-phenyl; 3-methylsulfonyl-phenyl; 4-methylsulfonyl-phenyl; 2-phenyl-phenyl (biphenyl-2-yl); 3-phenyl-phenyl (biphenyl-3-yl); 4-phenyl-phenyl (biphenyl-4-yl); 2-phenoxy-phenyl; 3-phenoxy-phenyl: 4-phenoxy-phenyl; 2,4-dimethyl-phenyl; 3,4-dimethyl-phenyl; 2,4,6-trimethyl-phenyl; 2,3,5,6-tetramethyl-phenyl; pentamethyl-phenyl; 2,5-dimethoxy-phenyl; 3,4-dimethoxy-phenyl; 2,3-dichloro-phenyl; 2,4-dichloro-phenyl; 2,5-dichlory-phenyl; 3,4-dichloro-phenyl; 3,5-dichloro-phenyl; 2,6-dichloro-phenyl; 2,4-difluoro-phenyl; 3,4-difluorophenyl; 2,5-difluoro-phenyl; 2,6-difluoro-phenyl; 3-chloro-2-fluoro-phenyl; 3-chloro-4-fluoro-phenyl; 5-chloro-2-fluoro-phenyl; 2,3,4-trichloro-phenyl; 2,4,5-trichloro-phenyl; 2,4,6-trichloro-phenyl; 2,4,5-trifluoro-phenyl; 2,3,4-trifluorophenyl-; 2-chloro-4,5-difluoro-phenyl; 2-bromo-4-fluoro-phenyl; 2-bromo-4,6-difluoro-phenyl; 4-chloro-2,5-difluoro-phenyl; 5-chloro-2,4-difluoro-phenyl; 4-bromo-2,5-difluoro-phenyl; 5-bromo-2,4-difluoro-phenyl; pentafluoro-phenyl; 2,4-dinitro-phenyl; 4-chloro-3-nitro-phenyl; 2-methyl-5-nitro-phenyl; 5-bromo-2-methoxy-phenyl; 3-chloro-2-methyl-phenyl; 4-bromo-3-methyl-phenyl; 4-chloro-2,5-dimethyl-phenyl; 4-fluoro-3-methyl-phonyl; 5-fluoro-2-methyl-phenyl; 2-nitro-4-trifluoromethyl-phenyl; 2-methoxy-4-methyl-phenyl; 3,5-dichloro-2-hydroxy-phenyl; 3,5-dichloro-4-hydroxy-phenyl; 5-chloro-2,4-difluoro-phenyl; 3-chloro-4-(NH)-(C=O)-CH₃-phenyl; 2-chloro-6-methyl-phenyl; 2-chloro-5 trifluoromethyl-phenyl; 2-chloro-5-trifluoromethoxy-phenyl; 4-bromo-2-trifluoromethoxy-phenyl; 4-bromo-2-trifluoromethyl-phenyl; 4-bromo-3-trifluoromethyl-phenyl; 3-carboxy-4-fluoro-phenyl; 3-carboxy-4-chloro-6-fluoro-phenyl; 4-methoxy-2,3,6-trimethyl-phenyl-; or one of the following groups: 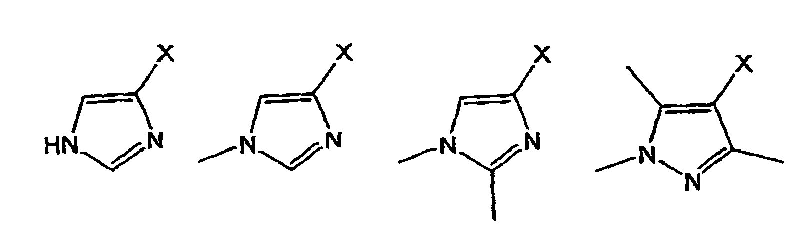 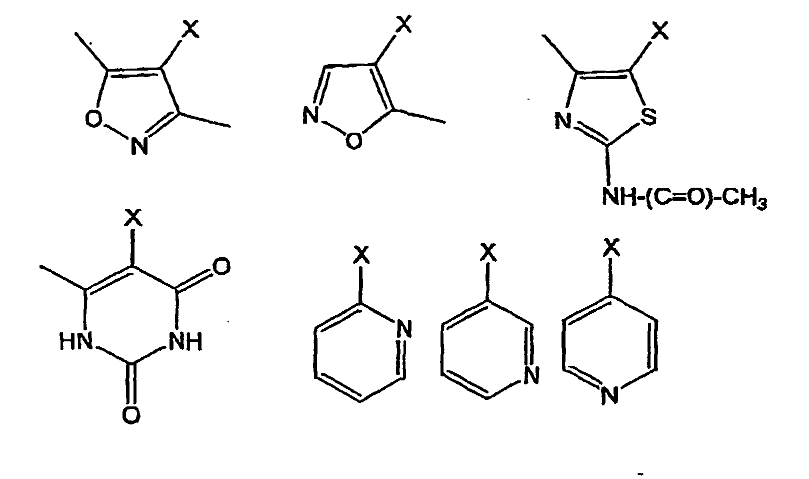 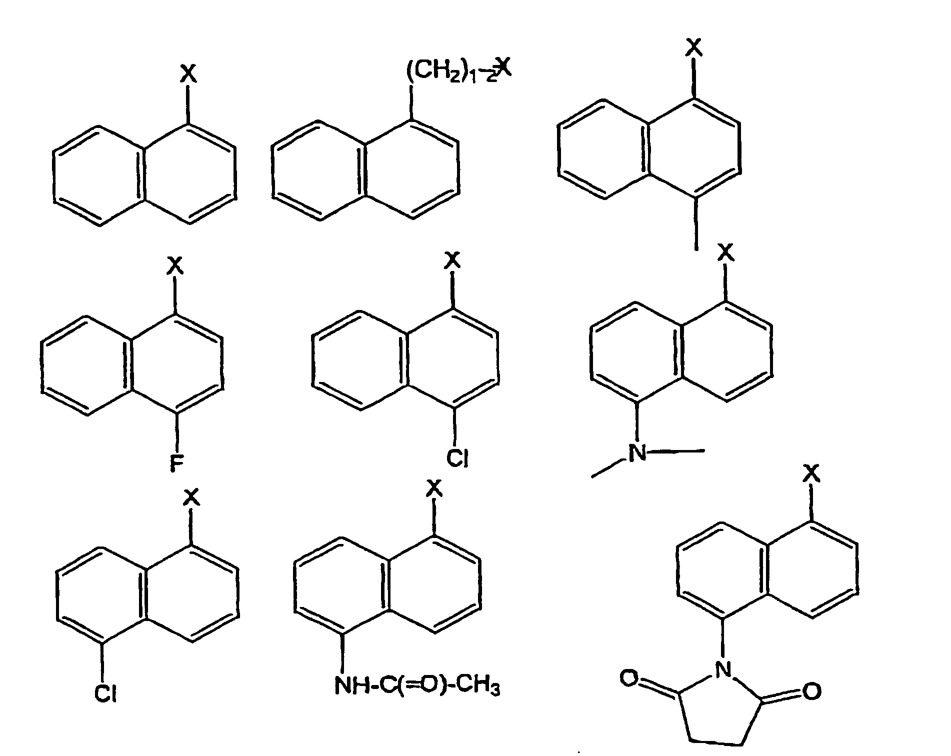 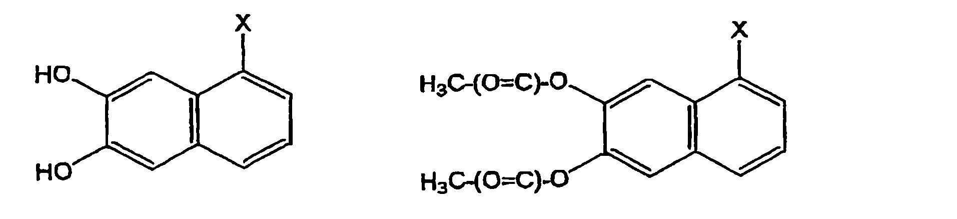 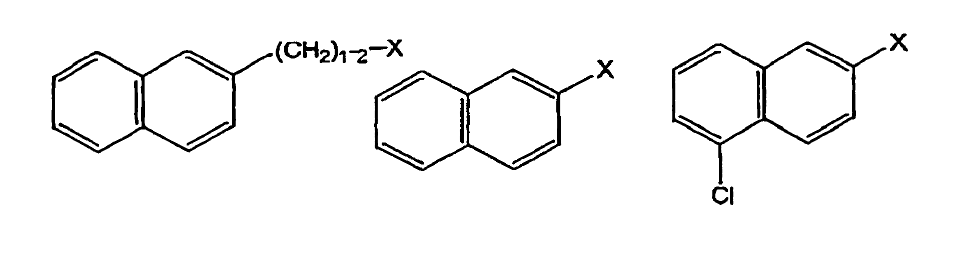 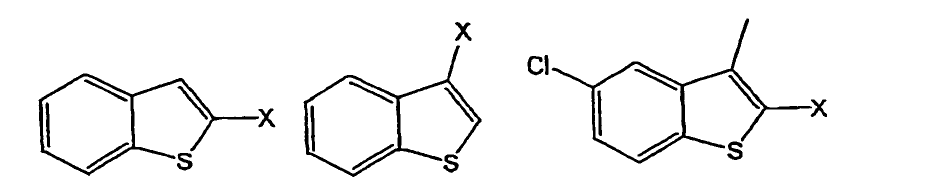 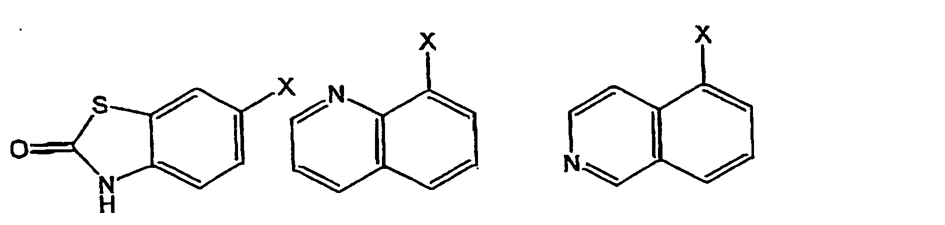 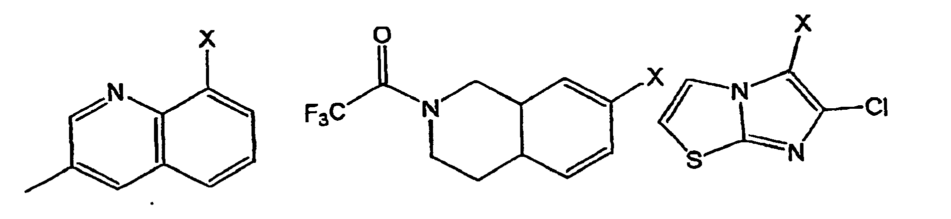 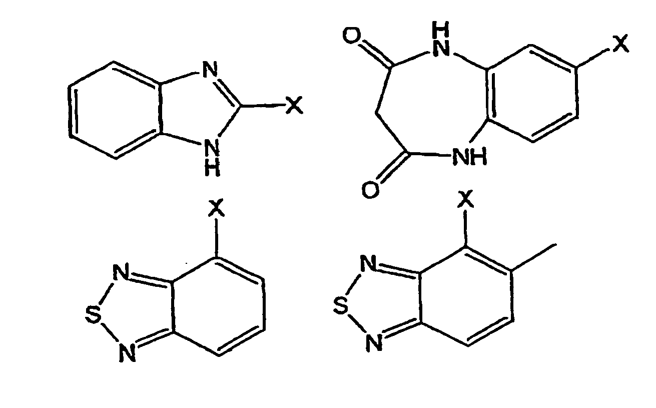 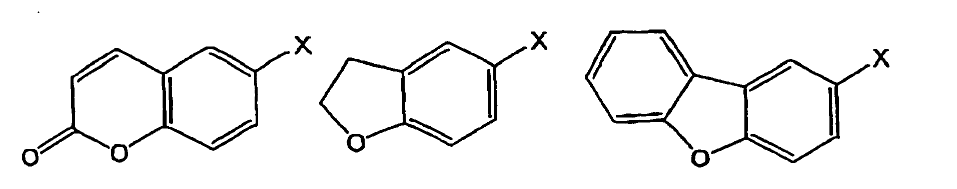 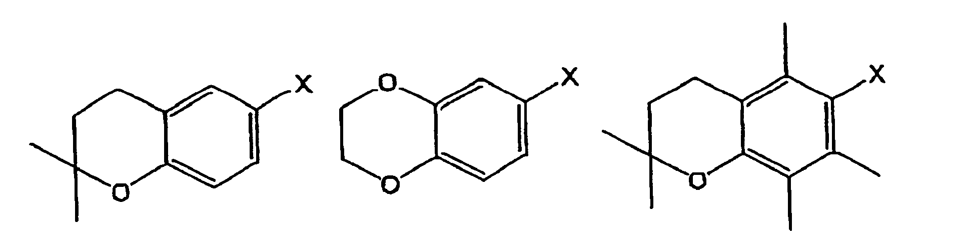 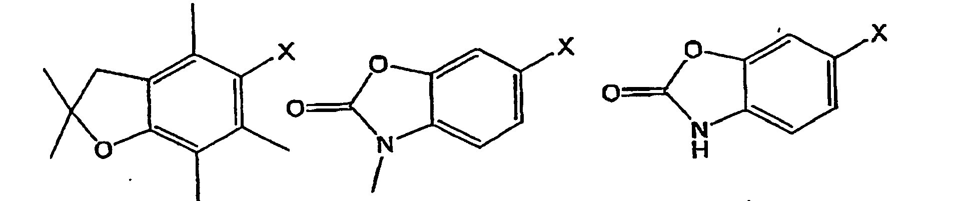 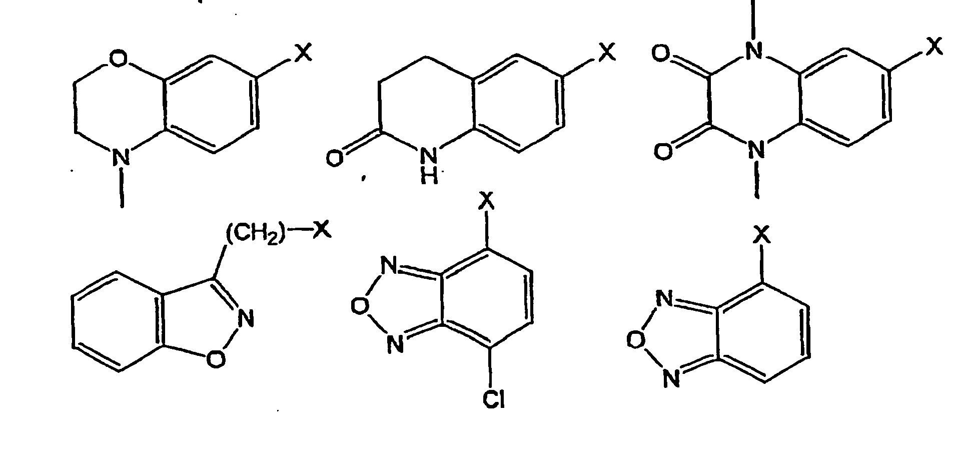 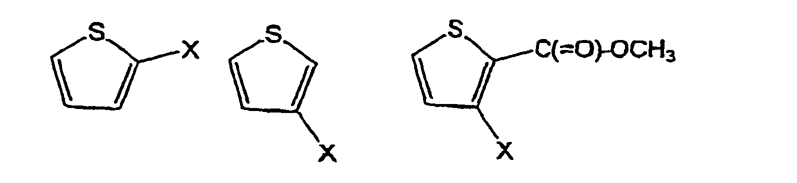 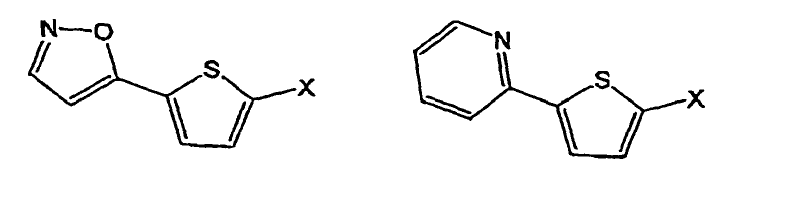 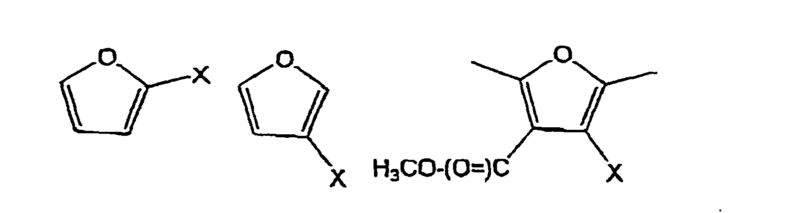 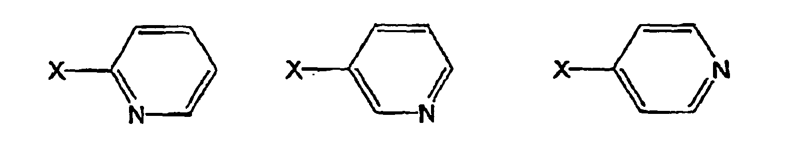 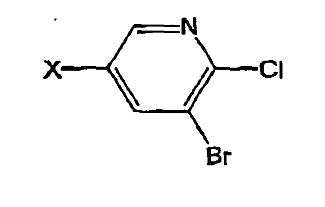 whereby in each case X denotes the position by which the respective substituent W^{b} is bonded to the -SO₂ group of formula (lb).

47. Compounds according to any one of claims 42 to 46, **characterized in that** R^{10b} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono-or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl.

48. Compounds according to any one of claims 42 to 47, **characterized in that** R^{11b} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylone group and/or may be condensed with an optionally at least mono-substituted mono-or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably H, a linear or branched C₁₋₄-alkyl radical, a cyclohexyl radical or a phenyl radical, more preferably H, -CH₃, -C₂H₅ or phenyl.

49. Compounds according to any one of claims 42 to 48, **characterized in that** R^{12b} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably H, -CH₃, -C₂H₅ or phenyl,

50. Compounds according to any one of claims 42 to 49, **characterized in that** R^{13b} and R^{14b} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system, preferably are each independently selected from the group consisting of H, a linear or branched C₁₋₄-alkyl radical, cyclohexyl and a phenyl radical, more preferably are each independently selected from the group consisting of H, -CH₃, -C₂H₅ and phenyl.

51. Compounds according to any one of claims 42 to 50, **characterized in that** R^{13b} and R^{14b} together with the bridging nitrogen atom form a saturated, unsaturated or aromatic, 5- or 6-membered heterocyclic ring, which may be at least mono-substituted and/or contain at least one further heteroatom as a ring member, preferably form an unsubstituted piperidin or morpholine group.

52. Compounds according to any one of claims 42 to 51, **characterized in that** R^{15b} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system, preferably represents H, a linear or branched C₁₋₄-alkyl radical, a cyclohexyl radical or a phenyl radical, more preferably represents H, -CH₃, -C₂H₅ or phenyl.

53. Compounds according to any one of claims 42 to 52, **characterized in that** R^{16b} represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical.

54. Compounds according to any one of claims 42 to 53 **characterized in that** R^{17b} represents an unbranched or branched, saturated or unsaturated, optionally at least mono-substituted C₁₋₆ aliphatic radical, preferably an unbranched or branched, saturated, unsubstituted C₁₋₃ alkyl radical, more preferably a methyl radical.

55. Compounds according to any one of claims 42 to 54 **characterized in that** R¹⁸ represents a phenyl radical, which is optionally at least mono-substituted by a C₁₋₆ aliphatic radical, more preferably a phenyl radical, which is optionally at least mono-substituted by a methyl group.

56. Compounds according to any one of claims 42 to 55, **characterized in that**
R^{1b}, R^{2b}, R^{3b}, R^{4b} are each independently selected from the group consisting of a hydrogen atom; a fluorine atom; a chlorine atom; a bromine atom; a methyl group and a methoxy group;
R^{5b} represents a hydrogen atom;
R^{6b}, R^{7b}, R^{8b}, R^{9b} each represent a hydrogen atom;
W^{b} represents
an alkyl radical selected from the group consisting of methyl; ethyl; n-propyl; iso-propyl; n-butyl; sec-butyl; iso-butyl and tert-butyl; vinyl (CH₂=CH-); - N(CH₃)₂; 1-naphthyl; benzyl; 2-naphtyl; phenyl; 2-methyl-phenyl; 3-methyl-phenyl; 4-methyl-phenyl; 2-ethyl-phenyl; 3-ethyl-phenyl; 4-ethyl-phenyl; 2-n-propyl-phenyl; 3-n-propyl-phenyl; 4-n-propyl-phenyl; 2-isopropyl-phenyl; 3-isopropyl-phenyl; 4-isopropyl-phenyl; 2-n-butyl-phenyl; 3-n-butyl-phenyl; 4-n-butyl-phenyl; 2-iso-butyl-phenyl; 3-iso-butyl-phenyl; 4-iso-butyl-phenyl; 2-tert-butyl-phenyl; 3-tert-butyl-phenyl; 4-tert-butyl-phenyl; 1,1-dimethylpropyl-phenyl; 2-cyclopentyl-phenyl; 3-cyclopentyl-phenyl; 4-cyclopentyl-phenyl 2-cyclohexyl-phenyl; 3-cyclohexyl-phenyl; 4-cyclohexyl-phenyl; 2-methoxy-phenyl; 3-methoxy-phenyl; 4-methoxy-phenyl; 2-ethoxy-phenyl; 3-ethoxy-phenyl; 4-ethoxy-phenyl; 2-n-propoxy-phenyl; 3-n-propoxy-phenyl; 4-n-propoxy-phenyl; 2-iso-propoxy-phenyl; 3-iso-propoxy-phenyl; 4-isopropoxyphenyl;2-fluoro-phenyl; 3-fluoro-phenyl; 4-fluoro-phenyl; 2-chloro-phenyl; 3-chloro-phenyl; 4-chloro-phenyl, 2-bromo-phenyl; 3-bromo-phenyl; 4-bromo-phenyl; 2-tifluoromethyl-phenyl; 3-trifluoromethyl-phenyl; 4-trifluoromethyl-phenyl; 2-trifluoromethoxy-phenyl; 3-trifluoromethoxy-phenyl; 4-trifluoromethoxy-phenyl; 2-carboxy-phenyl; 3-carboxy-phenyl; 4-carboxyphenyl; 2-acetyl-phenyl; 3-acetyl-phenyl; 4-acetyl-phenyl; 2-(C=O)-C-CH₃-phenyl; 3-(C=O)-O-CH₃-phenyl; 4-(C=O)-O-CH₃-phenyl; 2-(CH₂)-(CH₂)-(C=O)-O-CH-₃; 3-(CH₂)-(CH₂)-(C=O)-O-CH₃; 4-(CH₂)-(CH₂)-(C=O)-O-CH₃; 2-cyano-phenyl; 3-cyano-phenyl; 4-cyano-phenyl; 2-nitro-phenyl; 3-nitro-phenyl; 4-nitrophenyl; 4-(4-bromophenoxy)-phenyl; 2-methylsulfonyl-phenyl; 3-methylsulfonyl-phenyl; 4-methylsulfonyl-phenyl; 2-phenyl-phenyl (biphenyl-2-yl); 3-phenyl-phenyl (biphenyl-3-yl); 4-phenyl-phenyl (biphenyl-4-yl); 2-phenoxy-phenyl; 3-phenoxy-phenyl; 4-phenoxy-phenyl; 2.4-dimethyl-phenyl; 3,4-dimethyl-phenyl; 2,4,6-trimethyl-phenyl; 2,3,5,6-tetramethyl-phenyl; pentamethyl-phenyl; 2,5-dimethoxy-phenyl; 3,4-dimethoxy-phenyl; 2,3-dichloro-phenyl; 2,4-dichloro-phenyl; 2,5-dichloro-phenyl; 3,4-dichloro-phenyl; 3,5-dichloro-phenyl; 2,6-dichloro-phenyl; 2,4-difluoro-phenyl; 3,4-difluorophenyl; 2,5-difluoro-phenyl; 2,6-difluoro-phenyl; 3-chloro-2-fluoro-phenyl; 3-chloro-4-fluoro-phenyl; 5-chloro-2-fluoro-phenyl; 2,3,4-trichloro-phenyl; 2,4,5-trichloro-phenyl; 2,4,6-trichloro-phenyl; 2,4,5-tifluoro-phenyl; 2,3,4-trifluorophenyl-; 2-chloro-4,5-difluoro-phenyl; 2-bromo-4-fluoro-phenyl; 2-bromo-4,6-difluoro-phenyl; 4-chloro-2,5-difluoro-phenyl; 5-chloro-2,4-difluoro-phenyl; 4-bromo-2,5-difluoro-phenyl; 5-bromo-2,4-difluoro-phenyl; pentafluoro-phenyl; 2,4-dinitro-phenyl; 4-chloro-3-nitro-phenyl; 2-methyl-5-nitro-phenyl; 5-bromo-2-methoxy-phenyl; 3-chloro-2-methyl-phenyl; 4-bromo-3-methyl-phenyl; 4-chloro-2,5-dimethyl-phenyl; 4-fluoro-3-methyl-phenyl; 5-fluoro-2-methyl-phenyl; 2-nitro-4-trifluoromethyl-phenyl; 2-methoxy-4-methyl-phenyl; 3,5-dichloro-2-hydroxy phenyl; 3,5-dichloro-4-hydroxy-phenyl; 5-chloro-2,4-difluoro-phenyl; 3-chloro-4-(NH)-(C=O)-CH₃-phenyl; 2-chloro-6-methyl-phenyl; 2-chloro-5-trifluoromethyl-phenyl; 2-chloro-5-trifluoromethoxy-phenyl; 4-bromo-2-trifluoromethoxy-phenyl; 4-bromo-2-trifluoromethyl-phenyl; 4-bromo-3-trifluoromethyl-phenyl; 3-carboxy-4-fluoro-phenyl; 3-carboxy-4-chloro-6-fluoro-phenyl; 4-methoxy-2,3,6-trimethyl-phenyl-; or one of the following groups: 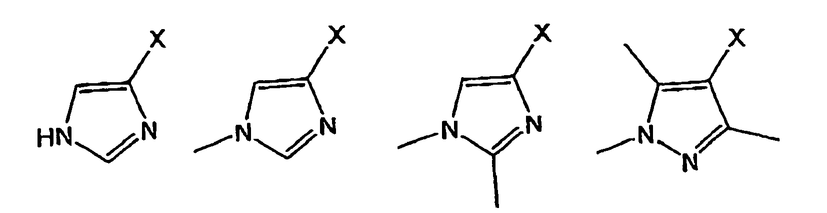 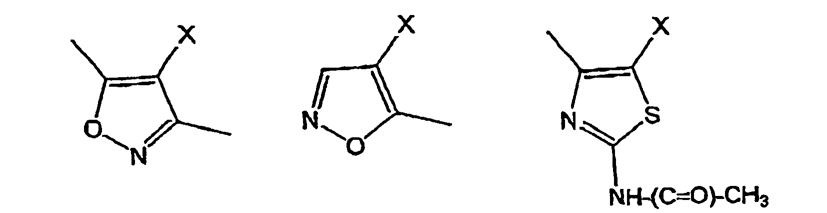 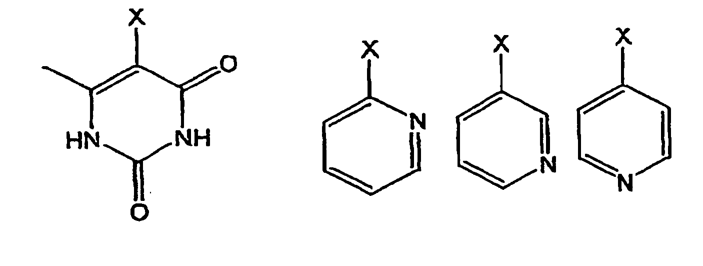 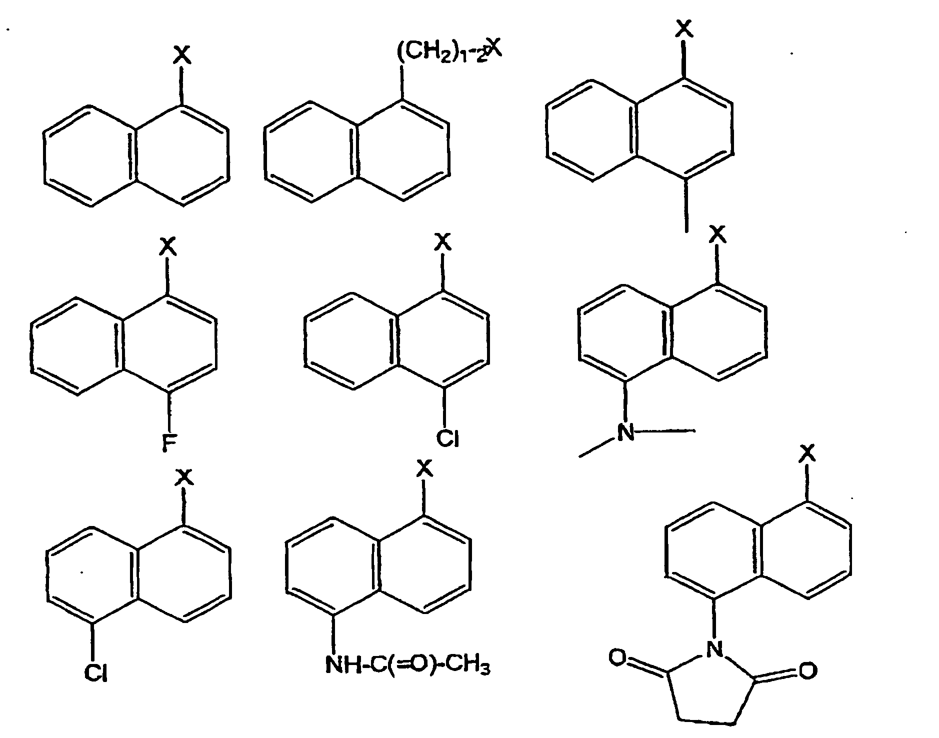 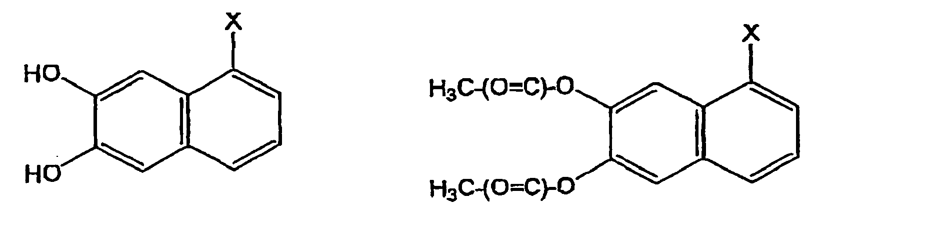 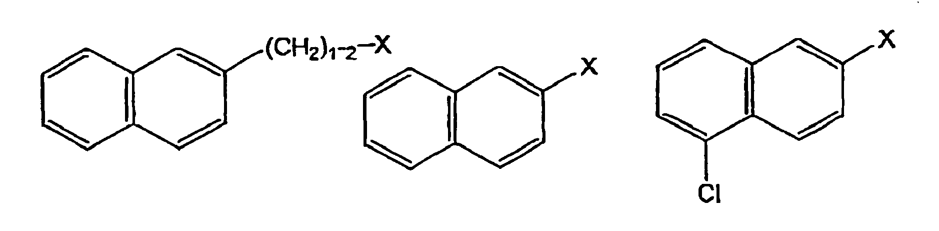 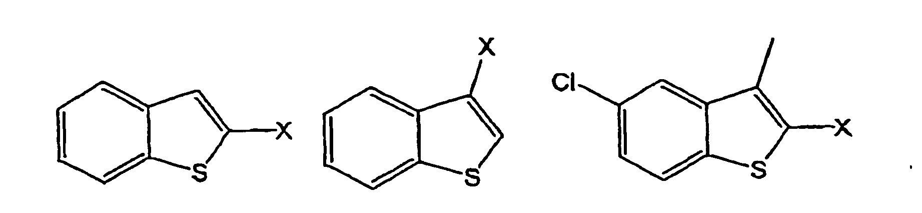 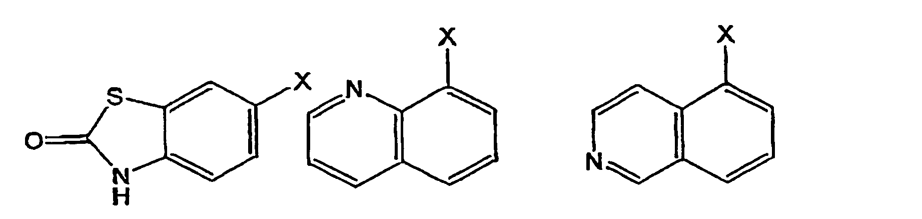 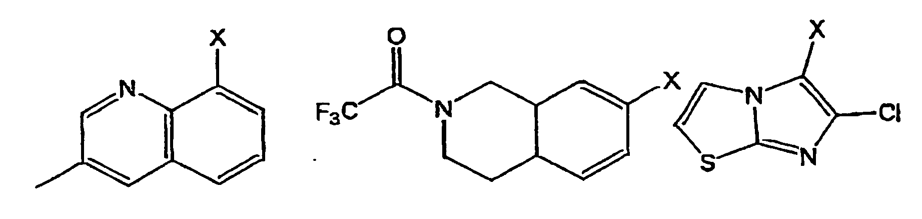 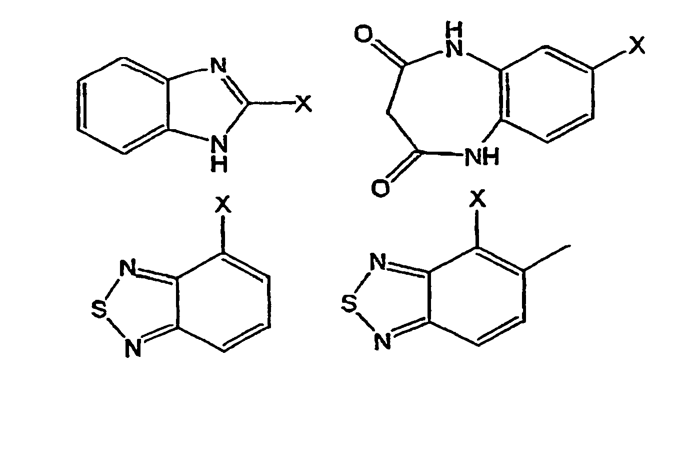 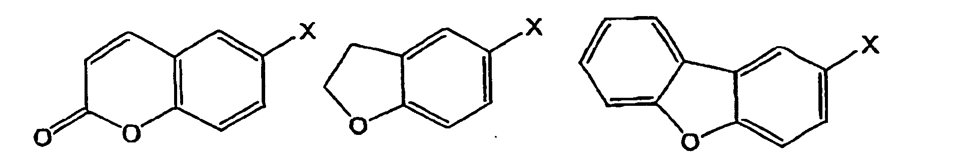 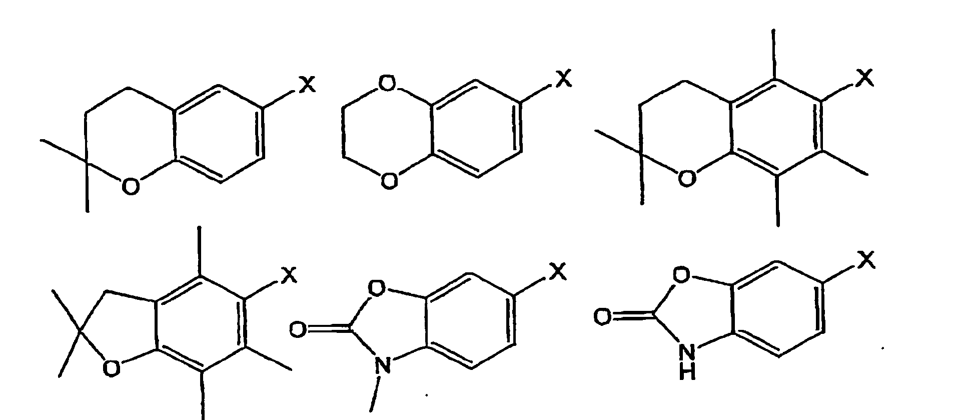 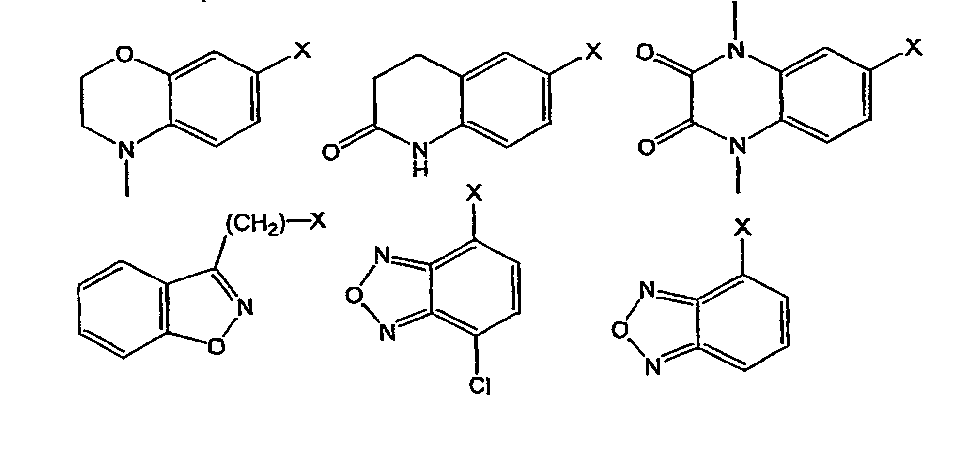 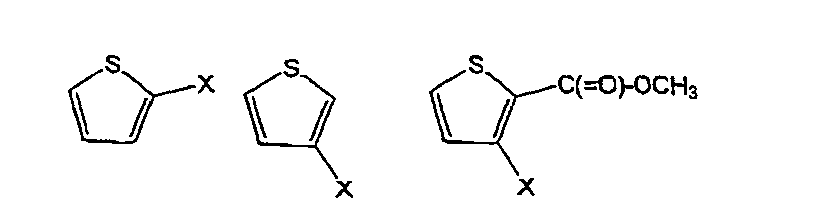 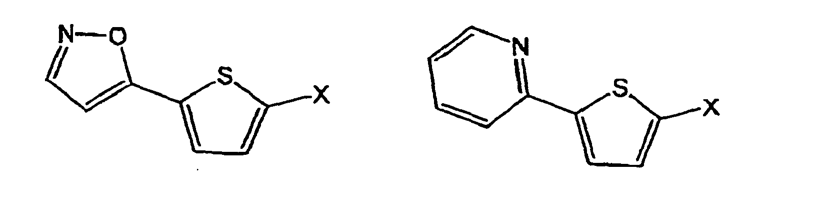 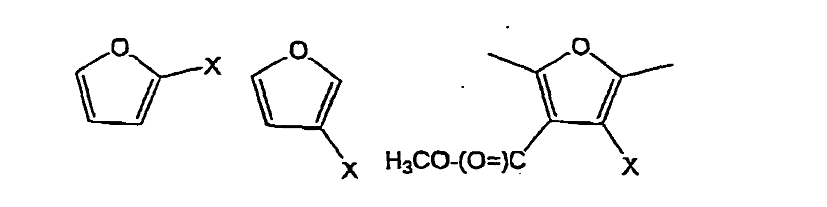 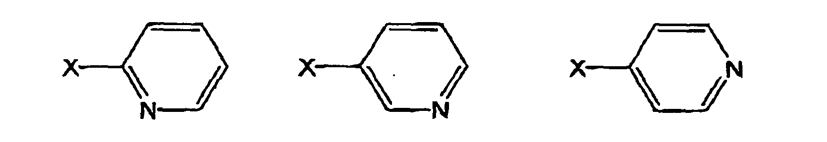 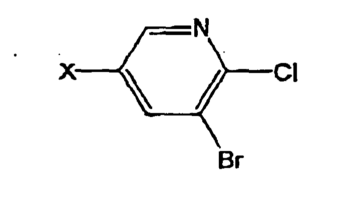 whereby in each case X denotes the position by which the respective substituent W^{b} is bonded to the -SO₂ group of formula (Ib).
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

57. Compounds according to any one of claims 42 to 56 selected from the following group:
| **N°** | **Compound** |
|---|---|
| 1 | 1-[1-(Naphthalene-2-sulfonyl)-piperidin-4-yl]-1,4-dihydro-benzo[d][1,3]oxazin-2-one |
| 2 | 1-[1-(Toluene-4-sulfonyl)-piperidin-4-yl]-1,4-dihydro-benzo[d][1,3]oxazin-2-one |
| 3 | 1-(1-Phenylmethanesulfonyl-piperidin-4-yl)-1,4-dilhydro-benzo[d][1,3]oxazin-2-one |
| 4 | 1-(1-Benzenesulfonyl-piperidin-4-yl)-6-chloro-1,4-dihydro-benzo[d][1,3]oxazin-2-one |
| 5 | 6-Chloro-1-[1-(toluene-4-sulfonyl)-piperidin-4-yl]-1,4-dihydro-benzo[d][1,3]oxazin-2-one |
| 6 | 6-Chloro-1-(1-phenylmethanesulfonyl-piperidin-4-yl)-1,4-dihydro-benzo[d][1,3]oxazin-2-one |
| 7 | 6-Chloro-1-[1-(naphthalene-1-sulfonyl)-piperidin-4-yl]-1,4-dihydro-benzo[d][1,3]oxazin-2-one |
| 8 | 6-Chloro-1-[1-(naphthalene-2-sulfonyl)-piperidin-4-yl]-1,4-dihydro-benzo[d][1,3]oxazin-2-one |
| 9 | 6-Chloro-1-[1-(5-chloro-3-methyl-benzo[b]thiophene-2-sulfonyl)-piperidin-4-yl]-1,4-dihydro-benzo[d][1,3]oxazin-2-one |
| 10 | 1-[1-(Thiophene-2-sulfonyl)piperidin-4-yl]-1,4-dihydro-benzo[d][1,3]oxazin-2-one |
| 11 | 1-[1-(4-Acetyl-benzenesulfonyl)-piperidin-4-yl]-1,4-dihydro-benzo[d][1,3]oxazin-2-one |
| 12 | 2-[4-(2-Oxo-4H-benzo[d][1,3]oxazin-1-yl)-piperidine-1-sulfonyl]-benzanitrile |
| 13 | 1-[1-(2,4-Dimethyl-benzenesulfonyl)-piperidin-4-yl]-1,4-dihydro-benzo[d][1,3]oxazin-2-one |
| 14 | 1-[1-(4-Methoxy-benzenesulfonyl)-piperidin-4-yl]-1,4-dihydro-benzo[d][1.3]oxazin-2-one |
| 15 | 1-[1-(2'Naphthalen-1-yl-ethanesulfonyl)-piperidin-4-yl]-1,4-dihydro-benzo[d][1,3]oxazin-2-one |
| 16 | 8-Methyl-1-[1-(thiophene-2-sulfonyl)-piperidin-4-yl]-1,4-dihydro-benzo[d][1,3]oxazin-2-one |
| 17 | 1-[1-(4-Acetyl-benzenesulfonyl)-piperidin-4-yl]-8-methyl-1,4-dihydro-benzo[d][1,3]oxazin-2-one |
| 18 | 2-[4-(8-Methyl-2-oxo-4H-benzo[d][1,3]oxazin-1-yl)-piperidine-1-sulfonyl]-benzonitrile |
| 19 | 1-[1-(2,4-Dimethyl-benzenesulfonyl)-piperidin-4-yl]-8-methyl-1,4-dihydro-benzo[d][1,3]oxazin-2-one |
| 20 | 1-[1-(4-Methoxy-benzenesulfonyl)-piperidin-4-yl]-8-methyl-1,4-dihydro-benzo[d][1,3]oxazin-2-one |
| 21 | 8-Methyl-1-[1-(2-naphthalen-1-yl-ethanesulfonyl)-piperidin-4-yl]-1,4-dihydro-benzo[d][1,3]oxazin-2-one |
| 22 | 4-(8-Methyl-2-oxo-4H-benzo[d][1,3]oxazin-1-yl)-piperidine-1-sulfonic acid dimethylamide |
| 23 | 2-[4-(2-Oxo-4H-benzo[d][1,3]oxazin-1-yl)-piperidine-1-sulfonyl]-benzoic acid methyl ester |
| 24 | 1-[1-(3-Trifluoromethyl-benzenesulfonyl)-piperidin-4-yl]-1,4-dihydro-benzo[d][1,3]oxazin-2-one |
| 25 | 2-[4-(8-Methyl-2-oxo-4H-benzo[d][1,3]oxazin-1-yl)-piperidine-1-sulfonyl]-benzoic acid methyl ester |
| 26 | 8-Methyl-1-[1-(3-trifluoromethyl-benzenesulfonyl)-piperidin-4-yl]-1,4-dihydro-benzo[d][1,3]oxazin-2-one |
| 27 | 1-[1-(4-Acetyl-benzenesulfonyl)-piperidin-4-yl]-6-chloro-1,4-dihydro-benzo[d][1,3]oxazin-2-one |
| 28 | 2-[4-(6-Chloro-2-oxo-4H-benzo[d][1,3]oxazin-1-yl)-piperidine-1-sulfonyl-benzonitrile |
| 29 | 6-Chloro-1-[1-(4-methoxy-benzenesulfonyl]-piperidin-4-yl]-1,4-dihydro-benzo[d][1,3]oxazin-2-one |
| 30 | 2-[4-(6-Chloro-2-oxo-4H-benzo[d][1,3]oxazin-1-yl)-piperidine-1-sulfonyl]-benzoic acid methyl ester |
| 31 | 6-Chloro-1-[1-(2,4-dimethyl-benzenesulfonyl)-piperidin-4-yl]-1,4-dihydro-benzo[d][1,3]oxazin-2-one |
| 32 | 6-Chloro-1-[1-(3-trifluoromethyl-benzenesulfonyl)-piperidin-4-yl]-1,4-dihydro-benzo[d][1,3]oxazin-2-one |
| 33 | 1-[1-(5-Chloro-3-methyl-benzo[b]thiophene-2-sulfonyl-piperidin-4-yl]-8-methyl-1,4-dihydro-benzo[d][1,3]oxazin-2-one |
| 34 | 1-{1-[4-(4-Bromo-phenoxy)-benzenesulfonyl]-piperidin-4-yl}-8-methyl-1,4-dihydro-benzo[d][1,3]oxazin-2-one |
| 35 | 1-[1-(4-Fluoro-benzenesulfonyl)-piperidin-4-yl]-8-methyl-1,4-dihydro-benzo[d][1,3]oxazin-2-one |
| 36 | 8-Methyl-1-[1-(naphthalene-2-sulfonyl)-piperidin-4-yl-1,4-dihydro-benzo[d][1,3]oxazin-2-one |
| 37 | 8-Methyl-1-(1-phenylmethanesulfonyl-piperidin-4-yl)-1,4-dihydro-benzo[d][1,3]oxazin-2-one |
| 38 | 1-[1-(4-Bromo-benzenesulfonyl)-piperidin-4-yl]-6-chloro-1,4-dihydro-benzo[d][1.3]oxazin-2-one |
| 39 | 8-Chloro-1-[1-(4-methanesulfanyl-benzenesulfonyl)-piperidin-4-yl]-1,4-dihydro-benzo[d][1,3]oxazin-2-one |
| 40 | 1-[1-(Butane-1-sulfonyl)-piperidin-4-yl]-6-chloro-1,4-dihydro-benzo[d][1,3]oxazin-2-one |
| 41 | 1-[1-(4-Bromo-benzenesulfonyl)-piperidin-4-yl]-8-methyl-1,4-dihydro-benzo[d][1,3]oxazin-2-one |
| 42 | 1-[1-(4-Methanesulfonyl-benzenesulfonyl)-piperidin-4-yl]-8-methyl-1,4-dihydro-benzo[d][1,3]oxazin-2-one |
| 43 | 1-[1-(Butane-1-sulfonyl)-piperidin-4-yl]-8-methyl-1,4-dihydro-benzo[d][1,3]oxazin-2-one |
| 44 | 6-Chloro-1-[1-(2-nitro-benzenesulfonyl)-piperidin-4-yl]-1,4-dihydro-benzo[d][1,3]oxazin-2-one |
| 45 | 6-Chloro-1-[1-(3-nitro-benzenesulfonyl)-piperidin-4-yl]-1,4-dihydro-benzo[d][1,3]oxazin-2-one |
| 46 | 1-[1-(Biphenyl-4-sulfonyl)-piperidin-4-yl]-6-chloro-1,4-dihydro-benzo[d][1,3]oxazin-2-one |
| 47 | 8-Methyl-1-[1-(2-nitro-benzenesulfonyl)-piperidin-4-yl]-1,4-dihydro-benzo[d][1,3]oxazin-2-one |
| 48 | 8-Methyl-1-[1-(3-nitro-benzenesulfonyl)-piperidin-4-yl]-1,4-dihydro-benzo[d][1,3]oxazin-2-one |
| 49 | 1-[1-(Biphenyl-4-sulfonyl)-piperidin-4-yl]-8-methyl-1,4-dihydro-benzo[d][1,3]oxazin-2-one |
| 50 | 8-Methyl-1-[1-(4-nitro-benzenesulfonyl)-piperidin-4-yl]-1,4-dihydro-benzo[d][1,3]oxazin-2-one |
| 51 | 6-Chloro-1-[1-(4-nitro-benzenesulfonyl)-piperidin-4-yl]-1,4-dihydro-benzo[d][1,3]oxazin-2-one |
| 52 | 1-(1-Ethanesulfonyl-piperidin-4-yl)-1,4-dihydro-benzo[d][1,3]oxazin-2-one |
| 53 | 1-[1-(Propane-1-sulfonyl)-piperidin-4-yl]-1,4-dihydro-benzo[d][1,3]oxazin-2-one |
| 54 | 1-[1-(Propane-2-sulfonyl)-piperidin-4-yl]-1,4-dihydro-benzo[d][1,3]oxazin-2-one |
| 55 | 6-Chloro-1-(1-ethanesulfonyl-piperidin-4-yl)-1,4-dihydro-benzo[d][1,3]oxazin-2-one |
| 56 | 6-Chloro-1-[1propane-1-sulfonyl)-piperidin-4-yl]-1,4-dihydro-benzo[d][1,3]oxazin-z-one |
| 57 | 6-Chloro-1-[1-(propane-2-sulfonyl)-piperidin-4-yl]-1,4-dihydro-benzo[d][1,3]oxazin-2-one |
| 58 | 6-Chloro-1-[1-(quinoline-8-sulfonyl)-piperidin-4-yl]-1,4-dihydro-benzo[d][1,3]oxazin-2-one |
| 59 | 1-[1-(4-Nitro-benzenesulfonyl)-piperidin-4-yl]-1,4-dihydro-benzo[d][1,3]oxazin-2-one |
| 60 | 6-Methyl-1-[1-(quinoline-8-sulfonyl)-piperidin-4-yl)-1,4-dihydro-benzo[d][1,3]oxazin-2-one |
| 61 | 6-Methyl-1-[1-(2-naphthalen-1-yl-ethanesulfonyl)-piperidin-4-yl]-1,4-dihydro-benzo[d][1,3]oxazin-2-one |
| 62 | 6-Methyl-1-[1-(toluene-4-sulfonyl)-piperidin-4-yl]-1,4-dihydro-benzo[d][1,3]oxazin-2-one |
| 63 | 1-[1-(4-Fluoro-benzenesulfonyl)-piperidin-4-yl]-6-methyl-1,4-dihydro-benzo[d][1,3]oxazin-2-one |
| 64 | 6-Methyl-1-[1-(naphthalene-1-sulfonyl)-piperidin-4-yl]-1,4-dihydro-benzo[d][1,3]oxazin-2-one |
| 65 | 6-Methyl-1-[1-(naphthalene-2-sulfonyl)-piperidin-4-yl]-1,4-dihydro-benzo[d][1,3]oxazin-2-one |
| 66 | 1-[1-(5-Chloro-3-methyl-benzo[b]thiophene-2-sulfonyl)-piperidin-4-yl]-6-methyl-1,4-dihydro-benzo[d][1,3]oxazin-2-one |
| 67 | 6-Methyl-1-[1-(4-nitro-benzenesulfonyl)-piperidin-4-yl]-1,4-dihydro-benzo[d][1,3]oxazin-2-one |
| 68 | 1-(1-Benzenesulfonyl-piperidin-4-yl)-6-methyl-1,4-dihydro-benzo[d][1,3]oxazin-2-one |
| 69 | 1-[1-(4-Chloro-3-nitro-benzenesulfonyl)-piperidin-4-yl]-6-methyl-1,4-dihydro-benzo[d][1,3]oxazin-2-one |
| 70 | 1-[1-(5-Dimethylamino-naphthalene-1-sulfonyl)-piperidin-4-yl]-6-methyl-1,4-dihydro-benzo[d][1,3]oxazin-2-one |
| 71 | 1-[1-(4-Chloro-3-nitro-benzenesulfonyl)-piperidin-4-yl]-8-methyl-1,4-dihydro-benzo[d][1,3]oxazin-2-one |
| 72 | 1-[1-(4-Chloro-3-nitro-benzenesulfonyl)-piperidin-4-yl]-1,4-dihydro-benzo[d][1,3]oxazin-2-one |
| 73 | 6-Chloro-1-[1-(4-chloro-3-nitro-benzenesulfonyl)-piperidin-4-yl]-1,4-dihydro-benzo[d][1,3]oxazin-2-one |
| 74 | 6-Chloro-1-[1-(5-dimethylamino-naphthalene-1-sulfonyl)-piperidin-4-yl]-1,4-dihydro-benzo[d][1,3]oxazin-2-one |
| | |
| 75 | 1-[1-(4-Methoxy-2,3,6-trimethyl-benzenesulfonyl)-piperidin-4-yl]-1,4-dihydro-benzo[d][1,3]oxazin-2-one |
| 76 | 1-[1-(4-Methoxy-2,3,6-trimethyl-benzenesulfonyl)-piperidin-4-yl]-8-methyl-1,4-dihydro-benzo[d][1,3]oxazin-2-one |
| 77 | 6-chloro-1-[1-(4-methoxy-2,3,6-trimethyl-benzenesulfonyl)-piperidin-4-yl]-1,4-dihydro-benzo[d][1,3]oxazin-2-one |
| 78 | 1-[1-(4-Methoxy-2,3,6-trimethyl-benzenesulfonyl)-piperidin-4-yl]-6-methyl-1,4-dihydro-benzo[d][1,3]oxazin-2-one |
| 79 | 1-[1-(2-Bromo-benzenesulfonyl-piperidin-4-yl]-1,4-dihydro-benzo[d][1,3]oxazin-2-one |
| 80 | 1-[1-(2-Bromo-benzenesulfonyl)-piperidin-4-yl]-8-methyl-1,4-dihydro-benzo[d][1,3]oxazin-2-one |
| 81 | 1-[1-(2-Bromo-benzenesulfonyl)-piperidin-4-yl]-6-chloro-1,4-dihydro-benzo[d][1,3]oxazin 2-one |
| 82 | 1-[1-(2-Bromo-benzenesulfonyl)-piperidin-4-yl]-6-methyl-1,4-dihydro-benzo[d][1,3]oxazin-2-one |
| 83 | 6-Chloro-1-[1-(2,3-dichloro-benzenesulfonyl)-piperidin-4-yl]-1,4-dihydro-benzo[d][1,3]oxazin-2-one |
| 84 | 1-[1-(2,3-Dichloro-benzenesulfonyl)-piperidin-4-yl]-6-methyl-1,4-dihydro-benzo[d][1,3]oxazin-2-one |
| 85 | 1-[1-(2,4,5-Trichloro-benzenesulfonyl)-piperidin-4-yl]-1,4-dihydro-benzo[d][1,3]oxazin-2-one |
| 86 | 8-Methyl-1-[1-(2,4,5-trichloro-benzenesulfonyl)-piperidin-4-yl]-1,4-dihydro-benzo[d][1,3]oxazin-2-one |
| 87 | 6-Chloro-1-[1-(2,4,5-trichloro-benzenesulfonyl)-piperidin-4-yl]-1,4-dihydro-benzo[d][1,3]oxazin-2-one |
| 88 | 6-Methyl-1-[1-(2,4,5-trichloro-benzenesulfonyl)-piperidin-4-yl]1,4-dihydro-benzo[d][1,3]oxazin-2-one |
| 89 | 1-[1-(5-Bromo-2-methoxy-benzenesulfonyl)-piperidin-4-yl]-1,4-dihydro-benzo[d][1,3]oxazin-2-one |
| 90 | 1-[1-(5-Bromo-2-methoxy-benzenesulfonyl)-piperidin-4-yl]-8-methyl-1,4-dihydro-benzo[d][1,3]oxazin-2-one |
| 91 | 1-[1-(5-Bromo-2-methoxy-benzenesulfonyl)-piperidin-4-yl]-6-chloro-1,4-dihydro-benzo[d][1,3]oxazin-2-one |
| 92 | 1-[1-(5-Bromo-2-methoxy-benzenesulfonyl)-piperidin-4-yl]-6-methyl-1,4-dihydro-benzo[d][1,3]oxazin-2-one |
| 93 | 1-[1-(2,5-Dimethoxy-benzenesulfonyl)-piperidin-4-yl]-1,4-dihydro-benzo[d][1.3]oxazin-2-one |
| 94 | 1-[1-(2,5-Dimethoxy-benzenesulfonyl)-piperidin-4-yl]-8-methyl-1,4-dihydro-benzo[d][1,3]oxazin-2-one |
| 95 | 6-Chloro-1-[1-(2,5-dimethoxy-benzenesulfonyl)-piperidin-4-yl]-1,4-dihydro-benzo[d][1,3]oxazin-2-one |
| 96 | 1-[1-(2,5-Dimethoxy-benzenesulfonyl)-piperidin-4-yl]-6-methyl-1,4-dihydro-benzo[d][1,3]oxazin-2-one |
| 97 | 1-(1-Pentamethylbenzenesulfonyl-piperidin-4-yl)-1,4-dihydro-benzo[d][1,3]oxazin-2-one |
| 98 | 8-Methyl-1-(1-pentamethylbenzenesulfonyl-piperidin-4-yl)-1,4-dihydro-benzo[d][1,3]oxazin-2-one |
| 99 | 6-Chloro-1-(1-pentamethylbenzenesulfonyl-piperidin-4-yl)-1,4-dihydro-benzo[d][1,3]oxazin-2-one |
| 100 | 6-Methyl-1-(1-pentamethylbenzenesulfonyl-piperidin-4-yl)-1,4-dihydro-benzo[d][1,3]oxazin-2-one |
| 101 | 1-{1-[2-(2,2,2,-Trifluoro-acetyl)-1,2,3,4-tetrahydro-isoquinoline-7-sulfonyl]-piperidin-4-yl)-1,4-dihydro-benzo[d][1,3]oxazin-2-one |
| 102 | 8-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 103 | 6-Chloro-1-{1-{2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 104 | 6-Methyl-1-{1-[2-(2,2,2-trifiuoro-acetyl)-1,2,3,4-tetrahydro-isoquinoline-7-sulfonyl]-piperidin-4-yl}-1,4-dihydro-benzo[d][1,3]oxazin-2-one |
| 105 | 1-[1-(2-Methyl-5-nitro-benzenesulfonyl)-piperidin-4-yl]-1,4-dihydro-benzo[d][1,3]oxazin-2-one |
| 106 | 8-Methyl-1-[1-(2-Methyl-5-nitro-benzenesulfonyl)-piperidin-4-yl]-1,4-dihydro-benzo[d][1,3]oxazin-2-one |
| 107 | 6-Chloro-1-[1-(2-methyl-5-nitro-benzenesulfonyl)-piperidin-4-yl]-1,4-dihydro-benzo[d][1,3]oxazin-2-one |
| 108 | 6-Methyl-1-[1-(2-methyl-5-nitro-benzenesulfonyl)-piperidin-4-yl]-1,4-dihydro-benzo[d][1,3]oxazin-2-one |
| 109 | 1-[1-(4-Bromo-2,5-difluoro-benzenesulfonyl)-piperidin-4-yl]-1,4-dihydro-benzo[d][1,3]oxazin-2-one |
| 110 | 1-[1-(4-Bromo-2,5-difluoro-benzenesulfonyl)-piperidin-4-yl]-8-methyl-1,4-dihydro-benzo[d][1,3]oxazin-2-one |
| 111 | 1-[1-(4-Bromo-2,5-difluoro-benzenesulfonyl)-piperidin-4-yl]-6-chloro-1,4-dihydro-benzo[d][1,3]oxazin-2-one |
| 112 | 1-[1-(4-Bromo-2,5-difluoro-benzenesulfony)-piperidin-4-yl]-6-methyl-1,4-dihydro-benza[d]oxazin-2-one |
| 113 | 1-[1-(4-Chloro-2,5-dimethyl-benzenesulfonyl)-piperidin-4-yl]-1,4-dihydro-benzo[d][1,3]oxazin-2-one |
| 114 | 1-[1-(4-Chloro-2,5-dimethyl-benzenesulfonyl)piperidin-4-yl]-8-methyl-1,4-dihydro-benzo[d][1,3]oxazin-2-one |
| 115 | 6-Chloro-1-[1-(4-chloro-2,5-dimethyl-benzenesulfonyl]-piperidin-4-yl]-1,4-dihydro-benzo[d][1,3]oxazin-Z-one |
| 116 | -1-[1-(4-Chloro-2,5-dimethyl-benzenesulfonyl)-piperidin-4-yl]-8-methyl-1,4-dihydro-benzo[d][1,3]oxazin-2-one |
| 117 | 1-[1-(4-Methoxy-benzenesulfonylfonyl)-piperidin-4-yl]-1,4-dihydro-benzo[d][1,3]oxazin-2-one |
| 118 | 1-[1-(4-Isopropyl-benzenesulfonyl)-piperidin-4-yl]-1,4-dihydro-benzo[d][1,3]oxazin-2-one |
| 119 | 1-[1-(4-Isopropyl-benzenesulfonyl)-piperidin-4-yl]-8-methyl-1,4-dihydro-benzo[d][1,3]oxazin-2-one |
| 120 | 6-Chloro-1-[1-(4-isopropyl-benzenesulfonyl)-piperidin-4-yl)-1,4-dihydro-benzo[d][1,3]oxazin-2-one |
| 121 | 1-[1-(4-Isopropyl-benzenesulfonyl)-piperidin-4-yl]-6-methyl-1,4-dihydro-benzo[d][1,3]oxazin-2-one |
| 122 | 1-[1-(3-Chloro-4-flucro-benzenesulfonyl)-piperidin-4-yl]-1,4-dihydro-benzo[d][1,3]oxazin-2-one |
| 123 | 1-[1-(3-Chloro-4-fluoro-benzenesulfonyl)-piperidin-4-yl]-8-methyl-1,4-dihydro-benzo[d][1,3]oxazin-2-one |
| 124 | 6-Chloro-1-[1-(3-chloro-4-fluoro-benzenesulfonyl)-piperidin-4-yl]-1,4-dihydro-benzo[d]oxazin-2-one |
| 125 | 1-[1-(3-Chloro-4-fluoro-benzenesulfonyl)-piperidin-4-yl]-6-methyl-1,4-dihydro-benzo[d][1,3]oxazin-2-one |
| 126 | 1-[1-(4-Bromo-benzenesulfoyl)-piperidin-4-yl]-6-methyl-1,4-dihydro-benzo[d][1,3]oxazin-2-one |
| 127 | 6-Methyl-1-[1-(3-nitro-benzenesulfonyl)-piperidin-4-yl]-1,4-dihydro-benzo[d][1,3]oxazin-2-one |
| 128 | 6-Methyl-1-[3-trifluoromethyl-benzenesulfonyl)-piperidin-4-yl]-1,4-dihydro-benzo[d][1,3]oxazin-2-one |
| 129 | 1-[1-(4-Trifluoromethoxy-benzenesulfonyl)-piperidin-4-yl]-1,4-dihydro-benzo[d][1,3]oxazin-2-one |
| 130 | 1-[1-(2-Nitro-4-trifluoromethyl-benzenesulfonyl)-piperidin-4-yl]-1,4-dihydro-benzo[d][1,3]oxazin-2-one |
| 131 | 1-[1-(3-Fluoro-benzenesulfonyl)-piperidin-4-yl]-1,4-dihydro-benzo[d][1,3]oxazin-2-one |
| 132 | 1-[1-(2,4-Dichloro-benzenesulfonyl)-piperidin-4-yl]-1,4-dihydro-benzo[d][1,3]oxazin-2-one |
| 133 | 1-[1-(2,4,6-Trimethyl-benzenesulfonyl)-piperidin-4-yl]-1,4-dihydro-benzo[d][1,3]oxazin-2-one |
| 134 | 1-[1-(2-Trifluoromethyl-benzenesulfonyl)-piperidin-4-yl]-1,4-dihydro-benzo[d][1,3]oxazin-2-one |
| 135 | 8-Methyl-1-[1-(4-trifluoromethoxy-benzenesulfonyl)-piperidin-4-yl]-1,4-dihydro-benzo[d][1,3]oxazin-2-one |
| 136 | 8-Methyl-1-[1-(2-nitro-4-trifluoromethyl-benzenesulfonyl)-piperidin-4-yl]-1,4-dihydro-benzo[d][1,3]oxazin-2-one |
| 137 | 1-[1-(3-Fluoro-benzenesulfonyl)-piperidin-4-yl]-8--methyl-1,4-dihydro-benzo[d][1,3]oxazin-2-one |
| 138 | 1-[1-(2,4-Dichloro-benzenesulfonyl)-piperidin-4-yl]-8-methyl-1,4-dihydro-benzo[d][1,3]oxazin-2-one |
| 139 | 8-Methyl-1-[1-(2,4,6-trimethyl-benzenesulfonyl)-piperidin-4-yl]-1,4-dihydro-benzo[d][1,3]oxazin-2-one |
| 140 | 8-Methhy-1-[1-(2-trifluoromethyl-benzenesulfonyl]-piperidin-4-yl]-1,4-dihydro-benzo[d][1,3]oxazin-2-one |
| 141 | 1-[1-(4-Fluoro-benzenesulfonyl)-piperidin-4-yl]-1,4-dihydro-benzo[d][1,3]oxazin-2-one |
| 142 | 1-[1-(4-Bromo-benzenesulfonyl)-piperidin-4-yl]-1,4-dihydno-benzo[d][1,3]oxazin-2-one |
| 143 | 1-[1-(3-Nitro-benzenesulfonyl)-piperidin-4-yl]-1,4-dihydro-benzo[d][1,3]oxazin-2-one |
| 144 | 1-{1-[4-(4-Bromo-phenoxy)-benzenesulfonyl]-piperidin-4-yl}-1,4-dihydro-benzo[d][1,3]oxazin-2-one |
| 145 | 1-[1-(3-Methoxy-benzenesulfonyl)-piperidin-4-yl]-1,4-dihydro-benzo[d][1,3]-oxazin-2-ane |
| 146 | 1-[1-(2-Nitro-benzenesulfonyl)-piperidin-4-yl]-1,4-dihydro-benzo[d][1,3]oxazin-2-one |
| 147 | 8-Methyl-1-[1-(toluene-4-sulfonyl)-piperidin-4-yl]-1,4-dihydro-benzo[d][1,3]oxazin-2-one |
| 148 | 1-[1-Benzenesulfonyl-piperidin-4-yl)-8-methyl-1,4-dihydro-benzo[d][1,3]oxazin-2-one |
| 149 | 1-[1-(3-Methoxy-benzenesulfonyl)-piperidin-4-yl]-8-methyl-1,4-dihydro-benzo[d][1,3]oxazin-2-one |
| 150 | 1-[1-(2,4-Dimethyl-benzenesulfonyl)-piperidin-4-yl]-6-methyl-1,4-dihydro-benzo[d][1,3]oxazin-2-one |
| 151 | 1-{1-[4-(4-Bromo-phenoxy)-benzenesulfonyl]-piperidin-4-yl}-6-methyl-1,4-dihydro-benzo[d][1,3]oxazin-2-one |
| 152 | 6-Methyl-1-[1-(thiophene-2-sulfonyl)-piperidin-4-yl]-1,4-dihydro-benzo[d][1,3]oxazin-2-one |
| 153 | 1-[1-(Toluene-3-sulfonyl)-piperidin-4-yl]-1,4-dihydro-benzo[d][1,3]oxazin-2-one |
| 154 | 1-[1-(5-Fluoro-2-methyl-benzenesulfonyl)-piperidin-4-yl]-1,4-dihydro-benzo[d][1,3]oxazin-2-one |
| 155 | 1-[1-(4-Isopropoxy-benzenesulfonyl)-piperidin-4-yl]-1,4-dihydro-benzo[d][1,3]oxazin-2-one |
| 156 | 1-[-3-Chloro-benzenesulfonyl]-piperidin-4-yl]-1,4-dihydro-benzo[d][1,3]oxazin-2-one |
| 157 | 1-[1-(3,4-Dimethoxy-benzenesulfonyl)-piperidin-4-yl]-1,4-dihydro-benzo[d][1,3]oxazin-2-one |
| 158 | 1-(1-Pentafluorobenzenesulfonyl-piperidin-4-yl)-1,4-dihydro-benzo[d][1,3]oxazin-2-one |
| 159 | 8-Methyl-1-[1-(toluene-3-sulfonyl)-piperidin-4-yl]-1,4-dihydro-benzo[d][1,3]oxazin-2-one |
| 160 | 1-[1-(5-Fluoro-2-methyl-benzenesulfonyl)-piperidin-4-yl]-8-methyl-1,4-dihydrobenzo[d][1,3]oxazin-2-one |
| 161 | 1-[1-(4-Isopropoxy-benzenesulfonyl)-piperidin-4-yl]-8-methyl-1,4-dihydro-benzo[d][1,3] oxazin-2-one |
| 162 | 1-[1-(3-Chloro-benzenesulfonyl)-piperidin-4-yl]-methyl-1,4-dihydro-benzo[d][1,3]oxazin-2-one |
| 163 | 1-[1-(3,4-Dimethoxy-benzenesulfonyl)-piperidin-4-yl]-8-methyl-1,4-dihydro-benzo[d][1,3] oxazin-2-one |
| 164 | 8-Methyl-1-(1-pentafluorobenzenesulfonyl-piperidin-4-yl)-1,4-dihydro-benzo[d][1,3]oxazin-2-one |
| 165 | 6-Methyl-1-[1-(toluene-3-sulfonyl)-piperidin-4-yl]-1,4-dihydro-benzo[d][1,3]oxazin-2-one |
| 166 | 1-[1-(5-Fluoro-2-methyl-benzenesulfonyl)-piperidin-4-yl]-6-methyl-1,4-dihydro-benzo[d][1,3]oxazin-2-one |
| 167 | 1-[1-(4-Isopropoxy-benzenesulfonyl)-piperidin-4-yl]-6-methyl-1,4-dihydro-benzo[d][1,3]oxazin-2-one |
| 168 | 1-[1-(3-Chloro-benzenesulfonyl)-piperidin-4-yl]-6-methyl-1,4-dihydro-benzo[d][1,3]oxazin-2-one |
| 169 | 1-[1-(3,4-Dimethoxy-benzenesulfonyl)-piperidin-4-yl)-6-methyl-1,4-dihydro-benzo[d][1,3]oxazin-2-one |
| 170 | 6-Methyl-1-(1-pentafluorobenzenesulfonyl-piperidin-4-yl)-1,4-dihydro-benzo[d][1,3]oxazin-2-one |
| 171 | 6-Methyl-1-[1-(4-trifluoromethoxy-benzenesulfonyl)-piperidin-4-yl-1,4-dihydro-benzo[d][1,3]oxazin-2-one |
| 172 | 6-Methyl-1-[1-(2-nitro-4-trifluoromethyl-benzenesulfonyl)-piperidin-4-yl]-1,4-dihydro-benzo[d][1,3]oxazin-2-one |
| 173 | 1-[1-(3-Fluoro-benzenesulfonyl)-piperidin-4-yl]-6-methyl-1,4-dihydro-benzo[d][1,3]oxazin-2-one |
| 174 | 1-[1-(2,4-Dichloro-benzenesulfonyl)-piperidin-4-yl]-6-methyl-1,4-dihydro-benzo[d][1,3]oxazin-2-one |
| 175 | 6-Methyl-1-[1-(2,4,6-trimethyl-benzenesulfonyl)-piperidin-4-yl]-1,4-dihydro-benzoo[d][1,3]oxazin-2-one |
| 176 | 6-Methyl-1-[1-(2-trifluoromethyl-benzenesufonyl)piperidin-4-yl]-1,4-dihydro-benzo[d][1,3]oxazin-2-one |
| 177 | 1-[1-(3-Methoxy-benzenesulfonyl)-piperidin-4-yl]-6-methyl-1,4-dihydro-benzo[d][1,3]oxazin-2-one |
| 178 | 6-Methyl-1-[1-(2-nitro-benzenesulfonyl)-piperidin-4-yl]-1,4-dihydro-benzo[d][1,3]oxazin-2-one |
| 179 | 1-[1-(4-Acetyl-benzenesulfonyl)-piperidin-4-yl]-6-methyl-1,4-dihydro-benzo[d][1,3]oxazin-2-one |
| 180 | 1-[1-(4-Methanesulfonyl-benzenesulfonyl)-piperidin-4-yl]-6-methyl-1,4-dihydro-benzo[d][1,3]oxazin-2-one |
| 181 | 6-Methyl-1-(1-phenylmethanesulfonyl-piperidin-4-yl)-1,4-dihydro-benzo[d][1,3]oxazin-2-one |
| 182 | 2-[4-(6-Methyl-oxo-4H-benzo[d][1,3]oxazin-1-yl)-piperidine-1-sulfonyl]benzoic acid methyl ester |
| 183 | 6-Methyl-1-[1-(2-oxo-2H-chromene-6-sulfonyl)-piperidin-4-yl]-1,4-dihydro-benzo[d][1,3]oxazin-2-one |
| 184 | 6-Chloro-1-[1-(4-fluoro-benzenesulfonyl)-piperidin-4-yl]-1,4-dihydro-benzo[d][1,3]oxazin-2-one |
| 185 | 6-Chloro-1-[1-(3,5-dichloro-benzonesulfonyl)-piperidin-4-yl]-1,4-dihydro-benzo[d][1,3]oxazin-2-one |
| 186 | 1-{1-[4-(4-Bromo-phenoxy)-benzenesulfonyl]-piperidin-4-yl]-6-chloro-1,4-dihydro-benzo[d][1,3]oxazin-2-one |
| 187 | 6-Chloro-1-[1-(thiophene-2-sulfonyl)-piperidin-4-yl]-1,4-dihydro-benzo[d][1,3]oxazin-2-one |
| 188 | 6-Chloro-1-[1-(3-methoxy-benzenesulfonyl)-piperidin-4-yl]-1,4-dihydro-benzo[d][1,3]oxazin-2-one |
| 189 | 6-Chloro-1-[1-(2-oxo-2H-chromene-6-sulfonyl)-piperidin-4-yl]-1,4-dihydro-benzo[d][1,3]oxazin-2-ane |
| 190 | 6-Chloro-1-[1-(toluene-3-sulfonyl)-piperidin-4-yl]-1,4-dihydro-benzo[d][1,3]oxazin-2-one |
| 191 | 6-Chloro-1-[1-(5-fluoro-2-methyl-benzenesulfonyl)-piperidin-4-yl]-1,4-dihydro-benzo[d][1,3]oxazin-2-one |
| 192 | 6-Chloro-1-[1-(4-isopropoxy-benzenesulfonyl)-piperidin-4-yl]-1,4-dihydro-benzo[d][1,3]-oxazin-2-one |
| 193 | 6-Chloro-1-[1-(3-chloro-benzenesulfonyl)-piperidin-4-yl]-1,4-dihydro-benzo[d][1,3]oxazin-2-one |
| 194 | 6-Chloro-1-[1-(3,4-dimethoxy-benzenesulfonyl)-piperidin-4-yl]-1,4-dihydro-benzo[d][1,3]axazin-2-one |
| 195 | 6-Chloro-1-(1-pentafluorobenzenesulfonyl-piperidin-4-yl)-1,4-dihydro-benzo[d][1,3]oxazin-2-one |
| 196 | 6-Chloro-1-[1-(4-trifluoromethoxy-benzenesulfonyl)-piperidin-4-yl]-1,4-dihydro-benzo[d][1,3]oxazin-2-one |
| 197 | 6-Chloro-1-[1-(2-nitro-4-trifluoromethyl-benzenesulfonyl)-piperidin-4-yl]-1,4-dihydro-benzo[d][1,3]oxazin-2-one |
| 198 | 6-Chloro-1-[1-(3-fluoro-benzenesulfonyl)-piperidin-4-yl]-1,4-dihydro-benzo[d][1,3]oxazin-2-one |
| 199 | 6-Chloro-1-[1-(2,4-dichloro-benzenesulfonyl)-piperidin-4-yl]-1,4-dihydro-benzo[d][1,3]oxazin-2-one |
| 200 | 6-Chloro-1-[1-(2,4,6-trimethyl-benzenesulfonyl)-piperidin-4-yl]-1,4-dihydro-benzo[d][1,3]oxazin-2-one |
| 201 | 6-Chloro-1-[1-(2-trifluoromethyl-benzenesulfonyl)-piperidin-4-yl]-1,4-dihydro-benzo[d][1,3]oxazin-2-one |
| 202 | 1-[1-(2-Oxo-2H-chromene-6-sulfonyl)-piperidin-4-yl]-1,4-dihydro-benzo[d][1,3]oxazin-2-one |
| 203 | 1-[1-(3,5-Dichloro-benzenesulfonyl)-piperidin-4-yl]-1,4-dihydro-benzo[d][1,3]oxazin-2-one |
| 204 | 1-[1-(2,5-Dichloro-benzenesulfonyl)-piperidin-4-yl]-1,4-dihydro-benzo[d][1,3]oxazin-2-one |
| 205 | 1-[1-(5-Bromo-6-chloro-pyridine-3-sulfonyl)-piperidin-4-yl]-1,4-dihydro-benzo[d][1,3]oxazin-2-one |
| 206 | 1-[1-(4-Chloro-benzenesulfonyl)-piperidin-4-yl]-1,4-dihydro-benzo[d][1,3]oxazin-2-one |
| 207 | 1-[1-(2,8-Dichloro-benzenesulfonyl)-piperidin-4-yl]-1,4-dihydro-benzo[d][1,3]oxazin-2-one |
| 208 | 8-Methyl-1-[1-(2-oxo-2H-chromene-6-sulfonyl)-piperidin-4-yl]-1,4-dihydro-benzo[d][1,3]oxazin-2-one |
| 209 | 1-[1-(3,5-Dichloro-benzensulfonyl)-piperidin-4-yl]-8-methyl-1,4-dihydro-benzo[d][1,3]oxazin-2-one |
| 210 | 1-[1-(2,5-Dichloro-benzenesulfonyl)-piperidin-4-yl]-8-methyl-1,4-dihydro-benzo[d][1,3]oxazin-2-one |
| 211 | 1-[1-(5-Bromo-6-chloro-pyridine-3-sulfonyl)piperidin-4-yl]-8-methyl-1,4-dihydro-benzo[d][1,3]oxazin-2-one |
| 212 | 1-[1-(4-Chloro-benzenesulfonyl)-piperidin-4-yl]-8-methyl-1,4-dihydro-benzo[d][1,3]oxazin-2-one |
| 213 | 1-[1-(2,6-Dichloro-benzenesulfonyl)-piperidin-4-yl-8-methyl-1,4-dihydro-benzo[d][1,3]oxazin-2-one |
| 214 | 1-[1-(Biphenyl-4-sulfonyl)-piperidin-4-yl]-1,4-dihydro-benzo[d][1,3]oxazin-2-one |
| 215 | 6-Chloro-1-[1-(2,5-dichloro-benzenesulfonyl)-piperidin-4-yl]-1,4-dihydro-benzo[d][1,3]oxazin-2-one |
| 216 | 1-[1-(5-Bromo-6-chloro-pyridine-3-sulfonyl)-piperidin-4-yl]-6-chloro-1,4-dihydro-benzo[d][1,3]oxazin-2-one |
| 217 | 6-Chloro-1-[1-(4-chloro-benzenesulfonyl)-piperidin-4-yl]-1,4-dihydro-benzo[d][1,3]oxazin-2-one |
| 218 | 6-Chloro-1-[1-(2,6-dichloro-benzenesulfonyl)-piperidin-4-yl]-1,4-dihydro-benzo[d][1,3]oxazin-2-one |
| 219 | 1-[1-(Biphenyl-4-sulfonyl]-piperidin-4-yl]-6-methyl-1,4-dihydro-benzo[d][1,3]oxazin-2-one |
| 220 | 2-[4-(6-Methyl-2-oxo-4H-benzo[d][1,3]oxazin-1-yl)-piperidine-1-sulfonyl]-benzonitrile |
| 221 | 1-[1-(2,5-Dichloro-benzenesulfonyl)-piperidin-4-yl]-6-methyl-1,4-dihydro-benzo[d][1,3]oxazin-2-one |
| 222 | 1-[1-(5-Bromo-6-chloro-pyridine-3-sulfonyl)-piperidin-4-yl]-6-methyl-1,4-dihydro-benzo[d][1,3]oxazin-2-one |
| 223 | 1-[1-(4-Chloro-benzenesulfonyl)-piperidin-4-yl]-6-methyl-1,4-dihydro-benzo[d][1,3]oxazin-2-one |
| 224 | 1-[1-(2,6-Dichloro-benzenesulfonyl)-piperidin-4-yl]-8-methyl-1,4-dihydro-benzo[1,3]oxazn-2-one |
| 225 | 1-[1-(3,5-Dichloro-benzenesulfonyl)-piperidin-4-yl]-6-methyl-1,4-dihydro-benzo[d][1,3]oxazin-2-one |
| 226 | 6-Methyl[d]-1-[1-(1-methyl-1H-imidazole-4-sulfonyl)-piperidin-4-yl]-1,4-dihydro-benzo[d][1,3]oxazin-2-one |
| 227 | 1-[1-(5-Bromo-2,4-difluoro-benzenesulfonyl)-piperidin-4-yl]-6-methyl-1,4-dihydro-benzo[d][1,3]oxazin-2-one |
| 228 | 1-[1-(4-Methanesulfonyl-benzenesulfonyl)-piperidin-4-yl]-1,4-dihydro-benzo[d][1,3]oxazin-2-one |
| 229 | 1-[1-(1-Methyl-1H-imidazole-4-sulfonyl)-piperidin-4-yl]-1,4-dihydro-benzo[d][1,3]oxazin-2-one |
| 230 | 1-[1-(5-Bromo-2,4-difluoro-benzenesulfonyl)-piperidin-4-yl]-1,4-dihydro-benzo[d][1,3]oxazin-2-one |
| 231 | 1-[1-(6-Chloro-imidazo[2,1-b]thiazole-5-sulfonyl)-piperidin-4-yl]-1,4-dihydro-benzo[d][1,3]oxazin-2-one |
| 232 | 1-[1-(4-Ethyl-benzenesulfonyl)-piperidin-4-yl]-1,4-dihydro-benzo[d][1,3]oxazin-2-one |
| 233 | 1-[1-(Benzo[b]thiophene-3-sulfonyl)-piperidin-4-yl]-1,4-dihydro-benzo[d][1,3]oxazin-2-one |
| 234 | 1-[1-(6-Chloro-imidazo[2,1-b]thiazole-5-sulfonyl)-piperidin-4-yl]-8-methyl-1,4-dihydro-benzo[d][1,3]oxazin-2-one |
| 235 | 1-[1-(4-Ethyl-benzenesulfonyl)-piperidin-4-yl]-8-methyl-1,4-dihydro-benzo[d][1,3]oxazin-2-one |
| 236 | 1-[1-(Benzo[b]thiophene-3-sulfonyl)-piperidin-4-yl]-8-methyl-1,4-dihydro-benzo[d][1,3]oxazin-2-one |
| 237 | 6-Chloro-1-[1-(6-chloro-imidazo[2,1-b]thiazole-5-sulfonyl)-piperidin-4-yl]-1,4-dihydro-benzo[d][1,3]oxazin-2-one |
| 238 | 6-Chloro-1-[1-(4-ethyl-benzenesulfonyl)-piperidin-4-yl]-1,4-dihydro-benzo[1,3]oxazin-2-one |
| 239 | 1-[1-(Benzo[b]thiophene-3-sulfonyl)-piperidin-4-yl]-6-chloro-1,4-dihydro-benzo[d][1,3]oxazin-2-one |
| 240 | 1-[1-(6-Chloro-imidazo[2,1-b]thiazole-5-sulfonyl)-piperidin-4-yl]-6-methyl-1,4-dihydro-benzo[d][1,3]oxazin-2-one |
| 241 | 1-[1-(4-Ethyl-benzenesulfonyl)-piperidin-4-yl]-6-methyl-1,4-dihydro-benzo[d][1,3]oxazine-2-one |
| 242 | 1-[1-(Benzo[b]thiophene-3-sulfonyl)piperidin-4-yl]-6-methyl-1,4-dihydro-benzo[1,3]oxazin-2-one |
| 243 | 1-[1-(7-Chloro-benzo[1,2,5]oxadiazole-4-sulfonyl)-piperidin-4-yl]-1,4-dihydro-benzo[d][1,3]oxazin-2-one |
| 244 | 1-[1-(2-Methoxy-4-methyl-benzenesulfonyl)-piperidin-4-yl]-1,4-dihydro-benzo[d][1,3]oxazin-2-one |
| 245 | 3{4-[4-[2-Oxo-4H-benzo[d][1,3]oxazin-1-yl)-piperidine-1-sulfonyl]-phenyl]-propionic acid methyl ester |
| 246 | 1-[1-(2,4-Dinitro-benzenesulfonyl)-piperidin-4-yl]-1,4-dihydro-benzo[d][1,3]oxazin-2-one |
| 247 | 1-[1-(7-Chloro-benzo[1,2,5]oxadiazole-4-sulfonyl)-piperidin-4-yl]-8-methyl-1,4-dihydro-benzo[d][1,3]oxazin-2-one |
| 248 | 1-[1-(2-Methoxy-4-methyl-benzenesulfonyl)-piperidin-4-yl]-8-methyl-1,4-dihydro-benzo[d][1,3]oxazin-2-one |
| 249 | 3-(4-[4-(8-Methyl-2-oxo-4H-benzo[d][1,3]oxazin-1-yl)-piperidine-1-sulfonyl]-phenyl]-propionic acid methyl ester |
| 250 | 1-[1-(2,4-Dinitro-benzenesulfonyl)-piperidin-4-yl]-methyl-1,4-dihydro-benzo[d][1,3]oxazin-2-one |
| 251 | 1-[1-(7-Chloro-benzo[1,2,5]oxadiazole-4-sulfonyl)-piperidin-4-yl]-6-methyl-1,4-dihydro-benzo[d][1,3]oxazin-2-one |
| 252 | 1-[1-(2-Methoxy-4-methyl-benzenesulfonyl)-piperidin-4-yl]-6-methyl-1,4-dihydro-benzo[d][1,3]oxazin-2-one |
| 253 | 3-{4-[4-(6-Methyl-2-oxo-4H-benzo[d][1,3]oxazin-1-yl)-piperidine-1-sulfonyl]-phenyl}-propionic acid methyl ester |
| 254 | 1-[1-(2,4-Dinitro-benzenesulfonyl)-piperidin-4-yl]-6-methyl-1,4-dihydro-benzo[d][1,3]oxazin-2-one |
| 255 | 6-Chloro-1-[1-(7-chloro-benzo[1,2,5]oxadiazole-4-sulfonyl)-piperidin-4-yl]-1,4-dihydro-benzo[d][1,3]oxazin-2-one |
| 256 | 6-Chloro-1-[1-(2-methoxy-4-methyl-benzenesulfonyl)-piperidin-4-yl]-1,4-dihydro-benzo[d][1,3]oxazin-2-one |
| 257 | 3-(4-[4-(6-Chloro-2-oxo-4H-benzo[d][1,3]oxazin-1-yl)-piperidine-1-sulfonyl]-phenyl}-propionic acid methyl ester |
| 258 | 6-Chloro-1-[1-(2,4-dinitro-benzenesulfonyl)-piperidin-4-yl]-1,4-dihydro-benzo[d][1,3]oxazin-2-one |
| 259 | 6-Chloro-1-[1(1-methyl-1H-imidazole-4-sulfonyl)-piperidin-4-yl]-1,4-dihydro-benzo[d][1,3]oxazin-2-one |
| 260 | 1-[1-(5-Bromo-2,4-difiuoro-benzenesulfonyl)-piperidin-4-yl]-6-chloro-1,4-dihydro-benzo[d][1,3]oxazin-2-one |
| 261 | 8-Methyl-1-[1-(1-methyl-1H-imidazole-4-sulfonyl)-piperidin-4-yl]-1,4-dihydro-benzo[d][1,3]oxazin-2-one |
| 262 | 1-[1-(5-Bromo-2,4-difluoro-benzenesulfonyl)-piperidin-4-yl]-8-methyl-1,4-dihydro-benzo[d][1,3]oxazin-2-one |
| 263 | 1-[1-(Benzo[b]thiophene-2-sulfonyl)-piperidin-4-yl]-8-methyl-1,4-dihydro-benzo[d][1,3]oxazin-2-one |
| 264 | 1-[1-(Benzo[b]thiophene-2-sulfonyl)-piperidin-4-yl]-1,4-dihydro-benzo[d][1,3]oxazin-2-one |
| 265 | 1-[1-(Benzo[b]thiophene-2-sulfonyl)-piperidin-4-yl]-6-chloro-1,4-dihydro-benzo[d][1,3]oxazin-2-one |
| 265 | 1-[1-(Benzo[b]thiopheno-2-sulfonyl)-piperidin-4-yl]-6-methyl-1,4-dihydro-benzo[d][1,3]oxazin-2-one |
| 267 | 1-[1-[2,5-Difluoro-benzenesulfonyl)-piperidin-4-yl]-1,4-dihydro-benzo[d][1,3]oxazin-2-one |
| 268 | 1-[1-(2,5-Difluoro-benzenesulfonyl)-piperidin-4-yl]-8-methyl-1,4-dihydro-benzo[d][1,3]oxazin-2-one |
| 269 | 6-Chloro-1-[1-(2,5-difluoro-benzenesulfonyl)-piperidin-4-yl]-1,4-dihydro-benzo[d][1,3]oxazin-2-one |
| 270 | 1-[1-(2,5-Difluoro-benzenesulfonyl)-piperidin-4-yl]-6-methyl-1,4-dihydro-benzo[d][1,3]oxazin-2-one |
| 271 | 1-[1-(4-Chloro-2,5-difluoro-benzenesulfonyl)-piperidin-4-yl]-1,4-dihydro-benzo[d][1,3]oxazin-2-one |
| 272 | 1-[1-(4-Chloro-2,5-difluoro-benzenesulfonyl)-piperidin-4-yl]-8-methyl-1,4-dihydro-benzo[d][1,3]oxazin-2-one |
| 273 | 6-Chloro-1-[1-(4-chloro-2,5-difluoro-benzenesulfonyl)-piperidin-4-yl]-1,4-dihydro-benzo[d][1,3]oxazin-2-one |
| 274 | 1-[1-(4-Chloro-2,5-difluoro-benzenesulfonyl)-piperidin-4-yl]-6-methyl-1,4-dihydro-benzo[d][1,3]oxazin-2-one |
| 275 | 1-[1-(2,4,5-Trifluoro-benzenesulfonyl)-piperidin-4-yl]-1,4-dihydro-benzo[d][1,3]oxazin-2-one |
| 276 | 8-Methyl-1-[1-(2,4,5-trifluoro-benzenesulfonyl}-pipendin-4-yl]-1,4-dihydro-benzo[d][1,3]oxazin-2-one |
| 277 | 6-Chloro-1-[1-(2,4,5-trifluoro-benzenesulfonyl)-piperidin-4-yl]-1,4-dihydro-benzo[d][1,3]oxazin-2-one |
| 278 | 6-Methyl-1-[1-(2,4,5-trifluoro-benzenesulfonyl)-piperidin-4-yl]-1,4-dihydro-benzo[d][1,3]oxazin-2-one |
| 279 | 1-[1-(3,5-Dichloro-2-hydroxy-benzenesulfonyl)-piperidin-4-yl]-1,4-dihydro-benzo[d][1,3]oxazin-2-one |
| 280 | 1-[1-(2,6-Difluoro-benzenesulfonyl)-piperidin-4-yl]-1,4-dihydro-benzo[d][1,3]oxazin-2-one |
| 281 | 1-[1-(2,6-Difluoro-benzenesulfonyl)-piperidin-4-yl]-8-methyl-1,4-dihydro-benzo[d][1,3]oxazin-2-one |
| 282 | 6-Chloro-1-[1-(2,6-difluoro-benzenesulfonyl)-piperidin-4-yl]-1,4-dihydro-benzo[d][1,3]oxazin-2-one |
| 283 | 1-[1-(2,6-Difluoro-benzenesulfonyl)-piperidin4-yl]-6-methyl-1,4-dihydro-benzo[d][1,3]oxazin-2-one |
| 284 | 1-[1-(5-Chloro-2,4-difluoro-benzenesulfonyl)-piperidin-4-yl]-1,4-dihydro-benzo[d][1,3]oxazin-2-one |
| 285 | 1-[1-(5-Chloro-2,4-difluoro-benzenesulfonyl)-piperidin-4-yl]-8-methyl-1,4-dihydro-benzo[d][1,3]oxazin-2-one |
| 286 | 6-chloro-1-[1-(5-chloro-2,4-difluro-benzenesulfonyl)-piperidin-4-yl]-1,4-dihydro-benzo[d][1,3]oxazin-2-one |
| 287 | 1-[1-(5-Chloro-2,4-difluoro-benzeneslfonyl)-piperidin-4-yl]-6-methyl-1,4-dihydro-benzo[d][1,3]oxazin-2-one |
| 288 | 1-[1-(2-Chloro-benzenesulfonyl)-piperidin-4-yl]-1,4-dihydro-benzo[d][1,3]oxazin-2-one |
| 289 | 1-[1-(2-Chloro-benzenesulfonyl)-piperidin-4-yl]-8-methyl-1,4-dihydro-benzo[d][1,3]oxazin-2-one |
| 290 | 6-Chloro-1-[1-(2-chloro-benzenesulfonyl)-piperidin-4-yl]-1,4-dihydro-benzo[d][1,3]oxazin-2-one |
| 291 | 1-[1-(2-Chloro-benzenesulfonyl)-piperidin-4-yl]-6-methyl-1,4-dihydro-benzo[d][1,3]oxazin-2-one |
| 292 | 6-Chloro-1-[1-(2-naphthalen-1-yl-ethanesulfonyl)-piperidin-4-yl]-1,4-dihydro-benzo[d][1,3]oxazin-2-one |
| 293 | 6-Bromo-1[1-(4-bromo-benzenesulfanyl)-piperidin-4-yl]-1,4-dihydro-benzo[d][1,3]oxazin-2-one |
| 294 | 6-Bromo-1-[1-(toluene-4-sulfonyl)-piperidin-4-yl]-1,4-dihydro-benzo[d][1,3]oxazin-2-one |
| 295 | 6-Bromo-1-[1-(2,4-dimethyl-benzenesulfonyl)-piperidin-4-yl]-1,4-dihydro-' benzo[d][1,3]oxazin-2-one |
| 296 | 6-Bromo-1-[1-(2-naphthalen-1-yl-ethanesulfonyl)-piperidin-4-yl]-1,4-dihydro-benzo[d][1,3]oxazin-2-one |
| 297 | 6-Bromo-1-[1-(quinoline-8-sulfonyl)-piperidin-4-yl]-1,4-dihydro-benzo[d][1,3]oxazin-2-one |
| 298 | 6-Bromo-1-[1-(5-chloro-3-methyl-benzo[b]thiophene-2-sulfonyl)-piperidin-4-yl]-1,4-dihydro-298 benzo[d][1,3]oxazin-2-one |
| 299 | 6-Bromo-1-[1-(3-nitro-benzene-sulfonyl)-piperidin-4-yl]-1,4-dihydro-benzo[d][1,3]oxazin-2-one |
| 300 | 6-Brumo-1-[1-(naphthalene-1-sulfonyl)-piperidin-4-yl]-1,4-dihydro-benzo[d][1,3]oxazin-2-one |
| 301 | 6-Bromo-1-[1-(naphthalene-2-sulfonyl)-piperidin-4-yl]-1,4-dihydro-benzo[d][1,3]oxazin-2one |
| 302 | 1-(1-Benzenesulfonyl-piperidin-4-yl)-6-bromo-1,4-dihydro-benzo[d][1,3]oxazin-2-one |
| 303 | 6-Bromo-1-(1-[4-(4-bromo-phenoxy)-benzenesulfonyl]-piperidin-4-yl]-1,4-dihydro-benzo[d][1,3]oxazin-2-one |
| 304 | 6-Bromo-1-[1-(thiophene-2-sulfonyl)-piperidin-4-yl]-1,4-dihydro-benzo[d][1,3]oxazin-2-one |
| 305 | 6-Bromo-1-[1-(2-methyl-5-nitro-benzenesulfonyl)-piperidin-4-yl]-1,4-dihydro-benzo[d][1,3]oxazin-2-one |
| 306 | 6-Bromo-1-[1-(4-bromo-2,5-difluoro-benzenesulfonyl)-piperidin-4-yl]-1,4-dihydro-benzo[d][1,3]oxazin-2-one |
| 307 | 6-Bromo-1-[1-(toluene-3-sulfonyl)-piperidin-4-yl]-1,4-dihydro-benzo[d][1,3]oxazin-2-one |
| 308 | 6-Bromo-1-[1-(5-fluoro-2-methyl-benzonesulfonyl)-piperidin-4-yl]-1,4-dihydro-benzo[d][1,3]oxazin-2-one |
| 309 | 6-Bromo-1-[1-(4-isopropoxy-benzenesulfonyl)-piperidin-4-yl]-1,4-dihydro-benzo[d][1.3]oxazin-2-one |
| 310 | 6-Bromo-1-[1-(3-chloro-benzenesullfonyl)-piperidin-4-yl]-1,4-dihydro-benzo[d][1,3]oxazin-2-one |
| 311 | 6-Bromo-1-[1-(3,4,-dimethoxy-benzenesulfonyl-piperidin-4yl]-1,4-dihydro-benzo[d][1,3]oxazin-2-one |
| 312 | 6-Bromo-1-[1-pentafluorobenzenesulfonyl-piperidin-4-yl)-1,4-dihydro-benzo[d][1,3]oxazin-2-one |
| 313 | 6-Bromo-1-[1-(4-chloro-2,5-dimethyl-benzenesulfonyl)-piperidin-4-yl]-1,4-dihydro-benzo[d][1,3]oxazin-2-one |
| 314 | 6-Bromo-1-[1-(3-methoxy-benzenesulfonyl)-piperidin-4-yl]-1,4-dihydro-benzo[d][1,3]oxazin-2-one |
| 315 | 6-Bromo-1-[1-(4-isopropyl-benzenesulfonyl)-piperidin-4-yl]-1,4-dihydro-benzo[d][1,3]oxazin-2-one |
| 316 | 6-Bromo-1-[1-(4 fluoro-benzenesulfonyl)-piperidin-4-yl]-1,4-dihydro-benzo[d][1,3]oxazin-2-one |
| 317 | 6-Bromo-1-[1-(3-chloro-4-fluoro-benzenesulfonyl)-piperidin-4-yl]-1,4-dihydro-benzo[d][1,3]oxazin-2-one |
| 318 | 6-Bromo-1-(1-pentamethylbenzenesulfonyl-piperidin-4-yl)-1,4-dihydro-benzo[d][1,3]oxazin-2-one |
| 319 | 6-Bromo-1-[1-(2-nitro-benzenesulfonyl)-piperidin-4-yl]-1,4-dihydro-benzo[d][1,3]oxazin-2-one |
| 320 | 6-Bromo-1-[1-(4-chloro-3-nitro-benzenesulfonyl)-piperidin-4-yl]-1,4-dihydro-benzo[d][1,3]oxazin-2-one |
| 321 | 6-Bromo-1-[1-(5-dimethylamino-naphthatene-1-sulfonyl)-piperidin-4-yl]-1,4-dihydro-benzo[d][1,3]oxazin-2-one |
| 322 | 6-Bromo-1[1-(4-nitro-benzenesulfonyl)-piperidin-4-yl]-1,4-dihydro-benzo[d][1,3]oxazin-2-one |
| 323 | 1-[1-(4-Acetyl-benzenesulfonyl)-piperidin-4-yl]-6-bromo-1,4-dihydro-benzo[d][1,3]oxazin-2-one |
| 324 | 6-Bromo-1-[1-(4-methanesulfonyl-benzenesulfonyl)-piperidin-4-yl]-1,4-dihydro-benzo[d][1,3]oxazin-2-one |
| 325 | 1-[7-(Biphenyl-4-sulfonyl)-piperidin-yl]-6-bromo-1,4-dihydro-benzo[d][1,3]oxazin-2-one |
| 326 | 6-Bromo-1-(1-phenylmethanesulfonyl-piperidin-4-yl)-1,4-dihydro-benzo[d][1,3]oxazin-2-one |
| 327 | 6-Bromo-1-[1-(2,5-dimethoxy-benzenesulfonyl)-piperidin-4-yl]-1,4-dihydro-benzo[d][1,3]oxazin-2-one |
| 328 | 6-Bromo-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 329 | 5-Bromo-1-[1-(2,3-dichloro-benzenesulfonyl)-piperidin-4-yl-1,4-dihydro-benzo[d][1,3]oxazin-2-one |
| 330 | 6-Bromo-1-[1-(2,4,5-trichloro-benzenesulfonyl)-piperidin-4-yl]-1,4-dihydro-benzo[d][1,3]oxazin-2-one |
| 331 | 6-Bromo-1-[1-(5-bromo-2-methoxy-benzenesulfonyl)-piperidin-4-yl]-1,4-dihydro-benzo[d][1,3]oxazin-2-one |
| 332 | 6-Bromo-1-[1-(4-trifluoromethoxy-benzenesulfonyl)-piperidin-4-yl]-1,4-dihydro-benzo[d][1,3]oxazin-2-one |
| 333 | 6-Bromo-1-[1-(2-nitro-4-trifluoromethyl-benzenesulfonyl)-piperidin-4-yl]-1,4-dihydro-benzo[d][1,3]oxazin-2-one |
| 334 | 6-Bromo-1-[1-(3-fluoro-benzenesulfonyl)-piperidin-4-yl]-1,4-dihydro-benzo[d][1,3]oxazin-2-one |
| 335 | 6-Bromo-1-[1-(2,4-dichloro-benzenesulfonyl)-piperidin-4-yl]-1,4-dihydro-benzo[d][1,3]oxazin-2-one |
| 336 | 6-Bromo-1-[1-(2,4,6-trimethyl-benzenesulfonyl)-piperidin-4-yl]-1,4-dihydro-benzo[d][1,3]oxazin-2-one |
| 337 | 6-Bromo-1-[1-(2-trifluoromethyl-benzenesulfonyl)-plperidin-4-yl]-1,4-dihydro-benzo[d][1,3]oxazin-2-one |
| 338 | 6-Bromo-1-[1-(2-bromo-benzenesulfonyl)-piperidin-4-yl]-1,4-dihydro-benzo[d][1,3]oxazin-2-one |
| 339 | Bromo-1-[1-(4-methoxy-2,3,6-trimethyl-benzenesulfonyl)-piperidin-4-yl]-1,4]-dihydro-benzo[d][1,3]oxazin-2-one |
| 340 | 1-[1-(3,5-Dichloro-4-hydroxy-benzenesulfonyl)-piperidin-4-yl]-1,4-dihydro-benzo[d][1,3]oxazin-2-one |
| 341 | 1-[1-(3,5-Dichloro-4-hydroxy-benzenesulfonyl)-piperidin-4-yl]-8-methyl-1,4-dihydro-benzo[d][1,3]oxazin-2-one |
| 342 | 6-Chloro-1-[1-(3,5-dichloro-4-hydroxy-benzenesulfonyl)-piperidin-4-yl]-1,4-dihydro-benzo[d][1,3]oxazin-2-one |
| 343 | 1-[1-(3,5-Dichloro-4-hydroxy-benzenesulfonyl)-piperidin-4-yl]-6-methyl-1,4-dihydro-benzo[d][1,3]oxazin-2-one |
| 344 | 6-Bromo-1-[1-(3,5-dichloro-4-hydroxy-benzenesulfonyl-piperidin-4-yl]-1,4-dihydro-benzo[d][1,3]oxazin-2-one |
| 345 | 6-Chloro-1-[1-(3,5-dichloro-2-hydroxy-benzenesulfonyl)-piperidin-4-yl]-1,4-dihydro-benzo[d][1,3]oxazin-2-one |
| 346 | 6-Bromo-1-[1-(3,5-dichloro-2-hydroxy-benzenesulfonyl)-piperidin-4-yl]-1,4-dihydro-benzo[d][1,3]oxazin-2-one |
| 347 | 2-[4-(6-Bromo-2-oxo-4H-benzo[d]1,3]oxazin-1-yl)-piperidine-1-sulfonyl]-benzonitrile |
| 348 | 6-Bromo-1-[1-(4-methoxy-benzenesulfonyl)-piperidin-4-yl]-1,4-dihydro-benzo[d][1,3]oxazin-2-one |
| 349 | 2-[4-(6-Bromo-2-oxo-4H-benzo[d][1,3]oxazin-1-yl)-piperidine-1-sulfonyl]-benzoic acid methyl ester |
| 350 | 6-Bromo-1-[1-(3-trifluoromethyl-benzenesulfonyl)-piperidin-4-yl]-1,4-dihydro-benzo[d][1,3]oxazin-2-one |
| 351 | 6-Bromo-1-[1-[1-(2-oxo-2H-chromene-6-sulfonyl)-piperidin-4-yl]-1,4-dihydro-benzo[d][1,3]oxazin-2-one |
| 352 | 6-Bromo-1-[1-(3,5-dichloro-benzenesulfanyl)-piperidin-4-yl]-1,4-dihydro-benzo[d][1,3]oxazin-2-one |
| 353 | 6-Bromo-1-[1-(2,5-dichloro-benzenesulfonyl)-piperidin-4-yl]-1,4-dihydro-benzo[d][1,3]oxazin-2-one |
| 354 | 6-Bromo-1-[1-(5-bromo-6-chloro-pyridine-3-sulfonyl)-piperidin-4-yl]-1,4-dihydro-benzo[d][1,3]oxazin-2-one |
| 355 | 6-Bromo-1-[1-(4-chloro-benzenesulfonyl)-piperidin-4-yl]-1,4-dihydro-benzo[d][1,3]oxazin-2-one |
| 356 | 6-Bromo-1-[1-(2,6-dichloro-benzenesulfonyl)-piperidin-4-yl]-1,4-dihydro-benzo[d][1,3]oxazin-2-one |
| 357 | 6-Bromo-1-[1-(1-methyl-1H-imidazole-4-sulfonyl)-piperidin-4-yl]-1,4-dihydro-benzo[d][1,3]oxazin-2-one |
| 358 | 6-Bromo-1-[1-(5-bromo-2,4-difluoro-benzenesulfonyl)-piperidin-4-yl]-1,4-dihydro-benzo[d][1,3]oxazin-2-one |
| 359 | 6-Bromo-1[1-(4-ethyl-benzenesulfonyl)-piperidin-4-yl]-1,4-dihydro-benzo[d][1,3]oxazin-2 one |
| 360 | 6-Bromo-1-[1-(6-chloro-imidazo[2,1-b]thiazole-5-sulfonyl)-piperidin-4-yl]-1,4-dihydro-benzo[d]1,3]oxazin-2-one |
| 361 | 1-[Benzo[b]thiophene-3-sulfonyl)-piperidin-4-yl]-6-bromo-1,4-dihydro-benzo[d][1,3]oxazin-2-one |
| 362 | 6-Bromo-1-[1-(7-chloro-benzo[1,2,5]oxadiazole-4-sulfonyl)-piperidin-4-yl]-1,4-dihydro-benzo[d][1,3]oxazin-2-one |
| 363 | 6-Bromo-1-[1-(2-methoxy-4-methyl-benzenesulfonyl)-piperidin-4-yl]-1,4-dihydro-benzo[d][1,3]oxazin-2-one |
| 364 | 3{4-[4-(6-Bromo-2-oxo-4H-benzo[d][1,3]oxazin-1-yl)-piperidine-1-sulfonyl]-phenyl}-propionic acid methyl ester |
| 365 | 6-Bromo-1-[-(2,4-dinitro-benzenesulfonyl)-piperidin-4-yl]-1,4-dihydro-benzo[d][1,3]oxazin-2-one |
| 366 | 1-[1-(Benzo[b]thiophene-2-sulfonyl-piperidin-4-yl]-6-bromo-1,4-dihydro-benzo[d][1,3]oxazin-2-one |
| 367 | 6-Bromo-1-[1-(2,5-difluoro-benzenesulfonyl)-piperidin-4-yl]-1,4-dihydro-benzo[d][1,3]oxazin-2-one |
| 368 | 6-Bromo-1-[1-(4-chloro-2,5-difluoro-benzenesulfonyl)-piperidin-4-yl]-1,4-dihydro-benzo[d][1,3]oxazin-2-one |
| 369 | 6-Bromo-1-[1-(2,4,5-fluoro-benzenesulfonyl)-piperidin-4-yl]-1,4-dihydro-benzo[d][1,3]oxazin-2-one |
| 370 | 6-Bromo-1-[1-(2,6-difluoro-benzenesulfonyl)-piperidin-yl]-1,4-dihydro-benzo[d][1,3]oxazin-2-one |
| 371 | 6-Bromo-1-[1-(5-chloro-2,4-difluoro-benzenesulfonyl)-piperidin-4-yl]-1,4-dihydro-benzo[d][1,3]oxazin-2-one |
| 372 | 6-Bromo-1-[1-(2-chloro-benzenesulfonyl)-piperidin-4-yl]-1,4-dihydro-benzo[d][1,3]oxazin-2-one |
| 373 | 6-Bromo-1-[1-(2,3,4-trifluoro-benzenesulfonyl)-piperidin-4-yl]-1,4-dihydro-benzo[d][1,3]oxazin-2-one |
| 374 | N-{4-[4-(6-Bromo-2-oxo-4H-benzo[d][1,3]oxazin-1-yl)-piperidine-1-sulfonyl]-2-chloro-phenyl]-acetamide |
| 375 | 1-[1-(2,3,4-Trifluoro-benzenesulfonyl)-piperidin-4-yl]-1,4-dihydro-benzo[d][1,3]oxazin-2-one |
| 376 | 8-Methyl-1-[1-(2,3,4-trifluoro-benzenesulfonyl)-piperidin-4-yl]-1,4-dihydro-benzo[d][1,3]oxazin-2-one |
| 377 | 6-Chloro-1-[1-(2,3,4-trifluoro-benzenesulfonyl)-piperidin-4-yl]-1,4-dihydro-benzo[d][1,3]oxazin-2-one |
| 378 | 6-Methyl-1-[1-(2,3,4-trifluoro-benzenesulfonyl)-piperidin-4-yl]-1,4-dihydro-benzo[d][1,3]oxazin-2-one |
| 379 | N-(2-Chloro-4[4-(6-methyl-2-oxo-4H-benzo[d][1,3]oxazin-1-yl)-piperidine-1-sulfonyl]-phenyl}-acetamide |
| 380 | 1-[1-(3,4-Difluoro-benzenesulfonyl)-piperidin-4-yl]-1,4-dihydro-benzo[d][1,3]oxazin-2-one |
| 381 | 1-[1-(3,4-Difluoro-benzenesulfonyl)-piperidin-4-yl]-8-methyl-1,4-dihydro-benzo[d][1,3]oxazin-2-one |
| 382 | 6-Chloro-1-[1-(3,4-difluoro-benzenesulfonyl)-piperidin-4-yl]-1,4-dihydro-benzo[d][1,3]oxazin-2-one |
| 383 | 1-[1-(3,4-Difluoro-benzenesulfonyl)-piperidin-4-yl]-6-methyl-1,4-dihydro-benzo[d][1,3]oxazin-2-one |
| 384 | 8-Bromo-1-{1-(3,4-difluoro-benzenesulfonyl)-piperidin-4-yl]-1,4-dihydro-benzo[d][1,3]oxazin-2-one |
| 385 | N-{2-Chloro-4-[4-(8-methyl-2-oxo-4H-benzo[d][1,3]oxazin-1-yl)-piperidine-1-sulfonyl]-phenyl}-acetamide |
| 386 | 1-(1-(2-Chloro-4,5-difluoro-benzenesulfonyl)-piperidin-4-yl]-1,4-dihydro-benzo[d][1,3]oxazin-2-one |
| 387 | 1-[1-(2-Chloro-4,5-difluoro-benzenesulfonyl)-piperidin-4-yl]-8-methyl-1,4-dihydro-benzo[d][1,3]oxazin-2-one |
| 388 | 6-Chloro-1-[1-(2-chloro-4,5-difluoro-benzenesulfonyl)-piperidin-4-yl]-1,4-dihydro-benzo[d][1,3]oxazin-2-one |
| 389 | 1-[1-(2-Chloro-4,5-difuoro-benzenesulfonyl)-piperidin-4-yl]-6-methyl-1,4-dihydro-benzo[d][1,3]oxazin-2-one |
| 390 | 6-Bromo-1-[1-(2-chloro-4,5-difluoro-benzenesulfonyl)-piperidin-4-yl]-1,4-dihydro-benzo[d][1,3]oxazin-2-one |
| 391 | N-[2-Chloro-4-(2-oxo-4H-benzo[d][1,3]oxazin-1-yl)-piperidine-1-sulfonyl]-phenyl]-acetamide |
| 392 | 1-[1-(Benzo[1,2,5]oxadiazole-4-sulfonyl)-piperidin-4-yl]-1,4-dihydro-benzo[d][1,3]oxazin-2-one |
| 393 | 1-[1-(Benzo[1,2,5]oxadiazole-4-sulfonyl)-piperidin-4-yl]-8-methyl-1,4-dihydro-benzo[d][1,3]oxazin-2-one |
| 394 | 1-[1-(Benzo[1,2,5]oxadiazole-4-sulfonyl)-piperidin-4-yl]-6-chloro-1,4-dihydro-benzo[d][1,3]oxazin-2-one |
| 395 | 1-[1-(Benzo[1,2,5]oxadiazole-4-sulfonyl)-piperidin-4-yl]-6-methyl-1,4-dihydro-benzo[d][1,3]oxazin-2-one |
| 396 | 1-[1-(Benzo[1,2,5]oxadiazole-4-sulfonyl)-piperidin-4-yl]-6-bromo-1,4-dihydro-benzo[d][1,3]oxazin-2-one |
| 397 | N-{2-Chloro-4-[4-(6-chloro-2-oxo-4H-benzo[d][1,3]oxazin-1-yl)-piperidine-1-sulfonyl]-phenyl}-acetamide |
| 398 | 1-[1-(Benzo[1,2,5]thiadiazole-4-sulfonyl)-piperidin-4-yl]-1,4-dihydro-benzo[d][1,3]oxazin-2-one |
| 399 | 1-[1-(Benzo[1,2,5]thiadiazole-4-sulfonyl)-piperidin-4-yl]-8-methyl-1,4-dihydro-benzo[d][1,3]oxazin-2-one |
| 400 | 1-[1-(Benzo[1,2,5]thiadiazole-4-sulfonyl)-piperidin-4-yl]-6-chloro-1,4-dihydro-benzo[d][1,3]oxazin-2-one |
| 401 | 1-[1-(Benzo[1,2,5]thiadiazole-4-sulfonyl)-piperidin-4-yl]-6-methyl-1,4-dihydro-benzo[d[1,3]oxazin-2-one |
| 402 | 1-[1-(Benzo[1,2,5]thiadiazole-4-sulfonyl)-pipendin-4-yl]-6-bromo-1,4-dihydro-402 benzo[d][1,3]oxazin-2-one |
| 403 | 1-(1-Ethanesulfonyl-piperidin-4-yl)-8-methyl-1,4-dihydro-benzo[d][1,3]oxazin-2-one |
| 404 | 1-[1-(2,4-Difluoro-benzenesulfonyl)-piperidin-4-yl]-1,4-dihydro-benzo[d][1,3]oxazin-2-one |
| 405 | 1-[1-(2,4-Difluoro-benzenesulfonyl)-piperidin-4-yl]-8-methyl-1,4-dihydro-benzo[d][1,3]oxazin-2-one |
| 406 | 6-Chloro-1-[1-(2,4-difluoro-benzenesulfonyl)-piperidin-4-yl]-1,4-dihydro-benzo[d][1,3]oxazin-2-once |
| 407 | 1-[1-(2,4-Difluoro-benzenesulfonyl]-piperidin-4-yl]-6-methyl-1,4-dihydro-benzo[d][1,3]oxazin-2-one |
| 408 | 6-Bromo-1-[1-(2,4-difluoro-benzenesulfonyl)-piperidin-4-yl]-1,4-dihydro-benzo[d][1,3]oxazin-2-one |
| 409 | 8-Methyl-1-[1-(propane-2-sulfonyl)-piperidin-4-yl]-1,4-dihydro-benzo[d][1,3]oxazin-2-one |
| 410 | 1-[1-(3,4-Dichloro-benzenesulfonyl)-piperidin-4-yl]-1,4-dihydro-benzo[d][1,3]oxazin-2-one |
| 411 | 1-[1-(3,4-Dichloro-benzenesulfonyl)-piperidin-4-yl]-8-methyl-1,4-dihydro-benzo[d][1,3]oxazin-2-one |
| 412 | 6-Chloro-1-[1-(3,4-dichloro-benzenesulfonyl)-piperidin-4-yl]-1,4-dihydro-benzo[d][1,3]oxazin-2-one |
| 413 | 1-[1-(3,4-Dichloro-benzenesulfonyl)-piperidin-4-yl)-6-methyl-1,4-dihydro-benzo[d][1,3]oxazin-2-one |
| 414 | 6-Bromo-1-[1-(3,4-dichloro-benzenesulfonyl)-piperidin-4-yl]-1,4-dihydro-benzo[d][1,3]oxazin-2-one |
| 415 | 8-Methyl-1-[1-propane-1-sulfonyl)-piperidin-4-yl]-1,4-dihydro-benzo[d][1,3]oxazin-2-one |
| 416 | 1-[1-(2-Chloro-6-methyl-benzenesulfonyl)-piperidin-4-yl]-1,4-dihydro-benzo[d][1,3]oxazin-2-one |
| 417 | 1-[1-(2-chloro-6-methyl-benzenesulfonyl)-piperidin-4-yl]-8-methyl-1,4-dhydro-benzo[d][1,3]oxazin-2-one |
| 418 | 6-Chloro-1-[1-(2-chloro-6-methyl-benzenesulfonyl)-piperidin-4-yl]-1,4-dihydro-benzo[d][1,3]oxazin-2-one |
| 419 | 1-[1-(2-Chloro-6-methyl-benzenesulfonyl)-piperidin-4-yl]-6-methyl-1,4-dihydro-benzo[d][1,3]oxazin-2-one. |
| 420 | 1-[1-(2-Chloro-6-methyl-benzenesulfonyl)-piperindin-4-yl]-6-methyl-1,4-dihydro-benzo[d][1,3]oxazin-2-one |
| 421 | 8-Methyl-1-[1-(2,3,5,8-tetramethyl-benzenesulfonyl)-piperidin-4-yl]-1,4-dihydro-benzo[d][1,3]oxazin-2-one |
| 422 | 1-[1-(2,3,4-Trichloro-benzenesulfonyl)-piperidin-4-yl]-1,4-dihydro[d][1,3]oxazin-2-one |
| 423 | 8-Methyl-1-[1-(2,3,4-trichloro-benzenesulfonyl)-piperidin-4-yl]-1,4-dihydro-benzo[d][1,3]oxazin-2-one |
| 424 | 6-Chloro-1-[1-(2,3,4-trichloro-benzenesulfonyl)-piperidin-4-yl]-1,4-dihydro-benzo[d][1,3]oxazin-2-one |
| 425 | 6-Methyl-1-[1-(2,3,4-trichloro-benzenesulfonyl)-piperidin-4-yl]-1,4-dihydro-benzo[d][1,3]oxazin-2-one |
| 426 | 6-Bromo-1-[1-(2,3,4-trichloro-benzenesulfonyl)-piperidin-4-yl]-1,4-dihydro benzo[d][1,3]oxazin-2-one |
| 427 | 1-[1-(2,3,5,6-Tetramethyl-benzenesulfonyl)-piperidin-4-]-1,4-dihydro-benzo[d][1,3]oxazin-2-one |
| 428 | 1-[1-(Thiophene-3-sulfonyl)-piperidin-9-yl]-1,4-dihydro-benzo[d][1,3]oxazin-2-one |
| 429 | 8-Methyl-1-[1-(thiophene-3-sulfonyl)-piperidin-4-yl]-1,4-dihydro-benzo[d][1,3]oxazin-2-one |
| 430 | 6-Chloro-1-[1-(thiophen-3-sulfonyl)-piperidin-4-yl]-1,4-dihydro-benzo[d][1,3]oxazin-2-one |
| 431 | 6-Methyl-1-[1-(thiophene-3-sulfonyl)-piperidin-4-yl]-1,4-dihydro-benzo[d][1,3]oxazin-2-one |
| 432 | 6-Bromo-1-[1-(thiophene-3-sulfonyl)-piperidin-4-yl]-1,4-dihydro-benzo[d][1,3]oxazin-2-one |
| 433 | 6-Bromo-1-[1-(thiophene-3-sulfonyl)-piperidin-4-yl]-1,4-dihydro-benzo[d][1,3]oxazin-2-one benzo[d][1,3]oxazin-2-one |
| 434 | 1-[1-(2,4,6-Trichloro-benzenesulfonyl)-piperidin-4-yl]-1,4-dihydro-benzo[d][1,3]oxazin-2-one |
| 435 | 8-Methyl-1-[1-(2,4,6-trichloro-benzenesulfonyl)-piperidin-4-yl]-1,4-dihydro-benzo[d][1,3]oxazin-2-one |
| 436 | 6-Chloro-1-[1-(2,4,6-trichloro-benzensulfonyl)-piperidin-4-yl]-1,4-dihydro-Denzo[d][1,3]axazin-2-one |
| 437 | 6-Methyl-1-[1-(2,4,6-trichloro-benzenesulfonyl)-piperidin-4-yl]-1,4-dihydro-benzo[d][1,3]oxazin-2-one |
| 438 | 6-Bromo-1-[1-(2,4,6-trichloro-benzenesulfonyl)-piperidin-4-yl]-1,4-dihydro-benzo[d][1,3]oxazin-2-one |
| 439 | 6-Methyl-1-[1-(2,3,5,6-tetramethyl-benzenesulfonyl)-piperidin-4-yl]-1,4-dihydro-benzo[d][1,3]oxazin-2-one |
| 440 | 1-[1-(2-Bromo-4,6-difluro-benzenesulfonyl)-piperidin-4-yl]-8-methyl-1,4-dihydro-benzo[d][1,3]oxazin-2-one |
| 441 | 1-[1-(2-Bromo-4,6-difluoro-benzenesulfonyl)-piperidin-4-yl]-6-chloro-1,4,-dihydro-benzo[d][1,3]oxazin-2-one |
| 442 | 1-[1-2-Bromo-4,6-difluoro-benzenzenesulfonyl)-piperidin-4-yl]-6-methyl-1,4-dihydro-benzo[d][1,3]oxazin-2-one |
| 443 | 6-Bromo-1-[1-(2-bromo-4,6-difluro-benzenesulfonyl)-piperidin-4-yl]-1,4-dihydro-benzo[d][1,3]oxazin-2-one |
| 444 | 6-Bromo-1-[1-(2,3,5,6-tetramethyl-benzenesulfonyl)-piperidin-4-yl]-1,4-dihydro-benzo[d][1,3]oxazin-2-one |
| 445 | 1-[1-(4-Bromo-2-trifluoromethoxy-benzenesulfonyl)-piperidin-4-yl]-1,4-dihydro-benzo[d][1,3[oxazin-2-one |
| 446 | 1-[1-(4-Bromo-2-trifluoromethoxy-benzenesulfonyl)-piperidin-4-yl]-8-methyl-1,4-dihydro-benzo[d][1,3]oxazin-2-one |
| 447 | 1-[1-(4-Bromo-2-trifluoromethoxy-benzenesulfonyl)-piperidin-4-yl]-6-chloro-1,4-dihydro-benzo[d][1,3]oxazin-2-one |
| 448 | 1-[1-(4-Bromo-2-trifluoromethoxy-benzenesulfonyl)-piperidin-4-yl]-6-methyl-1,4-dihydro-benzo[d][1,3]oxazin-2-one |
| 449 | 6-Bromo-1-[1-(4-bromo-2-trifluoromethoxy-benzenesulfonyl)-piperidin-4-yl]-1,4-dihydro-benzo[d][1,3]oxazin-2-one |
| 450 | 1-[1-(4-Phenoxy-benzenesulfonyl)-piperidin-4-yl]-1,4-dihydro-benzo[d][1,3]oxazin-2-one |
| 451 | 1-[1-(3-Bromo-benzenesulfonyl)-piperidin-4-yl]-4-dihydro-benzo[d][1,3]oxazin-2-one |
| 452 | 1-[1-(3-Bromo-benzenesulfonyl)-piperidin-4-yl]-8-methyl-1,4-dihydro-benzo[d][1,3]oxazin-2-one |
| 453 | -1-[1-(3-Bromo-benzenesulfonyl)-piperidin-4-yl]-6-chloro-1,4-dihydro-benzo[d][1,3]oxazin-2 one |
| 454 | 1-[1-(3-Bromo-benzenesulfonyl)-piperidin-4-yl]-6-methyl-1,4-dihydro-benzo[d][1,3]oxazin-2-one |
| 455 | 6-Bromo-1-[1-(3-bromo-benzenesulfonyl)-piperidin-4-yl]-1,4-dihydro-benzo[d][1,3]oxazin-2-one |
| 456 | 8-Methyl-1-{1-(4-phenoxy-benzenesulfonyl)-piperidin-4-yl]-1,4-dihydro-benzo[d][1,3]oxazin-2-one |
| 457 | 1-[1-(4-tert-Butyl-benzenesulfonyl)-piperidin-4-yl]-1,4-dihydro-benzo[d][1,3]oxazin-2-one |
| 458 | 1-[1-(4-tert-Butyl-benzenesulfonyl)-piperidin-4-yl]-8-methyl-1,4-dihydro-benzo[d][1,3]oxazin-2-one |
| 459 | 1-[1-(4-tert-Butyl-benzenesulfonyl)-piperidin-4-yl]-6-chloro-1,4-dihydro-benzo[d][1,3]oxazin-one |
| 460 | 1-[1-(4-tert-Butyl-benzenesulfonyl)-piperidin-4-yl]-6-methyl-1,4-dihydro-benzo[d][1,3]oxazin-2-one |
| 461 | 6-Bromo-1-[1-(4-tert-butyl-benzenesulfonyl)-piperidin-4-yl]-1,4-dihydro-benzo[d][1,3]oxazin-2-one |
| 462 | 6-Chloro-1-[1-(4-phenoxy-benzenesulfonyl)-piperidin-4-yl]-1,4-dihydro-benzo[d][1,3]oxazin-2-one |
| 463 | 1-[1-(2-Bromo-4,6-difluoro-benzenesulfonyl)-piperidin-4-yl]-1,4-dihydro-benzo[d][1,3]oxazin-2-one |
| 464 | 1-[1-(2-Methanesulfonyl-benzenesulfonyl)-piperidin-4-yl]-8-methyl-1,4-dihydro-benzo[d][1,3]oxazin-2-one |
| 465 | 6-Chloro-1-[1-(2-methanesulfonyl-benzenesulfonyl)-piperidin-4-yl]-1,4-dihydro-benzo[d][1,3]oxazin-2-one |
| 466 | 1-[1-(2-Methanesulfonyl-benzenesulfonyl)-piperidin-4-yl]-6-methyl-1,4-dihydro-benzo[d][1,3]oxazin-2-one |
| 467 | 6-Bromo-1-[1-(2-methanesulfonyl)-benzenesulfonyl)-piperidin-4-yl]-1,4-dihydro-benzo[d][1,3]oxazin-2-one |
| 468 | 468 8-Methyl-1-[1-(4-propyl-benzenesulfonyl)-piperidin-4-yl]-1,4-dihydrobenzo[d][1,3]oxazin-2-one |
| 469 | 6-Chloro-1-[1-(4-propyl-benzenesulfonyl)-piperidin-4-yl]-1,4-dihydro-benzo[d][1,3]oxazin-2-one |
| 470 | 6-Methyl-1-[1-(4-propyl-benzenesulfonyl)-piperidin-4-yl]-1,4-dihydro-benzo[d][1,3]oxazin-2-one |
| 471 | 6-Bromo-1-[1-(4-propyl-benzenesulfonyl)-piperidin-4-yl]-1,4-dihydro-benzo[d][1,3]oxazin-2-one |
| 472 | 1-{1-(3-Chloro-2-methyl-benzenesulfonyl)-piperidin-4-yl]-8-methyl-1,4-dihydro-benzo[d][1,3]oxazin-2-one |
| 473 | 6-Chloro-1-[1-(3-chloro-2-methyl-benzenesulfonyl)-piperidin-4-yl]-1,4-dihydro-benzo[d][1,3]oxazin-2-one |
| 474 | 1-[1-(3-Chloro-2-methyl-benzenesulfonyl)-piperidin-4-yl]-6-methyl-1,4-dihydro-benzo[d][1,3]oxazin-2-one |
| 475 | 6-Bromo-1-[1-(3-chloro-2-methyl-benzenesulfonyl)-piperidin-4-yl]-1,4-dihydro-benzo[d][1,3]oxazin-2-one |
| 476 | 1-[1-(4-Butyl-benzenesulfonyl)-piperidin-4-yl]-8-methyl-1,4-dihydro-benzo[d][1,3]oxazin-2-one |
| 477 | 1-[1-(4-Butyl-benzenesulfonyl)-piperidin-4-yl]-6-chloro-1,4-dihydro-benzo[d][1,3]oxazin-2-one |
| 478 | 1-[1-(4-Butyl-benzenesulfonyl)-piperidin-4-yl]-6-methyl-1,4-dihydro-benzo[d][1,3]oxazin-2-one |
| 479 | 6-Bromo-1-[1-(4-butyl-benzenesulfonyl)-piperidin-4-yl]-1,4-dihydro-benzo[d][1,3]oxazin-2-one |
| 480 | 1-[1-(4-Bromo-3-methyl-benzenesulfonyl)-piperidin-4-yl)-8-methyl-1,4-dihydro-benzo[d][1,3]oxazin-2-one |
| 481 | 1-[1-(4-Bromo-3-methyl-benzenesulfonyl)-piperidin-4-yl]-6-chloro-1,4-dihydro-benzo[d][1,3]oxazin-2-one |
| 482 | 1-[1-(4-Bromo-3-methyl-benzenesulfonyl)-piperidin-4-yl]-6-methyl-1,4-dihydro-benzo[d][1,3]oxazin-2-one |
| 483 | 6-Bromo-1-[1-(4-bromo-3-methyl-benzenesulfonyl)-piperidin-4-yl]-1,4-dihydro-benzo[d][1,3]oxazin-2-one |
| 484 | 1-{1-[4-(1,1-Dimethyl-propyl)-benzenesulfonyl]-piperidin-4-yl}-8-methyl-1,4-dihydro-benzo[d][1,3]oxazin-2-one |
| 485 | 6-Chloro-1-{1-[4-(1,1-dimethyl-propyl)-benzenesulfonyl)-piperidin-4-yl}-1,4-dihydro-benzo[d][1,3]oxazin-2-one |
| 486 | 1-{1-{4-(1,1-Dimethyl-propyl)-benzenesulfonyl)-piperidin-4-yl)-6-methyl-1,4-dihydro-benzo[d][1,3]oxazin-2-one |
| 487 | 6-Bromo-1-(1-[4-(1,1-dimethyl-propyl)-benzenesulfonyl]-piperidin-4-yl]-1,4-dihydro-benzo[d][1,3]oxazin-2-one |
| 488 | 1-(1-Ethenesulfonyl-piperidin-4-yl)-8-methyl-1,4-dihydro-benzo[d][1,3]oxazin-2-one |
| 489 | 3-[4-(8-Methyl-2-oxo-4H-benzo[d][1,3]oxazin-1-yl)-piperdine-1-sulfanyl]-benzoic acid |
| 490 | 3-[4-(6-Methyl-2-oxo-4H-benzo[d][1,3]oxazin-1-yl)-piperidine-1-sulfanyl]-benzoic acid |
| 491 | 3-[4-(8-Methyl-2-oxo-4H-benzo[d][1,3]oxazin-1-yl)-piperdine-1-sulfanyl]-benzoic acid |
| 492 | 1-[-(3-Chloro-2-fluoro-benzenesulfonyl)-piperidin-4-yl]-8-methyl-1,4-dihydro-benzo[d][1,3]oxazin-2-one |
| 493 | 6-Chloro-1-[1-(3-chloro-2-fluoro-benzenesulfonyl)-piperidin-4-yl]-1,4-dihydro-benzo[d][1,3]oxazin-2-one |
| 494 | 1-[1-(3-Chloro-2-fluoro-benzenesulfonyl)-piperidin-4-yl]-6-methyl-1,4-dihydro-benzo[d][1,3]oxazin-2-one |
| 495 | 6-Bromo-1-[1-(3-chloro-2-fluoro-benzenesulfonyl)-piperidin-4-yl)-1,4-dihydro-benzo[d][1,3]oxazin-2-one |
| 496 | N-{4-Methyl-5-[4-(8-methyl-2-oxo-4H-benzo[d][1,3]oxazin-1-yl)-piperidine-1-sulfonyl]-thiazol-2-yl}-acetamide |
| 497 | N-[5-[4-(6-Chloro-2-oxo-4H-benzo[d][1,3]oxazin-1-yl)-piperidine-1-sulfonyl]-4-methyl-thiazol-2-yl}-acetamide |
| 498 | N-[4-Methyl-5-[4-(6-methyl-2-oxo-4H-benzo[d][1,3]oxazin-1-yl]-piperidine-1-sulfonyl]-thiazol-2-yl}-acetamide |
| 499 | N-{5-[4-(6-Bromo-2-oxo-4H-benzo[d][1,3]oxazin-1-yl)-piperidine-1-sulfonyl]-4-methyl-thiazol-2-yl}-acetamide |
| 500 | 1-[1-(2-Bromo-4-fluoro-benzenesulfonyl)-piperidin-4-yl]-8-methyl-1,4-dihydro-benzo[d][1,3]oxazin-2-one |
| 501 | 1-[1-(2-Bromo-4-fluoro-benzenesulfonyl)-piperidin-4-yl]-6-chloro-1,4-dihydro benzo[d][1,3]oxazin-2-one |
| 502 | 1-[1-(2-Bromo-4-fluoro-benzenesulfonyl)-piperidin-4-yl]-6-methyl-1,4-dihydro-benzo[d][1,3]oxazin-2-one |
| 503 | 6-Bromo-1-[1-(2-bromo-4-fluoro-benzenesulfonyl)-piperidin-4-yl]-1,4-dihydro-benzo[d][1,3]oxazin-2-one |
| 504 | 1-[1-(5-Chloro-2-fluoro-benzenesulfonyl)-piperidin4-yl]-8-methyl-1.4-dihydro-benzo[d]oxazin-2-one |
| 505 | 6-Chloro-1-[1-(5-chloro-2-fluoro-benzenesulfonyl)-piperidin-4-yl]-1,4-dihydro-benzo[d][1.3]oxazin-2-one |
| 506 | 1-[1-(5-Chloro-2-fluoro-benzenesulfonyl)-piperidin-4-yl]-6-methyl-1,4-dihydro-benzo[d][1,3]oxazin-2-one |
| 507 | 6-Bromo-1-[1-(5-chloro-2-flooro-benzenesulfonyl)-piperidin-4-yl]-1,4-dihydro-benzo[d][1.3]oxazin-2-one |
| 508 | 1-[1-(4-Bromo-3-trifluoromethyl-benzenesulfonyl)-piperidin-4-yl]-8-methyl-1,4-dihydro-benzo[d][1,3]oxazin-2-one |
| 509 | 1-[1-(4-Bromo-3-trifluoromethyl-benzenesulfonyl)-piperidin-4-yl]-6-chloro-1,4-dihydro-benzo[d][1,3]oxazin-2-one |
| 510 | 1-[1-(4-Bromo-3-trifluoromethyl-benzenesulfonyl)-piperidin-4-yl]-6-methyl-1,4-dihydro-benzo[d][1,3]oxazin-2-one |
| 511 | 6-Bromo-1-[1-(4-bromo-3-trifluoromethyl-benzenesulfonyl)-piperidin-4-yl]-1,4-dihydro-benzo[d][1,3]oxazin-2-one |
| 512 | 1-[1-(2-Methanesulfonyl-benzenesulfonyl)-piperidin-4-yl]-1.4-dihydro-benzo[d][1.3]oxazin-2-one |
| S13 | 1-[1-(4-Propyl-benzenesulfonyl)-piperidin-4-yl]-1,4-dihydro--benzo[d][1,3]oxazin-2-one |
| 514 | 1-[1-(3-Chloro-2-methyl-benzenesulfonyl)-piperidin-4-yl]-1,4-dihydro-benzo[d][1,3]oxazin-2-one |
| 515 | 1-[1-(4-Butyl-benzenesulfonyl)-piperidin-4-yl]-1,4-dihydro-benzo[d][1.3]oxazin-2-one |
| 516 | 1-[1-(4-Bromo-3-methyl-benzenesulfonyl)-piperidin-4-yl]-1,4-dihydro-benzo[d][1,3]oxazin-2-one |
| 517 | 1-(1-[4-(1,1-Dimethyl-propyl)-benzenesulfonyl)-piperidin-4-yl)-1,4-dihydro-benzo[d][1,3]oxazin-2-one |
| 518 | N-[4-Methyl-5-[4-(2-oxo-4H-benzo[d][1.3]oxazin-1-yl)-piperidine-1-sulfonyl]-thiazol-2-yl}-acetamide |
| 519 | 1-[1-(3-Chloro-2-fluoro-benzenesulfonyl)-piperidin-4-yl]-1,4-dihydro-benzo[dl[1,3]oxazin-2-one |
| 520 | 1-[1-(2-Bromo-4-fluoro-benzenesulfonyl)-piperidin-4-yl]1,4-dihydro-benzo[d][1,3]oxazin-2-one |
| 521 | 1-[1-(4-Bromo-3-trifluoromethyl-benzenesulfonyl)-piperidin-4-yl]-1.4-dihydro-benzo[d][1,3]oxazin-2-one |
| 522 | 1-[1-(5-Chloro-2-fluoro-benzenesuffonyl)-piperidin-4-yl]-1,4-dihydro-benzo[d][1,3]ozazin-2-one |
| 523 | 1-[1-(Isoquinoline-5-sulfonyl)-piperidin-4-yl]-1,4-dihydro-benzo[d][1.3]oxazin-2-one |
| 524 | 6-Fluoro-1-[1-(2-methanesulfonyl-benzenesulfonyl)-piperidin-4-yl]-1,4-dihydro-benzo[d][1,3]oxazin-2-one |
| 525 | 6-Fluoro-1-[1-(4-propyl-benzenesulfonyl)-piperidin-4-yl]-1,4-dihydro-benzo[d][1,3]oxazin-2-one |
| 526 | 1-[1-(3-Chloro-2-methyl-benzenesulfonyl)-piperidin-4-yl]-6-fluoro-1,4-dihydro-benzo[d][1,3]oxazin-2-one |
| 527 | 1-[1-(4-Butyl-benzenesulfonyl)-piperidin-4-yl]-6-fluoro-1.4-dihydro-benzo[d][1,3]oxazin-2-one |
| 528 | 1-[4-Bromo-3-methyl-benzenesulfonyl)-piperidin-4-yl]-6-fluoro-1,4-dihydro-benzo[d][1,3]oxazin-2-one |
| 529 | 1-[1-[4-(1,1-Dimethyl-propyl)-benzenesulfonyl-piperidin-4-yl]-6-fluoro-1,4-dihydro-benzo[d][1,3]oxazin-2-one |
| 530 | N-[5-[4-(6-Fluoro-2-oxo-4H-benzo[d][1,3]oxazin-1-yl)-piperidine-1-sulfonyl]-4-methyl-thiazol-2-yl)-acetamide |
| 531 | 1-[1-(3-Chloro-2-fluoro-benzenesulfonyl)-piperidin-yl]-6-fluoro-1,4-dihydro-benzo[d][1,3]oxazin-2-one |
| 532 | 1-[1-(2-Bromo-4-fluoro-benzenesulfonyl)-piperidin-4-yl]-6-fluoro-1,4-dihydro-benzo[d][1,3]oxazin-2-one |
| 533 | 1-[1-(4-Bromo-3-trifiuoromethyl-benzenesulfonyl)-piperidin-4-yl]-6-fluoro-1,4-dihydro-benzo[d][1,3]oxazin-2-one |
| 534 | 1-[1-(5-Chloro-2-fluoro-benzenesulfonyl)-piperidin-4-yl]-6-fluoro-1,4-dihydro-benzo[d][1,3]oxazin-2-one |
| 535 | 6-Fluoro-1-[1-(Isoquinoline-5-sulfonyl)-piperidin-4-yl]-1,4-dihydro-benzo[d][1,3]oxazin-2-one |
| 536 | 6-Fluoro-1-[1-(quinoline-8-sulfonyl)-piperidin-4-yl]-1,4-dihydro-benzo[d][1,3]oxazin-2-one |
| 537 | 1-[1-(5-Chloro-3-methyl-benzo[b]thiophene-2-sulfonyl)-piperidin-4-yl]-6-fluoro-1,4-dihydro-benzo[d][1,3]oxazin-2-one |
| 538 | 6-Fluoro-1-[1-(naphthalene-1-sulfonyl)-piperidin-4-yl]-1,4-dihydro-benzo[d][1,3]oxazin-2-one |
| 539 | 6-Fluoro-1-[1-(naphthalene-2-sulfonyl)-piperidin-4-yl]-1,4-dihydro-benzo[d][1,3]oxazin-2-one |
| 540 | 1-[1-(Benzo[b]thiophene-2-sulfonyl)-piperidin-4-yl]-6-fluoro-1,4-dihydro-benzo[d][1,3]oxazin-2-one |
| 541 | 1-[1-(Benzo[b]thiophene-3-sulfonyl)-piperidin-4-yl]-6-fluoro-1,4-dihydro-benzo[d][1,3]oxazin-2-one |
| 542 | 8-Methoxy-1-[1-(quinoline-8-sulfonyl)-piperidin-4-yl]-1,4-dihydro-benzo[d][1,3]oxazin-2-one |
| 543 | 1-[1-(5-Chloro-3-methyl-benzo[b]thiophene-2-sulfonyl)-piperidin-4-yl]-8-methoxy-1,4-dihydro-benzo[d][1,3]oxazin-2-one |
| 544 | 8-Methoxy-1-[1-(naphthalene-1-sulfonyl)-piperidin4-yl]-1,4-dihydro-benzo[d][1,3]oxazin-2-one |
| 545 | 8-Methoxy-1-[1-(naphthalene-2-sulfonyl)-piperidin-4-yl)-1,4-dihydro-benzo[d][1,3]oxazin-2-one |
| 546 | 1-[1-(Benzo[b]thiophene-2-sulfonyl)-piperidin-4-yl]-8-methoxy-1.4-dihydro-benzo[d][1,3]oxazin-2-one |
| 547 | 1-[1-(Benzo[b]thiophene-3-sulfonyl)-piperidin-4-yl]-8-methoxy-1,4-dihydro-benzo[d][1,3]oxazin-2-one |
| 548 | 5-Chloro-1-[1-(2-methanesulfonyl-benzenesulfonyl)-piperidin-4-yl]-1,4-dihydro-benzo[d][1,3]oxazin-2-one |
| 549 | 5-Chloro-1-[1-(4-propyl-benzenesulfonyl)-piperidin-4-yll]-1,4-dihydro-benzo[d][1,3]oxazin-2-one |
| 550 | 5-Chloro-1-[1-(3-chloro-2-methyl-benzenesulfonyl)-piperidin-4-yl]-1,4-dihydro-benzo[d][1,3]oxazin-2-one |
| 551 | 1-[1-(4-Butyl-benzenesulfonyl)-piperidin-4-yl]-5-chloro-1,4-dihydro-benzo[d][1,3]oxazin-2-one |
| 552 | 1-[1-(4-Bromo-3-methyl-benzenesulfonyl)-piperidin-4-yl]-5-chloro-1,4-dihydro-benzo[d][1,3]oxazin-2-one |
| 553 | 5-Chloro-1-{1-[4-(1,1-dimethyl-propyl)-benzenesulfonyl]-piperidin-4-yl]-1,4-dihydro-benzo[d][1,3]oxazin-2-one |
| 554 | N-{5-[4-(5-Chloro-2-oxo-4H-benzo[d][1,3]oxazin-1-yl)-piperidine-1-sulfonyl]-4-methyl-thiazol-2-yl]-acetamide |
| 555 | 5-Chloro-1-[1-(3-chloro-2-fluoro-benzenesulfonyl)-piperidin-4-yl]-1,4-dihydro-benzo[d][1,3]oxazin-2-one |
| 556 | 1-[1-(2-Bromo-4-fluoro-benzenesulfonyl)-piperidin-4-yl]-5-chloro-1,4-dihydro-benzo[d][1,3]oxazin-2-one |
| 557 | 1-[1-(4-Bromo-3-trifluoromethyl-benzenesulfonyl)-piperidin-4-yl]-5-chloro-1,4-dihydro-benzo[d][1,3]oxazin-2-one |
| 558 | 5-Chloro-1-[1-(5-chloro-2-fluoro-benzenesulfonyl)-piperidin-4-yl]-1,4-dihydro-benzo[d][1,3]oxazin-2-one |
| 559 | 5-Chloro-1-[1-(isoquinoline-5-sulfonyl)-piperidin-4-yl]-1,4-dihydro-benzo[d][1,3]oxazin-2-one |
| 560 | 1-[1-(2-Methanesulfonyl-benzenesulfonyl)-piperidin-4-yl-8-methoxy-1,4-dihydro-benzo[d][1,3]oxazin-2-one |
| 561 | 1-[1-(2-Methanesulfonyl-benzenesulfonyl)-piperidin-4-yl]8-methoxy-1,4-dihydro-benzo[d][1,3]oxazin-2-one |
| 562 | 1-[1-(3-Chloro-2-methyl-benzenesulfonyl)-piperidin-4-yl]-8-methoxy-1,4-dihydro-benzo[d][1,3[oxazin-2-one |
| 563 | 1-[1-(4-Butyl-benzenesulfonyl)-piperidin-4-yl]-8-methoxy-1,4-dihydro-benzo[d][1,3]oxazin-2-one |
| 564 | 1-[1-(4-Bromo-3-methyl-benzenesulfonyl)-piperidin-4-yl]-8-methoxy-1,4-dihydro-benzo[d][1,3]oxazin-2-one |
| 565 | 1-{1-[4-(1,1-Dimethyl-propyl)-benzenesulfonyl]-piperidin-4-yl]-8-methoxy-1,4-dihydro-benzo[d][1,3]oxazin-2-one |
| 566 | N-[5-[4-(8-Methoxy-2-oxo-4H-benzo[d][1,3]oxazin-1-yl)-piperidine-1-sulfonyl]-4-methyl-thiazol-2-yl]-acetamide |
| 567 | 1-[1-(3-Chloro-2-fluoro-benzenesulfonyl)-piperidin-4-yl]-8-methoxy-1,4-dihydro-benzo[d][1,3]oxazin-2-one |
| 568 | 1-[1-(2-Bromo-4-fluoro-benzenesulfonyl)-piperidin-4-yl]-8-methoxy-1,4-dihydro-benzo[d][1,3]oxazin-2-one |
| 569 | 1-[1-(4-Bromo-3-trifluoromethyl-benzenesulfonyl)-piperidin-4-yl]-8-methoxy-1,4-dihydro-benzo[d][1,3]oxazin-2-one |
| 570 | 1-[1-(5-Chloro-2-fluoro-benzenesulfonyl)-piperidin-4-yl)-8-methoxy-1,4-dihydro-benzo[d][1,3]oxazin-2-on |
| 571 | 1-[1-(Isoquinoline-5-sulfonyl)-piperidin-4-yl]-8-methoxy-1.4-dihydro-benzo[d][1,3]oxazin-2-one; hydrochloride |
| 572 | 1-[1-(4-Methyl-naphthalene-1-sulfonyl)-piperidin-4-yl]-1.4-dihydro-benzo[d][1,3]oxazin-2-one |
| 573 | Chloro-1-[1-(4-methyl-naphthalene-1-sulfonyl)-piperidin-4-yl]-1,4-dihydro-benzo[d][1,3]oxazin-2-one |
| 574 | 6-Methyl-1-[1-(4-methyl-naphthalene-1-sulfonyl)-piperidin-4-yl]-1,4-dihydro-benzo[d][1,3]oxazin-2-one |
| 575 | 8-Methyl-1-[1-(4-methyl-naphthalene-1-sulfonyl)-piperidin-4-yl]-1,4-dihydro-benzo[d][1,3]oxazin-2-one |
| 576 | 6-Fluoro-1-[1-(4-methyl-naphthalene-1-sulfonyl)-piperidin-4-yl]-1,4-dihydro-benzo[d][1,3]oxazin-2-one |
| 577 | 8-Methoxy-1-[1-(4-methyl-naphthalene-1-sulfonyl)-piperidin-4-yl]-1,4-dihydro-benzo[d][1,3]axazin-2-one |
| 578 | 5-Chloro-1-[1-(4-methyl-naphthalene-1-sulfonyl)-piperidin4-yl]-1,4-dihydro-benzo[d][1,3]oxazin-2-one |
| 579 | 5-Chloro-1-[1-(naphthalene-1-sulfonyl)piperidin-4-yl]-1,4-dihydro-benzo[d][1,3]oxazin-2-one |
| 580 | 5-Chloro-1-[-1-naphthalene-2-sulfonyl)-piperidin-4-yl]-1,4-dihydro-benzo[d][1,3]oxazin-2-one |
| 581 | 5-Chloro-1-[1-(quinoline-8-sulfonyl)-piperidin-4-yl)-1,4-dihydro)-benzo[d][1,3]oxazin-2-one |
| 582 | 5-Chloro-1-[1-(5-chloro-3-methyl-benzo[b]thiophene-2-sulfonyl)-piperidin-4-yl]-1,4-dihydro-benzo[d][1,3]oxazin-2-one |
| 583 | [Benzo[b]thiophene-2-sulfonyl)-piperidin-4-yl]-5-chloro-1.4-dihydro-benzo[d][1,3]oxazin-2-one |
| 584 | 1-[1-(Benzo[b]thiophene-3-sulfonyl)-piperidin-4-yl]-5-chloro-1,4-dihydro-benzo[d][1,3]oxazin-2-one |
| 585 | 6-Bromo-1-[1-(4-methyl-naphthalene-1-sulfonyl)-piperidin-4-yl]-1,4-dihydro-benzo[d][1,3]oxazin-2-one |
| 586 | 2-Chloro-4-fluoro-5-[4-(8-methyl-2-oxo-4H-benzo[d][1,3]oxazin-1-yl)piperidine-1-sulfonyl]-benzoic acid |
| 587 | 2-Chloro-5-[4-(6-chloro-2-oxo-4H-benzo[d][1,3]oxazin-1-yl)-piperidine-1-sulfonyl]-4-fluoro-benzoic acid |
| 588 | 2-Chloro-4-fluoro-5-[4-(6-methyl-2-oxo-4H-benzo[d][1,3]oxazin-1-yl)-piperidine-1-sulfonyl]-benzoic acid |
| 589 | 2-Chloro-4-fluoro-5-[4-(2-axo-4H-benzo[d][1,3]oxazin-1yl)-piperidine-1-sulfonyl]-benzoic acid |
| 590 | 2-Chloro-4-fluoro-5-[4-(8-methoxy-2-oxo-4H-benzo[d][1,3]oxazin-1-yl)-piperidine-1-sulfonyl]. benzoic acid |
| 591 | 2-Chloro-5-[4-(5-chloro-2-oxo-4H-benzo[d][1,3]oxazin-1-yl)-piperidine-1-sulfonyl]-4-fluoro-benzoic acid |
| 592 | 3-[4-(2-Oxo-4H-benzo[d][1.3]oxazin-1-yl)-piperidinr-1-sulfonyl]-benzoic acid |
| 593 | 3-[4-(8-Methoxy-2-oxo-4H-benzo[d]\|1,3]oxazin-1-yl)-piperidine-1-sulfonyl]-benzoic acid |
| 594 | 3-[4-(5-Chloro-2-oxo-4H-benzo[d][1,3]oxazin-1-yl)-piperidine-1-sulfonyl]-benzoic acid |
| 595 | 1-[1-(Isoquinoline-5-sulfonyl]-piperidin-4-yl]-8-methyl-1,4-dihydro-benzo[d][1,3]oxazin-2-one; hydrochloride |
| 596 | 6-Chloro-1-[1-(isoquinoline-5-sulfonyl)-piperidin-4-yl]-1,4-dihydro-benzo[d][1,3]oxazin-2-one; hydrochloride |
| 597 | -[1-(Isoquinoline-5-sulfonyl)-piperidin-4-yl]-6-methyl-1,4-dihydro-benzo[d][1,3]oxazin-2-one; hydrochloride |
| 598 | 6,7-Difluoro-1-[1-(quinoline-8-sulfonyl)-piperidin-4-yl]1,4-dihydro-benzo[d][1,3]oxazin-2-one |
| 599 | 1-[1-[5-Chloro-3-methyl-benzo[b]thiophene-2-sulfonyl)-piperidin-4-yl)-6,7-difluoro-1,4-dihydro-benzo[d][1,3]axazin-2-one |
| 600 | 6,7-Difluoro-1-[1-(naphthalene-1-sulfonyl)-piperidin-4-yl]-1,4-dihydro-benzo[d][1,3]oxazin-2-one |
| 601 | 6,7-Difluoro-1-[1-(naphalene-2-sulfonyl)-piperidin-yl]-1,4-dihydro-benzo[d][1,3]oxazin-2-one |
| 602 | 1-[1-(Benzo[b]thiophene-2-sulfonyl)-piperidin-4-yl]-6,7-difluoro-1,4-dihydro-benzo[d][1,3]oxazin-2-one |
| 603 | 1-[1-(Benzo[b]thiophene-3-sulfonyl)-piperidin-4-yl]-6,7-difluoro-1,4-dihydro-benzo[d][1,3]oxazin-2-one |
| 604 | 1-[1-(5-Dimethylamino-naphthalene-1-sulfonyl)-piperidin-4-yl]-6,7-difluoro-1,4-dihydro-benzo[d][1,3]oxazin-2-one |
| 605 | 1-[1-(Biphenyl-4-sulfonyl)-piperidin-4-yl]-6,7-difluoro-1.4-dihydro-benzo[d][1,3]oxazin-2-one |
| 606 | 1-[1-(Benzo[1,2,5]thiadiazole-4-sulfonyl)-piperidin-4-yl]-6,7-difluoro-1,4-dihydro-benzo[d][1,3]oxazin-2-one |
| 607 | 1-[1-(Benzo[1,2,5]oxadiazole-4-sulfonyl)-piperidin-4-yl]-6,7-difluoro-1,4-dihydro-benzo[d][1,3]oxazin-2-one |
| 608 | 1-[1-(7-Chloro-benzo[1,2,5]oxadiazole-4-sulfonyl)-piperidin-4-yl]-6,7-difluoro-1,4-dihydro-benzo[d][1,3]oxazin-2-one |
| 609 | 6,7-Difluoro-1-[1-(4-methyl-naphthalene-1-sulfonyl)-piperidin-4-yl]-1,4-dihydro-benzo[d][1,3]oxazin-2-one |
| 610 | 1-[1-(4-Chloro-naphthalene-1-sulfonyl)-piperidin-4-yl]-1.4-dihydro-benzo[d][1,3]oxazin-2-one |
| 611 | 1-[1-(4-Fluoro-naphthalene-1-sulfonyl)-piperidin-4-yl]-1.4-dihydro-benzo[d][1,3]oxazin-2-one |
| 612 | 1-[1-(Dibenzofuran-2-sulfonyl)-piperidin-4-yl]-1,4-dihydro-benzo[d][1,3]oxazin-2-one |
| 613 | 1-[1-(2,3-Dihydro-benzofuran-5-sulfonyl)-piperidin-4-yl]-1,4-dihydro-benzo[d][1,3]oxazin-2-one |
| 614 | 1-[1-(Biphenyl-2-sulfonyl)-piperidin-4-yl]-1,4-dihydro-benzo[d][1,3]oxazin-2-one |
| 615 | 1-[1-(5-Isoxazol-5-yl-thiophene-2-sulfonyl)-piperidin-4-yl]-1,4-dihydro-benzo[d][1,3]oxazin-2-one |
| 616 | 1-[1-(4-Chloro-naphthalen-1-sulfonyl)-piperidin-4-yl]-8-methyl-1,4-dihydro-benzo[d][1,3]oxazin-2-one |
| 617 | 1-[1-(4-Fluoro-naphthalene-1-sulfonyl)-piperidin-4-yl]-8-methyl-1,4-dihydro-benzo[d][1,3]oxazin-2-one |
| 618 | 1-[1-(Dibenzofuran-2-sulfonyl)-piperidin-4-yl]-8-methyl-1,4-dihydro-benzo[d][1,3]oxazin-2-one |
| 619 | 1-[1-(2,3-Dihydro-benzofuran-5-sulfonyl)-plperidin-4-yl]-8-methyl-1,4-dihydro-benzo[d][1,3]oxazin-2-one |
| 620 | 1-[1-(Biphenyl-2-sulfonyl)-piperidin-4-methyl-1,4-dihydro-benzo[d][1,3]oxazin-2-one |
| 621 | 1-[1-(5-Isoxazol-5-yl-thiophene-2-sulfonyl)-piperidin-4-yl]-8-methyl-1,4-dihydro-benzo[d][1,3]oxazin-2-one |
| 622 | 5-Chloro-1-[1-(4-chloro-naphthalene-1-sulfonyl)-piperidin4-yl]-1,4-dihydro-benzo[d][1,3]oxazin-2-one |
| 623 | 5-Chloro-1-[1-(4-fluoro-naphthalene-1-sulfonyl)-piperidin-4-yl]-1,4-dihydro-benzo[d][1,3]oxazin-2-one |
| 624 | 5-Chloro-1-[1-(dibenzofuran-2-sulfonyl)-piperidin-4-yl]-1,4-dihydro-benzo[d][1,3]oxazin-2-one |
| 625 | 5-Chloro-1-[1-(2,3-dihydro-benzofuran-5-sulfonyl)-piperidin-4-yl]-1,4-dihydro-benzo[d][1,3]oxazin-2-one |
| 626 | 1-[1-(Biphenyl-2-sulfonyl)-piperidin-4-yl]-5-chloro-1,4-dihydro-benzo[d][1,3]oxazin-2-one |
| 627 | 5-Chloro-1-[1-(5-isoxazol-5-yl-thiophene-2-sulfonyl)-piperidin4-yl)-1,4-dihydro-benzo[d][1,3]oxazin-2-one |
| 628 | 1-[1-(4-Chloro-naphthalene-1-sulfonyl)-pipridin-4-yl]-8-methoxy-1,4-dihydro-benzo[dl[1,3]oxazin-2-one |
| 629 | 1-[1-(4-Fluoro-naphthalene-1-sulfonyl)-piperidin-4-yl]-8-methoxy-1,4-dihydro-benzo[d][1,3]oxazin-2-one |
| 630 | 1-[1-(Dibenzofuran-2-sulfonyl)-piperirlin-4-yl)-8-methoxy-1,4-dihydro-benzo[d][1,3]oxazin-2-one |
| 631 | 1-[1-(2,3-Dihydro-benzofuran-5-sulfonyl)-piperidin-4-yl]-8-methoxy-1,4-dihydro benzo[d][1,3]oxazin-2-one |
| 632 | 1-[1-(Biphenyl-2-sulfonyl)-piperidin-4-yl]-8-methoxy-1,4-dihydro-benzo[d][1,3]oxazin-2-one |
| 633 | 1-[1-(5-Isoxazol-5-yl-thiophene-2-sulfonyl)-piperidin-4-yl]-8-methoxy-1,4-dihydro benzo[d][1,3]oxazin-2-one |
| 634 | 6-Chloro-1-[1-(4-chloro-naphthalene-1-sulfonyl)-piperidin-4-yl]-1,4-dihydro-benzo[d][1,3]oxazin-2-one |
| 635 | 6-Chloro-1-[1-(4-fluoro-naphthalene-1-sulfonyl)-piperidin-4-yl]-1,4-dihydro-benzo[d][1,3]oxazin-2-one |
| 636 | 6-Chloro-1-[1-(dibenzofuran-2-sulfonyl)-piperidin-4-yl]-1,4-dihydro-benzo[d][1,3]oxazin-2-one |
| 637 | 6-Chloro-1-[1-(2,3-dihydro-benzofuran-5-sulfonyl)-piperidin4-yl]-1,4-dihydro-benzo[d][1,3]oxazin-2-one |
| 638 | 1-[1-(Biphenyl-2-sulfonyl)-piperidin-4-yl)-6-chloro-1.4-dihydro-benzo[d][1.3]oxazin-2-one |
| 839 | 6-Chloro-1-[1-(5-isoxazol-5-yl-thiophene-2-sulfonyl)-piperidin-4-yl]-1.4-dihydro-benzo[d][1,3]oxazin-2-one |
| 640 | 1-[1-(4-Chloro-naphthalene-1-sulfonyl)-piperidin-4-yl]-6-methyl-1,4-dihydro-benzo[d][1,3]oxazin-2-one |
| 641 | 1-[1-(4-Fluoro-naphthalene-1-sulfonyl)-piperidin-4-yl]-6-methyl-1,4-dihydro-benzo[d][1,3]oxazin-2-one |
| 642 | 1-[1-(Dibenzofuran-2-sulfonyl)-piperidin-4-yl]-6-methyl-1,4-dihydro-benzo[d][1,3]oxazin-2-one |
| 643 | 1-[1-(2,3-Dihydro-benzofuran-5-sulfonyl)-piperidin-4-yl]-6-methyl-1,4-dihydro-benzo[d][1,3]oxazin-2-one |
| 644 | 1-[1-(Biphenyl-2-sulfonyl)-piperidin-4-yl]-6-methyl-1,4-dihydro-benzo[d][1.3]oxazin-2-one |
| 645 | 1-[1-(5-Isoxazol-5-yl-thiophene-2-sulfonyl)-piperidin-4-yl]-6-methyl-1.4-dihydro-benzo[d][1,3]oxazin-2-one |
| 646 | 1-[1-(4-Chloro-naphthalene-1-sulfonyl)-piperidin-4-yl]-6,7-difluoro-1,4-dihydro-benzo[d][1,3]oxazin-2-one |
| 647 | 6,7-Difluoro-1-[1-(4-fluoro-naphthalene-1-sulfonyl)-piperidin-4-yl]-1,4-dihydro-benzo[d][1,3]axazin-2-one |
| 648 | 1-[1-(Dibenzofuran-2-sulfonyl)-piperidin-4-yl]-6,7-difluoro-1,4-dihydro-benzo[d][1,3]oxazin-2-one |
| 649 | 1-[1-(2,3-Dihydro-benzofuran-5-sulfonyl)-piperidin-4-yl]-6,7-difluoro-1,4-dihydro-benzo[d][1,3]oxazin-2-one |
| 650 | 1-(1-(Biphenyl-2-sulfonyl)-piperidin-4-yl]-6,7-difluoro-1,4-dihydro-benzo[d][1,3]oxazin-2-one |
| 651 | 8,7-Difluoro-1-[1-(5-isoxazol-5-thiophene-2-sulfonyl)-piperidin-4-yl]-1,4-dihydro.-benzo[d][1,3]oxazin-2-one |
| 652 | 1-[1-(1,2-Dimethyl-1H-midazole-4-sulfonyl)-piperidin-4-yl]1,4-dihydro-benzo[d][1.,3]oxazin-2-one |
| 653 | 1-[1-(5-Methyl-benzo[1,2,5]thiadiazole-4-sulfonyl)-piperidin-4-yl]-1,4-dihydro-benzo[d][1,3]oxazin-2-one |
| 654 | 1-[1-(3,5-Dimethyl-isoxazole-4-sulfonyl)-piperidin-4-yl]-1,4-dihydro-benzo[d][1,3]oxazin-2-one |
| 655 | 1-[1-(1,2-Dimethyl-1H-imidazole-4-sulfonyl)-piperidin-4-yl]-8-methyl-1,4-dihydro-benzo[d][1,3]oxazin-2-one |
| 656 | 8-Methyl-1-[1-(5-methyl-benzo[1,2.5]thiadiazole-4-sulfonyl)-piperidin-4-yl]-1,4-dihydro-benzo[d][1,3]oxazin-2-one |
| 657 | 1-[1-(3,5-Dimethyl-isoxazole-4-sulfonyl)-piperidin-4-yl]-8-methyl-1,4-dihydro-benzo[d][1,3]oxazin-2-one |
| 658 | 6-Chloro-1-[1-(1,2-dimethyl-1H-imidazole-4-sulfonyl)-piperidin-4-yl]-1.4-dihydro-benzo[d][1.3]oxazin-2-one |
| 659 | 6-Chloro-1-[1-(5-methyl-benzo[1,2,5]thiadiazole-4-sulfonyl)-piperidin-4-yl]-1,4-dihydro-benzo[d][1,3]oxazin-2-one |
| 660 | 6-Chloro-1-[1-(3,5-dimethyl-isoxazole-4-sulfonyl)-piperidin-4-yl]-1,4-dihydro-benzo[d][1,3]axazin-2-one |
| 661 | 1-[1-(1,2-Dimethyl-1H-imidazole-sulfonyl)-piperidin-4yl]-8-methoxy-1,4-dihydro-benzo[d][1.3]oxazin-2-one |
| 662 | 8-Methoxy-1-[1-(5-methyl-benzo[1,2,5]thiadiazole-4-sulfonyl)-piperidin-4-yl]-1,4-dihydro-benzo[d][1.3]oxazin-2-one |
| 663 | 1-[1-(3,5-Dimethyl-isoxazole-4-sulfonyl)-piperidin-4-yl]-8-methoxy-1,4-dihydro-benzo[d][1,3]oxazin-2-one |
| 664 | 5-Chloro-1-[1-(1,2-dimethyl-1H-imidazole-4-sulfonyl]-piperidin-4-yo]-1,4-dihydro-benzo[d][1,3]oxazin-2-one |
| 665 | 5-Chloro-1-[1-(5-methyl-benzo[1.2.5]thiadiazole-4-sulfonyl)-piperidin-4-yl]-1,4-dihydro-benzo[d][1,3]oxazin-2-one |
| 666 | 5-Chloro-1-[1-(3,5-dimethyl-isoxazole-4-sulfonyl)-piperidin-4-yl]-1,4-dihydro-benzo[d][1,3]oxazin-2-one |
| 667 | 1-[1-(1,2-Dimethyl-1H-imidazole-4-sulfonyl)-piperidin-4-yl]-6-methyl-1.4-dihydro-benzo[d][1,3]oxazin-2-one |
| 668 | 6-Methyl-1-[1-(5-methyl-benzo[1,2,5]thiadiazole-4-sulfonyl)-piperidin-4yl]-1,4-dihydro-benzo[d][1,3]oxazin-2-one |
| 669 | 1-[1-(3,5-Dimethyl-isoxazole-4-sulfonyl)-piperidin-4-yl]-6-methyl-1,4-dihydro-benzo[d][1,3]oxazin-2-one |
| 670 | 1-[1-(1,2-Dimethyl-1H-Imidazole-4-sulfonyl)-piperidin-4-yl]-6-fluoro-1,4-dihydro-benzo[d][1,3]oxazin-2-one |
| 671 | 6-Fluoro-1-[1-(5-methyl-benzo[1,2.5]thiadiazole-4-sulfonyl)-piperidin-4-yl]-1,4-dihydro-benzo[d][1,3]oxazin-2-one |
| 672 | 1-[1-(3,5-Dimethyl-isoxazole-4-sulfonyl)-piperidin-4-yl]-6-fluoro-1,4-dihydro-benzo[d][1,3]oxazin-2-one |
| 673 | 1-[1-(1,2-Dimethyl-1 H-imidazole-4-sulfonyl}-piperidin-4-yl]-6,7-difluoro-1,4-dihydro-benzo[d][1,3]oxazin-2-one |
| 674 | 6,7-Difluoro-1-[1-(5-methyl-benzo[1,2,5]thiadiazole-4-sulfonyl)-piperidin-4-yl]-1,4-dihydro-benzo[d][1,3]oxazin-2-one |
| 675 | 1-[1-(3,5-Dimethyl-isoxazole-4-sulfonyl)-piperidin-4-yl]-6,7-difluoro-1,4-dihydro-benzo[d][1.3]oxazin-2-one |
| 676 | 1-[1-(5-Chloro-naphthalene-1-sulfonyl)-piperidin-4-yl]-1,4-dihydro-benzo[d][1,3]oxazin-2-one |
| 677 | 1-[1-(5-Chloro-naphthalene-1-sulfonyl)-piperidin-4-yl]-1,4-dihydro-benzo[d][1,3]oxazin-2-one |
| 678 | N-[5-[4-(2-Oxo-4H-benzo[d][1,3]oxazin-1-yl)-piperidine-1-sulfonyl]-naphthalen-1-yl]-acetamide |
| 679 | 1-[1-(5-Chloro-naphthalene-1-sulfonyl)-piperidin-4-yl]-8-methyl-1,4-dihydro-benzo[d][1,3]oxazin-2-one |
| 680 | -1-[1-(5-Chloro-naphthalene-2-sulfonyl)-piperidin-4-yl]-8-methyl-1,4-dihydro-benzo[d][1.3]oxazin-2-one |
| 681 | N-(5-[4-(8-Methyl-2-oxo-4H-benzo[d][1,3]oxazin-1-yl)-piperidine-1-sulfonyl]-naphthalen-1-yl)-acetamide |
| 682 | 5-Chloro-1-[1-(5-chloro-naphthalene-1-sulfonyl)-piperidin-4-yl]-1,4-dihydro-benzo[d][1,3]oxazin-2-one |
| 683 | 5-Chloro-1-[1,3]-(5-chloro-naphthalene-2-sulfonyl)-piperidin-4-yl)-1,4-dihydro-benzo[d][1,3]oxazin-2-one |
| 684 | N-[5-[4-(5-Chloro-2-oxo-4H-benzo[d][1,3]oxazin-1-yl)-piperidine-1-sulfonyl]-naphthalen-1-yl]-acetamide |
| 685 | 1-[1-(5-Chloro-naphthalene-1-sulfonyl]-piperidin-4-yl]-8-methoxy-1,4-dihydro-benzo[d][1,3]oxazin-2-one |
| 686 | 1-[1-(5-Chloro-naphthalene-2-sulfonyl)-piperidin-4-yl]-8-methoxy-1,4-dihydro-benzo[d][1,3]oxazin-2-one |
| 687 | N-(5-[4-(8-Methoxy-2-oxo-4H-benzo[d][1,3]oxazin-1-yl)-piperidine-1-sulfonyl]-naphthalen-1-yl]-acetamide |
| 688 | 2,5-Dimethyl-4-[4-(8-methyl-2-oxo-4H-benzo[d][1.3]oxazin-1-yl)-piperidine-1-sulfonyl]-furan-3-carboxylic acid methyl ester |
| 689 | 8-Methyl-1-[1-(2-oxo-2-,3-dihydro-benzothiazole-6-sulfonyl)-piperidin-4-yl]-1,4-dihydro-benzo[d][1,3]oxazin-2-one |
| 690 | 1-[1-(4-Fluoro-3-methyl-benzenesulfonyl)-piperidin-4-yl]-8-methyl-1,4-dihydro-benzo[d][1.3]oxazin-2-one |
| 691 | 8-Methyl-1-[1-(2-oxo-2.3-dihydro-benzooxazole-6-sulfonyl)-piperidin-4-yl]-1,4-dihydro-benzo[d][1,3]oxazin-2-one |
| 692 | 1-[1-(4-Cyclohexyl-benzenesulfonyl-piperidin-4-yl]-8-methyl-1,4-dihydro-benzo[d][1,3]oxazin-2-one |
| 693 | 2,5-Dimethyl-4-[4-(2-oxo-4H-benz0[d][1,3]oxazin-1-yl)-piperidine-1-sulfonyl]-furan-3-carboxylic acid methyl ester |
| 694 | 1-[1-(4-Fluoro-3-methyl-benzenesulfonyl)-piperidin-4-yl]-1.4-dihydro-benzo[d][1,3]oxazin-2-one |
| 695 | 1-[1-(2-Oxo-2,3-dihydro-benzooxazole-6-sulfonyl)-piperidin-4-yl]-1,4-dihydro-benzo[d][1,3]oxazin-2-one |
| 696 | 1-[1-(4-Cyclohexyl-benzenesulfonyl)-piperidin-4-yl]-1,4-dihydro-benzo[d][1,3]oxazin-2-one |
| 697 | 2-Fluoro-5-[4-(8-methyl-2-oxo-4H-benzo[d][1,3]oxazin-1-yl)-piperidine-1-sulfonyl]-benzoic acid |
| 698 | 2-Fluoro-5-[4-(2-oxo-4H-benzo[d][1.3]oxazin-1-yl)-piperidine-1-sulfonyl]-benzoic acid |
| 699 | 1-[1-(2-Oxo-2,3-dihydro-benzothiazole-6-sulfonyl)-piperidin-4-yl]-1,4-dihydro-benzo[d][1,3]oxaz-2-one |
| 700 | 1-[1-(5-Pyridin-2-yl-thiophene-2-sulfonyl)-piperidin-4-yl]-1,4-dihydro -benzo[d][1,3]oxazin-2-one |
| 701 | 3-[4-(2-Oxo-4H-benzo[d][1.3]oxazin-1-yl)-piperidine-1-sulfonyl}-benzonitrile |
| 702 | 3-[4-(2-Oxo-4H-benzo[d][1,3]oxazin-1-yl)-piperidine-1-sulfonyl]-thiophene-2-carboxylic acid methyl ester |
| 703 | -{5-[4-{2-Oxo-4H-benzo[d][1,3]oxazin-1-yl)-piperidine-1-sulfonyl]-naphthalen-1-yl]-pyrrolidine-2,5-dione |
| 704 | 1-[1-(2-Chloro-5-trifluoromethyl-benzenesulfonyl)-piperidin-4-yl]-1,4-dihydro-benzo[d][1,3]oxazin-2-one |
| 705 | 1-[1-(3,4-Dimethyl-benzenesulfonyl)-piperidin-4-yl)-1,4-dihydro-benzo[d][1,3]oxazin-2-one |
| 706 | 8-Methyl-1-[1-(5-pyridin-2-yl-thiophene-2-sulfonyl)-piperidin-4-yl]-1,4-dihydro-benzo[d][1,3]oxazin-2-one |
| 707 | 3-[4-(8-Methyl-2-oxo-4H-benzo[d][1,3]oxazin-1-yl)-piperidine-1-sulfonyl]-benzonitrile |
| 708 | 3-[4-(8-Methyl-2-oxo-4H-benzo[d][1,3]oxazin-1-yl)-piperidine-1-sulfonyl]-thiophene-2-carboxylic add methyl ester |
| 709 | 1-(5-[4-(8-Methyl-2-oxo-4H-benzo[d][1.3]oxazin-1-yl)-piperidine-1-sulfonyl]-naphthalen-1-yl-pyrrolidine-2,5-dione |
| 710 | 1-[1-(2-Chloro-5-trifluoromethyl-benzenesulfonyl)-piperidin-4-yl]-8methyl-1,4-dihydro-benzo[d][1,3]oxazin-2-one |
| 711 | 1-[1-(3,4-Dimethyl-benzenesulfonyl)-piperidin-4-yl]-8-methyl-1,4-dihydro benzo[d][1,3]oxazin-2-one |
| 712 | 5-Chloro-1-[1-(5-pyridin-2-yl-thiophene-2-sulfonyl)-piperidin-4-yl]-1,4-dihydro-benzo[d][1,3]oxazin-2-one |
| 713 | 3-[4-(5-Chloro-2-oxo-4H-benzo[d][1.3]oxazin-1-yl)-piperidine-1-sulfonyl]-benzonitrile |
| 714 | 3-[4-(5-Chloro-2-oxo-4H-benzo[d][1.3]oxazin-1-yl)-piperidine-1-sulfonyl]-thiophene-2-carboxylic add methyl ester |
| 715 | 1-{5-[4-(5-Chloro-2-oxo-4H-benzo[d][1,3]oxazin-1-yl)-piperidine-1-sulfonyl]-naphthalen-1-yl)-pyrrolidine-2,5-dione |
| 716 | 5-Chloro-1-[1-(2-chloro-S-trifluoromethyl-benzenesulfonyl)-piperidin-4-yl]-1,4-dihydro-benzo[1][1,3]oxazin-2-one |
| 717 | 5-Chloro-1-[1-(3,4-dimethyl-benzenesulfonyl)-piperidin-4-yl]-1,4-dihydro-benzo[d][1,3]oxazin-2-one |
| 718 | 6-Methyl-1-[1-(5-pyridin-2-yl-thiophene-2-sulfonyl)-piperidin-4-yl]-1,4-dihydro-benzo[d][1,3]oxazin-2-one |
| 719 | 3-[4-(6-Methyl-2-oxo-4H-benzo[d][1,3]oxazin-1-yl)-piperidine-1-sulfonyl]-benzonitrile |
| 720 | 3-[4-(6-Methyl-2-oxo-4H-benzo[d][1,3]oxazin-1-yl)-piperidine-1-sulfonyl]-thiophene-2-carboxylic acid methyl ester |
| 721 | 1-{5-[4-(6-Methyl-2-oxo-4H-benzo[d][1,3]oxazin-1-yl)-piperidine-1-sulfonyl]-thiophene-2-pyrrolidine-2,5-dione |
| 722 | 1-[1-(2-Chloro-5-trifluoromethyl-benzenesulfonyl-piperidine-4-yl]-6-methyl-1,4-dihydro-benzo[d][1,3]oxazin-2-one |
| 723 | 1-[1-(3,4-Dimethyl-benzenesulfonyl)-piperidin-4-yl]-6-methyl-1,4-dihydro-benzo[d][1,3]oxazin-2-one |
| 724 | 6-Chloro-1-[1-(5-pyridin-yl-thiophene-2-sulfonyl)-piperidin-4-yl]-1,4-dihydro-benzo[d][1,3]oxazin-2-one |
| 725 | 3-[4-(6-Chloro-2-oxo-4H-benzo[d][1,3]oxazin-1-yl)-piperidine-1-sulfonyl]-benzonitrile |
| 726 | 3-[4-Chloro-2-oxo-4H-benzo[d][1,3]oxazin-1-yl)-piperidine-1-sulfonyl]thiophene-2-carboxylic acid methyl ester |
| 727 | 1-[5-[4-(6-Chloro-2-oxo)-4H-benzo[d][1,3]oxazin-1-yl)-piperidine-1-sulfonyl]-naphthalen-1-yl]-pyrrolidine-2,5-dione |
| 728 | 6-Chloro-1-[1-(2-chloro-5-trifluoromethyl-benzenesulfonyl)-piperidin-4-yl]-1,4-dihydro-benzo[d][1,3]oxazin-2-one |
| 729 | 6-Chloro-1-[1-(3,4-dimethyl-benzenesulfonyl)-piperidin-4-yl-1,4-dihydro-benzo[d][1,3]oxazin-2-one |
| 730 | 1-[1-(5-Methyl-isoxazole-4-sulfonyl)-piperidin-4-yl]-1,4-dihydro-benzo[d][1,3]oxazin-2-one |
| 731 | 1-[1-(2,2-Dimethyl-chroman-6-sulfonyl)-piperidin-4-yl]-1,4-dihydro-benzo[d][1,3]oxazin-2-one |
| 732 | 1-[1-(4-Methyl-3,4-dihydro-2H-benzo[1,4]oxazine-7-sulfonyl)-piperidin-4-yl]-1,4-dihydro-benzo[d][1,3]oxazin-2-one |
| 733 | 1-[1-(2,3-Dihydro-benzo[1,4]dioxine-6-sulfonyl)-piperidin-4-yl]-1,4-dihydro-benzo[d][1,3]oxazin-2-one |
| 734 | 1-[1-(1,3,5-Trimethyl-1H-pyrazole-4-sulfonyl)-piperidin-4-yl]-1,4-dihydro-benzo[d][1,3]oxazin-2-one |
| 735 | 1-[1-(3-Methyl-2-oxo-2,3-dihydro-benzooxazole-6-sulfonyl)-piperidin-4-yl]-1,4-dihydro-benzo[d][1,3]oxazin-2-one |
| 736 | 8-Methyl-1-[1-(5-methyl-isoxazole-4-sulfonyl)-piperidin-4-yl]-1,4-dihydro-benzo[d][1,3]oxazin-2-one |
| 737 | 1-[1-(2,2-Dimethyl-chroman-6-sulfonyl)-piperidin-4-yl]-8-methyl-1,4-dihydro-benzo[d][1,3]oxazin-2-one |
| 738 | 8-Methyl-1-[1-(4-methyl-3,4-dihydro-2H-benzo[1.4]oxazine-7-sulfonyl)-piperidin-4-yl]-1,4-dihydro-benzo[d][1,3]oxazin-2-one |
| 739 | 1-[1-(2,3-Dihydro-benzo[1,4]dioxine-6-sulfonyl)-piperidin-4-yl]-8-methyl-1,4-dihydro-benzo[d][1,3]oxazin-2-one |
| 740 | 8-Methyl-1-[1-(1,3,5-trimethyl-1H-pyrazole-4-sulfonyl)-piperidin-4-yl]-1,4-dihydro-benzo[d][1,3]oxazin-2-one |
| 741 | 8-Methyl-1-[1-(3-methyl-2-oxo-2,3-dihydro-benzooxazole-6-sulfonyl)-piperidin-4-yl]-1,4-dihydro-benzo[d][1,3]oxazin-2-one |
| 742 | 8-Methoxy-1-[1-(1,3,5-trimethyl-1H-pyrazole-4-sulfonyl)-piperidin-4-yl]-1,4-dihydro-benzo[d][1,3]oxazin-2-one |
| 743 | 8-Methoxy-1-[1-(3-methyl-2-oxo-2,3-dihydro-benzooxazole-6-sulfonyl)-piperidin-4-yl]-1,4-dihydro-benzo[d][1,3]oxazin-2-one |
| 744 | 1-[1-(Benzo[d]isoxazol-3-ylmethanesulfonyl)-piperidin-4-yl]-1,4-dihydro-benzo[d][1,3]oxazin-2-one |
| 745 | 1-[1-(2,2,4,6,7-Pentamethyl-2,3-dihydro-benzofuran-5-sulfonyl)-piperidin-4-yl)-1,4-dihydro-benzo[d][1,3]oxazin-2-one |
| 746 | Methyl-5-[4-(2-oxo-4H-benzo[d][1,3]oxazin-1-yl)-piperidine-1-sulfonyl]-1H-pyrimidine-2,4-dione |
| 747 | 1-[1-(3-Methyl-quinoline-8-sulfonyl)-piperidin-4-yl]-1,4-dihydro-benzo[d][1,3]oxazin-2-one |
| 748 | 1-[1-(2,2,5,7,8-Pentamethyl-chroman-6-sulfonyl)-piperidin-4-yl]-1,4-dihydro-benzo[d][1,3]oxazin-2-one |
| 749 | 1,4-Dimethyl-8-[4-(2-oxo-4H-benzo[d][1,3]oxazin-1-yl)piperidine-1-sulfonyl]-1,4-dihydro-quinoxaline-2,3-dione |
| 750 | 1-[1-(1H-Imidazole-4-sulfonyl)piperidin-4-yl]-1,4-dihydro-benzo[d][1,3]oxazin-2-one |
| 751 | 1-[1-(2-Oxo-1,2,3,4-tetrahydro-quinoline-6-sulfonyl)-piperidin-4-yl]-1,4-dihydro-benzo[d][1,3]oxazin-2-one |
| 752 | 7-[4-(2-Oxo-4H-benzo[d][1,3]oxazin-1-yl)-piperidine-1-sulfonyl]-1,5-dihydro-benzo[b][1,4]diazepine-2,4-dione |
| 753 | 8-Methyl-1-[1-(3-methyl-quinoline-8-sulfonyl)-piperidin-4-yl]-1,4-dihydro-benzo[d][1,3]oxazin-2-one |
| 754 | 6-Chloro-1-[1-(3-methyl-quinoline-8-sulfonyl)-piperidin-4-yl]-1,4-dihydro-benzo[d][1,3]oxazin-2-one |
| 755 | 5-Chloro-1-[1-(3-methyl-quinoline-8-sulfonyl)-piperidin-4-yl]-1,4-dihydro-benzo[d][1,3]oxazin-2-one |
| 756 | 8-Methoxy-1-[1-(3-methyl-quinoline-8-sulfonyl)-piperidin-4-yl)-1,4-dihydro-benzo[d][1,3]oxazin-2-one |
| 757 | 1-[1-(Pyridine-2-sulfonyl)-piperidin-4-yl]-1,4-dihydro-benzo[d][1,3]oxazin-2-one |
| 758 | 1-[1-(6,7-Dihydroxy-naphthalene-1-sulfonyl)-piperidin-4-yl]-1,4-dihydro-benzo[d][1,3]oxazin-2-one |
| 759 | Acetic acid 3-acetoxy-5-[4-(2-oxo-4H-benzo[d][1,3]oxazin-1-yl)-piperidine-1-sulfonyl]-naphthalen-2-yl ester |
| 760 | 1-M-(1H-Benzoimidazole-2-sulfonyl)-piperidin-4-yl]-1,4-dihydro-benzo[d][1,3]oxazin-2-one |
| 761 | 1-[1-{1H-Benzoimidazole-2-sulfonyl)-piperidin-4-yl]-8-methoxy-1,4-dihydro-benzo[d][1,3]oxazin-2-one |
| 762 | 1-[1-(1H-Benzoimidazole-2-sulfonyl)-piperidin-4-yl]-5-chloro-1,4-dihydro-benzo[d][1.3]oxazin-2-one |
| 763 | 1-[1-(2,5-Dimethoxy-benzenesulfonyl)-piperidin-4-yl]-6-fluoro-1,4-dihydro-benzo[d][1,3]oxazin-2-one |
| 764 | 1-[1-(2,5-Dimethoxy-benzenesulfonyl-piperidin-4-yl]-8-methoxy-1,4-dihydro-benzo[d][1,3]oxazin-2-one |
| 765 | 1-[1-(2,5-Dimethoxy-benzenesulfonyl)-piperidin-4-yl]-6,7-difluoro-1,4-dihydro-benzo[d][1,3]oxazin-2-one |
| 766 | 5-Chloro-1-[1-(2,5-dimethoxy-benzenesulfonyl)-piperidin-4-yl]-1,4-dihydro-benzo[d][1,3]oxazin-2-one |
| 767 | 1-(1-(5-Dimethylamino-naphthalene-1-sulfonyl-piperidin-4-yl]-8-methoxy-1,4-dihydro-benzo[d][1,3]oxazin-2-one |
| 768 | 5-Chloro-1-[1-(5-dimethylamino-naphthalene-1-sulfonyl)-piperidin-4-yl]-1,4-dihydro-benzo[d][1,3]oxazin-2-one |
| 769 | 6-Chloro-1-[1-(5-chloro-naphthalene-1-sulfonyl)piperidin-4-yl]-1,4-dihydro-benzo[d][1,3]oxazin-2-one |
| 770 | 1-[1-(5-Chloro-naphthalene-1-sulfonyl)-piperidin-4-yl]-6-methyl-1,4-dihydro-benzo[d][1,3]oxazin-2-one |
| 771 | 1-[1-(5-Chloro-naphthatene-1-sulfonyl)-piperidin-4-yl]-6-fluoro-1,4-dihydro-benzo[d][1.3]oxazin-2-one |
| 772 | 6-Chloro-1-[1-(5-chloro-naphthalene-2-sulfonyl)-piperidin-4-yl]-1,4-dihydro-benzo[d][1,3]oxazin-2-one |
| 773 | 1-[1-(5-Chloro-naphthalene-2-sulfonyl)-piperidin-4-yl]-6-methyl-1,4-dihydro-benzo[d][1,3]oxazin-2-one |
| 774 | 1-[1-(5-Chloro-naphthalene-2-sulfonyl)-piperidin-4-yl]-6-fluoro-1,4-dihydro-benzo[d][1,3]oxazin-2-one |
| 775 | 6-Methyl-1-[1-(3-methyl-quinoline-8-sulfonyl)-1,4-dihydro-banzo[d][1,3]oxazin-2-one |
| 776 | 6-Fluoro-1-[1-(3-methyl-quinoline-8-sulfonyl)-piperidin-4-yl]-1,4-dihydro-benzo[d][1,3]axazin-2-one |
| 777 | 6,7-Difluoro-1-[1-(3-methyl-quinoline-8-sulfonyl)-piperidin-4-yl]-1,4-dihydro-benzo[d][1,3]oxazin-2-one |
| 778 | 6-Chloro-1-[1-(3-methyl-2-oxo-2,3-dihydro-benzooxazole-6-sulfonyl)piperidin-4-yl]-1,4-dihydro-benzo[d][1,3]oxazin-2-one |
| 779 | 6-Methyl-1-[1-(3-methyl-2-oxo-2.3-dihydro-benzooxazole-6-sulfonyl)-piperidin-4-yl]-1,4-dihydro-benzo[d][1,3]oxazin-2-one |
| 780 | 6-Fluoro-1-[1-(3-methyl-2-oxo-2,3-dihydro-benzooxazole-6-sulfonyl)-piperidin-4-yl]-1,4-dihydro-benzo[d][1,3]oxazin-2-one |
| 781 | 6,7-Difluoro-1-[1-(3-methyl-2-oxo-2,3-dihydro-benzooxazole-6-sulfonyl)-piperidin-4-yl]-1,4-dihydro-benzo[d][1,3]oxazin-2-one |
| 782 | 5-Chloro-1-[1-(3-methyl-2-oxo-2,3-dihydro-benzooxazole-6-sulfonyl)-piperidin-4-yl]-1,4-dihydro-benzo[d][1,3]oxazin-2-one |
| 783 | 6-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 784 | 6-Methyl-[1-[1-(4-methyl-3,4-dihydro-2H-benzo[1,4]oxazine-7-sulfonyl)piperidin-4-yl]-1,4-dihydro-benzo[d][1,3]oxazin-2-one |
| 785 | 6-Fluoro-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 786 | 8-Methoxy-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 787 | 5-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-one |

58. Process for the preparation of benzoxazinone-derived sulfonamide compounds of general formula (Ib) according to one or more of claim 42 to 57, **characterized in that** it comprises reacting at least one piperidine compound of general formula (Ilb), wherein R^{1b} to R^{9b} have the meaning according to one or more of claims 42-57 and/or a salt, preferably a hydrochloride salt, thereof, 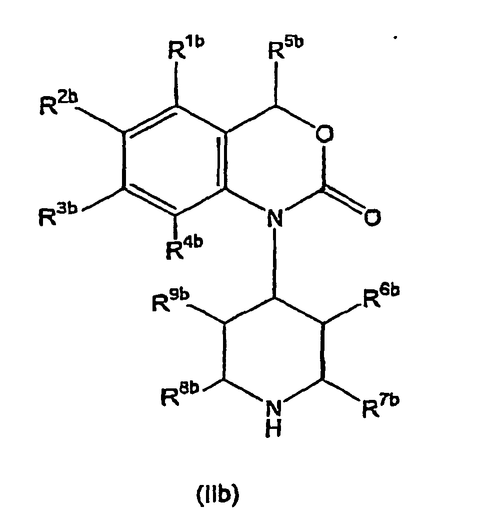 with at least one compound of general formula (IIIb), 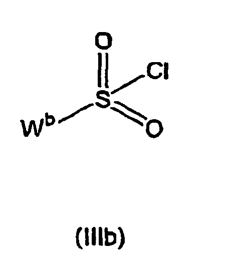 wherein W^{b} has the meaning according to one or more of claims 42-57, in a suitable reaction medium, optionally in the presence of at least one base and/or at least one auxiliary agent.

59. Process for the preparation of a physiologically acceptable salt of the benzoxazinone-derived sulphonamide compounds according to claims 42-57, **characterized in that** at least one compound of general formula (Ib) having at least one basic group is reacted with at least one acid, preferably an inorganic or organic acid, preferably in the presence of a suitable reaction medium.

60. Process for the preparation of a physiologically acceptable salt of the benzoxazinone-derived sulphonamide compounds according to claims 42-57, **characterized in that** at least one compound of general formula (Ib) having at least one acidic group is reacted with at least one base, preferably in the presence of a suitable reaction medium.

61. Medicament comprising at least one benzoxazinone-derived sulphonamide compound according to any one of claims 42-57 and optionally one or more Pharmaceutical acceptable adjuvants.

62. Medicament according to claim 61 for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment type II diabetes (non-insulin dependent diabetes mellitus).

63. Medicament according to claim 61 for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment cognitive disorders, preferably memory disorders; for improvement of cognition (for cognitive enhancement); for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

64. Use of at least one benzoxazinone-derived sulphonamide compound according to any one of claims 42-57 for the manufacture of a medicament for the prophylaxis and/or treatment of food intake disorders.

65. Use according to claim 64 for the regulation of appetite, for the reduction, increase or maintenance of body weight; for the prophylaxis and/or treatment of obesity, for the prophylaxis and/or treatment of bulimia, for the prophylaxis and/or treatment of anorexia; for the prophylaxis and/or treatment of cachexia; or for the prophylaxis and/or treatment of type II diabetes.

66. Use of at least one benzoxazinone-derived sulphonamide compound according to any one of claims 42-57 for the manufacture of a medicament for the prophylaxis and/or treatment of of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression: for the prophylaxis and/or treatment of bipolar disorders; for the prophylaxis and/or treatment cognitive disorders, preferably memory disorders; for improvement of cognition (for cognitive enhancement); for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia; or for the prophylaxis and/or treatment hyperactivity disorder (ADHD, attention deficit, hyperactivity disorder).

## Patentansprüche

1. Benzoxazinon-abgeleitete Sulfonamidverbindungen der allgemeinen Formel (I) 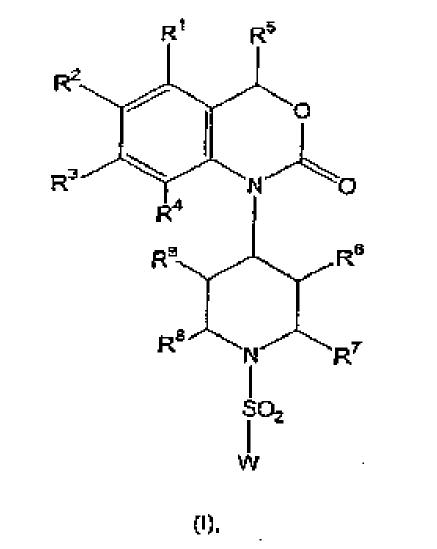 wobei
R¹, R², R³, R⁴ jeweils unabhängig voneinander ausgewählt sind unter Wasserstoff, Halogen, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, welches gebunden sein kann über eine gegebenenfalls wenigstens monosubstituierte Alkylengrupe und/oder welches kondensiert sein kann mit einem gegebenenfalls wenigstens monosubstituierten mono- oder polycyclischen Ringsystem, einem gegebenenfalls wenigstens monosubstituierten Aryl- oder Heteroarylradikal, das gebunden sein kann über eine gegebenenfalls wenigstens monosubstituierte Alkylengruppe und/oder kondensiert sein kann mit einem gegebenenfalls wenigstens monosubstituierten mono- oder polycyclischen Ringsystem, einer Nitrogruppe, einer Cyanogruppe, -OR¹⁰, OC(=O)R¹¹, -(C=O)-OR¹¹, -SR¹², - SOR¹², SO₂R¹², -NH-SO₂R¹², -SO₂NH₂ und einem -NR¹³R¹⁴ -Rest,
R⁵ für Wasserstoff, ein unverzweigtes oder verzweigtes, gesättigtes oder ungesättigtes, gegebenenfalls wenigstens monosubstituiertes aliphatisches Radikal oder ein gesättigtes oder ungesättigtes, gegebenenfalls wenigstens monosubstituiertes, gegebenenfalls wenigstens ein Heteroatom als Ringbestandteil enthaltendes cycloaliphatisches Radikal steht,
R⁶, R⁷, R⁸, R⁹ jeweils unabhängig voneinander ausgewählt sind unter Wasserstoff, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, einer Cyanogruppe und einem -COOR¹⁵-Rest,
Ws für ein unverzweigtes oder verzweigtes, gesättigtes oder ungesättigtes, aliphatisches Radikal steht, das substituiert sein kann mit einem oder mehrere Substituenten, die ausgewählt sind unter Hydroxy, Halogen, verzweigtem oder unverzweigtem C₁₋₄-Alkoxy, verzweigtem oder unverzweigtem C₁₋₄-Perfluoralkoxy, verzweigtem oder unverzweigtem C₁₋₄-Perfluoralkyl, Amino, Carboxy, Amido, Cyano, Nitro, -SO₂NH₂, -CO-C₁₋₄-Alkyl, -SO-C₁₋₄-Alkyl, SO₂-C₁-₄-Alkyl, -NH-SO₂-C₁₋₄-Alkyl, worin das C₁₋₄-Alkyl in jedem Fall verzweigt oder unverzweigt, ein unsubstituiertes oder wenigstens monosubstituiertes Phenyl- oder Naphthylradikal und ein unsubstituiertes oder wenigstens monosubstituiertes Furanyl-, Thienyl-, Pyrrolyl-, Imidazolyl-, Pyrazolyl-, Pyridininyl-, Pyrimidinyl-, Chinolyl- und Isochinolylradikal sein kann,
wobei die Substituenten wenigstens mit F, Cl, Methyl und Methoxy monosubstituiert sein können,
für ein gesättigtes oder ungesättigtes, gegebenenfalls wenigstens ein Heteroatom als Ringbestandteil enthaltendes cycloaliphatisches Radikal steht, wobei das cycloaliphatische Radikal mit einem oder mehreren Substituenten substituiert sein kann, die ausgewählt sind unter Hydroxy, Nitro, Carboxy, Cyano, Keto, Halogen, C₁₋₂₀-Alkyl, teilweise fluoriertem C₁₋₄-Alkyl, teilweise chloriertem C₁₋₄-Alkyl, teilweise bromiertem C₁₋₄-Alkyl, C₁₋₅-Alkoxy, teilweise fluoriertem C₁₋₄-Alkoxy, teilweise chloriertem C₁₋₄-Alkoxy, teilweise bromiertem C₁₋₄-Alkoxy, C₂₋₆-Alkenyl, SO₂-C₁₋₄-Alkyl, -(C=O)-C₁₋₅-Alkyl, -(C=O)-O-C₁₋₅-Alkyl, -(C=O)-Cl, -S-C₁₋₄-Alkyl, -(C=O)-H, -NH-(C=O)-NH-C₁₋₅-Alkyl, -(C=O)-C₁₋₄-Perfluoralkyl, -NR^{A}R^{B}, wobei R^{A} und R^{B} unabhängig voneinander ausgewählt sind unter H, C₁₋₄-Alkyl und Phenyl, NH-(C=O)-C₁₋₅-Alkyl, -C₁₋₅-Alkylen-(C=O)-C₁₋₅-Alkyl, (1,3-Dihydro-1-oxo-2H-isoindol-2-yl), N-Phthalimidinyl-, (1,3-Dioxo-2-azaspiro[4,4]-non-2-yl, substituiertem oder unsubstituiertem Phenyl, -SO₂-Phenyl, Phenoxy, Pyridinyl, Pyridinyloxy, Pyrazolyl, Pyrimidinyl, Pyrrolidinyl-, - SO₂-Pyrrolidinyl, Morpholinyl, SO₂-Morpholinyl-, Thiadiazolyl, Oxadiazolyl, Oxazolyl, Thiazolyl, Isoxazolyl, O-CH₂-Thiazolyl, -NH-Phenyl, und -C₁₋₄-Alkylen-N H-(C=O)-Phenyl,
wobei die Substitutenten mit einem oder mehreren Substitutenten substituiert sein können, die ausgewählt sind unter Halogen, Nitro, Cyano, Hydroxy, - (C=O)-C₁₋₄-Alkyl, C₁₋₄-Alkyl, wenigstens teilweise fluoriertem C₁₋₄-Alkyl, wenigstens teilweise chloriertem C₁₋₄-Alkyl, wenigstens teilweise bromiertem C₁₋₄-Alkyl, -S-C₁₋₄-Alkyl, -C(=O)-O-C₁₋₅-Alkyl, -(C=O)-CH₂-F, -(C=O)-CH₂-Cl und - (C=O)-CH₂-Br,
und wobei das cycloaliphatische Radikal über eine gegebenenfalls monosubstituierte Alkylengruppe gebunden sein kann und/oder mit einem gegebenenfalls wenigstens monosubstituierten aromatischen mono- oder polycyclischen Ringsystem kondensiert sein kann,
für ein gegebenenfalls wenigstens monosubstituiertes Heteroarylradikal steht, das über eine gegebenenfalls monosubstituierte Alkylengruppe gebunden sein kann und/oder mit einem gegebenenfalls wenigstens monosubstituierten mono- oder polycyclischen Ringsystem kondensiert sein kann,
für ein gegebenenfalls wenigstens monosubstituiertes, monocyclisches Arylradikal steht, das mit einem gegebenenfalls wenigstens monosubstituierten aromatischen mono- oder polycyclischen Ringsystem kondensiert ist und über eine gegebenenfalls wenigstens monosubstituierte Alkylengruppe gebunden sein kann,
für einen NR¹⁶R¹⁷-Rest steht,
für einen -COR¹⁶-Rest steht,
oder für ein Phenylradikal steht, das wenigstens monosubstituiert ist mit einem der Substitutenten, die ausgewählt sind unter:
2,2,2,-Trifluorethoxy-, C₂₋₆-Alkenyl-, 1,3-Dihydro-1-oxo-2H-isoindol-2-yl-, N-Phthalimidinyl-, [(2-Chlor-1,3-thiazolyl-5-yl)-methoxy, Ethyl-5-yl-2-methyl-3-furoat, C₁₁₋₂₀-Alkyl-, 1,3-Dioxo-2-azaspiro [4,4]non-2-yl-, Pyrazolyl-, (1,3-Oxazol-5-yl)-, (5-Methyl-1,3,4-oxadiazol-2-yl)-, Difluormethoxy, Dichlormethoxy, 1-Pyrrolidinylsulfonyl, Morpholinosulfonyl, 2-Methyl-4-pyrimidinyl-, einer Phenoxygruppe, die wenigstens monosubstitiert ist mit C₁₋₅-Alkoxy, einer Phenylgruppe, die wenigstens monosubstituiert ist mit einem der Substituenten, die ausgewählt sind unter Nitro, C₁₋₅-Alkoxy, F, Cl, Br, einem wenigstens teilweise fluoriertem C₁₋₅- Alkyl, einem wenigstens teilweise chloriertem C₁₋₅-Alkyl, [(2-Chlor-1,3-thiazol-5-yl)-methoxy]-, - (C=O)-H und -(C=O)-C₁₋₅-Alkyl, einer Pyridinylgruppe, die wenigstens monosubstituiert ist mit C₁₋₅-Alkoxy, einer Pyridinyloxygruppe, die wenigstens monosubstituiert ist mit C₁₋₅-Alkoxy, einer Phenoxygruppe, die wenigstens disubstituiert ist und einer Pyridinyloxygruppe, die wenigstens disubstituiert ist und einer Pyrdinyloxygruppe, die wenigstens disubstituiert ist,
mit der Maßgabe, dass W nicht für unsubstituiertes Furyl, unsubstituiertes Thienyl- oder Thienyl, das substituiert ist mit einem Substituenten, der ausgewählt ist unter C₁₋₅-Alkoxycarbonyl, C₁₋₅-Alkylcarbonyl, Carboyxl und Pyridyl, unsubstituiertes Pyrrolyl, unsubstituiertes Naphthyl, unsubstituiertes Indolyl, unsubstituiertes Tehtrahydronaphthyl, substituiertes oder unsubstituiertes Pyridyl, unsubstituiertes Pyrazinyl, unsubstituiertes Chinolyl, C₁₋₅-alkylsubstituiertes Pyrrolyl, und unsubstituiertes Cyclohexyl oder Cyclohexyl, das substituiert ist, mit einem oder zwei Bestandteilen, die ausgewählt sind unter Oxo, Hydroxyl, C₁₋₅-Alkoxyl, C₁₋₅-Alkoxy-carbonylamino-C₁₋₅-alkyl und Amino-C₁₋₅-Alkyl, steht
R¹⁰ für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder über ein gegebenenfalls wenigstens monosubstituiertes mono- oder polyzyklisches Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder über ein gegebenenfalls wenigstens monosubstituiertes mono- oder polyzyklisches Ringsystem kondensiert sein kann, steht,
R¹¹ für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einer gegebenenfalls wenigstens monosubstituierten mono- oder polyzyklischen Ringsystem kondensiert sein kann, steht,
R¹² für ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R¹³ und R¹⁴ unabhängig voneinander ausgewählt sind unter Wasserstoff, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder einem gegebenenfalls wenigstens monosubstituiertem Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann,
oder R¹³ und R¹⁴ zusammen mit dem überbrückenden Stickstoffatom einen gesättigten, ungesättigten oder aromatischen heterozyklischen Ring bilden, der wenigstens monosubstituiert und/oder wenigstens ein weiteres Heteroatom als Ringbestandteil enthalten kann,
R¹⁵ für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R¹⁶ für ein unverzweigtes oder verzweigtes, gesättigtes oder ungesättigtes, gegebenenfalls wenigstens monosubstituiertes aliphatisches Radikal steht,
R¹⁷ für ein unverzweigtes oder verzweigtes, gesättigtes oder ungesättigtes, gegebenenfalls wenigstens monosubstituiertes aliphatisches Radikal steht,
R¹⁸ für ein gegebenenfalls wenigstens monosubstituiertes Arylradikal steht,
gegebenenfalls in Form eines ihrer Stereoisomere, vorzugsweise Enantiomere oder Diastereomere, ihres Racemats oder in Form eines Gemisches von wenigstens zwei ihrer Stereoisomere, vorzugsweise Enantiomere oder Diastereomere, in einem beliebigen Mischungsverhältnis, oder ein ensprechendes Salz davon, oder ein entsprechendes Solvat.

2. Verbindungen nach Anspruch 1, **dadurch gekennzeichnet, dass** R¹, R², R³, R⁴ jeweils unabhängig voneinander ausgewählt sind unter H, F, Cl, Br, einem unverzweigten oder verzweigten, gesättigten oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteoratom als Ringbestandteil enthaltenden C₃₋₈-cycloaliphatischen Radikal, das über eine gegebenenfalls wenigstens monosubstituierte C₁₋₆-Alkylengruppe gebunden sein kann und/oder mit einem gegebenenfalls wenigstens monosubstituierten mono- oder polyzyklischen Ringsystem kondensiert sein kann, einem gegebenenfalls wenigstens monosubstituierten 5- oder 6-gliedrigen Aryl- oder Heteroarylradikal, das über eine gegebenenfalls wenigstens monosubstituierte C₁₋₆-Alkylengruppe gebunden sein kann und/oder mit einem gegebenenfalls wenigstens monosubstituierten mono- oder polyzyklischen Ringsystem kondensiert sein kann, einer Nitrogruppe, einem Cyano-, OR¹⁰, OC(=O)R¹¹, - SR¹², -SOR¹², SO₂R¹², -NH-SO₂R¹², -SO₂NH₂ und einem -NR¹³R¹⁴ -Rest, vorzugsweise ausgewählt sind unter H, F, Cl, Br, einem gesättigten, verzweigten oder unverzweigten, gegebenenfalls wenigstens monosubstituierten C₁₋₃-aliphatischen Radikal, einem gesättigtem, gegebenenfalls wenigstens monosubstituierten, gegebenenfalls wenigstens ein Heteroatom als Ringbestandteil enthaltenden C₅- oder C₆-cycloaliphatischen Radikal, das über eine gegebenenfalls wenigstens monosubstituierte C₁- oder C₂-Alkylengruppe, einen Nitro-, Cyano-, -OR¹⁰, OC(=O)R¹¹, -SR¹², und einen - NR¹³R¹⁴-Rest gebunden sein kann, besonders bevorzugt ausgewählt sind unter H, F, Cl, -CH₃, -CH₂CH₃, -CF₃, -CF₂CF₃, Cyclopentyl, Cyclohexyl, einer Nitrogruppe, einer Cyanogruppe und -OR¹⁰.

3. Verbindungen nach Anspruch 1 oder Anspruch 2, **dadurch gekennzeichnet, dass** R⁵ für Wasserstoff, ein unverzweigtes oder verzweigtes, gesättigtes oder ungesättigtes, gegebenenfalls wenigstens monosubstituiertes C₁₋₆-aliphatisches Radikal oder ein gesättigtes oder ungesättigtes, gegebenenfalls wenigstens monosubstituiertes, gegebenenfalls wenigstens ein Heteroatom als Ringbestandteil enthaltendes C₃₋₈-cycloaliphatisches Radikal, vorzugsweise für H oder ein verzweigtes oder unverzweigtes C₁₋₃-Alkylradikal , besonders bevorzugt für H, -CH₃ oder-CH₂-CH₃ steht.

4. Verbindungen nach einem der Ansprüche 1 bis 3, **dadurch gekennzeichnet, dass** R⁶, R⁷, R⁸, R⁹ jeweils unabhängig voneinander ausgewählt sind unter Wasserstoff, einem unverzweigten oder verzweigten, gesättigtem oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteroatom als Ringbestandteil enthaltenden C₃₋₈-cycloaliphatischen Radikal, einer Cyanogruppe und einem -COOR¹⁵-Rest, vorzugsweise ausgewählt sind unter H, einem verzweigten oder unverzweigten C₁₋₃-Alkylradikal, einer Cyanogruppe und einem -COOR¹⁵- Rest, besonders bevorzugt ausgewählt sind unter H, - CH₃, CH₂CH₃ und einer Cyanogruppe.

5. Verbindungen nach einem der Ansprüche 1 bis 4, **dadurch gekennzeichnet, dass** W für ein unverzweigtes oder verzweigtes C₁₁₋₂₀-Alkylradikal, das mit einem oder mehreren Substitutenten substituiert sein kann, die ausgewählt sind unter Hydroxy, Halogen, verzweigtem oder unverzweigtem C₁₋₄-Alkoxy, verzweigtem oder unverzweigtem C₁₋₄-Perfluoralkoxy, verzweigtem oder unverzweigetem C₁₋₄-Perfluoralkyl, Amino, Carboxy, Amido, Cyano, Nitro, - SO₂NH₂, -CO-C₁₋₄-Alkyl, -SO-C₁₋₄-Alkyl, -SO₂-C₁₋₄-Alkyl, -NH-SO₂-C₁₋₄-Alkyl, wobei das C₁₋₄-Alkyl in jedem Fall verzweigt oder unverzweigt sein kann, einem unsubstituierten oder wenigstens monosubstituierten Phenyl- oder Naphthylradikal und einem unsubstituierten oder wenigstens monosubstituierten Furanyl-, Thienyl-, Pyrrolyl-, Imidazolyl-, Pyrazolyl-, Pyridinyl-, Pyrimidinyl- Chinolinyl- und Isochinolinylradikal, wobei die Substituenten wenigstens monosubstituiert sein können mit F, Cl, Methyl und Methoxy; einer Naphthylgruppe, die wenigstens monosubstituiert ist, einer Chinolylgruppe, die wenigstens monosubstituiert ist, einer Pyrrolylgruppe, die wenigstens monosubstituiert ist mit einem von C₁₋₅-Alkyl verschiedenen Substituenten, einer gegebenenfalls wenigstens monosubstituierten Thiazolyl-, Benzo[b]-thiophenyl-, Benzo[b]-furanyl-, Isochinolinyl-, Tetrahydroisochinolinyl-, Pyrazolyl-, Isoazolyl-, Chromanyl-, Benzothiadiazolyl-, Imidazolyl-, Benzofurazanyl-, Dibenzo[b,d]-furanyl-, Benzoxadiazolyl-, Imidazo[2,1-b]-thiazolyl-, Anthracenyl-, Coumarinyl-, 2,3-Dihydro-1,4-benzodioxinyl-, 2,3-Dihydrobenzo[b]furanyl-, 3,4-Dihydro-2H-1,4-benzoxazinyl-, 3,4-Dihydro-2H-1,5-benzodioxepinyl-, Benzothiazolyl-, Imidazo[1,2-a]-pyridinyl-, einer Chromonylgruppe, einer Isatinylgruppe, eine Pentamethyldihydrobenzofuranylgruppe, einer Cyclopropyl- oder Cyclopentylgruppe, wobei die Cyclopropylgruppe oder die Cyclopentylgruppe substituiert sein kann durch einen oder mehrere Substituenten, die ausgewählt sind unter Hydroxy, Nitro, Carboxy, Cyano, Keto, Halogen, C₁₋₂₀-Alkyl, teilweise fluoriertem C₁₋₄-Alkyl, teilweise chloriertem C₁₋₄-Alkyl, teilweise bromiertem C₁₋₄-Alkyl, C₁₋₅-Alkoxy, teilweise fluoriertem C₁₋₄-Alkoxy, teilweise chloriertem C₁₋₄-Alkoxy, teilweise bromiertem C₁₋₄-Alkoxy, C₂₋₆-Alkenyl, SO₂-C₁₋₄-Alkyl, -(C=O)-C₁₋₅-Alkyl, -(C=O)-O-C₁₋₅-Alkyl, -(C=O)-Cl, S-C₁₋₄-Alkyl-, -(C=O)-H, -NH-(C=O)-NH-C₁₋₅-Alkyl, -(C=O)-C₁₋₄-Perfluoralkyl, -NR^{A}R^{B}, wobei R^{A} und R^{B} unabhängig voneinander ausgewählt sind unter H, C₁₋₄-Alkyl und Phenyl, NH-(C=O)-C₁₋₅-Alkyl, -C₁₋₅-Alkylen-(C=O)-C₁₋₅-Alkyl, (1,3-Dihydro-1-oxo-2H-isoindol-2-yl), N-Phthalimidinyl-, (1,3-Dioxo-2-azaspiro[4,4]-non-2-yl, substituiertem oder unsubstituiertem Phenyl, -SO₂-Phenyl, Phenoxy, Pyridinyl, Pyridinyloxy, Pyrazolyl, Pyrimidinyl, Pyrrolidinyl-, -SO₂-Pyrrolidinyl, Morpholinyl, SO₂-Morpholinyl-, Thiadiazolyl, Oxadiazolyl, Oxazolyl, Thiazolyl, Isoxazolyl, O-CH₂-Thiazolyl, -NH-Phenyl, und -C₁₋₄-Alkylen-NH-(C=O)-Phenyl, wobei die Substituenten mit einem oder mehreren Substituenten substituiert sein können, die ausgewählt sind unter Halogen, Nitro, Cyano, Hydroxy, - (C=O)-, C₁₋₄-Alkyl, C₁₋₄-Alkyl, wenigstens teilweise fluoriertem C₁₋₄-Alkyl, wenigstens teilweise chloriertem C₁₋₄-Alkyl, wenigstens teilweise bromiertem C₁₋₄-Alkyl, -S-C₁₋₄-Alkyl, -C(=O)-O-C₁₋₅-Alkyl, -(C=O)-CH₂-F, -(C=O)-CH₂-Cl und - (C=O)-CH₂-Br, einem 2-(1,3-Dioxo-1,3-dihydro-isoindol-2-yl-)-ethyl, einer Thienylgruppe, die wenigstens monosubstituert ist mit einem oder mehreren Substituenten, die unabhängig voneinander ausgewählt sind unter F, Cl, Br, C₁₋₅-Alkoxy-, CF₃, -SO₂-C₁₋₅-Alkyl und gegebenenfalls wenigstens monosubstituiertem Benzoylaminomethyl-, Phenylsulfonyl-, Isoxazolyl-, Benamidomethyl-, Pyrimidyl-, Thiazolyl-, Pyrazolyl-, Phenyl-, 1,2,4-Thiazolyl-, Benzamidomethyl-, Pyrimidyl-, Thiazolyl-, Pyrazolyl-, Phenyl-, 1,2,4-Thiadiazolyl-, 1,3-Oxazolyl-, oder 1,2,4-Oxadiazolyl-, einer Furylgruppe, die wenigstens monosubstituiert ist mit einem oder mehreren Substitutenten, die unabhängig voneinander ausgewählt sind unter einem C₁₋₅-Alkylradikal, das wenigstens teilweise fluoriert oder chloriert sein kann, einem gegebenenfalls wenigstens monosubstituierten Phenyl und einer-(C=O)-O-C₁₋₅-Alkylgruppe, steht,
für einen NR¹⁶R¹⁷-Rest steht,
für einen COR¹⁸-Rest steht,
oder für ein Phenylradikal steht, das wenigstens monosubstituiert ist mit einem der Substitutenten, die ausgewählt sind unter:
2,2,2,-Trifluorethoxy-, C₂₋₆-Alkenyl-, 1,3-Dihydro-1-oxo-2H-isoindol-2-yl-, N-Phthalimidinyl-, [(2-Chlor-1,3-thiazolyl-5-yl)-methoxy, Ethyl-5-yl-2-methyl-3-furoat, C₁₁₋₂₀-Alkyl-, 1,3-Dioxo-2-azaspiro [4,4]non-2-yl-, Pyrazolyl-, (1,3-Oxazol-5-yl)-, (5-Methyl-1,3,4-oxadiazol-2-yl)-, Difluormethoxy, Dichlormethoxy, 1-Pyrrolidinylsulfonyl, Morpholinosulfonyl, 2-Methyl-4-pyrimidinyl-, einer Phenoxygruppe, die wenigstens monosubstitiert ist mit C₁₋₅-Alkoxy, einer Phenylgruppe, die wenigstens monosubstituiert ist mit einem der Substituenten, die ausgewählt sind unter Nitro, C₁₋₅-Alkoxy, F, Cl, Br, einem wenigstens teilweise fluoriertem C₁₋₅-Alkyl, einem wenigstens teilweise chloriertem C₁₅-Alkyl, [(2-Chlor-1,3-thiazol-5-yl)-methoxy]-, -(C=O)-H und -(C=O)-C₁₋₅-Alkyl, einer Pyridinylgruppe, die wenigstens monosubstituiert ist mit C₁₋₅-Alkoxy, einer Pyridinyloxygruppe, die wenigstens monosubstituiert ist mit C₁₋₅-Alkoxy, einer Phenoxygruppe, die wenigstens disubstituiert ist und einer Pyridinyloxygruppe, die wenigstens disubstituiert ist,
W besonders bevorzugt steht für einen Rest, der ausgewählt ist unter 5-Dimethylamino-napth-1-yl, 2-Acetamido-4-methyl-5-thiazolyl-, Trifluormethyl-, Trichlormethyl-, Isopropyl-, Methyl-, 2,2,2-Trifluorethyl-, Ethyl-, Hexadecyl-, 2-Chlorethyl, n-Propyl-, 3-Chlor-propyl-, n-Butyl-, Dichlormethyl-, Chlormethyl-, Dodecyl-, 1-Octyl-, 6-(p-toluidino)-naphth-2-yl- , 4,5-Dibrom-thiophen-2-yl-, Benzoylchlorid-3-yl-, 1-Octadecyl, 4-Brom-2,5-dichlor-thiophen-3-yl-, 2,5-Dichlor-thiophen-3-yl-, 5-Chlor-thiophen-2-yl-, 1-Decyl-, 3,5-Dichlor-4-(2-chlor-4-nitrophenoxy)-phenyl-, 2,3- Dichlorthiophen-5-yl-, 3-Brom-2-chlor-thiophen-5-yl-, 3-Brom-5-chlor-thiophen-2-yl-, 2-(Benzoylaminomethyl)-thiophen-5-yl-, 4-(Phenyl-sulphonyl)-thiophen-2-yl-, 2-Phenyl-sulphonyl-thiophen-5-yl-, 2-[1-Methyl-5-(trifluormethyl)pyrazol-3-yl]-thiophen-5-yl-, 5-Chlor-1,3-dimethylpyrazol-4-yl-, 3,5-Dimethylisoxazol-4-yl-, 2-(2,4-Dichlorphenoxy)-phenyl, 4-(2-Chlor-6-nitro-phenoxy)-phenyl-, 4-(3-Chlor-2-cyanophenoxy)-phenyl, 2,4-Dimethyl-1,3-thiazol-5-yl-, Methyl-methan-sulfonyl-, 2,5-Bis-(2, 2, 2-Trifluorethoxy)-phenyl-, 5-(Di-n-propylamino)-naphth-1-yl-, 2,2,5,7,8-Pentamethyl-chroman-6-yl-, 5-Chlor-4-nitro-thiophen-2-yl-, 2,1,3-Benzothiadiazol-4-yl-, 1-Methyl-imidazole-4-yl-, Benzofurazan-4-yl-, 5-(Isoxazol-3-yl)-thiophen-2-yl-, Vinyl-phenyl-4-yl-, 5-Dichlor-methyl-furan-2-yl-, 5-Bromthiophen-2-yl-, 5- (4-Chlorbenzamidomethyl)-thiophen-2-yl-, Dibenzo[b,d]-furan-2-yl-, 5-Chlor-3-methylbenzo[b]-thiophen-2-yl-, 3-Methoxy-4-(methoxycarbonyl)-thiophen-2-yl-, 5-[2-(Methylthio)-pyrimidin-4-yl-]-thiophen-2-yl-, 4-Chlor-2,1,3-benzoxadiazol-7-yl-, 5-Chlor-2,1,3-benzoxadiazol-4-yl-, 6-Chlor-imidazo(2,1-b)-thiazol-5-yl-, 3-Methyl-benzo[b]-thiophen-2-yl-, 4-[[3-Chlor-5-(trifluormethyl)-2-pyridyl]oxy-phenyl-, 5-Chlor-naphth-1-yl-, 5-Chlor-naphth-2-yl-, 9,10-Dibromanthracen-2-yl-, Isochinolin-5-yl-, 4-Methoxy-2,3,6-trimethylbenzoyl-, 4'-Nitro-biphenyl-4-yl-, (1,3-Dihydro-1-oxo-2H-isoindol-2-yl-)-4-phenyl-, 5-(2-Methyl-1,3-thiazol-4-yl)-thiophen-2-yl-, 5-(1-Methyl-3-(trifluormethyl)pyrazol-5-yl]-thiophen-2-yl-, 5-[5-Trifluormethyl)-isoxazol-3- yl]-thiophen-2-yl-, p-Dodecyl-phenyl-, 4-[(3-Cyano-4-methoxy-2-pyridinyl)oxy]-phenyl-, 4- (N-Phthalimidinyl)-phenyl-, 1,2,3,4-Tetrahydro-2-(trifluoracetyl)-isochinolin-7-yl-, 1,2-Dimethylimidazol-4-yl-, 2,2,4,6,7-Pentamethyldihydrobenzofuran-5-yl-, 4-Chlornaphth-1-yl-, 2,5-Dichlor-4-nitro-thiophen-3-yl-, 4-(4-Methoxy-phenoxy)-phenyl-, [4-(3,5- Dichlorphenoxy)phenyl]-, [4-(3,4-Dichlorphenoxy)phenyl]-, [4-(3,5)-Bis (trifluormethylphenoxy)phenyl]-, 3-(2-Methoxy-phenoxy)-phenyl, 3-(4-Methoxy-phenyl)-phenyl-, 3-(4-Chlor-phenyl)-phenyl-, 3-(3,5-Dichlor-phenyl)-phenyl-, 3-(3,4-Dichlor-phenyl)-phenyl-, 3-(4-Fluorphenyl)-phenyl-, 3-[4-(Trifluormethyl)-phenyl]-phenyl-, 3-[3,5-Bis-(trifluormethyl)-phenyl]-phenyl-, 4-(2-Methoxyphenoxy)-phenyl-, 4-(2-Methyl-phenoxy)-phenyl-, 4-(4-Methoxy-phenoxy)-phenyl-, 4-(4-Chlorphenyl)-phenyl-, 4-(3,5- Dichlorphenyl)-phenyl-, 4-(3,4-Dichlorphenyl)-phenyl-, 4-(4-Fluorphenyl)-phenyl-, 4-[4-(Trifluormethyl)-phenyl]-phenyl-, 4-[3,5-Bis-(trifluormethyl)-phenyl]-phenyl-, Cyclopropyl-, 2-(2-Chlorphenyl)-2-phenylethyl-, 2-(2-Trifluormethylphenyl)-2-phenylethyl-, 5-[4-Cyano-1-methyl-5-(methylthio)-1 H-pyrazol-3-yl-thiophen-2-yl-, 3-Cyano-2,4-bis-(2,2,2-trifluorethoxy)-phenyl-, 4-[(2-Chlor-1,3-thiazol-5-yl)-methoxy]-phenyl-, 2-(1,3-Dioxo-1,3-dihydro-isoindol-2-yl)-ethyl-, 5-lod-naphth-1-yl-, Ethyl-2,5-dimethyl-1-phenylpyrrol-4- carboxylat-3-yl-, Ethyl-2-methyl-1,5-diphenyl-1 H-pyrrol-3-carboxylat-4-yl-, Ethyl-5-(4-chlorphenyl)-2-methyl-3-furoat-4-yl, Ethyl-5-(4-chlorphenyl)-2-methyl-1-phenyl-3-carboxylat-4-yl-, Ethyl-2,5-dimethyl-3-furoat-4-yl-, 3-Chlor-4-(1,3-dioxo-2-azaspiro[4,4] non-2-yl)-phenyl-, Coumarin-6-yl-, 3-(4-Methoxy-phenoxy)-phenyl-, [3-(3,5-Dichlorphenoxy)]-phenyl-, [3-(3,4-Dichlorphenoxy)]-phenyl-, 3,5-Bis-(trifluormethyl)phenoxyphenyl-, 2,2-Diphenylethyl-, 4-Phenyl-5-(trifluormethyl)-thiophen-3-yl-, Methyl-4-phenyl-5-(trifluormethyl)thiophen-2-carboxylat-3-yl-, Methyl-1,2,5- trimethylpyrrol-3-carboxylat-4-yl-, 4-Fluor-naphth-1-yl-, 5-Fluor-3-methylbenzo[b]-thiophen-2-yl-, Methyl-2,5-dimethyl-3-furoat-4-yl-, Methyl-2-furoat-5-yl-, Methyl-2-methyl-3-furoat-5-yl-, Methyl-1-methyl-1 H-pyrrol-2-carboxylat-5-yl-, 2-(5-Chlor-1,2,4-thiadiazol-3-yl)-thiophen-5-yl-, 1,3,5-Trimethyl-1 H-pyrazol-4-yl-, Pentafluorethoxytetrafluorethyl-, 5-(5-Isoxazyl)-thiophen-2-yl-, 5-(5-Isoxazol-yl)-2-furyl-, 5-Methyl-2,1,3-benzothiadiazol-4- yl-, 2,3-Dihydro-1,4-benzodioxin-6-yl-, 4-Methyl-naphth-1-yl-, 5-Methyl-2-(trifluormethyl)-3-Furyl-, 2,3-Dihydrobenzo[b]furan-5-yl-, 1-Benzothiophen-3-yl-, 4-Methyl-3,4-dihydro-2H-1,4-benzoxazin-7-yl-, 5-Methyl-1-phenyl-1 H-pyrazol-4-yl-, 6-Morpholino-3-pyridinyl-, 4-(1 H-Pyrazol-1-yl)phenyl-, 6-Phenoxy-3-pyridyl-, 3,4-Dihydro-2H-1,5-benzodioxepin-7-yl-, 5-(1,3-Oxazol-5-yl)-2-thienyl-, 4-(1,3-Oxazol-5-yl)-phenyl-, 5-Methyl-4-isoxazolyl, 2,1,3-Benzothiadiazol-5-yl-, 5-Acetamidonaphth-1-yl-, 3-Methyl-8-chinolinyl-, 1,3-Benzothiazol-6-yl-, 2-Morpholino-3-pyridyl-, 2,5-Dimethyl-3-thienyl-, 5-[5- (Chlormethyl)-1,2,4-oxadiazol-3-yl]-2-thienyl-, Ethyl-3-[5-yl-2-thienyl-] 1,2,4-oxadiazol-5-carboxylat-, 3-(5-Methyl-1,3,4-oxadiazol-2-yl)-phenyl-, 4-(Difluormethoxy)-phenyl-, 3- (Difluormethoxy)-phenyl-, 2,2-Dimethyl-6-chromanyl-, Ethyl-3,5-dimethyl-1 H-pyrrol-2-carboxylat-4-yl-, Imidazo[1,2-A]pyridin-3-yl-, 3-(1,3-Oxazol-5-yl)-phenyl-, Ethyl-5-[4-yl)-phenyl]-2-methyl-3-furoat, 1-Pyrrolidinylphenylsulfonyl-, Methyl-5-yl-4-methyl-2-thiophen- carboxylat, Methyl-3-yl-4-(isopropylsulfonyl)-2-thiophen, 7-Chlorchromon-3-yl-, 4'-Brom-biphenyl-4-yl-, 4'-Acetyl-biphenyl-4-yl-, 4'-Brom-2'-fluor-biphenyl-4-yl-, 1-Methyl-5-isatinyl-, 2-Chlor-3-thiophencarbonsäure-5-yl-, 2-Methoxy-5-(N-phthalimidinyl)-phenyl-, 1-Benzothiophen-2-yl-, Morpholinophenylsulfonyl- und 3-(2-Methyl-4-pyrimidinyl)-phenyl-.

6. Verbindungen nach einem der Ansprüche 1 bis 5, **dadurch gekennzeichnet, dass** R¹⁰ steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischem Ringsystem,
vorzugsweise für H, ein lineares oder verzweigtes C₁₋₄-Alkylradikal, ein Cyclohexylradikal oder ein Phenylradikal steht, besonders bevorzugt für H, - CH₃, -C₂H₅ oder Phenyl steht.

7. Verbindungen nach einem der Ansprüche 1 bis 6, **dadurch gekennzeichnet, dass** R¹¹ steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₈-Alkylengruppe und/oder kondensiert sein kann mit einem gegebenenfalls wenigstens monosubstituierten mono- oder polyzyklischen Ringsystem,
vorzugsweise für H, ein lineares oder verzweigtes C₁₋₄-Alkylradikal, ein Cyclohexylradikal oder ein Phenylradikal steht, besonders bevorzugt H, -CH₃, - C₂H₅ oder Phenyl steht.

8. Verbindungen nach einem der Ansprüche 1 bis 7, **dadurch gekennzeichnet, dass** R¹² steht für ein unverzweigtes oder verzweigtes, gesättigtes oder ungesättigtes, gegebenenfalls wenidstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cycloaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstitierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischem Ringsystem,
vorzugsweise für H, ein lineares oder verzweigtes C₁₋₄-Alkylradikal, ein Cyclohexylradikal oder ein Phenylradikal steht, besonders bevorzugt für H, - CH₃, -C₂H₅ oder Phenyl steht.

9. Verbindungen nach einem der Ansprüche 1 bis 8, **dadurch gekennzeichnet, dass** R¹³ und R¹⁴ unabhängig voneinander ausgewählt sind unter Wasserstoff, einem unverzweigten oder verzweigten, gesättigten oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteroatom als Ringbestandteil enhaltenden C₃₋₈-cycloaliphatischen Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder einem gegebenenfalls wenigstens monosubstituierten, 5- oder 6-gliedrigen Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem,
vorzugsweise jeweils unabhängig voneinander ausgewählt sind unter H, einem linearen oder verzweigten C₁₋₄-Alkylradikal, einem Cyclohexylradikal und einem Phenylradikal, besonders bevorzugt jeweils unabhängig voneinander ausgewählt sind unter H, -CH₃, -C₂H₅ und Phenyl.

10. Verbindungen nach einem der Ansprüche 1 bis 8, **dadurch gekennzeichnet, dass** R¹³ und R¹⁴ zusammen mit dem überbrückenden Stickstoffatom einen gesättigten, ungesättigten oder aromatischen, 5- oder 6-gliedrigen heterozyklischen Ring bilden, der wenigstens monosubstituiert sein kann und/oder wenigstens ein weiteres Heteroatom als Ringbestandteil enthalten kann, vorzugsweise eine unsubstituierte Piperidin- oder Morpholingruppe bilden.

11. Verbindungen nach einem der Ansprüche 1 bis 10, **dadurch gekennzeichnet, dass** R¹⁵ steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, vorzugsweise für H, ein lineares oder verzweigtes C₁₋₄-Alkylradikal, ein Cyclohexylradikal oder ein Phenylradikal steht, besonders bevorzugt für H, -CH₃, -C₂H₅ oder Phenyl steht.

12. Verbindungen nach einem der Ansprüche 1 bis 11, **dadurch gekennzeichnet, dass** R¹⁶ für ein unverzweigtes oder verzweigtes, gesättigtes oder ungesättigtes, gegebenenfalls wenigstens monosubstituiertes C₁₋₆-aliphatisches Radikal, vorzugsweise für ein unverzweigtes oder verzweigtes, gesättigtes, unsubstituiertes C₁₋₃-Alkylradikal, besonders bevorzugt für ein Methylradikal steht.

13. Verbindungen nach einem der Ansprüche 1 bis 12, **dadurch gekennzeichnet, dass** R¹⁷ für ein unverzweigtes oder verzweigtes, gesättigtes oder ungesättigtes, gegebenenfalls wenigstens monosubstituiertes C₁₋₆-aliphatisches Radikal, vorzugsweise für ein unverzweigtes oder verzweigtes, gesättigtes, unsubstituiertes C₁₋₃-Alkylradikal, besonders bevorzugt für ein Methylradikal steht.

14. Verbindungen nach einem oder mehreren der Ansprüche 1 bis 13:
1-[1-(5-Chlor-3-methyl-benzo[b]thiophenyl-2-sulfonyl)-piperidin-4-yl]-1,4-dihydro-benzo[d][1,3]oxazin-2-on,
1-[1-(5-Dimethylamino-naphthyl-1-sulfonyl)-piperidin-4-yl]-8-methyl-1 ,4-dihydro-benzo[d][1,3]oxazin-2-on,
1-[1-(5-Dimethylamino-naphthyl-1-sulfonyl)-piperidin-4-yl]-1 ,4-dihydro-benzo[d][1,3]oxazin-2-on,
und deren entsprechende Salze und deren entsprechende Solvate.

15. Verfahren zur Herstellung Benzoxazinon-abgeleiteter Sulfonamidverbindungen der allgemeinen Formel (I) nach einem der Ansprüche 1 bis 14, **dadurch gekennzeichnet, dass** man bei diesem Verfahren wenigstens eine Piperidinverbindung der allgemeinen Formel (II), worin R¹ bis R⁹ die in Anspruch 1 angegebene Bedeutung haben, und/oder ein Salz davon, vorzugsweise ein Hydrochloridsalz, 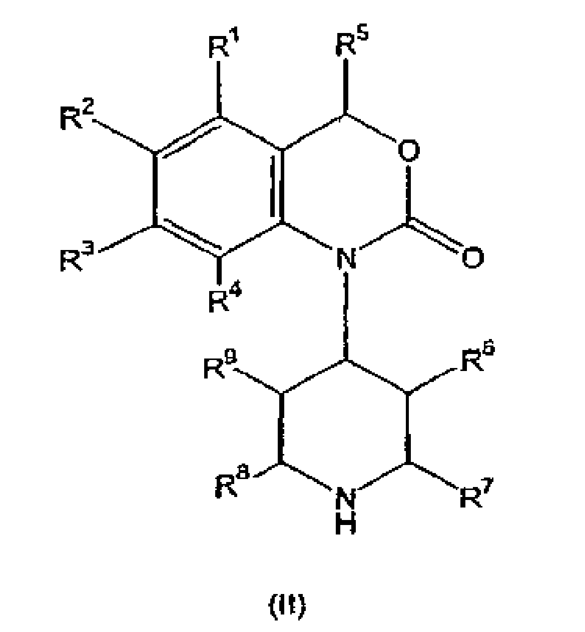 mit wenigstens einer Verbindung der allgemeinen Formel (III) 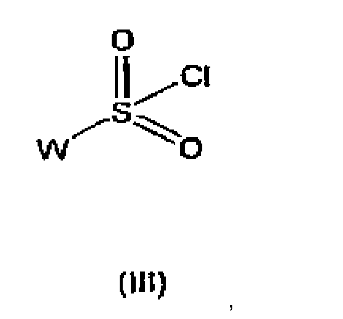 wobei W die in Anspruch 1 angegebene Bedeutung hat,
in einem geeigneten Reaktionsmedium, gegebenenfalls in Gegenwart wenigstens einer Base und/oder wenigstens eines Hilfsmittels umsetzt.

16. Verfahren zur Herstellung eines physiologisch akzeptablen Salzes der Benzoxazinon-abgeleiteten Sulfonamidverbindungen nach den Ansprüchen 1 bis14, **dadurch gekennzeichnet, dass** wenigstens eine Verbindung der allgemeinen Formel (I), die wenigstens eine basische Gruppe aufweist, mit wenigstens einer Säure, vorzugsweise einer anorganischen oder organischen Säure umgesetzt wird, vorzugsweise in Gegenwart eines geeigneten Reaktionsmediums.

17. Verfahren zur Herstellung eines physiologisch akzeptablen Salzes der Benzoxazinon-abgeleiteten Sulfonamidverbindungen nach den Ansprüchen 1 bis 14, **dadurch gekennzeichnet, dass** wenigstens eine Verbindung der allgemeinen Formel (I), die wenigstens eine saure Gruppe aufweist, mit wenigstens einer Base umgesetzt wird, vorzugsweise in Gegenwart eines geeigneten Reaktionsmediums.

18. Medikament, umfassend wenigstens eine Benzoxazinon-abgeleitete Sulfonamidverbindung nach einem der Ansprüche 1 bis 14, gegebenenfalls in Form eines ihrer Stereoisomere, vorzugsweise Enantiomere oder Diastereomere, ihres Racemats oder in Form eines Gemisches von wenigstens zwei ihrer Stereoisomere in einem beliebigen Mischungsverhältnis, oder ein physiologisch akzeptables Salz davon, beziehungsweise ein Solvat, und gegebenenfalls ein oder mehrere pharmazeutisch akzeptable Adjuvantien.

19. Medikament nach Anspruch 18 zur kognitiven Leistungssteigerung, zur Vorbeugung und/oder Behandlung von Störungen der Nahrungsaufnahme (Nahrungseinnahme), insbesondere zur Appetitregulierung, zur Aufrechterhaltung, Steigerung oder Verringerung des Körpergewichts, zur Vorbeugung und/oder Behandlung von Adipositas, Bulimie, Anorexie, Kachexie oder Typ-II-Diabetes (nicht-insulinabhängiger Diabetes mellitus), vorzugsweise Typ-II-Diabetes, hervorgerufen durch Adipositas, Störungen des Zentralnervensystems, Störungen des Gastrointestinaltrakts, wie beispielsweise Reizdarmsyndrom, Angst, Panik, Depression, kognitiven Gedächtnisstörungen, senilen Demenzstörungen, wie beispielsweise Morbus Alzheimer, Morbus Parkinson und Morbus Huntington, Schizophrenie, Psychose, infantiler Hyperkinese oder ADHC (Aufmerksamkeitsdefizit, Hyperaktivitätsstörungen).

20. Verwendung wenigstens einer Benzoxazinon-abgeleiteten Sulfonamidverbindung nach einem der Ansprüche 1 bis 14, gegebenenfalls in Form einer ihrer Stereoisomere, vorzugsweise Enantiomere oder Diastereomere, ihres Racemats oder in Form eines Gemisches von wenigstens zwei ihrer Stereoisomere in einem beliebigen Mischungsverhältnis, oder eines physiologisch akzeptablen Salzes davon, oder eines Solvats, zur Herstellung eines Medikaments zur kognitiven Leistungssteigerung, zur Vorbeugung und/oder Behandlung von Störungen der Nahrungsaufnahme (Nahrungseinnahme), insbesondere zur Appetitregulierung, zur Aufrechterhaltung, Steigerung oder Verringerung des Körpergewichts, zur Vorbeugung und/oder Behandlung von Adipositas, Bulimie, Anorexie, Kachexie oder Typ-II-Diabetes (nicht-insulinabhängigem Diabetes mellitus), vorzugsweise Typ-II-Diabetes, hervorgerufen durch Adipositas, Störungen des Zentralnervensystems, Störungen des Gastrointestinaltrakts, wie beispielsweise Reizdarmsyndrom, Angst, Panik, Depression, kognitiven Gedächtnisstörungen, senilen Demenzstörungen, wie beispielsweise Morbus Alzheimer, Morbus Parkinson und Morbus Huntington, Schizophrenie, Psychose, infantiler Hyperkinese oder ADHC (Aufmerksamkeitsdefizit, Hyperaktivitätsstörungen).

21. Verwendung wenigstens einer Benzoxazinon-abgeleiteten Sulfonamidverbindung der allgemeinen Formel (la), 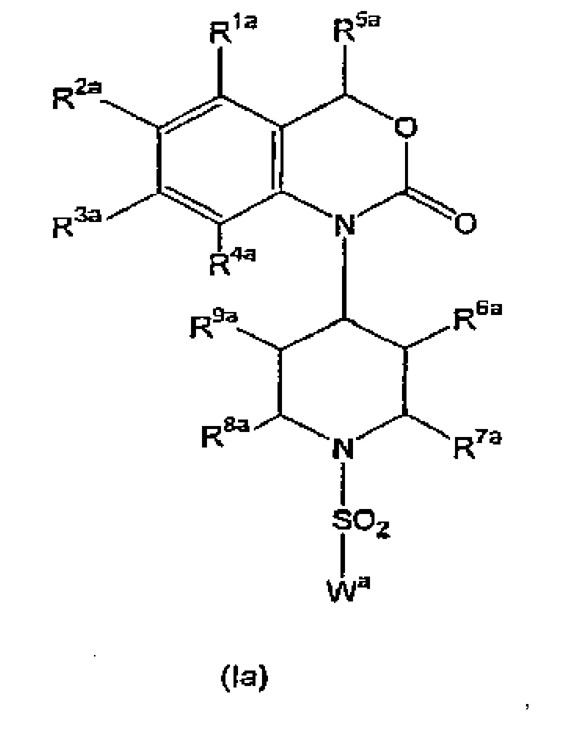 worin
R^{1a}, R^{2a}, R^{3a} , R^{4a} jeweils unabhängig voneinander ausgewählt sind unter Wasserstoff, Halogen, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, welches gebunden sein kann über eine gegebenenfalls wenigstens monosubstituierte Alkylengrupe und/oder kondensiert sein kann mit einem gegebenenfalls wenigstens monosubstituierten mono- oder polycyclischen Ringsystem, einem gegebenenfalls wenigstens monosubstituierten Aryl- oder Heteroarylradikal, das gebunden sein kann über eine gegebenenfalls wenigstens monosubstitierte Alkylengruppe und/oder kondensiert sein kann mit einem gegebenenfalls wenigstens monosubstituierten mono- oder polycyclischen Ringsystem, einer Nitrogruppe, einer Cyanogruppe, -OR^{10a}, OC(=O)R^{11a}, -(C=O)-OR^{11a}, -SR^{12a}, -SOR^{12a}, SO₂R¹²a, -NH-SO₂R^{12a}, -SO₂NH₂ und einem -NR^{13a}R^{14a}-Rest,
R^{5a} für Wasserstoff, ein unverzweigtes oder verzweigtes, gesättigtes oder ungesättigtes, gegebenenfalls wenigstens monosubstituiertes aliphatisches Radikal oder gesättigtes oder ungesättigtes, gegebenenfalls wenigstens monosubstituiertes, gegebenenfalls wenigstens ein Heteroatom als Ringbestandteil enthaltendes cycloaliphatisches Radikal steht,
R^{6a} , R^{7a} , R^{8a} , R^{9a} jeweils unabhängig voneinander ausgewählt sind unter Wasserstoff, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, einer Cyanogruppe und einem -COOR^{15a}-Rest,
W^{a} steht für ein unverzweigtes oder verzweigtes, gesättigtes oder ungesättigtes, gegebenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das gebunden sein kann über eine gegebenenfalls wenigstens monosubstituierte Alkylengruppe und/oder kondensiert sein kann mit einem gegebenenfalls wenigstens monosubstituierten mono- oder polyzyklischen Ringsystem, ein gegebenenfalls wenigstens monosubstituiertes Aryl- oder Heteroarylradikal, das gebunden sein kann über eine gegebenenfalls wenigstens monosubstituierte Alkylengruppe und/oder kondensiert sein kann mit einem gegebenenfalls wenigstens monosubstituierten mono- oder polyzyklischen Ringsystem, einen NR^{16a}R^{17a}-Rest oder einen COR^{18a}-Rest,
R^{10a} für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{11a} für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{12a} für ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{13a} und R^{14a} unabhängig voneinander ausgewählt sind unter Wasserstoff, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder einem gegebenenfalls wenigstens monosubstituierten Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann,
oder R^{13a} und R^{14a} zusammen mit dem überbrückenden Stickstoffatom einen gesättigten, ungesättigten oder aromatischen heterozyklischen Ring bilden, der wenigstens monosubstituiert sein kann und/oder wenigstens ein weiteres Heteroatom als Ringbestandteil enthalten kann,
R^{15a} für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{16a} für ein unverzweigtes oder verzweigtes, gesättigtes oder ungesättigtes, gegebenenfalls wenigstens monosubstituiertes aliphatisches Radikal steht,
R^{17a} für ein unverzweigtes oder verzweigtes, gesättigtes oder ungesättigtes, gegebenenfalls wenigstens monosubstituiertes aliphatisches Radikal steht,
R^{18a} für ein gegebenenfalls wenigstens monosubstituiertes Arylradikal steht,
gegebenenfalls in Form eines ihrer Stereoisomere, vorzugsweise Enantiomere oder Diastereomere, ihres Racemats oder in Form eines Gemisches von wenigstens zwei ihrer Stereoisomere, vorzugsweise Enantiomere oder Diastereomere, in einem beliebigen Mischungsverhältnis, oder eines ensprechenden Salzes davon beziehungsweise eines entsprechenden Solvats,
zur Herstellung eines Medikaments zur Vorbeugung und/oder Behandlung von Störungen der Nahrungsaufnahme; Angst, Panik, Depression, kognitiven Störungen, vorzugweise Gedächtnisstörungen; senilen Demenzprozessen, vorzugsweise ausgewählt unter Morbus Alzheimer, Morbus Parkinson, Morbus Huntington; Psychose; infantiler Hyperkinese; ADHC (Aufmerksamkeitsdefizit/Hyperaktivitätsstörung); Störungen des Gastrointestinaltrakts, vorzugsweise Darmsyndrom; Schizophrenie oder zur kognitiven Leistungssteigerung.

22. Verwendung nach Anspruch 21, **dadurch gekennzeichnet, dass** R^{1a}, R^{2a}, R^{3a}, R^{4a} jeweils unabhängig voneinander ausgewählt sind unter H, F, Cl, Br, einem unverzweigten oder verzweigten, gesättigten oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteoratom als Ringbestandteil enthaltenden C₃₋₈-cycloaliphatischen Radikal, das über eine gegebenenfalls wenigstens monosubstituierte C₁₋₆-Alkylengruppe gebunden sein kann und/oder mit einem gegebenenfalls wenigstens monosubstituierten mono- oder polyzyklischen Ringsystem kondensiert sein kann, einem gegebenenfalls wenigstens monosubstituierten 5- oder 6-gliedrigen Aryl- oder Heteroarylradikal, das über eine gegebenenfalls wenigstens monosubstituierte C₁₋₆-Alkylengruppe gebunden sein kann und/oder mit einem gegebenenfalls wenigstens monosubstituierten mono- oder polyzyklischen Ringsystem kondensiert sein kann, einer Nitrogruppe, einer Cyanogruppe, -OR^{10a}, - OC(=O)R^{11a}, -SR^{12a}, -SOR^{12a}, SO₂R^{12a}, -NH-SO₂R^{12a}, -SO₂NH₂ und einem - NR^{13a}R^{14a}-Rest, vorzugsweise ausgewählt sind unter H, F, Cl, Br, einem gesättigten, verzweigten oder unverzweigten, gegebenenfalls wenigstens monosubstituierten C₁₋₃-cycloaliphatischen Radikal, einem gesättigten, gegebenenfalls wenigstens monosubstituierten, gegebenenfalls wenigstens ein Heteroatom als Ringbestandteil enthaltenden C₅- oder C₆-cycloaliphatischen Radikal, das über eine gegebenenfalls wenigstens monosubstituierte C₁₋ oder C₂-Alkylengruppe, eine Nitrogruppe, eine Cyanogruppe, -OR^{10a}, OC(=O)R^{11a}, - SR¹²a, und einen -NR^{13a}R^{14a} -Rest gebunden sein kann, besonders bevorzugt ausgewählt sind unter H, F, Cl, -CH₃, -CH₂CH₃, -CF₃, -CF₂CF₃, Cyclopentyl, Cyclohexyl, Nitro, Cyano und -OR^{10a}.

23. Verwendung nach Anspruch 21 oder Anspruch 22, **dadurch gekennzeichnet, dass** R^{5a} für Wasserstoff, ein unverzweigtes oder verzweigtes, gesättigtes oder ungesättigtes, gegebenenfalls wenigstens monosubstituiertes C₁₋₆-aliphatisches Radikal oder ein gesättigtes oder ungesättitgtes, gegebenenfalls wenigstens monosubstituiertes, gegebenenfalls wenigstens ein Heteroatom als Ringbestandteil enthaltendes C₃₋₈-cycloaliphatisches Radikal, vorzugsweise für H oder ein verzweigtes oder unverzweigtes C₁₋₃-Alkylradikal , besonders bevorzugt für H, -CH₃ oder-CH₂₋CH₃ steht.

24. Verwendung nach einem der Ansprüche 21 bis 23, **dadurch gekennzeichnet, dass** R^{6a} , R^{7a} , R^{8a}, R^{9a} unabhängig voneinander ausgewählt sind unter Wasserstoff, einem unverzweigten oder verzweigten, gesättigten oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteroatom als Ringbestandteil enthaltenden C₃₋₈-cycloaliphatischen Radikal, einer Cyanogruppe und einem -COOR^{15a} -Rest, vorzugsweise ausgewählt sind unter H, einem verzweigten oder unverzweigten C₁₋₃-Alkylradikal, einer Cyanogruppe und einer -COOR^{15a}-Gruppe, besonders bevorzugt ausgewählt sind unter H, - CH₃, -CH₂CH₃ und einem Cyano-Rest.

25. Verwendung nach einem der Ansprüche 21 bis 24, **dadurch gekennzeichnet, dass** W^{a} steht für ein unverzweigtes oder verzweigtes, gesättigtes oder ungesättigtes, gegebenenfalls wenigstens monosubstituiertes C₁₋₂₀-aliphatisches Radikal, ein gesättigtes oder ungesättigtes, gegebenenfalls wenigstens monosubstituiertes, gegebenenfalls wenigstens ein Heteroatom als Ringbestandteil enthaltendes C₃₋₈-cycloaliphatisches Radikal, das gebunden sein kann über eine gegebenenfalls wenigstens monosubstituierte C₁₋₆-Alkylengrupe und/oder kondensiert sein kann mit einem gegebenenfalls wenigstens monosubstituierten mono- oder polyzyklischen Ringsystem, ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, einen NR¹⁶aR¹⁷-Rest oder einen COR¹⁸a-Rest,
vorzugsweise ausgewählt ist unter 1-Naphthyl-, 5-Dimethylamino-napth-1-yl, 2-Naphthyl-, 2-Acetamido-4-methyl-5-thiazolyl-, 2-Thienyl-, 8-Chinolinyl-, Phenyl-, Pentafluorphenyl-, 2,4,5-Trichlor-phenyl-, 2,5-Dichlor-phenyl-, 2-Nitrophenyl-, 2,4-Dinitro-phenyl-, 3,5-Dichlor-2-hydroxy-phenyl-, 2,4,6-Trisisopropyl-phenyl-, 2-Mesityl-, 3-Nitro-phenyl-, 4-Brom-phenyl-, 4-Fluor-phenyl-, 4-Chlor-phenyl-, 4-Chlor-3-nitro-phenyl-, 4-Iod-phenyl-, N-Acetyl-sulfanilyl-, 4-Nitro-phenyl-, 4-Methoxy-phenyl-, Benzoesäure-4-yl-, 4-tert-Butyl-phenyl-, p-Tolyl-, Trifluormethyl-, Trichlormethyl-, Isopropyl-, Methyl-, Benzyl-, trans-Styryl-, 2,2,2-Trifluorethyl- , Ethyl-, Hexadecyl-, 2-Chlorethyl-, n-Propyl-, 3-Chlor-propyl-, n-Butyl-, Methyl-benzoat-2-yl-, 2-Nitro-4-(trifluormethyl)-phenyl-, Pentamethyl-phenyl- , 2,3,5,6-Tetramethyl-phenyl-, 3-(Trifluormethyl)-phenyl-, 3,5-Bis-(trifluormethyl)-phenyl-, Dichlormethyl-, Chlormethyl-, Dodecyl-, 1-Octyl-, 2,3,4-Trichlor-phenyl-, 2,5-Dimethoxy-phenyl-, o-Tolyl-, p-Xylyl-2-yl-, Benzoesäure-3-yl-, 4-Chlor-3- (trifluormethyl)-phenyl-, 4-Chlor-5-nitro-benzoesäure- 3-yl-, 6-(p-Toluidino)-naphth-2-yl-, 4-Methoxy-2,3,6-trimethylphenyl-, 3,4-Dichlorphenyl-, 4,5-Dibrom-thiophen-2-yl-, 3-Chlor-4-fluor-phenyl-, 4-Ethyl-phenyl-, 4-n-Propyl-phenyl-, 4-(1,1-Dimethylpropyl)-phenyl-, 4-Isopropyl-phenyl-, 4-Brom-2,5-difluorphenyl-, 2-Fluor-phenyl-, 3-Fluor-phenyl-, 4- (Trifluormethoxy)-phenyl-, 4-(Trifluormethyl)-phenyl-, 2,4-Difluor-phenyl-, 2,4-Dichlor-5-methyl-phenyl-, 4-Chlor-2,5-dimethyl-phenyl-, 5-Diethylamino-napth-2-yl-, Benzoylchlorid-3-yl-, 2-Chlor-phenyl-, 1-Octadecyl-, 4-Brom-2,5-dichlor-thiophen-3-yl-, 2,5-Dichlorthiophen-3-yl-, 5-Chlor-thiophen- 2-yl-, 2-Methyl-5-nitro-phenyl-, 2-(Trifluormethyl)-phenyl-, 3-Chlor-phenyl-, 3,5-Dichlor-phenyl-, 1-Decyl-, 3-Methyl-phenyl-, 2-Chlor-6-methyl-, 5-Brom-2-methoxy-phenyl-, 3,4-Dimethoxy-phenyl-, 2-3-Dichlor-phenyl-, 2-Brom-phenyl-, 3,5-Dichlor-4-(2-chlor-4-nitrophenoxy)-phenyl-, 2,3-Dichlor-thiophen-5-yl-, 3-Brom-2-chlor-thiophen-5-yl-, 3-Brom-5-chlor-thiophen-2-yl-, 2-(Benzoylaminomethyl)-thiophen-5-yl-, 4-(Phenyl-sulphonyl)-thiophen-2-yl-, 2-Phenyl-sulphonyl-thiophen-5-yl-, 3-Chlor-2-methyl-phenyl-, 2-[1-Methyl-5-(trifluormethyl)pyrazol-3-yl]-thiophen-5-yl-, 5-Pyrid-2-yl-thiophen-2-yl-, 2-Chlor-5-(trifluormethyl)-phenyl-, 2,6-Dichlor-phenyl-, 3-Brom-phenyl-, 2-(Trifluormethoxy)-phenyl-, 4-Cyano-phenyl-, 2- Cyano-phenyl-, 4-n-Butoxy-phenyl-, 4-Acetamido-3-chlor-phenyl, 2,5-Dibrom-3,6-difluor-phenyl-, 5-Chlor-1,3-dimethylpyrazol-4-yl-, 3,5-Dimethylisoxazol-4-yl-, 2-(2,4-Dichlorphenoxy)-phenyl-, 4-(2-Chlor-6-nitro-phenoxy)-phenyl-, 4-(3-Chlor-2-cyano-phenoxy)-phenyl-, 2,4-Dichlor-phenyl-, 2,4-Dimethyl-1,3-thiazol-5-yl-, Methyl-methan-sulfonyl-, 2,5-Bis-(2,2,2-trifluorethoxy)-phenyl-, 2-Chlor-4-(trifluormethyl)-phenyl-, 2-Chlor-4-fluor-phenyl-, 5-Fluor-2-methyl-phenyl-, 5-Chlor-2-methoxy-phenyl-, 2,4,6-Trichlor-phenyl-, 2-Hydroxy-benzoesäure-5-yl-, 5-(Di-n-propylamino)-naphth-1-yl-, 6-Methoxy-m-tolyl-, 2,5-Difluor-phenyl-, 2,4-Dimethoxy-phenyl-, 2,5-Dibrom-phenyl-, 3,4-Dibrom-phenyl-, 2,2,5,7,8-Pentamethyl-chroman-6-yl-, 2-Methoxy-benzoesäure-5-yl-, 5-Chlor-4-nitrothiophen-2-yl-, 2,1,3- Benzothiadiazol-4-yl-, 1-Methyl-imidazol-4-yl-, Benzofurazan-4-yl-, 2-(Methoxycarbonyl)-thiophen-3-yl-, 5-(Isoxazol-3-yl)-thiophen-2-yl-, 2,4,5-Trifluor-phenyl-, Biphenyl-4-yl-, Vinyl-phenyl-4-yl-, 2-Nitro-benzyl-, 5-Dichlor- methyl-furan-2-yl-, 5-Brom-thiophen-2-yl-, 5-(4-Chlorbenzamidomethyl)-thiophen-2-yl-, 2, 6-Difluor-phenyl-, 2,5-Dimethoxy-4-nitro-phenyl-, Dibenzo[b,d]-furan-2-yl-, 2,3,4-Trifluor-phenyl-, 3-Nitro-p-tolyl-, 4-Methoxy-2-nitro-phenyl-, 3,4-Difluor-phenyl-, 4-(Bromethyl)-phenyl-, 3,5-Dichlor-4-hydroxy-phenyl-, 4-n-Amyl-phenyl-, 5-Chlor-3-methylbenzo[b]-thiophen-2-yl- , 3-Methoxy-4-(methoxycarbonyl)-thiophen-2-yl-, 4-n-Butyl-phenyl-, 2-Chlor-4-cyano-phenyl-, 5-[2-(Methylthio)-pyrimidin-4-yl-]-thiophen-2-yl-, 3,5-Dinitro-4-methoxy-phenyl-, 4-Brom-2-(trifluormethoxy)-phenyl-, 4-Chlor-2,1,3-Benzoxadiazol-7-yl-, 2-(1-Naphthyl)-ethyl-, 3-Cyano-phenyl-, 5-Chlor-2,1,3-Benzoxadiazol-4-yl-, 3-Chlor-4-methyl-phenyl-, 4-Brom-2-ethyl-phenyl-, 2,4-Dichlor-6-methyl-phenyl-, 6-Chlor-imidazo(2,1-B)-thiazol-5-yl-, 3- Methylbenzo[b]-thiophen-2-yl-, 4-Methyl-sulphonyl-phenyl-, 2-Methyl-sulphonyl-phenyl-, 4-Brom-2-methyl-phenyl-, 2,6-Dichlor-4- (trifluormethyl)-phenyl-, 4-[[3-Chlor-5-(trifluormethyl)-2-pyridinyl]oxy]-phenyl-, 5-Chlor naphth-1-yl-, 5-Chlor-naphth-2-yl-, 9,10-Dibromanthracen-2-yl-, Isochinolin-5-yl-, 4-Methoxy-2,3,6-trimethyl-phenyl-, 4'-Nitro-biphenyl-4-yl-, (4-Phenoxy)-phenyl-, (1,3-Dihydro-1-oxo-2H-isoindol-2-yl-)-4-phenyl-, 4-Acetyl-phenyl-, 5-(2-Methyl-1,3-thiazol-4-yl)-thiophen-2-yl-, 5-(1-Methyl-3-(trifluormethyl)pyrazol-5-yl-]-thiophen-2-yl-, 5-[5-Trifluormethyl)-isoxazol-3-yl]-thiophen-2-yl-, 2-Iod-phenyl-, p-Dodecyl-phenyl-, 4-[(3-Cyano-4-methoxy-2-pyridinyl)oxy]-phenyl-, 4-(N-phthalimidinyl)-phenyl-, 1,2,3,4-Tetrahydro-2-(trifluoracetyl-isochinolin-7-yl-, 4-Brom-2-fluor-phenyl-, 2-Fluor-5- (trifluormethyl)-phenyl-, 4-Fluor-2-(trifluormethyl)-phenyl-, 4-Fluor-3-(trifluormethyl)-phenyl-, 2,4,6-Trifluor-phenyl-, 3-(Trifluormethoxy)-phenyl-, 1,2-Dimethylimidazol-4-yl-, Ethyl-4-Carboxylat-3-yl-, 2,2,4,6,7-Pentamethyldihydrobenzofuran-5-yl-, 3-Brom-2- chlorpyridin-5-yl-, 3-Methoxy-phenyl-, 2-Methoxy-4-methyl-phenyl-, 2-Chlor-4-fluorbenzosäure-5-yl-, 4-Chlor-naphth-1-yl-, 2,5-Dichlor-4-nitro-thiophen-3-yl-, 4-(4-Methoxy-phenoxy)-phenyl-, 4-(4-Chlor-phenoxy)-phenyl-, 4-(3,5-Dichlor-phenoxy)-phenyl-, 4-(3,4-Dichlorphenoxy)-phenyl-, 4-(4-Fluor-phenoxy)-phenyl-, 4-(4-Methyl-phenoxy)-phenyl-, 4-[4-(Trifluormethyl)-phenoxy-phenyl-, 4-[3,5-Bis-(trifluormethyl)-phenoxy]-phenyl-, 3-(2-Methoxy-phenoxy)-phenyl-, [3-(2-Chlor-phenoxy)-phenyl-, 3-(2-Methyl-phenoxy)-phenyl-, 4-[2-(Trifluormethyl)-phenoxy]-phenyl-, 3-Phenyl-phenyl-, 3- (4-Methoxy-phenyl)-phenyl-, 3-(4-Chlor-phenyl)-phenyl-, 3-(3,5-Dichlor-phenyl)-phenyl-, 3-(3,4-Dichlor-phenyl)-phenyl-, 3-(4-Fluorphenyl)-phenyl-, 3-(4-Methylphenyl)-phenyl-, 3- [4-(Trifluormethyl)-phenyl]-phenyl-, 3-[3,5-Bis-(Trifluormethyl)-phenyl]-phenyl-, 4-(4-Pyridyloxy)-phenyl)-, 4-(2-Methoxy-phenoxy)-phenyl-, 4-(2-Chlor-phenoxy)-phenyl-, 4- (2-Methylphenoxy)-phenyl-, 4-(4-Methoxy-phenoxy)-phenyl-, 4-(4-Chlorphenyl)-phenyl-, 4-(3,5-Dichlorphenyl)-phenyl-, 4-(3,4-Dichlorphenyl)-phenyl-, 4-(4-Fluorphenyl)-phenyl-, 4-(4-Methylphenyl)-phenyl-, 4-[4-(Trifluormethyl)-phenyl]-phenyl-, 4-[3,5-Bis- (Trifluormethyl)-phenyl]-phenyl-, [3-(Trifluormethyl)-phenyl]-methyl-, (4-Chlorphenyl)-methyl-, (3,5-Dichlorphenyl)-methyl-, (3,5-Dichlorphenyl)-methyl-, (4-Fluorphenyl)-methyl-, 4-Methylphenylmethyl-, [4-(Trifluormethyl)-phenyl]-methyl-, Cyclopropyl-, 2-(2-Chlorphenyl)-2-Phenylethyl-, 2-(2-Trifluormethylphenyl)-2-phenylethyl-, 5-[4-Cyano-1 -methyl-5-(methylthio)-1 H-pyrazol-3-yl-thiophen-2-yl-, 3-Cyano-2,4-bis-(2,2,2-Trifluorethoxy)-phenyl-, 4-[(2-Chlor-1,3-Thiazol-5-yl)-methoxy]-phenyl-, 3-Nitro-phenylmethyl-, 4-Formylphenyl-, 2-(1,3-Dioxo-1,3-dihydro-isoindol-2-yl)-ethyl-, [3,5-Bis-(trifluormethyl)-phenyl]-methyl-, (4-(2-Pyridyloxy)-phenyl)-, (4-(3-Pyridyloxy)-phenyl)-, 5-lod-naphth-1-yl-, Ethyl-2,5-dimethyl-1-phenylpyrrol-4-carboxylat-3-yl-, Ethyl-2-methyl-1,5-diphenyl-1 H-pyrrol-3-carboxylat-4-yl-, Ethyl-5-(4-chlorphenyl)-2-methyl-3-furoat-4-yl, Ethyl-5-(4-chlorphenyl)-2-methyl-1-phenyl-3-carboxylat-4-yl-, Ethyl-2,5-dimethyl-3-furoat-4-yl-, 3-Chlor-4-(1,3-dioxo-2-azaspiro[4,4]non-2-yl)-phenyl-, 5-Brom-2,4-difluor-phenyl-, 5-Chlor-2,4-difluorphenyl-, Coumarin-6-yl, 2-Methoxy-phenyl, (3-Phenoxy)-phenyl-, 3-(4-Methoxy-phenoxy)-phenyl-, 3-(4-Chlorphenoxy)-phenyl-, 3-(3,5-Dichlorphenoxy)-phenyl-, 3-(3,4-Dichlorphenoxy)-phenyl-, 3-(4-Fluorphenoxy)-phenyl-, 3-(4-Methylphenoxy)-phenyl-, 3-[4-(Trifluormethyl)-phenoxy]-phenyl-, 3-[3,5-(Trifluormethyl)-phenoxy]-phenyl-, 3-[2-(Trifluormethyl)-phenoxy]-phenyl-, 2,2-Diphenylethyl-, 4-Phenyl-5-(trifluormethyl)-thiophen-3-yl-, Methyl-4-phenyl-5-(trifluormethyl)-thiophen-2-carboxylat-3-yl-, Methyl-1, 2,5-trimethylpyrrol-3-carboxylat-4-yl-, 4-Fluor-naphth-1-yl-, 3,5-Difluorphenyl-, 3-Fluor-4-methoxy-phenyl-, 4-Chlor-2,5-difluorphenyl-, 2-Chlor-4,5-difluor-phenyl-, 5-Fluor-3-methylbenzo[b]-thiophen-2-yl-, Methyl-3-phenylpropionate-4-yl, Dihydrozimtsäure-4-yl-, Methyl-2,5-dimethyl-3-furoat-4-yl-, Methyl-2-furoat-5-yl-, Methyl-2-methyl-3-furoat-5-yl-, Methyl-1-methyl-1 H-pyrrol-2-carboxylat-5-yl-, 2-(5-Chlor-1,2,4-thiadiazol-3-yl)-thiophen-5-yl-, 1,3,5-Trimethyl-1 H-pyrazol-4-yl-, 3-Chlor-5-fluor-2-methylphenyl-, Pentafluorethoxytetrafluorethyl-, 5-(5-Isoxazyl)-thiophen-2-yl-, 5-(5-lsoxazol-yl)-2-furyl-, 5-Methyl-2,1,3-benzothiadiazol-4-yl-, Biphenyl-2-yl-, 2,3-Dihydro-1,4-benzodioxine-6-yl-, 4-Methyl-naphth-1-yl-, 5-Methyl-2-(trifluormethyl)-3-furyl-, 2,3-Dihydrobenzo[b]furan-5-yl-, 1-Benzothiophen-3-yl-, 4-Methyl-3,4-dihydro-2H-1,4-benzoxazin-7-yl-, 5-Methyl-1-phenyl-1 H-pyrazol-4-yl-, 6-Morpholino-3-pyridinyl-, 4-(1 H-Pyrazol-1-yl)-phenyl-, 6- Phenoxy-3-pyridyl-, 3,4-Dihydro-2H-1,5-benzodioxepin-7-yl-, 5-(1,3-Oxazol-5-yl)-2-thienyl-, 4-(1,3-Oxazol-5-yl)-phenyl-, 5-Methyl-4-isoxazolyl, 2,1,3-Benzothiadiazol-5-yl-, 3-Thienyl-, 2-Methyl-benzyl-, 3-Chlor-benzyl-, 5-Acetamido-naphth-1-yl-, 3-Methyl-8-chinolinyl-, 4-Chlor-2-nitrophenyl-, 6-Chinolinyl-, 1,3-Benzothiazol-6-yl-, 2-Morpholino-3-pyridyl-, 2,5-Dimethyl-3-thienyl-, 5-[5-(Chlormethyl)-1,2,4-oxadiazol-3-yl]-2-thienyl-, Ethyl-3-[5-yl-2-thienyl]1,2,4-oxadiazol-5-carboxylat-, 3-(5-Methyl-1,3,4-oxadiazol-2-yl)- phenyl-, 4-lsopropoxyphenyl-, 2,4-Dibromphenyl-, 3-Cyano-4-fluorphenyl-, 2,5-Bis-(trifluormethyl)-phenyl, 2-Brom-4-fluorphenyl-, 4-Brom-3-fluorphenyl-, 4-(Difluormethoxy)-phenyl-, 3-(Difluormethoxy)-phenyl-, 5-Chlor-2-fluor-phenyl-, 3-Chlor-2-fluorphenyl-, 2-Fluor-4-methylphenyl-, 4-Nitro-3-(trifluormethyl)-phenyl-, 3-Fluor-4-methylphenyl-, 4-Fluor-2-methylphenyl-, 4-Brom-3-(trifluormethyl)-phenyl-, 4-Brom-2- (trifluormethyl)-phenyl-, 3-Brom-5-(trifluormethyl)-phenyl-, 2-Brom-4-(trifluormethyl)-phenyl-, 2-Brom-5-(trifluormethyl)-phenyl-, 2,4-Dichlor-5-fluorphenyl-, 4,5-Dichlor-2-fluorphenyl-, 3,4,5-Trifluorphenyl-, 4-Chlor-2-fluorphenyl-, 2-Brom-4,6-difluorphenyl-, 2-Ethylphenyl-, 4-Brom-2-chlorphenyl-, 4-Brom-2,6-dichlorphenyl-, 2-Brom-4,6-dichlor-phenyl-, 4-Brom-2,6-dimethylphenyl-, 3,5-Dimethylphenyl-, 4-Brom-3-methylphenyl-, 2- Methoxy-4-nitrophenyl-, 2,2-Dimethyl-6-chromanyl-, Ethyl-3,5-dimethyl-1 H-pyrrol-2-carboxylat-4-yl-, Imidazo[1,2-A]pyridin-3-yl-, 3-(1,3-Oxazol-5-yl)-phenyl-, Ethyl-5-[4-yl)-phenyl]-2-methyl-3-furoat, Methyl-3-(yl)-4-methoxybenzoat, 1-Pyrrolidinylphenylsulfonyl-, Methyl-5-yl-4-methyl-2-thiophen-carboxylat, Methyl-3-yl-4-(isopropylsulfonyl)-2-thiophen, 2-Pyridyl-, 3-Fluor-4-nitrophenyl-, 7-Chlorchromon-3-yl-, 4'-Brombiphenyl-4-yl-, 4'-Acetyl-biphenyl-4-yl-, 4'-Brom-2'-fluor-biphenyl-4-yl-, 2-Chlor-4-(3-propyl-ureido)-phenyl-, 3-(Bromacetyl)-phenyl-, 2-Brom-3-(trifluormethyl)-phenyl-, 1-Methyl-5-isatinyl-, 4-Isopropylbenzoesäure-3-yl-, 2-Chlor-3-thiophencarbonsäure-5-yl-, 3-Pyridyl-, Cyclohexylmethyl-, 2-Methoxy-5-(N-phthalimidinyl)-phenyl-, 1-Benzothiophen-2-yl-, Morpholinophenylsulfonyl-, 3-(2-Methyl-4-pyrimidinyl)-phenyl-, und 2-Cyano-5-methylphenyl-.

26. Verwendung nach einem der Ansprüche 21 bis 25, **dadurch gekennzeichnet, dass** R^{10a} steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischem Ringsystem,
vorzugsweise für H, ein lineares oder verzweigtes C₁₋₄-Alkylradikal, ein Cyclohexylradikal oder ein Phenylradikal steht, besonders bevorzugt für H, - CH₃, -C₂H₅ oder Phenyl steht.

27. Verwendung nach einem der Ansprüche 21 bis 26, **dadurch gekennzeichnet, dass** R^{11a} steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischem Ringsystem,
vorzugsweise für H, ein lineares oder verzweigtes C₁₋₄-Alkylradikal, ein Cyclohexylradikal oder ein Phenylradikal steht, besonders bevorzugt für H, - CH₃, -C₂H₅ oder Phenyl steht.

28. Verwendung nach einem der Ansprüche 21 bis 27, **dadurch gekennzeichnet, dass** R^{12a} steht für ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cycloaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischem Ringsystem,
vorzugsweise steht für H, ein lineares oder verzweigtes C₁₋₄-Alkylradikal, ein Cyclohexylradikal oder ein Phenylradikal, besonders bevorzugt für H, -CH₃, - C₂H₅ oder Phenyl steht.

29. Verwendung nach einem der Ansprüche 21 bis 28, **dadurch gekennzeichnet, dass** R^{13a} und R^{14a} jeweils unabhängig voneinander ausgewählt sind unter Wasserstoff, einem unverzweigten oder verzweigten, gesättigten oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteroatom als Ringbestandteil enhaltenden C₃₋₈-cycloaliphatischen Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder einem gegebenenfalls wenigstens monosubstituierten, 5- oder 6-gliedrigen Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem,
vorzugsweise jeweis unabhängig voneinander ausgewählt sind unter H, einem linearen oder verzweigten C₁₋₄-Alkylradikal, einem Cyclohexylradikal oder einem Phenylradikal, besonders bevorzugt jeweils unabhängig voneinander ausgewählt sind unter H, -CH₃, -C₂H₅ und Phenyl.

30. Verwendung nach einem der Ansprüche 21 bis 29, **dadurch** gekennzeichent, dass R^{13a} und R^{14a} zusammen mit dem überbrückenden Stickstoffatom einen gesättigten, ungesättigten oder aromatischen, 5- oder 6-gliedrigen heterozyklischen Ring bilden, der wenigstens monosubstituiert sein kann und/oder wenigstens ein weiteres Heteroatom als Ringbestandteil enthalten kann, vorzugsweise eine unsubstituierte Piperidin- oder Morpholingruppe bilden.

31. Verwendung nach einem der Ansprüche 21 bis 30, **dadurch gekennzeichnet, dass** R^{15a} steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cycloaliphatisches Radikal oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, vorzugsweise für H, ein lineares oder verzweigtes C₁₋₄-Alkylradikal, ein Cyclohexylradikal oder ein Phenylradikal steht, besonders bevorzugt für H, -CH₃, -C₂H₅ oder Phenyl steht.

32. Verwendung nach einem der Ansprüche 21 bis 31, **dadurch gekennzeichnet, dass** R^{16a} für ein unverzweigtes oder verzweigtes, gesättigtes oder ungesättigtes, gegebenenfalls wenigstens monosubstituiertes C₁₋₆-aliphatisches Radikal, vorzugsweise ein unverzweigtes oder verzweigtes, gesättigtes, unsubstituiertes C₁₋₃-Alkylradikal, besonders bevorzugt für ein Methylradikal steht.

33. Verwendung nach einem der Ansprüche 21 bis 32, **dadurch gekennzeichnet, dass** R^{17a} für ein unverzweigtes oder verzweigtes, gesättigtes oder ungesättigtes, gegebenenfalls wenigstens monosubstituiertes C₁₋₆-aliphatisches Radikal, vorzugsweise für ein unverzweigtes oder verzweigtes, gesättigtes, unsubstituiertes C₁₋₃-Alkylradikal, besonders bevorzugt für ein Methylradikal steht.

34. Verwendung nach einem der Ansprüche 21 bis 33, **dadurch gekennzeichnet**, das ein oder mehrere Benzoxazinon-abgeleitete Sulfonamidverbindungen der allgemeinen Formel (la) ausgewählt sind unter:
1-[1-(Naphthyl-1-sulfonyl)-piperidin-4-yl]-1,4-dihydro-benzo[d][1,3]oxazin-2-on, 1-(1-Phenylsulfonyl-piperidin-4-yl]-1,4-dihydro-benzo[d][1,3]-oxazin-2-on,
1-[1-(5-Chlor-3-methyl-benzo[b]thiophenyl-2-sulfonyl)-piperidin-4-yl]-1,4-dihydro-benzo[d][1,3]oxazin-2-on,
8-Methyl-1-[1-naphthyl-1-sulfonyl)-piperidin-4-yl]-1,4-dihydro-benzo[d][1,3]oxazin-2-on,
1- [1-(Chinolin-8-sulfonyl)-piperidin-4-yl]-1,4-dihydro-benzo[d][1,3]oxazin-2-on,
8-Methyl-1-[1-(chinolin-8-sulfonyl)-piperidin-4-yl]-1,4-dihydro-benzo[d][1,3]oxazin-2-on,
1-[1-(5-Dimethylamino-naphthyl-1-sulfonyl)-piperidin-4-yl]-8-methyl-1,4-dihydrobenzo[d][1,3]oxazin-2-on,
1-[1-(5-Dimethylamino-naphthyl-1-sulfonyl)-piperidin-4-yl]-1,4-dihydro-benzo[d][1,3]oxazin-2-on,
1-[1-(2,3-Dichlor-phenylsulfonyl)-piperidin-4-yl]-1,4-dihydro-benzo[d][1,3]oxazin-2-on,
1-[1-(2,3-Dichlor-phenylsulfonyl)-piperidin-4-yl]-8-methyl-1,4-dihydro-benzo[d][1,3]oxazin-2-on, und
entsprechenden Salzen davon, oder entsprechenden Solvaten davon.

35. Verwendung nach einem der Ansprüche 21 bis 34 zur Appetitregulation.

36. Verwendung nach einem der Ansprüche 21 bis 34 zur Verringerung, Steigerung oder Aufrechterhaltung von Körpergewicht.

37. Verwendung nach einem der Ansprüche 21 bis 34 zur Vorbeugung und/oder Behandlung von Adipositas.

38. Verwendung nach einem der Ansprüche 21 bis 34 zur Vorbeugung und/oder Behandlung von Bulimie.

39. Verwendung nach einem der Ansprüche 21 bis 34 zur Vorbeugung und/oder Behandlung von Anorexie.

40. Verwendung nach einem der Ansprüche 21 bis 34 zur Vorbeugung und/oder Behandlung von Kachexie.

41. Verwendung nach einem der Ansprüche 21 bis 34 zur Vorbeugung und/oder Behandlung von Typ-II-Diabetes.

42. Benzoxazinone-abgeleitete Sulfonamidverbindung der allgemeinen Formel (Ib) 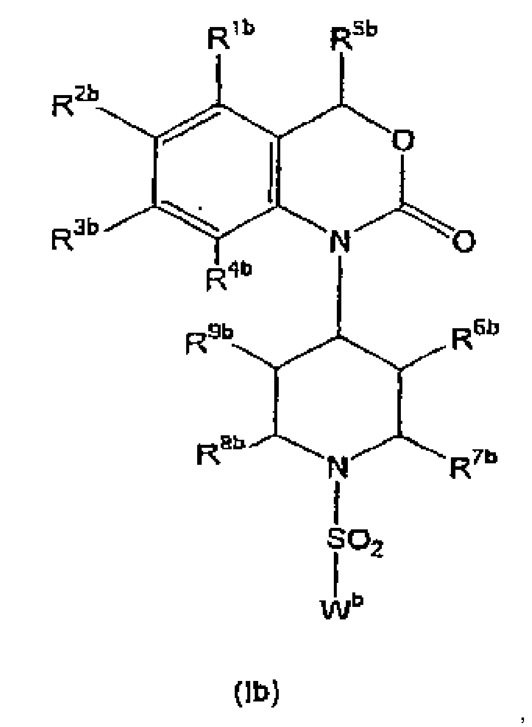 wobei
R^{1b}, R^{2b}, R^{3b}, R^{4b} jeweils unabhängig voneinander ausgewählt sind unter Wasserstoff, Halogen, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, das gebunden sein kann über eine gegebenenfalls wenigstens monosubstituierte Alkylengrupe und/oder kondensiert sein kann mit einem gegebenenfalls wenigstens monosubstituierten mono- oder polycyclischen Ringsystem, einem gegebenenfalls wenigstens monosubstituierten Aryl- oder Heteroarylradikal, das gebunden sein kann über eine gegebenenfalls wenigstens monosubstituierte Alkylengruppe und/oder kondensiert sein kann mit einem gegebenenfalls wenigstens monosubstituierten mono- oder polycyclischen Ringsystem, einer Nitrogruppe, einer Cyanogruppe, -OR^{10b}, OC(=O)R^{11b}, -(C=O)-OR^{11b}, -SR^{12b}, -SOR^{12b}, SO₂R^{12b}, -NH-SO₂R¹²b, -SO₂NH₂ und einem -NR^{13b}R^{14b}-Rest,
R^{5b} für Wasserstoff, ein unverzweigtes oder verzweigtes, gesättigtes oder ungesättigtes, gegebenenfalls wenigstens monosubstituiertes aliphatisches Radikal oder ein gesättigtes oder ungesättigtes, gegebenenfalls wenigstens monosubstituiertes, gegebenenfalls wenigstens ein Heteroatom als Ringbestandteil enthaltendes cycloaliphatisches Radikal steht,
R^{6b}, R^{7b} , R^{8b}, R^{9b} jeweils unabhängig voneinander ausgewählt sind unter Wasserstoff, einem unverzweigten oder verzweigten, gesättigten oder ungesättigten, gegebenenfalls wenigstens monosubstituierten aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, einer Cyanogruppe und einem -COOR^{15b}-Rest,
W^{b} für ein unverzweigtes oder verzweigtes, gesättigtes oder ungesättigtes, aliphatisches Radikal steht, das substituiert sein kann mit einem oder mehreren Substituenten, die ausgewählt sind unter Hydroxy, Halogen, verzweigtem oder unverzweigtem C₁₋₄-Alkoxy, verzweigtem oder unverzweigtem C₁₋₄-Perfluoralkoxy, verzweigtem oder unverzweigtem C₁₋₄-Perfluoralkyl, Amino, Carboxy, Amido, Cyano, Nitro, -SO₂NH₂, -CO-C₁₋₄-Alkyl, -SO-C₁₋₄-Alkyl, SO₂-C₁₋₄-Alkyl, -NH-SO₂-C₁₋₄-Alkyl, worin das C₁₋₄-Alkyl in jedem Fall verzweigt oder unverzweigt sein kann, einem unsubstituierten oder wenigstens monosubstituierten Phenyl- oder Naphthylradikal und einem unsubstituierten oder wenigstens monosubstituierten Furanyl-, Thienyl-, Pyrrolyl-, Imidazolyl-, Pyrazolyl-, Pyridininyl-, Pyrimidinyl-, Chinolyl- und Isochinolylradikal,
wobei die Substituenten wenigstens mit F, Cl, Methyl und Methoxy monosubstituiert sein können,
für ein gesättigtes oder ungesättigtes, gegebenenfalls wenigstens ein Heteroatom als Ringbestandteil enthaltendes cycloaliphatisches Radikal steht, wobei das cycloaliphatische Radikal mit einem oder mehreren Substituenten substituiert sein kann, die ausgewählt sind unter Hydroxy, Nitro, Carboxy, Cyano, Keto, Halogen, C₁₋₂₀-Alkyl, teilweise fluoriertem C₁₋₄-Alkyl, teilweise chloriertem C₁₋₄-Alkyl, teilweise bromiertem C₁₋₄-Alkyl, C₁₋₅-Alkoxy, teilweise fluoriertem C₁₋₄-Alkoxy, teilweise chloriertem C₁₋₄-Alkoxy, teilweise bromiertem C₁₋₄-Alkoxy, C₂₋₆-Alkenyl, SO₂-C₁₋₄-Alkyl, -(C=O)-C₁₋₅-Alkyl, -C(O)-O-C₁₋₅-Alkyl, - (C=O)-Cl, -S-C₁₋₄-Alkyl, -(C=O)-H, -NH-(C=O)-NH-C₁₋₅-Alkyl, -(C=O)-C₁₋₄-Perfluoralkyl" NR^{A}R^{B}, wobei RA und RB unabhängig voneinander ausgewählt sind unter H, C₁₋₄-Alkyl und Phenyl, -NH-(C=O)-C₁₋₅-Alkyl, -C₁₋₅-Alkylen-(C=O)-C₁₋₅-Alkyl, (1,3-Dihydro-1-oxo-2H-isoindol-2-yl), N-Phthalinidinyl-, (1,3-Dioxo-2-azaspiro[4,4]-non-2-yl, substituiertem oder unsubstituiertem Phenyl, -SO₂-Phenyl, Phenoxy, Pyridinyl, Pyridinyloxy, Pyrazolyl, Pyrimidinyl, Pyrrolidinyl-, - SO*₂-Pyrrolidinyl, Morpholinyl, SO₂-Morpholinyl-, Thiadiazolyl, Oxadiazolyl, Oxazolyl, Thiazolyl, Isoxazolyl, O-CH₂-Thiazolyl, -NH-Phenyl, und -C₁₋₄-Alkylen-NH-(C=O)-Phenyl,
wobei die Substitutenten mit einem oder mehreren Substitutenten substituiert sein können, die ausgewählt sind unter Halogen, Nitro, Cyano, Hydroxy, - (C=O)-C₁₋₄-Alkyl, C₁₋₄-Alkyl, wenigstens teilweise fluoriertem C₁₋₄-Alkyl, wenigstens teilweise chloriertem C₁₋₄-Alkyl, wenigstens teilweise bromiertem C₁₋₄-Alkyl, -S-C₁₋₄-Alkyl, -C(O)-O-C₁₋₅-Alkyl, -(C=O)-CH₂-F, -(C=O)-CH₂-Cl und - (C=O)-CH₂-Br,
und wobei das cycloaliphatische Radikal über eine gegebenenfalls wenigstens monosubstituierte Alkylengruppe gebunden sein kann und/oder mit einem gegebenenfalls wenigstens monosubstituierten aromatischen mono- oder polycyclischen Ringsystem kondensiert sein kann,
für ein gegebenenfalls wenigstens monosubstituiertes Aryl- oder Heteroarylradikal steht, das über eine gegebenenfalls wenigstens monosubstituierte Alkylengruppe gebunden sein kann und/oder mit einem gegebenenfalls wenigstens monosubstituierten aromatischen mono- oder polycyclischen Ringsystem kondensiert sein kann, einen NR^{16b}R^{17b}-Rest oder einen COR^{18b}-Rest steht,
R^{10b} für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{11b} für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{12b} für ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{13b} und R^{14b} jeweils unabhängig voneinander ausgewählt sind unter Wasserstoff, einem unverzweigten oder verzweigten, gesättigten oder ungesättigten, gegebenenfalls wenigstens monosubstituierte aliphatischen Radikal, einem gesättigten oder ungesättigten, gegebenenfalls wenigstens monosubstituierten, gegebenenfalls wenigstens ein Heteroatom als Ringbestandteil enthaltenden cycloaliphatischen Radikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, oder einem gegebenenfalls wenigstens monosubstituiertem Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann,
oder R^{13b} und R^{14b} zusammen mit dem überbrückenden Stickstoffatom einen gesättigten, ungesättigten oder aromatischen heterozyklischen Ring bilden, der wenigstens monosubstituiert sein kann und/oder wenigstens ein weiteres Heteroatom als Ringbestandteil enthalten kann,
R^{15b} für Wasserstoff, ein unverzweigtes oder verzweigtes, gesättigtes oder ungesättigtes, gegebenenfalls wenigstens monosubstituiertes aliphatisches Radikal, ein gesättigtes oder ungesättigtes, gegebenenfalls wenigstens monosubstituiertes, gegebenenfalls wenigstens ein Heteroatom als Ringbestandteil enthaltendes cycloaliphatisches Radikal oder ein gegebenenfalls wenigstens monosubstituiertes Aryl- oder Heteroarylradikal, das über eine gegebenenfalls wenigstens monosubstituierte Alkylengruppe gebunden sein kann und/oder mit einem gegebenenfalls wenigstens monosubstituierten mono- oder polyzyklischen Ringsystem kondensiert sein kann, steht,
R^{16b} für ein unverzweigtes oder verzweigtes, gesättigtes oder ungesättigtes, gegebenenfalls wenigstens monosubstituiertes aliphatisches Radikal steht,
R^{17b} für ein unverzweigtes oder verzweigtes, gesättigtes oder ungesättigtes, gegebenenfalls wenigstens monosubstituiertes aliphatisches Radikal steht,
R^{18b} für ein gegebenenfalls wenigstens monosubstituiertes Arylradikal steht,
gegebenenfalls in Form eines ihrer Stereoisomere, vorzugsweise Enantiomere oder Diastereomere, ihres Racemats oder in Form eines Gemisches von wenigstens zwei ihrer Stereoisomere, vorzugsweise Enantiomere oder Diastereomere, in einem beliebigen Mischungsverhältnis, oder ein physiologisch akzeptables Salz davon, beziehungsweise ein Solvat.

43. Verbindungen nach Anspruch 42, **dadurch gekennzeichnet, dass** R^{1a}, R^{2b}, R^{3b}, R^{4b} jeweils unabhängig voneinander ausgewählt sind unter H, F, Cl, Br, einem unverzweigten oder verzweigten, gesättigten oder ungesättigten, gegebenenfalls wenigstens monosubstituierten C₁₋₆-aliphatischem Radikal, einem gesättigten oder ungesättigten, gegebenenfalls wenigstens monosubstituierten, gegebenenfalls wenigstens ein Heteoratom als Ringbestandteil enthaltenden C₃₋₈-cycloaliphatischen Radikal, das über eine gegebenenfalls wenigstens monosubstituierte C₁₋₆-Alkylengruppe gebunden sein kann und/oder mit einem gegebenenfalls wenigstens monosubstituierten mono- oder polyzyklischen Ringsystem kondensiert sein kann, einem gegebenenfalls wenigstens monosubstituierten, 5- oder 6-gliedrigen Aryl- oder Heteroarylradikal, das über eine gegebenenfalls wenigstens monosubstituierte C₁₋₆-Alkylengruppe gebunden sein kann und/oder mit einem gegebenenfalls wenigstens monosubstituierten mono- oder polyzyklischen Ringsystem kondensiert sein kann, einer Nitrogruppe, einer Cyanogruppe, OR^{10b}, OC(=O)R^{11b}, -SR^{12b}, -SOR^{12b}, SO₂R^{12b}, -NH-SO₂R¹²b, -SO₂NH₂ und einem - NR^{13b}R¹⁴b -Rest, vorzugsweise ausgewählt sind unter H, F, Cl, Br, einem gesättigten, verzweigten oder unverzweigten, gegebenenfalls wenigstens monosubstituierten C₁₋₃-aliphatischen Radikal, einem gesättigten, gegebenenfalls wenigstens monosubstituierten, gegebenenfalls wenigstens ein Heteroatom als Ringbestandteil enthaltenden C₅- oder C₆-cycloaliphatischen Radikal, das über eine gegebenenfalls wenigstens monosubstituierte C₁- oder ₂-Alkylengruppe gebunden sein kann, einer Nitrogruppe, eine Cyanogruppe, - OR^{10b}, OC(=O)R^{11b}, -SR^{12b}, und einem -NR^{13b}R^{14b}-Rest, besonders bevorzugt ausgewählt sind unter H, F, Cl, Br, -CH₃, -CH₂CH₃, -CF₃, -CF₂CF₃, Cyclopentyl, Cyclohexyl, Nitro, Cyano und -OR^{10b}.

44. Verbindungen nach Anspruch 42 oder Anspruch 42, **dadurch gekennzeichnet, dass** R^{5b} für Wasserstoff, ein unverzweigtes oder verzweigtes, gesättigtes oder ungesättigtes, gegebenenfalls wenigstens monosubstituiertes C₁₋₆-aliphatisches Radikal oder ein gesättigtes oder ungesättigtes, gegebenenfalls wenigstens monosubstituiertes, gegebenenfalls wenigstens ein Heteroatom als Ringbestandteil enthaltendes C₃₋₈-cycloaliphatisches Radikal, vorzugsweise für H oder ein verzweigtes oder unverzweigtes C₁₋₃-Alkylradikal , besonders bevorzugt für H, -CH₃ oder-CH₂₋CH₃ steht, ganz besonders bevorzugt für ein Wasserstoffatom steht.

45. Verbindungen nach einem der Ansprüche 42 bis 44, **dadurch gekennzeichnet, dass** R^{6b}, R^{7b}, R^{8b}, R^{9b} jeweils unabhängig voneinander ausgewählt sind unter Wasserstoff, einem unverzweigten oder verzweigten, gesättigten oder ungesättigten, gegebenenfalls wenigstens monosubstituierten C₁₋₆-aliphatischen Radikal, einem gesättigten oder ungesättigten, gegebenenfalls wenigstens monosubstituierten, gegebenenfalls wenigstens ein Heteroatom als Ringbestandteil enthaltenden C₃₋₈-cycloaliphatischen Radikal, einer Cyanogruppe und einem -COOR^{15b}-Rest, vorzugsweise ausgewählt sind unter H, einem verzweigten oder unverzweigten C₁₋₃-Alkylradikal, einer Cyanogruppe und einer -COOR^{15b}-Gruppe, besonders bevorzugt ausgewählt sind unter H, - CH₃, -CH₂CH₃ und einer Cyanogruppe, ganz besonders bevorzugt R^{6b} , R^{7b} , R^{8b} und R^{9b} jeweils für ein Wasserstoffatom stehen.

46. Verbindungen nach einem der Ansprüche 42 bis 45, **dadurch gekennzeichnet, dass** W^{b} steht für ein unverzweigtes oder verzweigtes, gesättigtes oder ungesättigtes C₁₋₂₀-aliphatisches Radikal, das substituiert sein kann mit einem oder mehreren Substituenten, die ausgewählt sind unter Hydroxy, Halogen, verzweigtem oder unverzweigtem C₁₋₄-Alkoxy, verzweigtem oder unverzweigtem C₁-₄-Perfluoralkoxy, verzweigtem oder unverzweigtem C₁₋₄-Perfluoralkyl, Amino, Carboxy, Amido, Cyano, Nitro, -SO₂NH₂, -CO-C₁₋₄-Alkyl, - SO-C₁-₄-Alkyl, -SO₂-C₁₋₄-Alkyl, -NH-SO₂-C₁₋₄-Alkyl, wobei das C₁₋₄-Alkyl in jedem Fall verzweigt oder unverzweigt sein kann, einem unsubstituierten oder wenigstens monosubstituierten Phenyl- oder Naphthylradikal und einem unsubstituierten oder wenigstens monosubstituierten Furanyl-, Thienyl-, Pyrrolyl-, Imidazolyl-, Pyrazolyl-, Pyridinyl-, Pyrimidinyl- Chinolinyl- und Isochinolinylradikal, wobei die Substituenten wenigstens monosubstituiert sein können mit F, Cl, Methyl und Methoxy; ein gesättigtes oder ungesättigtes, gegebenenfalls wenigstens ein Heteroatom als Ringbestandteil enthaltendes C₃₋₈-cycloaliphatisches Radikal, wobei das C₃₋₈-cycloaliphatische Radikal substituiert sein kann mit einem oder mehreren Substituenten, die ausgewählt sind unter Hydroxy, Nitro, Carboxy, Cyano, Keto, Halogen, C₁₋₂₀-Alkyl, teilweise fluoriertem C₁₋₄-Alkyl, teilweise chloriertem C₁₋₄-Alkyl, teilweise bromiertem C₁-₄-Alkyl, C₁₋₅-Alkoxy, teilweise fluoriertem C₁₋₄-Alkoxy, teilweise chloriertem C₁₋₄-Alkoxy, teilweise bromiertem C₁₋₄-Alkoxy, C₂₋₆-Alkenyl, SO₂-C₁₋₄-Alkyl, -(C=O)-C₁₋₅-Alkyl, -(C=O)-O-C₁₋₅-Alkyl, -(C=O)-Cl, -S-C1-4-Alkyl-, -(C=O)-H, -NH-(C=O)-NH-C₁₋₅-Alkyl, -(C=O)-C₁₋₄-Perfluoralkyl, -NR^{A}R^{B}, wobei R^{A} und R^{B} unabhängig voneinander ausgewählt sind unter H, C₁₋₄-Alkyl und Phenyl, NH-(C=O)-C₁₋₅-Alkyl, -C₁₋₅-Alkylen-(C=O)-C₁₋₅-Alkyl, (1,3-Dihydro-1-oxo-2H-isoindol-2-yl), N-Phthalinidinyl-, (1,3-Dioxo-2-azaspiro[4,4]-non-2-yl, substituiertem oder unsubstituiertem Phenyl, -SO₂-Phenyl, Phenoxy, Pyridinyl, Pyridinyloxy, Pyrazolyl, Pyrimidinyl, Pyrrolidinyl-, -SO₂-Pyrrolidinyl, Morpholinyl, SO₂-Morpholinyl-, Thiadiazolyl, Oxadiazolyl, Oxazolyl, Thiazolyl, Isoxazolyl, O-CH₂-Thiazolyl, -NH-Phenyl, und -C₁₋₄-Alkylen-NH-(C=O)-Phenyl, wobei die Substitutenten mit einem oder mehreren Substitutenten substituiert sein können, die ausgewählt sind unter Halogen, Nitro, Cyano, Hydroxy, -(C=O)-C₁₋₄-Alkyl, C₁₋₄-Alkyl, wenigstens teilweise fluoriertem C₁₋₄-Alkyl, wenigstens teilweise chloriertem C₁₋₄-Alkyl, wenigstens teilweise bromiertem C₁₋₄-Alkyl, -S-C₁₋₄-Alkyl, -C(=O)-O-C₁₋₅-Alkyl, -(C=O)-CH₂-F, -(C=O)-CH₂-Cl und -(C=O)-CH₂-Br,
und wobei das C₃₋₈-cycloaliphatische Radikal über eine gegebenenfalls wenigstens monosubstituierte C₁₋₆-Alkylengruppe gebunden sein kann und/oder mit einem gegebenenfalls wenigstens monosubstituierten aromatischen mono- oder polycyclischen Ringsystem kondensiert sein kann,
ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, einen NR^{16b}R^{17b}-Rest oder einen COR^{18b}-Rest,
W^{b} vorzugsweise steht für
ein unverzweigtes oder verzweigtes C₁₋₂₀-Alkylradikal, vorzugsweise ein Alkylradikal, das ausgewählt ist unter Methyl, Ethyl, n-Propyl, Isopropyl, n-Butyl, Isobutyl, sec-Butyl, tert-Butyl and 1,1-Dimethyl-propyl; einem unverzweigten oder verzweigten C₂₋₂₀-Alkenylradikal; vorzugsweise einem Vinylradikal; -CF₃; - CHF₂; -CH₂F; -CCl₃; -CHCl₂; - CH₂Cl; -CH₂-CF₃; -CH₂-CH₂-Cl; -CH₂-CH₂-CH₂-Cl; -CH₂-S(=O)₂-CH₃; einem Cyclopropylradikal; einem Cyclobutylradikal; einem Cyclopentylradikal; einem Cyclohexylradikal; -CH₂-Cyclopropyl; -CH₂-Cyclobutyl; -CH₂-Cyclopentyl; -CH₂-Cyclohexyl; -N(CH₃)₂; -N(C₂H₅)₂ ; -N(n-CH₂-CH₂-CH₃)₂; Phenyl; Benzyl; Naphthyl; -CH=CH-phenyl; -(CF₂)-(CF₂)-O-phenyl; - (CH₂)-Naphthyl; -(CH₂)-(CH₂)-naphthyl; Anthracenyl; -(C=O)-Phenyl; Thiophenyl; Benzo[b]thiophenyl; Furanyl; 2-Oxo-2H- chromenyl; Dibenzofuranyl; 2,3-Dihydrobenzofuranyl; Chromanyl; 2,3-Dihydro-benzo[1,4]dioxinyl; 3,4-Dihydro-2H-1,5-benzo-dioxepinyl; Chromonyl; 1 H-Imidazolyl; Pyridinyl; Pyrrolidin-2,5-dionyl; Pyrrolyl; 1H-pyrazolyl; 1 H-pyrimidin-2,4-dionyl; Chinolinyl; Isochinolinyl; 1 H-Benzoimidazolyl; 1,4-Dihydrochinoxalin-2,3-dionyl; 1,2,3,4-Tetrahydro-isochinolinyl; 1,4-Dihydro-benzo[b][1,4]diazepine-2,4-dionyl; 1,3-Dihydro-1-oxo-2H-isoindolyl; Phthalimidinyl; 2-(1,3-Dioxo-1,3-dihydro-isoindol-2-yl)-ethyl; Imidazo[1,2-a]pyridin; Isatinyl; Thiazolyl; 1,3-Thiazolyl; 1,2,4-Thiadiazolyl; Imidazo[2,1-b]thiazolyl; 1,3-Benzothiazolyl; Benzo[1,2,5]Thiadiazolyl; 2-Oxo-2,3-dihydrobenzothiazolyl; 2,1,3-benzothiadiazolyl; Imidazo[2,1-b]thiazolyl; Isoxazolyl; Benzo[1,2,5]oxadiazolyl; Benzo[d]isoxazolyl; Benzofurazanyl; 2-Oxo-2,3-dihydro-benzooxazolyl; 3,4-Dihydro-2H-benzo[1,4]oxazinyl; oder 2,1,3-Benzoxadiazolyl;
wobei alle der vorgenannten zyklischen Reste gegebenenfalls substituiert sein können mit 1, 2, 3, 4 or 5 Substitutenten, die unabhängig voneinander ausgewählt sind unter Methyl; Ethyl; n-Propyl; Isopropyl; n-Butyl; Isobutyl; sec-Butyl; tert-Butyl; 1,1-Dimethyl-propyl; n-Pentyl; Vinyl; Cyclopropyl; Cyclobutyl; Cyclopentyl; Cyclohexyl; Morpholino; Methoxy; Ethoxy; n-Propoxy; Isopropoxy; n-Propoxy; F; Cl; Br; I; -CN; -OH;-CF₃; -CF₂H;-CH₂F; -CCl₃; -CClH₂; -CHCl₂; -CH₂-F; -CH₂-Cl; -CH₂-Br; -(C=O)-CH₂-Br; -OCF₃; - O-CH₂-CF₃; -O-CHF₂; -NO₂; -NH₂; -N(CH₃)₂; -N(C₂H₅)₂; -N(n-CH₂-CH₂-CH₃)₂; -N(n-CH₂-CH₂-CH₂-CH₃)₂; -NH-(C=O)-CH₃; -NH-phenyl; -(C=O)-CF₃; -(C=O)-OH; =O (oxo); -(C=O)-H; -S(=O)₂-CH₃; -S(=O)₂-Isopropyl; -S(=O)₂-Phenyl; -S(=O)₂-Pyrrolidinyl; -S(=O)₂-Morpholino;- (CH₂)-(CH₂)-(C=O)-O-CH₃; -NH-(C=O)-NH-CH₂-CH₂-CH₃; -(C=O)-CH₃; -(C=O)-O-CH₃; -(C=O)-O-C₂H₅; -(CH₂)-NH-(C=O)-Phenyl; -CH₂-C(H)(Phenyl)(phenyl); -O-CH₂-Thiazolyl; 1,3-Dioxo-2-azaspiro[4.4]non-2-yl; Phenyl; Phenoxy; Isoxazolyl; 1,3-Oxazolyl; 1,2,4-Oxadiazolyl; 1,3,4-Oxadiazolyl; Pyridinyl; Pyridinyloxy; Pyrazolyl; Pyrimidinyl und Phthalimidinyl;
und wobei jede dieser zyklischen Gruppen der zuvor genanten Substituenten gegebenenfalls substituiert sein kann mit 1, 2, 3, 4 oder 5 Substituenten, die unabhängig voneinander ausgewählt sind unter Methyl; Ethyl; n-Propyl; Isopropyl; F; CI; Br; I; CN; -CH₂-F; -CH₂-Cl; -CH₂-Br; -CF₃ und -S-CH₃,
W^{b} vorzugsweise steht für
ein Alkylradikal, das ausgewählt ist unter Methyl; Ethyl; n-Propyl; Isopropyl; n-Butyl; sec-Butyl; Isobutyl und tert-Butyl; Vinyl (CH₂=CH-); -N(CH₃)₂; 1-Naphthyl; Benzyl; 2-Naphtyl; Phenyl; 2-Methyl-phenyl; 3-Methyl-phenyl; 4-Methyl-phenyl; 2-Ethyl-phenyl; 3-Ethyl-phenyl; 4-Ethyl-phenyl; 2-n-Propyl-phenyl; 3-n-Propyl-phenyl; 4-n-Propyl-phenyl; 2-Isopropyl-phenyl; 3-Isopropyl-phenyl; 4-Isopropyl-phenyl; 2-n-Butyl-phenyl; 3-n-Butyl-phenyl; 4-n-Butyl-phenyl; 2-Isobutyl-phenyl; 3- Isobutyl-phenyl; 4-Isobutyl -phenyl; 2-tert-Butyl-phenyl; 3-tert-Butyl-phenyl; 4-tert-Butyl-phenyl; 1,1-Dimethylpropyl-phenyl; 2-Cyclopentyl-phenyl; 3-Cyclopentyl-phenyl; 4-Cyclopentyl-phenyl 2-Cyclohexyl-phenyl; 3-Cyclohexyl-phenyl; 4-Cyclohexyl-phenyl; 2-Methoxy-phenyl; 3-Methoxy-phenyl; 4-Methoxy-phenyl; 2-Ethoxy-phenyl; 3-Ethoxy-phenyl; 4-Ethoxy-phenyl; 2-n-Propoxy-phenyl; 3-n-Propoxy-phenyl; 4-n-Propoxy-phenyl; 2-lsopropoxy-phenyl; 3-Isopropoxy -phenyl; 4-Isopropoxy -phenyl; 2-Fluor-phenyl; 3-Fluor-phenyl; 4-Fluor-phenyl; 2-Chlor-phenyl; 3-Chlor-phenyl; 4-Chlor-phenyl; 2-Brom-phenyl; 3-Brom-phenyl; 4-Brom-phenyl; 2-Trifluormethyl-phenyl; 3-Trifluormethyl-phenyl; 4-Trifluormethyl-phenyl; 2-Trifluormethoxy-phenyl; 3-Trifluormethoxy-phenyl; 4- Trifluormethoxy-phenyl; 2-Carboxy-phenyl; 3-Carboxy-phenyl; 4-Carboxy-phenyl; 2-Acetyl-phenyl; 3-Acetyl-phenyl; 4-Acetyl-phenyl; 2-(C=O)-O-CH₃-phenyl; 3-(C=O)-O-CH₃-phenyl; 4-(C=O)-O-CH₃-Phenyl; 2-(CH₂)-(CH₂)-(C=O)-O-CH₃; 3-(CH₂)-(CH₂)- (C=O)-O-CH₃; 4-(CH₂)-(CH₂)-(C=O)-O-CH₃; 2-Cyano-phenyl; 3-Cyano-phenyl; 4-Cyano-phenyl; 2-Nitro-phenyl; 3-Nitro-phenyl; 4-Nitro-phenyl; 4-(4-Bromphenoxy)-phenyl; 2-Methylsulfonyl-phenyl; 3-Methylsulfonyl-phenyl; 4-Methylsulfonyl-phenyl; 2-Phenyl-phenyl (Biphenyl-2-yl); 3-Phenyl-phenyl (Biphenyl-3-yl); 4-Phenyl-phenyl (Biphenyl-4-yl); 2-Phenoxy-phenyl; 3-Phenoxy-phenyl; 4-Phenoxy-phenyl; 2,4-Dimethyl-phenyl; 3,4-Dimethyl-phenyl; 2,4,6-Trimethyl-phenyl; 2,3,5,6-Tetramethyl-phenyl; Pentamethyl-phenyl; 2,5-Dimethoxy-phenyl; 3,4-Dimethoxy-phenyl; 2,3-Dichlorphenyl; 2,4-Dichlor-phenyl; 2,5-Dichlor-phenyl; 3,4-Dichlor-phenyl; 3,5-Dichlorphenyl; 2,6-Dichlor-phenyl; 2,4-Difluor-phenyl; 3,4-Difluor-phenyl; 2,5-Difluorphenyl; 2,6-Difluor-phenyl; 3-Chlor-2-fluor-phenyl; 3-Chlor-4-fluor-phenyl; 5-Chlor-2-fluor-phenyl; 2,3,4-Trichlor-phenyl; 2,4,5- Trichlor-phenyl; 2,4,6-Trichlor-phenyl; 2,4,5-Trifluor-phenyl; 2,3,4-Trifluor-phenyl-; 2-Chlor-4,5-difluor-phenyl; 2-Brom-4-fluor-phenyl; 2-Brom-4,6-difluor-phenyl; 4-Chlor-2,5-difluor-phenyl; 5-Chlor-2,4-difluor-phenyl; 4-Brom-2,5-difluor-phenyl; 5-Brom-2,4-difluor-phenyl; Pentafluor-phenyl; 2,4-Dinitro-phenyl; 4-Chlor-3-nitro-phenyl; 2-Methyl-5-nitrophenyl; 5-Brom-2-methoxy-phenyl; 3-Chlor-2-methyl-phenyl; 4-Brom-3-methyl-phenyl; 4- Chlor-2,5-dimethyl-phenyl; 4-Fluor-3-methyl-phenyl; 5-Fluor-2-methyl-phenyl; 2-Nitro-4-trifluormethyl-phenyl; 2-Methoxy-4-methyl-phenyl; 3,5-Dichlor-2-hydroxy-phenyl; 3,5-Dichlor-4-hydroxy-phenyl; 5-Chlor-2,4-difluorphenyl; 3-Chlor-4-(NH)-(C=O)-CH₃-phenyl; 2-Chlor-6-methyl-phenyl; 2-Chlor-5-trifluormethyl-phenyl; 2-Chlor-5-trifluormethoxy-phenyl; 4-Brom-2-trifluormethoxy-phenyl; 4-Brom-2-trifluormethyl-phenyl; 4-Brom-3- trifluormethyl-phenyl; 3-Carboxy-4-fluor-phenyl; 3-Carboxy-4-chlor-6-fluor-phenyl; 4-Methoxy-2,3,6-trimethyl-phenyl-; oder eine der folgenden Gruppen 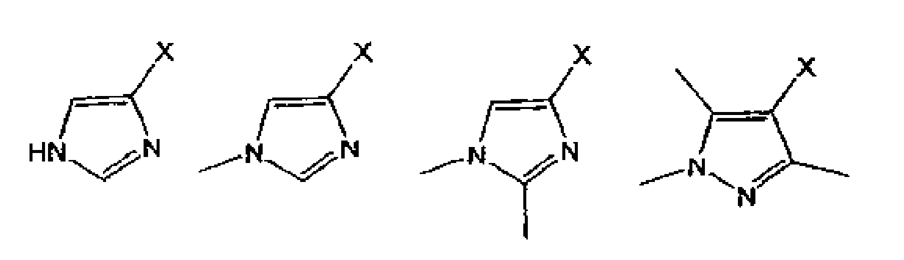 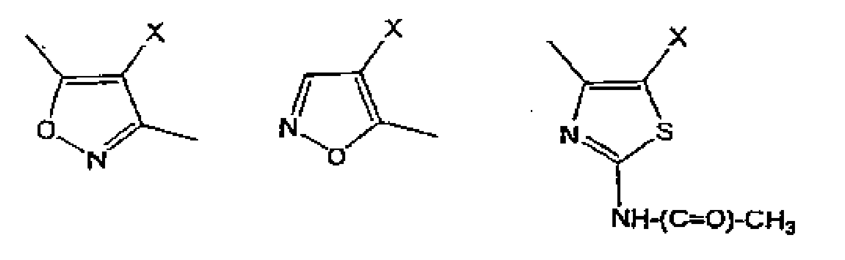 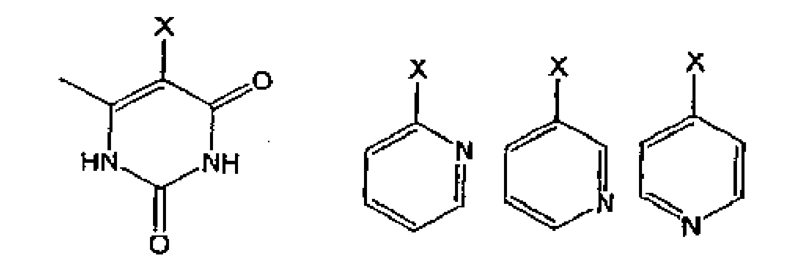 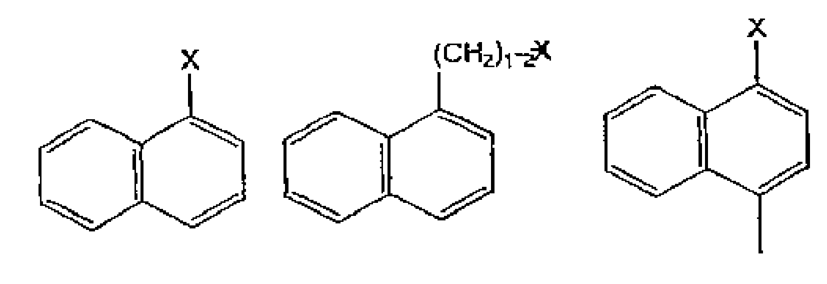 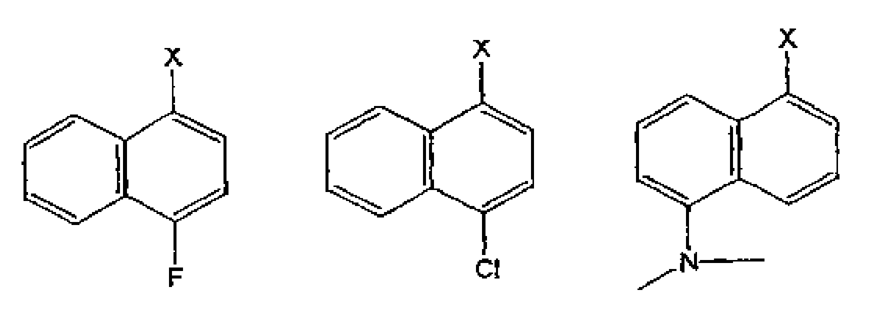 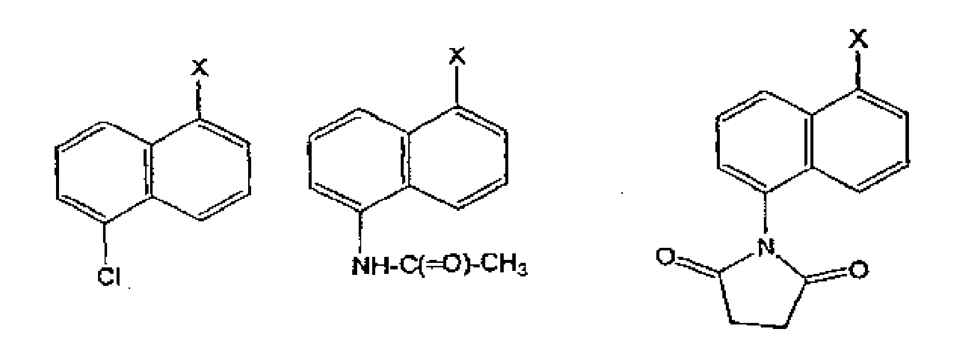 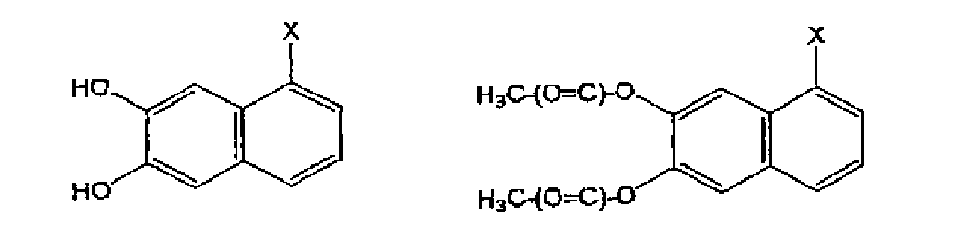 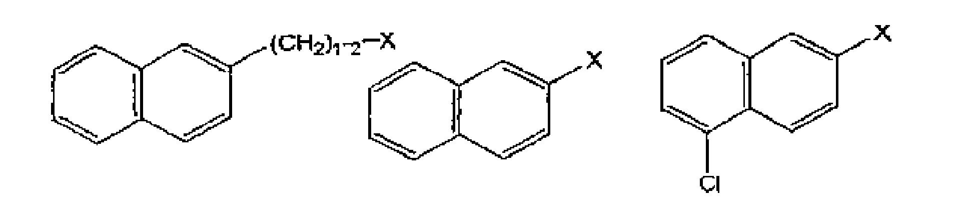 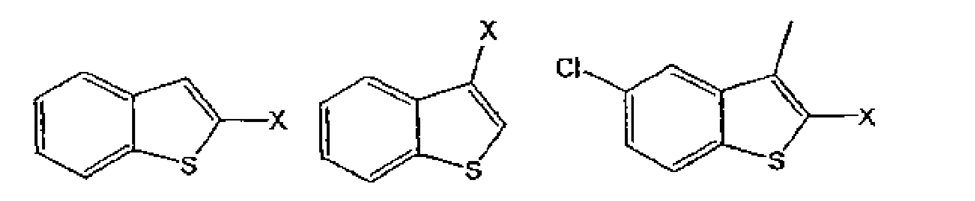 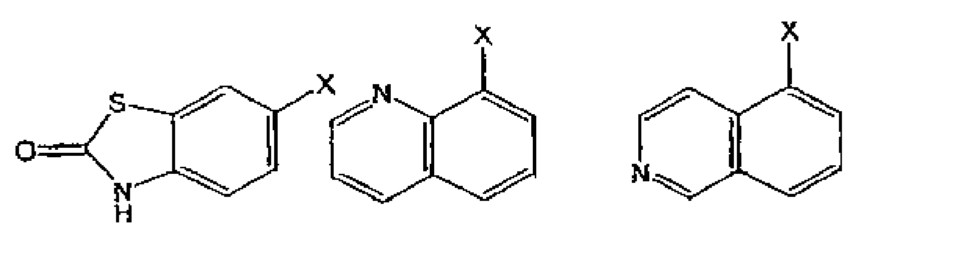 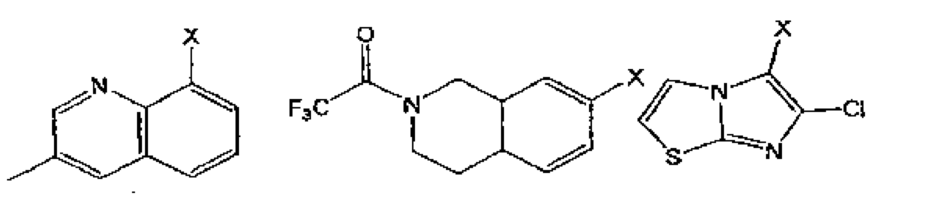 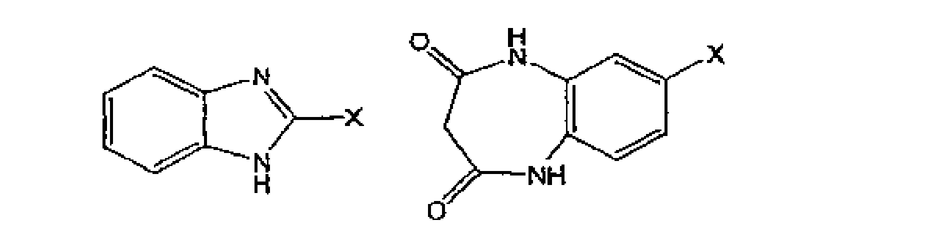 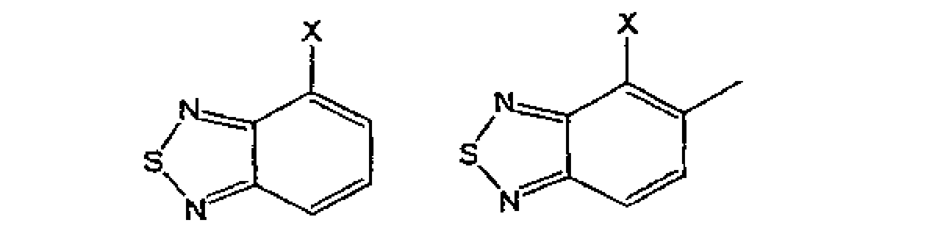 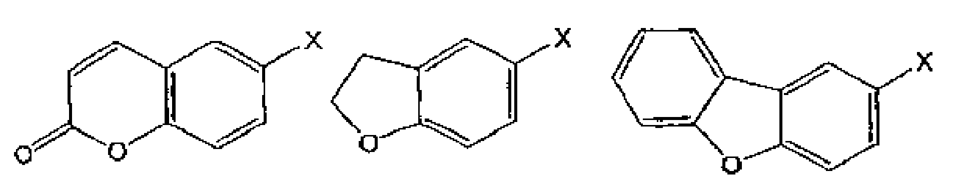 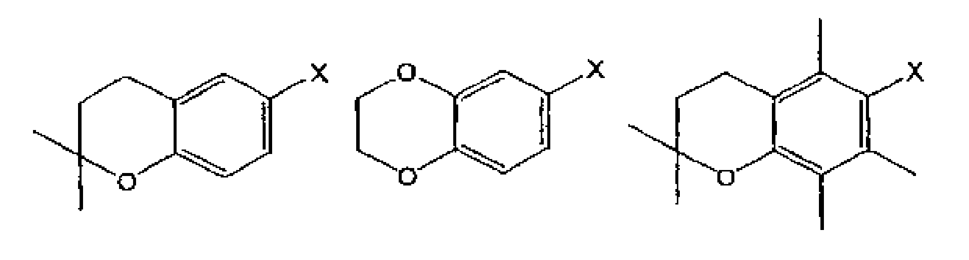 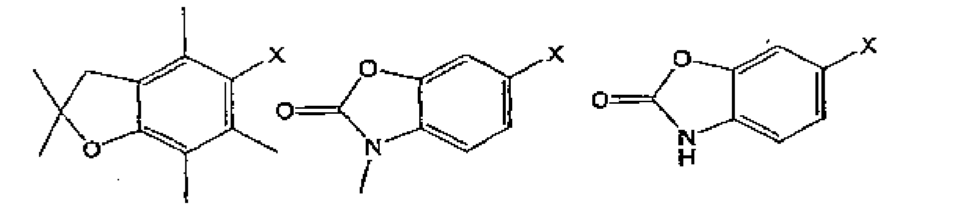 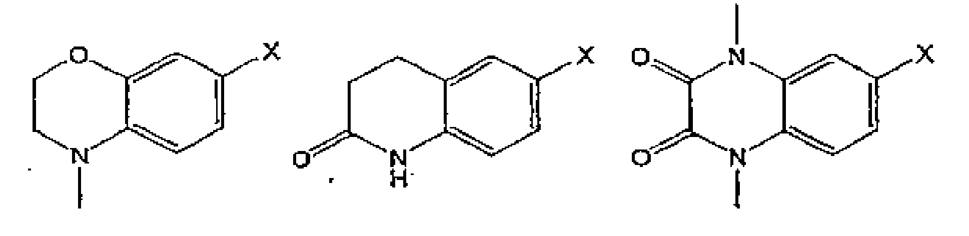 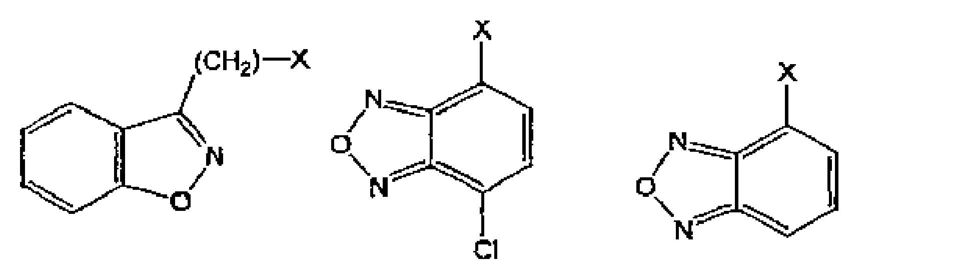 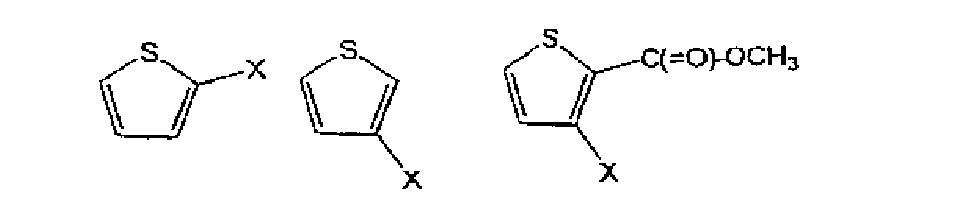 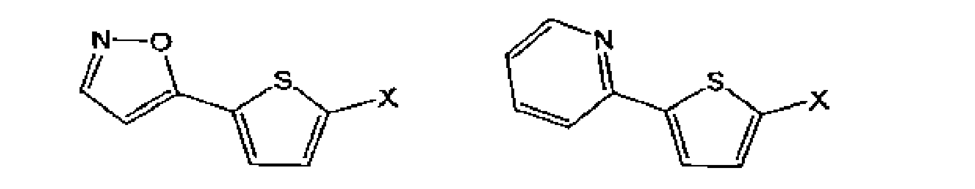 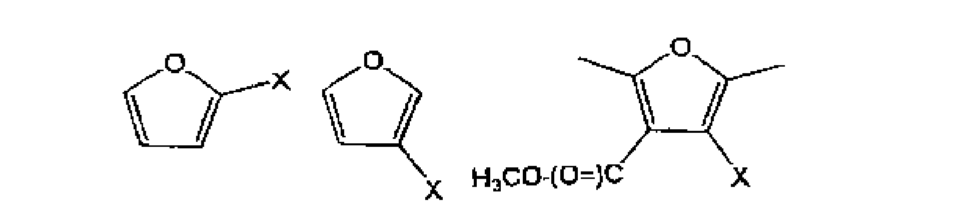 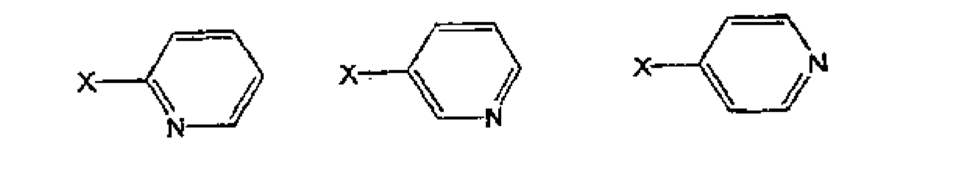 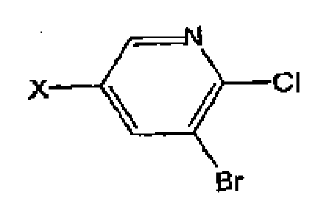 wobei in jedem Fall X die Position anzeigt, in der der jeweilige Substituent W^{b} an die -SO₂Gruppe der Formel (Ib) gebunden ist.

47. Verbindungen nach einem der Ansprüche 42 bis 46, **dadurch gekennzeichnet, dass** R^{10b} steht für Wasserstoff, ein unverzweigtes oder verzweigtes, gesättigtes oder ungesättigtes, gegebenenfalls monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem,
vorzugsweise für H, ein lineares oder verzweigtes C₁₋₄-Alkylradikal, ein Cyclohexylradikal oder ein Phenylradikal, besonders bevorzugt für H, -CH₃, - C₂H₅ oder Phenyl steht.

48. Verbindungen nach einem der Ansprüche 42 bis 46, **dadurch gekennzeichnet, dass** R^{11b} steht für Wasserstoff, ein unverzweigtes oder verzweigtes, gesättigtes oder ungesättigtes, gegebenenfalls monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem,
vorzugsweise für H, ein lineares oder verzweigtes C₁₋₄-Alkylradikal, ein Cyclohexylradikal oder ein Phenylradikal, besonders bevorzugt für H, -CH₃, - C₂H₅ oder Phenyl steht.

49. Verbindungen nach einem der Ansprüche 42 bis 48, **dadurch gekennzeichnet, dass** R^{12b} steht für Wasserstoff, ein unverzweigtes oder verzweigtes, gesättigtes oder ungesättigtes, gegebenenfalls wenigstens monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem,
vorzugsweise für H, ein lineares oder verzweigtes C₁₋₄-Alkylradikal, ein Cyclohexylradikal oder ein Phenylradikal, besonders bevorzugt für H, -CH₃, - C₂H₅ oder Phenyl steht.

50. Verbindungen nach einem der Ansprüche 42 bis 49, **dadurch gekennzeichnet, dass** R^{13b} und R^{14b} jeweils unabhängig voneinander ausgewählt sind unter Wasserstoff, einem unverzweigten oder verzweigten, gesättigten oder ungesättigten, gegebenenfalls wenigstens monosubstituierten C₁₋₆-aliphatisches Radikal, einem gesättigten oder ungesättigten, gegebenenfalls wenigstens monosubstituierten, gegebenenfalls wenigstens ein Heteroatom als Ringbestandteil enhaltenden C₃₋₈-cydoaliphatischen Radikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, oder einem gegebenenfalls wenigstens monosubstituierten, 5- oder 6-gliedrigen Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklische Ringsystem,
vorzugsweise jeweils unabhängig voneinander ausgewählt sind unter H, einem linearen oder verzweigten C₁₋₄-Alkylradikal, einem Cyclohexylradikal oder einem Phenylradikal, besonders bevorzugt jeweils unabhängig voneinander ausgewählt sind unter H, -CH₃, -C₂H₅ und Phenyl.

51. Verbindungen nach einem der Ansprüche 42 bis 50, **dadurch gekennzeichnet, dass** R^{13b} und R^{14b} zusammen mit dem überbrückenden Stickstoffatom einen gesättigten, ungesättigten oder aromatischen, 5- oder 6-gliedrigen heterozyklischen Ring bilden, der wenigstens monosubstituiert sein kann und/oder wenigstens ein weiteres Heteroatom als Ringbestandteil enthalten kann, vorzugsweise eine unsubstituierte Piperidin- oder Moroholingruppe bilden.

52. Verbindungen nach einem der Ansprüche 42 bis 51, **dadurch gekennzeichnet, dass** R^{15b} steht für Wasserstoff, ein unverzweigtes oder verzweigtes, gesättigtes oder ungesättigtes, gegebenenfalls monosubstituiertes C₁₋₆-aliphatisches Radikal, ein gesättigtes oder ungesättigtes, gegebenenfalls wenigstens monosubstituiertes, gegebenenfalls wenigstens ein Heteroatom als Ringbestandteil enhaltendes C₃₋₈-cydoaliphatisches Radikal oder ein gegebenenfalls wenigstens monosubstituiertes, 5- oder 6-gliedriges Aryl- oder Heteroarylradikal, das gebunden sein kann über eine gegebenenfalls wenigstens monosubstituierte C₁₋₆-Alkylengruppe und/oder kondensiert sein kann mit einem gegebenenfalls wenigstens monosubstituierten mono- oder polyzyklischen Ringsystem, vorzugsweise für H, ein lineares oder verzweigtes C₁₋₄-Alkylradikal, ein Cyclohexylradikal oder ein Phenylradikal steht, besonders bevorzugt für H, -CH₃, -C₂H₅ oder Phenyl steht.

53. Verbindungen nach einem der Ansprüche 42 bis 52, **dadurch** charakterisiert, dass R^{16b} für ein unverzweigtes oder verzweigtes, gesättigtes oder ungesättigtes, gegebenenfalls wenigstens monosubstituiertes C₁₋₆-aliphatisches Radikal, vorzugsweise für ein unverzweigtes oder verzweigtes, gesättigtes, unsubstituiertes C₁₋₃-Alkylradikal, besonders bevorzugt für ein Methylradikal steht.

54. Verbindungen nach einem der Ansprüche 42 bis 53, **dadurch** charakterisiert, dass R^{17b} für ein unverzweigtes oder verzweigtes, gesättigtes oder ungesättigtes, gegebenenfalls wenigstens monosubstituiertes C₁₋₆-aliphatisches Radikal, vorzugsweise für ein unverzweigtes oder verzweigtes, gesättigtes, unsubstituiertes C₁₋₃-Alkylradikal, besonders bevorzugt für ein Methylradikal steht.

55. Verbindung nach einem der Ansprüche 42 bis 53, **dadurch** charakterisiert, dass R^{18b} für ein Phenylradikal steht, das gegebenenfalls wenigstens monosubstituiert ist mit einem C₁₋₆-aliphatischen Radikal, besonders bevorzugt für ein Phenylradikal steht, das gegebenenfalls wenigstens mit einer Methylgruppe monosubstituiert ist.

56. Verbindungen nach einem der Ansprüche 42 bis 55, **dadurch gekennzeichnet, dass**
R^{1b}, R^{2b}, R^{3b}, R^{4b} jeweils unabhängig voneinander ausgewählt sind unter einem Wasserstoffatom; einem Fluoratom; einem Chloratom; einem Bromatom; einer Methylgruppe und einer Methoxygruppe;
R^{5b} für ein Wasserstoffatom steht;
R^{6b} , R^{7b} , R^{8b} , R^{9b} jeweils für ein Wasserstoffatom stehen,
Wb steht für
ein Alkylradikal, das ausgewählt ist unter Methyl; Ethyl; n-Propyl; Isopropyl; n-Butyl; sec-Butyl; Isobutyl und tert-Butyl; Vinyl (CH₂=CH-); -N(CH₃)₂; 1-Naphthyl; Benzyl; 2-Naphthyl; Phenyl; 2-Methyl-phenyl; 3-Methyl-phenyl; 4-Methyl-phenyl; 2-Ethyl-phenyl; 3-Ethyl-phenyl; 4-Ethyl-phenyl; 2-n-Propyl-phenyl; 3-n-Propyl-phenyl; 4-n-Propyl-phenyl; 2-Isopropyl-phenyl; 3-Isopropyl-phenyl; 4-Isopropyl-phenyl; 2-n-Butyl-phenyl; 3-n-Butyl-phenyl; 4-n-Butyl-phenyl; 2-Isobutyl-phenyl; 3-Isobutyl-phenyl; 4-Isobutyl-phenyl; 2-tert-Butyl-phenyl; 3-tert-Butyl-phenyl; 4-tert-Butyl-phenyl; 1,1-Dimethylpropyl-phenyl; 2-Cyclopentyl-phenyl; 3-Cyclopentyl-phenyl; 4-Cyclopentyl-phenyl 2-Cyclohexyl-phenyl; 3-Cyclohexyl-phenyl; 4-Cyclohexyl-phenyl; 2-Methoxy-phenyl; 3-Methoxy-phenyl; 4-Methoxy-phenyl; 2-Ethoxy-phenyl; 3-Ethoxy-phenyl; 4-Ethoxy-phenyl; 2-n-Propoxy-phenyl; 3-n-Propoxy-phenyl; 4-n-Propoxy-phenyl; 2-lsopropoxy-phenyl; 3-Isopropoxy-phenyl; 4-lsopropoxy-phenyl; 2-Fluor-phenyl; 3-Fluor-phenyl; 4-Fluor-phenyl; 2-Chlor-phenyl; 3-Chlor-phenyl; 4-Chlor-phenyl; 2-Brom-phenyl; 3-Brom-phenyl; 4-Brom-phenyl; 2-Trifluormethyl-phenyl; 3-Trifluormethyl-phenyl; 4-Trifluormethyl-phenyl; 2-Trifluormethoxy-phenyl; 3-Trifluormethoxy-phenyl; 4- Trifluormethoxy-phenyl; 2-Carboxy-phenyl; 3-Carboxy-phenyl; 4-Carboxy-phenyl; 2-Acetyl-phenyl; 3-Acetyl-phenyl; 4-Acetyl-phenyl; 2-(C=O)-O-CH₃-phenyl; 3-(C=O)-O-CH₃-phenyl; 4-(C=O)-O-CH₃-Phenyl; 2-(CH₂)-(CH₂)-(C=O)-O-CH₃; 3-(CH₂)-(CH₂)-(C=O)-O-CH₃; 4-(CH₂)-(CH₂)-(C=O)-O-CH₃; 2-Cyano-phenyl; 3-Cyano-phenyl; 4-Cyano-phenyl; 2-Nitro-phenyl; 3-Nitro-phenyl; 4-Nitro-phenyl; 4-(4-Bromphenoxy)-phenyl; 2-Methylsulfonyl-phenyl; 3-Methylsulfonyl-phenyl; 4-Methylsulfonyl-phenyl; 2-Phenyl-phenyl (Biphenyl-2-yl); 3-Phenyl-phenyl (Biphenyl-3-yl); 4-Phenyl-phenyl (Biphenyl-4-yl); 2-Phenoxy-phenyl; 3-Phenoxy-phenyl; 4-Phenoxy-phenyl; 2,4-Dimethyl-phenyl; 3,4-Dimethyl-phenyl; 2,4,6-Trimethyl-phenyl; 2,3,5,6-Tetramethyl-phenyl; Pentamethyl-phenyl; 2,5-Dimethoxy-phenyl; 3,4-Dimethoxy-phenyl; 2,3-Dichlorphenyl; 2,4-Dichlor-phenyl; 2,5-Dichlor-phenyl; 3,4-Dichlor-phenyl; 3,5-Dichlorphenyl; 2,6-Dichlor-phenyl; 2,4-Difluor-phenyl; 3,4-Difluor-phenyl; 2,5-Difluorphenyl; 2,6-Difluor-phenyl; 3-Chlor-2-fluor-phenyl; 3-Chlor-4-fluor-phenyl; 5-Chlor-2-fluor-phenyl; 2,3,4-Trichlor-phenyl; 2,4,5-Trichlor-phenyl; 2,4,6-Trichlor-phenyl; 2,4,5-Trifluor-phenyl; 2,3,4-Trifluor-phenyl-; 2-Chlor-4,5-difluor-phenyl; 2-Brom-4-fluor-phenyl; 2-Brom-4,6-difluor-phenyl; 4-Chlor-2,5-difluor-phenyl; 5-Chlor-2,4-difluor-phenyl; 4-Brom-2,5-difluor-phenyl; 5-Brom-2,4-difluor-phenyl; Pentafluor-phenyl; 2,4-Dinitro-phenyl; 4-Chlor-3-nitro-phenyl; 2-Methyl-5-nitrophenyl; 5-Brom-2-methoxy-phenyl; 3-Chlor-2-methyl-phenyl; 4-Brom-3-methyl-phenyl; 4-Chlor-2,5-dimethyl-phenyl; 4-Fluor-3-methyl-phenyl; 5-Fluor-2-methyl-phenyl; 2-Nitro-4-trifluormethyl-phenyl; 2-Methoxy-4-methyl-phenyl; 3,5-Dichlor-2-hydroxy-phenyl; 3,5-Dichlor-4-hydroxy-phenyl; 5-Chlor-2,4-difluor-phenyl; 3-Chlor-4-(NH)-(C=O)-CH₃-phenyl; 2-Chlor-6-methyl-phenyl; 2-Chlor-5-trifluormethyl-phenyl; 2-Chlor-5-trifluormethoxy-phenyl; 4-Brom-2-trifluormethoxy-phenyl; 4-Brom-2-trifluormethyl-phenyl; 4-Brom-3-trifluormethyl-phenyl; 3-Carboxy-4-fluor-phenyl; 3-Carboxy-4-chlor-6-fluor-phenyl; 4-Methoxy-2,3,6-trimethyl-phenyl- ; oder eine der folgenden Gruppen 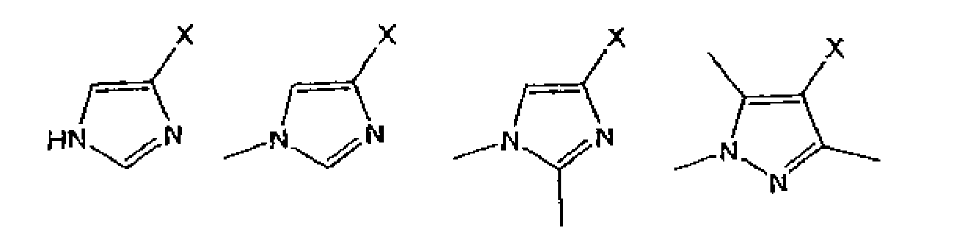 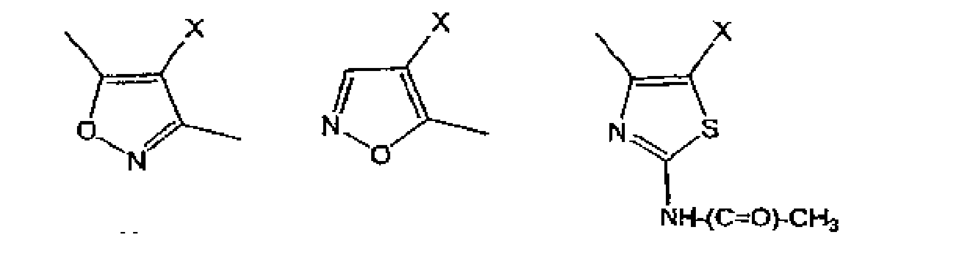 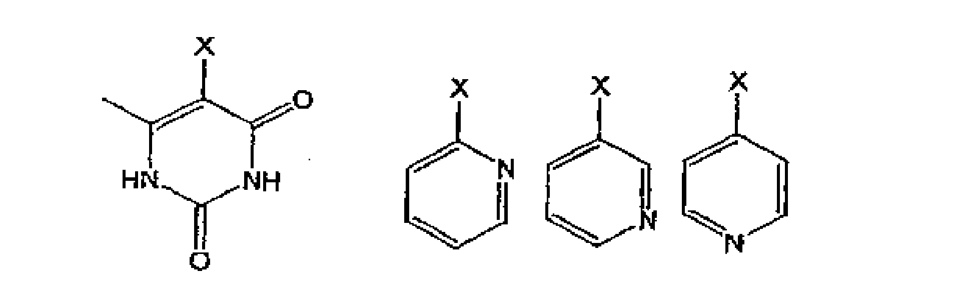 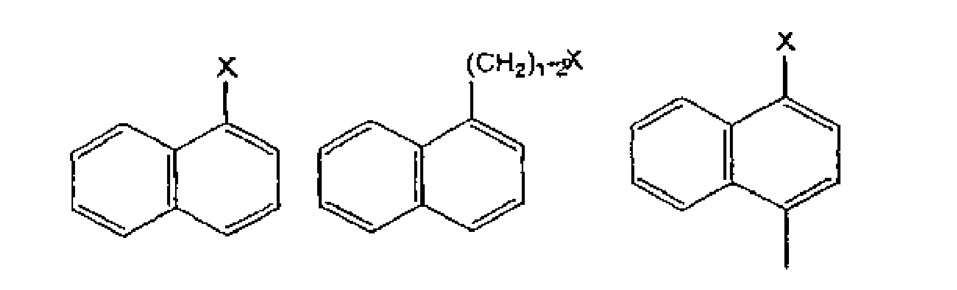 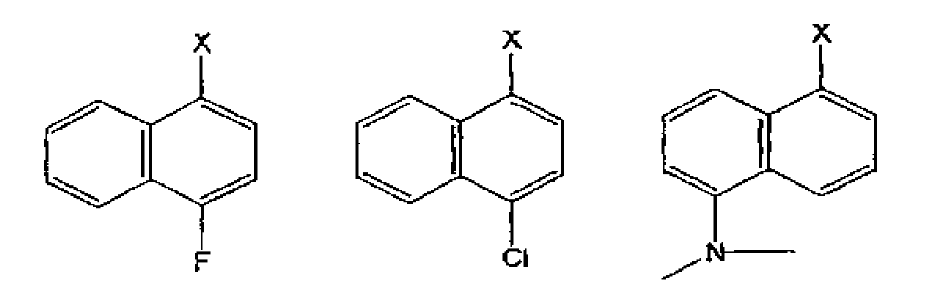 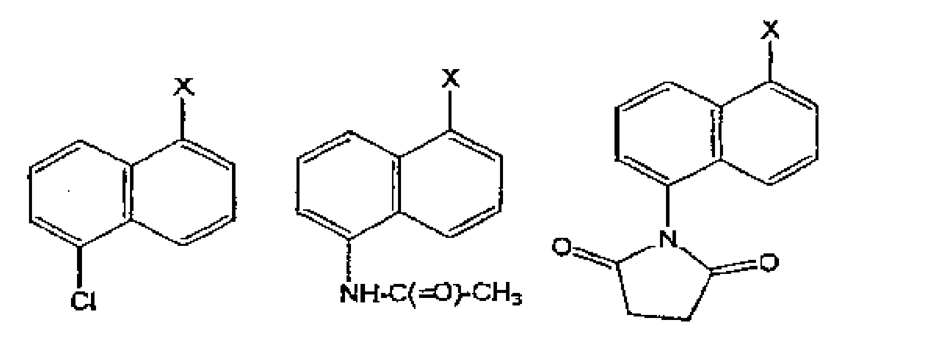 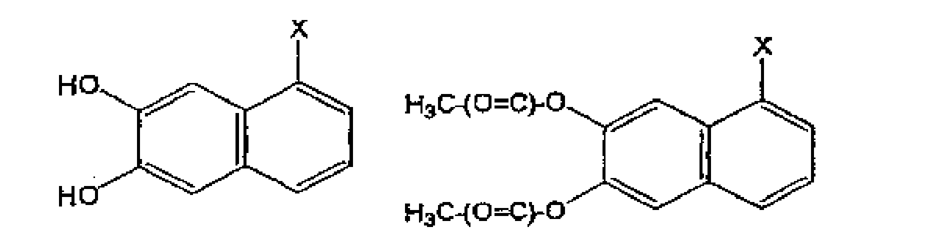 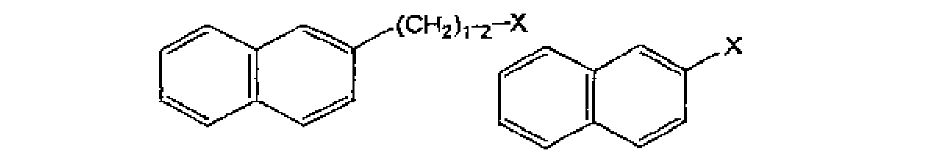 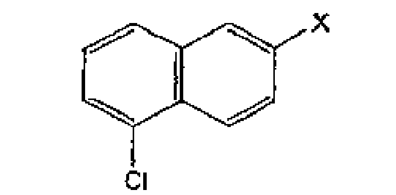 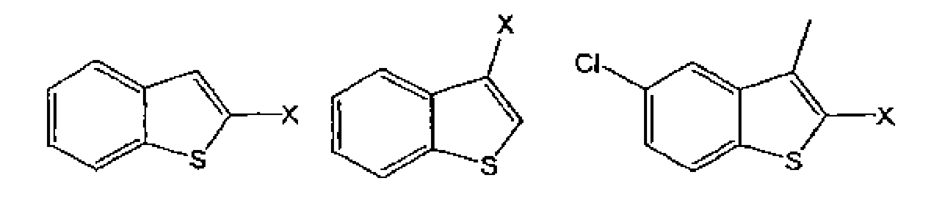 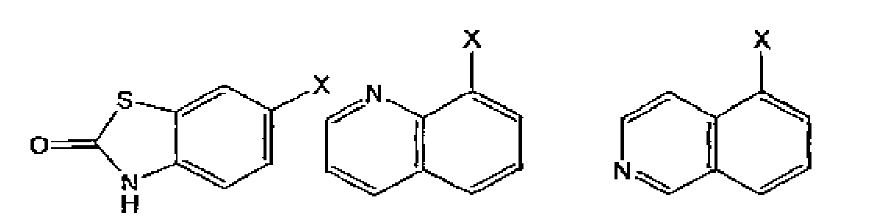 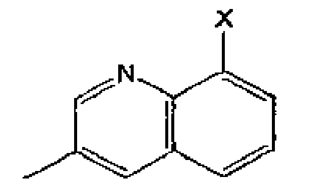 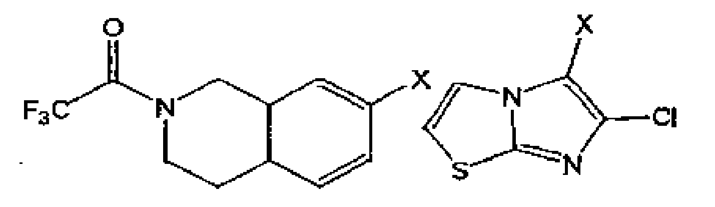 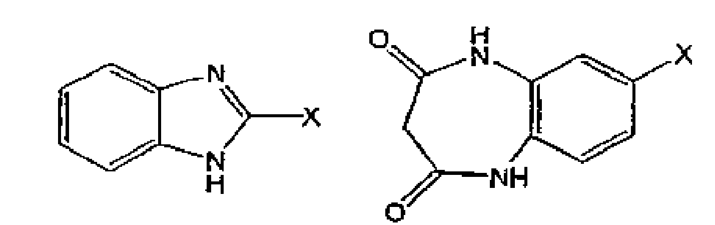 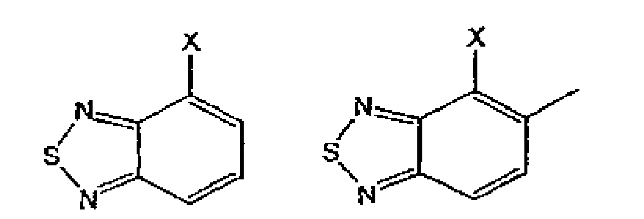 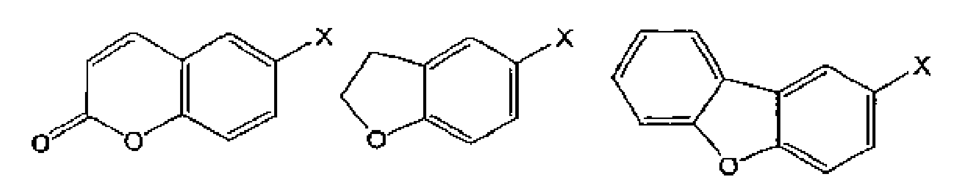 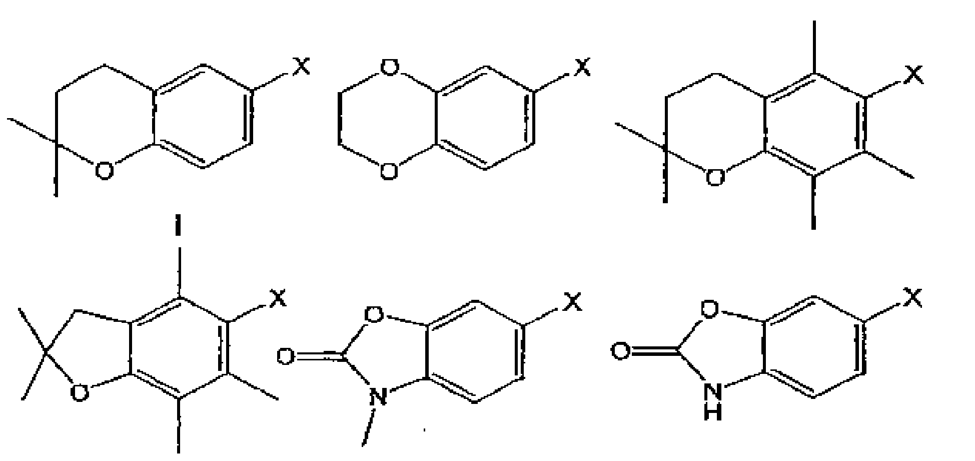 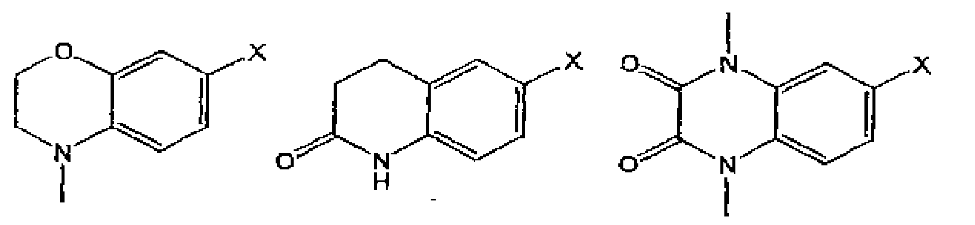 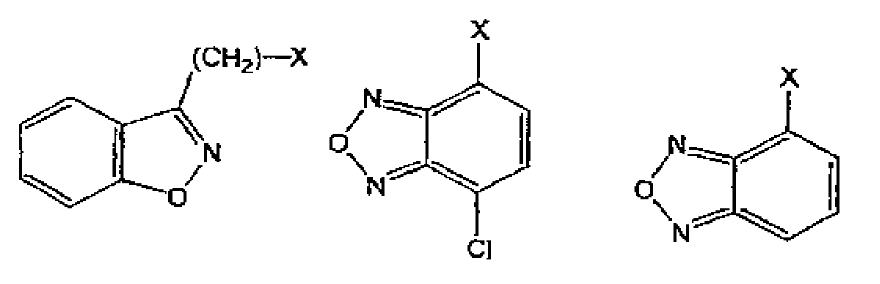 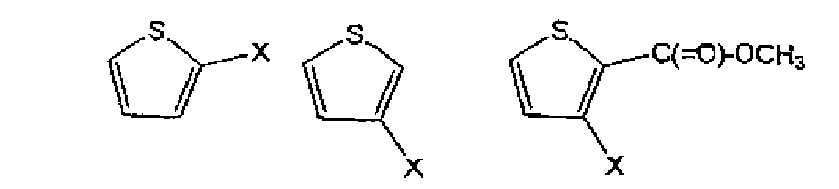 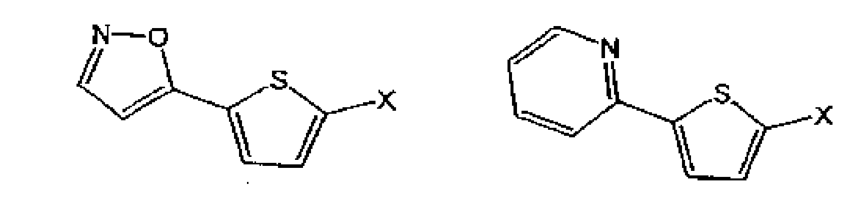 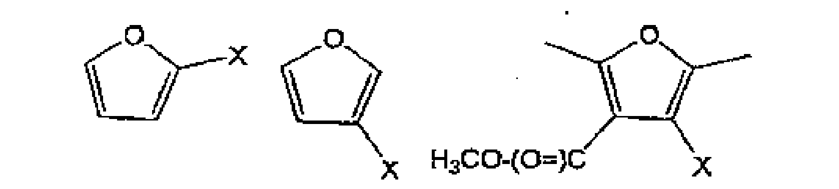 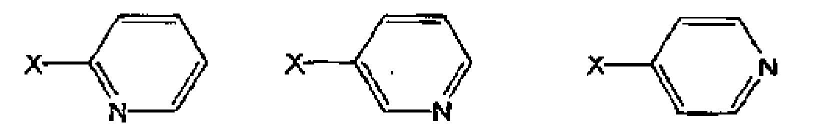 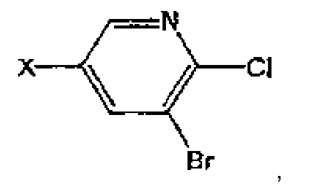 wobei in jedem Fall X die Position anzeigt, in der der jeweilige Substituent W^{b} an die -SO₂Gruppe der Formel (Ib) gebunden ist,
gegebenenfalls in Form eines ihrer Stereoisomere, vorzugsweise Enantiomere oder Diastereomere, ihrer Racemate oder in Form eines Gemisches von wenigstens zwei ihrer Stereoisomere, vorzugsweise Enantiomere oder Diastereomere, in einem beliebigen Mischungsverhältnis, oder ein physiologisch akzeptables Salz davon, beziehungsweise ein Solvat.

57. Verbindungen nach einem der Ansprüche 42 bis 56, ausgewählt aus der folgenden Gruppe:
| Nr. | Verbindung |
|---|---|
| 1 | 1-[1-(Naphthalen-2-sulfonyl)-piperidin-4-yl]-1,4-dihydro-benzo[d][1,3] oxazin-2-on |
| 2 | 1-[1-(Toluol-4-sulfonyl)-piperidin-4-yl]-1,4-dihydro-benzo[d][1,3]oxazin-2-on |
| 3 | 1-(1-Phenylmethansulfonyl-piperidin-4-yl)-1,4-dihydro-benzo[d][1,3]oxazin-2-on |
| 4 | 1-(1-Benzolsulfonyl-piperidin-4-yl)-6-chlor-1,4-dihydro-benzo[d][1,3]oxazin-2-on |
| 5 | 6-Chlor-1-[1-(toluol-4-sulfonyl)-piperidin-4-yl]-1,4-dihydro-benzo[d][1,3]oxazin-2-on |
| 6 | 6-Chlor-1-(1-phenylmethansulfonyl-piperidin-4-yl)-1,4-dihydro-benzo[d][1,3]oxazin-2-on |
| 7 | 6-Chlor-1-[1-(naphthalen-1-sulfonyl)-piperidin-4-yl]-1,4-dihydro-benzo[d][1,3]oxazin-2-on |
| 8 | 6-Chlor-1-[1-(naphthalen-2-sulfonyl)-piperidin-4-yl]-1,4-dihydro-benzo[d][1,3]oxazin-2-on |
| 9 | 6-Chlor-1-[1-(5-chlor-3-methyl-benzo[b]thiophen-2-sulfonyl)-piperidin-4-yl]-1,4-dihydro-benzo[d][1,3]oxazin-2-on |
| 10 | 1-[1-(Thiophen-2-sulfonyl)-piperidin-4-yl]-1,4-dihydro-benzo[d][1,3]oxazin-2-on |
| 11 | 1-[1-(4-Acetyl-benzolsulfonyl)-piperidin-4-yl]-1,4-dihydro-benzo[d][1,3]oxazin-2-on |
| 12 | 2-[4-(2-Oxo-4H-benzo[d][1,3]oxazin-1-yl)-piperidin-1-sulfonyl]-benzonitril |
| 13 | 1-[1-(2,4-Dimethyl-benzolsulfonyl)-piperidin-4-yl]-1,4-dihydro-benzo[d][1,3]oxazin-2-on |
| 14 | 1-[1-(4-Methoxy-benzolsulfonyl)-piperidin-4-yl]-1,4-dihydro-benzo[d][1,3]oxazin-2-on |
| 15 | 1-[1-(2-Naphthalen-1-yl-ethansulfonyl)-piperidin-4-yl]-1,4-dihydro-benzo[d][1,3]oxazin-2-on |
| 16 | 8-Methyl-1-[1-(thiophen-2-sulfonyl)-piperidin-4-yl]-1,4-dihydro-benzo[d][1,3]oxazin-2-on |
| 17 | 1-[1-(4-Acetyl-benzolsulfonyl)-piperidin-4-yl]-8-methyl-1,4-dihydro-benzo[d][1,3]oxazin-2-on |
| 18 | 2-[4-(8-Methyl-2-oxo-4H-benzo[d][1,3]oxazin-1-yl]-piperidin-1-sulfonyl]-benzonitril |
| 19 | 1-[1-(2,4-Dimethyl-benzolsulfonyl)-piperidin-4-yl]-8-methyl-1,4-dihydro-benzo[d][1,3]oxazin-2-on |
| 20 | 1-[1-(4-Methoxy-benzolsulfonyl)-piperidin-4-yl]-8-methyl-1,4-dihydro-benzo[d][1,3]oxazin-2-on |
| 21 | 8-Methyl-1-[1-(2-naphthalen-1-yl-ethansulfonyl)-piperidin-4-yl]-1,4-dihydro-benzo[d][1,3]oxazin-2-on |
| 22 | 4-(8-Methyl-2-oxo-4H-benzo[d][1,3]oxazin-1-yl)-piperidin-1-sulfonsäure-dimethylamid |
| 23 | 2-[4-(2-Oxo-4H-benzo[d][1,3]oxazin-1-yl)-piperidin-1-sulfonyl]-benzoesäure-methylester |
| 24 | 1-[1-(3-Trifluormethyl-benzolsulfonyl)-piperidin-4-yl]-1,4-dihydro-benzo[d][1,3]oxazin-2-on |
| 25 | 2-[4-(8-Methyl-2-oxo-4H-benzo[d][1,3]oxazin-1-yl)-piperidin-1-sulfonyl]-benzoesäure-methylester |
| 26 | 8-Methyl-1-[1-(3-trifluormethyl-benzolsulfonyl)-piperidin-4-yl]-1,4-dihydro-benzo[d][1,3]oxazin-2-on |
| 27 | 1-[1-(4-Acetyl-benzolsulfonyl)-piperidin-4-yl]-6-chlor-1,4-dihydro-benzo[d][1,3]oxazin-2-on |
| 28 | 2-[4-(6-Chlor-2-oxo-4H-benzo[d][1,3]oxazin-1-yl)-piperidin-1-sulfonyl]-benzonitril |
| 29 | 6-Chlor-1-[1-(4-methoxy-benzolsulfonyl)-piperidin-4-yl]-1,4-dihydro-benzo[d][1,3]oxazin2-on |
| 30 | 2-[4-(6-Chlor-2-oxo-4H-benzo[d][1,3]oxazin-1-yl)-piperidin-1-sulfonyl]-benzoesäure-methylester |
| 31 | 6-Chlor-1-[1-(2,4-dimethyl-benzolsulfonyl)-piperidin-4-yl]-1,4-dihydro-benzo[d][1,3]oxazin-2-on |
| 32 | 6-Chlor-1-[1-(3-trifluormethyl-benzolsulfonyl)-piperidin-4-yl]-1,4-dihydro-benzo[d][1,3]oxazin-2-on |
| 33 | 1-[1-(5-Chlor-3-methyl-benzo[b]thiophen-2-sulfonyl)-piperidin-4-yl]-8-methyl-1,4-dihydro-benzo[d][1,3]oxazin-2-on |
| 34 | 1-{1-[4-(4-Brom-phenoxy)-benzolsulfonyl]-piperidin-4-yl}-8-methyl-1,4-dihydro-benzo[d][1,3]oxazin-2-on |
| 35 | 1-[1-(4-Fluor-benzolsulfonyl)-piperidin-4-yl]-8-methyl-1,4-dihydro-benzo[d][1,3]oxazin-2-on |
| 36 | 8-Methyl-1-[1-(naphthalen-2-sulfonyl)-piperidin-4-yl]-1,4-dihydro-benzo[d][1,3]oxazin-2-on |
| 37 | 8-Methyl-1-(1-phenylmethansulfonyl-piperidin-4-yl)-1,4-dihydro-benzo[d][1,3]oxazin-2-on |
| 38 | 1-[1-(4-Brom-benzolsulfonyl)-piperidin-4-yl]-6-chlor-1,4-dihydro-benzo[d][1,3]oxazin-2-on |
| 39 | 6-Chlor-1-[1-(4-methansulfonyl-benzolsulfonyl)-piperidin-4-yl]-1,4-dihydro-benzo[d][1,3]oxazin-2-on |
| 40 | 1-[1-(Butan-1-sulfonyl)-piperidin-4-yl]-6-chlor-1,4-dihydro-benzo[d][1,3]oxazin-2-on |
| 41 | 1-[1-(4-Brom-benzolsulfonyl)-piperidin-4-yl]-8-methyl-1,4-dihydro-benzo[d][1,3]oxazin-2-on |
| 42 | 1-[1-(4-Methansulfonyl-benzolsulfonyl)-piperidin-4-yl]-8-methyl-1,4-dihydro-benzo[d][1,3]oxazin-2-on |
| 43 | 1-[1-(Butan-1-sulfonyl)-piperidin-4-yl]-8-methyl-1,4-dihydro-benzo[d][1,3]oxazin-2-on |
| 44 | 6-Chlor-1-[1-(2-nitro-benzolsulfonyl)-piperidin-4-yl]-1,4-dihydro-benzo[d][1,3]oxazin-2-on |
| 45 | 6-Chlor-1-[1-(3-nitro-benzolsulfonyl)-piperidin-4-yl]-1,4-dihydro-benzo[d][1,3]oxazin-2-on |
| 46 | 1-[1-(Biphenyl-4-sulfonyl)-piperidin-4-yl]-6-chlor-1,4-dihydro-benzo[d][1,3]oxazin-2-on |
| 47 | 8-Methyl-1-[1-(2-nitro-benzolsulfonyl)-piperidin-4-yl]-1,4-dihydro-benzo[d][1,3]oxazin-2-on |
| 48 | 8-Methyl-1-[1-(3-nitro-benzolsulfonyl)-piperidin-4-yl]-1,4-dihydro-benzo[d][1,3]oxazin-2-on |
| 49 | 1-[1-(Biphenyl-4-sulfonyl)-piperidin-4-yl]-8-methyl-1,4-dihydro-benzo[d][1,3]oxazin-2-on |
| 50 | 8-Methyl-1-[1-(4-nitro-benzolsulfonyl)-piperidin-4-yl]-1,4-dihydro-benzo[d][1,3]oxazin-2-on |
| 51 | 6-Chlor-1-[1-(4-nitro-benzolsulfonyl)-piperidin-4-yl]-1,4-dihydro-benzo[d][1,3]oxazin-2-on |
| 52 | 1-(1-Ethansulfonyl-piperidin-4-yl)-1,4-dihydro-benzo[d][1,3]oxazin-2-on |
| 53 | 1-[1-(Propan-1-sulfonyl)-piperidin-4-yl]-1,4-dihydro-benzo[d][1,3]oxazin-2-on |
| 54 | 1-[1-(Propan-2-sulfonyl)-piperidin-4-yl]-1,4-dihydro-benzo[d][1,3]oxazin-2-on |
| 55 | 6-Chlor-1-(1-ethansulfonyl-piperidin-4-yl)-1,4-dihydro-benzo[d][1,3]oxazin-2-on |
| 56 | 6-Chlor-1-[1-(propan-1-sulfonyl)-piperidin-4-yl]-1,4-dihydro-benzo[d][1,3]oxazin-2-on |
| 57 | 6-Chlor-1-[1-(propan-2-sulfonyl)-piperidin-4-yl]-1,4-dihydro-benzo[d][1,3]oxazin-2-on |
| 58 | 6-Chlor-1-[1-(chinolin-8-sulfonyl)-piperidin-4-yl]-1,4-dihydro-benzo[d][1,3]oxazin-2-on |
| 59 | 1-[1-(4-Nitro-benzolsulfonyl)-piperidin-4-yl]-1,4-dihydro-benzo[d][1,3]oxazin-2-on |
| 60 | 6-Methyl-1-[1-(chinolin-8-sulfonyl)-piperidin-4-yl]-1,4-dihydro-benzo[d][1,3]oxazin-2-on |
| 61 | 6-Methyl-1-[1-(2-naphthalen-1-yl-ethansulfonyl)-piperidin-4-yl]-1,4-dihydro-benzo[d][1,3]oxazin-2-on |
| 62 | 6-Methyl-1-[1-(toluol-4-sulfonyl)-piperidin-4-yl]-1,4-dihydro-benzo[d][1,3]oxazin-2-on |
| 63 | 1-[1-(4-Fluor-benzolsulfonyl)-piperidin-4-yl]-6-methyl-1,4-dihydro-benzo[d][1,3]oxazin-2-on |
| 64 | 6-Methyl-1-[1-(naphthalen-1-sulfonyl)-piperidin-4-yl]-1,4-dihydro-benzo[d][1,3]oxazin-2-on |
| 65 | 6-Methyl-1-[1-(naphthalen-2-sulfonyl)-piperidin-4-yl]-1,4-dihydro-benzo[d][1,3]oxazin-2-on |
| 66 | 1-[1-(5-Chlor-3-methyl-benzo[b]thiophen-2-sulfonyl)-piperidin-4-yl]-6-methyl-1,4-dihydro-benzo[d][1,3]oxazin-2-on |
| 67 | 6-Methyl-1-[1-(4-nitro-benzolsulfonyl)-piperidin-4-yl]-1,4-dihydro-benzo[d][1,3]oxazin-2-on |
| 68 | 1-(1-Benzolsulfonyl-piperidin-4-yl)-6-methyl-1,4-dihydro-benzo[d][1,3]oxazin-2-on |
| 69 | 1-[1-(4-Chlor-3-nitro-benzolsulfonyl)-piperidin-4-yl]-6-methyl-1,4-dihydro-benzo[d][1,3]oxazin-2-on |
| 70 | 1-[1-(5-Dimethylamino-naphthalen-1-sulfonyl)-piperidin-4-yl]-6-methyl-1,4-dihydro-benzo[d][1,3]oxazin-2-on |
| 71 | 1-[1-(4-Chlor-3-nitro-benzolsulfonyl)-piperidin-4-yl]-8-methyl-1,4-dihydro-benzo[d][1,3]oxazin-2-on |
| 72 | 1-[1-(4-Chlor-3-nitro-benzolsulfonyl)-piperidin-4-yl]-1,4-dihydro-benzo[d][1,3]oxazin-2-on |
| 73 | 6-Chlor-1-[1-(4-chlor-3-nitro-benzolsulfonyl)-piperidin-4-yl]-1,4-dihydro-benzo[d][1,3]oxazin-2-on |
| 74 | 6-Chlor-1-[1-(5-dimethylamino-naphthalen-1-sulfonyl)-piperidin-4-yl]-1,4-dihydro-benzo[d][1,3]oxazin-2-on |
| 75 | 1-[1-(4-Methoxy-2,3,6-trimethyl-benzolsulfonyl)-piperidin-4-yl]-1,4-dihydro-benzo[d][1,3]oxazin-2-on |
| 76 | 1-[1-(4-Methoxy-2,3,6-trimethyl-benzolsulfonyl)-piperidin-4-yl]-8-methyl-1,4-dihydro-benzo[d][1,3]oxazin-2-on |
| 77 | 6-Chlor-1-[1-(4-methoxy-2,3,6-trimethyl-benzolsulfonyl)-piperidin-4-yl]-1,4-dihydro-benzo[d][1,3]oxazin-2-on |
| 78 | 1-[1-(4-Methoxy-2,3,6-trimethyl-benzolsulfonyl)-piperidin-4-yl]-6-methyl-1,4-dihydro-benzo[d][1,3]oxazin-2-on |
| 79 | 1-[1-(2-Brom-benzolsulfonyl)-piperidin-4-yl]-1,4-dihydro-benzo[d][1,3]oxazin-2-on |
| 80 | 1-[1-(2-Brom-benzolsulfonyl)-piperidin-4-yl]-8-methyl-1,4-dihydro-benzo[d][1,3]oxazin-2-on |
| 81 | 1-[1-(2-Brom-benzolsulfonyl)-piperidin-4-yl]-6-chlor-1,4-dihydro-benzo[d][1,3]oxazin-2-on |
| 82 | 1-[1-(2-Brom-benzolsulfonyl)-piperidin-4-yl]-6-methyl-1,4-dihydro-benzo[d][1,3]oxazin-2-on |
| 83 | 6-Chlor-1-[1-(2,3-dichlor-benzolsulfonyl)-piperidin-4-yl]-1,4-dihydro-benzo[d][1,3]oxazin-2-on |
| 84 | 1-[1-(2,3-Dichlor-benzolsulfonyl)-piperidin-4-yl]-6-methyl-1,4-dihydro-benzo[d][1,3]oxazin-2-on |
| 85 | 1-[1-(2,4,5- Trichlor-benzolsulfonyl)-piperidin-4-yl]-1,4-dihydro-benzo[d][1,3]oxazin-2-on |
| 86 | 8-Methyl-1-[1-(2,4,5-trichlor-benzolsulfonyl)-piperidin-4-yl]-1,4-dihydro-benzo[d][1,3]oxazin-2-on |
| 87 | 6-Chlor-1-[1-(2,4,5-trichlor-benzolulfonyl)-piperidin-4-yl]-1,4-dihydro-benzo[d][1,3]oxazin-2-on |
| 88 | 6-Methyl-1-[1-(2,4,5-trichlor-benzolsulfonyl)-piperidin-4-yl]-1,4-dihydro-benzo[d][1,3]oxazin-2-on |
| 89 | 1-[1-(5-Brom-2-methoxy-benzolsulfonyl)-piperidin-4-yl]-1,4-dihydro-benzo[d][1,3]oxazin-2-on |
| 90 | 1-[1-(5-Brom-2-methoxy-benzolsulfonyl)-piperidin-4-yl]-8-methyl-1,4-dihydro-benzo[d][1,3]oxazin-2-on |
| 91 | 1-[1-(5-Brom-2-methoxy-benzolsulfonyl)-piperidin-4-yl]-6-chlor-1,4-dihydro-benzo[d][1,3]oxazin-2-on |
| 92 | 1-[1-(5-Brom-2-methoxy-benzolsulfonyl)-piperidin-4-yl]-6-methyl-1,4-dihydro-benzo[d][1,3]oxazin-2-on |
| 93 | 1-1-(2,5-Dimethoxy-benzolsulfonyl)-piperidin-4-yl]-1,4-dihydro-benzo[d][1,3]oxazin-2-on |
| 94 | 1-[1-(2,5-Dimethoxy-benzolsulfonyl)-piperidin-4-yl]-8-methyl-1,4-dihydro-benzo[d][1,3]oxazin-2-on |
| 95 | 6-Chlor-1-[1-(2,5-dimethoxy-benzolsulfonyl)-piperidin-4-yl]-1,4-dihydro-benzo[d][1,3]oxazin-2-on |
| 96 | 1-[l -(2,5-Dimethoxy-benzolsulfonyl)-piperidin-4-yl]-6-methyl-1,4-dihydro-benzo[d][1,3]oxazin-2-on |
| 97 | 1-(1-Pentamethylbenzolsulfonyl-piperidin-4-yl)-1,4-dihydro-benzo[d][1,3]oxazin-2-on |
| 98 | 8-Methyl-1-benzolsulfonyl-piperidin-4-yl)-1,4-dihydro-benzo[d][1,3]oxazin-2-on |
| 99 | 6-Chlor-1-(1-pentamethylbenzolsulfonyl-piperidin-4-yl)-1,4-dihydro-benzo[d][1,3]oxazin-2-on |
| 100 | 6-Methyl-1-(1-pentamethylbenzolsulfonyl-piperidin-4-yl)-1,4-dihydro-benzo[d][1,3]oxazin-2-on |
| 101 | 1-{1-[2-(2,2,2-Trifluor-acetyl)-1,2,3,4-tetrahydro-isochinolin-7-sulfonyl]-piperidin-4-yl}-1,4-dihydro-benzo[d][1,3]oxazin-2-on |
| 102 | 8-Methyl-1-{1-[2-(2,2,2-trifluor-acetyl)-1,2,3,4-tetrahydro-isochinolin-7-sulfonyl]-piperidin-4-yl}-1,4-dihydro-benzo[d][1,3]oxazin-2-on |
| 103 | 6-Chlor-1-{1-[2-(2,2,2-trifluor-acetyl)-1,2,3,4-tetrahydro-isochinolin-7-sulfonyl]-piperidin- 4-yl}-1,4-dihydro-benzo[d][1,3]oxazin-2-on |
| 104 | 6-Methyl-1-{1-[2-(2,2,2-trifluor-acetyl)-1,2,3,4-tetrahydro-isochinolin-7-sulfonyl]-piperidin-4-yl}-1,4-dihydro-benzo[d][1,3]oxazin-2-on |
| 105 | 1-[1-(2-Methyl-5-nitro-benzolsulfonyl)-piperidin-4-yl]-1,4-dihydro-benzo[d][1,3]oxazin-2-on |
| 106 | 8-Methyl-1-[1-(2-methyl-5-nitro-benzolsulfonyl)-piperidin-4-yl]-1,4-dihydro-benzo[d][1,3]oxazin-2-on |
| 107 | 6-Chlor-1-[1-(2-methyl-5-nitro-benzolsulfonyl)-piperidin-4-yl]-1,4-dihydro-benzo[d][1,3]oxazin-2-on |
| 108 | 6-Methyl-1-[1-(2-methyl-5-nitro-benzolsulfonyl)-piperidin-4-yl]-1,4-dihydro-benzo[d][1,3]oxazin-2-on |
| 109 | 1-[1-(4-Brom-2,5-difluor-benzolsulfonyl)-piperidin-4-yl]-1,4-dihydro-benzo[d][1,3]oxazin-2-on |
| 110 | 1-[1-(4-Brom-2,5-difluor-benzolsulfonyl)-piperidin-4-yl]-8-methyl-1,4-dihydro-benzo[d][1,3]oxazin-2-on |
| 111 | 1-[1-(4-Brom-2,5-difluor-benzolsulfonyl)-piperidin-4-yl]-6-chlor-1,4-dihydro-benzo[d][1,3]oxazin-2-on |
| 112 | 1-[1-(4-Brom-2,5-difluor-benzolsulfonyl)-piperidin-4-yl]-6-methyl-1,4-dihydro-benzo[d][1,3]oxazin-2-on |
| 113 | 1-[1-(4-Chlor-2,5-dimethyl-benzolsulfonyl)-piperidin-4-yl]-1,4-dihydro-benzo[d][1,3]oxazin-2-on |
| 114 | 1-[1-(4-Chlor-2,5-dimethyl-benzolsulfonyl)-piperidin-4-yl]-8-methyl-1,4-dihydro-benzo[d][1,3]oxazin-2-on |
| 115 | 6-Chlor-1-[1-(4-chlor-2,5-dimethyl-benzolsulfonyl)-piperidin-4-yl]-1,4-dihydro-benzo[d][1,3]oxazin-2-on |
| 116 | 1-[1-(4-Chlor-2,5-dimethyl-benzolsulfonyl)-piperidin-4-yl]-6-methyl-1,4-dihydro-benzo[d][1,3]oxazin-2-on |
| 117 | 1[1-(4-Methoxy-benzolsulfonyl)-piperidin-4-yl]-6-methyl-1,4-dihydro-benzo[d][1,3]oxazin-2-on |
| 118 | 1-[1-(4-lsopropyl-benzolsulfonyl)-piperidin-4-yl]-1,4-dihydro-benzo[d][1,3]oxazin-2-on |
| 119 | 1-[1-(4-lsopropyl-benzolsulfonyl)-piperidin-4-yl]-8-methyl-1,4-dihydro-benzo[d][1,3]oxazin-2-on |
| 120 | 6-Chlor-1-[1-(4-isopropyl-benzolsulfonyl)-piperidin-4-yl]-1,4-dihydro-benzo[d][1,3]oxazin-2-on |
| 121 | 1-[1-(4-Isopropyl-benzolsulfonyl)-piperidin-4-yl]-6-methyl-1,4-dihydro-benzo[d][1,3]oxazin-2-on |
| 122 | 1-[-(3-Chlor-4-fluor-benzolsulfonyl)-piperidin-4-yl]-1,4-dihydro-benzo[d][1,3]oxazin-2-on |
| 123 | 1-[1-(3-Chlor-4-fluor-benzolsulfonyl)-piperidin-4-yl]-8-methyl-1,4-dihydro-benzo[d][1,3]oxazin-2-on |
| 124 | 6-Chlor-1-[1-(3-chlor-4-fluor-benzolsulfonyl)-piperidin-4-yl]-1,4-dihydro-benzo[d][1,3]oxazin-2-on |
| 125 | 1-[1-(3-Chlor-4-fluor-benzolsulfonyl)-piperidin-4-yl]-6-methyl-1,4-dihydro-benzo[d][1,3]oxazin-2-on |
| 126 | 1-[1-(4-Brom-benzolsulfonyl)-piperidin-4-yl]-6-methyl-1,4-dihydro-benzo[d][1,3]oxazin-2-on |
| 127 | 6-Methyl-1-[1-(3-nitro-benzolsulfonyl)-piperidin-4-yl]-1,4-dihydro-benzo[d][1,3]oxazin-2-on |
| 128 | 6-Methyl-1-[1-(3-trifluormethyl-benzolsulfonyl)-piperidin-4-yl]-1,4-dihydro-benzo[d][1,3]oxazin-2-on |
| 129 | 1-[1-(4-Trifluormethoxy-benzolsulfonyl)-piperidin-4-yl]-1,4-dihydro-benzo[d][1,3]oxazin-2-on |
| 130 | 1-[1-(2-Nitro-4-trifluormethyl-benzolsulfonyl)-piperidin-4-yl]-1,4-dihydro-benzo[d][1,3]oxazin-2-on |
| 131 | 1-[1-(3-Fluor-benzolsulfonyl)-piperidin-4-yl]-1,4-dihydro-benzo[d][1,3]oxazin-2-on |
| 132 | 1-[1-(2,4-Dichlor-benzolsulfonyl)-piperidin-4-yl]-1,4-dihydro-benzo[d][1,3]oxazin-2-on |
| 133 | 1-[1-(2,4,6-Trimethyl-benzolsulfonyl)-piperidin-4-yl]-1,4-dihydro-benzo[d][1,3]oxazin-2-on |
| 134 | 1-[1-(2-Trifluormethyl-benzolsulfonyl)-piperidin-4-yl]-1,4-dihydro-benzo[d][1,3]oxazin-2-on |
| 135 | 8-Methyl-1-[1-(4-trifluormethoxy-benzolsulfonyl)-piperidin-4-yl]-1,4-dihydro-benzo[d][1,3]oxazin-2-on |
| 136 | 8-Methyl-1-[1-(4-trifluormethoxy-benzolsulfonyl)-piperidin-4-yl]-1,4-dihydro-benzo[d][1,3]oxazin-2-on |
| 137 | 1-[1-(3-Fluor-benzolsulfonyl)-piperidin-4-yl]-8-methyl-1,4-dihydro-benzo[d][1,3]oxazin-2-on |
| 138 | 1-[1-(2,4-Dichlor-benzolsulfonyl)-piperidin-4-yl]-8-methyl-1,4-dihydro-benzo[d][1,3]oxazin-2-on |
| 139 | 8-Methyl-1-[1-(2,4,6-trimethyl-benzolsulfonyl)-piperidin-4-yl]-1,4-dihydro-benzo[d][1,3]oxazin-2-on |
| 140 | 8-Methyl-1-[1-(2-trifluormethyl-benzolsulfonyl)-piperidin-4-yl]-1,4-dihydro-benzo[d][1,3]oxazin-2-on |
| 141 | 1-[1-(4-Fluor-benzolsulfonyl)-piperidin-4-yl]-1,4-dihydro-benzo[d][1,3]oxazin-2-on |
| 142 | 1-[1-(4-Brom-benzolsulfonyl)-piperidin-4-yl]-1,4-dihydro-benzo[d][1,3]oxazin-2-on |
| 143 | 1-[1-(3-Nitro-benzolsulfonyl)-piperidin-4-yl]-1,4-dihydro-benzo[d][1,3]oxazin-2-on |
| 144 | 1-{1-[4-(4-Brom-phenoxy)-benzolsulfonyl]-piperidin-4-yl}-1,4-dihydro-benzo[d][1,3]oxazin-2-on |
| 145 | 1-[1-(3-Methoxy-benzolsulfonyl)-piperidin-4-yl]-1,4-dihydro-benzo[d][1,3]oxazin-2-on |
| 146 | 1-[1-(2-Nitro-benzolsulfonyl)-piperidin-4-yl]-1,4-dihydro-benzo[d][1,3]oxazin-2-on |
| 147 | 8-Methyl-1-[1-(toluol-4-sulfonyl)-piperidin-4-yl]-1,4-dihydro-benzo[d][1,3]oxazin-2-on |
| 148 | 1-(1-Benzolsulfonyl-piperidin-4-yl)-8-methyl-1,4-dihydro-benzo[d][1,3]oxazin-2-on |
| 149 | 1-[1-(3-Methoxy-benzolsulfonyl)-piperidin-4-yl]-8-methyl-1,4-dihydro-benzo[d][1,3]oxazin-2-on |
| 150 | 1-[1-(2,4-Dimethyl-benzolsulfonyl)-piperidin-4-yl]-6-methyl-1,4-dihydro-benzo[d][1,3]oxazin-2-on |
| 151 | {1-[4-(4-Brom-phenoxy)-benzolsulfonyl]-piperidin-4-yl}-6-methyl-1,4-dihydro-benzo[d][1,3]oxazin-2-on |
| 152 | 6-Methyl-1-[1-(thiophen-2-sulfonyl)-piperidin-4-yl]-1,4-dihydro-benzo[d][1,3]oxazin-2-on |
| 153 | 1-[1-(Toluol-3-sulfonyl)-piperidin-4-yl]-1,4-dihydro-benzo[d][1,3]oxazin-2-on |
| 154 | 1[1-(5-Fluor-2-methyl-benzolsulfonyl)-piperidin-4-yl]-1,4-dihydro-benzo[d][1,3]oxazin-2-on |
| 155 | 1-[1-(4-lsopropoxy-benzolsulfonyl)-piperidin-4-yl]-1,4-dihydro-benzo[d][1,3]oxazin-2-on |
| 156 | 1-[1-(3-Chlor-benzolsulfonyl)-piperidin-4-yl]-1,4-dihydro-benzo[d][1,3]oxazin-2-on |
| 157 | 1-[1-(3,4-Dimethoxy-benzolsulfonyl)-piperidin-4-yl]-1,4-dihydro-benzo[d][1,3]oxazin-2-on |
| 158 | 1-(1-Pentafluorbenzolsulfonyl-piperidin-4-yl)-1,4-dihydro-benzo[d][1,3]oxazin-2-on |
| 159 | 8-Methyl-1-[1-(toluol-3-sulfonyl)-piperidin-4-yl]-1,4-dihydro-benzo[d][1,3]oxazin-2-on |
| 160 | 1-[1-(5-Fluor-2-methyl-benzolsulfonyl)-piperidin-4-yl]-8-methyl-1,4-dihydrobenzo[d][1,3]oxazin-2-on |
| 161 | 1-[1-(4-Isopropoxy-benzolsulfonyl)-piperidin-4-yl]-8-methyl-1,4-dihydro-benzo[d][1,3]oxazin-2-on |
| 162 | 1-[1-(3-Chlor-benzolsulfonyl)-piperidin-4-yl]-8-methyl-1,4-dihydro-benzo[d][1,3]oxazin-2-on |
| 163 | 1-[1-(3,4-Dimethoxy-benzolsulfonyl)-piperidin-4-yl]-8-methyl-1,4-dihydro-benzo[d][1,3]oxazin-2-on |
| 164 | 8-Methyl-1-(1-pentafluorbenzolsulfonyl-piperidin-4-yl)-1,4-dihydro-benzo[d][1,3]oxazin-2-on |
| 165 | 6-Methyl-1-[1-(toluol-3-sulfonyl)-piperidin-4-yl]-1,4-dihydro-benzo[d][1,3]oxazin-2-on |
| 166 | 1-[1-(5-Fluor-2-methyl-benzolsulfonyl)-piperidin-4-yl]-6-methyl-1,4-dihydro-benzo[d][1,3]oxazin-2-on |
| 167 | 1-[1-(4-Isopropoxy-benzolsulfonyl)-piperidin-4-yl]-6-methyl-1,4-dihydro-benzo[d][1,3]oxazin-2-on |
| 168 | 1-[1-(3-Chlor-benzolsulfonyl)-piperidin-4-yl]-6-methyl-1,4-dihydro-benzo[d][1,3]oxazin-2-on |
| 169 | 1-[1-(3,4-Dimethoxy-benzolsulfonyl)-piperidin-4-yl]-6-methyl-1,4-dihydro-benzo[d][1,3]oxazin-2-on |
| 170 | 6-Methyl-1-(1-pentafluorbenzolsulfonyl-piperidin-4-yl)-1,4-dihydro-benzo[d][1,3]oxazin-2-on |
| 171 | 6-Methyl-1-[1-(4-trifluormethoxy-benzolsulfonyl)-piperidin-4-yl]-1,4-dihydro-benzo[d][1,3]oxazin-2-on |
| 172 | 6-Methyl-1-[1-(2-nitro-4-trifluormethyl-benzolsulfonyl)-piperidin-4-yl]-1,4-dihydro-benzo[d][1,3]oxazin-2-on |
| 173 | 1-[1-(3-Fluor-benzolsulfonyl)-piperidin-4-yl]-6-methyl-1,4-dihydro-benzo[d][1,3]oxazin-2-on |
| 174 | 1-[1-(2,4-Dichlor-benzolsulfonyl)-piperidin-4-yl]-6-methyl-1,4-dihydro-benzo[d][1,3]oxazin-2-on |
| 175 | 6-Methyl-1-[1-(2,4,6-trimethyl-benzolsulfonyl)-piperidin-4-yl]-1,4-dihydro-benzo[d][1,3]oxazin-2-on |
| 176 | 6-Methyl-1-[1-(2-trifluormethyl-benzolsulfonyl)-piperidin-4-yl]-1,4-dihydro-benzo[d][1,3]oxazin-2-on |
| 177 | 1-[1-(3-Methoxy-benzolsulfonyl)-piperidin-4-yl]-5-methyl-1,4-dihydro-benzo[d][1,3]oxazin-2-on |
| 178 | 6-Methyl-1-[1-(2-nitro-benzolsulfonyl)-piperidin-4-yl]-1,4-dihydro-benzo[d][1,3]oxazin-2-on |
| 179 | 1-[1-(4-Acetyl-benzolsulfonyl)-piperidin-4-yl]-6-methyl-1,4-dihydro-benzo[d][1,3]oxazin-2-on |
| 180 | 1-[1-(4-Methansulfonyl-benzolsulfonyl)-piperidin-4-yl]-6-methyl-1,4-dihydro-benzo[d][1,3]oxazin-2-on |
| 181 | 6-Methyl-1-(1-phenylmethansulfonyl-piperidin-4-yl)-1,4-dihydro-benzo[d][1,3]oxazin-2-on |
| 182 | 2-[4-(6-Methyl-2-oxo-4H-benzo[d][1,3]oxazin-1-yl)-piperidin-1-sulfonyl]benzoesäure-methylester |
| 183 | 6-Methyl-1-[1-(2-oxo-2H-chromen-6-sulfonyl)-piperidin-4-yl]-1,4-dihydro-benzo[d][1,3]oxazin-2-on |
| 184 | 6-Chlor-1-[1-(4-fluor-benzolsulfonyl)-piperidin-4-yl]-1,4-dihydro-benzo[d][1,3]oxazin-2-on |
| 185 | 6-Chlor-1-[1-(3,5-dichlor-benzolsulfonyl)-piperidin-4-yl]-1,4-dihydro-benzo[d][1,3]oxazin-2-on |
| 186 | 1-[{1-[4-(4-Brom-phenoxy)-benzolsulfonyl]-piperidin-4-yl}-6-chlor-1,4-dihydro-benzo[d][1,3]oxazin-2-on |
| 187 | 6-Chlor-1-[1-(thiophen-2-sulfonyl)-piperidin-4-yl]-1,4-dihydro-benzo[d][1,3]oxazin-2-on |
| 188 | 6-Chlor-1-[1-(3-methoxy-benzolsulfonyl)-piperidin-4-yl]-1,4-dihydro-benzo[d][1,3]oxazin-2-on |
| 189 | 6-Chlor-1-[1-(2-oxo-2H-chromen-6-sulfonyl)-piperidin-4-yl]-1,4-dihydro-benzo[d][1,3]oxazin-2-on |
| 190 | 6-Chlor-1-[1-(toluol-3-sulfonyl)-piperidin-4-yl]-1,4-dihydro-benzo[d][1,3]oxazin-2-on |
| 191 | 6-Chlor-1-[1-(5-fluor-2-methyl-benzolsulfonyl)-piperidin-4-yl]-1,4-dihydro-benzo[d][1,3]oxazin-2-on |
| 192 | 6-Chlor-1-[1-(4-isopropoxy-benzolsulfonyl)-piperidin-4-yl]-1,4-dihydro-benzo[d][1,3]oxazin-2-on |
| 193 | 6-Chlor-1-[1-(3-chlor-benzolsulfonyl)-piperidin-4-yl]-1,4-dihydro-benzo[d][1,3]oxazin-2-on |
| 194 | 6-Chlor-1-[1-(3,4-dimethoxy-benzolsulfonyl)-piperidin-4-yl]-1,4-dihydro-benzo[d][1,3]oxazin-2-on |
| 195 | 6-Chlor-1-(1-pentafluorbenzolsulfonyl-piperidin-4-yl)-1,4-dihydro-benzo[d][1,3]oxazin-2-on |
| 196 | 6-Chlor-1-[1-(4-trifluormethoxy-benzolsulfonyl)-piperidin-4-yl]-1,4-dihydro-benzo[d][1,3]oxazin-2-on |
| 197 | 6-Chlor-1-[1-(2-nitro-4-trifluormethyl-benzolsulfonyl)-piperidin-4-yl]-1,4-dihydro-benzo[d][1,3]oxazin-2-on |
| 198 | 6-Chlor-1-[1-(3-fluor-benzolsulfonyl)-piperidin-4-yl]-1,4-dihydro-benzo[d][1,3]oxazin-2-on |
| 199 | 6-Chlor-1-(1-(2,4-dichlor-benzolsulfonyl)-piperidin-4-yl]-1,4-dihydro-benzo[d][1,3]oxazin-2-on |
| 200 | 6-Chlor-1-[1-(2,4,6-trimethyl-benzolsulfonyl)-piperidin-4-yl]-1,4-dihydro-benzo[d][1,3]oxazin-2-on |
| 201 | 6-Chlor-1-[1-(2-trifluormethyl-benzolsulfonyl)-piperidin-4-yl]-1,4-dihydro-benzo[d][1,3]oxazin-2-on |
| 202 | 1-[1-(2-Oxo-2H-chromen-6-sulfonyl)-piperidin-4-yl]-1,4-dihydro-benzo[d][1,3]oxazin-2-on |
| 203 | 1-[1-(3,5-Dichlor-benzolsulfonyl)-piperidin-4-yl]-1,4-dihydro-benzo[d][1,3]oxazin-2-on |
| 204 | 1-[1-(2,5-Dichlor-benzolsulfonyl)-piperidin-4-yl]-1,4-dihydro-benzo[d][1,3]oxazin-2-on |
| 205 | 1-[1-(5-Brom-6-chlor-pyridin-3-sulfonyl)-piperidin-4-yl]-1,4-dihydro-benzo[d][1,3]oxazin- 2-on |
| 206 | 1-[1-(4-Chlor-benzolsulfonyl)-piperidin-4-yl]-1,4-dihydro-benzo[d][1,3]oxazin-2-on |
| 207 | 1-[1-(2,6-Dichlor-benzolsulfonyl)-piperidin-4-yl]-1,4-dihydro-benzo[d][1,3]oxazin-2-on |
| 208 | 8-Methyl-1-[1-(2-oxo-2H-chromen-6-sulfonyl)-piperidin-4-yl]-1,4-dihydro-benzo[d][1,3]oxazin-2-on |
| 209 | 1-[1-(3,5-Dichlor-benzolsulfonyl)-piperidin-4-yl]-8-methyl-1,4-dihydro-benzo[d][1,3]oxazin-2-on |
| 210 | 1-[1-(2,5-Dichlor-benzolsulfonyl)-piperidin-4-yl]-8-methyl-1,4-dihydro-benzo[d][1,3]oxazin-2-on |
| 211 | 1-[1-(5-Brom-6-chlor-pyridin-3-sulfonyl)-piperidin-4-yl]-8-methyl-1,4-dihydro-benzo[d][1,3]oxazin-2-on |
| 212 | 1-[1-(4-Chlor-benzolsulfonyl)-piperidin-4-yl]-8-methyl-1,4-dihydro-benzo[d][1,3]oxazin-2-on |
| 213 | 1-[1-(2,6-Dichlor-benzolsulfonyl)-piperidin-4-yl]-8-methyl-1,4-dihydro-benzo[d][1,3]oxazin-2-on |
| 214 | 1-[1-(Biphenyl-4-sulfonyl)-piperidin-4-yl]-1,4-dihydro-benzo[d][1,3]oxazin-2-on |
| 215 | 6-Chlor-1-[1-(2,5-dichlor-benzolsulfonyl)-piperidin-4-yl]-1,4-dihydro-benzo[d][1,3]oxazin-2-on |
| 216 | 1-[1-(5-Brom-6-chlor-pyridin-3-sulfonyl)-piperidin-4-yl]-6-chlor-1,4-dihydro-benzo[d][1,3]oxazin-2-on |
| 217 | 6-Chlor-1-[1-(4-chlor-benzolsulfonyl)-piperidin-4-yl]-1,4-dihydro-benzo[d][1,3]oxazin-2-on |
| 218 | 6-Chlor-1-[1-(2,6-dichlor-benzolsulfonyl)-piperidin-4-yl]-1,4-dihydro-benzo[d][1,3]oxazin-2-on |
| 219 | 1-[1-(Biphenyl-4-sulfonyl)-piperidin-4-yl]-6-methyl-1,4-dihydro-benzo[d][1,3]oxazin-2-on |
| 220 | 2-[4-(6-Methyl-2-oxo-4H-benzo[d][1,3]oxazin-1-yl)-piperidin-1-sulfonyl]-benzonitril |
| 221 | 1-[1-(2,5-Dichlor-benzolsulfonyl)-piperidin-4-yl]-6-methyl-1,4-dihydro-benzo[d][1,3]oxazin-2-on |
| 222 | 1-[1-(5-Brom-6-chlor-pyridin-3-sulfonyl)-piperidin-4-yl]-6-methyl-1,4-dihydro-benzo[d][1,3]oxazin-2-on |
| 223 | 1-[1-(4-Chlor-benzolsulfonyl)-piperidin-4-yl]-6-methyl-1,4-dihydro-benzo[d][1,3]oxazin-2-on |
| 224 | 1-[1-(2,6-Dichlor-benzolsulfonyl)-piperidin-4-yl]-6-methyl-1,4-dihydro-benzo[d][1,3]oxazin-2-on |
| 225 | 1-[1-(3,5-Dichlor-benzolsulfonyl)-piperidin-4-yl]-6-methyl-1,4-dihydro-benzo[d][1,3]oxazin-2-on |
| 226 | 6-Methyl-1-[1-(1-methyl-1 H-imidazol-4-sulfonyl)-piperidin-4-yl]-1,4-dihydro-benzo[d][1,3]oxazin-2-on |
| 227 | 1-[1-(5-Brom-2,4-difluor-benzolsulfonyl)-piperidin-4-yl]-6-methyl-1,4-dihydro-benzo[d][1,3]oxazin-2-on |
| 228 | 1-[1-(4-Methansulfonyl-benzolsulfonyl)-piperidin-4-yl]-1,4-dihydro-benzo[d][1,3]oxazin-2-on |
| 229 | 1-[1-(1-Methyl-1 H-imidazol-4-sulfonyl)-piperidin-4-yl]-1,4-dihydro-benzo[d][1,3]oxazin-2-on |
| 230 | 1-[1-(5-Brom-2,4-difluor-benzolsulfonyl)-piperidin-4-yl]-1,4-dihydro-benzo[d][1,3]oxazin-2-on |
| 231 | 1-[1-(6-Chlor-imidazo[2,1-b]thiazol-5-sulfonyl)-piperidin-4-yl]-1,4-dihydro-benzo[d][1,3]oxazin-2-on |
| 232 | 1-[1-(4-Ethyl-benzolsulfonyl)-piperidin-4-yl]-1,4-dihydro-benzo[d]1,3]oxazin-2-on |
| 233 | 1-1-(Benzo[b]thiophen-3-sulfonyl)-piperidin-4-yl]-1,4-dihydro-benzo[d][1,3]oxazin-2-on |
| 234 | 1-[1-(6-Chlor-imidazo[2,1-b]thiazol-5-sulfonyl)-piperidin-4-yl]-8-methyl-1,4-dihydro-benzo[d][1,3]oxazin-2-on |
| 235 | 1-[1-(4-Ethyl-benzolsulfonyl)-piperidin-4-yl]-8-methyl-1,4-dihydro-benzo[d][1,3]oxazin-2-on |
| 236 | 1-[1-(Benzo[b]thiophen-3-sulfonyl)-piperidin-4-yl]-8-methyl-1,4-dihydro-benzo[d][1,3]oxazin-2-on |
| 237 | 6-Chlor-1-[1-(6-chlor-imidazo[2,1-b]thiazol-5-sulfonyl)-piperidin-4-yl]-1,4-dihydro-benzo[d][1,3]oxazin-2-on |
| 238 | 6-Chlor-1-[1-(4-ethyl-benzolsulfonyl)-piperidin-4-yl]-1,4-dihydro-benzo[d][1,3]oxazin-2-on |
| 239 | 1-[1-(Benzo[b]thiophen-3-sulfonyl)-piperidin-4-yl]-6-chlor-1,4-dihydro-benzo[d][1,3]oxazin-2-on |
| 240 | 1-[1-(6-Chlor-imidazo[2,1-b]thiazol-5-sulfonyl)-piperidin-4-yl]-6-methyl-1,4-dihydro-benzo[d][1,3]oxazin-2-on |
| 241 | 1-[1-(4-Ethyl-benzolsulfonyl)-piperidin-4-yl]-6-methyl-1,4-dihydro-benzo[d][1,3]oxazin-2-on |
| 242 | 1-[1-(Benzo[b]thiophen-3-sulfonyl)-piperidin-4-yl]-6-methyl-1,4-dihydro-benzo[d][1,3]oxazin-2-on |
| 243 | 1-[1-(7-Chlor-benzo[1,2,5]oxadiazol-4-sulfonyl)-piperidin-4-yl]-1,4-dihydro-benzo[d][1,3]oxazin-2-on |
| 244 | 1-[1-(2-Methoxy-4-methyl-benzolsulfonyl)-piperidin-4-yl]-1,4-dihydro-benzo[d][1,3] oxazin-2-on |
| 245 | 3-(4-[4-(2-Oxo-4H-benzo[d][1,3]oxazin-1-yl)-piperidin-1-sulfonyl]-phenyl)-propionsäure-methylester |
| 246 | 1-[1-(2,4-Dinitro-benzolsulfonyl)-piperidin-4-yl]-1,4-dihydro-benzo[d][1,3]oxazin-2-on |
| 247 | 1-[1-(7-Chlor-benzo[1,2,5]oxadiazol-4-sulfonyl)-piperidin-4-yl]-8-methyl-1,4-dihydro-benzo[d][1,3]oxazin-2-on |
| 248 | 1-[1-(2-Methoxy-4-methyl-benzolsulfonyl)-piperidin-4-yl]-8-methyl-1,4-dihydro-benzo[d][1,3]oxazin-2-on |
| 249 | 3-{4-[4-(8-Methyl-2-oxo-4H-benzo[d][1,3]oxazin-1-yl)-piperidin-1-sulfonyl]-phenyl}-propionsäure-methylester |
| 250 | 1-[1-(2,4-Dinitro-benzolsulfonyl)-piperidin-4-yl]-8-methyl-1,4-dihydro-benzo[d][1,3] oxazin-2-on |
| 251 | 1-[1-(7-Chlor-benzo[1,2,5]oxadiazol-4-sulfonyl)-piperidin-4-yl]-6-methyl-1,4-dihydro-benzo[d][1,3]oxazin-2-on |
| 252 | 1-[1-(2-Methoxy-4-methyl-benzolsulfonyl)-piperidin-4-yl]-6-methyl-1,4-dihydro-benzo[d][1,3]oxazin-2-on |
| 253 | 3-{4-[4-(6-Methyl-2-oxo-4H-benzo[d][1,3]oxazin-1-yl)-piperidin-1-sulfonyl]-phenyl}-propionsäure-methylester |
| 254 | 1-[1-(2,4-Dinitro-benzolsulfonyl)-piperidin-4-yl]-6-methyl-1,4-dihydro-benzo[d][1,3]oxazin-2-on |
| 255 | 6-Chlor-1-[1-(7-chlor-benzo[1,2,5]oxadiazol-4-sulfonyl)-piperidin-4-yl]-1,4-dihydro-benzo[d][1,3]oxazin-2-on |
| 256 | 6-Chlor-1-[1-(2-methoxy-4-methyl-benzolsulfonyl)-piperidin-4-yl]-1,4-dihydro-benzo[d][1,3]oxazin-2-on |
| 257 | 3-{4-[4-(6-Chlor-2-oxo-4H-benzo[d][1,3]oxazin-1-yl)-piperidin-1-sulfonyl]-phenyl}-propionsäure-methylester |
| 258 | 6-Chlor-1-[1-(2,4-dinitro-benzolsulfonyl)-piperidin-4-yl]-1,4-dihydro-benzo[d][1,3]oxazin-2-on |
| 259 | 6-Chlor-1-[1-(1-methyl-1H-imidazol-4-sulfonyl)-piperidin-4-yl]-1,4-dihydro-benzo[d][1,3]oxazin-2-on |
| 260 | 1-[1-(5-Brom-2,4-difluor-benzolsulfonyl)-piperidin-4-yl]-6-chlor-1,4-dihydro-benzo[d][1,3]oxazin-2-on |
| 261 | 8-Methyl-1-[1-(1-methyl-1H-imidazol-4-sulfonyl)-piperidin-4-yl]-1,4-dihydro-benzo[d][1,3]oxazin-2-on |
| 262 | 1-[1-(5-Brom-2,4-difluor-benzolsulfonyl)-piperidin-4-yl]-8-methyl-1,4-dihydro-benzo[d][1,3]oxazin-2-on |
| 263 | 1-[1-(Benzo[b]thiophen-2-sulfonyl)-piperidin-4-yl]-8-methyl-1,4-dihydro-benzo[d][1,3]oxazin-2-on |
| 264 | 1-[1-(Benzo[b]thiophen-2-sulfonyl)-piperidin-4-yl]-1,4-dihydro-benzo[d][1,3]oxazin-2-on |
| 265 | 1-[1-(Benzo[b]thiophen-2-sulfonyl)-piperidin-4-yl]-6-chlor-1,4-dihydro-benzo[d][1,3]oxazin-2-on |
| 266 | 1-[1-(Benzo[b]thiophen-2-sulfonyl)-piperidin-4-yl]-6-methyl-1,4-dihydro-benzo[d][1,3]oxazin-2-on |
| 267 | 1-[1-(2,5-Difluor-benzolsulfonyl)-piperidin-4-yl]-1,4-dihydro-benzo[d][1,3]oxazin-2-on |
| 268 | 1-[1-(2,5-Difluor-benzolsulfonyl)-piperidin-4-yl]-8-methyl-1,4-dihydro-benzo[d][1,3]oxazin-2-on |
| 269 | 6-Chlor-1-[1-(2,5-difluor-benzolsulfonyl)-piperidin-4-yl]-1,4-dihydro-benzo[d][1,3]oxazin-2-on |
| 270 | 1-[1-(2,5-Difluor-benzolsulfonyl)-piperidin-4-yl]-6-methyl-1,4-dihydro-benzo[d][1,3]oxazin-2-on |
| 271 | 1-[1-(4-Chlor-2,5-difluor-benzolsulfonyl)-piperidin-4-yl]-1,4-dihydro-benzo[d][1,3]oxazin-2-on |
| 272 | 1-[1-(4-Chlor-2,5-difluor-benzolsulfonyl)-piperidin-4-yl]-8-methyl-1,4-dihydro-benzo [d][1,3]oxazin-2-on |
| 273 | 6-Chlor-1-[1-(4-chlor-2,5-difluor-benzolsulfonyl)-piperidin-4-yl]-1,4-dihydro-benzo[d][1,3]oxazin-2-on |
| 274 | 1-[1-(4-Chlor-2,5-difluor-benzolsulfonyl)-piperidin-4-yl]-6-methyl-1,4-dihydro-benzo[d][1,3]oxazin-2-on |
| 275 | 1-[1-(2,4,5-Trifluor-benzolsulfonyl)-piperidin-4-yl]-1,4-dihydro-benzo[d][1,3]oxazin-2-on |
| 276 | 8-Methyl-1-[1-(2,4,5-trifluor-benzolsulfonyl)-piperidin-4-yl]-1,4-dihydro-benzo[d][1,3]oxazin-2-on |
| 277 | 6-Chlor-1-[1-(2,4,5-trifluor-benzolsulfonyl)-piperidin-4-yl]-1,4-dihydro-benzo[d][1,3]oxazin-2-on |
| 278 | 6-Methyl-1-[1-(2,4,5-trifluor-benzolsulfonyl)-piperidin-4-yl]-1,4-dihydro-benz[d][1,3]oxazin-2-on |
| 279 | 1-[1-(3,5-Dichlor-2-hydroxy-benzolsulfonyl)-piperidin-4-yl]-1,4-dihydro-benzo[d][1,3]oxazin-2-on |
| 280 | 1-[1-(2,6-Difluor-benzolsulfonyl)-piperidin-4-yl]-1,4-dihydro-benzo[d][1,3]oxazin-2-on |
| 281 | 1-[1-(2,6-Difluor-benzolsulfonyl)-piperidin-4-yl]-8-methyl-1,4-dihydro-benzo[d][1,3]oxazin-2-on |
| 282 | 6-Chlor-1-[1-(2,6-difluor-benzolsulfonyl)-piperidin-4-yl]-1,4-dihydro-benzo[d][1,3]oxazin-2-on |
| 283 | 1-[1-(2,6-Difluor-benzolsulfonyl)-piperidin-4-yl]-6-methyl-1,4-dihydro-benzo[d][1,3]oxazin-2-on |
| 284 | 1-[1-(5-Chlor-2,4-difluor-benzolsulfonyl)-piperidin-4-yl]-1,4-dihydro-benzo[d][1,3]oxazin-2-on |
| 285 | 1-[1-(5-Chlor-2,4-difluor-benzolsulfonyl)-piperidin-4-yl]-8-methyl-1,4-dihydro-benzo[d][1,3]oxazin-2-on |
| 286 | 6-Chlor-1-[1-(5-chlor-2,4-difluor-benzolsulfonyl)-piperidin-4-yl]-1,4-dihydro-benzo[d][1,3]oxazin-2-on |
| 287 | 1-[1-(5-Chlor-2,4-difluor-benzolsulfonyl)-piperidin-4-yl]-6-methyl-1,4-dihydro-benzo[d][1,3]oxazin-2-on |
| 288 | 1-1-(2-Chlor-benzolsulfonyl)-piperidin-4-yl]-1,4-dihydro-benzo[d][1,3]oxazin-2-on |
| 289 | 1-[1-(2-Chlor-benzolsulfonyl)-piperidin-4-yl]-8-methyl-1,4-dihydro-benzo[d][1,3]oxazin-2-on |
| 290 | 6-Chlor-1-[1-(2-chlor-benzolsulfonyl)-piperidin-4-yl]-1,4-dihydro-benzo[d][1,3]oxazin-2-on |
| 291 | 1-[1-(2-Chlor-benzolsulfonyl)-piperidin-4-yl]-6-methyl-1,4-dihydro-benzo[d][1,3]oxazin-2-on |
| 292 | 6-Chlor-1-[1-(2-naphthalen-1-yl-ethansulfonyl)-piperidin-4-yl]-1,4-dihydro-benzo[d][1,3]oxazin-2-on |
| 293 | 6-Brom-1-[1-(4-brom-benzolsulfonyl)-piperidin-4-yl]-1,4-dihydro-benzo[d][1,3]oxazin-2-on |
| 294 | 6-Brom-1-[1-(toluol-4-sulfonyl)-piperidin-4-yl]-1,4-dihydro-benzo[d][1,3]oxazin-2-on |
| 295 | 6-Brom-1-[1-(2,4-dimethyl-benzolsulfonyl)-piperidin-4-yl]-1,4-dihydro-benzo[d][1,3]oxazin-2-on |
| 296 | 6-Brom-1-[1-(2-naphthalen-1-yl-ethansulfonyl)-piperidin-4-yl]-1,4-dihydro-benzo[d][1,3]oxazin-2-on |
| 297 | 6-Brom-1-[1-(chinolin-8-sulfonyl)-piperidin-4-yl]-1,4-dihydro-benzo[d][1,3]oxazin-2-on |
| 298 | 6-Brom-1-[1-(5-chlor-3-methyl-benzo[b]thiophen-2-sulfonyl)-piperidin-4-yl]-1,4-dihydro-benzo[d][1,3]oxazin-2-on |
| 299 | 6-Brom-1-[1-(3-nitro-benzolsulfonyl)-piperidin-4-yl]-1,4-dihydro-benzo[d][1,3]oxazin-2-on |
| 300 | 6-Brom-1-[1-(naphthalen-1-sulfonyl)-piperidin-4-yl]-1,4-dihydro-benzo[d][1,3]oxazin-2-on |
| 301 | 6-Brom-1-[1-(naphthalen-2-sulfonyl)-piperidin-4-yl]-1,4-dihydro-benzo[d][1,3]oxazin-2-on |
| 302 | 1-(1-Benzolsulfonyl-piperidin-4-yl)-6-brom-1,4-dihydro-benzo[d][1,3]oxazin-2-on |
| 303 | 6-Brom-1-{1-[4-(4-brom-phenoxy)-benzolsulfonyl]-piperidin-4-yl}-1,4-dihydro-benzo[d][1,3]oxazin-2-on |
| 304 | 6-Brom-1-[1-(thiophen-2-sulfonyl)-piperidin-4-yl]-1,4-dihydro-benzo[d][1,3]oxazin-2-on |
| 305 | 6-Brom-1-[1-(2-methyl-5-nitro-benzolsulfonyl)-piperidin-4-yl]-1,4-dihydro-benzo[d][1,3]oxazin-2-on |
| 306 | 6-Brom-1-[1-(4-brom-2,5-difluor-benzolsulfonyl)-piperidin-4-yl]-1,4-dihydro-benzo[d][1,3]oxazin-2-on |
| 307 | 6-Brom-1-[1-(toluol-3-sulfonyl)-piperidin-4-yl]-1,4-dihydro-benzo[d][1,3]oxazin-2-on |
| 308 | 6-Brom-1-[1-(5-fluor-2-methyl-benzolsulfonyl)-piperidin-4-yl]-1,4-dihydro-benzo[d][1,3]oxazin-2-on |
| 309 | 6-Brom-1-[1-(4-isopropoxy-benzolsulfonyl)-piperidin-4-yl]-1,4-dihydro-benzo[d][1,3]oxazin-2-on |
| 310 | 6-Brom-1-[1-(3-chlor-benzolsulfonyl)-piperidin-4-yl]-1,4-dihydro-benzo[d][1,3]oxazin-2-on |
| 311 | 6-Brom-1-[1-(3,4-dimethoxy-benzolsulfonyl)-piperidin-4-yl]-1,4-dihydro-benzo[d][1,3]oxazin-2-on |
| 312 | 6-Brom-1-(1-pentafluorbenzolsulfonyl-piperidin-4-yl)-1,4-dihydro-benzo[d][1,3]oxazin-2-on |
| 313 | 6-Brom-1-[1-(4-chlor-2,5-dimethyl-benzolsulfonyl)-piperidin-4-yl]-1,4-dihydro-benzo[d][1,3]oxazin-2-on |
| 314 | 6-Brom-1-[1-(3-methoxy-benzolsulfonyl)-piperidin-4-yl]-1,4-dihydro-benzo[d][1,3]oxazin-2-on |
| 315 | 6-Brom-1-[1-(4-isopropyl-benzolsulfonyl)-piperidin-4-yl]-1,4-dihydro-benzo[d][1,3]oxazin-2-on |
| 316 | 6-Brom-1-[1-(4-fluor-benzolsulfonyl)-piperidin-4-yl]-1,4-dihydro-benzo[d][1,3]oxazin-2-on |
| 317 | 6-Brom-1-[1-(3-chlor-4-fluor-benzolsulfonyl)-piperidin-4-yl]-1,4-dihydro-benzo[d][1,3]oxazin-2-on |
| 318 | 6-Brom-1-(1-pentamethylbenzolsulfonyl-piperidin-4-yl)-1,4-dihydro-benzo[d][1,3]oxazin-2-on |
| 319 | 6-Brom-1-[1-(2-nitro-benzolsulfonyl)-piperidin-4-yl]-1,4-dihydro-benzo[d][1,3]oxazin-2-on |
| 320 | 6-Brom-1-[1-(4-chlor-3-nitro-benzolsulfonyl)-piperidin-4-yl]-1,4-dihydro-benzo[d][1,3]oxazin-2-on |
| 321 | 6-Brom-1-[1-(5-dimethylamino-naphthalen-1-sulfonyl)-piperidin-4-yl]-1,4-dihydro-benzo[d][1,3]oxazin-2-on |
| 322 | 6-Brom-1-[1-(4-nitro-benzolsulfonyl)-piperidin-4-yl]-1,4-dihydro-benzo[d][1,3]oxazin-2-on |
| 323 | 1-[1-(4-Acetyl-benzolsulfonyl)-piperidin-4-yl]-6-brom-1,4-dihydro-benzo[d][1,3]oxazin-2-on |
| 324 | 6-Brom-1-[1-(4-methansulfonyl-benzolsulfonyl)-piperidin-4-yl]-1,4-dihydro-benzo[d][1,3]oxazin-2-on |
| 325 | 1-[1-(Biphenyl-4-sulfonyl)-piperidin-4-yl]-6-brom-1,4-dihydro-benzo[d][1,3]oxazin-2-on |
| 326 | 6-Brom-1-(1-phenylmethansulfonyl-piperidin-4-yl)-1,4-dihydro-benzo[d][1,3]oxazin-2-on |
| 327 | 6-Brom-1-[1-(2,5-dimethoxy-benzolsulfonyl)-piperidin-4-yl]-1,4-dihydro-benzo[d][1,3]oxazin-2-on |
| 328 | 6-Brom-1-{-[2-(2,2,2-trifluor-acetyl)-1,2,3,4-tetrahydro-isochinolin-7-sulfonyl]-piperidin-4-yl}-1,4-dihydro-benzo[d][1,3]oxazin-2-on |
| 329 | 6-Brom-1-[1-(2,3-dichlor-benzolsulfonyl)-piperidin-4-yl]-1,4-dihydro-benzo[d][1,3]oxazin-2-on |
| 330 | 6-Brom-1-[1-(2,4,5-trichlor-benzolsulfonyl)-piperidin-4-yl]-1,4-dihydro-benzo[d][1,3]oxazin-2-on |
| 331 | 6-Brom-1-[1-(5-brom-2-methoxy-benzolsulfonyl)-piperidin-4-yl]-1,4-dihydro-benzo[d][1,3]oxazin-2-on |
| 332 | 6-Brom-1-[1-(4-trifluormethoxy-benzolsulfonyl)-piperidin-4-yl]-1,4-dihydro-benzo[d][1,3]oxazin-2-on |
| 333 | 6-Brom-1-[1-(2-nitro-4-trifluormethyl-benzolsulfonyl)-piperidin-4-yl]-1,4-dihydro-benzo[d][1,3]oxazin-2-on |
| 334 | 6-Brom-1-[1-(3-fluor-benzolsulfonyl)-piperidin-4-yl]-1,4-dihydro-benzo[d][1,3]oxazin-2-on |
| 335 | 6-Brom-1-[1-(2,4-dichlor-benzolsulfonyl)-piperidin-4-yl]-1,4-dihydro-benzo[d][1,3]oxazin-2-on |
| 336 | 6-Brom-1-[1-(2,4,6-trimethyl-benzolsulfonyl)-piperidin-4-yl]-1,4-dihydro-benzo[d][1,3]oxazin-2-on |
| 337 | 6-Brom-1-[1-(2-trifluormethyl-benzolsulfonyl)-piperidin-4-yl]-1,4-dihydro-benzo[d][1,3]oxazin-2-on |
| 338 | 6-Brom-1-[1-(2-brom-benzolsulfonyl)-piperidin-4-yl]-1,4-dihydro-benzo[d][1,3]oxazin-2-on |
| 339 | 6-Brom-1-[1-(4-methoxy-2,3,6-trimethyl-benzolsulfonyl)-piperidin-4-yl]-1,4-dihydro-benzo[d][1,3]oxazin-2-on |
| 340 | 1-[1-(3,5-Dichlor-4-hydroxy-benzolsulfonyl)-piperidin-4-yl]-1,4-dihydro-benzo[d][1,3]oxazin-2-on |
| 341 | 1-[1-(3,5-Dichlor-4-hydroxy-benzolsulfonyl)-piperidin-4-yl]-8-methyl-1,4-dihydro-benzo[d][1,3]oxazin-2-on |
| 342 | 6-Chlor-1-[1-(3,5-dichlor-4-hydroxy-benzolsulfonyl)-piperidin-4-yl]-1,4-dihydro-benzo[d][1,3]oxazin-2-on |
| 343 | 1-[1-(3,5-Dichlor-4-hydroxy-benzolsulfonyl)-piperidin-4-yl]-6-methyl-1,4-dihydro-benzo[d][1,3]oxazin-2-on |
| 344 | 6-Brom-1-[1-(3,5-dichlor-4-hydroxy-benzolsulfonyl)-piperidin-4-yl]-1,4-dihydro-benzo[d][1,3]oxazin-2-on |
| 345 | 6-Chlor-1-[1-(3,5-dichlor-2-hydroxy-benzolsulfonyl)-piperidin-4-yl]-1,4-dihydro-benzo[d][1,3]oxazin-2-on |
| 346 | 6-Brom-1-[1-(3,5-dichlor-2-hydroxy-benzolsulfonyl)-piperidin-4-yl]-1,4-dihydro-benzo[d][1,3]oxazin-2-on |
| 347 | 2-[4-(6-Brom-2-oxo-4H-benzo[d][1,3]oxazin-2-yl)-piperidin-1-sulfonyl]-benzonitril |
| 348 | 6-Brom-1-[1-(4-methoxy-benzolsulfonyl)-piperidin-4-yl]-1,4-dihydro-benzo[d][1,3]oxazin-2-on |
| 349 | 2-[4-(6-Brom-2-oxo-4H-benzo[d][1,3]oxazin-1-yl)-piperidin-1-sulfonyl]-benzoesäure-methylester |
| 350 | 6-Brom-1-[1-(3-trifluormethyl-benzolsulfonyl)-piperidin-4-yl]-1,4-dihydro-benzo[d][1,3]oxazin-2-on |
| 351 | 6-Brom-1-[1-(2-oxo-2H-chromen-6-sulfonyl)-piperidin-4-yl]-1,4-dihydro-benzo[d][1,3]oxazin-2-on |
| 352 | 6-Brom-1-[1-(3,5-dichlor-benzolsulfonyl)-piperidin-4-yl]-1,4-dihydro-benzo[d][1,3]oxazin-2-on |
| 353 | 6-Brom-1-[1-(2,5-dichlor-benzolsulfonyl)-piperidin-4-yl]-1,4-dihydro-benzo[d][1,3]oxazin-2-on |
| 354 | 6-Brom-1-[1-(5-brom-6-chlor-pyridin-3-sulfonyl)-piperidin-4-yl]-1,4-dihydro-benzo[d][1,3]oxazin-2-on |
| 355 | 6-Brom-1-[1-(4-chlor-benzolsulfonyl)-piperidin-4-yl]-1,4-dihydro-benzo[d][1,3]oxazin-2-on |
| 356 | 6-Brom-1-[1-(2,6-dichlor-benzolsulfonyl)-piperidin-4-yl]-1,4-dihydro-benzo[d][1,3]oxazin-2-on |
| 357 | 6-Brom-1-[1-(1-methyl-1H-imidazol-4-sulfonyl)-piperidin-4-yl]-1,4-dihydro-benzo[d][1,3]oxazin-2-on |
| 358 | 6-Brom-1-[1-(5-brom-2,4-difluor-benzolsulfonyl)-piperidin-4-yl]-1,4-dihydro-benzo[d][1,3]oxazin-2-on |
| 359 | 6-Brom-1-[1-(4-ethyl-benzolsulfonyl)-piperidin-4-yl]-1,4-dihydro-benzo[d][1,3]oxazin-2-on |
| 360 | 6-Brom-1-[1-(6-chlor-imidazo[2,1-b]thiazol-5-sulfonyl)-piperidin-4-yl]-1,4-dihydro-benzo[d][1,3]oxazin-2-on |
| 361 | 1-[1-(Benzo[b]thiophen-3-sulfonyl)-piperidin-4-yl]-6-brom-1,4-dihydro-benzo[d][1,3]oxazin-2-on |
| 362 | 6-Brom-1-[1-(7-chlor-benzo[1,2,5]oxadiazol-4-sulfonyl)-piperidin-4-yl]-1,4-dihydro-benzo[d][1,3]oxazin-2-on |
| 363 | 6-Brom-1-[1-(2-methoxy-4-methyl-benzolsulfonyl)-piperidin-4-yl]-1,4-dihydro-benzo[d][1,3]oxazin-2-on |
| 364 | 3-{4-[4-(6-Brom-2-oxo-4H-benzo[d][1,3]oxazin-1-yl)-piperidin-1-sulfonyl]-phenyl}-propionsäure-methylester |
| 365 | 6-Brom-1-[1-(2,4-dinitro-benzolsulfonyl)-piperidin-4-yl]-1,4-dihydro-benzo[d][1,3]oxazin-2-on |
| 366 | 1-[1-(Benzo[b]thiophen-2-sulfonyl)-piperidin-4-yl]-6-brom-1,4-dihydro-benzo[d][1,3]oxazin-2-on |
| 367 | 6-Brom-1-[1-(2,5-difluor-benzolsulfonyl)-piperidin-4-yl]-1,4-dihydro-benzo[d][1,3]oxazin-2-on |
| 368 | 6-Brom-1-[1-(4-chlor-2,5-difluor-benzolsulfonyl)-piperidin-4-yl]-1,4-dihydro-benzo[d][1,3]oxazin-2-on |
| 369 | 6-Brom-1-[1-(2,4,5-trifluor-benzolsulfonyl)-piperidin-4-yl]-1,4-dihydro-benzo[d][1,3]oxazin-2-on |
| 370 | 6-Brom-1-[1-(2,6-difluor-benzolsulfonyl)-piperidin-4-yl]-1,4-dihydro-benzo[d][1,3]oxazin-2-on |
| 371 | 6-Brom-1-[1-(5-chlor-2,4-difluor-benzolsulfonyl)-piperidin-4-yl]-1,4-dihydro-benzo[d][1,3]oxazin-2-on |
| 372 | 6-Brom-1-[1-(2-chlor-benzolsulfonyl)-piperidin-4-yl]-1,4-dihydro-benzo[d][1,3]oxazin-2-on |
| 373 | 6-Brom-1-[1-(2,3,4-trifluor-benzolsulfonyl)-piperidin-4-yl]-1,4-dihydro-benzo[d][1,3]oxazin-2-on |
| 374 | N-{4-[4-(6-Brom-2-oxo-4H-benzo[d][1,3]oxazin-1-yl)-piperidin-1-sulfonyl]-2-chlor-phenyl}-acetamid |
| 375 | 1-[1-(2,3,4-Trifluor-benzolsulfonyl)-piperidin-4-yl]-1,4-dihydro-benzo[d][1,3]oxazin-2-on |
| 376 | 8-Methyl-1-[1-(2,3,4-trifluor-benzolsulfonyl)-piperidin-4-yl]-1,4-dihydro-benzo[d][1,3]oxazin-2-on |
| 377 | 6-Chlor-1-[1-(2,3,4-trifluor-benzolsulfonyl)-piperidin-4-yl]-1,4-dihydro-benzo[d][1,3]oxazin-2-on |
| 378 | 6-Methyl-1-[1-(2,3,4-trifluor-benzolsulfonyl)-piperidin-4-yl]-1,4-dihydro-benzo[d][1,3]oxazin-2-on |
| 379 | N-{2-Chlor-4-[4-(6-methyl-2-oxo-4H-benzo[d][1,3]oxazin-1-yl)-piperidin-1-sulfonyl]-phenyl}-acetamid |
| 380 | 1-[1-(3,4-Difluor-benzolsulfonyl)-piperidin-4-yl]-1,4-dihydro-benzo[d][1,3]oxazin-2-on |
| 381 | 1-[1-(3,4-Difluor-benzolsulfonyl)-piperidin-4-yl]-8-methyl-1,4-dihydro-benzo[d][1,3]oxazin-2-on |
| 382 | 6-Chlor-1-[1-(3,4-difluor-benzolsulfonyl)-piperidin-4-yl]-1,4-dihydro-benzo[d][1,3]oxazin-2-on |
| 383 | 1-[1-(3,4-Difluor-benzolsulfonyl)-piperidin-4-yl]-6-methyl-1,4-dihydro-benzo[d][1,3]oxazin-2-on |
| 384 | 6-Brom-1-[1-(3,4-difluor-benzolsulfonyl)-piperidin-4-yl]-1,4-dihydro-benzo[d][1,3]oxazin-2-on |
| 385 | N-{2-Chlor-4-[4-(8-methyl-2-oxo-4H-benzo[d][1,3]oxazin-1-yl)-piperidin-1-sulfonyl]-phenyl)-acetamid |
| 386 | 1-[1-(2-Chlor-4,5-difluor-benzolsulfonyl)-piperidin-4-yl]-1,4-dihydro-benzo[d][1,3]oxazin-2-on |
| 387 | 1-[1-(2-Chlor-4,5-difluor-benzolsulfonyl)-piperidin-4-yl]-8-methyl-1,4-dihydro-benzo[d][1,3]oxazin-2-on |
| 388 | 6-Chlor-1-[1-(2-chlor-4,5-difluor-benzolsulfonyl)-piperidin-4-yl]-1,4-dihydro-benzo[d][1,3]oxazin-2-on |
| 389 | 1-[1-(2-Chlor-4,5-difluor-benzolsulfonyl)-piperidin-4-yl]-6-methyl-1,4-dihydro-benzo[d][1,3]oxazin-2-on |
| 390 | 6-Brom-1-[1-(2-chlor-4,5-difluor-benzolsulfonyl)-piperidin-4-yl]-1,4-dihydro-benzo[d][1,3]oxazin-2-on |
| 391 | N-{2-Chlor-4-[4-(2-oxo-4H-benzo[d][1,3]oxazin-1-yl)-pipendin-1-sulfonyl]-phenyl}-acetamid |
| 392 | 1-[1-(Benzo[1,2,5]oxadiazol-4-sulfonyl)-piperidin-4-yl]-1,4-dihydro-benzo[d][1,3]oxazin-2-on |
| 393 | 1-[1-(Benzo[1,2,5]oxadiazol-4-sulfonyl)-piperidin-4-yl]-8-methyl-1,4-dihydro-benzo[d][1,3]oxazin-2-on |
| 394 | 1-[1-(Benzo[1,2,5]oxadiazol-4-sulfonyl)-piperidin-4-yl]-6-chlor-1,4-dihydro-benzo[d][1,3]oxazin-2-on |
| 395 | 1-[1-(Benzo[1,2,5]oxadiazol-4-sulfonyl)-piperidin-4-yl]-6-methyl-1,4-dihydro-benzo[d][1,3]oxazin-2-on |
| 396 | 1-[1-(Benzo[1,2,5]oxadiazol-4-sulfonyl)-piperidin-4-yl]-6-brom-1,4-dihydro-benzo[d][1,3]oxazin-2-on |
| 397 | N-{2-Chlor-4-[4-(6-chlor-2-oxo-4H-benzo[d][1,3]oxazin-1-yl)-piperidin-1-sulfonyl]-phenyl}-acetamid |
| 398 | 1-[1-(Benzo[1,2,5]thiadiazol-4-sulfonyl)-piperidin-4-yl]-1,4-dihydro-benzo[d][1,3]oxazin-2-on |
| 399 | 1-[1-(Benzo[1,2,5]thiadiazol-4-sulfonyl)-piperidin-4-yl]-8-methyl-1,4-dihydro-benzo[d][1,3]oxazin-2-on |
| 400 | 1-[1-(Benzo[1,2,5]thiadiazol-4-sulfonyl)-piperidin-4-yl]-6-chlor-1,4-dihydro-benzo[d][1,3]oxazin-2-on |
| 401 | 1-[1-(Benzo[1,2,5]thiadiazol-4-sulfonyl)-piperidin-4-yl]-6-methyl-1,4-dihydro-benzo[d][1,3]oxazin-2-on |
| 402 | 1-[1-(Benzo[1,2,5]thiadiazol-4-sulfonyl)-piperidin-4-yl]-6-brom-1,4-dihydro-benzo[d][1,3]oxazin-2-on |
| 403 | 1-(1-Ethansulfonyl-piperidin-4-yl)-8-methyl-1,4-dihydro-benzo[d][1,3]oxazin-2-on |
| 404 | 1-[1-(2,4-Difluor-benzolsulfonyl)-piperidin-4-yl]-1,4-dihydro-benzo[d][1,3]oxazin-2-on |
| 405 | 1-[1-(2,4-Difluor-benzolsulfonyl)-piperidin-4-yl]-8-methyl-1,4-dihydro-benzo[d][1,3]oxazin-2-on |
| 406 | 6-Chlor-1-[1-(2,4-difluor-benzolsulfonyl)-piperidin-4-yl]-1,4-dihydro-benzo[d][1,3]oxazin-2-on |
| 407 | 1-[1-(2,4-Difluor-benzolsulfonyl)-piperidin-4-yl]-6-methyl-1,4-dihydro-benzo[d][1,3]oxazin-2-on |
| 408 | 6-Brom-1-[1-(2,4-difluor-benzolsulfonyl)-piperidin-4-yl]-1,4-dihydro-benzo[d][1,3]oxazin-2-on |
| 409 | 8-Methyl-1-[1-(propan-2-sulfonyl)-piperidin-4-yl]-1,4-dihydro-benzo[d][1,3]oxazin-2-on |
| 410 | 1-[1-(3,4-Dichlor-benzolsulfonyl)-piperidin-4-yl]-1,4-dihydro-benzo[d][1,3]oxazin-2-on |
| 411 | 1-[1-(3,4-Dichlor-benzolsulfonyl)-piperidin-4-yl]-8-methyl-1,4-dihydro-benzo[d][1,3]oxazin-2-on |
| 412 | 6-Chlor-1-[1-(3,4-dichlor-benzolsulfonyl)-piperidin-4-yl]-1,4-dihydro-benzo[d][1,3]oxazin-2-on |
| 413 | 1-[1-(3,4-Dichlor-benzolsulfonyl)-piperidin-4-yl]-6-methyl-1,4-dihydro-benzo[d][1,3]oxazin-2-on |
| 414 | 6-Brom-1-[1-(3,4-dichlor-benzolsulfonyl)-piperidin-4-yl]-1,4-dihydro-benzo[d][1,3]oxazin-2-on |
| 415 | 8-Methyl-1-[1-(propan-1-sulfonyl)-piperidin-4-yl]-1,4-dihydro-benzo[d][1,3]oxazin-2-on |
| 416 | 1-[1-(2-Chlor-6-methyl-benzolsulfonyl)-piperidin-4-yl]-1,4-dihydro-benzo[d][1,3]oxazin-2-on |
| 417 | 1-[1-(2-Chlor-6-methyl-benzolsulfonyl)-piperidin-4-yl]-8-methyl-1,4-dihydro-benzo[d][1,3]oxazin-2-on |
| 418 | 6-Chlor-1-[1-(2-chlor-6-methyl-benzolsulfonyl)-piperidin-4-yl]-1,4-dihydro-benzo[d][1,3]oxazin-2-on |
| 419 | 1-[1-(2-Chlor-6-methyl-benzolsulfonyl)-piperidin-4-yl]-6-methyl-1,4-dihydro-benzo[d][1,3]oxazin-2-on |
| 420 | 1-[1-(2-Chlor-6-methyl-benzolsulfonyl)-piperidin-4-yl]-6-methyl-1,4-dihydro-benzo[d][1,3]oxazin-2-on |
| 421 | 8-Methyl-1-[1-(2,3,5,6-tetramethyl-benzolsulfonyl)-piperidin-4-yl]-1,4-dihydro-benzo[d][1,3]oxazin-2-on |
| 422 | 1-[1-(2,3,4-Trichlor-benzolsulfonyl)-piperidin-4-yl]-1,4-dihydro-benzo[d][1,3]oxazin-2-on |
| 423 | 8-Methyl-1-[1-(2,3,4-trichlor-benzolsulfonyl)-piperidin-4-yl]-1,4-dihydro-benzo[d][1,3] oxazin-2-on |
| 424 | 6-Chlor-1-[1-(2,3,4-trichlor-benzolsulfonyl)-piperidin-4-yl]-1,4-dihydro-benzo[d][1,3]oxazin-2-on |
| 425 | 6-Methyl-1-[1-(2,3,4-trichlor-benzolsulfonyl)-piperidin-4-yl]-1,4-dihydro-benzo[d][1,3]oxazin-2-on |
| 426 | 6-Brom-1-[1-(2,3,4-trichlor-benzolsulfonyl)-piperidin-4-yl]-1,4-dihydro-benzo[d][1,3]oxazin-2-on |
| 427 | 1-[1-(2,3,5,6-Tetramethyl-benzolsulfonyl)-piperidin-4-yl]-1,4-dihydro-benzo[d][1,3]oxazin-2-on |
| 428 | 1-[1-(Thiophen-3-sulfonyl)-piperidin-4-yl]-1,4-dihydro-benzo[d][1,3]oxazin-2-on |
| 429 | 8-Methyl-1-[1-(thiophen-3-sulfonyl)-piperidin-4-yl]-1,4-dihydro-benzo[d][1,3]oxazin-2-on |
| 430 | 6-Chlor-1-[1-(thiophen-3-sulfonyl)-piperidin-4-yl]-1,4-dihydro-benzo[d][1,3]oxazin-2-on |
| 431 | 6-Methyl-1-[1-(thiophen-3-sulfonyl)-piperidin-4-yl]-1,4-dihydro-benzo[d][1,3]oxazin-2-on |
| 432 | 6-Brom-1-[1-(thiophen-3-sulfonyl)-piperidin-4-yl]-1,4-dihydro-benzo[d][1,3]oxazin-2-on |
| 433 | 6-Chlor-1-[1-(2,3,5,6-tetramethyl-benzolsulfonyl)-piperidin-4-yl]-1,4-dihydro-benzo[d][1,3]oxazin-2-on |
| 434 | 1-[1-(2,4,6- Trichlor-benzolsulfonyl)-piperidin-4-yl]-1,4-dihydro-benzo[d][1,3]oxazin-2-on |
| 435 | 8-Methyl-1-[1-(2,4,6-trichlor-benzolsulfonyl)-piperidin-4-yl]-1,4-dihydro-benzo[d][1,3]oxazin-2-on |
| 436 | 6-Chlor-1-[1-(2,4,6-trichlor-benzolsulfonyl)-piperidin-4-yl]-1,4-dihydro-benzo[d][1,3]oxazin-2-on |
| 437 | 6-Methyl-1-[1-(2,4,6-trichlor-benzolsulfonyl)-piperidin-4-yl]-1,4-dihydro-benzo[d][1,3]oxazin-2-on |
| 438 | 6-Brom-1-[1-(2,4,6-trichlor-benzolsulfonyl)-piperidin-4-yl]-1,4-dihydro-benzo[d][1,3]oxazin-2-on |
| 439 | 6-Methyl-1-[1-(2,3,5,6-tetramethyl-benzolsulfonyl)-piperidin-4-yl]-1,4-dihydro-benzo[d][1,3]oxazin-2-on |
| 440 | 1-[1-(2-Brom-4,6-difluor-benzolsulfonyl)-piperidin-4-yl]-8-methyl-1,4-dihydro-benzo[d][1,3]oxazin-2-on |
| 441 | 1-[1-(2-Brom-4,6-difluor-benzolsulfonyl)-piperidin-4-yl]-6-chlor-1,4-dihydro-benzo[d][1,3]oxazin-2-on |
| 442 | 1-[1-(2-Brom-4,6-difluor-benzolsulfonyl)-piperidin-4-yl]-6-methyl-1,4-dihydro-benzo[d][1,3]443oxazin-2-on |
| 443 | 6-Brom-1-[1-(2-brom-4,6-difluor-benzolsulfonyl)-piperidin-4-yl]-1,4-dihydro-benzo[d][1,3]oxazin-2-on |
| 444 | 6-Brom-1-[1-(2,3,5,6-tetramethyl-benzolsulfonyl)-piperidin-4-yl]-1,4-dihydro-benzo[d][1,3]oxazin-2-on |
| 445 | 1-[1-(4-Brom-2-trifluormethoxy-benzolsulfonyl)-piperidin-4-yl]-1,4-dihydro-benzo[d][1,3]oxazin-2-on |
| 446 | 1-1-(4-Brom-2-trifluormethoxy-benzolsulfonyl)-piperidin-4-yl]-8-methyl-1,4-dihydro-benzo[d][1,3]oxazin-2-on |
| 447 | 1-1-(4-Brom-2-trifluormethoxy-benzolsulfonyl)-piperidin-4-yl]-6-chlor-1,4-dihydro-benzo[d][1,3]oxazin-2-on |
| 448 | 1-[1-(4-Brom-2-trifluormethoxy-benzolsulfonyl)-piperidin-4-yl]-6-methyl-1,4-dihydro-benzo[d][1,3]oxazin-2-on |
| 449 | 6-Brom-1-[1-(4-brom-2-trifluormethoxy-benzolsulfonyl)-piperidin-4-yl]-1,4-dihydro-benzo[d][1,3]oxazin-2-on |
| 450 | 1-[1-(4-Phenoxy-benzolsulfonyl)-piperidin-4-yl]-1,4-dihydro-benzo[d][1,3]oxazin-2-on |
| 451 | 1-[1-(3-Brom-benzolsulfonyl)-piperidin-4-yl]-1,4-dihydro-benzo[d][1,3]oxazin-2-on |
| 452 | 1-[1-(3-Brom-benzolsulfonyl)-piperidin-4-yl]-8-methyl-1,4-dihydro-benzo[d][1,3]oxazin-2-on |
| 453 | 1-[1-(3-Brom-benzolsulfonyl)-piperidin-4-yl]-6-chlor-1,4-dihydro-benzo[d][1,3]oxazin-2-on |
| 454 | 1-[1-(3-Brom-benzolsulfonyl)-piperidin-4-yl]-6-methyl-1,4-dihydro-benzo[d][1,3]oxazin-2-on |
| 455 | 6-Brom-1-[1-(3-brom-benzolsulfonyl)-piperidin-4-yl]-1,4-dihydro-benzo[d][1,3]oxazin-2-on |
| 456 | 8-Methyl-1-[1-(4-phenoxy-benzolsulfonyl)-piperidin-4-yl]-1,4-dihydro-benzo[d][1,3]oxazin-2-on |
| 457 | 1-[1-(4-tert-Butyl-benzolsulfonyl)-piperidin-4-yl]-1,4-dihydro-benzo[d][1,3]oxazin-2-on |
| 458 | 1-[1-(4-tert-Butyl-benzolsulfonyl)-piperidin-4-yl]-8-methyl-1,4-dihydro-benzo[d][1,3]oxazin-2-on |
| 459 | 1-[1-(4-tert-Butyl-benzolsulfonyl)-piperidin-4-yl]-6-chlor-1,4-dihydro-benzo[d][1,3]oxazin-2-on |
| 460 | 1-[1(4-tert-Butyl-benzolsulfonyl)-piperidin-4-yl]-6-methyl-1,4-dihydro-benzo[d][1,3]oxazin-2-on |
| 461 | 6-Brom-1-[1-(4-tert-butyl-benzolsulfonyl)-piperidin-4-yl]-1,4-dihydro-benzo[d][1,3]oxazin-2-on |
| 462 | 6-Chlor-1-[1-(4-phenoxy-benzolsulfonyl)-piperidin-4-yl]-1,4-dihydro-benzo[d][1,3]oxazin-2-on |
| 463 | 1-[1-(2-Brom-4,6-difluor-benzolsulfonyl)-piperidin-4-yl]-1 ,4-dihydro-benzo[d][1,3]oxazin-2-on |
| 464 | 1-[1-(2-Methansulfonyl-benzolsulfonyl)-piperidin-4-yl]-8-methyl-1,4-dihydro-benzo[d][1,3]oxazin-2-on |
| 465 | 6-Chlor-1-[1-(2-methansulfonyl-benzolsulfonyl)-piperidin-4-yl]-1,4-dihydro-benzo[d][1,3]oxazin-2-on |
| 466 | 1-[1-(2-Methansulfonyl-benzolsulfonyl)-piperidin-4-yl]-6-methyl-1,4-dihydro-benzo[d][1,3]oxazin-2-on |
| 467 | 6-Brom-1-[1-(2-methansulfonyl-benzolsulfonyl)-piperidin-4-yl]-1,4-dihydro-benzo[d][1,3]oxazin-2-on |
| 468 | 8-Methyl-1-[1-(4-propyl-benzolsulfonyl)-piperidin-4-yl]-1,4-dihydro-benzo[d][1,3]oxazin-2-on |
| 469 | 6-Chlor-1-[1-(4-propyl-benzolsulfonyl)-piperidin-4-yl]-1,4-dihydro-benzo[d][1,3]oxazin-2-on |
| 470 | 6-Methyl-1-[1-(4-propyl-benzolsulfonyl)-piperidin-4-yl]-1,4-dihydro-benzo[d][1,3]oxazin-2-on |
| 471 | 6-Brom-1-[1-(4-propyl-benzolsulfonyl)-piperidin-4-yl]-1,4-dihydro-benzo[d][1,3]oxazin-2-on |
| 472 | 1-[1-(3-Chlor-2-methyl-benzolsulfonyl)-piperidin-4-yl]-8-methyl-1,4-dihydro-benzo[d][1,3]oxazin-2-on |
| 473 | 6-Chlor-1-[1-(3-chlor-2-methyl-benzolsulfonyl)-piperidin-4-yl]-1,4-dihydro-benzo[d][1,3]oxazin-2-on |
| 474 | 1-[1-(3-Chlor-2-methyl-benzolsulfonyl)-piperidin-4-yl]-6-methyl-1,4-dihydro-benzo[d][1,3]oxazin-2-on |
| 475 | 6-Brom-1-[1-(3-chlor-2-methyl-benzolsulfonyl)-piperidin-4-yl]-1,4-dihydro-benzo[d][1,3]oxazin-2-on |
| 476 | 1-[1-(4-Butyl-benzolsulfonyl)-piperidin-4-yl]-8-methyl-1,4-dihydro-benzo[d][1,3]oxazin-2-on |
| 477 | 1-[1-(4-Butyl-benzolsulfonyl)-piperidin-4-yl]-6-chlor-1,4-dihydro-benzo[d][1,3]oxazin-2-on |
| 478 | 1-[1-(4-Butyl-benzolsulfonyl)-piperidin-4-yl]-6-methyl-1,4-dihydro-benzo[d][1,3]oxazin-2-on |
| 479 | 6-Brom-1-[1-(4-butyl-benzolsulfonyl)-piperidin-4-yl]-1,4-dihydro-benzo[d][1,3]oxazin-2-on |
| 480 | 1-[1-(4-Brom-3-methyl-benzolsulfonyl)-piperidin-4-yl]-8-methyl-1,4-dihydro-benzo[d][1,3]oxazin-2-on |
| 481 | 1-[1-(4-Brom-3-methyl-benzolsulfonyl)-piperidin-4-yl]-6-chlor-1,4-dihydro-benzo[d][1,3]oxazin-2-on |
| 482 | 1-[1-(4-Brom-3-methyl-benzolsulfonyl)-piperidin-4-yl]-6-methyl-1,4-dihydro-benzo[d][1,3]oxazin-2-on |
| 483 | 6-Brom-1-[1-(4-brom-3-methyl-benzolsulfonyl)-piperidin-4-yl]-1,4-dihydro-benzo[d][1,3]oxazin-2-on |
| 484 | 1-{1-[4-(1,1-Dimethyl-propyl)-benzolsulfonyl]-piperidin-4-yl}-8-methyl-1,4-dihydro-benzo[d][1,3]oxazin-2-on |
| 485 | 6-Chlor-1-{1-[4-(1,1-dimethyl-propyl)-benzolsulfonyl]-piperidin-4-yl}-1,4-dihydro-benzo[d][1,3]oxazin-2-on |
| 486 | 1-{1-[4-(1,1-Dimethyl-propyl)-benzolsulfonyl]-piperidin-4-yl}-6-methyl-1,4-dihydro-benzo[d][1,3]oxazin-2-on |
| 487 | 6-Brom-1-{1-[4-(1,1-dimethyl-propyl)-benzolsulfonyl]-piperidin-4-yl}-1,4-dihydro-benzo[d][1,3]oxazin-2-on |
| 488 | 1-(1-Ethensulfonyl-piperidin-4-yl)-8-methyl-1,4-dihydro-benzo[d][1,3]oxazin-2-on |
| 489 | 3-[4-(8-Methyl-2-oxo-4H-benzo[d][1,3]oxazin-2-yl)-piperidin-1-sulfonyl]-benzoesäure |
| 490 | 3-[4-(6-Methyl-2-oxo-4H-benzo[d][1,3]oxazin-2-yl)-piperidin-1-sulfonyl]-benzoesäure |
| 491 | 3-[4-(6-Brom-2-oxo-4H-benzo[d][1,3]oxazin-1-yl)-piperidin-1-sulfonyl]-benzoesäure |
| 492 | 1-[1-(3-Chlor-2-fluor-benzolsulfonyl)-piperidin-4-yl]-8-methyl-1,4-dihydro-benzo[d][1,3]oxazin-2-on |
| 493 | 6-Chlor-1-[1-(3-chlor-2-fluor-benzolsulfonyl)-piperidin-4-yl]-1,4-dihydro-benzo[d][1,3]oxazin-2-on |
| 494 | 1-[1-(3-Chlor-2-fluor-benzolsulfonyl)-piperidin-4-yl]-6-methyl-1,4-dihydro-benzo[d][1,3]oxazin-2-on |
| 495 | 6-Brom-1-[1-(3-chlor-2-fluor-benzolsulfonyl)-piperidin-4-yl]-1,4-dihydro-benzo[d][1,3]oxazin-2-on |
| 496 | N-{4-Methyl-5-[4-(8-methyl-2-oxo-4H-benzo[d][1,3]oxazin-1-yl)-piperidin-1-sulfonyl]-thiazol-2-yl}-acetamid |
| 497 | N-{5-[4-(6-Ch!or-2-oxo-4H-benzo[d][1,3]oxazin-1-yl)-piperidin-1-sulfonyl]-4-methyl-thiazol-2-yl}-acetamid |
| 498 | N-{4-Methyl-5-[4-(6-methyl-2-oxo-4H-benzo[d][1,3]oxazin-1-yl)-piperidin-1-sulfonyl]-thiazol-2-yl}-acetamid |
| 499 | N-{5-[4-(6-Brom-2-oxo-4H-benzo[d][1,3]oxazin-1-yl)-piperidin-1-sulfonyl]-4-methyl-thiazol-2-yl}-acetamid |
| 500 | 1-[1-(2-Brom-4-fluor-benzolsulfonyl)-piperidin-4-yl]-8-methyl-1,4-dihydro-benzo[d]1,3]oxazin-2-on |
| 501 | 1-[1-(2-Brom-4-fluor-benzolsulfonyl)-piperidin-4-yl]-6-chlor-1,4-dihydro-benzo[d][1,3]oxazin-2-on |
| 502 | 1-[1-(2-Brom-4-fluor-benzolsulfonyl)-piperidin-4-yl]-6-methyl-1,4-dihydro-benzo[d][1,3]oxazin-2-on |
| 503 | 6-Brom-1-[1-(2-brom-4-fluor-benzolsulfonyl)-piperidin-4-yl]-1,4-dihydro-benzo[d][1,3]oxazin-2-on |
| 504 | 1-[1-(5-Chlor-2-fluor-benzolsulfonyl)-piperidin-4-yl]-8-methyl-1,4-dihydro-benzo[d][1,3]oxazin-2-on |
| 505 | 6-Chlor-1-[1-(5-chlor-2-fluor-benzolsulfonyl)-piperidin-4-yl]-1,4-dihydro-benzo[d][1,3]oxazin-2-on |
| 506 | 1-1-(5-Chlor-2-fluor-benzolsulfonyl)-piperidin-4-yl]-6-methyl-1,4-dihydro-benzo[d][1,3]oxazin-2-on |
| 507 | 6-Brom-1-[1-(5-chlor-2-fluor-benzolsulfonyl)-piperidin-4-yl]-1,4-dihydro-benzo[d][1,3]oxazin-2-on |
| 508 | 1-[1-(4-Brom-3-trifluormethyl-benzolsulfonyl)-piperidin-4-yl]-8-methyl-1,4-dihydro-benzo[d][1,3]oxazin-2-on |
| 509 | 1-[1-(4-Brom-3-trifluormethyl-benzolsulfonyl)-piperidin-4-yl]-6-chlor-1,4-dihydro-benzo[d][1,3]oxazin-2-on |
| 510 | 1-[1-(4-Brom-3-trifluormethyl-benzolsulfonyl)-piperidin-4-yl]-6-methyl-1,4-dihydro-benzo[d][1,3]oxazin-2-on |
| 511 | 6-Brom-1-[1-(4-brom-3-trifluormethyl-benzolsulfonyl)-piperidin-4-yl]-1,4-dihydro-benzo[d][1,3]oxazin-2-on |
| 512 | 1-[1-(2-Methansulfonyl-benzolsulfonyl)-piperidin-4-yl]-1,4-dihydro-benzo[d][1,3]oxazin-2-on |
| 513 | 1-[1-(4-Propyl-benzolsulfonyl)-piperidin-4-yl]-1,4-dihydro-benzo[d][1,3]oxazin-2-on |
| 514 | 1-[1-(3-Chlor-2-methyl-benzolsulfonyl)-piperidin-4-yl]-1,4-dihydro-benzo[d][1,3]oxazin-2-on |
| 515 | 1-[1-(4-Butyl-benzolsulfonyl)-piperidin-4-yl]-1,4-dihydro-benzo[d][1,3]oxazin-2-on |
| 516 | 1-[1-(4-Brom-3-methyl-benzolsulfonyl)-piperidin-4-yl]-1,4-dihydro-benzo[d][1,3]oxazin-2-on |
| 517 | 1-{1-[4-(1,1-Dimethyl-propyl)-benzolsulfonyl]-piperidin-4-yl}-1,4-dihydro-benzo[d][1,3]oxazin-2-on |
| 518 | N-{4-Methyl-5-[4-(2-oxo-4H-benzo[d][1,3]oxazin-1-yl)-piperidin-1-sulfonyl]-thiazol-2-yl}-acetamid |
| 519 | 1-[1-(3-Chlor-2-fluor-benzolsulfonyl)-piperidin-4-yl]-1,4-dihydro-benzo[d][1,3]oxazin-2-on |
| 520 | 1-[1-(2-Brom-4-fluor-benzolsulfonyl)-piperidin-4-yl]-1,4-dihydro-benzo[d][1,3]oxazin-2-on |
| 521 | 1-[1-(4-Brom-3-trifluormethyl-benzolsulfonyl)-piperidin-4-yl]-1,4-dihydro-benzo[d][1,3]oxazin-2-on |
| 522 | 1-[1-(5-Chlor-2-fluor-benzolsulfonyl)-piperidin-4-yl]-1,4-dihydro-benzo[d][1,3]oxazin-2-on |
| 523 | 1-[1-(Isochinolin-5-sulfonyl)-piperidin-4-yl]-1,4-dihydro-benzo[d][1,3]oxazin-2-on |
| 524 | 6-Fluor-1-[1-(2-methansulfonyl-benzolsulfonyl)-piperidin-4-yl]-1,4-dihydro-benzo[d][1,3]oxazin-2-on |
| 525 | 6-Fluor-1-[1-(4-propyl-benzolsulfonyl)-piperidin-4-yl]-1,4-dihydro-benzo[d][1,3]oxazin-2-on |
| 526 | 1-[1-(3-Chlor-2-methyl-benzolsulfonyl)-piperidin-4-yl]-6-fluor-1,4-dihydro-benzo[d][1,3]oxazin-2-on |
| 527 | 1-[1-(4-Butyl-benzolsulfonyl)-piperidin-4-yl]-6-fluor-1,4-dihydro-benzo[d][1,3]oxazin-2-on |
| 528 | 1-[1-(4-Brom-3-methyl-benzolsulfonyl)-piperidin-4-yl]-6-fluor-1,4-dihydro-benzo[d][1,3]oxazin-2-on |
| 529 | 1-{1-[4-(1,1-Dimethyl-propyl)-benzolsulfonyl]-piperidin-4-yl}-6-fluor-1,4-dihydro-benzo[d][1,3]oxazin-2-on |
| 530 | N-{5-[4-(6-Fluor-2-oxo-4H-benzo[d][1,3]oxazin-1-yl)-piperidin-1-sulfonyl]-4-methyl-thiazol-2-yl}-acetamid |
| 531 | 1-[1-(3-Chlor-2-fluor-benzolsulfonyl)-piperidin-4-yl]-6-fluor-1,4-dihydro-benzo[d][1,3]oxazin-2-on |
| 532 | 1-[1-(2-Brom-4-fluor-benzolsulfonyl)-piperidin-4-yl]-6-fluor-1,4-dihydro-benzo[d][1,3]oxazin-2-on |
| 533 | 1-[1-(4-Brom-3-trifluormethyl-benzolsulfonyl)-piperidin-4-yl]-6-fluor-1,4-dihydro-benzo[d][1,3]oxazin-2-on |
| 534 | 1-[1-(5-Chlor-2-fluor-benzolsulfonyl)-piperidin-4-yl]-6-fluor-1,4-dihydro-benzo[d][1,3]oxazin-2-on |
| 535 | 6-Fluor-1-[1-(isochinolin-5-sulfonyl)-piperidin-4-yl]-1,4-dihydro-benzo[d][1,3]oxazin-2-on |
| 536 | 6-Fluor-1-[1-(chinolin-8-sulfonyl)-piperidin-4-yl]-1,4-dihydro-benzo[d][1,3]oxazin-2-on |
| 537 | 1-[1-(5-Chlor-3-methyl-benzo[b]thiophen-2-sulfonyl)-piperidin-4-yl]-6-fluor-1,4-dihydro-benzo[d][1,3]oxazin-2-on |
| 538 | 6-Fluor-1-[1-(naphthalen-1-sulfonyl)-piperidin-4-yl]-1,4-dihydro-benzo[d][1,3]oxazin-2-on |
| 539 | 6-Fluor-1-[1-(naphthalen-2-sulfonyl)-piperidin-4-yl]-1,4-dihydro-benzo[d][1,3]oxazin-2-on |
| 540 | 1-[1-(Benzo[b]thiophen-2-sulfonyl)-piperidin-4-yl]-6-fluor-1,4-dihydro-benzo[d][1,3]oxazin-2-on |
| 541 | 1-[1-(Benzo[b]thiophen-3-sulfonyl)-piperidin-4-yl]-6-fluor-1,4-dihydro-benzo[d][1,3]oxazin-2-on |
| 542 | 8-Methoxy-1-[1-(chinolin-8-sulfonyl)-piperidin-4-yl]-1,4-dihydro-benzo[d][1,3]oxazin-2-on |
| 543 | 1-[1-(5-Chlor-3-methyl-benzo[b]thiophen-2-sulfonyl)-piperidin-4-yl]-8-methoxy-1,4-dihydro-benzo[d][1,3]oxazin-2-on |
| 544 | 8-Methoxy-1-(1-(naphthalen-1-sulfonyl)-piperidin-4-yl]-1,4-dihydro-benzo[d][1,3]oxazin-2-on |
| 545 | 8-Methoxy-1-(1-(naphthalen-2-sulfonyl)-piperidin-4-yl]-1,4-dihydro-benzo[d][1,3]oxazin-2-on |
| 546 | 1-[1-(Benzo[b]thiophen-2-sulfonyl)-piperidin-4-yl]-8-methoxy-1,4-dihydro-benzo[d][1,3]oxazin-2-on |
| 547 | 1-[1-(Benzo[b]thiophen-3-sulfonyl)-piperidin-4-yl]-8-methoxy-1,4-dihydro-benzo[d][1,3]oxazin-2-on |
| 548 | 5-Chlor-1-[1-(2-methansulfonyl-benzolsulfonyl)-piperidin-4-yl]-1,4-dihydro-benzo[d][1,3]oxazin-2-on |
| 549 | 5-Chlor-1-[1-(4-propyl-benzolsulfonyl)-piperidin-4-yl]-1,4-dihydro-benzo[d][1,3]oxazin-2-on |
| 550 | 5-Chlor-1-[1-(3-chlor-2-methyl-benzolsulfonyl)-piperidin-4-yl]-1,4-dihydro-benzo[d][1,3]oxazin-2-on |
| 551 | 1-[1-(4-Butyl-benzolsulfonyl)-piperidin-4-yl]-5-chlor-1,4-dihydro-benzo[d][1,3]oxazin-2-on |
| 552 | 1-[1-(4-Brom-3-methyl-benzolsulfonyl)-piperidin-4-yl]-5-chlor-1,4-dihydro-benzo[d][1,3]oxazin-2-on |
| 553 | 5-Chlor-1-{1-[4-(1,1-dimethyl-propyl)-benzolsulfonyl]-piperidin-4-yl}-1,4-dihydro-benzo[d][1,3]oxazin-2-on |
| 554 | N-{5-[4-(5-Chlor-2-oxo-4H-benzo[d][1,3]oxazin-1-yl)-piperidin-1-sulfonyl]-4-methyl-thiazol-2-yl}-acetamid |
| 555 | 5-Chlor-1-[1-(3-chlor-2-fluor-benzolsulfonyl)-piperidin-4-yl]-1,4-dihydro-benzo[d][1,3]oxazin-2-on |
| 556 | 1-[1-(2-Brom-4-fluor-benzolsulfonyl)-piperidin-4-yl]-5-chlor-1,4-dihydro-benzo[d][1,3]oxazin-2-on |
| 557 | 1-[1-(4-Brom-3-trifluormethyl-benzolsulfonyl)-piperidin-4-yl]-5-chlor-1,4-dihydro-benzo[d][1,3]oxazin-2-on |
| 558 | 5-Chlor-1-[1-(5-chlor-2-fluor-benzolsulfonyl)-piperidin-4-yl]-1,4-dihydro-benzo[d][1,3]oxazin-2-on |
| 559 | 5-Chlor-1-[1-(isochinolin-5-sulfonyl)-piperidin-4-yl]-1,4-dihydro-benzo[d][1,3]oxazin-2-on |
| 560 | 1-[1-(2-Methansulfonyl-benzolsulfonyl)-piperidin-4-yl]-8-methoxy-1,4-dihydro-benzo[d][1,3]oxazin-2-on |
| 561 | 1-[1-(2-Methansulfonyl-benzolsulfonyl)-piperidin-4-yl]-8-methoxy-1,4-dihydro-benzo[d][1,3]oxazin-2-on |
| 562 | 1-[1-(3-Chlor-2-methyl-benzolsulfonyl)-piperidin-4-yl]-8-methoxy-1,4-dihydro-benzo[d][1,3]oxazin-2-on |
| 563 | 1-[1-(4-Butyl-benzolsulfonyl)-piperidin-4-yl]-8-methoxy-1,4-dihydro-benzo[d][1,3]oxazin-2-on |
| 564 | 1-1-(4-Brom-3-methyl-benzolsulfonyl)-piperidin-4-yl]-8-methoxy-1,4-dihydro-benzo[d][1,3]oxazin-2-on |
| 565 | 1-{1-[4-(1,1-Dimethyl-propyl)-benzolsulfonyl]-piperidin-4-yl}-8-methoxy-1,4-dihydro-benzo[d][1,3]oxazin-2-on |
| 566 | N-{5-[4-(8-Methoxy-2-oxo-4H-benzo[d][1,3]oxazin-1-yl)-piperidin-1-sulfonyl]-4-methyl-thiazol-2-yl}-acetamid |
| 567 | 1-[1-(3-Chlor-2-fluor-benzolsulfonyl)-piperidin-4-yl]-8-methoxy-1,4-dihydro-benzo[d][1,3]oxazin-2-on |
| 568 | 1-[1-(2-Brom-4-fluor-benzolsulfonyl)-piperidin-4-yl]-8-methoxy-1,4-dihydro-benzo[d][1,3]oxazin-2-on |
| 569 | 1-[1-(4-Brom-3-trifluormethyl-benzolsulfonyl)-piperidin-4-yl]-8-methoxy-1,4-dihydro-benzo[d][1,3]oxazin-2-on |
| 570 | 1-[1-(5-Chlor-2-fluor-benzolsulfonyl)-piperidin-4-yl]-8-methoxy-1,4-dihydro-benzo[d][1,3]oxazin-2-on |
| 571 | 1-[1-(Isochinolin-5-sulfonyl-piperidin-4-yl]-8-methoxy-1,4-dihydro-benzo[d][1,3]oxazin-2-on; Hydrochlorid |
| 572 | 1-[1-(4-Methyl-naphthalen-1-sulfonyl)-piperidin-4-yl]-1,4-dihydro-benzo[d]1,3]oxazin-2-on |
| 573 | 6-Chlor-1-[1-(4-methyl-naphthalen-1-sulfonyl)-piperidin-4-yl]-1,4-dihydro-benzo[d][1,3]oxazin-2-on |
| 574 | 6-Methyl-1-[1-(4-methyl-naphthalen-1-sulfonyl)-piperidin-4-yl]-1,4-dihydro-benzo[d][1,3]oxazin-2-on |
| 575 | 8-Methyl-1-[1-(4-methyl-naphthalen-1-sulfonyl)-piperidin-4-yl]-1,4-dihydro-benzo[d][1,3]oxazin-2-on |
| 576 | 6-Fluor-1-[1-(4-methyl-naphthalen-1-sulfonyl)-piperidin-4-yl]-1,4-dihydro-benzo[d][1,3]oxazin-2-on |
| 577 | 8-Methoxy-1-[1-(4-methyl-naphthalen-1-sulfonyl)-piperidin-4-yl]-1,4-dihydro-benzo[d][1,3]oxazin-2-on |
| 578 | 5-Chlor-1-[1-(4-methyl-naphthalen-1-sulfonyl)-piperidin-4-yl]-1,4-dihydro-benzo[d][1,3]oxazin-2-on |
| 579 | 5-Chlor-1-[1-(naphthalen-1-sulfonyl)-piperidin-4-yl]-1,4-dihydro-benzo[d][1,3]oxazin-2-on |
| 580 | 5-Chlor-1-[1-(naphthalen-2-sulfonyl)-piperidin-4-yl]-1,4-dihydro-benzo[d][1,3]oxazin-2-on |
| 581 | 5-Chlor-1-[1-(chinolin-8-sulfonyl)-piperidin-4-yl]-1,4-dihydro-benzo[d][1,3]oxazin-2-on |
| 582 | 5-Chlor-1-[1-(5-chlor-3-methyl-benzo[b]thiophen-2-sulfonyl)-piperidin-4-yl]-1,4-dihydro-benzo[d][1,3]oxazin-2-on |
| 583 | 1-[1-(Benzo[b]thiophen-2-sulfonyl)-piperidin-4-yl]-5-chlor-1,4-dihydro-benzo[d][1,3]oxazin-2-on |
| 584 | 1-[1-(Benzo[b]thiophen-3-sulfonyl)-piperidin-4-yl]-5-chlor-1,4-dihydro-benzo[d][1,3]oxazin-2-on |
| 585 | 6-Brom-1-[1-(4-methyl-naphthalen-1-sulfonyl)-piperidin-4-yl]-1,4-dihydro-benzo[d][1,3]oxazin-2-on |
| 586 | 2-Chlor-4-fluor-5-[4-(8-methyl-2-oxo-4H-benzo[d][1,3]oxazin-1-yl)-piperidin-1-sulfonyl]-benzoesäure |
| 587 | 2-Chlor-5-[4-(6-chlor-2-oxo-4H-benzo[d][1,3]oxazin-1-yl)-piperidin-1-sulfonyl]-4-fluor-benzoesäure |
| 588 | 2-Chlor-4-fluor-5-[4-(6-methyl-2-oxo-4H-benzo[d][1,3]oxazin-1-yl)-piperidin-1-sulfonyl]-benzoesäure |
| 589 | 2-Chlor-4-fluor-5-[4-(2-oxo-4H-benzo[d][1,3]oxazin-1-yl)-piperidin-1-sulfonyl]-benzoesäure |
| 590 | 2-Chlor-4-fluor-5-[4-(8-methoxy-2-oxo-4H-benzo[d][1,3]oxazin-1-yl)-piperidin-1-sulfonyl]-benzoesäure |
| 591 | 2-Chlor-5-[4-(5-chlor-2-oxo-4H-benzo[d][1,3]oxazin-1-yl)-piperidin-1-sulfonyl]-4-fluor-benzoesäure |
| 592 | 3-[4-(2-Oxo-4H-benzo[d][1,3]oxazin-1-yl)-piperidin-1-sulfonyl]-benzoesäure |
| 593 | 3-[4-(8-Methoxy-2-oxo-4H-benzo[d][1,3]oxazin-1-yl)-piperidin-1-sulfonyl]-benzoesäure |
| 594 | 3-[4-(5-Chlor-2-oxo-4H-benzo[d][1,3]oxazin-1-yl)-piperidin-1-sulfonyl]-benzoesäure |
| 595 | 1-[1-(Isochinolin-5-sulfonyl)-piperidin-4-yl]-8-methyl-1,4-dihydro-benzo[d][1,3]oxazin-2-on; Hydrochlorid |
| 596 | 6-Chlor-1-[1-(isochinolin-5-sulfonyl)-piperidin-4-yl]-1,4-dihydro-benzo[d][1,3]oxazin-2-on;Hydrochlorid |
| 597 | 1-[1-(Isochinolin-5-sulfonyl)-piperidin-4-yl]-6-methyl-1,4-dihydro-benzo[d][1,3]oxazin-2-ons; Hydrochloride |
| 598 | 6,7-Difluor-1-[1-(chinolin-8-sulfonyl)-piperidin-4-yl]-1,4-dihydro-benzo[d][1,3]oxazin-2-on |
| 599 | 1-[1-(5-Chlor-3-methyl-benzo[b]thiophen-2-sulfonyl)-piperidin-4-yl]-6,7-difluor-1,4-dihydro-benzo[d][1,3]oxazin-2-on |
| 600 | 6,7-Difluor-1-[1-(naphthalen-1-sulfonyl)-piperidin-4-yl]-1,4-dihydro-benzo[d][1,3]oxazin-2-on |
| 601 | 6,7-Difluor-1-[1-(naphthalen-2-sulfonyl)-piperidin-4-yl]-1,4-dihydro-benzo[d][1,3]oxazin-2-on |
| 602 | 1-[1-(Benzo[b]thiophen-2-sulfonyl)-piperidin-4-yl]-6,7-difluor-1,4-dihydro-benzo[d][1,3]oxazin-2-on |
| 603 | 1-[1-(Benzo[b]thiophen-3-sulfonyl)-piperidin-4-yl]-6,7-difluor-1,4-dihydro-benzo[d][1,3]oxazin-2-on |
| 604 | 1-[1-(5-Dimethylamino-naphthalen-1-sulfonyl)-piperidin-4-yl]-6,7-difluor-1,4-dihydro-benzo[d][1,3]oxazin-2-on |
| 605 | 1-[1-(Biphenyl-4-sulfonyl)-piperidin-4-yl]-6,7-difluor-1,4-dihydro-benzo[d][1,3]oxazin-2-on |
| 606 | 1-[1-(Benzo[1,2,5]thiadiazol-4-sulfonyl)-piperidin-4-yl]-6,7-difluor-1,4-dihydro-benzo[d][1,3]oxazin-2-on |
| 607 | 1-[1-(Benzo[1,2,5]oxadiazol-4-sulfonyl)-piperidin-4-yl]-6,7-difluor-1,4-dihydro-benzo[d][1,3]oxazin-2-on |
| 608 | 1-[1-(7-Chlor-benzo[1,2,5]oxadiazol-4-sulfonyl)-piperidin-4-yl]-6,7-difluor-1,4-dihydro-benzo[d][1,3]oxazin-2-on |
| 609 | 6,7-Difluor-1-[1-(4-methyl-naphthalen-1-sulfonyl)-piperidin-4-yl]-1,4-dihydro-benzo[d][1,3]oxazin-2-on |
| 610 | 1-[1-(4-Chlor-naphthalen-1-sulfonyl)-piperidin-4-yl]-1,4-dihydro-benzo[d][1,3]oxazin-2-on |
| 611 | 1-[1-(4-Fluor-naphthalen-1-sulfonyl)-piperidin-4-yl]-1,4-dihydro-benzo[d][1,3]oxazin-2-on |
| 612 | 1-[1-(Dibenzofuran-2-sulfonyl)-piperidin-4-yl]-1,4-dihydro-benzo[d][1,3]oxazin-2-on |
| 613 | 1-[1-(2,3-Dihydro-benzofuran-5-sulfonyl)-piperidin-4-yl]-1,4-dihydro-benzo[d][1,3]oxazin-2-on |
| 614 | 1-[1-(Biphenyl-2-sulfonyl)-piperidin-4-yl]-1,4-dihydro-benzo[d][1,3]oxazin-2-on |
| 615 | 1-[1-(5-Isoxazol-5-yl-thiophen-2-sulfonyl)-piperidin-4-yl]-1,4-dihydro-benzo[d][1,3]oxazin-2-on |
| 616 | [1-(4-Chlor-naphthalen-1-sulfonyl)-piperidin-4-yl]-8-methyl-1,4-dihydro-benzo[d][1,3]oxazin-2-on |
| 617 | [1-(4-Fluor-naphthalen-1-sulfonyl)-piperidin-4-yl]-8-methyl-1,4-dihydro-benzo[d][1,3]oxazin-2-on |
| 618 | 1-[1-(Dibenzofuran-2-sulfonyl)-piperidin-4-yl]-8-methyl-1,4-dihydro-benzo[d][1,3]oxazin-2-on |
| 619 | 1-[1-(2,3-Dihydro-benzofuran-5-sulfonyl)-piperidin-4-yl]-8-methyl-1,4-dihydro-benzo[d][1,3]oxazin-2-on |
| 620 | 1-[1-(Biphenyl-2-sulfonyl)-piperidin-4-yl]-8-methyl-1,4-dihydro-benzo[d][1,3]oxazin-2-on |
| 621 | 1-[1-(5-Isoxazol-5-yl-thiophen-2-sulfonyl)-piperidin-4-yl]-8-methyl-1,4-dihydro-benzo[d][1,3]oxazin-2-on |
| 622 | 5-Chlor-1-[1-(4-chlor-naphthalen-1-sulfonyl)-piperidin-4-yl]-1,4-dihydro-benzo[d][1,3]oxazin-2-on |
| 623 | 5-Chlor-1-[1-(4-fluor-naphthalen-1-sulfonyl)-piperidin-4-yl]-1,4-dihydro-benzo[d][1,3]oxazin-2-on |
| 624 | 5-Chlor-1-[1-(dibenzofuran-2-sulfonyl)-piperidin-4-yl]-1,4-dihydro-benzo[d][1,3]oxazin-2-on |
| 625 | 5-Chlor-1-[1-(2,3-dihydro-benzofuran-5-sulfonyl)-piperidin-4-yl]-1,4-dihydro-benzo[d][1,3]oxazin-2-on |
| 626 | 1-[1-(Biphenyl-2-sulfonyl)-piperidin-4-yl]-5-chlor-1,4-dihydro-benzo[d][1,3]oxazin-2-on |
| 627 | 5-Chlor-1-[1-(5-isoxazol-5-yl-thiophen-2-sulfonyl)-piperidin-4-yl]-1,4-dihydro-benzo[d][1,3]oxazin-2-on |
| 628 | 1-[1-(4-Chlor-naphthalen-1-sulfonyl)-piperidin-4-yl]-8-methoxy-1,4-dihydro-benzo[d][1,3]oxazin-2-on |
| 629 | 1-[1-(4-Fluor-naphthalen-1-sulfonyl)-piperidin-4-yl]-8-methoxy-1,4-dihydro-benzo[d][1,3]oxazin-2-on |
| 630 | 1-[1-(Dibenzofuran-2-sulfonyl)-piperidin-4-yl]-8-methoxy-1,4-dihydro-benzo[d][1,3]oxazin-2-on |
| 631 | 1-[1-(2,3-Dihydro-benzofuran-5-sulfonyl)-piperidin-4-yl]-8-methoxy-1,4-dihydro-benzo[d][1,3]oxazin-2-on |
| 632 | 1-[1-(Biphenyl-2-sulfonyl)-piperidin-4-yl]-8-methoxy-1,4-dihydro-benzo[d][1,3]oxazin-2-on |
| 633 | 1-[1-(5-Isoxazol-5-yl-thiophen-2-sulfonyl)-piperidin-4-yl]-8-methyoxy-1,4-dihydro-benzo[d][1,3]oxazin-2-on |
| 634 | 6-Chlor-1-[1-(4-chlor-naphthalen-1-sulfonyl)-piperidin-4-yl]-1,4-dihydro-benzo[d][1,3]oxazin-2-on |
| 635 | 6-Chlor-1-[1-(4-fluor-naphthalen-1-sulfonyl)-piperidin-4-yl]-1,4-dihydro-benzo[d][1,3]oxazin-2-on |
| 636 | 6-Chlor-1-[1-(dibenzofuran-2-sulfonyl)-piperidin-4-yl]-1,4-dihydro-benzo[d][1,3]oxazin-2-on |
| 637 | 6-Chlor-1-[1-(2,3-dihydro-benzofuran-5-sulfonyl)-piperidin-4-yl]-1,4-dihydro-benzo[d][1,3]oxazin-2-on |
| 638 | 1-[1-(Biphenyl-2-sulfonyl)-piperidin-4-yl]-6-chlor-1,4-dihydro-benzo[d][1,3]oxazin-2-on |
| 639 | 6-Chlor-1-[1-(5-isoxazol-5-yl-thiophen-2-sulfonyl)-piperidin-4-yl]-1,4-dihydro-benzo[d][1,3]oxazin-2-on |
| 640 | 1-[1-(4-Chlor-naphthalen-1-sulfonyl)-piperidin-4-yl]-6-methyl-1,4-dihydro-benzo[d][1,3]oxazin-2-on |
| 641 | 1-[1-(4-Fluor-naphthalen-1-sulfonyl)-piperidin-4-yl]-6-methyl-1,4-dihydro-benzo[d][1,3]oxazin-2-on |
| 642 | 1-[1-(Dibenzofuran-2-sulfonyl)-piperidin-4-yl]-6-methyl-1,4-dihydro-benzo[d][1,3]oxazin-2-on |
| 643 | 1-[1-(2,3-Dihydro-benzofuran-5-sulfonyl)-piperidin-4-yl]-6-methyl-1,4-dihydro-benzo[d][1,3]oxazin-2-on |
| 644 | 1-[1-(Biphenyl-2-sulfonyl)-piperidin-4-yl]-6-methyl-1,4-dihydro-benzo[d][1,3]oxazin-2-on |
| 645 | 1-[1-(5-Isoxazol-5-yl-thiophen-2-sulfonyl)-piperidin-4-yl]-6-methyl-1,4-dihydro-benzo[d][1,3]oxazin-2-on |
| 646 | 1-[1-(4-Chlor-naphthalen-1-sulfonyl)-piperidin-4-yl]-6,7-difluor-1,4-dihydro-benzo[d][1,3]oxazin-2-on |
| 647 | 6,7-Difluor-1-[1-(4-fluor-naphthalen-1-sulfonyl)-piperidin-4-yl]-1,4-dihydro-benzo[d][1,3]oxazin-2-on |
| 648 | 1-[1-(Dibenzofuran-2-sulfonyl)-piperidin-4-yl]-6,7-difluor-1,4-dihydro-benzo[d][1,3]oxazin-2-on |
| 649 | 1-[1-(2,3-Dihydro-benzofuran-5-sulfonyl)-piperidin-4-yl]-6,7-difluor-1,4-dihydro-benzo[d][1,3]oxazin-2-on |
| 650 | 1-[1-(Biphenyl-2-sulfonyl)-piperidin-4-yl]-6,7-difluor-1,4-dihydro-benzo[d][1,3]oxazin-2-on |
| 651 | 6,7-Difluor-1-[1-(5-isoxazol-5-yl-thiophen-2-sulfonyl)-piperidin-4-yl]-1,4-dihydro-benzo[d][1,3]oxazin-2-on |
| 652 | 1-[1-(1,2-Dimethyl-1H-imidazol-4-sulfonyl)-piperidin-4-yl]-1,4-dihydro-benzo[d][1,3]oxazin-2-on |
| 653 | 1-[1-(5-Methyl-benzo[1,2,5]thiadiazol-4-sulfonyl)-piperidin-4-yl]-1,4-dihydro-benzo[d][1,3]oxazin-2-on |
| 654 | 1-[1-(3,5-Dimethyl-isoxazol-4-sulfonyl)-piperidin-4-yl]-1,4-dihydro-benzo[d][1,3]oxazin-2-on |
| 655 | 1-[1-(1,2-Dimethyl-1H-imidazol-4-sulfonyl)-piperidin-4-yl]-8-methyl-1,4-dihydro-benzo[d][1,3]oxazin-2-on |
| 656 | 8-Methyl-1-[1-(5-methyl-benzo[1,2,5]thiadiazol-4-sulfonyl)-piperidin-4-yl]-1,4-dihydro-benzo[d][1,3]oxazin-2-on |
| 657 | 1-[1-(3,5-Dimethyl-isoxazol-4-sulfonyl)-piperidin-4-yl]-8-methyl-1,4-dihydro-benzo[d][1,3]oxazin-2-on |
| 658 | 6-Chlor-1-[1-(1,2-dimethyl-1H-imidazol-4-sulfonyl)-piperidin-4-yl]-1,4-dihydro-benzo[d][1,3]oxazin-2-on |
| 659 | 6-Chlor-1-[1-(5-methyl-benzo[1,2,5]thiadiazol-4-sulfonyl)-piperidin-4-yl]-1,4-dihydro-benzo[d][1,3]oxazin-2-on |
| 660 | 6-Chlor-1-[1-(3,5-dimethyl-isoxazol-4-sulfonyl)-piperidin-4-yl]-1,4-dihydro-benzo[d][1,3] oxazin-2-on |
| 661 | 1-[1-(1,2-Dimethyl-1H-imidazol-4-sulfonyl)-piperidin-4-yl]-8-methoxy-1,4-dihydro-benzo[d][1,3]oxazin-2-on |
| 662 | 8-Methoxy-1-[1-(5-methyl-benzo[1,2,5]thiadiazol-4-sulfonyl)-piperidin-4-yl]-1,4-dihydro-benzo[d][1,3]oxazin-2-on |
| 663 | 1-[1-(3,5-Dimethyl-isoxazol-4-sulfonyl)-piperidin-4-yl]-8-methoxy-1,4-dihydro-benzo[d][1,3]oxazin-2-on |
| 664 | 5-Chlor-1-[1-(1,2-dimethyl-1H-imidazol-4-sulfonyl)-piperidin-4-yl]-1,4-dihydro-benzo[d][1,3]oxazin-2-on |
| 665 | 5-Chlor-1-[1-(5-methyl-benzo[1,2,5]thiadiazol-4-sulfonyl)-piperidin-4-yl]-1,4-dihydro-benzo[d][1,3]oxazin-2-on |
| 666 | 5-Chlor-1-[1-(3,5-dimethyl-isoxazol-4-sulfonyl)-piperidin-4-yl]-1,4-dihydro-benzo[d][1,3]oxazin-2-on |
| 667 | 1-[1-(1,2-Dimethyl-1H-imidazol-4-sulfonyl)-piperidin-4-yl]-6-methyl-1,4-dihydro-benzo[d]]1,3]oxazin-2-on |
| 668 | 6-Methyl-1-[1-(5-methyl-benzo[1,2,5]thiadiazol-4-sulfonyl)-piperidin-4-yl]-1,4-dihydro-benzo[d][1,3]oxazin-2-on |
| 669 | 1-[1-(3,5-Dimethyl-isoxazol-4-sulfonyl)-piperidin-4-yl]-6-methyl-1,4-dihydro-benzo[d][1,3]oxazin-2-on |
| 670 | 1-[1-(1,2-Dimethyl-1H-imidazol-4-sulfonyl)-piperidin-4-yl]-6-fluor-1,4-dihydro-benzo[d][1,3]oxazin-2-one |
| 671 | 6-Fluor-1-[1-(5-methyl-benzo[1,2,5]thiadiazol-4-sulfonyl)-piperidin-4-yl]-1,4-dihydro-benzo[d][1,3]oxazin-2-on |
| 672 | 1-[1-(3,5-Dimethyl-isoxazol-4-sulfonyl)-piperidin-4-yl]-6-fluor-1,4-dihydro-benzo[d][1,3]oxazin-2-on |
| 673 | 1-[1-(1,2-Dimethyl-1H-imidazol-4-sulfonyl)-piperidin-4-yl]-6,7-difluor-1,4-dihydro-benzo[d][1,3]oxazin-2-on |
| 674 | 6,7-Difluor-1-[1-(5-methyl-benzo[1,2,5]thiadiazol-4-sulfonyl)-piperidin-4-yl]-1,4-dihydro-benzo[d][1,3]oxazin-2-on |
| 675 | 1-1-(3,5-Dimethyl-isoxazol-4-sulfonyl)-piperidin-4-yl]-6,7-difluor-1,4-dihydro-benzo[d][1,3]oxazin-2-on |
| 676 | 1-[1-(5-Chlor-naphthalen-1-sulfonyl)-piperidin-4-yl]-1,4-dihydro-benzo[d][1,3]oxazin-2-on |
| 677 | 1-[1-(5-Chlor-naphthalen-2-sulfonyl)-piperidin-4-yl]-1,4-dihydro-benzo[d][1,3]oxazin-2-on |
| 678 | N-{5-[4-(2-Oxo-4H-benzo[d][1,3]oxazin-1-yl)-piperidin-1-sulfonyl]-naphthalen-1-yl)-acetamid |
| 679 | 1-[1-(5-Chlor-naphthalen-1-sulfonyl)-piperidin-4-yl]-8-methyl-1,4-dihydro-benzo[d][1,3] oxazin-2-on |
| 680 | 1-1-(5-Chlor-naphthalen-2-sulfonyl)-piperidin-4-yl]-8-methyl-1,4-dihydro-benzo[d][1,3]oxazin-2-on |
| 681 | N-{5-[4-(8-Methyl-2-oxo-4H-benzo[d][1,3]oxazin-1-yl)-piperidin-1-sulfonyl]-naphthalen-1-yl}-acetamid |
| 682 | 5-Chlor-1-[1-(5-chlor-naphthalen-1-sulfonyl)-piperidin-4-yl]-1,4-dihydro-benzo[d][1,3]oxazin-2-on |
| 683 | 5-Chlor-1-[1-(5-chlor-naphthalen-2-sulfonyl)-piperidin-4-yl]-1,4-dihydro-benzo[d][1,3]oxazin-2-on |
| 684 | N-{5-[4-(5-Chlor-2-oxo-4H-benz[d][1,3]oxazin-1-yl)-piperidin-1-sulfonyl]-naphthalen-1-yl}-acetamid |
| 685 | 1-[1-(5-Chlor-naphthalen-1-sulfonyl)-piperidin-4-yl]-8-methoxy-1,4-dihydro-benzo[d][1,3]oxazin-2-on |
| 686 | 1-[1-(5-Chlor-naphthalen-2-sulfonyl)-piperidin-4-yl]-8-methoxy-1,4-dihydro-benzo[d][1,3]oxazin-2-on |
| 687 | N-{5-[4-(8-Methoxy-2-oxo-4H-benzo[d][1,3]oxazin-1-yl)-piperidin-1-sulfonyl]-naphthalen-1-yl}-acetamid |
| 688 | 2,5-Dimethyl-4-[4-(8-methyl-2-oxo-4H-benzo[d][1,3]oxazin-1-yl)-piperidin-1-sulfonyl]-furan-3-carbonsäure-methylester |
| 689 | 8-Methyl-1-[1-(2-oxo-2,3-dihydro-benzothiazol-6-sulfonyl)-piperidin-4-yl]-1,4-dihydro-benzo[d][1,3]oxazin-2-on |
| 690 | 1-[1-(4-Fluor-3-methyl-benzolsulfonyl)-piperidin-4-yl]-8-methyl-1,4-dihydro-benzo[d][1,3]oxazin-2-on |
| 691 | 8-Methyl-1-[1-(2-oxo-2,3-dihydro-benzooxazol-6-sulfonyl)-piperidin-4-yl]-1,4-dihydro-benzo[d][1,3]oxazin-2-on |
| 692 | 1-[1-(4-Cyclohexyl-benzolsulfonyl)-piperidin-4-yl]-8-methyl-1,4-dihydro-benzo[d][1,3]oxazin-2-on |
| 693 | 2,5-Dimethyl-4-[4-(2-oxo-4H-benzo[d][1,3]oxazin-1-yl)-piperidin-1-sulfonyl]-furan-3-carbonsäure-methylester |
| 694 | 1-[1-(4-Fluor-3-methyl-benzolsulfonyl)-piperidin-4-yl]-1,4-dihydro-benzo[d][1,3]oxazin-2-on |
| 695 | 1-[1-(2-Oxo-2,3-dihydro-benzooxazol-6-sulfonyl)-piperidin-4-yl]-1,4-dihydro-benzo[d][1,3]oxazin-2-on |
| 696 | 1-[1-(4-Cyclohexyl-benzolsulfonyl)-piperidin-4-yl]-1,4-dihydro-benzo[d][1,3]oxazin-2-on |
| 697 | 2-Fluor-5-[4-(8-methyl-2-oxo-4H-benzo[d][1,3]oxazin-1-yl)-piperidin-1-sulfonyl]-benzoesäure |
| 698 | 2-Fluor-5-[4-(2-oxo-4H-benzo[d][1,3]oxazin-1-yl)-piperidin-1-sulfonyl]-benzoesäure |
| 699 | 1-[1-(2-Oxo-2,3-dihydro-benzothiazol-6-sulfonyl)-piperidin-4-yl]-1,4-dihydro-benzo[d][1,3]oxazin-2-on |
| 700 | 1-[1-(5-Pyridin-2-yl-thiophen-2-sulfonyl)-piperidin-4-yl]-1,4-dihydro-benzo[d][1,3]oxazin-2-on |
| 701 | 3-[4-(2-Oxo-4H-benzo[d][1,3]oxazin-1-yl)-piperidin-1-sulfonyl-benzonitril |
| 702 | 3-[4-(2-Oxo-4H-benzo[d][1,3]oxazin-1-yl)-piperidin-1-sulfonyl]-thiophen-2-carbonsäure-methylester |
| 703 | 1-{5-[4-(2-Oxo-4H-benzo[d][1,3]oxazin-1-yl)-piperidin-1-sulfonyl]-naphthalen-1-yl}-pyrrolidin-2,5-dion |
| 704 | 1-[1-(2-Chlor-5-trifluormethyl-benzolsulfonyl)-piperidin-4-yl]-1,4-dihydro-benzo[d][1,3]oxazin-2-on |
| 705 | 1-[1-(3,4-Dimethyl-benzolsulfonyl)-piperidin-4-yl]-1,4-dihydro-benzo[d][1,3]oxazin-2-on |
| 706 | 8-Methyl-1-[1-(5-pyridin-2-yl-thiophen-2-sulfonyl)-piperidin-4-yl]-1,4-dihydro-benzo[d][1,3]oxazin-2-on |
| 707 | 3-[4-(8-Methyl-2-oxo-4H-benzo[d][1,3]oxazin-1-yl)-piperidin-1-sulfonyl]-benzonitril |
| 708 | 3-[4-(8-Methyl-2-oxo-4H-benzo[d][1,3]oxazin-1-yl)-piperidin-1-sulfonyl]-thiophen-2-carbonsäure-methylester |
| 709 | 1-{5-[4-(8-Methyl-2-oxo-4H-benzo[d][1,3]oxazin-1-yl)-piperidin-1-sulfonyl]-naphthalen-1-yl}-pyrrolidin-2,5-dion |
| 710 | 1-[1-(2-Chlor-5-trifluormethyl-benzolsulfonyl)-piperidin-4-yl]-8-methyl-1,4-dihydro-benzo[d][1,3]oxazin-2-on |
| 711 | 1-[1-(3,4-Dimethyl-benzolsulfonyl)-piperidin-4-yl]-8-methyl-1,4-dihydro-benzo[d][1,3]oxazin-2-on |
| 712 | 5-Chlor-1-[1-(5-pyridin-2-yl-thiophen-2-sulfonyl)-piperidin-4-yl]-1,4-dihydro-benzo[d][1,3]oxazin-2-on |
| 713 | 3-[4-(5-Chlor-2-oxo-4H-benzo[d][1,3]oxazin-1-yl)-piperidin-1-sulfonyl]-benzonitril |
| 714 | 3-[4-5-Chlor-2-oxo-4H-benzo[d][1,3]oxazin-1-yl)-piperidin-1-sulfonyl]-thiophen-2-carbonsäure-methylester |
| 715 | 1-{5-[4-(5-Chlor-2-oxo-4H-benzo[d][1,3]oxazin-1-yl)-piperidin-1-sulfonyl]-naphthalen-1-yl}-pyrrolidin-2,5-dion |
| 716 | 5-Chlor-1-[1-(2-chlor-5-trifluormethyl-benzolsulfonyl)-piperidin-4-yl]-1,4-dihydro-benzo[d][1,3]oxazin-2-on |
| 717 | 5-Chlor-1-[1-(3,4-dimethyl-benzolsulfonyl)-piperidin-4-yl]-1,4-dihydro-benzo[d][1,3]oxazin-2-on |
| 718 | 6-Methyl-1-[1-(5-pyridin-2-yl-thiophen-2-sulfonyl)-piperidin-4-yl]-1,4-dihydro-benzo[d][1,3]oxazin-2-on |
| 719 | 3-[4-(6-Methyl-2-oxo-4H-benzo[d][1,3]oxazin-1-yl)-piperidin-1-sulfonyl]-benzonitril |
| 720 | 3-[4-(6-Methyl-2-oxo-4H-benzo[d][1,3]oxazin-1-yl)-piperidin-1-sulfonyl]-thiophen-2-carbonsäure-methylester |
| 721 | 1-{5-[4-(6-Methyl-2-oxo-4H-benzo[d][1,3]oxazin-1-yl)-piperidin-1-sulfonyl]-naphthalen-1-yl}-pyrrolidine-2,5-dion |
| 722 | 1-[1-(2-Chlor-5-trifluormethyl-benzolsulfonyl)-piperidin-4-yl]-6-methyl-1,4-dihydro-benzo[d][1,3]oxazin-2-on |
| 723 | 1-[1-(3,4-Dimethyl-benzolsulfonyl)-piperidin-4-yl]-6-methyl-1,4-dihydro-benzo[d][1,3]oxazin-2-on |
| 724 | 6-Chlor-1-[1-(5-pyridin-2-yl-thiophen-2-sulfonyl)-piperidin-4-yl]-1,4-dihydro-benzo[d][1,3]oxazin-2-on |
| 725 | 3-[4-(6-Chlor-2-oxo-4H-benzo[d][1,3]oxazin-1-y)-piperidin-1-sulfonyl]-benzonitril |
| 726 | 3-[4-(6-Chlor-2-oxo-4H-benzo[d][1,3]oxazin-1-yl)-piperidin-1-sulfonyl]-thiophen-2-carbonsäure-methylester |
| 727 | 1-{5-[4-(6-Chlor-2-oxo-4H-benzo[d][1,3]oxazin-1-yl)-piperidin-1-sulfonyl]-naphthalen-1-yl}-pyrrolidin-2,5-dion |
| 728 | 6-Chlor-1-[1-(2-chlor-5-trifluormethyl-benzolsulfonyl)-piperidin-4-yl]-1,4-dihydro-benzo[d][1,3]oxazin-2-on |
| 729 | 6-Chlor-1-[1-(3,4-dimethyl-benzolsulfonyl)-piperidin-4-yl]-1,4-dihydro-benzo[d][1,3]oxazin-2-on |
| 730 | 1-[1-(5-Methyl-isoxazol-4-sulfonyl)-piperidin-4-yl]-1,4-dihydro-benzo[d][1,3]oxazin-2-on |
| 731 | 1-[1-(2,2-Dimethyl-chroman-6-sulfonyl)-piperidin-4-yl]-1,4-dihydro-benzo[d][1,3]oxazin-2-on |
| 732 | 1-[1-(4-Methyl-3,4-dihydro-2H-benzo[1,4]oxazin-7-sulfonyl)-piperidin-4-yl]-1,4-dihydro-benzo[d][1,3]oxazin-2-on |
| 733 | 1-[1-(2,3-Dihydro-benzo[1,4]dioxin-6-sulfonyl)-piperidin-4-yl]-1,4-dihydro-benzo[d][1,3]oxazin-2-on |
| 734 | 1-[1-(1,3,5-Trimethyl-1H-pyrazol-4-sulfonyl)-piperidin-4-yl]-1,4-dihydro-benzo[d][1,3]oxazin-2-on |
| 735 | 1-[1-(3-Methyl-2-oxo-2,3-dihydro-benzooxazol-6-sulfonyl)-piperidin-4-yl]-1,4-dihydro-benzo[d][1,3]oxazin-2-on |
| 736 | 8-Methyl-1-[1-(5-methyl-isoxazol-4-sulfonyl)-piperidin-4-yl]-1,4-dihydro-benzo[d][1,3]oxazin-2-on |
| 737 | 1-[1-(2,2-Dimethyl-chroman-6-sulfonyl)-piperidin-4-yl]-8-methyl-1,4-dihydro-benzo[d][1,3]oxazin-2-on |
| 738 | 8-Methyl-1-[1-(4-methyl-3,4-dihydro-2H-benzo[1,4]oxazin-7-sulfonyl)-piperidin-4-yl]-1,4-dihydro-benzo[d][1,3]oxazin-2-on |
| 739 | 1-[1-(2,3-Dihydro-benzo[1,4]dioxin-6-sulfonyl)-piperidin-4-yl]-8-methyl-1,4-dihydro-benzo[d][1,3]oxazin-2-on |
| 740 | 8-Methyl-1-[1-(1,3,5-trimethyl-1H-pyrazol-4-sulfonyl)-piperidin-4-yl]-1,4-dihydro-benzo[d][1,3]oxazin-2-on |
| 741 | 8-Methyl-1-[1-(3-methyl-2-oxo-2,3-dihydro-benzooxazol-6-sulfonyl)-piperidin-4-yl]-1,4-dihydro-benzo[d][1,3]oxazin-2-on |
| 742 | 8-Methoxy-1-[1-(1,3,5-trimethyl-1H-pyrazol-4-sulfonyl)-piperidin-4-yl]-1,4-dihydro-benzo[d][1,3]oxazin-2-on |
| 743 | 8-Methoxy-1-[1-(3-methyl-2-oxo-2,3-dihydro-benzooxazol-6-sulfonyl)-piperidin-4-yl]-1,4-dihydro-benzo[d][1,3]oxazin-2-on |
| 744 | 1-[1-(Benzo[d]isoxazol-3-yl-methansulfonyl)-piperidin-4-yl]-1,4-dihydro-benzo[d][1,3]oxazin-2-on |
| 745 | 1-[1-(2,2,4,6,7-Pentamethyl-2,3-dihydro-benzofuran-5-sulfonyl)-piperidin-4-yl]-1,4-dihydro-benzo[d][1,3]oxazin-2-on |
| 746 | 6-Methyl-5-[4-(2-oxo-4H-benzo[d][1,3]oxazin-1-yl)-piperidin-1-sulfonyl]-1H-pyrimidin-2,4-dion |
| 747 | 1-[1-(3-Methyl-chinolin-8-sulfonyl)-piperidin-4-yl]-1,4-dihydro-benzo[d][1,3]oxazin-2-on |
| 748 | 1-[1-(2,2,5,7,8-Pentamethyl-chroman-6-sulfonyl)-piperidin-4-yl]-1,4-dihydro-benzo[d][1,3]oxazin-2-on |
| 749 | 1,4-Dimethyl-6-[4-(2-oxo-4H-benzo[d][1,3]oxazin-1-yl)-piperidin-1-sulfonyl]-1,4-dihydro-chinoxalin-2,3-dion |
| 740 | 1-[1-(1 H-Imidazol-4-sulfonyl)-piperidin-4-yl]-1,4-dihydro-benzo[d][1,3]oxazin-2-on |
| 751 | 1-[1-(2-Oxo-1,2,3,4-tetrahydro-chinolin-6-sulfonyl)-piperidin-4-yl]-1,4-dihydro-benzo[d][1,3]oxazin-2-on |
| 752 | 7-[4-(2-Oxo-4H-benzo[d][1,3]oxazin-1-yl)-piperidin-1-sulfonyl]-1,5-dihydro-benzo[b][1,4]diazepin-2,4-dion |
| 753 | 8-Methyl-1-[1-(3-methyl-chinolin-8-sulfonyl)-piperidin-4-yl]-1,4-dihydro-benzo[d][1,3]oxazin-2-on |
| 754 | 6-Chlor-1-[1-(3-methyl-chinolin-8-sulfonyl)-piperidin-4-yl]-1,4-dihydro-benzo[d][1,3]oxazin-2-on |
| 755 | 5-Chlor-1-[1-(3-methyl-chinolin-8-sulfonyl)-piperidin-4-yl]-1,4-dihydro-benzo[d][1,3]oxazin-2-on |
| 756 | 8-Methoxy-1-[1-(3-methyl-chinolin-8-sulfonyl)-piperidin-4-yl]-1,4-dihydro-benzo[d][1,3]oxazin-2-on |
| 757 | 1-[1-(Pyridin-2-sulfonyl)-piperidin-4-yl]-1,4-dihydro-benzo[d][1,3]oxazin-2-on |
| 758 | 1-[1-(6,7-Dihydroxy-naphthalen-1-sulfonyl)-piperidin-4-yl]-1,4-dihydro-benzo[d][1,3]oxazin-2-on |
| 759 | Essigsäure-3-acetoxy-5-[4-(2-oxo-4H-benzo[d][1,3]oxazin-1-yl)-piperidin-1-sulfonyl]-naphthalen-2-yl-ester |
| 760 | 1-[1-(1 H-Benzoimidazol-2-sulfonyl)-piperidin-4-yl]-1,4-dihydro-benzo[d][1,3]oxazin-2-on |
| 761 | 1-[1-(1 H-Benzoimidazol-2-sulfonyl)-piperidin-4-yl]-8-methoxy-1,4-dihydro-benzo[d][1,3]oxazin-2-on |
| 762 | 1-[1-(1 H-Benzoimidazol-2-sulfonyl)-piperidin-4-yl]-5-chlor-1,4-dihydro-benzo[d][1,3]oxazin-2-on |
| 763 | 1-[1-(2,5-Dimethoxy-benzolsulfonyl)-piperidin-4-yl]-6-fluor-1,4-dihydro-benzo[d][1,3]oxazin-2-on |
| 764 | 1-[1-(2,5-Dimethoxy-benzolsulfonyl)-piperidin-4-yl]-8-methoxy-1,4-dihydro-benzo[d][1,3]oxazin-2-on |
| 765 | 1-[1-(2,5-Dimethoxy-benzolsulfonyl)-piperidin-4-yl]-6,7-difluor-1,4-dihydro-benzo[d][1,3]oxazin-2-on |
| 766 | 5-Chlor-1-[1-(2,5-dimethoxy-benzolsulfonyl)-piperidin-4-yl]-1,4-dihydro-benzo[d][1,3]oxazin-2-on |
| 767 | 1-[1-(5-Dimethylamino-naphthalen-1-sulfonyl)-piperidin-4-yl]-8-methoxy-1,4-dihydro-benzo[d][1,3]oxazin-2-on |
| 768 | 5-Chlor-1-[1]-(5-dimethylamino-naphthalen-1-sulfonyl)-piperidin-4-yl]-1,4-dihydro-benzo[d][1,3]oxazin-2-on |
| 769 | 6-Chlor-1-[1-(5-chlor-naphthalen-1-sulfonyl)-piperidin-4-yl]-1,4-dihydro-benzo[d][1,3]oxazin-2-on |
| 770 | 1-[1-(5-Chlor-naphthalen-1-sulfonyl)-piperidin-4-yl]-6-methyl-1,4-dihydro-benzo[d][1,3]oxazin-2-on |
| 771 | 1-[1-(5-Chlor-naphthalen-1-sulfonyl)-piperidin-4-yl]-6-fluor-1,4-dihydro-benzo[d][1,3]oxazin-2-on |
| 772 | 6-Chlor-1-[1-(5-chlor-naphthalen-2-sulfonyl)-piperidin-4-yl]-1,4-dihydro-benzo[d][1,3] oxazin-2-on |
| 773 | 1-[1-(5-Chlor-naphthalen-2-sulfonyl)-piperidin-4-yl]-6-methyl-1,4-dihydro-benzo[d][1,3]oxazin-2-on |
| 774 | 1-[1-(5-Chlor-naphthalen-2-sulfonyl)-piperidin-4-yl]-6-fluor-1,4-dihydro-benzo[d][1,3]oxazin-2-on |
| 775 | 6-Methyl-1-[1-(3-methyl-chinolin-8-sulfonyl)-piperidin-4-yl]-1,4-dihydro-benzo[d][1,3]oxazin-2-on |
| 776 | 6-Fluor-1-[1-(3-methyl-chinolin-8-sulfonyl)-piperidin-4-yl]-1,4-dihydro-benzo[d][1,3]oxazin-2-on |
| 777 | 6,7-Difluor-1-[1-(3-methyl-chinolin-8-sulfonyl)-piperidin-4-yl]-1,4-dihydro-benzo[d][1,3] oxazin-2-on |
| 778 | 6-Chlor-1-[1-(3-methyl-2-oxo-2,3-dihydro-benzooxazol-6-sulfonyl)-piperidin-4-yl]-1,4-dihydro-benzo[d][1,3]oxazin-2-on |
| 779 | 6-Methyl-1-[1-(3-methyl-2-oxo-2,3-dihydro-benzooxazol-6-sulfonyl)-piperidin-4-yl]-1,4-dihydro-benzo[d][1,3]oxazin-2-on |
| 780 | 6-Fluor-1-[1-(3-methyl-2-oxo-2,3-dihydro-benzooxazol-6-sulfonyl)-piperidin-4-yl]-1,4-dihydro-benzo[d][1,3]oxazin-2-on |
| 781 | 6,7-Difluor-1-[1-(3-methyl-2-oxo-2,3-dihydro-benzooxazol-6-sulfonyl)-piperidin-4-yl]-1,4-dihydro-benzo[d][1,3]oxazin-2-on |
| 782 | 5-Chlor-1-[1-(3-methyl-2-oxo-2,3-dihydro-benzooxazol-6-sulfonyl)-piperidin-4-yl]-1,4-dihydro-benzo[d][1,3]oxazin-2-on |
| 783 | 6-Chlor-1-[1-(4-methyl-3,4-dihydro-2H-benzo[1,4]oxazin-7-sulfonyl)-piperidin-4-yl]-1,4-dihydro-benzo[d][1,3]oxazin-2-on |
| 784 | 6-Methyl-1-[1-(4-methyl-3,4-dihydro-2H-benzo[1,4]oxazin-7-sulfonyl)-piperidin-4-yl]-1,4-dihydro-benzo[d][1,31oxazin-2-on |
| 785 | 6-Fluor-1-[1-(4-methyl-3,4-dihydro-2H-benzo[1,4]oxazin-7-sulfonyl)-piperidin-4-yl]-1,4-dihydro-benzo[d][1,3]oxazin-2-on |
| 786 | 8-Methoxy-1-[1-(4-methyl-3,4-dihydro-2H-benzo[1,4]oxazin-7-sulfonyl)-piperidin-4-yl]-1,4-dihydro-benzo[d][1,3]oxazin-2-on |
| 787 | 5-Chlor-1-[1-(4-methyl-3,4-dihydro-2H-benzo[1,4]oxazin-7-sulfonyl)-piperidin-4-yl]-1,4-dihydro-benzo[d][1,3]oxazin-2-on |

58. Verfahren zur Herstellung von Benzoxazinon-abgeleiteten Sulfonamidverbindungen der allgemeinen Formel (Ib) nach einem oder mehreren der Ansprüche 42 bis 57, **dadurch gekennzeichnet, dass** man wenigstens eine Piperidinverbindung der allgemeinen Formel (IIb), wobei R^{1b} bis R^{9b} die für einen oder mehrere der Ansprüche 42 bis 57 angegebene Bedeutung haben, und/oder ein Salz, vorzugsweise ein Hydrochloridsalzes davon, 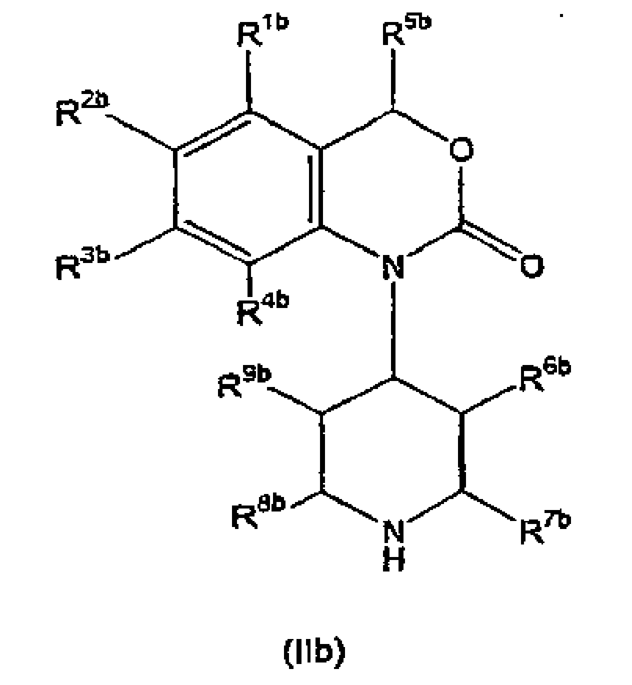 mit wenigstens einer Verbindung der allgemeinen Formel (IIIb) 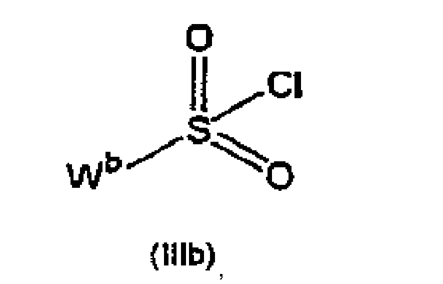 worin W^{b} die in einem oder mehreren der Ansprüche 42 bis 57 angegebene Bedeutung hat, in einem geeigneten Reaktionsmedium, gegebenenfalls in Gegenwart wenigstens einer Base und/oder wenigstens eines Hilfsmittels umsetzt.

59. Verfahren zur Herstellung eines physiologisch akzeptablen Salzes der Benzoxazinon-abgeleiteten Sulfonamidverbindungen gemäß den Ansprüchen 42 bis 57, **dadurch gekennzeichnet, dass** wenigstens eine Verbindung der allgmeinene Formel (Ib), die wenigstens eine basische Gruppe aufweist, umgesetzt wird mit wenigstens einer Säure, vorzugsweise einer anorganischen oder einer organischen Säure, vorzugsweise in Gegenwart eines geeigneten Reaktionsmediums.

60. Verfahren zur Herstellung eines physiologisch akzeptablen Salzes der Benzoxazinon-abgeleiteten Sulfonamidverbindungen gemäß den Ansprüchen 42 bis 57, **dadurch gekennzeichnet, dass** wenigstens eine Verbindung der allgmeinene Formel (Ib), die wenigstens eine saure Gruppe aufweist, umgesetzt wird mit wenigstens einer Base, vorzugsweise in Gegenwart eines geeigneten Reaktionsmediums.

61. Medikament, umfassend wenigstens eine Benzoxazinon-abgeleitete Sulfonamidverbindung nach einem der Ansprüche 42 bis 57, und gegebenenfalls ein oder mehrerere pharmazeutisch akzeptable Adjuvanzien.

62. Medikament nach Anspruch 61 zur Vorbeugung und/oder Behandlung von Störungen der Nahrungsaufnahme, vorzugsweise zur Appetitregulierung, zur Aufrechterhaltung, Steigerung oder Verringerung des Körpergewichts, zur Vorbeugung und/oder Behandlung von Adipositas, zur Vorbeugung und/oder Behandlung von Bulimie, zur Vorbeugung und/oder Behandlung von Anorexie, zur Vorbeugung und/oder Vorbeugung von Kachexie, zur Vorbeugung und/oder Behandlung von oder Typ-II-Diabetes (nicht-insulinabhängigem Diabetes mellitus).

63. Medikament nach Anspruch 61 zur Vorbeugung und/oder Behandlung von gastrointestinalen Störungen, vorzugsweise von Reizdarmsyndrom; zur Vorbeugung und/oder Behandlung von Störungen des Zentralnervensystems, zur Vorbeugung und/oder Behandlung von Angst; zur Vorbeugung und/oder Behandlung von Panikattacken; zur Vorbeugung und/oder Behandlung von Depression; zur Vorbeugung und/oder Behandlung von bipolaren Störungen; zur Vorbeugung und/oder Behandlung von kognitiven Störungen, vorzugsweise Gedächtnisstörungen; zur Verbesserung der Kognition (zur kognitiven Leistungssteigerung); zur Vorbeugung und/oder Behandlung von seniler Demenz; zur Vorbeugung und/oder Behandlung von Psychose; zur Vorbeugung und/oder Behandlung von neurodegenerativen Störungen, vorzugsweise ausgewählt unter Morbus Alzheimer, Morbus Parkinson, Morbus Huntington und Multipler Sklerose; zur Vorbeugung und/oder Behandlung von Schizophrenie oder zur Vorbeugung und/oder Behandlung von Hyperaktivititätsstörung (ADHD, Aufmerksamkeitsdefizit, Hyperaktivitätsstörung).

64. Verwendung wenigstens einer Benzoxazinon-abgeleiteten Sulfonamidverbindung nach einem der Ansprüche 42 bis 57 zur Herstellung eines Medikaments zur Vorbeugung und/oder Behandlung von Störungen der Nahrungsaufnahme.

65. Verwendung nach Anspruch 64 zur Appetitregulierung, zur Aufrechterhaltung, Steigerung oder Verringerung des Körpergewichts, zur Vorbeugung und/oder Behandlung von Adipositas, zur Vorbeugung und/oder Behandlung von Bulimie, zur Vorbeugung und/oder Behandlung von Anorexie, zur Vorbeugung und/oder Behandlung von Kachexie, oder zur Vorbeugung und/oder Behandlung von Typ-II-Diabetes.

66. Verwendung wenigstens einer Benzoxazinon-abgeleiteten Sulfonamidverbindung nach einem der Ansprüche 42 bis 57 zur Herstellung eines Medikaments zur Vorbeugung und/oder Behandlung von gastrointestinalen Störungen, vorzugsweise von Reizdarmsyndrom; zur Vorbeugung und/oder Behandlung von Störungen des Zentralnervensystems; zur Vorbeugung und/oder Behandlung von Angst; zur Vorbeugung und/oder Behandlung von Panikattacken; zur Vorbeugung und/oder Behandlung von Depression; zur Vorbeugung und/oder Behandlung von bipolaren Störungen; zur Vorbeugung und/oder Behandlung von kognitiven Störungen, vorzugsweise Gedächtnisstörungen; zur Verbesserung der Kognition (zur kognitiven Leistungssteigerung); zur Vorbeugung und/oder Behandlung von seniler Demenz; zur Vorbeugung und/oder Behandlung von Psychose; zur Vorbeugung und/oder Behandlung von neurodegenerativen Störungen, vorzugsweise ausgewählt unter Morbus Alzheimer, Morbus Parkinson, Morbus Huntington und Multipler Sklerose; zur Vorbeugung und/oder Behandlung von Schizophrenie; oder zur Vorbeugung und/oder Behandlung von Hyperaktivititätsstörung (ADHD, Aufmerksamkeitsdefizit, Hyperaktivitätsstörung).

## Revendications

1. Composés de type sulfonamide dérivés de benzoxazinone de formule générale (I) : 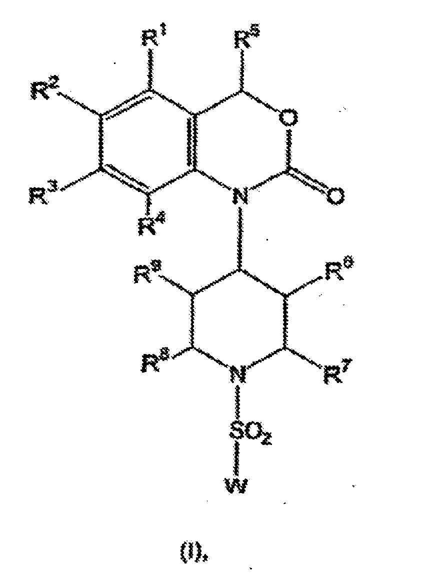 dans laquelle :
R¹, R², R³ et R⁴ sont indépendamment choisis chacun parmi le groupe consistant en un atome d'hydrogène, d'halogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué et/ou peut être condensé avec un système de noyau mono ou polycyclique facultativement au moins monosubstitué, un radical aryle ou hétéroaryle facultativement au moins monosubstitué, qui peut être lié par l'intermédiaire d'un groupe alkylène facultativement au moins monosubstitué et/ou peut être condensé avec un système de noyau mono ou polycyclique facultativement au moins monosubstitué, un groupe nitro, un groupe cyano, et les parties -OR¹⁰, -OC(=O)-R¹¹, -(C=O)-OR¹¹,-SR¹², -SOR¹², -SO₂R¹², -NH-SO₂R¹², -SO₂NH₂ et -NR¹³R¹⁴,
R⁵ représente un atome d'hydrogène, un radical aliphatique non ramifié ou ramifié, saturé ou insaturé, facultativement au moins monosubstitué ou un radical cycloaliphatique saturé ou insaturé, facultativement au moins monosubstitué, contenant facultativement au moins un hétéroatome en tant que chaînon de cycle,
R⁶, R⁷, R⁸ et R⁹ sont indépendamment choisis chacun parmi le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, un groupe cyano et une partie -COOR¹⁵,
W représente un radical aliphatique non ramifié ou ramifié, saturé ou insaturé, qui peut être substitué par un ou plusieurs substituants choisis parmi le groupe consistant en un groupe hydroxy, un atome d'halogène, les radicaux alkoxy en C₁₋₄ ramifiés ou non ramifiés, perfluoroalkoxy en C₁₋₄ ramifiés ou non ramifiés, perfluoroalkyle en C₁₋₄ ramifiés ou non ramifiés, amino, carboxy, amido, cyano, nitro, -SO₂NH₂, -CO-(alkyle en C₁₋₄), -SO-(alkyle en C₁₋₄), -SO₂-(alkyle en C₁₋₄), -NH-SO₂-(alkyle en C₁₋₄), dans lesquels la partie alkyle en C₁₋₄ peut être, dans chaque cas, ramifiée ou non ramifiée, un radical phényle ou naphtyle non substitué ou au moins monosubstitué, et les radicaux furannyle, thiényle, pyrrolyle, imidazolyle, pyrazolyle, pyridinyle, pyrimidinyle, quinolinyle et isoquinolinyle non substitué ou au moins monosubstitué,
de sorte que lesdits substituants peuvent être au moins monosubstitués avec un substituant F, Cl, méthyle et méthoxy,
un radical cycloaliphatique saturé ou insaturé, contenant facultativement au moins un hétéroatome en tant que chaînon de cycle, de sorte que ledit radical cycloaliphatique peut être substitué par un ou plusieurs substituants choisis parmi le groupe consistant en les groupes hydroxy, nitro, carboxy, cyano, céto, halogéno, alkyle en C₁₋₂₀, alkyle en C₁₋₄ partiellement fluorés, alkyle en C₁₋₄ partiellement chlorés, alkyle en C₁₋₄ partiellement bromés, alkoxy en C₁₋₅, alkoxy en C₁₋₄ partiellement fluorés, alkoxy en C₁₋₄ partiellement chlorés, alkoxy en C₁₋₄ partiellement bromés, alcényle en C₂₋₆, -SO₂-(alkyle en C₁₋₄), -(C=O)-(alkyle en C₁₋₅) -(C=O)-O-(alkyle en C₁₋₅), -(C=O)-Cl, -S-(alkyle en C₁₋₄), -(C=O)-H, -NH-(C=O)-NH-(alkyle en C₁₋₅), -(C=O)-perfluoroalkyle en C₁₋₄), -NR^{A}R^{B}, dans lesquels R^{A} et R^{B} sont indépendamment choisis parmi le groupe consistant en H, les radicaux alkyle en C₁₋₄ et phényle, NH-(C=O)-(alkyle en C₁₋₅), -(alkylène en C₁₋₅)-(C=O)-(alkyle en C₁₋₅), 1,3-dihydro-1-oxo-2H-isoindol-2-yle, N-phtalimidinyle, 1,3-dioxo-2-azaspiro[4,4]-non-2-yle, phényle substitués ou non substitués, -SO₂-phényle, phénoxy, pyridinyle, pyridinyloxy, pyrazolyle, pyrimidinyle, pyrrolidinyle, -SO₂-pyrrolidinyle, morpholinyle, -SO₂-morpholinyle, thiadiazolyle, oxadiazolyle, oxazolyle, thiazolyle, isoxazolyle, -O-CH₂-thiazolyle, -NH-phényle, et -(alkylène en C₁₋₄)-NH-(C=O)-phényle, dans lesquels lesdits substituants peuvent être substitués par un ou plusieurs substituants choisis parmi le groupe consistant en un atome d'halogène, les radicaux nitro, cyano, hydroxy, -(C=O)-(alkyle en C₁₋₄), (alkyle en C₁₋₄), alkyle en C₁₋₄ au moins partiellement fluorés, alkyle en C₁₋₄ au moins partiellement chlorés, alkyle en C₁₋₄ au moins partiellement bromés, -S-(alkyle en C₁₋₄), -(C=O)-O-(alkyle en C₁₋₅), -(C=O)-CH₂-F, -(C=O)-CH₂-Cl et -(C=O)-CH₂-Br, et de sorte que ledit radical cycloaliphatique peut être lié par l'intermédiaire d'un groupe alkylène facultativement monosubstitué, et/ou peut être condensé avec un système de noyau aromatique mono ou polycyclique facultativement au moins monosubstitué,
un radical hétéroaryle facultativement au moins monosubstitué, qui peut être lié par l'intermédiaire d'un groupe alkylène facultativement monosubstitué, et/ou peut être condensé avec un système de noyau mono ou polycyclique facultativement au moins monosubstitué,
un radical aryle monocyclique facultativement au moins monosubstitué, qui est condensé avec un système de noyau mono ou polycyclique facultativement au moins monosubstitué, et qui peut être lié par l'intermédiaire d'un groupe alkylène facultativement au moins monosubstitué,
une partie -NR¹⁶R¹⁷,
une partie -COR¹⁸ ou
un radical phényle, qui est au moins monosubstitué avec l'un des substituants choisis parmi le groupe consistant en :
les radicaux 2,2,2-trifluoroéthoxy-, alcényle en C₂₋₆, 1,3-dihydro-1-oxo-2H-isoindol-2-yle, N-phtalimidinyle, (2-chloro-1,3-thiazolyl-5-yl)-méthoxy, éthyl-5-yl-2-méthyl-3-furoate, alkyle en C₁₁₋₂₀, 1,3-dioxo-2-azaspiro[4.4] non-2-yle, pyrazolyle, 1,3-oxazol-5-yle, 5-méthyl-1,3,4-oxadiazol-2-yle, difluorométhoxy, dichlorométhoxy, 1-pyrrolidinylsulfonyle, morpholinosulfonyle, 2-méthyl-4-pyrimidinyle, un groupe phénoxy qui est au moins monosubstitué avec un groupe alkoxy en C₁₋₅, un groupe phényle qui est au moins monosubstitué avec un des substituants choisis parmi le groupe consistant en les substituants nitro, alkoxy en C₁₋₅, F, Cl, Br, alkyle en C₁₋₅ au moins partiellement fluorés, alkyle en C₁₋₅ au moins partiellement chlorés, (2-chloro-1,3-thiazol-5-yl)-méthoxy, -(C=O)-H et -(C=O)-(alkyle en C₁₋₅), un groupe pyridinyle qui est au moins monosubstitué avec un groupe alkoxy en C₁₋₅, un groupe pyridinyloxy qui est au moins monosubstitué avec un groupe alkoxy en C₁₋₅, un groupe phénoxy qui est au moins disubstitué, et un groupe pyridinyloxy qui est au moins disubstitué,
à condition que W ne représente pas un radical furyle non substitué, thiényle non substitué ou thiényle substitué avec un substituant choisi parmi le groupe consistant en les radicaux (alkoxy en C₁₋₅)carbonyle, (alkyle en C₁₋₅)carbonyle, carboxyle et pyridyle, pyrrolyle non substitués, naphtyle non substitués, indolyle non substitués, tétrahydronaphtyle non substitués, pyridyle substitués ou non substitués, pyrazinyle non substitués, quinolinyle non substitués, pyrrolyle (alkyle en C₁₋₅)-substitués, et cyclohexyle non substitués ou cyclohexyle substitués avec un ou deux radicaux choisis parmi le groupe consistant en les substituants oxo, hydroxy, alkoxy en C₁₋₅, (alkoxy en C₁₋₅)-carbonylamino-(alkyle en C₁₋₅) et amino-(alkyle en C₁₋₅),
R¹⁰ représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R¹¹ représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R¹² représente un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R¹³ et R¹⁴ sont indépendamment choisis chacun parmi le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, ou
R¹³ et R¹⁴ forment ensemble avec l'atome d'azote pontant, un noyau hétérocyclique saturé, insaturé ou aromatique, qui peut être au moins monosubstitué et/ou contenir au moins un autre hétéroatome en tant que chaînon de cycle,
R¹⁵ représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R¹⁶ représente un radical aliphatique non ramifié ou ramifié, saturé ou insaturé, facultativement au moins monosubstitué,
R¹⁷ représente un radical aliphatique non ramifié ou ramifié, saturé ou insaturé, facultativement au moins monosubstitué, et
R¹⁸ représente un radical aryle facultativement au moins monosubstitué,
facultativement sous la forme de l'un de leurs stéréoisomères, de préférence, les énantiomères ou les diastéréoisomères, leur racémate ou sous la forme d'un mélange d'au moins deux de leurs stéréoisomères, de préférence, les énantiomères ou les diastéréoisomères, en un quelconque rapport de mélange, ou un sel correspondant de ceux-ci, ou un solvat correspondant.

2. Composés selon la revendication 1, **caractérisés en ce que** R¹, R², R³ et R⁴ sont indépendamment choisis chacun parmi le groupe consistant en H, F, Cl et Br, un radical aliphatique en C₁₋₆ non ramifié ou ramifié, saturé ou insaturé, facultativement au moins monosubstitué, un radical cycloaliphatique en C₃-₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆ facultativement au moins monosubstitué et/ou peut être condensé avec un système de noyau mono ou polycyclique facultativement au moins monosubstitué, un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, un groupe nitro, un groupe cyano, et les parties -OR¹⁰, -OC(=O)-R¹¹, -SR¹² -SOR¹², -SO2R¹², -NH-SO₂R¹², -SO₂NH₂ et -NR¹³R¹⁴, de préférence, choisis parmi le groupe consistant en H, F, Cl et Br, un radical aliphatique en C₁₋₃ non ramifié ou ramifié, saturé ou insaturé, facultativement au moins monosubstitué, un radical cycloaliphatique en C₅ ou C₆ saturé ou insaturé, facultativement au moins monosubstitué, contenant facultativement au moins un hétéroatome en tant que chaînon de cycle, qui peut être lié par l'intermédiaire d'un groupe alkylène en C₁ ou C₂ facultativement au moins monosubstitué, un groupe nitro, un groupe cyano, et les parties -OR¹⁰, -OC(=O)-R¹¹, -SR¹² et -NR¹³R¹⁴, plus préférablement, choisis parmi le groupe consistant en H, F, Cl, -CH₃, -CH₂CH₃, -CF₃, - CF₂CF₃, les groupes cyclopentyle, cyclohexyle, nitro, cyano et -OR¹⁰.

3. Composés selon la revendication 1 ou 2, **caractérisés en ce que** R⁵ représente un atome d'hydrogène, un radical aliphatique en C₁₋₆ non ramifié ou ramifié, saturé ou insaturé, facultativement au moins monosubstitué ou un radical cycloaliphatique en C₃₋₈ saturé ou insaturé, facultativement au moins monosubstitué, contenant facultativement au moins un hétéroatome en tant que chaînon de cycle, représente, de préférence, H ou un radical alkyle en C₁₋₃ ramifié ou non ramifié, et représente, plus préférablement, H, -CH₃ ou -CH₂CH₃.

4. Composés selon l'une quelconque des revendications 1 à 3, **caractérisés en ce que** R⁶, R⁷, R⁸ et R⁹ sont indépendamment choisis chacun parmi le groupe consistant en un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, un groupe cyano et une partie -COOR¹⁵, de préférence, choisis parmi le groupe consistant en H, un radical alkyle en C₁₋₃ ramifié ou non ramifié, un groupe cyano et un groupe -COOR¹⁵, plus préférablement, parmi le groupe consistant en H, -CH₃, -CH₂CH₃ et une partie cyano.

5. Composés selon l'une quelconque des revendications 1 à 4, **caractérisés en ce que** W représente un radical aliphatique en C₁₁₋₂₀ non ramifié ou ramifié, qui peut être substitué par un ou plusieurs substituants choisis parmi le groupe consistant en un groupe hydroxy, un atome d'halogène, les radicaux alkoxy en C₁₋₄ ramifiés ou non ramifiés, perfluoroalkoxy en C₁₋₄ ramifiés ou non ramifiés, perfluoroalkyle en C₁₋₄ ramifiés ou non ramifiés, amino, carboxy, amido, cyano, nitro, -SO₂NH₂, -CO-(alkyle en C₁₋₄), -SO-(alkyle en C₁₋₄), -SO₂-(alkyle en C₁₋₄), -NH-SO₂-(alkyle en C₁₋₄), dans lesquels la partie alkyle en C₁₋₄ peut être, dans chaque cas, ramifiée ou non ramifiée, un radical phényle ou naphtyle non substitué ou au moins monosubstitué, et les radicaux furannyle, thiényle, pyrrolyle, imidazolyle, pyrazolyle, pyridinyle, pyrimidinyle, quinolinyle et isoquinolinyle non substitués ou au moins monosubstitués, de sorte que lesdits substituants peuvent être au moins monosubstitués avec un substituant F, Cl, méthyle et méthoxy ; un groupe naphtyle qui est au moins monosubstitué, un groupe quinolinyle qui est au moins monosubstitué, un groupe pyrrolyle qui est au moins monosubstitué par un substituant autre qu'un groupe alkyle en C₁₋₅, un groupe thiazolyle, un groupe benzo[b]-thiophényle, un groupe benzo[b]-furannyle, un groupe isoquinolinyle, un groupe tétrahydroisoquinolinyle, un groupe pyrazolyle, un groupe isoazolyle, un groupe chromanyle, un groupe benzothiadiazolyle, un groupe imidazolyle, un groupe benzofurazannyle, un groupe dibenzo[b,d]-furannyle, un groupe benzoxadiazolyle, un groupe imidazo[2,1-b]-thiazolyle, un groupe anthracényle, un groupe coumarinyle, un groupe 2,3-dihydro-1,4-benzodioxinyle, un groupe 2,3-dihydrobenzo[b] furannyle, un groupe 3,4-dihydro-2H-1,4-benzoxazinyle, un groupe 3,4-dihydro-2H-1,5-benzodioxépinyle, un groupe benzothiazolyle, un groupe imidazo[1,2-a] pyridinyle facultativement au moins monosubstitués, un groupe chromonyle, un groupe isatinyle, un groupe pentaméthyldihydrobenzofurannyle, un groupe cyclopropyle ou cyclopentyle, de sorte que ledit groupe cyclopropyle ou cyclopentyle peut être substitué par un ou plusieurs substituants choisis parmi le groupe consistant en les groupes hydroxy, nitro, carboxy, cyano, céto, halogéno, alkyle en C₁₋₂₀, alkyle en C₁₋₄ partiellement fluorés, alkyle en C₁₋₄ partiellement chlorés, alkyle en C₁₋₄ partiellement bromés, alkoxy en C₁₋₅, alkoxy en C₁₋₄ partiellement fluorés, alkoxy en C₁₋₄ partiellement chlorés, alkoxy en C₁₋₄ partiellement bromés, alcényle en C₂₋₆, -SO₂-(alkyle en C₁₋₄), -(C=O)-(alkyle en C₁₋₅), -(C=O)-O-(alkyle en C₁₋₅), -(C=O)-Cl, -S-(alkyle en C₁₋₄), -(C=O)-H, -NH-(C=O)-NH-(alkyle en C₁₋₅), -(C=O)-perfluoro alkyle en C₁₋₄), -NR^{A}R^{B}, dans lesquels R^{A} et R^{B} sont indépendamment choisis parmi le groupe consistant en H, les radicaux alkyle en C₁₋₄ et phényle, NH-(C=O)-(alkyle en C₁₋₅), - (alkylène en C₁₋₅)-(C=O)-(alkyle en C₁₋₅), 1,3-dihydro-1-oxo-2H-isoindol-2-yle, N-phtalimidinyle, 1,3-dioxo-2-azaspiro[4,4]-non-2-yle, phényle substitués ou non substitués, -SO₂-phényle, phénoxy, pyridinyle, pyridinyloxy, pyrazolyle, pyrimidinyle, pyrrolidinyle, -SO₂-pyrrolidinyle, morpholinyle, -SO₂-morpholinyle, thiadiazolyle, oxadiazolyle, oxazolyle, thiazolyle, isoxazolyle, -O-CH₂-thiazolyle, -NH-phényle, et - (alkylène en C₁₋₄)-NH-(C=O)-phényle, dans lesquels lesdits substituants peuvent être substitués par un ou plusieurs substituants choisis parmi le groupe consistant en un atome d'halogène, les radicaux nitro, cyano, hydroxy, -(C=O)-(alkyle en C₁₋₄), alkyle en C₁₋₄, alkyle en C₁₋₄ au moins partiellement fluorés, alkyle en C₁₋₄ au moins partiellement chlorés, alkyle en C₁₋₄ au moins partiellement bromés, -S-(alkyle en C₁₋₄), -(C=O)-O-(alkyle en C₁₋₅), -(C=O)-CH₂-F, -(C=O)-CH₂-Cl et -(C=O)-CH₂-Br, un groupe 2-(1,3-dioxo-1,3-dihydro-isoindol-2-yl)-éthyle, un groupe thiényle, qui est au moins monosubstitué par un ou plusieurs substituants indépendamment choisis parmi le groupe consistant en F, Cl, Br, alkoxy en C₁₋₅, CF₃, -SO₂-(alkyle en C₁₋₅) et un groupe benzoylaminométhyle, phénylsulfonyle, isoxazolyle, benzamidométhyle, pyrimidyle, thiazolyle, pyrazolyle, phényle, 1,2,4-thiadiazolyle, 1,3-oxazolyle ou 1,2,4-oxadiazolyle, facultativement au moins monosubstitué, un groupe furyle qui est au moins monosubstitué par un ou plusieurs substituants indépendamment choisis parmi le groupe consistant en un radical alkyle en C₁₋₅, qui peut être au moins partiellement fluoré ou chloré, un groupe phényle facultativement au moins monosubstitué et un groupe -(C=O)-O-(alkyle en C₁₋₅),
une partie -NR¹⁶R¹⁷,
une partie -COR¹⁸, ou
un radical phényle, qui est au moins monosubstitué avec l'un des substituants choisis parmi le groupe consistant en :
les radicaux 2,2,2-trifluoroéthoxy, alcényle en C₂₋₆, 1,3-dihydro-1-oxo-2H-isoindol-2-yle, N-phtalimidinyle, (2-chloro-1,3-thiazolyl-5-yl)-méthoxy, 5-yl-2-méthyl-3-furoate d'éthyle, alkyle en C₁₁₋₂₀, 1,3-dioxo-2-azaspiro[4,4] non-2-yle, pyrazolyle, 1,3-oxazol-5-yle, 5-méthyl-1,3,4-oxadiazol-2-yle, difluorométhoxy, dichlorométhoxy, 1-pyrrolidinylsulfonyle, morpholinosulfonyle, 2-méthyl-4-pyrimidinyle, un groupe phénoxy qui est au moins monosubstitué avec un groupe alkoxy en C₁₋₅, un groupe phényle qui est au moins monosubstitué avec un des substituants choisis parmi le groupe consistant en les substituants nitro, alkoxy en C₁₋₅, F, Cl, Br, alkyle en C₁₋₅ au moins partiellement fluorés, alkyle en C₁₋₅ au moins partiellement chlorés, (2-chloro-1,3-thiazol-5-yl)-méthoxy, -(C=O)-H et -(C=O)- (alkyle en C₁₋₅), un groupe pyridinyle qui est au moins monosubstitué avec un groupe alkoxy en C₁₋₅, un groupe pyridinyloxy qui est au moins monosubstitué avec un groupe alkoxy en C₁₋₅, un groupe phénoxy qui est au moins disubstitué, et un groupe pyridinyloxy qui est au moins disubstitué,
W représente, plus préférablement, une partie choisie parmi le groupe consistant en les **radicaux** 5-diméthylamino-napht-1-y 1e , 2-acétamido-4-méthyl-5-thiazolyle, trifluorométhyle, trichlorométhyle, isopropyle, méthyle, 2,2,2-trifluoroéthyle, éthyle, hexadécyle, 2-chloroéthyle, n-propyle, 3-chloropropyle, n-butyle, dichlorométhyle, chlorométhyle, dodécyle, 1-octyle, 6-(p-toluidino)-napht-2-yle, 4,5-dibromo-thiophène-2-yle, (chlorure de benzoyle)-3-yle, 1-octadécyle, 4-bromo-2,5-dichloro-thiophène-3-yle, 2,5-dichloro-thiophène-3-yle, 5-chloro-thiophène-2-yle, 1-décyle, 3,5-dichloro-4-(2-chloro-4-nitrophénoxy)-phényle, 2,3-dichlorothiophène-5-yle, 3-bromo-2-chlorothiophène-5-yle , 3-bromo-5-chloro-thiophène-2-yle, 2-(benzoylaminométhyl)-thiophène-5-yle, 4-(phényl-sulfonyl)-thiophène-2-yle, 2-phényl-sulfonyl-thiophène-5-y 1 e , 2-[1-méthyl-5-(trifluorométhyl)pyrazol-3-yl]-thiophène-5-yle, 5-chloro-1,3-diméthylpyrazole-4-yle, 3,5-diméthylisoxazole-4-yle, 2-(2,4-dichlorophénoxy)-phényle, 4-(2-chloro-6-nitro-phénoxy)-phényle, 4-(3-chloro-2-cyanophénoxy)-phényle, 2,4-diméthyl-1,3-thiazole-5-yle, méthyl-méthane-sulfonyle, 2,5-bis-(2,2,2-trifluoroéthoxy)-phényle, 5-(di-n-propylamino)-napht-1-yle, 2,2,5,7,8-pentaméthyl-chroman-6-yle, 5-chloro-4-nitro-thiophène-2-yle, 2,1,3-benzothiadiazole-4-yle, 1-méthyl-imidazole-4-yle, benzofurazan-4-yle, 5-(isoxazol-3-yl)-thiophène-2-yle, vinyl-phényl-4-yle, 5-dichlorométhyl-furan-2-yle, 5-bromo-thiophène-2-yle, 5-(4-chlorobenzamidométhyl)-thiophène-2-yle, dibenzo[b,d]-furan-2-yle, 5-chloro-3-méthylbenzo[b]-thiophène-2-yle, 3-méthoxy-4-(méthoxycarbonyl)-thiophène-2-yle, 5-[2-(méthylthio)-pyrimidin-4-yl]-thiophène-2-yle, 4-chloro-2,1,3-benzoxadiazole-7-yle, 5-chloro-2,1,3-benzoxadiazole-4-yle, 6-chloro-imidazo (2,1-b)-thiazole-5-yle, 3-méthyl-benzo[b]-thiophène-2-yle, 4-[3-chloro-5-(trifluorométhyl)-2-pyridyl] oxy-phényle, 5-chloro-napht-1-yle, 5-chloronapht-2-yle, 9,10-dibromoanthracène-2-yle, isoquinoléine-5-yle, 4-méthoxy-2,3,6-triméthylbenzoyle, 4'-nitro-biphényl-4-yle, (1,3-dihydro-1-oxo-2H-isoindol-2-yl)-4-phényle, 5-(2-méthyl-1,3-thiazole-4-yl)-thiophène-2-yle, 5-[1-méthyl-3-(trifluorométhyl)pyrazol-5-yl]-thiophène-2-yle, 5-[5-trifluorométhyl)-isoxazol-3-yl]-thiophène-2-yle, p-dodécyl-phényle, 4-[(3-cyano-4-méthoxy-2-pyridinyl)oxy]-phényle, 4-(N-phtalimidinyl)-phényle, 1,2,3,4-tétrahydro-2-(trifluoroacétyl)-isoquinoléine-7-yle, 1,2-diméthylimidazole-4-yle, 2,2,4,6,7-pentaméthyldihydrobenzofuran-5-yle, 4-chloronapht-1-yle, 2,5-dichloro-4-nitro-thiophène-3-yle, 4-(4-méthoxy-phénoxy)-phényle, 4-(3,5-dichlorophénoxy)phényle, 4-(3,4-dichloro phénoxy)phényle, 4-(3,5)-bis (trifluorométhylphénoxy)phényle, 3-(2-méthoxy-phénoxy)-phényle, 3-(4-méthoxyphényl)-phényle, 3-(4-chloro-phényl)-phényle, 3-(3,5-dichloro-phényl)-phényle, 3-(3,4-dichloro-phényl)-phényle, 3-(4-fluoro phényl)-phényle, 3-[4-(trifluorométhyl)-phényl]-phényle, 3-[3,5-bis-(trifluorométhyl)-phényl]-phényle, 4-(2-méthoxy-phénoxy)-phényle, 4-(2-méthyl-phénoxy)-phényle, 4-(4-méthoxy-phénoxy)-phényle, 4-(4-chloro phényle)-phényle, 4-(3,5-dichloro phényl)-phényle, 4-(3,4-dichloro phényl)-phényle, 4-(4-fluorophényl)-phényle, 4-[4-(trifluorométhyl)-phényl]-phényle, 4-[3,5-bis-(trifluorométhyl)-phényl]-phényle, cyclopropyle, 2-(2-chlorophényl)-2-phényléthyle, 2-(2-trifluorométhylphényl)-2-phényléthyle, 5-[4-cyano-1-méthyl-5-(méthylthio)-1H-pyrazol-3-yl-thiophène-2-yle, 3-cyano-2,4-bis-(2,2,2-trifluorethoxy)-phényle, 4-[(2-chloro-1,3-thiazol-5-yl)-méthoxy]-phényle, 2-(1,3-dioxo-1,3-dihydro-isomdol-2-yl)-éthyle, 5-iodo-napht-1-yle, éthyl-2,5-diméthyl-1-phénylpyrrole-4-carboxylate-3-yle, éthyl-2-méthyl-1,5-diphényl-1H-pyrrole-3-carboxylate-4-yle, éthyl-5-(4-chlorophényl)-2-méthyl-3-furoate-4-yle, éthyl-5-(4-chlorophényl)-2-méthyl-1-phényl-3-carboxylate-4-yle, éthyl-2,5-diméthyl-3-furoate-4-yle, 3-chloro-4-(1,3-dioxo-2-azaspiro[4,4] non-2-yl)-phényle, coumarin-6-yle, 3-(4-méthoxy-phénoxy)-phényle, 3-(3,5-dichlorophénoxy)]-phényle , 3-(3,4-dichlorophénoxy)]-phényle, 3,5-bis (trifluorométhyl)phénoxyphényle, 2,2-diphényléthyle, 4-phényl-5-(trifluorométhyl)-thiophène-3-yle, méthyl-4-phényl-5-(trifluorométhyl)-thiophène-2-carboxylate-3-yle, méthyl-1,2,5-triméthylpyrrole-3-carboxylate-4-yle, 4-fluoro-napht-1-yle, 5-fluor-3-méthylbenzo[b]-thiophène-2-yle, méthyl-2,5-diméthyl-3-furoate-4-yle, méthyl-2-furoate-5-yle, méthyl-2-méthyl-3-furoate-5-yle, méthyl-1-méthyl-1H-pyrrole-2-carboxylate-5-yle, 2-(5-chloro-1,2,4-thiadiazol-3-yl)-thiophène-5-yle, 1,3,5-triméthyl-1H-pyrazole-4-yle, pentafluoroéthoxytétrafluoroéthyle, 5-(5-isoxazyl)-thiophène-2-yle, 5-(5-isoxazolyl)-2-furyle, 5-méthyl-2,1,3-benzothiadiazole-4-yle, 2,3-dihydro-1,4-benzodioxine-6-yle, 4-méthyl-napht-1-yle, 5-méthyl-2-(trifluorométhyl)-3-furyle, 2,3-dihydrobenzo[b] furan-5-yle, 1-benzothiophène-3-yle, 4-méthyl-3,4-dihydro-2H-1,4-benzoxazine-7-yle, 5-méthyl-1-phényl-1H-pyrazole-4-yle, 6-morpholino-3-pyridinyle, 4-(1H-pyrazol-1-yl)-phényle, 6-phénoxy-3-pyridyle, 3,4-dihydro-2H-1,5-benzodioxépine-7-yle, 5-(1,3-oxazol-5-yl)-2-thiényle, 4-(1,3-oxazol-5-yl)-phényle, 5-méthyl-4-isoxazolyle, 2,1,3- benzothiadiazole-5-yle, 5-acétamido-napht-1-yle, 3-méthyl-8-quinolinyle, 1,3-benzothiazole-6-yle, 2-morpholino-3-pyridyle, 2,5-diméthyl-3-thiényle, 5-[5-(chlorométhyl)-1,2,4-oxadiazol-3-yl]-2-thiényle, 3-[5-yl-2-thiényl]-1,2,4-oxadiazole-5-carboxylate d'éthyle, 3-(5-méthyl-1,3,4-oxadiazol-2-yl)-phényle, 4-(difluorométhoxy)-phényle, 3-(difluorométhoxy)-phényle, 2,2-diméthyl-6-chromanyle, éthyl-3,5-diméthyl-1H-pyrrole-2-carboxylate-4-yle, imidazo[1,2-A] pyridine-3-yle, 3-(1,3-oxazol-5-yl)-phényle, éthyl-5-[4-yl-phényl]-2-méthyl-3-furoate 1-pyrrolidinylphénylsulfonyle, méthyl-5-yl-4-méthyl-2-thiophène-carboxylate, méthyl-3-yl-4-(isopropylsulfonyl)-2-thiophène, 7-chlorochromone-3-yle, 4'-bromobiphényl-4-yle, 4'-acétyl-biphényl-4-yle, 4'-bromo-2'-fluoro-biphényl-4-yle, 1-méthyl-5-isatinyle, (acide 2-chloro-3-thiophènecarboxylique)-5-yle, 2-méthoxy-5-(N-phataliminidinyl)-phényle, 1-benzothiophène-2-yle, morpholinophénylsulfonyle et 3-(2-méthyl-4-pyrimidinyl)-phényle.

6. Composés selon l'une quelconque des revendications 1 à 5, **caractérisés en ce que** R¹⁰ représente un atome d'hydrogène, un radical aliphatique en C₁₋₆, non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆ facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, de préférence, H, un radical alkyle en C₁₋₄ linéaire ou ramifié, un radical cyclohexyle ou un radical phényle, plus préférablement, H, -CH₃, -C₂H₅ ou un radical phényle.

7. Composés selon l'une quelconque des revendications 1 à 6, **caractérisés en ce que** R¹¹ représente un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, de préférence, H, un radical alkyle en C₁₋₄ linéaire ou ramifié, un radical cyclohexyle ou un radical phényle, plus préférablement, H, -CH₃, -C₂H₅ ou un radical phényle.

8. Composés selon l'une quelconque des revendications 1 à 7, **caractérisés en ce que** R¹² représente un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, représente, de préférence, H, un radical alkyle en C₁₋₄ linéaire ou ramifié, un radical cyclohexyle ou un radical phényle, plus préférablement, H, -CH₃, -C₂H₅ ou un radical phényle.

9. Composés selon l'une quelconque des revendications 1 à 8, **caractérisés en ce que** R¹³ et R¹⁴ sont indépendamment choisis chacun parmi le groupe consistant en un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, sont, de préférence, indépendamment choisis chacun parmi le groupe consistant en H, un radical alkyle en C₁₋₄ linéaire ou ramifié, un radical cyclohexyle et un radical phényle, et sont, plus préférablement, indépendamment choisis chacun parmi le groupe consistant en H, -CH₃, -C₂H₅ et un radical phényle.

10. Composés selon l'une quelconque des revendications 1 à 8, **caractérisés en ce que** R¹³ et R¹⁴ forment ensemble avec l'atome d'azote pontant, un noyau hétérocyclique à 5 ou 6 chaînons, saturé, insaturé ou aromatique, qui peut être au moins monosubstitué et/ou contenir au moins un autre hétéroatome en tant que chaînon de cycle, et forment, de préférence, un groupe pipéridine ou morpholine non substitué.

11. Composés selon l'une quelconque des revendications 1 à 10, **caractérisés en ce que** R¹⁵ représente un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, représente, de préférence, H, un radical alkyle en C₁₋₄ linéaire ou ramifié, un radical cyclohexyle ou un radical phényle, plus préférablement, H, -CH₃, -C₂H₅ ou un radical phényle.

12. Composés selon l'une quelconque des revendications 1 à 11, **caractérisés en ce que** R¹⁶ représente un radical aliphatique en C₁₋₆, non ramifié ou ramifié, saturé ou insaturé, facultativement au moins monosubstitué, de préférence, un radical alkyle saturé, non substitué en C₁₋₃, non ramifié ou ramifié, plus préférablement, un radical méthyle.

13. Composés selon l'une quelconque des revendications 1 à 12, **caractérisés en ce que** R¹⁷ représente un radical aliphatique en C₁₋₆, non ramifié ou ramifié, saturé ou insaturé, facultativement au moins monosubstitué, de préférence, un radical alkyle saturé, non substitué en C₁₋₃, non ramifié ou ramifié, plus préférablement, un radical méthyle.

14. Composés selon une ou plusieurs des revendications 1 à 13 :
la 1-[1-(5-chloro-3-méthyl-benzo[b] thiophényl-2-sulfonyl)-pipéridine-4-yl]-1,4-dihydro-benzo[d][1,3]oxazin-2-one,
la 1-[1-(5-diméthylamino-naphtyl-1-sulfonyl)-pipéridine-4-yl]-8-méthyl-1,4-dihydro-benzo[d][1,3] oxazin-2-one,
la 1-[1-(5-diméthylamino-naphtyl-1-sulfonyl)-pipéridine-4-yl]-1,4-dihydro-benzo[d][1,3] oxazin-2-one, et
les sels correspondants de ceux-ci et les solvats correspondants.

15. Procédé pour la préparation de composés de type sulfonamide dérivés de benzoxazinone de formule générale (I) selon une ou plusieurs des revendications 1 à 14, **caractérisé en ce qu'**il comprend la réaction d'au moins un composé dérivé de pipéridine de formule générale (II), dans laquelle R¹ à R⁹ ont la définition selon la revendication 1, et/ou d'un sel, de préférence, un sel de type chlorhydrate de ceux-ci, 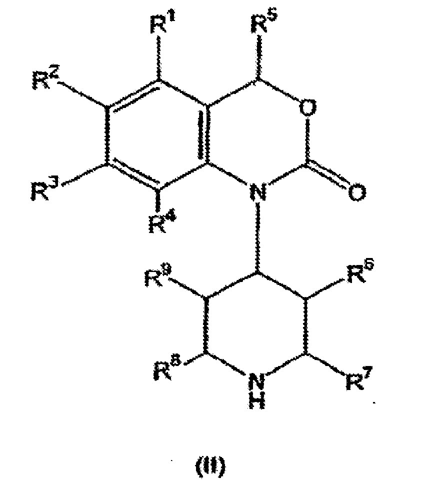 avec au moins un composé de formule générale (III), 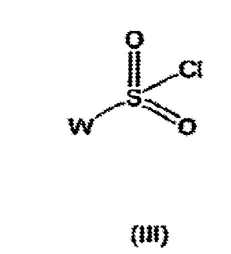 dans laquelle W a la définition selon la revendication 1, dans un milieu de réaction approprié, facultativement en présence d'au moins une base et/ou d'au moins un agent auxiliaire.

16. Procédé pour la préparation d'un sel physiologiquement acceptable des composés et de type sulfonamide dérivés de benzoxazinone selon les revendications 1 à 14, **caractérisé en ce qu'**on fait réagir au moins un composé de formule générale (I), comportant au moins un groupe basique, avec au moins un acide, de préférence, un acide inorganique ou organique, de préférence, en présence d'un milieu de réaction approprié.

17. Procédé pour la préparation d'un sel physiologiquement acceptable des composés et de type sulfonamide dérivés de benzoxazinone selon les revendications 1 à 14, **caractérisé en ce qu'**on fait réagir au moins un composé de formule générale (I), comportant au moins un groupe acide, avec au moins une base, de préférence, en présence d'un milieu de réaction approprié.

18. Médicament comprenant au moins un composé de type sulfonamide dérivé de benzoxazinone selon l'une quelconque des revendications 1 à 14, facultativement sous la forme d'un de ses stéréoisomères, de préférence, les énantiomères ou les diastéréoisomères, son racémate, ou sous la forme d'un mélange d'au moins deux de ses stéréoisomères, en un quelconque rapport de mélange, ou, respectivement, un sel physiologiquement acceptable de celui-ci, ou un solvat, et facultativement, un ou plusieurs adjuvants pharmaceutiquement acceptables.

19. Médicament selon la revendication 18 pour la stimulation cognitive, pour la prophylaxie et/ou le traitement des troubles de l'ingestion d'aliments (consommation d'aliments), en particulier, pour la régulation de l'appétit, pour le maintien, l'accroissement ou la diminution du poids corporel, pour la prophylaxie et/ou le traitement de l'obésité, la boulimie, l'anorexie, la cachéxie ou le diabète de type II (diabète sucré non insulinodépendant), de préférence, le diabète de type II, qui est provoqué par l'obésité, de troubles du système nerveux central, de troubles du tube digestif, tels que le syndrome de l'intestin irritable, l'anxiété, la panique, la dépression, les troubles de la mémoire cognitive, les troubles de démence sénile, tels que la maladie d'Alzheimer, la maladie de Parkinson et la maladie de Huntington, la schizophrénie, une psychose, l'hyperkinésie infantile, ou les troubles d'hyperactivité et de déficit de l'attention (ADHC).

20. Utilisation d'au moins un composé de type sulfonamide dérivé de benzoxazinone selon l'une quelconque des revendications 1 à 14, facultativement sous la forme d'un de ses stéréoisomères, de préférence, les énantiomères ou les diastéréoisomères, son racémate, ou sous la forme d'un mélange d'au moins deux de ses stéréoisomères, en un quelconque rapport de mélange, ou d'un sel physiologiquement acceptable de celui-ci, ou d'un solvat, pour la fabrication d'un médicament pour la stimulation cognitive, pour la prophylaxie et/ou le traitement des troubles de l'ingestion d'aliments (consommation d'aliments), en particulier, pour la régulation de l'appétit, pour le maintien, l'accroissement ou la diminution du poids corporel, pour la prophylaxie et/ou le traitement de l'obésité, la boulimie, l'anorexie, la cachéxie ou le diabète de type II (diabète sucré non insulinodépendant), de préférence, le diabète de type II, qui est provoqué par l'obésité, de troubles du système nerveux central, de troubles du tube digestif, tels que le syndrome de l'intestin irritable, l'anxiété, la panique, la dépression, les troubles de la mémoire cognitive, les troubles de démence sénile, tels que la maladie d'Alzheimer, la maladie de Parkinson et la maladie de Huntington, la schizophrénie, une psychose, l'hyperkinésie infantile, ou les troubles d'hyperactivité et de déficit de l'attention (ADHC).

21. Utilisation d'au moins un composé de type sulfonamide dérivé de benzoxazinone de formule générale (Ia), 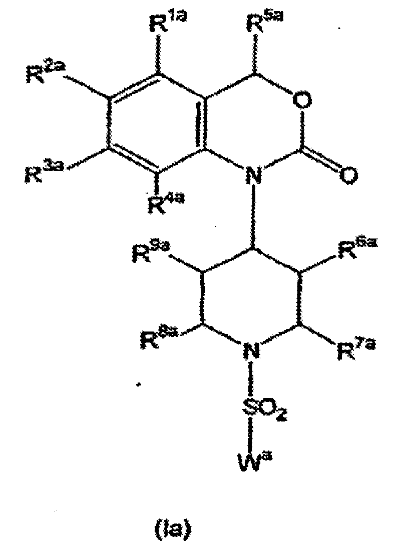 dans laquelle :
R^{1a}, R^{2a} R^{3a} et R^{4a} sont indépendamment choisis chacun parmi le groupe consistant en un atome d'hydrogène, d'halogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué et/ou peut être condensé avec un système de noyau mono ou polycyclique facultativement au moins monosubstitué, un radical aryle ou hétéroaryle facultativement au moins monosubstitué, qui peut être lié par l'intermédiaire d'un groupe alkylène facultativement au moins monosubstitué et/ou peut être condensé avec un système de noyau mono ou polycyclique facultativement au moins monosubstitué, un groupe nitro, un groupe cyano, et les parties -OR^{10a}, -OC(=O)-R^{11a}, -(C=O)-OR^{11a}, -SR^{12a}, -SOR^{12a}, -SO₂R^{12a}, -NH-SO₂R^{12a}, -SO₂NH₂ et -NR^{13a}R^{14a},
R^{5a} représente un atome d'hydrogène, un radical aliphatique non ramifié ou ramifié, saturé ou insaturé, facultativement au moins monosubstitué ou un radical cycloaliphatique saturé ou insaturé, facultativement au moins monosubstitué, contenant facultativement au moins un hétéroatome en tant que chaînon de cycle,
R^{6a}, R^{7a} ,R^{8a} et R^{9a} sont indépendamment choisis chacun parmi le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, un groupe cyano et une partie -COOR^{15a},
W^{a} représente un radical aliphatique non ramifié ou ramifié, saturé ou insaturé, facultativement au moins monosubstitué, un radical cycloaliphatique saturé ou insaturé, contenant facultativement au moins un hétéroatome en tant que chaînon de cycle, facultativement au moins monosubstitué, qui peut être lié par l'intermédiaire d'un groupe alkylène facultativement monosubstitué, et/ou peut être condensé avec un système de noyau aromatique mono ou polycyclique facultativement au moins monosubstitué, un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, une partie -NR^{16a}R^{17a} ou une partie COR¹⁸a
R^{10a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{11a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{12a} représente un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{13a} et R^{14a} sont indépendamment choisis chacun parmi le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, ou
R^{13a} et R^{14a} forment ensemble avec l'atome d'azote pontant, un noyau hétérocyclique saturé, insaturé ou aromatique, qui peut être au moins monosubstitué et/ou contenir au moins un autre hétéroatome en tant que chaînon de cycle,
R^{15a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{16a} représente un radical aliphatique non ramifié ou ramifié, saturé ou insaturé, facultativement au moins monosubstitué,
R^{17a} représente un radical aliphatique non ramifié ou ramifié, saturé ou insaturé, facultativement au moins monosubstitué, et
R^{18a} représente un radical aryle facultativement au moins monosubstitué,
facultativement sous la forme de l'un de ses stéréoisomères, de préférence, les énantiomères ou les diastéréoisomères, son racémate ou sous la forme d'un mélange d'au moins deux de ses stéréoisomères, de préférence, les énantiomères ou les diastéréoisomères, en un quelconque rapport de mélange, ou, respectivement, d'un sel physiologiquement acceptable de celui-ci, ou d'un solvat,
pour la fabrication d'un médicament pour la prophylaxie et/ou le traitement des troubles de l'ingestion d'aliments ; l'anxiété ; la panique ; la dépression ; les troubles cognitifs, de préférence, les troubles de la mémoire ; les processus de démence sénile, de préférence, choisis parmi le groupe consistant en la maladie d'Alzheimer, la maladie de Parkinson et la maladie de Huntington; une psychose ; l'hyperkinésie infantile ; les troubles d'hyperactivité et de déficit de l'attention (ADHC) ; des troubles du tube digestif, de préférence, un syndrome intestinal ; la schizophrénie ou pour la stimulation cognitive.

22. Utilisation selon la revendication 21, **caractérisée en ce que** R^{1a}, R^{2a}, R^{3a} et R^{4a} sont indépendamment choisis chacun parmi le groupe consistant en H, F, Cl et Br, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆ facultativement au moins monosubstitué et/ou peut être condensé avec un système de noyau mono ou polycyclique facultativement au moins monosubstitué, un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, un groupe nitro, un groupe cyano, et les parties -OR^{10a}, -OC(=O)-R^{11a}, -SR^{12a}, -SOR^{12a}, -SO₂R^{12a}, -NH-SO₂R^{12a}, -SO₂NH₂ et NR^{13a}R^{14a}, de préférence, choisis parmi le groupe consistant en H, F, Cl et Br, un radical aliphatique en C₁₋₃ non ramifié ou ramifié, saturé ou insaturé, facultativement au moins monosubstitué, un radical cycloaliphatique en C₅ ou C₆ saturé ou insaturé, facultativement au moins monosubstitué, contenant facultativement au moins un hétéroatome en tant que chaînon de cycle, qui peut être lié par l'intermédiaire d'un groupe alkylène en C₁ ou C₂ facultativement au moins monosubstitué, un groupe nitro, un groupe cyano, et les parties OR^{10a}, -OC(=O)-R^{11a}, -SR^{12a} et -NR^{13a}R^{14a}, plus préférablement, choisis parmi le groupe consistant en H, F, Cl, -CH₃, -CH₂CH₃, -CF₃, -CF₂CF₃, les groupes cyclopentyle, cyclohexyle, nitro, cyano et -OR^{10a}.

23. Utilisation selon la revendication 21 ou 22, **caractérisée en ce que** R^{5a} représente un atome d'hydrogène, un radical aliphatique en C₁₋₆ non ramifié ou ramifié, saturé ou insaturé, facultativement au moins monosubstitué ou un radical cycloaliphatique en C₃₋₈ saturé ou insaturé, facultativement au moins monosubstitué, contenant facultativement au moins un hétéroatome en tant que chaînon de cycle, représente, de préférence, H ou un radical alkyle en C₁₋₃ ramifié ou non ramifié, et représente, plus préférablement, H, -CH₃ ou -CH₂CH₃.

24. Utilisation selon l'une quelconque des revendications 21 à 23, **caractérisée en ce que** R^{6a}, R^{7a}, R^{8a} et R^{9a} sont indépendamment choisis chacun parmi le groupe consistant en un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, un groupe cyano et une partie -COOR^{15a} , de préférence, choisis parmi le groupe consistant en H, un radical alkyle en C₁₋₃ ramifié ou non ramifié, un groupe cyano et un groupe -COOR^{15a}, plus préférablement, parmi le groupe consistant en H, -CH₃, -CH₂CH₃ et une partie cyano.

25. Utilisation selon l'une quelconque des revendications 21 à 24, **caractérisée en ce que** W^{a} représente un radical aliphatique en C₁₋₂₀, non ramifié ou ramifié, saturé ou insaturé, facultativement au moins monosubstitué, un radical cycloaliphatique en C₃₋₈, saturé ou insaturé, contenant facultativement au moins un hétéroatome en tant que chaînon de cycle, facultativement au moins monosubstitué, qui peut être lié par l'intermédiaire d'un groupe alkylène en C₁₋₆, facultativement monosubstitué, et/ou peut être condensé avec un système de noyau aromatique mono ou polycyclique facultativement au moins monosubstitué, un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, une partie -NR^{16a}R^{17a} ou une partie -COR^{18a}, et
est, de préférence, choisi parmi le groupe consistant en les radicaux 1-naphtyle, 5-diméthylamino-napht-1-yle, 2-naphtyle, 2-acétamido-4-méthyl-5-thiazolyle, 2-thiényle, 8-quinolinyle, phényle, pentafluorophényle, 2,4,5-trichlorophényle, 2,5-dichlorophényle, 2-nitrophényle, 2,4-dinitrophényle, 3,5-dichloro-2-hydroxyphényle, 2,4,6-triisopropylphényle, 2-mésityle, 3-nitro-phényle, 4-bromophényle, 4-fluorophényle, 4-chlorophényle, 4-chloro-3-nitro-phényle, 4-iodo-phényle, N-acétylsulfaninyle, 4-nitro-phényle, 4-méthoxy-phényle, (acide benzoïque)-4-yle, 4-tert-butyl-phényle, p-tolyle, trifluorométhyle, trichlorométhyle, isopropyle, méthyle, benzyle, trans-styryle, 2,2,2-trifluoroéthyle, éthyle, hexadécyle, 2-chloroéthyle, n-propyle, 3-chloropropyle, n-butyle, (benzoate de méthyle)-2-yle, 2-nitro-4-(trifluorométhyl)-phényle, pentaméthyl-phényle, 2,3,5,6-tétraméthyl-phényle, 3-(trifluorométhyl)-phényle, 3,5-bis-(trifluorométhyl)-phényle, dichlorométhyle, chlorométhyle, dodécyle, 1-octyle, 2,3,4-trichloro-phényle, 2,5-diméthoxy-phényle, o-tolyle, p-xylyl-2-yle, (acide benzoïque)-3-y 1 e , 4-chloro-3-(trifluorométhyl)-phényle , (acide 4-chloro-5-nitrobenzoïque)-3-yle, 6-(p-toluidino)-napht-2-yle, 4-méthoxy-2,3,6-triméthylphényle, 3,4-dichlorophényle, 4,5-dibromo-thiophène-2-yle, 3-chloro-4-fluoro-phényle 4-éthyl-phényle, 4-n-propyl-phényle, 4-(1,1-diméthylpropyl)-phényle, 4-isopropyl-phényle, 4-bromo-2,5-difluoro-phényle, 2-fluoro-phényle, 3-fluoro-phényle, 4-(trifluorométhoxy)-phényle, 4-(trifluorométhyl)-phényle, 2,4-difluoro-phényle, 2,4-dichloro-5-méthyl-phényle, 4-chloro-2,5-diméthyl-phényle, 5-diéthylamino-napht-2-yle, (chlorure de benzoyle)-3-yle, 2-chloro-phényle, 1-octadécyle, 4-bromo-2,5-dichloro-thiophène-3-yle, 2,5-dichloro-thiophène-3-yle, 5-chloro-thiophène-2-yle, 2-méthyl-5-nitro-phényle, 2-(trifluorométhyl)-phényle, 3-chloro-phényle, 3,5-dichloro-phényle, 1-décyle, 3-méthyl-phényle, 2-chloro-6-méthyle, 5-bromo-2-méthoxy-phényle, 3,4-diméthoxy-phényle, 2,3-dichloro-phényle, 2-bromo-phényle, 3,5-dichloro-4-(2-chloro-4-nitrophénoxy)-phényle, 2,3-dichlorothiophène-5-yle, 3-bromo-2-chloro-thiophène-5-yle, 3-bromo-5-chloro-thiophène-2-yle, 2-(benzoylaminométhyl)-thiophène-5-yle, 4-(phényl-sulfonyl)-thiophène-2-yle, 2-phényl-sulfonyl-thiophène-5-yle, 3-chloro-2-méthyl-phényle, 2-[1-méthyl-5-(trifluorométhyl)pyrazol-3-yl]-thiophène-5-yle, 5-pyrid-2-yl-thiophène-2-yle, 2-chloro-5-(trifluorométhyl)-phényle, 2,6-dichloro-phényle, 3-bromo-phényle, 2-(trifluorométhoxy)-phényle, 4-cyano-phényle, 2-cyano-phényle, 4-n-butoxy-phényle, 4-acétamido-3-chloro-phényle, 2,5-dibromo-3,6-difluoro-phényle, 5-chloro-1,3-diméthylpyrazole-4-yle, 3,5-diméthylisoxazole-4-yle, 2-(2,4-dichlorophénoxy)-phényle, 4-(2-chloro-6-nitro-phénoxy)-phényle, 4-(3-chloro-2-cyanophénoxy)-phényle, 2,4-dichlorophényle, 2,4-diméthyl-1,3-thiazole-5-yle, méthyl-méthane-sulfonyle, 2,5-bis-(2,2,2-trifluoroéthoxy)-phényle, 2-chloro-4-(trifluorométhyl)-phényle, 2-chloro-4-fluoro-phényle, 5-fluoro-2-méthyl-phényle, 5-chloro-2-méthoxy-phényle, 2,4,6-trichloro-phényle, (acide 2-hydroxy-benzoïque)-5-yle, 5-(di-n-propylamino)-napht-1-yle, 6-méthoxy-m-tolyle, 2,5-difluoro-phényle, 2,4-diméthoxy-phényle, 2,5-dibromophényle, 3,4-dibromo-phényle, 2,2,5,7,8-pentaméthyl-chroman-6-yle, (acide 2-méthoxy-benzoïque)-5-yle, 5-chloro-4-nitro-thiophène-2-yle, 2,1,3-benzothiadiazole-4-yle, 1-méthyl-imidazole-4-yle, benzofurazan-4-yle, 2-(méthoxycarbonyl)-thiophène-3-yle, 5-(isoxazol-3-yl)-thiophène-2-yle, 2,4,5-trifluoro-phényle, biphényl-4-yle, vinylphényl-4-yle, 2-nitro-benzyle, 5-dichloro-méthyl-furan-2-yle, 5-bromo-thiophène-2-yle, 5-(4-chlorobenzamidométhyl)-thiophène-2-yle, 2,6-difluoro-phényle, 2,5-diméthoxy-4-nitro-phényle, dibenzo[b,d]-furan-2-yle, 2,3,4-trifluoro-phényle, 3-nitro-p-tolyle, 4-méthoxy-2-nitro-phényle, 3,4-difluoro-phényle, 4-(bromoéthyl)-phényle, 3,5-dichloro-4-hydroxy-phényle, 4-n-amyl-phényle, 5-chloro-3-méthylbenzo[b]-thiophène-2-yle, 3-méthoxy-4-(méthoxycarbonyl)-thiophène-2-yle, 4-n-butyl-phényle, 2-chloro-4-cyano-phényle, 5-[2-(méthylthio)-pyrimidin-4-yl]-thiophène-2-yle, 3,5-dinitro-4-méthoxy-phényle, 4-bromo-2-(trifluorométhoxy)-phényle, 4-chloro-2,1,3-benzoxadiazole-7-yle, 2-(1-naphtyl)-éthyle, 3-cyano-phényle, 5-chloro-2,1,3-benzoxadiazole-4-yle, 3-chloro-4-méthyl-phényle, 4-bromo-2-éthyl-phényle, 2,4-dichloro-6-méthyl-phényle, 6-chloro-imidazo (2,1-B)-thiazole-5-yle, 3-méthyl-benzo[b]-thiophène-2-yle, 4-méthyl-sulfonyl-phényle, 2-méthyl-sulfonyl-phényle, 4-bromo-2-méthyl-phényle, 2,6-dichloro-4-(trifluorométhyl)-phényle, 4-[3-chloro-5-(trifluorométhyl)-2-pyridyl] oxy-phényle, 5-chloro-napht-1-yle, 5-chloro-napht-2-yle, 9,10-dibromoanthracène-2-yle, isoquinoléine-5-yle, 4-méthoxy-2,3,6-triméthylbenzoyle, 4'-nitro-biphényl-4-yle, (4-phénoxy)-phényle, (1,3-dihydro-1-oxo-2H-isoindol-2-yl)-4-phényle, 4-acétyl-phényle, 5-(2-méthyl-1,3-thiazole-4-yl)-thiophène-2-yle, 5-[1-méthyl-3-(trifluorométhyl)pyrazol-5-yl]-thiophène-2-yle, 5-[5-trifluorométhyl)-isoxazol-3-yl]-thiophène-2-yle, 2-iodo-phényle, p-dodécyl-phényle, 4-[(3-cyano-4-méthoxy-2-pyridinyl)oxy]-phényle, 4-(N-phtalimidinyl)-phényle, 1,2,3,4-tétrahydro-2-(trifluoroacétyl)-isoquinoleine-7-yle, 4-bromo-2-fluoro-phényle, 2-fluoro-5-(trifluorométhyl)-phényle, 4-fluoro-2-(trifluorométhyl)-phényle, 4-fluoro-3-(trifluorométhyl)-phényle, 2,4,6-trifluoro-phényle, 3-(trifluorométhoxy)-phényle, 1,2-diméthylimidazole-4-yle, éthyl-4-carboxylate-3-yle, 2,2,4,6,7-pentaméthyldihydrobenzofuran-5-yle, 3-bromo-2-chloropyridine-5-yle, 3-méthoxy-phényle, 2-méthoxy-4-méthyl-phényle, (acide 2-chloro-4-fluorobenzoïque)-5-yle, 4-chloro-napht-1-yle, 2,5-dichloro-4-nitro-thiophène-3-yle , 4-(4-méthoxyphénoxy)-phényle, 4-(4-chloro-phénoxy)-phényle, 4-(3,5-dichlorophénoxy)phényle, 4-(3,4-dichloro phénoxy)phényle, 4-(4-fluoro-phénoxy)-phényle, 4-(4-méthyl-phénoxy)-phényle , 4-[4-(trifluorométhyl)-phénoxy]-phényle, 4-(3,5)-bis (trifluorométhylphénoxy)] phényle, 3-(2-méthoxy-phénoxy)-phényle, 3-(2-chlorophénoxy)-phényle, 3-(2-méthyl-phénoxy)-phényle, 4-[2-(trifluorométhyl)-phénoxy]-phényle, 3-phényl-phényle, 3-(4-méthoxy-phényl)-phényle, 3-(4-chloro-phényl)-phényle, 3-(3,5-dichloro-phényl)-phényle, 3-(3,4-dichloro-phényl)-phényle, 3-(4-fluoro phényl)-phényle, 3-(4-méthylphényl)-phényle, 3-[4-(trifluorométhyl)-phényl]-phényle, 3-[3,5-bis-(trifluorométhyl)-phényl]-phényle, 4-(4-pyridyloxy)-phényle, 4-(2-méthoxyphénoxy)-phényle, 4-(2-chloro-phénoxy)-phényle, 4-(2-méthyl-phénoxy)-phényle, 4-(4-méthoxy-phénoxy)-phényle, 4-(4-chloro phényle)-phényle, 4-(3,5-dichloro phényl)-phényle, 4-(3,4-dichloro phényl)-phényle, 4-(4-fluorophényl)-phényle, 4-(4-méthylphényl)-phényle, 4-[4-(trifluorométhyl)-phényl]-phényle , 4-[3,5-bis-(trifluorométhyl)-phényl]-phényle, 3-(trifluorométhyl)-phényl]-phényl, (4-chlorophényl)-méthyle, (3,5-dichlorophényl)-méthyle, (4-fluorophényl)-méthyle, 4-méthylphénylméthyle, 4-(trifluorométhyl)-phényl]-méthyle, cyclopropyle, 2-(2-chlorophényl)-2-phényléthyle, 2-(2-trifluorométhylphényl)-2-phényléthyle, 5-[4-cyano-1-méthyl-5-(méthylthio)-1H-pyrazol-3-yl-thiophène-2-yle, 3-cyano-2,4-bis-(2,2,2-trifluorethoxy)-phényle, 4-[(2-chloro-1,3-thiazol-5-yl)-méthoxy]-phényle, 3-nitrophénylméthyl, 4-formylphényle, 2-(1,3-dioxo-1,3-dihydro-isoindol-2-yle)-éthyle, 3,5-bis-(trifluorométhyl)-phényl]-méthyle, 4-(2-pyridyloxy)-phényle, 4-(3-pyridyloxy)-phényle, 5-iodo-napht-1-yle, éthyl-2,5-diméthyl-1-phénylpyrrole-4-carboxylate-3-yle, éthyl-2-méthyl-1,5-diphényl-1H-pyrrole-3-carboxylate-4-yle, éthyl-5-(4-chlorophényl)-2-méthyl-3-furoate-4-yle, éthyl-5-(4-chlorophényl)-2-méthyl-1-phényl-3-carboxylate-4-yle, éthyl-2,5-diméthyl-3-furoate-4-yle, 3-chloro-4-(1,3-dioxo-2-azaspiro[4,4] non-2-yl)-phényle, 5-bromo-2,4-difluoro-phényle, 5-chloro-2,4-difluorophényle, coumarin-6-yle, 2-méthoxy-phényle, (3-phénoxy)-phényle, 3-(4-méthoxy-phénoxy)-phényle, 3-(4-chlorophénoxy)-phényle, 3-(3,5-dichlorophénoxy)]-phényle, 3-(3,4-dichlorophénoxy)]-phényle, 3-(4-fluorophénoxy)-phényle, 3-(4-méthylphénoxy)-phényle, 3-[4-(trifluorométhyl)-phénoxy]-phényle, 3-[3,5-(trifluorométhyl)-phénoxy]-phényle, 3-[2-(trifluorométhyl)-phénoxy]-phényle, 2,2-diphényléthyle, 4-phényl-5-(trifluorométhyl)-thiophène-3-yle, méthyl-4-phényl-5-(trifluorométhyl)-thiophène-2-carboxylate-3-yle, méthyl-1,2,5-triméthylpyrrole-3-carboxylate-4-y 1 e , 4-fluoro-napht-1-yle, 3,5-difluorophényle, 3-fluoro-4-méthoxy-phényle, 4-chloro-2,5-difluorophényle, 2-chloro-4,5-difluoro-phényle, 5-fluoro-3-méthylbenzo[b]-thiophène-2-yle, méthyl-3-phénylproprionate-4-yle, (acide dihydrocinnamique)-4-yle, méthyl-2,5-diméthyl-3-furoate-4-yle, méthyl-2-furoate-5-yle, méthyl-2-méthyl-3-furoate-5-yle, méthyl-1-méthyl-1H-pyrolle-2-carboxylate-5-yle, 2-(5-chloro-1,2,4-thiadiazol-3-yl)-thiophène-5-yle, 1,3,5-triméthyl-1H-pyrazole-4-yle , 3-chloro-5-fluoro-2-méthoxyphényle, pentafluoroéthoxytétrafluoroéthyle, 5-(5-isoxazyl)-thiophène-2-yle, 5-(5-isoxazolyl)-2-furyle, 5-méthyl-2,1,3-benzothiadiazole-4-yle, biphényl-2-yle, 2,3-dihydro-1,4-benzodioxine-6-yle, 4-méthyl-napht-1-yle, 5-méthyl-2-(trifluorométhyl)-3-furyle, 2,3-dihydrobenzo[b] furan-5-yle, 1-benzothiophène-3-yle, 4-méthyl-3,4-dihydro-2H-1,4-benzoxazine-7-yle, 5-méthyl-1-phényl-1H-pyrazole-4-yle, 6-morpholino-3-pyridinyle, 4-(1H-pyrazol-1-yle)-phényle, 6-phénoxy-3-pyridyle, 3 , 4-dihydro-2H-1,5-benzodioxépine-7-yle, 5-(1,3-oxazol-5-yl)-2-thiényle, 4-(1,3-oxazol-5-yl)-phényle, 5-méthyl-4-isoxazolyle, 2,1,3-benzothiadiazole-5-yle, 3-thiényle, 2-méthyl-benzyle, 3-chloro-benzyle, 5-acétamido-napht-1-yle, 3-méthyl-8-quinolinyle, 4-chloro-2-nitrophényle, 6-quinolinyle, 1,3-benzothiazole-6-yle, 2-morpholino-3-pyridyle, 2,5-diméthyl-3-thiényle, 5-[5-(chlorométhyl)-1,2,4-oxadiazol-3-yl]-2-thiényle, éthyl-3-[5-yl-2-thiényl]-1,2,4-oxadiazole-5-carboxylate, 3-(5-méthyl-1,3,4-oxadiazol-2-yl)-phényle, 4-isopropoxyphényle, 2,4-dibromophényle, 3-cyano-4-fluorophényle, 2,5-bis-(trifluorométhyl)-phényle, 2-bromo-4-fluorophényle, 4-bromo-3-fluorophényle, 4-(difluorométhoxy)-phényle, 3-(difluorométhoxy)-phényle, 5-chloro-2-fluoro-phényle, 3-chloro-2-fluorophényle, 2-fluoro-4-méthylphényle, 4-nitro-3-(trifluorométhyl)-phényle, 3-fluoro-4-méthylphényle, 4-fluoro-2-méthylphényle, 4-bromo-3-(trifluorométhyl)-phényle, 4-bromo-2-(trifluorométhyl)-phényle, 3-bromo-5-(trifluorométhyl)-phényle, 2-bromo-4-(trifluorométhyl)-phényle, 2-bromo-5-(trifluorométhyl)-phényle, 2,4-dichloro-5-fluorophényle, 4,5-dichloro-2-fluorophényle, 3,4,5-trifluorophényle, 4-chloro-2-fluorophényle, 2-bromo-4,6-difluorophényle, 2-éthylphényle, 4-bromo-2-chlorophényle, 4-bromo-2,6-dichlorophényle, 2-bromo-4,6-dichloro-phényle, 4-bromo-2,6-diméthylphényle, 3,5-diméthylphényle, 4-bromo-3-méthylphényle, 2-méthoxy-4-nitrophényle, 2,2-diméthyl-6-chromanyle, éthyl-3,5-diméthyl-1H-pyrrole-2-carboxylate-4-yle, imidazo[1,2-A] pyridine-3-yle, 3-(1,3-oxazol-5-yl)-phényle, éthyl-5-[4-yl-phényl]-2-méthyl-3-furoate, méthyl-3-(yl)-4-méthoxybenzoate, 1-pyrrolidinylphénylsulfonyle, méthyl-5-yl-4-méthyl-2-thiophènecarboxylate, méthyl-3-yl-4-(isopropylsulfonyl)-2-thiophène, 2-pyridyle, 3-fluoro-4-nitrophényle, 7-chlorochromone-3-yle, 4'-bromobiphényl-4-yle, 4'-acétyl-biphényl-4-yle, 4'-bromo-2'-fluoro-biphényl-4-yle, 2-chloro-4-(3-propyl-uréido)-phényle, 3-(bromoacétyl)-phényle, 2-bromo-3-(trifluorométhyl)-phényle, 1-méthyl-5-isatinyle, 4-(acide isopropyl benzoïque)-3-yle, (acide 2-chloro-3-thiophènecarboxylique)-5-yle, 3-pyridyle, cyclohexylméthyle, 2-méthoxy-5-(N-phataliminidinyl)-phényle, 1-benzothiophène-2-yle, morpholinophénylsulfonyle, 3-(2-méthyl-4-pyrimidinyl)-phényle et 2-cyano-5-méthylphényle.

26. Utilisation selon l'une quelconque des revendications 21 à 25, **caractérisée en ce que** R^{10a} représente un atome d'hydrogène, un radical aliphatique en C₁₋₆, non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆ facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, de préférence, H, un radical alkyle en C₁₋₄ linéaire ou ramifié, un radical cyclohexyle ou un radical phényle, plus préférablement, H, -CH₃, -C₂H₅ ou un radical phényle.

27. Utilisation selon l'une quelconque des revendications 21 à 26, **caractérisée en ce que** R^{11a} représente un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, de préférence, H, un radical alkyle en C₁₋₄ linéaire ou ramifié, un radical cyclohexyle ou un radical phényle, plus préférablement, H, -CH₃, -C₂H₅ ou un radical phényle.

28. Utilisation selon l'une quelconque des revendications 21 à 27, **caractérisée en ce que** R^{12a} représente un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, représente, de préférence, H, un radical alkyle en C₁₋₄ linéaire ou ramifié, un radical cyclohexyle ou un radical phényle, plus préférablement, H, -CH₃, -C₂H₅ ou un radical phényle.

29. Utilisation selon l'une quelconque des revendications 21 à 28, **caractérisée en ce que** R^{13a} et R^{14a} sont indépendamment choisis chacun parmi le groupe consistant en un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, sont, de préférence, indépendamment choisis chacun parmi le groupe consistant en H, un radical alkyle en C₁₋₄ linéaire ou ramifié, un radical cyclohexyle et un radical phényle, et sont, plus préférablement, indépendamment choisis chacun parmi le groupe consistant en H, -CH₃, -C₂H₅ et un radical phényle.

30. Utilisation selon l'une quelconque des revendications 21 à 29, **caractérisée en ce que** R¹³, et R¹⁴, forment ensemble avec l'atome d'azote pontant, un noyau hétérocyclique à 5 ou 6 chaînons, saturé, insaturé ou aromatique, qui peut être au moins monosubstitué et/ou contenir au moins un autre hétéroatome en tant que chaînon de cycle, et forment, de préférence, un groupe pipéridine ou morpholine non substitué.

31. Utilisation selon une quelconque des revendications 21 à 30, **caractérisée en ce que** R^{15a} représente un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, représente, de préférence, H, un radical alkyle en C₁₋₄ linéaire ou ramifié, un radical cyclohexyle ou un radical phényle, plus préférablement, H, -CH₃, -C₂H₅ ou un radical phényle.

32. Utilisation selon une quelconque des revendications 21 à 31, **caractérisée en ce que** R^{16a} représente un radical aliphatique en C₁₋₆, non ramifié ou ramifié, saturé ou insaturé, facultativement au moins monosubstitué, de préférence, un radical alkyle saturé, non substitué en C₁₋₃, non ramifié ou ramifié, plus préférablement, un radical méthyle.

33. Utilisation selon l'une quelconque des revendications 21 à 32, **caractérisée en ce que** R^{17a} représente un radical aliphatique en C₁₋₆, non ramifié ou ramifié, saturé ou insaturé, facultativement au moins monosubstitué, de préférence, un radical alkyle saturé, non substitué en C₁₋₃, non ramifié ou ramifié, plus préférablement, un radical méthyle.

34. Utilisation selon l'une quelconque des revendications 21 à 33, **caractérisée en ce qu'**un ou plusieurs composés de type sulfonamide dérivés de benzoxazinone de formule générale (Ia) sont choisis parmi le groupe consistant en :
la 1-[1-(naphtyl-1-sulfonyl)-pipéridine-4-yl]-1,4-dihydro-benzo[d][1,3] oxazin-2-one,
la 1-(1-phénylsulfonyl-pipéridine-4-yl]-1,4-dihydro-benzo[d][1,3]-oxazin-2-one,
la 1-[1-(5-chloro-3-méthyl-benzo[b] thiophényl-2-sulfonyl)-pipéridine-4-yl]-1,4-dihydro-benzo[d][1,3]oxazin-2-one,
la 8 méthyl-1-[1-naphtyl-1-sulfonyl)-pipéridine-4-yl]-1,4-dihydro-benzo[d][1,3] oxazin-2-one,
la 1-[1-(quinoléine-8-sulfonyl)-pipéridin-4-yl]-1,4-dihydro-benzo[d][1,3] oxazin-2-one,
la 8-méthyl-1-[1-(quinoléine-8-sulfonyl)-pipéridin-4-yl]-1,4-dihydro-benzo[d][1,3] oxazin-2-one,
la 1-[1-(5-diméthylamino-naphtyl-1-sulfonyl)-pipéridine-4-yl]-8-méthyl-1,4-dihydro-benzo[d][1,3] oxazin-2-one,
la 1-[1-(5-diméthylamino-naphtyl-1-sulfonyl)-pipéridine-4-yl]-1,4-dihydro-benzo[d][1,3] oxazin-2-one,
la 1-[1-(2,3-dichloro-phénylsulfonyl)-pipéridine-4-yl]-1,4-dihydro-benzo[d][1,3] oxazin-2-one,
la 1-[1-(2,3-dichloro-phénylsulfonyl)-pipéridin-4-yl]-8-méthyl-1,4-dihydro-benzo[d][1,3] oxazin-2-one, et
les sels correspondants de ceux-ci, ou les solvats correspondants de ceux-ci.

35. Utilisation selon l'une quelconque des revendications 21 à 34, pour la régulation de l'appétit.

36. Utilisation selon l'une quelconque des revendications 21 à 34, pour la réduction, l'augmentation ou le maintien du poids corporel.

37. Utilisation selon une quelconque des revendications 21 à 34, pour la prophylaxie et/ou le traitement de l'obésité.

38. Utilisation selon une quelconque des revendications 21 à 34, pour la prophylaxie et/ou le traitement de la boulimie.

39. Utilisation selon l'une quelconque des revendications 21 à 34, pour la prophylaxie et/ou le traitement de l'anorexie.

40. Utilisation selon une quelconque des revendications 21 à 34, pour la prophylaxie et/ou le traitement de la cachéxie.

41. Utilisation selon une quelconque des revendications 21 à 34, pour la prophylaxie et/ou le traitement du diabète de type II.

42. Composés de type sulfonamide dérivés de benzoxazinone de formule générale (Ib) : 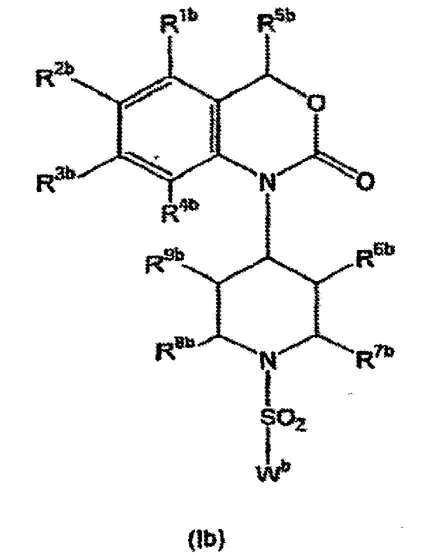 dans laquelle :
R^{1b} R^{2b}, R^{3b} et R^{4b} sont indépendamment choisis chacun parmi le groupe consistant en un atome d'hydrogène, d'halogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué et/ou peut être condensé avec un système de noyau mono ou polycyclique facultativement au moins monosubstitué, un radical aryle ou hétéroaryle facultativement au moins monosubstitué, qui peut être lié par l'intermédiaire d'un groupe alkylène facultativement au moins monosubstitué et/ou peut être condensé avec un système de noyau mono ou polycyclique facultativement au moins monosubstitué, un groupe nitro, un groupe cyano, et les parties -OR^{10b}, -OC(=O)-R^{11b}, -(C=O)-OR^{11b}, -SR^{12b}, -SOR^{12b}, -SO₂R^{12b}, -NH-SO₂R^{12b}, -SO₂NH₂ et -NR^{13b}R^{14b},
R^{5b} représente un atome d'hydrogène, un radical aliphatique non ramifié ou ramifié, saturé ou insaturé, facultativement au moins monosubstitué ou un radical cycloaliphatique saturé ou insaturé, facultativement au moins monosubstitué, contenant facultativement au moins un hétéroatome en tant que chaînon de cycle,
R^{6b}, R^{7b} R^{8b} et R^{9b} sont indépendamment choisis chacun parmi le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, un groupe cyano et une partie -COOR^{15b},
W^{b} représente un radical aliphatique non ramifié ou ramifié, saturé ou insaturé, qui peut être substitué par un ou plusieurs substituants choisis parmi le groupe consistant en un groupe hydroxy, un atome d'halogène, les radicaux alkoxy en C₁₋₄ ramifiés ou non ramifiés, perfluoroalkoxy en C₁₋₄ ramifiés ou non ramifiés, perfluoroalkyle en C₁₋₄ ramifiés ou non ramifiés, amino, carboxy, amido, cyano, nitro, -SO₂NH₂, -CO-(alkyle en C₁₋₄), -SO-(alkyle en C₁₋₄), -SO₂-(alkyle en C₁₋₄), -NH-SO₂-(alkyle en C₁₋₄), dans lesquels la partie alkyle en C₁₋₄ peut être, dans chaque cas, ramifiée ou non ramifiée, un radical phényle ou naphtyle non substitué ou au moins monosubstitué, et les radicaux furannyle, thiényle, pyrrolyle, imidazolyle, pyrazolyle, pyridinyle, pyrimidinyle, quinolinyle et isoquinolinyle non substitués ou au moins monosubstitués,
de sorte que lesdits substituants peuvent être au moins monosubstitués avec un substituant F, Cl, méthyle et méthoxy,
un radical cycloaliphatique saturé ou insaturé, contenant facultativement au moins un hétéroatome en tant que chaînon de cycle,
de sorte que ledit radical cycloaliphatique peut être substitué par un ou plusieurs substituants choisis parmi le groupe consistant en les radicaux hydroxy, nitro, carboxy, cyano, céto, halogéno, alkyle en C₁₋₂₀, alkyle en C₁₋₄ partiellement fluorés, alkyle en C₁₋₄ partiellement chlorés, alkyle en C₁₋₄ partiellement bromés, alkoxy en C₁₋₅ , alkoxy en C₁₋₄ partiellement fluorés, alkoxy en C₁₋₄ partiellement chlorés, alkoxy en C₁₋₄ partiellement bromés, alcényle en C₂₋₆, -SO₂-(alkyle en C₁₋₄), -(C=O)-(alkyle en C₁₋₅), -(C=O)-O-(alkyle en C₁₋₅), -(C=O)-Cl, -S-(alkyle en C₁₋₄), -(C=O)-H, -NH-(C=O)-NH-(alkyle en C₁₋₅), -(C=O)-(perfluoroalkyle en C₁₋₄), -NR^{A}R^{B}, dans lesquels R^{A} et R^{B} sont indépendamment choisis parmi le groupe consistant en H, les radicaux alkyle en C₁₋₄ et phényle, NH-(C=O)-(alkyle en C₁₋₅), -(alkylène en C₁₋₅)-(C=O)-(alkyle en C₁₋₅), 1,3-dihydro-1-oxo-2H-isoindol-2-yle, N-phtalimidinyle, 1,3-dioxo-2-azaspiro[4,4]-non-2-yle, phényle substitués ou non substitués, -SO₂-phényle, phénoxy, pyridinyle, pyridinyloxy, pyrazolyle, pyrimidinyle, pyrrolidinyle, -SO₂-pyrrolidinyle, morpholinyle, -SO₂-morpholinyle, thiadiazolyle, oxadiazolyle, oxazolyle, thiazolyle, isoxazolyle, -0-CH₂-thiazolyle, -NH-phényle, et -(alkylène en C₁₋₄)-NH-(C=O)-phényle, dans lesquels lesdits substituants peuvent être substitués par un ou plusieurs substituants choisis parmi le groupe consistant en un atome d'halogène, les radicaux nitro, cyano, hydroxy, -(C=O)-(alkyle en C₁₋₄), alkyle en C₁₋₄, alkyle en C₁₋₄ au moins partiellement fluorés, alkyle en C₁₋₄ au moins partiellement chlorés, alkyle en C₁₋₄ au moins partiellement bromés, -S-(alkyle en C₁₋₄), -(C=O)-O-(alkyle en C₁₋₅), -(C=O)-CH₂-F, -(C=O)-CH₂-Cl et -(C=O)-CH₂-Br, et de sorte que ledit radical cycloaliphatique peut être lié par l'intermédiaire d'un groupe alkylène facultativement monosubstitué, et/ou peut être condensé avec un système de noyau aromatique mono ou polycyclique facultativement au moins monosubstitué,
un radical aryle ou hétéroaryle, facultativement au moins monosubstitué, qui peut être lié par l'intermédiaire d'un groupe alkylène facultativement monosubstitué, et/ou peut être condensé avec un système de noyau mono ou polycyclique facultativement au moins monosubstitué, une partie -NR^{16b}R^{17b} ou une partie -COR^{18b},
R^{10b} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{11b} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{12b} représente un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{13b} et R^{14b} sont indépendamment choisis chacun parmi le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, ou
R^{13b} et R^{14b} forment ensemble avec l'atome d'azote pontant, un noyau hétérocyclique saturé, insaturé ou aromatique, qui peut être au moins monosubstitué et/ou contenir au moins un autre hétéroatome en tant que chaînon de cycle,
R^{15b} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{16b} représente un radical aliphatique non ramifié ou ramifié, saturé ou insaturé, facultativement au moins monosubstitué,
R^{17b} représente un radical aliphatique non ramifié ou ramifié, saturé ou insaturé, facultativement au moins monosubstitué, et
R^{18b} représente un radical aryle facultativement au moins monosubstitué, facultativement sous la forme de l'un de leurs stéréoisomères, de préférence, les énantiomères ou les diastéréoisomères, leur racémate ou sous la forme d'un mélange d'au moins deux de leurs stéréoisomères, de préférence, les énantiomères ou les diastéréoisomères, en un quelconque rapport de mélange, ou, respectivement, un sel physiologiquement acceptable de ceux-ci, ou un solvat.

43. Composés selon la revendication 42, **caractérisés en ce que** R^{1b}, R^{2b}, R^{3b} et R^{4b} sont indépendamment choisis chacun parmi le groupe consistant en H, F, Cl et Br, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆ facultativement au moins monosubstitué et/ou peut être condensé avec un système de noyau mono ou polycyclique facultativement au moins monosubstitué, un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, un groupe nitro, un groupe cyano, et les parties -OR^{10b}, -OC(=O)-R^{11b}, -SR^{12b}, -SOR^{12b}, -SO₂R^{12b}, -NH-SO₂R^{12b}, -SO₂NH₂ et -NR^{13b}R^{14b}, de préférence, choisis parmi le groupe consistant en H, F, Cl et Br, un radical aliphatique en C₁₋₃ non ramifié ou ramifié, saturé ou insaturé, facultativement au moins monosubstitué, un radical cycloaliphatique en C₅ ou C₆ saturé ou insaturé, facultativement au moins monosubstitué, contenant facultativement au moins un hétéroatome en tant que chaînon de cycle, qui peut être lié par l'intermédiaire d'un groupe alkylène en C₁ ou C₂ facultativement au moins monosubstitué, un groupe nitro, un groupe cyano, et les parties -OR^{10b}, -OC(=O)R^{11b}, -SR^{12b} et -NR^{13b}R^{14b}, plus préférablement, choisis parmi le groupe consistant en H, F, Cl, Br, -CH₃, -CH₂CH₃, -CF₃, -CF₂CF₃, les groupes cyclopentyle, cyclohexyle, nitro, cyano et -OR^{10b}.

44. Composés selon la revendication 42 ou 43, **caractérisés en ce que** R^{5b} représente un atome d'hydrogène, un radical aliphatique en C₁₋₆ non ramifié ou ramifié, saturé ou insaturé, facultativement au moins monosubstitué ou un radical cycloaliphatique en C₃₋₈ saturé ou insaturé, facultativement au moins monosubstitué, contenant facultativement au moins un hétéroatome en tant que chaînon de cycle, représente, de préférence, H ou un radical alkyle en C₁₋₃ ramifié ou non ramifié, et représente, plus préférablement, H, -CH₃ ou -CH₂CH₃, et plus préférablement encore un atome d'hydrogène.

45. Composés selon l'une quelconque des revendications 42 à 44, **caractérisés en ce que** R^{6b}, R^{7b}, R^{8b} et R^{9b} sont indépendamment choisis chacun parmi le groupe consistant en un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, un groupe cyano et une partie -COOR^{15b}, de préférence, choisis parmi le groupe consistant en H, un radical alkyle en C₁₋₃ ramifié ou non ramifié, un groupe cyano et un groupe -COOR^{15b}, plus préférablement, parmi le groupe consistant en H, -CH₃, -CH₂CH₃ et une partie cyano, le plus préférablement, chacun des groupes R^{6b}, R^{7b}, R^{8b} et R^{9b} représente un atome d'hydrogène.

46. Composés selon l'une quelconque des revendications de 42 à 45, **caractérisés en ce que** W^{b} représente un radical aliphatique en C₁₋₂₀ non ramifié ou ramifié, saturé ou insaturé, qui peut être substitué par un ou plusieurs substituants choisis parmi le groupe consistant en un groupe hydroxy, un atome d'halogène, les radicaux alkoxy en C₁₋₄ ramifiés ou non ramifiés, perfluoroalkoxy en C₁₋₄ ramifiés ou non ramifiés, perfluoroalkyle en C₁₋₄ ramifiés ou non ramifiés, amino, carboxy, amido, cyano, nitro, -SO₂NH₂, -CO-(alkyle en C₁₋₄), -SO-(alkyle en C₁₋₄), -SO₂-(alkyle en C₁₋₄), -NH-SO₂-(alkyle en C₁₋₄), dans lesquels la partie alkyle en C₁₋₄ peut être, dans chaque cas, ramifiée ou non ramifiée, un radical phényle ou naphtyle non substitué ou au moins monosubstitué, et les radicaux furannyle, thiényle, pyrrolyle, imidazolyle, pyrazolyle, pyridinyle, pyrimidinyle, quinolinyle et isoquinolinyle non substitué ou au moins monosubstitué, de sorte que lesdits substituants peuvent être au moins monosubstitués avec un substituant F, Cl, méthyle et méthoxy ;
un radical cycloaliphatique en C₃₋₈ saturé ou insaturé, contenant facultativement au moins un hétéroatome en tant que chaînon de cycle, de sorte que ledit radical cycloaliphatique en C₃₋₈ peut être substitué par un ou plusieurs substituants choisis parmi le groupe consistant en les radicaux hydroxy, nitro, carboxy, cyano, céto, halogéno, alkyle en C₁₋₂₀, alkyle en C₁₋₄ partiellement fluorés, alkyle en C₁₋₄ partiellement chlorés, alkyle en C₁₋₄ partiellement bromés, alkoxy en C₁₋₅, alkoxy en C₁-4 partiellement fluorés, alkoxy en C₁₋₄ partiellement chlorés, alkoxy en C₁₋₄ partiellement bromés, alcényle en C₂₋₆, -SO₂-(alkyle en C₁₋₄), -(C=O)-(alkyle en C₁₋₅), - (C=O)-O-(alkyle en C₁₋₅), -(C=O)-Cl, -S-(alkyle en C₁₋₄), -(C=O)-H, -NH-(C=O)-NH-(alkyle en C₁₋₅), -(C=O)-(perfluoroalkyle en C₁₋₄), -NR^{A}R^{B}, dans lesquels R^{A} et R^{B} sont indépendamment choisis parmi le groupe consistant en H, les radicaux alkyle en C₁₋₄ et phényle, NH-(C=O)-(alkyle en C₁₋₅), -(alkylène en C₁₋₅)-(C=O)-(alkyle en C₁₋₅), 1,3-dihydro-1-oxo-2H-isoindol-2-yle, N-phtalimidinyle, 1,3-dioxo-2-azaspiro[4,4]-non-2-yle, phényle substitués ou non substitués, -SO₂-phényle, phénoxy, pyridinyle, pyridinyloxy, pyrazolyle, pyrimidinyle, pyrrolidinyle, -SO₂-pyrrolidinyle, morpholinyle, -SO₂-morpholinyle, thiadiazolyle, oxadiazolyle, oxazolyle, thiazolyle, isoxazolyle, -0-CH₂-thiazolyle, -NH-phényle, et -(alkylène en C₁₋₄)-NH-(C=O)-phényle, dans lesquels lesdits substituants peuvent être substitués par un ou plusieurs substituants choisis parmi le groupe consistant en un atome d'halogène, les radicaux nitro, cyano, hydroxy, -(C=O)-(alkyle en C₁₋₄), alkyle en C₁₋₄, alkyle en C₁₋₄ au moins partiellement fluorés, alkyle en C₁₋₄ au moins partiellement chlorés, alkyle en C₁₋₄ au moins partiellement bromés, -S-(alkyle en C₁₋₄), -(C=O)-O-(alkyle en C₁₋₅), -(C=O)-CH₂-F, -(C=O)-CH₂-Cl et -(C=O)-CH₂-Br, et de sorte que ledit radical cycloaliphatique en C₃₋₈ peut être lié par l'intermédiaire d'un groupe alkylène en C₁₋₆ facultativement monosubstitué, et/ou peut être condensé avec un système de noyau aromatique mono ou polycyclique facultativement au moins monosubstitué,
un radical aryle ou hétéroaryle à 5 ou 6 chaînons, facultativement au moins monosubstitué, qui peut être lié par l'intermédiaire d'un groupe alkylène en C₁₋₆ facultativement monosubstitué, et/ou peut être condensé avec un système de noyau mono ou polycyclique facultativement au moins monosubstitué, une partie -NR^{16b}R¹⁷b ou une partie COR^{18b}
W^{b} représente, de préférence :
un radical alkyle en C₁₋₂₀ linéaire ou ramifié, de préférence, un radical alkyle choisi parmi le groupe consistant en les radicaux méthyle, éthyle, n-propyle, iso-propyle, n-butyle, iso-butyle, sec-butyle, tert-butyle et 1,1-diméthyl-propyle, un radical alcényle en C₂₋₂₀ linéaire ou ramifié ; de préférence, un radical vinyle ; -CF₃ ; -CHF₂ ; - CH₂F ; -CCl₃ ; -CHCl₂ ; -CH₂Cl; -CH₂-CF₃ ; -CH₂-CH₂-Cl ; -CH₂-CH₂-CH₂-Cl ; -CH₂-S(=O)₂-CH₃ ; un radical cyclopropyle ; un radical cyclobutyle ; un radical cyclopentyle ; un radical cyclohexyle ; un radical -CH₂-cyclopropyle ; un radical -CH₂-cyclobutyle ; un radical -CH₂-cyclopentyle ; un radical -CH₂-cyclohexyle ; -N(CH₃)₂ ; -N(C₂H₅)₂ ; -N(n-CH₂-CH₂-CH₃)₂ ; phényle ; benzyle ; naphtyle ; -CH=CH-phényle ; -(CF₂)-(CF₂)-O-phényle ; -(CH₂)-naphtyle ; -(CH₂)-(CH₂)-naphtyle ; anthracényle ;-(C=O)-phényle ; thiophényle ; benzo[b] thiophényle ; furannyle ; 2-oxo-2H-chroményle ; dibenzofurannyle ; 2,3-dihydrobenzofurannyle ; chromanyle ; 2,3-dihydro-benzo[1,4] dioxinyle ; 3,4-dihydro-2H-1,5-benzo-dioxépinyle , chromonyle ; 1H-imidazolyle , pyridinyle ; pyrrolidine-2,5-dionyle ; pyrrolyle ; 1H-pyrazolyle ; 1H-pyrimidine-2,4-dionyle ; quinolinyle ; isoquinolinyle, 1H-benzoimidazolyle ; 1,4-dihydro-quinoxaline-2,3-dionyle ; 1,2,3,4-tétrahydro-isoquinolinyle ; 1,4-dihydro-benzo[b][1,4] diazépine-2,4-dionyle ; 1,3-dihydro-1-oxo-2H-isoindolyle ; phtalimidinyle ; 2-(1,3-dioxo-1,3-dihydro-isoindol-2-yl)-éthyle ; imidazo[1,2-a] pyridine ; isatinyle ; thiazolyle ; 1,3-thiazolyle ; 1,2,4-thiadiazolyle ; imidazo[2,1-b] thiazolyle ; 1,3-benzo thiazolyle ; benzo[1,2,5] thiadiazolyle ; 2-oxo-2,3-dihydro-benzothiazolyle ; 2,1,3-benzothiadiazolyle ; imidazo[2,1-b] thiazolyle ; isoxazolyle ; benzo[1,2,5] oxadiazolyle ; benzo[d] isoxazolyle ; benzofurazannyle ; 2-oxo-2,3-dihydro-benzoxazolyle ; 3,4-dihydro-2H-benzo[1,4] oxazinyle ; ou 2,1,3-benzoxadiazolyle ;
de sorte que chacune des parties cycliques mentionnées ci-dessus peut, facultativement, être substituée avec 1, 2, 3, 4 ou 5 substituants indépendamment choisis parmi le groupe consistant en les radicaux méthyle ; éthyle ; n-propyle ; iso-propyle ; n-butyle ; iso-butyle ; sec-butyle ; tert-butyle ; 1,1-diméthyl-propyle ; n-pentyle ; vinyle ; cyclopropyle ; cyclobutyle ; cyclopentyle ; cyclohexyle ; morpholino ; méthoxy ; éthoxy ; n-propoxy ; iso-propoxy; F ; Cl ; Br ; I ; -CN ; -OH ; -CF₃ ; -CF₂H ; -CCl₃ -CClH₂ ; -CHCl₂ ; -CH₂F ; -CH₂-Cl ; -CH₂-Br ; -(C=O)-CH₂-Br ; -OCF₃-; -O-CH₂-CF₃-; -O-CHF₂ ; -NO₂-; -NH₂ ; -N(CH₃)₂ ; -N(C₂H₅)₂ ; -N(n-CH₂-CH₂-CH₃)₂ ; -N(n-CH₂-CH₂-CH₂-CH₃)₂ ; -NH-(C=O)-CH₃ ; -NH-phényle ; -(C=O)-CF₃ ; -(C=O)-OH; =O (oxo) ; -(C=O)-H ; -S(=O)₂-CH₃ ; -S(=O)₂-isopropyle ; -S(=O)₂-phényle ; -S(=O)₂-pyrrolidinyle ; -S(=O)₂-morpholino ; -(CH)₂-(CH)₂-(C=O)-O-CH₃ ; NH-(C=O)-NH-CH₂-CH₂-CH₃ ; -(C=O)-CH₃ ; -(C=O)-O-CH₃ ; -(C=O)-O-C₂H₅ -(CH)₂-NH-(C=O)-phényle ; -CH₂-C(H)(phényle)(phényle) ; -O-CH₂-thiazolyle ; 1,3-dioxo-2-azaspiro[4,4] non-2-yle ; phényle ; phénoxy ; isoxazolyle ; 1,3-oxazolyle ; 1,2,4-oxadiazolyle ; 1,3,4-oxadiazolyle ; pyridinyle ; pyridinyloxy ; pyrazolyle ; pyrimidinyle et phtalimidinyle ; et
de sorte que chacune des parties cycliques des substituants mentionnés ci-dessus peut, facultativement, être substituée avec 1, 2, 3, 4 ou 5 substituants indépendamment choisis parmi le groupe consistant en les radicaux méthyle ; éthyle ; n-propyle ; iso-propyle ; F ; Cl ; Br ; I ; CN ; -CH₂F ; -CH₂-Cl ; -CH₂-Br ; CF₃ et -S-CH₃, et
W^{b} représente, plus préférablement :
un radical alkyle choisi parmi le groupe consistant en les radicaux méthyle ; éthyle ; n-propyle ; iso-propyle ; n-butyle; sec-butyle; iso-butyle et tert-butyle ; vinyle (CH₂=CH-) ; -N(CH₃)₂ ; 1-naphtyle ; benzyle ; 2-naphtyle ; phényle ; 2-méthyl-phényle ; 3-méthyl-phényle ; 4-méthyl-phényle ; 2-éthyl-phényle ; 3-éthyl-phényle ; 4-éthyl-phényle ; 2-n-propyl-phényle ; 3-n-propyl-phényle ; 4-n-propyl-phényle ; 2-isopropyl-phényle ; 3-isopropyl-phényle ; 4-isopropyl-phényle ; 2-n-butyl-phényle ; 3-n-butyl-phényle ; 4-n-butyl-phényle ; 2-iso-butyl-phényle ; 3-iso-butyl-phényle ; 4-iso-butyl-phényle ; 2-tert-butyl-phényle ; 3-tert-butyl-phényle ; 4-tert-butyl-phényle ; 1,1-diméthylpropyl-phényle ; 2-cyclopentyl-phényle ; 3-cyclopentyl-phényle ; 4-cyclopentyl-phényle ; 2-cyclohexyl-phényle ; 3-cyclohexyl-phényle ; 4-cyclohexyl-phényle ; 2-méthoxy-phényle ; 3-méthoxy-phényle ; 4-méthoxy-phényle ; 2-éthoxy-phényle ; 3-éthoxy-phényle ; 4-éthoxy-phényle ; 2-n-propoxy-phényle ; 3-n-propoxy-phényle ; 4-n-propoxy-phényle ; 2-iso-propoxy-phényle ; 3-iso-propoxy-phényle ; 4-isopropoxy-phényle ; 2-fluoro-phényle ; 3-fluoro-phényle ; 4-fluoro-phényle ; 2-chloro-phényle ; 3-chloro-phényle ; 4-chloro-phényle ; 2-bromo-phényle ; 3-bromo-phényle ; 4-bromo-phényle ; 2-trifluorométhyl-phényle ; 3-trifluorométhyl-phényle ; 4-trifluorométhyl-phényle ; 2-trifluorométhoxy-phényle ; 3-trifluorométhoxy-phényle ; 4-trifluorométhoxy-phényle ; 2-carboxy-phényle ; 3-carboxy-phényle ; 4-carboxy-phényle ; 2-acétyl-phényle ; 3-acétyl-phényle ; 4-acétyl-phényle ; 2-(C=O)-O-CH₃-phényle ; 3-(C=O)-O-CH₃-phényle ; 4-(C=O)-O-CH₃-phényle ; 2-(CH₂)-(CH₂)-(C=O)-O-CH₃ ; 3-(CH₂)-(CH₂)-(C=O)-O-CH₃ ; 4-(CH₂)-(CH₂)-(C=O)-O-CH₃ ; 2-cyano-phényle ; 3-cyano-phényle ; 4-cyano-phényle ; 2-nitro-phényle, 3-nitro-phényle ; 4-nitro-phényle ; 4-(4-bromophénoxy)-phényle ; 2-méthylsulfonyl-phényle ; 3-méthylsulfonyl-phényle ; 4-méthylsulfonyl-phényle ; 2-phényl-phényle (biphényl-2-yle) ; 3-phényl-phényle (biphényl-3-yle) ; 4-phényl-phényle (biphényl-4-yle) ; 2-phénoxy-phényle ; 3-phénoxy-phényle ; 4-phénoxy-phényle ; 2,4-diméthyl-phényle ; 3,4-diméthyl-phényle ; 2,4,6-triméthylphényle ; 2,3,5,6-tétraméthyl-phényle ; pentaméthyl-phényle ; 2,5-diméthoxy-phényle ; 3,4-diméthoxy-phényle ; 2,3-dichloro-phényle ; 2,4-dichloro-phényle ; 2,5-dichloro-phényle ; 3,4-dichloro-phényle ; 3,5-dichloro-phényle ; 2,6-dichloro-phényle ; 2,4-difluoro-phényle ; 3,4-difluoro-phényle ; 2,5-difluoro-phényle ; 2,6-difluoro-phényle ; 3-chloro-2-fluoro-phényle ; 3-chloro-4-fluoro-phényle ; 5-chloro-2-fluoro-phényle ; 2,3,4-trichloro-phényle ; 2,4,5-trichloro-phényle ; 2,4,6-trichloro-phényle ; 2,4,5-trifluoro-phényle ; 2,3,4-trifluoro-phényle ; 2-chloro-4,5-difluoro-phényle ; 2-bromo-4-fluoro-phényle ; 2-bromo-4,6-difluoro-phényle ; 4-chloro-2,5-difluoro-phényle ; 5-chloro-2,4-difluoro-phényle ; 4-bromo-2,5-difluoro-phényle ; 5-bromo-2,4-difluoro-phényle ; pentafluoro-phényle ; 2,4-dinitro-phényle ; 4-chloro-3-nitro-phényle ; 2-méthyl-5-nitro-phényle ; 5-bromo-2-méthoxy-phényle ; 3-chloro-2-méthyl-phényle ; 4-bromo-3-méthyl-phényle ; 4-chloro-2,5-diméthyl-phényle ; 4-fluoro-3-méthyl-phényle ; 5-fluoro-2-méthyl-phényle ; 2-nitro-4-trifluorométhyl-phényle ; 2-méthoxy-4-méthyl-phényle ; 3,5-dichloro-2-hydroxy-phényle ; 3,5-dichloro-4-hydroxy-phényle ; 5-chloro-2,4-difluoro-phényle ; 3-chloro-4-(NH)-(C=O)-CH₃-phényle ; 2-chloro-6-méthyl-phényle ; 2-chloro-5-trifluorométhyl-phényle ; 2-chloro-5-trifluorométhoxy-phényle ; 4-bromo-2-trifluorométhoxy-phényle ; 4-bromo-2-trifluorométhyl-phényle ; 4-bromo-3-trifluorométhyl-phényle ; 3-carboxy-4-fluoro-phényle ; 3-carboxy-4-chloro-6-fluoro-phényle ; 4-méthoxy-2,3,6-triméthyl-phényle ; ou l'un des groupes suivants : 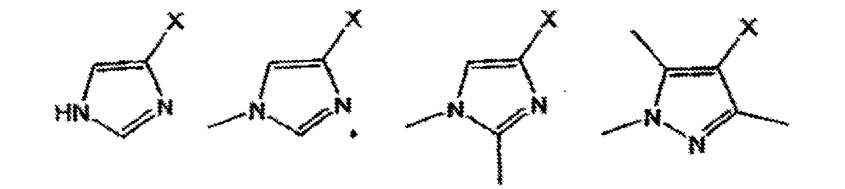 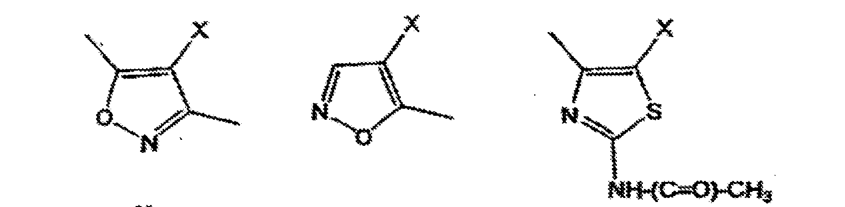 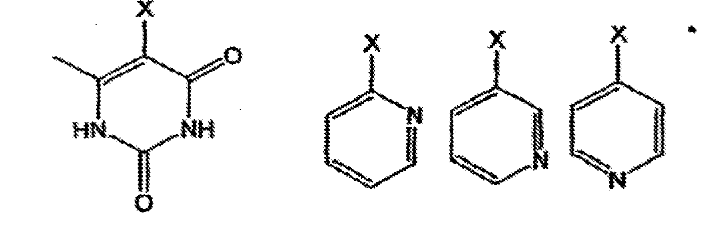 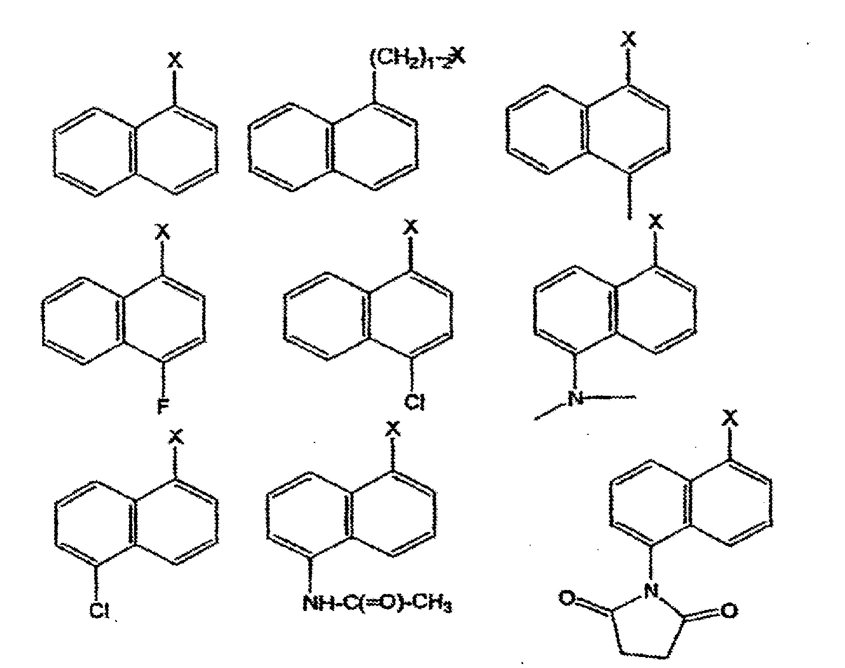 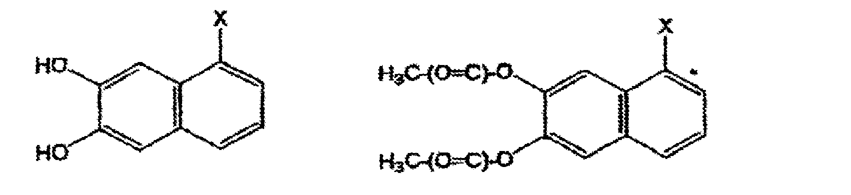 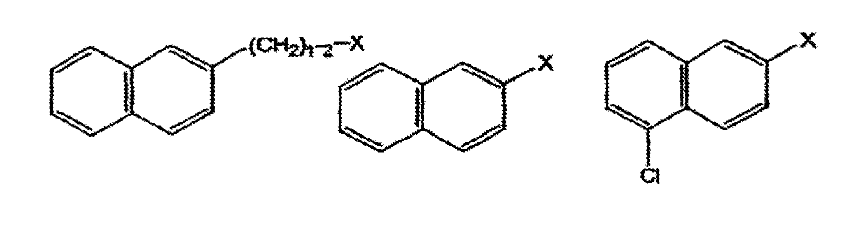 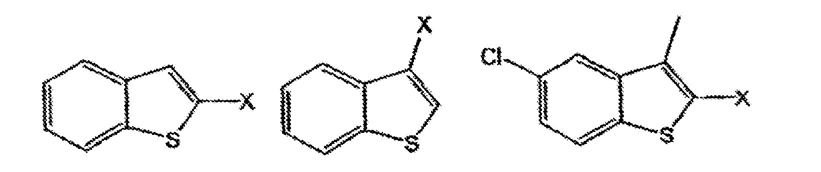 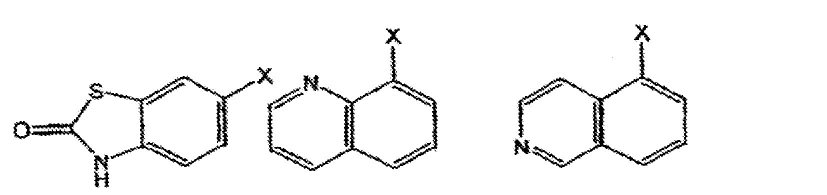 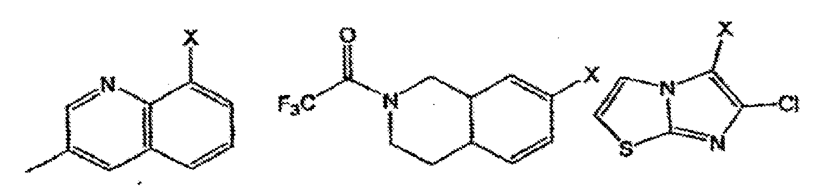 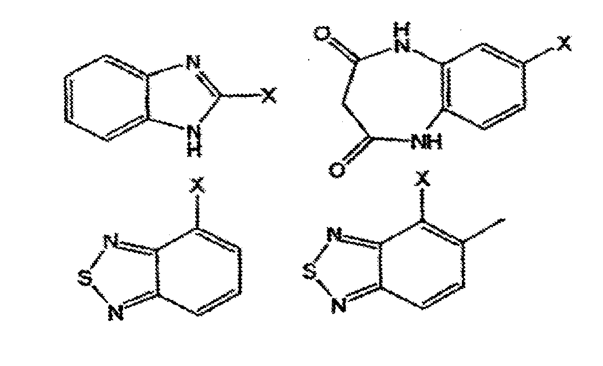 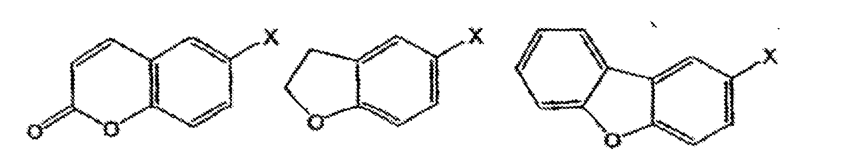 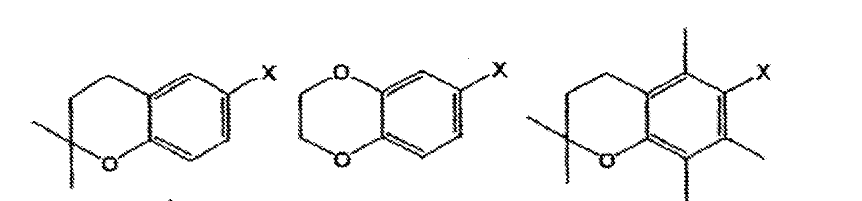 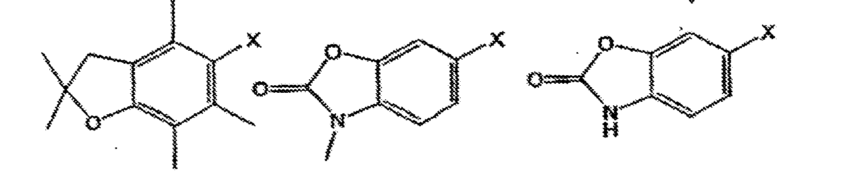 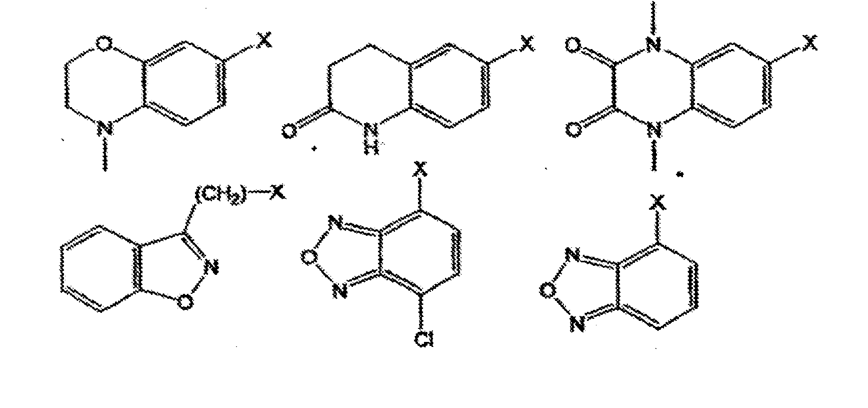 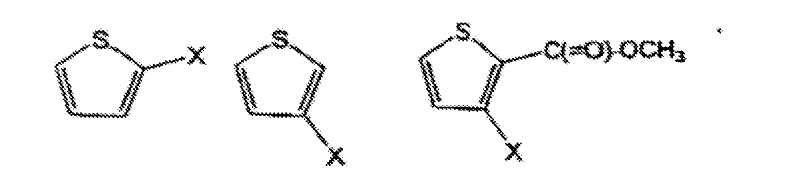 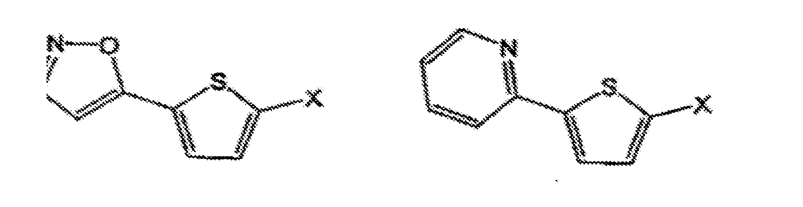 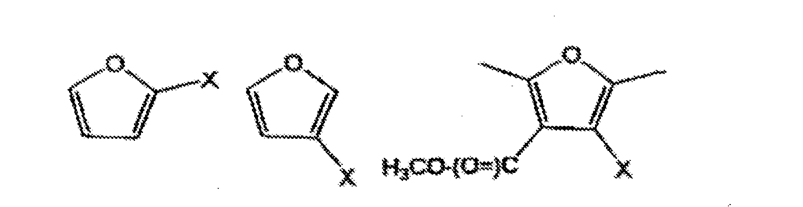 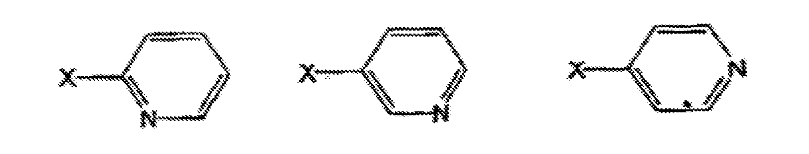 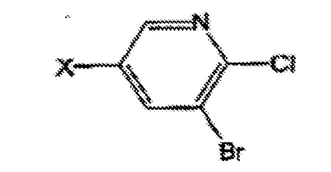
de sorte que, dans chaque cas, X indique la position à laquelle le substituant respectif W^{b} est lié au groupe -SO₂ de formule (Ib).

47. Composés selon l'une quelconque des revendications 42 à 46, **caractérisés en ce que** R^{10b} représente un atome d'hydrogène, un radical aliphatique en C₁₋₆, non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆ facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, de préférence, H, un radical alkyle en C₁₋₄ linéaire ou ramifié, un radical cyclohexyle ou un radical phényle, plus préférablement, H, -CH₃, -C₂H₅ ou un radical phényle.

48. Composés selon l'une quelconque des revendications 42 à 47, **caractérisés en ce que** R^{11b} représente un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, de préférence, H, un radical alkyle en C₁₋₄ linéaire ou ramifié, un radical cyclohexyle ou un radical phényle, plus préférablement, H, -CH₃, -C₂H₅ ou un radical phényle.

49. Composés selon l'une quelconque des revendications 42 à 48, **caractérisés en ce que** R^{12b} représente un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, représente, de préférence, H, un radical alkyle en C₁₋₄ linéaire ou ramifié, un radical cyclohexyle ou un radical phényle, plus préférablement, H, -CH₃,-C₂H₅ ou un radical phényle.

50. Composés selon l'une quelconque des revendications 42 à 49, **caractérisés en ce que** R^{13b} et R^{14b} sont indépendamment choisis chacun parmi le groupe consistant en un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, sont, de préférence, indépendamment choisis chacun parmi le groupe consistant en H, un radical alkyle en C₁₋₄ linéaire ou ramifié, un radical cyclohexyle et un radical phényle, et sont, plus préférablement, indépendamment choisis chacun parmi le groupe consistant en H, -CH₃, -C₂H₅ et un radical phényle.

51. Composés selon l'une quelconque des revendications 42 à 50, **caractérisés en ce que** R^{13b} et R^{14b} forment ensemble avec l'atome d'azote pontant, un noyau hétérocyclique à 5 ou 6 chaînons, saturé, insaturé ou aromatique, qui peut être au moins monosubstitué et/ou contenir au moins un autre hétéroatome en tant que chaînon de cycle, et forment, de préférence, un groupe pipéridine ou morpholine non substitué.

52. Composés selon l'une quelconque des revendications 42 à 51, **caractérisés en ce que** R^{15b} représente un atome d'hydrogène, un radical aliphatique en C₁₋₆ non ramifié ou ramifié, saturé ou insaturé, facultativement au moins monosubstitué, un radical cycloaliphatique en C₃₋₈ saturé ou insaturé, facultativement au moins monosubstitué, contenant facultativement au moins un hétéroatome en tant que chaînon de cycle, ou un radical aryle ou hétéroaryle à 5 ou 6 chaînons, facultativement au moins monosubstitué, qui peut être lié par l'intermédiaire d'un groupe alkylène en C₁₋₆, facultativement au moins monosubstitué, et/ou peut être condensé avec un système de noyau mono ou polycyclique facultativement au moins monosubstitué, représente, de préférence, H, un radical alkyle en C₁₋₄ linéaire ou ramifié, un radical cyclohexyle ou un radical phényle, et représente, plus préférablement, H, -CH₃, -C₂H₅ ou un radical phényle.

53. Composés selon l'une quelconque des revendications 42 à 52, **caractérisés en ce que** R^{16b} représente un radical aliphatique en C₁₋₆, non ramifié ou ramifié, saturé ou insaturé, facultativement au moins monosubstitué, de préférence, un radical alkyle saturé, non substitué en C₁₋₃, non ramifié ou ramifié, plus préférablement, un radical méthyle.

54. Composés selon l'une quelconque des revendications 42 à 53, **caractérisés en ce que** R^{17b} représente un radical aliphatique en C₁₋₆, non ramifié ou ramifié, saturé ou insaturé, facultativement au moins monosubstitué, de préférence, un radical alkyle saturé, non substitué en C₁₋₃, non ramifié ou ramifié, plus préférablement, un radical méthyle.

55. Composés selon l'une quelconque des revendications 42 à 54, **caractérisés en ce que** R^{18b} représente un radical phényle qui est facultativement au moins monosubstitué par un radical aliphatique en C₁₋₆, plus préférablement, un radical phényle qui est facultativement au moins monosubstitué par un groupe méthyle.

56. Composés selon l'une quelconque des revendications de 42 à 55, **caractérisés en ce que** :
R^{1b}, R^{2b} R^{3b} et R^{4b} sont indépendamment choisis chacun parmi le groupe consistant en un atome d'hydrogène ; un atome de fluor, un atome de chlore, un atome de brome, un groupe méthyle et un groupe méthoxy ;
R^{5b} représente un atome d'hydrogène,
R^{6b}, R^{7b}, R^{8b} et R^{9b} représentent chacun un atome d'hydrogène ;
W^{b} représente :
un radical alkyle choisi parmi le groupe consistant en les radicaux méthyle ; éthyle ; n-propyle ; iso-propyle ; n-butyle; sec-butyle; iso-butyle et tert-butyle ; vinyle (CH₂=CH-) ; -N(CH₃)₂ ; 1-naphtyle ; benzyle ; 2-naphtyle ; phényle ; 2-méthyl-phényle ; 3-méthyl-phényle ; 4-méthyl-phényle ; 2-éthyl-phényle ; 3-éthyl-phényle ; 4-éthyl-phényle ; 2-n-propyl-phényle ; 3-n-propyl-phényle ; 4-n-propyl-phényle ; 2-isopropyl-phényle ; 3-isopropyl-phényle ; 4-isopropyl-phényle ; 2-n-butyl-phényle ; 3-n-butyl-phényle ; 4-n-butyl-phényle ; 2-iso-butyl-phényle 3-isobutyl-phényle ; 4-isobutyl-phényle ; 2-tert-butyl-phényle ; 3-tert-butyl-phényle ; 4-tert-butyl-phényle ; 1,1-diméthylpropyl-phényle ; 2-cyclopentyl-phényle ; 3-cyclopentyl-phényle ; 4-cyclopentyl-phényle ; 2-cyclohexyl-phényle ; 3-cyclohexyl-phényle ; 4-cyclohexyl-phényle ; 2-méthoxy-phényle ; 3-méthoxy-phényle ; 4-méthoxy-phényle ; 2-éthoxy-phényle ; 3-éthoxy-phényle ; 4-éthoxy-phényle ; 2-n-propoxy-phényle ; 3-n-propoxy-phényle ; 4-n-propoxy-phényle ; 2-iso-propoxy-phényle ; 3-iso-propoxy-phényle ; 4-isopropoxy-phényle ; 2-fluoro-phényle ; 3-fluoro-phényle ; 4-fluoro-phényle ; 2-chlorophényle ; 3-chloro-phényle ; 4-chloro-phényle ; 2-bromo-phényle ; 3-bromo-phényle ; 4-bromo-phényle ; 2-trifluorométhyl-phényle ; 3-trifluorométhyl-phényle ; 4-trifluorométhyl-phényle ; 2-trifluorométhoxy-phényle ; 3-trifluorométhoxy-phényle ; 4-trifluorométhoxy-phényle ; 2-carboxy-phényle ; 3-carboxy-phényle ; 4-carboxy-phényle ; 2-acétyl-phényle ; 3-acétyl-phényle ; 4-acétyl-phényle ; 2-(C=O)-O-CH₃-phényle ; 3-(C=O)-O-CH₃-phényle ; 4-(C=O)-O-CH₃-phényle ; 2-(CH₂)-(CH₂)-(C=O)-O-CH₃ ; 3-(CH₂)-(CH₂)-(C=O)-O-CH₃ ; ; 4-(CH₂)-(CH₂)-(C=O)-O-CH₃ ; 2-cyano-phényle ; 3-cyano-phényle ; 4-cyano-phényle ; 2-nitro-phényle, 3-nitro-phényle ; 4-nitro-phényle ; 4-(4-bromophénoxy)-phényle ; 2-méthylsulfonyl-phényle ; 3-méthylsulfonyl-phényle ; 4-méthylsulfonyl-phényle ; 2-phényl-phényle (biphényl-2-yle) ; 3-phényl-phényle (biphényl-3-yle) ; 4-phényl-phényle (biphényl-4-yle) ; 2-phénoxy-phényle ; 3-phénoxy-phényle ; 4-phénoxy-phényle ; 2,4-diméthyl-phényle ; 3,4-diméthyl-phényle ; 2,4,6-triméthyl-phényle ; 2,3,5,6-tétraméthyl-phényle ; pentaméthyl-phényle ; 2,5-diméthoxy-phényle ; 3,4-diméthoxy-phényle ; 2,3-dichloro-phényle ; 2,4-dichloro-phényle ; 2,5-dichloro-phényle ; 3,4-dichloro-phényle ; 3,5-dichloro-phényle ; 2,6-dichloro-phényle ; 2,4-difluoro-phényle ; 3,4-difluoro-phényle ; 2,5-difluoro-phényle ; 2,6-difluoro-phényle ; 3-chloro-2-fluoro-phényle ; 3-chloro-4-fluoro-phényle ; 5-chloro-2-fluoro-phényle ; 2,3,4-trichloro-phényle ; 2,4,5-trichloro-phényle ; 2,4,6-trichloro-phényle ; 2,4,5-trifluoro-phényle ; 2,3,4-trifluoro-phényle ; 2-chloro-4,5-difluoro-phényle ; 2-bromo-4-fluoro-phényle ; 2-bromo-4,6-difluoro-phényle ; 4-chloro-2,5-difluoro-phényle ; 5-chloro-2,4-difluoro-phényle ; 4-bromo-2,5-difluoro-phényle ; 5-bromo-2,4-difluoro-phényle ; pentafluoro-phényle ; 2,4-dinitro-phényle ; 4-chloro-3-nitro-phényle ; 2-méthyl-5-nitro-phényle ; 5-bromo-2-méthoxy-phényle ; 3-chloro-2-méthyl-phényle ; 4-bromo-3-méthyl-phényle ; 4-chloro-2,5-diméthyl-phényle ; 4-fluoro-3-méthyl-phényle ; 5-fluoro-2-méthyl-phényle ; 2-nitro-4-trifluorométhyl-phényle ; 2-méthoxy-4-méthyl-phényle ; 3,5-dichloro-2-hydroxy-phényle ; 3,5-dichloro-4-hydroxy-phényle ; 5-chloro-2,4-difluoro-phényle ; 3-chloro-4-(NH)-(C=O)-CH3-phényle; 2-chloro-6-méthyl-phényle ; 2-chloro-5-trifluorométhyl-phényle ; 2-chloro-5-trifluorométhoxy-phényle ; 4-bromo-2-trifluorométhoxy-phényle ; 4-bromo-2-trifluorométhyl-phényle ; 4-bromo-3-trifluorométhyl-phényle ; 3-carboxy-4-fluoro-phényle ; 3-carboxy-4-chloro-6-fluoro-phényle ; 4-méthoxy-2,3,6-triméthyl-phényle ; ou l'un des groupes suivants : 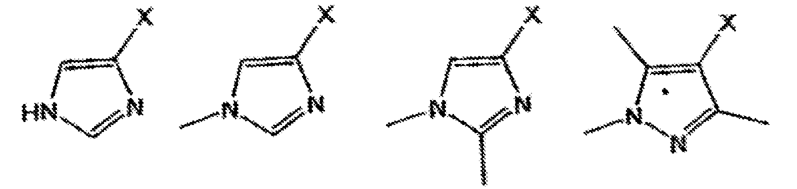 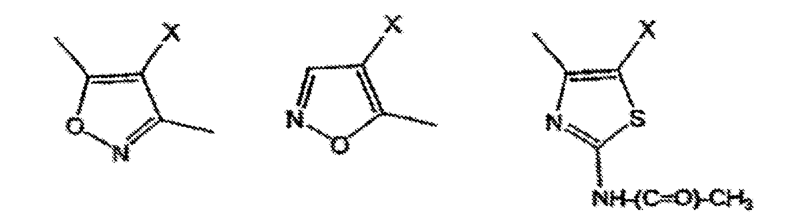 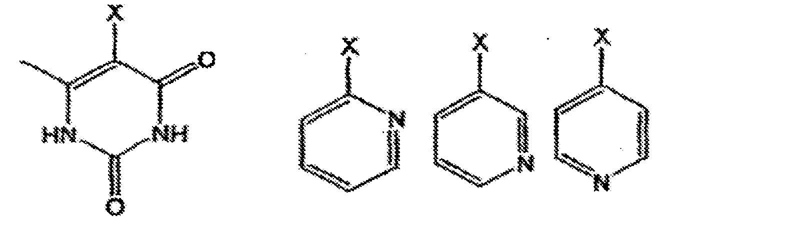 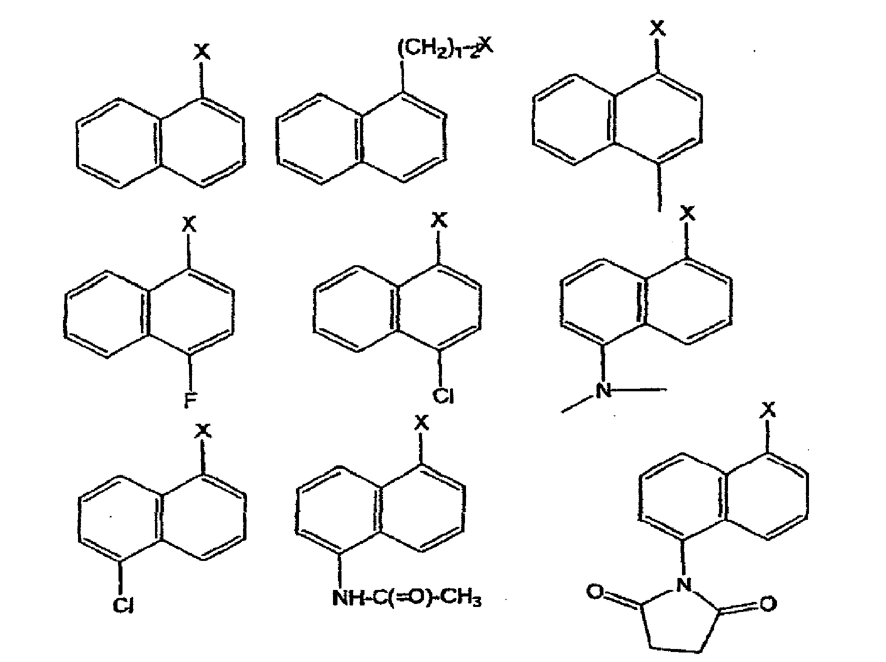 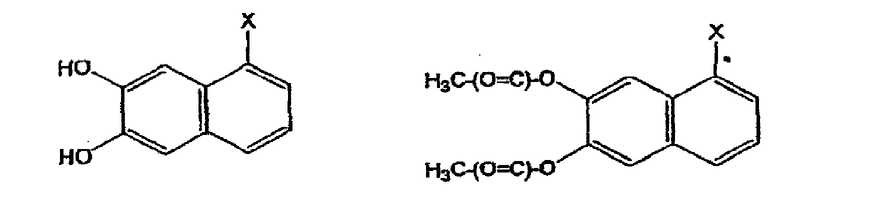 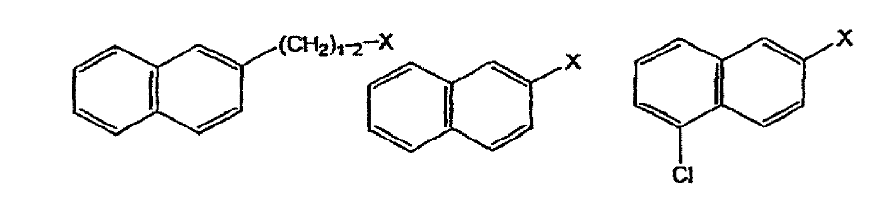 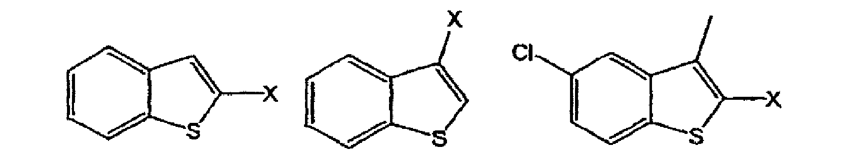 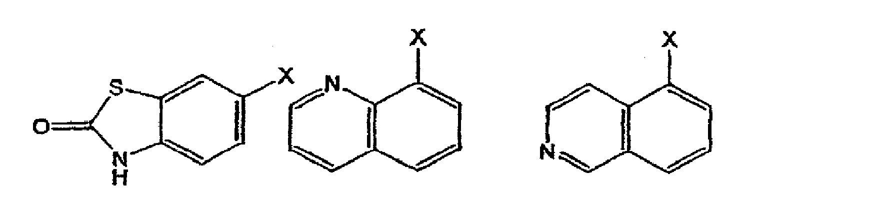 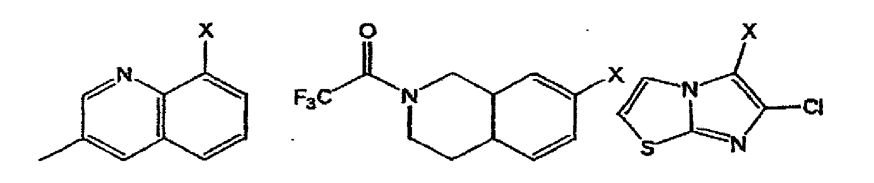 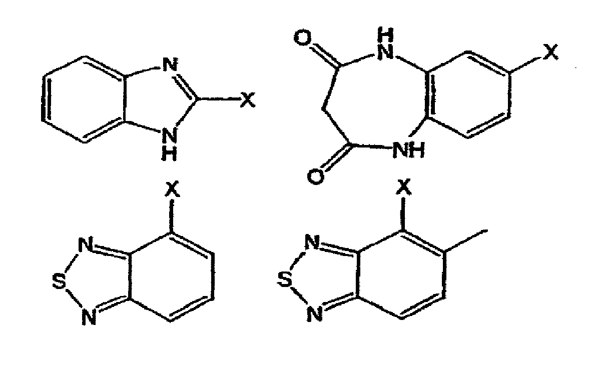 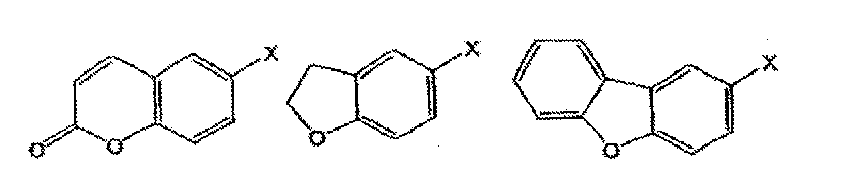 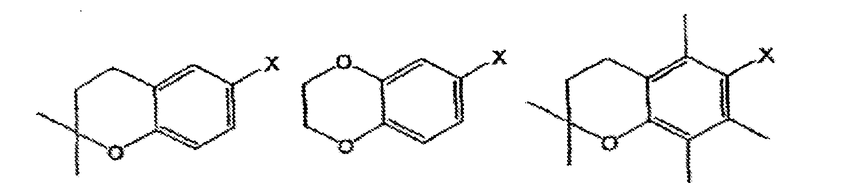 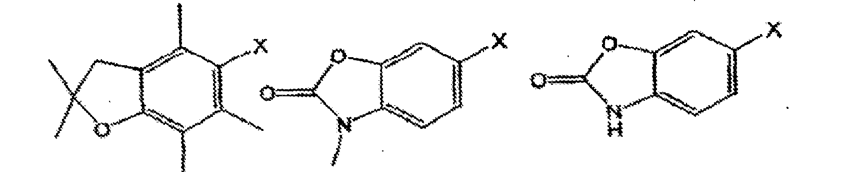 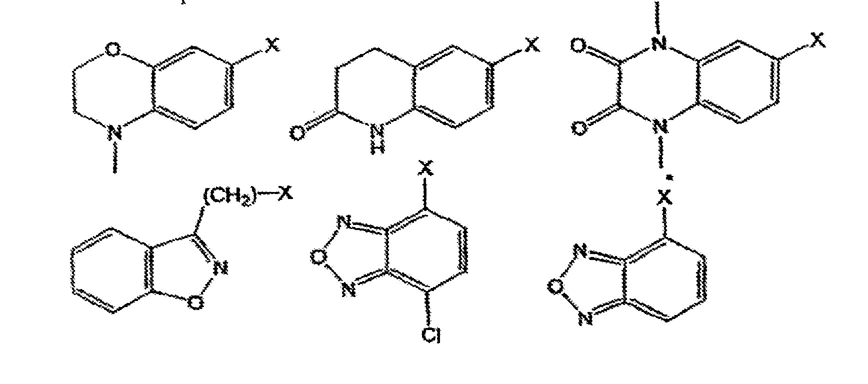 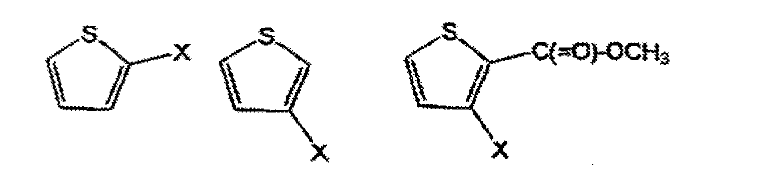 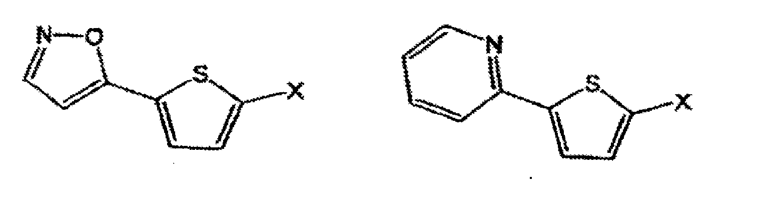 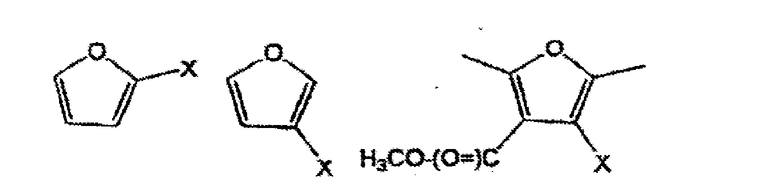 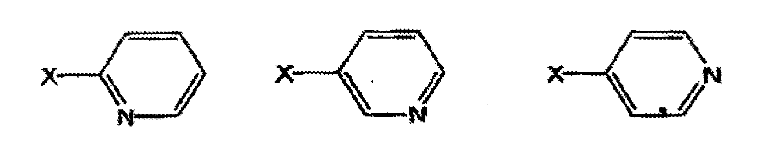 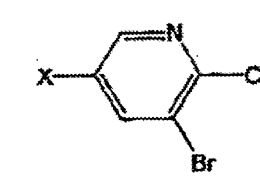
de sorte que, dans chaque cas, X indique la position à laquelle le substituant respectif W^{b} est lié au groupe -SO₂ de formule (Ib),
facultativement, sous la forme de l'un de leurs stéréoisomères, référence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport d'un mélange, ou, respectivement, un sel physiologiquement acceptable de ceux-ci, ou un solvat.

57. Composés selon l'une quelconque des revendications 42 à 56, choisis parmi le groupe suivant :
| N° | Composé |
|---|---|
| 1 | 1-[1-(naphtalène-2-sulfonyl)-pipéridin-4-yl]-1,4-dihydro-benzo[d] [1,3]oxazin-2-one |
| 2 | 1-[1-(toluène-4-sulfonyl)-pipéridin-4-yl]-1,4-dihydro-benzo[d][1,3]oxazin-2-one |
| 3 | 1-(1-phénylméthanesulfonyl-pipéridin-4-yl)-1,4-dihyro-benzo[d][1,3]oxazin-2-one |
| 4 | 1-(1-benzènesulfonyl-pipéridin-4-yl)-6-chloro-1,4-dihydro-benzo[d][1,3]oxazin-2-one |
| 5 | 6-chlore-1-[1-(toluène-4-sulfonyl)-pipéridin-4-yl]-1,4-dihydro-benzo[d][1,3]oxazin-2-one |
| 6 | 6-chloro-1-(1-phénylméthanesulfonyl-pipéridin-4-yl)-1 ,4-dihydro-benzo[d][1,3]oxazin-2-one |
| 7 | 6-chlore-1-[1-(naphtalène-1-sulfonyl)-pipéridin-4-yl]-1,4-dihydro-benzo[d][1,3]oxazin-2-one |
| 8 | 6-chlore-1-[1-(naphtalène-2-sulfonyl)-pipéridin-4-yl]-1,4-dihydro-benzo[d][1,3]oxazin-2-one |
| 9 | 6-chlore-1-[1-(5-chloro-3-méthyl-benzo[b]thiophène-2-sulfonyl)-pipéridin-4-yl]-1,4-dihydro-benzo[d] [1,3]oxazin-2-one |
| 10 | 1-[1-(thiophèn-2-sulfonyl)-pipéridin-4-yl]-1,4-dihydro-benzo[d][1,3]oxazin-2-one |
| 11 | 1-[1-(4-acétyl-benzènesulfonyl)-pipéridin-4-yl]-1,4-dihydro-benzo[d][1,3]oxazin-2-one |
| 12 | 2-[4-(2-oxo-4H-benzo[d][1,3]oxazin-1-yl)-pipéridine-1-sulfonyl]-benzonitrile |
| 13 | 1-[1-(2,4-diméthyl-benzènesulfonyl)-pipéridin-4-yl]-1,4-dihydro-benzo[d][1,3]oxazin-2-one |
| 14 | 1-[1-(4-méthoxy-benzènesulfonyl)-pipéridin-4-yl]-1,4-dihydro-benzo[d][1,3]oxazin-2-one |
| 15 | 1-[1-(2-naphtalén-1-yl-éthanesulfonyl)-pipéridin-4-yl]-1,4-dihydro-benzo[d][1,3]oxazin-2-one |
| 16 | 8-méthyle-1-[1-(thiophène-2-sulfonyl)-pipéridin-4-yl]-1,4-dihydro-benzo[d][1,3]oxazin-2-one |
| 17 | 1-[1-(4-acétyl-benzènesulfonyl)-pipéridin-4-yl]-8-méthyl-1,4-dihydro-benzo[d][1,3]oxazin-2-one |
| 18 | 2-[4-(8-méthyl-2-oxo-4H-benzo[d][1,3]oxazin-1-yl)-pipéridin-1-sulfonyl]benzonitrile |
| 19 | 1-[1-(2,4-diméthyl-benzènesulfonyl)-pipéridin-4-yl]-8-méthyl-1,4-dihydro-benzo[d][1,3]oxazin-2-one |
| 20 | 1-[1-(4-méthoxy-benzènesulfonyl)-pipéridin-4-yl]-8-méthyl-1,4-dihydro-benzo[d][1,3]oxazin-2-one |
| 21 | 8-méthyl-1-[1-(2-naphtalén-1-yl-éthanesulfonyl)-pipéridin-4-yl]-1,4-dihydro-benzo[d] [1,3]oxazin-2-one |
| 22 | Diméthylamide de l'acide 4-(8-méthyl-2-oxo-4H-benzo[d][1,3]oxazin-1-yl)-pipéridin-1-sulfonique |
| 23 | Ester méthylique de l'acide 2-[4-(2-oxo-4H-benzo[d][1,3]oxazin-1-yl)-pipéridin-1-sulfonyl]-benzoïque |
| 24 | 1-[ 1-(3-trifluorométhyl-benzènesulfonyl)-pipéridin-4-yl]-1,4-dihydro-benzo [d] [1,3]oxazin-2-one |
| 25 | Ester méthylique de l'acide 2-[4-(8-méthyl-2-oxo-4H-benzo [d][1,3]oxazin-1-yl)-pipéridin-1-sulfonyl]-benzoïque |
| 26 | 8-méthyl-1-[1-(3-trifluorométhyl-benzènesulfonyl)-pipéridin-4-yl]-1,4-dihydro-benzo[d][1,3]oxazin-2-one |
| 27 | 1-[1-(4-acétyl-benzènesulfonyl)-pipéridin-4-yl]-6-chloro-1,4-dihydro-benzo[d][1,3]oxazin-2-one |
| 28 | 2-[4-(6-chloro-2-oxo-4H-benzo[d] [1,3]oxazin-1-yl)-pipéridin-1-sulfonyl]-benzonitrile |
| 29 | 6-chlore-1-[1-(4-méthoxy-benzènesulfonyl)-pipéridin-4-yl]-1,4-dihydro-benzo[d] [1,3]oxazin-2-one |
| 30 | Ester méthylique de l'acide 2-[4-(6-chloro-2-oxo-4H-benzo[d][1,3]oxazin-1-yl)-pipéridin-1-sulfonyl]-benzoïque |
| 31 | 6-chloro-1-[1-(2,4-diméthyl-benzènenesulfonyl)-pipéridin-4-yl]-1,4-dihydro-benzo[d][1,3]oxazin-2-one |
| 32 | 6-chlore-1-[1-(3-trifluorométhyl-benzènesulfonyl)-pipéridin-4-yl]-1,4-dihydro-benzo[d][1,3]oxazin-2-one |
| 33 | 1-[1-(5-chloro-3-méthyl-benzo[b]thiophène-2-sulfonyl)-pipéridin-4-yl]-8-méthyl-1,4-dihydro-benzo[d][1,3]oxazin-2-one |
| 34 | 1-(1-[4-(4-bromo-phénoxy)-benzènesulfonyl]-pipéridin-4-yl)-8-méthyl-1,4-dihydro-benzo[d][1,3]oxazin-2-one |
| 35 | 1-[1-(4-fluoro-benzènesulfonyl)-pipéridin-4-yl]-8-méthyl-1,4-dihydro-benzo[d][1,3]oxazin-2-one |
| 36 | 8-méthyle-1-[1-(naphtalène-2-sulfonyl)-pipéridin-4-yl]-1,4-dihydro-benzo[d][1,3]oxazin-2-one |
| 37 | 8-méthyl-1-(1-phénylméthanesulfonyl-pipéridin-4-yl)-1,4-dihydro-benzo[d](1,3]oxazin-2-one |
| 38 | 1-[1-(4-bromo-benzènesulfonyl)-pipéridin-4-yl]-6-chloro-1,4-dihydro-benzo[d][1,3]oxazin-2-one |
| 39 | 6-chlore-1-[1-(4-méthanesulfonyl-benzènesufonyl)-pipéridin-4-yl]-1,4-dihydro-benzo[d][1,3]oxazin-2-one |
| 40 | 1-[1-(butane-1-sulfonyl)-pipéridin-4-yl]-6-chloro-1,4-dihydro-benzo[d][1,3]oxazin-2-one |
| 41 | 1-[1-(4-bromo-benzènesulfonyl)-pipéridin-4-yl]-8-méthyl-1,4-dihydro-benzo[d][1,3]oxazin-2-one |
| 42 | 1-[1-(4-méthanesulfonyl-benzènesufonyl)-pipéridin-4-yl]-8-méthyl-1,4-dihydro-benzo[d][1,3]oxazin-2-one |
| 43 | 1-[1-(butane-1-sulfonyl)-pipéridin-4-yl]-8-méthyl-1,4-dihydro-benzo[d][1,3]oxazin-2-one |
| 44 | 6-chlore-1-[1-(2-nitro-benzènesulfonyl)-pipéridin-4-yl]-1,4-dihydro-benzo[d][1,3]oxazin-2-one |
| 45 | 6-chlore-1-[1-(3-nitro-benzènesulfonyl)-pipéridin-4-yl]-1,4-dihydro-benzo[d][1,3]oxazin-2-one |
| 46 | 1-[1-(biphényl-4-sulfonyl)pipéridin-4-yl]-6-chloro-1,4-dihydro-benzo[d][1,3]oxazin-2-one |
| 47 | 8-méthyle-1-[1-(2-nitro-benzènesulfonyl)-pipéridin-4-yl]-1,4-dihydro-benzo[d][1,3]oxazin-2-one |
| 48 | 8-méthyle-1-[1-(3-nitro-benzènesulfonyl)-pipéridin-4-yl]-1,4-dihydro-benzo[d][1,3]oxazin-2-one |
| 49 | 1-[1-(biphényl-4-sulfonyl)pipéridin-4-yl]-8-méthyl-1,4-dihydro-benzo[d][1,3]oxazin-2-one |
| 50 | 8-méthyle-1-[1-(4-nitro-benzènesulfonyl)-pipéridin-4-yl]-1,4-dihydro-benzo[d][1,3]oxazin-2-one |
| 51 | 6-chloro-1-[1-(4-nitro-benzènesulfonyl)-pipéridin-4-yl]-1,4-dihydro- benzo[d][1,3]oxazin-2-one |
| 52 | 1-(1-éthanesulfonyl-pipéridin-4-yl)-1,4-dihydro-benzo[d][1,3]oxazin-2-one |
| 53 | 1-[1-(propane-1-sulfonyl)-pipéridin-4-yl]-1,4-dihydro-benzo[d][1,3]oxazin-2-one |
| 54 | 1-[1-(propane-2-sulfonyl)-pipéridin-4-yl]-1,4-dihydro-benzo[d][1,3]oxazin-2-one |
| 55 | 6-chloro-1-(1-éthanesulfonyl-pipéridin-4-yl)-1,4-dihydro-benzo[d][1,3]oxazin-2-one |
| 56 | 6-chlore-1-[1-(propane-1-sulfonyl)-pipéridin-4-yl]-1,4-dihydro-benzo[d][1,3]oxazin-2-one |
| 57 | 6-chlore-1-[1-(propane-2-sulfonyl)-pipéridin-4-yl]-1,4-dihydro-benzo[d][1,3]oxazin-2-one |
| 58 | 1-[1-(quinoléine-8-sulfonyl)-pipéridin-4-yl]-1,4-dihydro-benzo[d][1,3]oxazin-2-one |
| 59 | 1-[1-(4-nitro-benzènesulfonyl)-pipéridin-4-yl]-1,4-dihydro-benzo[d][1,3]oxazin-2-one |
| 60 | 6-méthyle-1-[1-(quinoléine-8-sulfonyl)-pipéridin-4-yl]-1,4-dihydro-benzo[d][1,3]oxazin-2-one |
| 61 | 6-méthyle-1-[1-(2-naphtalène-1-yl-éthanesulfonyl)-pipéridin-4-yl]-1,4-dihydro-benzo[d] [1,3]oxazin-2-one |
| 62 | 6-méthyle-1-[1-(toluène-4-sulfonyl)-pipéridin-4-yl]-1,4-dihydro-benzo[d][1,3]oxazin-2-one |
| 63 | 1-[[1-(4-fluoro-benzènesulfonyl)-pipéridin-4-yl]-6-méthyl-1,4-dihydro-benzo[d][1,3]oxazin-2-one |
| 64 | 6-méthyle-1-[1-(naphtalène-1-sulfonyl)-pipéridin-4-yl]-1,4-dihydro-benzo[d][1,3]oxazin-2-one |
| 65 | 6-méthyle-1-[ 1-(naphtalène-2-sulfonyl)-pipéridin-4-yl]-1,4-dihydro-benzo[d][1,3]oxazin-2-one |
| 66 | 1-[1-(5-chloro-3-méthyl-benzo[b]thiophène-2-sulfonyl)-pipéridin-4-yl]-6-méthyl-1,4-dihydro-benzo[d][1,3]oxazin-2-one |
| 67 | 6-méthyle-1-[1-(4-nitro-benzènesulfonyl)-pipéridin-4-yl]-1,4-dihydro-benzo[d][1,3]oxazin-2-one |
| 68 | 1-(1-benzènesulfonyl-pipéridin4-yl)-6-méthyl-1,4-dihydro-benzo[d][1,3]oxazin-2-one |
| 69 | 1-[[1-(4-chloro-3-nitro-benzènesulfonyl)-pipéridin-4-yl]-6-méthyl-1,4-dihydro-benzo[d][1,3]oxazin-2-one |
| 70 | 1-[1-(5-diméthylamino-naphtalène-1-sulfonyl)-pipéridin-4-yl]-6-méthyl-1,4-dihydro-benzo[d][1,3]oxazin-2-one |
| 71 | 1-[[1-(4-chloro-3-nitro-benzènesulfonyl)-pipéridin-4-yl]-8-méthyl-1,4-dihydro-benzo[d][1,3]oxazin-2-one |
| 72 | 1-[1-(4-chloro-3-nitro-benzènesulfonyl)-pipéridin-4-yl]-1,4-dihydro-benzo[d][1,3]oxazin-2-one |
| 73 | 6-chlore-1-[1-(4-chloro-3-nitro-benzènesulfonyl)-pipéridin-4-yl]-1,4-dihydro-benzo[d][1,3]oxazin-2-one |
| 74 | 6-chlore-1-[1-(5-diméthylamino-naphtalène-1-sulfonyl)-pipéridin-4-yl]-1,4-dihydro-benzo[d][1,3]oxazin-2-one |
| 75 | 1-[1-(4-méthoxy-2,3,6-triméthyl-benzènesulfonyl)-pipéridin-4-yl]-1,4-dihydro-benzo[d][1,3]oxazin-2-one |
| 76 | 1-[1-(4-méthoxy-2,3,6-triméthyl-benzènesulfonyl)-pipéridin-4-yl]-8-méthyl-1,4-dihydro-benzo[d][1,3]oxazin-2-one |
| 77 | 6-chlore-1-[1-(4-méthoxy-2,3,6-triméthyl-benzènesulfonyl)-pipéridin-4-yl]-1,4-dihydro-benzo[d][1,3]oxazin-2-one |
| 78 | 1-[1-(4-méthoxy-2,3,6-triméthyl-benzènesulfonyl)-pipéridin-4-yl]-6-méthyl-1,4-dihydro-benzo[d][1,3]oxazin-2-one |
| 79 | 1-[1-(2-bromo-benzènesulfonyl)-pipéridin-4-yl]-1,4-dihydro-benzo[d] [1,3]oxazin-2-one |
| 80 | 1-[1-(2-bromo-benzènesulfonyl)-pipéridin-4-yl]-8-méthyl-1,4-dihydro-benzo[d][1,3]oxazin-2-one |
| 81 | 1-[1-(2-bromo-benzènesulfonyl)-pipéridin-4-yl]-6-chloro-1,4-dihydro-benzo[d][1,3]oxazin-2-one |
| 82 | 1-[1-(2-bromo-benzènesulfonyl)-pipéridin-4-yl]-6-méthyl-1,4-dihydro-benzo[d][1,3]oxazin-2-one |
| 83 | 6-chlore-1-[1-(2,3-dichloro-benzènesulfonyl)-pipéridin-4-yl]-1,4-dihydro-benzo[d][1,3]oxazin-2-one |
| 84 | 1-[1-(2,3-dichloro-benzènesulfonyl)-pipéridin-4-yl]-6-méthyl-1,4-dihydro-benzo[d][1,3]oxazin-2-one |
| 85 | 1-[1-(2,4,5-trichloro-benzènesulfbnyl)-pipéridin-4-yl]-1,4-dihydro-benzo[benzo[d][1,3]oxazin-2-one |
| 86 | 8-méthyle-1-[1-(2,4,5-trichloro-benzènesulfonyl))-pipéridin-4-yl]-1,4-dihydro-benzo[d][1,3]oxazin-2-one |
| 87 | 6-chlore-1-[1-(2,4,5-trichloro-benzènesulfonyl))-pipéridin-4-yl]-1,4-dihydro-benzo[d][1,3]oxazin-2-one |
| 88 | 6-méthyle-1-[1-(2,4,5-trichloro-benzènesulfonyl))-pipéridin-4-yl]-1,4-dihydro-benzo[d][1,3]oxazin-2-one |
| 89 | 1-[1-(5-bromo-2-méthoxy-benzènesulfonyl))-pipéridin-4-yl]-1,4-dihydro-benzo[d][1,3]oxazin-2-one |
| 90 | 1-[1-(5-bromo-2-méthoxy-benzènesulfonyl)-pipéridin-4-yl]-8-méthyl-1,4-dihydro-benzo[d][1,3]oxazin-2-one |
| 91 | 1-[1-(5-bromo-2-méthoxy-benzènesulfonyl)-pipéridin-4-yl]-6-chloro-1,4-dihydro-benzo[d][1,3]oxazin-2-one |
| 92 | 1-[1-(5-bromo-2-méthoxy-benzènesulfonyl)-pipéridin-4-yl]-6-méthyl-1,4-dihydro-benzo[d][1,3]oxazin-2-one |
| 93 | 1-[1-(2,5-diméthoxy-benzènesulfonyl)-pipéridin-4-yl]-1,4-dihydro-benzo[d][1,3]oxazin-2-one |
| 94 | 1-[1-(2,5-diméthoxy-benzènesulfonyl)-pipéridin-4-yl]-8-méthyl-1,4-dihydro-benzo[d][1,3]oxazin-2-one |
| 95 | 6-chlore-1-[1-(2,5-diméthoxy-benzènesulfonyl)-pipéridin-4-yl]-1,4-dihydro-benzo[d]1,3]oxazin-2-one |
| 96 | 1-[1-(2,5-diméthoxy-benzènesulfonyl)-pipéridin-4-yl]-6-méthyl-1,4-dihydro-benzo[d][1,3]oxazin-2-one |
| 97 | 1-(1-pentaméthylbenzènesulfonyl-pipéridin-4-yl]-1,4-dihydro-benzo[d][1,3]oxazin-2-one |
| 98 | 8-méthyl-1-(1-pentaméthylbenzènesulfonyl-pipéridin-4-y1]-1,4-dihydro-benzo[d][1,3]oxazin-2-one |
| 99 | 6-chloro-1-(1-pentaméthylbenzènesulfonyl-pipéridin-4-yl)-1,4-dihydro-benzo[d][1,3]oxazin-2-one |
| 100 | 6-méthyl-1-(1-pentaméthylbenzènesulfonyl-pipéridin-4-y1)-1,4-dihydro-benzo[d][1,3]oxazin-2-one |
| 101 | 1-{1-[2-(2,2,2-trifluoro-acétyl)-1,2,3,4-tétrahydro-isoqumoléine-7-sulfonyl]-pipéridin-4-yl}-1,4-dihydro-benzo[d][1,3]oxazin-2-one |
| 102 | 8-méthyl-1-{1-[2-(2,2,2-trifluoro-acétyl)-1,2,3,4-tétrahydro-isoquinoléine-7-sulfonyl]-pipéridin-4-yl)-1,4-dihydro-benzo[d][1,3]oxazin-2-one |
| 103 | 6-chloro-1-{1-[2-(2,2,2-trifluoro-acétyl)-1,2,3,4-tétrahydro-isoquinoléine-7-sulfonyl] -pipéridin-4-yl)-1,4-dihydro-benzo[d][1,3]oxazin-2-one |
| 104 | 8-méthyle-1-{1-[2-(2,2,2-trifluoro-acétyl)-1,2,3,4-tétrahydro-isoquinoléine-7-sulfonyl]-pipéridin-4-yl}1,4-dihydro-benzo[d][1,3]oxazin-2-one |
| 105 | 1-1-(2-méthyl-5-nitro-benzènesulfonyl)-pipéridin-4-yl]-1,4-dihydro-benzo[d][1,3]oxazin-2-one |
| 106 | 8-méthyle-1-[1-(2-méthyl-5-niro-benzènesulfonyl)-pipéridin-4-yl]-1,4-dihydro-benzo[d][1,3]oxazin-2-one |
| 107 | 6-chloro-1-[1-(2-méthyl-5-nitro-benzènesulfonyl)-pipéridin-4-yl)-1,4-dihydro-benzo[d][1,3]oxazin-2-one |
| 108 | 6-méthyl-1-[1-(2-méthyl-5-nitro-benzènesulfonyl)-pipéridin-4-yl]-1,4-dihydro-benzo[d][1,3]oxazin-2-one |
| 109 | 1-[1-(4-bromo-2,5-difluoro-benzènesulfonyl)-pipéridin-4-yl]-1,4-dihydro-benzo[d][1,3]oxazin-2-one |
| 110 | 1-[1-(4-bromo-2,5-difluoro-benzènesulfonyl)-pipéridin-4-yl]-8-méthyl-1,4-dihydro-benzo[d][1,3]oxazin-2-one |
| 111 | 1-[1-(4-bromo-2,5-difluoro-benzènesulfonyl)-pipéridin-4-yl)-6-chloro-1,4-dihydro-benzo[d][1,3]oxazin-2-one |
| 112 | 1-[1-(4-bromo-2,5-difluoro-benzènesulfonyl)-pipéridin-4-yl)-6-méthyl-1,4-dihydro-benzo[d][1,3]oxazin-2-one |
| 113 | 1-[1-4-chloro-2,5-diméthyl-benzènesulfonyl)-pipéridin-4-yl]-1,4-dihydro-benzo[d][1,3]oxazin-2-one |
| 114 | 1-[1-4-chloro-2,5-diméthyl-benzènesulfonyl)-pipéridin-4-yl]-8-méthyl-1,4-dihydro-benzo[d][1,3]oxazin-2-one |
| 115 | 6-chloro-1-[1-(4-chloro-2,5-diméthyl-benzènesulfonyl)-pipéridin-4-yl]-1,4-dihydro-benzo[d][1,3]oxazin-2-one |
| 116 | 1-[1-4-chloro-2,5-diméthyl-benzènesulfonyl)-pipéridin-4-yl]-6-méthyl-1,4-dihydro-benzo[d][1,3]oxazin-2-one |
| 117 | 1-[1-(4-méthoxy-benzènesulfonyl)-pipéridin-4-yl]-6-méthyl-1,4-dihydro-benzo[d][1,3]oxazin-2-one |
| 118 | 1-[1-(4-isopropyl-benzènesulfonyl)-pipéridin-4-yl]-1,4-dihydro-benzo[d][1,3]oxazin-2-one |
| 119 | 1-[1-(4-isopropyl-benzènesulfonyl)-pipéridin-4-yl]-8-méthyl-1,4-dihydro-benzo[d][1,3]oxazin-2-one |
| 120 | 6-chloro-1-[1-(4-isopropyl-benzènesulfonyl)-pipéridin-4-yl]-1,4-dihydro-benzo[d][1,3]oxazin-2-one |
| 121 | 1-[1-(4-isopropyl-benzènesulfonyl)-pipéridin-4-yl]-6-méthyl-1,4-dihydro-benzo[d][1,3]oxazin-2-one |
| 122 | 1-[1-(3-chloro-4-fluoro-benzènesulfonyl)-pipéridin-4-yl]-1,4-dihydro-benzo[d][1,3]oxazin-2-one |
| 123 | 1-[1-(3-chloro-4-fluoro-benzènesulfonyl)-pipéridin-4-yl]-8-méthyl-1,4-dihydro-benzo[d][1,3]oxazin-2-one |
| 124 | 6-chlore-1-[1-(3-chloro-4-fluoro-benzènesulfonyl)-pipéridin-4-yl]-1,4-dihydro-benzo[d][1,3]oxazin-2-one |
| 125 | 1-[[1-(3-chloro-4-fluoro-benzènesulfonyl)-pipéridin-4-yl]-6-méthyl-1,4-dihydro-benzo[d][1,3]oxazin-2-one |
| 126 | 1-[1-(4-bromo-benzènesulfonyl)-pipéridin-4-yl]-6-méthyl-1,4-dihydro-benzo[d][1,3]oxazin-2-one |
| 127 | 6-méthyle-1-[1-(3-nitro-benzènesulfonyl)-pipéridin-4-yl]-1,4-dihydro-benzo[d][1,3]oxazin-2-one |
| 128 | 6-méthyle-1-[1-(3-trifluorométhyl-benzènesulfonyl)-pipéridin-4-yl]-1,4-dihydro-benzo[d][1,3]oxazin-2-one |
| 129 | 1-[1-(4-trifluorométhoxy-benzènesulfonyl)-pipéridin-4-yl]-1,4-dihydro-benzo[d][1,3]oxazin-2-one |
| 130 | 1-[1-(2-nitro-4-trifluorométhyl-benzènesulfonyl)-pipéridin-4-yl]-1,4-dihydro-benzo[d][1,3]oxazin-2-one |
| 131 | 1-[1-(3-fluoro-benzènesulfonyl)-pipéridin-4-yl]-1,4-dihydro-benzo[d][1,3]oxazin-2-one |
| 132 | 1-[1-(2,4-dichloro-benzènesulfonyl)-pipéndin-4-yl]-1,4-dihydro-benzo[d][1,3]oxazin-2-one |
| 133 | 1-[1-(2,4,6-triméthyl-benzènesulfonyl)-pipéridin-4-yl]-1,4-dihydro-benzo[d][1,3]oxazin-2-one |
| 134 | 1-[1-(2-trifluorométhyl-benzènesulfonyl)-pipéridin-4-yl]-1,4-dihydro-benzo[d][1,3]oxazin-2-one |
| 135 | 8-méthyl-1-[1-(4-trifluorométhoxy-benzènesulfonyl)-pipéridin-4-yl]-1,4-dihydro-benzo[d][1,3]oxazin-2-one |
| 136 | 8-méthyl-1-[1-(2-nitro-4-trifluorométhyl-benzènesulfonyl)-pipéridin-4-yl]-1,4-dihydro-benzo[d][1,3]oxazin-2-one |
| 137 | 1-[1-(3-fluoro-benzènesulfonyl)-pipéridin-4-yl]-8-méthyl-1,4-dihydro-benzo[d][1,3]oxazin-2-one |
| 138 | 1[1-(2,4-dichloro-benzènesulfonyl)-pipéridin-4-yl]-8-méthyl-1,4-dihydro-benzo[d][1,3]oxazin-2-one |
| 139 | 8-méthyl-1-[1-(2,4,6-triméthyl-benzènesulfonyl)-piperidin-4-yl]-1,4-dihydro-benzo[d][1,3]oxazin-2-one |
| 140 | 8-méthyle-1-[1-(2-trifluorométhyl-benzènesulfonyl)-pipéridin-4-yl]-1,4-dihydro-benzo[d][1,3]oxazin-2-one |
| 141 | 1-[1-(4-fluoro-benzènesulfonyl)-pipéridin-4-yl]-1,4-dihydro-benzo[d][1,3]oxazin-2-one |
| 142 | 1-[1-(4-bromo-benzènesulfonyl)-pipéridin-4-yl]-1,4-dihydro-benzo[d][1,3]oxazin-2-one |
| 143 | 1-[1-(3-nitro-benzènesulfonyl)-pipéridin-4-yl]-1,4-dihydro-benzo[d][1,3]oxazin-2-one |
| 144 | 1-{1-[4-(4-bromo-phénoxy)-benzènesulfonyl]-pipéridin-4-yl}-1,4-dihydro-benzo[d][1,3]oxazin-2-one |
| 145 | 1-[1-(3-méthoxy-benzènesulfonyl)-pipéridin-4-yl]-1,4-dihydro-benzo[d][1,3]oxazin-2-one |
| 146 | 1-[1-(2-nitro-benzènesulfonyl)-pipéridin-4-yl]-1,4-dihydro-benzo[d][1,3]oxazin-2-one |
| 147 | 8-méthyle-1-[[1-toluène-4-sulfonyl)-pipéridin-4-yl]-1,4-dihydro-benzo[d][1,3]oxazin-2-one |
| 148 | 1-(1-benzènesulfonyl-pipéridin-4-yl)-8-méthyl-1,4-dihydro-benzo[d][1,3]oxazin-2-one |
| 149 | 1-[1-(3-méthoxy-benzènesulfonyl)-pipéridin-4-yl]-8-méthyl-1,4-dihydro-benzo[d][1,3]oxazin-2-one |
| 150 | 1-[1-(2,4-diméthyl-benzènesulfonyl)-pipéridin-4-yl]-6-méthyl-1,4-dihydro-benzo[d]1,3]oxazin-2-one |
| 151 | 1-{1-[4-(4-bromo-phénoxy)-benzènesulfonyl]-pipéridin-4-yl}-6-méthyl-1,4-dihydro-benzo[d][1,3]oxazin-2-one |
| 152 | 6-méthyle-1-[1-(thiophène-2-sulfonyl)-pipéridin-4-yl]-1,4-dihydro-benzo[d][1,3]oxazin-2-one |
| 153 | 1-[1-(toluène-3-sulfonyl)-pipéridin-4-yl]-1,4-dihydro-benzo[d][1,3]oxazin-2-one |
| 154 | 1-[1-(5-fluoro-2-méthyl-benzènesulfonyl)-pipéridin-4-yl]-1,4-dihydro-benzo[d][1,3]oxazin-2-one |
| 155 | 1-[1-(4-isopropoxy-benzènesulfonyl)-pipéridin-4-yl]-1,4-dihydro-benzo[d]1,3]oxazin-2-one |
| 156 | 1-[1-(3-chloro-benzènesulfonyl)-pipéridin-4-yl]-1,4-dihydro-benzo[d][1,3]oxazin-2-one |
| 157 | 1-[1-(3,4-diméthoxy-benzènesulfonyl)-pipéridin-4-yl]-1,4-dihydro-benzo[d][1,3]oxazin-2-one |
| 158 | 1-(1-pentafluorobenzènesulfonyl-pipéridin-4-yl]-1,4-dihydro-benzo[d][1,3]oxazin-2-one |
| 159 | 8-méthyl-1-[1-(toluène-3-sulfonyl)-pipéridin-4-yl]-1,4-dihydro-benzo[d][1,3]oxazin-2-one |
| 160 | 1-[1-(5-fluoro-2-méthyl-benzènesulfonyl)-pipéridin-4-yl]-8-méthyl-1,4-dihydrobenzo[d][1,3]oxazin-2-one |
| 161 | 1-[[1-(4-isopropoxy-benzènesulfonyl)-pipéridin-4-yl]-8-méthyl-1,4-dihydro-benzo[d][1,3]oxazin-2-one |
| 162 | 1-[1-3-chloro-benzènesulfonyl)-pipéridin-4-yl]-8-méthyl-1,4-dihydro-benzo[d][1,3]oxazin-2-one |
| 163 | 1-[1-(3,4-diméthoxy-benzènesulfonyl)-pipéridin-4-yl]-8-méthyl-1,4-dihydro-benzo[d][1,3]oxazin-2-one |
| 164 | 8-méthyl-1-(1-pentafluorobenzènesulfonyl-pipéridin-4-yl]-1,4-dihydro-benzo[d][1,3]oxazin-2-one |
| 165 | 6-méthyl-1-(1-(toluène-3-sulfonyl)-pipéridin-4-yl] -1,4-dihydro-benzo[d][1,3]oxazin-2-one |
| 166 | 1-[1-(5-fluoro-2-méthyl-benzènesulfonyl)-pipéridin-4-yl]-6-méthyl-1,4-dihydrobenzo[d][1,3]oxazin-2-one |
| 167 | 1-[1-(4-isopropoxy-benzènesulfonyl)-pipéridin-4-yl]-6-méthyl-1,4-dihydro-benzo[d][1,3]oxazin-2-one |
| 168 | 1-[1-(3-chloro-benzènesulfonyl)-pipéridin-4-yl]-6-méthyl-1,4-dihydrobenzo[d][1,3]oxazin-2-one |
| 169 | 1-[1-(3,4-diméthoxy-benzènesulfonyl)-pipéridin-4-yl]-6-méthyl-1,4-dihydro-benzo[d][1,3]oxazin-2-one |
| 170 | 6-méthyle-1-(1-pentafluorobenzènesulfonyl-pipéridin-4-yl]-1,4-dihydro-benzo[d][1,3]oxazin-2-one |
| 171 | 6-méthyl-1-[1-(4-trifluorométhoxy-benzènesulfonyl)-pipéridin-4-yl]-1,4-dihydro-benzo[d][1,3]oxazin-2-one |
| 172 | 6-méthyl-1-[1-2-nitro-4-trifluorométhyl-benzènesulfonyl)-pipéridin-4-yl]-1,4-dihydro-benzo[d][1,3]oxazin-2-one |
| 173 | 1-[1-(3-fluoro-benzènesulfonyl)-pipéridin-4-yl]-6-méthyl-1,4-dihydro-benzo[d][[1,3]oxazin-2-one |
| 174 | 1-[[1-(2,4-dichloro-benzènesulfonyl)-pipéridin-4-yl]-6-méthyl-1,4-dihydro-benzo[d][1,3]oxazin-2-one |
| 175 | 6-méthyle-1-[1-(2,4,6-triméthyl-benzènesulfonyl)-pipéridin-4-yl]-1,4-dihydro-benzo[d][1,3]oxazin-2-one |
| 176 | 6-méthyle-1-[1-(2-trifluorométhyl-benzènesulfonyl)-pipéridin-4-yl]-1,4-dihydro-benzo[d][1,3]oxazin-2-one |
| 177 | 1-[1-(3-méthoxy-benzènesulfonyl)-pipéridin-4-yl]-6-méthyl-1,4-dihydro-benzo[d][1,3]oxazin-2-one |
| 178 | 6-méthyl-1-[1-(2-nitro-benzènesulfonyl)-pipéridin-4-yl]-1,4-dihydro-benzo[d][1,3]oxazin-2-one |
| 179 | 1-[1-(4-acétyl-benzènesulfonyl)-pipéridin-4-yl]-6-méthyl-1,4-dihydro-benzo[d][1,3]oxazin-2-one |
| 180 | 1-[1-(4-méthanesulfonyl-benzènesulfonyl)-pipéridin-4-yl]-6-méthyl-1,4-dihydro-benzo[d][1,3]oxazin-2-one |
| 181 | 6-méthyl-1-(1-phénylméthanesulfonyl-pipéridin-4-yl]-1,4-dihydro-benzo[d][1,3]oxazin-2-one |
| 182 | Ester méthylique de l'acide 2-[4-(6-méthyl-2-oxo-4-H-benzo[d][1,3]oxazin-1-yl)-pipéridin-1-sulfonyl]benzoïque |
| 183 | 6-méthyl-1-[1-(2-oxo-2-H-chromène-6-sulfonyl)-pipéridin-4-yl]-1,4-dihydro-benzo[d][1,3]oxazin-2-one |
| 184 | 6-chloro-1-[1-(4-fluoro-benzènesulfonyl)-pipéridin-4-yl]-1,4-dihydro-benzo[d][1,3]oxazin-2-one |
| 185 | 6-chloro-1-[1-(3,5-dichloro-benzènesulfonyl)-pipéridin-4-yl]-1,4-dihydro-benzo[d][1,3]oxazin-2-one |
| 186 | 1-(1-[4-(4-bromo-phénoxy)-benzènesulfonyl)-pipéridin-4-yl]-6-chloro-1,4-dihydro-benzo[d][1,3]oxazin-2-one |
| 187 | 6-chloro-1-[1-(thiophène-2-sulfonyl)-pipéridin-4-yl]-1,4-dihydro-benzo[d][1,3]oxazin-2-one |
| 188 | 6-chloro-1-[1-(3-méthoxy-benzènesulfonyl)-pipéridin-4-yl]-1,4-dihydro-benzo[d][1,3]oxazin-2-one |
| 189 | 6-chloro-1-[1-(2-oxo-2H-chromène-6-sulfonyl)-pipéridin-4-yl]-1,4-dihydro-benzo[d][1,3]oxazin-2-one |
| 190 | 6-chloro-1-[1-(toluène-3-sulfonyl)-pipéridin-4-yl]-1,4-dihydro-benzo[d][1,3]oxazin-2-one |
| 191 | 6-chloro-1-[1-(5-fluoro-2-méthyl-benzènesulfonyl)-pipéridin-4-yl]-1,4-dihydro-benzo [d][1,3]oxazin-2-one |
| 192 | 6-chlore-1-[1-(4-isopropoxy-benzènesulfonyl)-pipéridin-4-yl]-1,4-dihydro-benzo[d][1,3]oxazin-2-one |
| 193 | 6-chlore-1-[1-(3-chloro-benzènesulfonyl)-pipéridin-4-yl]-1,4-dihydro-benzo[d][1,3]oxazin-2-one |
| 194 | 6-chlore-1-[1-(3,4-diméthoxy-benzènesulfonyl)-pipéridin-4-yl]-1,4-dihydro-benzo[d][1,3]oxazin-2-one |
| 195 | 6-chloro-1-(1-pentafluorobenzènesulfonyl-pipéridin-4-yl)-1,4-dihydro-benzo[d][1,3]oxazin-2-one |
| 196 | 6-chlore-1-[1-(4-trifluorométhoxy-benzènesulfonyl)-pipéridin-4-yl]-1,4-dihydro-benzo[d][1,3]oxazin-2-one |
| 197 | 6-chloro-1-[1-(2-nitro-4-trifluorométhyl-benzènesulfonyl)-pipéridin-4-yl]-1,4-dihydro-benzo[d][1,3]oxazin-2-one |
| 198 | 6-chlore-1-[1-(3-fluoro-benzènesulfonyl)-pipéridin-4-yl]-1,4-dihydro-benzo[d][1,3]oxazin-2-one |
| 199 | 6-chlore-1-[1-(2,4-dichloro-benzènesulfonyl)-pipéridin-4-yl]-1,4-dihydro-benzo[d][1,3]oxazin-2-one |
| 200 | 6-chlore-1-[1-(2,4,6-triméthyl-benzènesulfonyl)-pipéridin-4-yl]-1,4-dihydro-benzo[d][1,3]oxazin-2-one |
| 201 | 6-chloro-1-[1-(2-trifluorométhyl-benzènesulfonyl)-pipéridin-4-yl]-1,4-dihydro-benzo[d][1,3]oxazin-2-one |
| 202 | 1-[1-(2-oxo-2H-chromène-6-sulfonyl)-pipéridin-4-yl]-1,4-dihydro-benzo[d][1,3]oxazin-2-one |
| 203 | 1-[1-(3,5-dichloro-benzènesulfonyl)-pipéridin-4-yl]-1,4-dihydro-benzo[d][1,3]oxazin-2-one |
| 204 | 1-[1-(2,5-dichloro-benzènesulfonyl)-pipéridin-4-yl]-1,4-dihydro-benzo[d][1,3]oxazin-2-one |
| 205 | 1-[1-(5-bromo-6-chloro-pyridine-3-sulfonyl)-pipéridin-4-yl]-1,4-dihydro-benzo[d][1,3]oxazin-2-one |
| 206 | 1-[1-(4-chloro-benzènesulfonyl)-pipéridin-4-yl]-1,4-dihydro-benzo[d][1,3]oxazin-2-one |
| 207 | 1-[1-(2, 6-dichloro-benzènesulfonyl)-pipéridin-4-yl]-1,4-dihydro-benzo[d] [1,3]oxazin-2-one |
| 208 | 8-méthyle-1-[1-(2-oxo-2H-chromène-6-sulfonyl)-pipéridin-4-yl]-1,4-dihydro-benzo[d][1,3]oxazin-2-one |
| 209 | 1-[1-(3,5-dichloro-benzènesulfonyl)-pipéridin-4-yl]-8-méthyl-1,4-dihydro-benzo[d][1,3]oxazin-2-one |
| 210 | 1-[1-(2,5-dichloro-benzènesulfonyl)-pipéridin-4-yl]-8-méthyl-1,4-dihydro-benzo[d][1,3]oxazin-2-one |
| 211 | 1-[1-(5-bromo-6-chloro-pyridine-3-sulfonyl)-pipéridin-4-yl]-8-méthyl-1,4-dihydro-benzo[d] [1,3]oxazin-2-one |
| 212 | 1-[1-(4-chloro-benzènesulfonyl)-pipéridin-4-yl]-8-méthyl-1,4-dihydro-benzo[d][1,3]oxazin-2-one |
| 213 | 1-[1-(2,6-dichloro-benzènesulfonyl)-pipéridin-4-yl]-8-méthyl-1,4-dihydro-benzo[d][1,3]oxazin-2-one |
| 214 | 1-[1-(biphényl-4-sulfonyl)-pipéridin-4-yl]-1,4-dihydro-benzo[d][1,3]oxazin-2-one |
| 215 | 6-chlore-1-[1-(2,5-dichloro-benzènesulfonyl)-pipéridin-4-yl]-1,4-dihydro-benzo[d][1,3]oxazin-2-one |
| 216 | 1-[1-(5-bromo-6-chloro-pyridine-3-sulfonyl)-pipéridin-4-yl]-6-chloro-1,4-dihydro-benzo[d][1,3]oxazin-2-one |
| 217 | 6-chlore-1-[1-(4-chloro-benzènesulfonyl)-pipéridin-4-yl]-1,4-dihydro-benzo[d][1,3]oxazin-2-one |
| 218 | 6-chlore-1-[1-(2,6-dichloro-benzènesulfonyl)-pipéridin-4-yl]-1,4-dihydro-benzo[d][1,3]oxazin-2-one |
| 219 | 1-[1-(biphényl-4-sulfonyl)-pipéridin-4-yl]-6-méthyl-1,4-dihydro-benzo[d][1,3]oxazin-2-one |
| 220 | 2-[4-(6-méthyl-2-oxo-4H-benzo[d][1,3]oxazin-1-yl)-pipéridin-1-sulfonyl)-benzonitrile |
| 221 | 1-[1-(2,5-dichloro-benzènesulfonyl)-pipéridin-4-yl]-6-méthyl-1,4-dihydro-benzo[d][1,3]oxazin-2-one |
| 222 | 1-[1-(5-bromo-6-chloro-pyridine-3-sulfonyl)-pipéridin-4-yl]-6-méthyl-1,4-dihydro-benzo[d][1,3]oxazin-2-one |
| 223 | 1-[1-(4-chloro-benzènesulfonyl)-pipéridin-4-yl]-6-méthyl-1,4-dihydro-benzo[d][1,3]oxazin-2-one |
| 224 | 1-[1-(2,6-dichloro-benzènesulfonyl)-pipéridin-4-yl]-6-méthyl-1,4-dihydro-benzo[d][1,3]oxazin-2-one |
| 225 | 1-[1-(3,5-dichloro-benzènesulfonyl)-pipéridin-4-yl]-6-méthyl-1,4-dihydro-benzo[d][1,3]oxazin-2-one |
| 226 | 6-méthyle-1-[1-(1-méthyl-1H-imidazole-4-sulfonyl)-pipéridin-4-yl]-1,4-dihydro-benzo[d][1,3]oxazin-2-one |
| 227 | 1-[1-(5-bromo-2,4-difluoro-benzènesulfonyl)-pipéridin-4-yl]-6-méthyl-1,4-dihydro-benzo[d][1,3]oxazin-2-one |
| 228 | 1-[1-(4-méthanesulfonyl-benzènesulfonyl)-pipéridin-4-yl]-1,4-dihydro-benzo[d][1,3]oxazin-2-one |
| 229 | 1-[1-(1-méthyl-1H-imidazole-4-sulfonyl)-pipéridin-4-yl]-1,4-dihydro-benzo[d][1,3]oxazin-2-one |
| 230 | 1-[1-(5-bromo-2,4-difluoro-benzènesulfonyl)-pipéridin-4-yl]-1,4-dihydro-benzo[d][1,3]oxazin-2-one |
| 231 | 1-[1-(6-chloro-imidazo[2,1-b]thiazole-5-sulfonyl)-pipéridin-4-yl]-1,4-dihydro-benzo[d][1,3]oxazin-2-one |
| 232 | 1-[1-(4-éthyl-benzènesulfonyl)-pipéridin-4-yl]-1,4-dihydro-benzo[d][1,3]oxazin-2-one |
| 233 | 1-[1-(benzo[b]thiophène-3-sulfonyl)-pipéridin-4-yl]-1,4-dihydro-benzo[d][1,3]oxazin-2-one |
| 234 | 1-[1-6-chloro-imidazo[2,1-b]thiazole-5-sulfonyl)pipéridin-4-yl]-8-méthyl-1,4-dihydro-benzo[d][1,3]oxazin-2-one |
| 235 | 1-[1-(4-éthyl-benzènesulfonyl)-pipéridin-4-yl]-8-méthyl-1,4-dihydro-benzo[d][1,3]oxazin-2-one |
| 236 | 1-[1-(benzo[b]thiophène-3-sulfonyl)-pipéridin-4-yl]-8-méthyl-1,4-dihydro-benzo[d][1,3]oxazin-2-one |
| 237 | 6-chloro-1-[1-(6-chloro-imidazo[2,1-b]thiazole-5-sulfonyl)-pipéridin-4-yl]-1,4-dihydro-benzo[d][1,3]oxazin-2-one |
| 238 | 6-chloro-1-[1-(4-éthyl-benzènesulfonyl)-pipéridin-4-yl]-1,4-dihydro-benzo[d][1,3]oxazin-2-one |
| 239 | 1-[1-(benzo[b]thiophène-3-sulfonyl)-pipéridin-4-yl]-6-chloro-1,4-dihydro-benzo[d][1,3]oxazin-2-one |
| 240 | 1-[1-(6-chloro-imidazo[2.1-b]thiazole-5-sulfonyl)-pipéridin-4-yl]-6-méthyl-1,4-dihydro-benzo[d][1,3]oxazin-2-one |
| 241 | 1-[1-(4-éthyl-benzènesulfonyl)-pipéridin-4-yl]-6-méthyl-1,4-dihydro-benzo[d][1,3]oxazin-2-one |
| 242 | 1-[1-(benzo[b]thiophène-3-sulfonyl)-pipéridin-4-yl]-6-méthyl-1,4-dihydro-benzo[d][1,3]oxazin-2-one |
| 243 | 1-[1-(7-chloro-benzo[1,2,5]oxadiazole-4-sulfonyl)-pipéridin-4-yl]-1,4-dihydro-benzo[d][1,3]oxazin-2-one |
| 244 | 1-[1-(2-méthoxy-4-méthyl-benzènesulfonyl)-pipéridin-4-yl]-1,4-dihydro-benzo[d][1,3]oxazin-2-one |
| 245 | Ester méthylique de l'acide 3-{4-[4-(2-oxo-4H-benzo[d][1,3]oxazin-1-yl)-pipéridin-1-sulfonyl]-phényl}-propionique |
| 246 | 1-[1-(2,4-dinitro-benzènesulfonyl)-pipéridin-4-yl]-1,4-dihydro-benzo[d][1,3]oxazin-2-one |
| 247 | 1-[1-(7-chloro-benzo[1,2,5]oxadiazole-4-sulfonyl)-pipéridin-4-yl]-8-méthyl-1,4-dihydro-benzo[d][1,3]oxazin-2-one |
| 248 | 1-[1-(2-méthoxy-4-méthyl-benzènesulfonyl)-pipéridin-4-yl]-8-méthyl-1,4-dihydro-benzo[d][1,3]oxazin-2-one |
| 249 | Ester méthylique de l'acide 3-{4-[4-(8-méthyl-2-oxo-4H-benzo[d][1,3]oxazin-1-yl)-pipéridin-1-sulfonyl]-phényl}-propionique |
| 250 | 1-[1-(2,4-dinitro-benzènesulfonyl)-pipéridin-4-yl]-8-méthyl-1,4-dihydro-benzo[d][1,3]oxazin-2-one |
| 251 | 1-[1-(7-chloro-benzo[1,2,5]oxadiazole-4-sulfonyl)-pipéridin-4-yl]-6-méthyl-1,4-dihydro-benzo[d][1,3]oxazin-2-one |
| 252 | 1-[1-(2-méthoxy-4-méthyl-benzènesulfonyl)-pipéridin-4-yl]-6-méthyl-1,4-dihydro-benzo[d][1,3]oxazin-2-one |
| 253 | Ester méthylique de l'acide 3-{4-[4-(6-méthyl-2-oxo-4H-benzo[d][1,3]oxazin-1-yl)-pipéridin-1-sulfonyl]-phényl}-propionique |
| 254 | 1-[1-(2,4-dinitro-benzènesulfonyl)-pipéridin-4-yl]-6-méthyl-1,4-dihydro-benzo[d][1,3]oxazin-2-one |
| 255 | 6-chloro-1-[1-(7-chloro-benzo[1,2,5]oxadiazole-4-sulfonyl)-pipéridin-4-yl]-1,4-dihydro-benzo[d][1,3]oxazin-2-one |
| 256 | 6-chloro-1-[1-(2-méthoxy-4-méthyl-benzènesulfonyl)-pipéridin-4-yl]-1,4-dihydro-benzo[d][1,3]oxazin-2-one |
| 257 | Ester méthylique de l'acide 3-(4-[4-(6-chloro-2-oxo-4H-benzo[d][1,3]oxazin-1-yl)-pipéridin-1-sulfonyl]-phényl)-propionique |
| 258 | 6-chloro-1-[1-(2,4-dinitro-benzènesulfonyl)-pipéridin-4-yl]-1,4-dihydro-benzo[d][1,3]oxazin-2-one |
| 259 | 6-chloro-1-[1-(1-méthyl-1H-imidazole-4-sulfonyl)-pipéridin-4-yl]-1,4-dihydro-benzo[d][1,3]oxazin-2-one |
| 260 | 1-[1-(5-bromo-2,4-difluoro-benzènesulfonyl)-pipéridin-4-yl]-6-chloro-1,4-dihydro-benzo[d][1,3]oxazin-2-one |
| 261 | 8-méthyl-1-[-(1-méthyl-1H-imidazole-4-sulfonyl)-pipéridin-4-yl]-1,4-dihydro-benzo[d][1,3]oxazin-2-one |
| 262 | 1-[1-(5-bromo-2,4-difluoro-benzènesulfonyl)-pipéridin-4-yl]-8-méthyl-1,4-dihydro-benzo[d][1,3]oxazin-2-one |
| 263 | 1-[1-(benzo[b]thiophène-2-sulfonyl)-pipéridin-4-yl]-8-méthyl-1,4-dihydro-benzo[d][1,3]oxazin-2-one |
| 264 | 1-[1-(benzo[b]thiophène-2-sulfonyl)-pipéridin-4-yl]-1,4-dihydro-benzo[d][1,3]oxazin-2-one |
| 265 | 1-[1-(benzo[b]thiophène-2-sulfonyl)-pipéridin-4-yl]-6-chloro-1,4-dihydro-benzo[d][1,3]oxazin-2-one |
| 266 | 1-[1-(benzo[b]thiophène-2-sulfonyl)-pipéridin-4-yl]-6-méthyl-1,4-dihydro-benzo[d][1,3]oxazin-2-one |
| 267 | 1-[1-(2,5-difluoro-benzènesulfonyl)-pipéridin-4-yl]-1,4-dihydro-benzo[d][1,3]oxazin-2-one |
| 268 | 1-[1-(2,5-difluoro-benzènesulfonyl)-pipéridin-4-yl]-8-méthyl-1,4-dihydro-benzo[d][1,3]oxazin-2-one |
| 269 | 6-chlore-1-[1-(2,5-difluoro-benzènesulfonyl)-pipéridin-4-yl]-1,4-dihydro-benzo[d][1,3]oxazin-2-one |
| 270 | 1-[1-(2,5-difluoro-benzènesulfonyl)-pipéridin-4-yl]-6-méthyl-1,4-dihydro-benzo[d][1,3]oxazin-2-one |
| 271 | 1-[1-(4-chloro-2,5-difluoro-benzènesulfonyl)-pipéridin-4-yl]-1,4-dihydro-benzo[d][1,3]oxazin-2-one |
| 272 | 1-[1-(4-chloro-2,5-difluoro-benzènesulfonyl)-pipéridin-4-yl]-8-méthyl-1,4-dihydro-benzo[d][1,3]oxazin-2-one |
| 273 | 6-chlore-1-[1-(4-chloro-2,5-difluoro-benzènesulfonyl)-pipéridin-4-yl]-1,4-dihydro-benzo[d][1,3]oxazin-2-one |
| 274 | 1-[1-(4-chloro-2,5-difluoro-benzènesulfonyl)-pipéridin-4-yl]-6-méthyl-1,4-dihydro-benzo[d][1,3]oxazin-2-one |
| 275 | 1-[1-(2,4,5-trifluoro-benzènesulfonyl)-pipéridin-4-yl]-1,4-dihydro-benzo[d][1,3]oxazin-2-one |
| 276 | 8-méthyle-1-[1-(2,4,5-trifluoro-benzènesulfonyl)-pipéridin-4-yl]-1,4-dihydro-benzo[d][1,3]oxazin-2-one |
| 277 | 6-chlore-1-[1-(2,4,5-trifluoro-benzènesulfonyl)-pipéridin-4-yl]-1,4-dihydro-benzo[d] [1,3]oxazin-2-one |
| 278 | 6-méthyl-1-[1-(2,4,5-trifluoro-benzènesulfonyl)-pipéridin-4-yl]-1,4-dihydro-benzo[d][1,3]oxazin-2-one |
| 279 | 1-[1-(3,5-dichloro-2-hydroxy-benzènesulfonyl)-pipéridin-4-yl]-1,4-dihydro-benzo[d][1,3]oxazin-2-one |
| 280 | 1-[1-(2,6-difluoro-benzènesulfonyl)-pipéridin-4-yl]-1,4-dihydro-benzo[d][1,3]oxazin-2-one |
| 281 | 1-[1-(2,6-difluoro-benzènesulfonyl)-pipéridin-4-yl]-8-méthyl-1,4-dihydro-benzo[d][1,3]oxazin-2-one |
| 282 | 6-chlore-1-[1-(2,6-difluoro-benzènesulfonyl)-pipéridin-4-yl]-1,4-dihydro-benzo[d][1,3]oxazin-2-one |
| 283 | 1-[1-(2,6-difluoro-benzènesulfonyl)-pipéridin-4-yl]-6-méthyl-1,4-dihydro-benzo[d][1,3]oxazin-2-one |
| 284 | 1-[1-(5-chloro-2,4-difluoro-benzènesulfonyl)-pipéridin-4-yl]-1,4-dihydro-benzo[d][1,3]oxazin-2-one |
| 285 | 1-[1-(5-chloro-2,4-difluoro-benzènesulfonyl)-pipéridin-4-yl]-8-méthyl-1,4-dihydro-benzo[d][1,3]oxazin-2-one |
| 286 | 6-chlore-1-[1-(5-chloro-2,4-difluoro-benzènesulfonyl)-pipéridin-4-yl]-1,4-dihydro-benzo[d][1,3]oxazin-2-one |
| 287 | 1-[1-(5-chloro-2,4-difluoro-benzènesulfonyl)-pipéridin-4-yl]-6-méthyl-1,4-dihydro-benzo[d][1,3]oxazin-2-one |
| 288 | 1-[1-(2-chloro-benzènesulfonyl)-pipéridin-4-yl]-1,4-dihydro-benzo [d][1,3]oxazin-2-one |
| 289 | 1-[1-(2-chloro-benzènesulfonyl)-pipéridin-4-yl]-8-méthyl-1,4-dihydro-benzo [d][1,3]oxazin-2-one |
| 290 | 6-chlore-1-[1-(2-chloro-benzènesulfonyl)-pipéridin-4-yl]-1,4-dihydro-benzo[d][1,3]oxazin-2-one |
| 291 | 1-[1-(2-chloro-benzènesulfonyl)-pipéridin-4-yl]-6-méthyl-1,4-dihydro-benzo [d][1,3]oxazin-2-one |
| 292 | 6-chloro-1-[1-(2-naphtalén-1-yl-éthanesulfonyl)-pipéridin-4-yl]-1,4-dihydro-benzo[d][1,3]oxazin-2-one |
| 293 | 6-brome-1[1-(4-bromo-benzènesulfonyl)-pipéridin-4-yl]-1,4-dihydro-benzo [d][1,3]oxazin-2-one |
| 294 | 6-bromo-1-[1-(toluène-4-sulfonyl))-pipéridin-4-yl]-1,4-dihydro-benzo [d][1,3]oxazin-2-one |
| 295 | 6-bromo-1-[1-(2,4-diméthyl-benzènesulfonyl)-pipéridin-4-yl]-1,4-dihydro-benzo[d][1,3]oxazin-2-one |
| 296 | 6-bromo-1-[1-(2-naphtalén-1-yl-éthanesulfonyl)-pipéridin-4-yl]-1,4-dihydro-benzo[d][1,3]oxazin-2-one |
| 297 | 6-bromo-1-[1-(quinoléine-8-sulfonyl))-pipéridin-4-yl]-1,4-dihydro-benzo [d][1,3]oxazin-2-one |
| 298 | 6-bromo-1-[1-(5-chloro-3-méthyl-benzo[b]thiophène-2-sulfonyl)-pipéridin-4-yl]-1,4-dihydro-benzo[d][1,3]oxazin-2-one |
| 299 | 6-brome-1[1-(3-nitro-benzènesulfonyl)-pipéridin-4-yl]-1,4-dihydro-benzo [d][1,3]oxazin-2-one |
| 300 | 6-bromo-1-[1-(naphtalén-1-sulfonyl)-pipéridin-4-yl]-1,4-dihydro-benzo [d][1,3]oxazin-2-one |
| 301 | 6-bromo-1-[1-(naphtalén-2-sulfonyl))-pipéridin-4-yl]-1,4-dihydro-benzo [d][1,3]oxazin-2-one |
| 302 | 1-(1-benzènesulfonyl)-pipéridin-4-yl)-6-bromo-1,4-dihydro-benzo [d][1,3]oxazin-2-one |
| 303 | 6-bromo-1-{1-[4-(4-bromo-phénoxy)-benzènesulfonyl]-pipéridin-4-yl}-1,4-dihydro-benzo[d][1,3]oxazin-2-one |
| 304 | 6-bromo-1[1-(thiophène-2-sulfonyl)-pipéridin-4-yl]-1,4-dihydro-benzo[d][1,3]oxazin-2-one |
| 305 | 6-bromo-1-[1-(2-méthyl-5-nitro-benzènesulfonyl)-pipéridin-4-yl]-1,4-dihydro-benzo[d][1,3]oxazin-2-one |
| 306 | 6-bromo-1-[1-(4-bromo-2,5-difluoro-benzènesulfonyl]-pipéridin-4-yl]-1,4-dihydro-benzo[d][1,3]oxazin-2-one |
| 307 | 6-brome-1-[1-(toluène-3-sulfonyl)-pipéridin-4-yl]-1,4-dihydro-benzo [d][1,3]oxazin-2-one |
| 308 | 6-bromo-1-[1-(5-fluoro-2-méthyl-benzènesulfonyl)-pipéridin-4-yl]-1,4-dihydro-benzo[d][1,3]oxazin-2-one |
| 309 | 6-brome-1-[1-(4-isopropoxy-benzènesulfonyl)-pipéridin-4-yl]-1,4-dihydro-benzo[d][1,3]oxazin-2-one |
| 310 | 6-bromo-1-[1-(3-chloro-benzènesulfonyl)-pipéridin-4-yl]-1,4-dihydro-benzo[d] [1,3]oxazin-2-one |
| 311 | 6-bromo-1-[1-(3,4-diméthoxy-benzènesulfonyl)-pipéridin-4-yl]-1,4-dihydro-benzo[d][1,3]oxazin-2-one |
| 312 | 6-bromo-1-(1-pentafluorobenzènesulfonyl-pipéridin-4-yl]-1,4-dihydro-benzo[d][1,3]oxazin-2-one |
| 313 | 6-brome-1-[1-(4-chloro-2,5-diméthyl-benzènesulfonyl)-pipéridin-4-yl]-1,4-dihydro-benzo[d][1,3]oxazin-2-one |
| 314 | 6-brome-1-[1-(3-méthoxy-benzènesulfonyl)-pipéridin-4-yl]-1,4-dihydro-benzo[d][1,3]oxazin-2-one |
| 315 | 6-brome-1-[1-(4-isopropyl-benzènesulfonyl)-pipéridin-4-yl]-1,4-dihydro-benzo[d] [1,3]oxazin-2-one |
| 316 | 6-brome-1-[1-(4-fluoro-benzènesulfonyl)-pipéridin-4-yl]-1,4-dihydro-benzo[d][1,3]oxazin-2-one |
| 317 | 6-bromo-1-[1-(3-chloro-4-fluoro-benzènesulfonyl)-pipéridin-4-yl]-1,4-dihydro-benzo[d][1,3]oxazin-2-one |
| 318 | 6-bromo-1-(1-pentaméthylbenzènesulfonyl)-pipéridin-4-yl]-1,4-dihydro-benzo[d][1,3]oxazin-2-one |
| 319 | 6-bromo-1-[1-(2-nitro-benzènesulfonyl)-pipéridin-4-yl]-1,4-dihydro-benzo[d][1,3]oxazin-2-one |
| 320 | 6-bromo-1-[1-(4-chloro-3-nitro-benzènesulfonyl)-pipéridin-4-yl]-1,4-dihydro-benzo[d][1,3]oxazin-2-one |
| 321 | 6-bromo-1-[1-(5-diméthylamino-naphtalène-1-sulfonyl)-pipéridin-4-yl]-1,4-dihydro-benzo[d][1,3]oxazin-2-one |
| 322 | 6-bromo-1-[1-(4-nitro-benzènesulfonyl)-pipéridin-4-yl]-1,4-dihydro-benzo[d][1,3]oxazin-2-one |
| 323 | 1-[1-(4-acétyl-benzènesulfonyl)-pipéridin-4-yl]-6-bromo-1,4-dihydro-benzo[d][1,3]oxazin-2-one |
| 324 | 6-bromo-1-[1-(4-méthanesulfonyl-benzènesulfonyl)-pipéridin-4-yl]-1,4-dihydro-benzo[d] [1,3]oxazin-2-one |
| 325 | 1-[1-(biphényl-4-sulfonyl)-pipéridin-4-yl]-6-bromo-1,4-dihydro-benzo[d][1,3]oxazin-2-one |
| 326 | 6-bromo-1-(1-phénylméthanesulfonyl-pipéridin-4-yl)-1,4-dihydro-benzo[d][1,3]oxazin-2-one |
| 327 | 6-bromo-1-[1-(2,5-diméthoxy-benzènesulfonyl)-pipéridin-4-yl]-1,4-dihydro-benzo[d][1,3]oxazin-2-one |
| 328 | 6-bromo-1-{1-[2-(2,2,2-trifluoro-acétyl)-1,2,3,4-tétrahydro-isoquinoléine-7-sulfonyl]-pipéridin-4-yl}-1,4-dihydro-benzo[d][1,3]oxazin-2-one |
| 329 | 6-brome-1-[1-(2,3-dichloro-benzènesulfonyl)-pipéridin-4-yl]-1,4-dihydro-benzo[d][1,3]oxazin-2-one |
| 330 | 6-bromo-1-[1-(2,4,5-trichloro-benzènesulfonyl)-pipéridin-4-yl]-1,4-dihydro-benzo[d][1,3]oxazin-2-one |
| 331 | 6-bromo-1-[1-(5-bromo-2-méthoxy-benzènesulfonyl)-pipéridin-4-yl]-1,4-dihydro-benzo[d][1,3]oxazin-2-one |
| 332 | 6-bromo-1-[1-(4-trifluorométhoxy-benzènesulfonyl)-pipéridin-4-yl]-1,4-dihydro-benzo [d][1,3]oxazin-2-one |
| 333 | 6-bromo-1-[1-(2-nitro-4-trifluorométhyl-benzènesulfonyl)-pipéridin-4-yl]-1,4-dihydro-benzo[d][1,3]oxazin-2-one |
| 334 | 6-bromo-1-[1-(3-fluoro- benzènesulfonyl)-pipéridin-4-yl]-1,4-dihydro-benzo[d][1,3]oxazin-2-one |
| 335 | 6-bromo-1-[1-(2,4-dichloro-benzènesulfonyl)-pipéridin-4-yl]-1,4-dihydro-benzo[d][1,3]oxazin-2-one |
| 336 | 6-bromo-1-[1-(2,4,6-triméthyl-benzènesulfonyl)-pipéridin-4-yl]-1,4-dihydro-benzo[d][1,3]oxazin-2-one |
| 337 | 6-bromo-1-[1-(2-trifluorométhyl-benzènesulfonyl)-pipéridin-4-yl]-1,4-dihydro-benzo[d][1,3]oxazin-2-one |
| 338 | 6-bromo-1-[1-(2-bromo-benzènesulfonyl)-pipéridin-4-yl]-1,4-dihydro-benzo[d][1,3]oxazin-2-one |
| 339 | 6-bromo-1-[1-(4-méthoxy-2,3,6-triméthyl-benzènesulfonyl)-pipéridin-4-yl]-1,4-dihydro-benzo[d][1,3]oxazin-2-one |
| 340 | 1-[1-(3,5-dichloro-4-hydroxy-benzènesulfonyl)-pipéridin-4-yl]-1,4-dihydro-benzo[d][1,3]oxazin-2-one |
| 341 | 1-[1-(3,5-dichloro-4-hydroxy-benzènesulfonyl)-pipéridin-4-yl]-8-méthyl-1,4-dihydro-benzo[d][1,3]oxazin-2-one |
| 342 | 6-chloro-1-[1-(3,5-dichloro-4-hydroxy-benzènesulfonyl)-pipéridin-4-yl]-1,4-dihydro-benzo[d][1,3]oxazin-2-one |
| 343 | 1-[1-(3,5-dichloro-4-hydroxy-benzènesulfonyl)-pipéridin-4-yl]-6-méthyl-1,4-dihydro-benzo[d][1,3]oxazin-2-one |
| 344 | 6-bromo-1-[1-(3,5-dichloro-4-hydroxy-benzènesulfonyl)-pipéridin-4-yl]-1,4-dihydro-benzo[d][1,3]oxazin-2-one |
| 345 | 6-chloro-1-[1-(3,5-dichloro-2-hydroxy-benzènesulfonyl)-pipéridin-4-yl]-1,4-dihydro-benzo[d][1,3]oxazin-2-one |
| 346 | 6-bromo-1-[1-(3,5-dichloro-2-hydroxy-benzènesulfonyl)-pipéridin-4-yl]-1,4-dihydro-benzo[d][1,3]oxazin-2-one |
| 347 | 2-[4-(6-bromo-2-oxo-4H-benzo[d][1,3]oxazin-1-yl)-pipéridine-1-sulfonyl]-benzonotrile |
| 348 | 6-bromo-1-[1-(4-méthoxy-benzènesulfonyl)-pipéridin-4-yl]-1,4-dihydro-benzo[d][1,3]oxazin-2-one |
| 349 | Ester méthylique d'acide 2-[4-(6-bromo-2-oxo-4H-benzo[d][1,3]oxazin-1-yl)-pipéridine-1-sulfonyl]-benzoïque |
| 350 | 6-bromo-1-[1-(3-trifluorométhyl-benzènesulfonyl)-pipéridin-4-yl]-1,4-dihydro-benzo[d][1,3]oxazin-2-one |
| 351 | 6-bromo-1-[1-(2-oxo-2H-chromène-6-sulfonyl))-pipéridin-4-yl]-1,4-dihydro-benzo[d][1,3]oxazin-2-one |
| 352 | 6-bromo-1-[1-(3,5-dichloro-benzènesulfonyl)-pipéridin-4-yl]-1,4-dihydro-benzo[d][1,3]oxazin-2-one |
| 353 | 6-bromo-1-[1-(2,5-dichloro-benzènesulfonyl)-pipéridin-4-yl]-1,4-dihydro-benzo[d][1,3]oxazin-2-one |
| 354 | 6-bromo-1-[1-(5-bromo-6-chloro-pyridine-3-sulfonyl)-pipéridin-4-yl]-1,4-dihydro-benzo[d][1,3]oxazin-2-one |
| 355 | 6-bromo-1-[1-(4-chloro-benzènesulfonyl)-pipéridin-4-yl]-1,4-dihydro-benzo[d][1,3]oxazin-2-one |
| 356 | 6-bromo-1-[1-(2,6-dichloro-benzènesulfonyl)-pipéridin-4-yl]-1,4-dihydro-benzo[d] [1,3]oxazin-2-one |
| 357 | 6-bromo-1-[1-(1-méthyl-1H-imidazole-4-sulfonyl))-pipéridin-4-yl]-1,4-dihydro-benzo[d][1,3]oxazin-2-one |
| 358 | 6-bromo-1-[1-(5-bromo-2,4-difluoro-benzènesulfonyl)-pipéridin-4-yl]-1,4-dihydro-benzo[d][1,3]oxazin-2-one |
| 359 | 6-bromo-1-[1-(4-éthyl-benzènesulfonyl)-pipéridin-4-yl]-1,4-dihydro-benzo[d][1,3]oxazin-2-one |
| 360 | 6-bromo-1-[1-(6-chloro-imidazo[2,1-b]thiazole-5-sulfonyl))-pipéridin-4-yl]-1,4-dihydro-benzo[d][1,3]oxazin-2-one |
| 361 | 1-[1-(benzo[b]thiophène-3-sulfonyl)-pipéridin-4-yl]-6-bromo-1,4-dihydro-benzo[d][1,3]oxazin-2-one |
| 362 | 6-bromo-1-[1-(7-chloro-benzo[1,2,5]oxadiazole-4-sulfonyl))-pipéridin-4-yl]-1,4-dihydro-benzo[d][1,3]oxazin-2-one |
| 363 | 6-bromo-1-[1-(2-méthoxy-4-méthyl-benzènesulfonyl)-pipéridin-4-yl]-1,4-dihydro-benzo[d][1,3]oxazin-2-one |
| 364 | Ester méthylique de l'acide 3-(4-[4-(6-bromo-2-oxo-4H-benzo[d][1,3]oxazin-1-yl)-pipéridin-1-sulfonyl]-phényl)-propionique |
| 365 | 6-bromo-1-[1-(2,4-dinitro-benzènesulfonyl)-pipéridin-4-yl]-1,4-dihydro-benzo[d][1,3]oxazin-2-one |
| 366 | 1-(1-benzo[b]thiophène-2-sulfonyl)-pipéridin-4-yl]-6-bromo-1,4-dihydro-benzo[d][1,3]oxazin-2-one |
| 367 | 6-bromo-1-[1-(2,5-difluoro-benzènesulfonyl)-pipéridin-4-yl]-1,4-dihydro-benzo[d][1,3]oxazin-2-one |
| 368 | 6-bromo-1-[1-(4-chloro-2,5-difluoro-benzènesulfonyl)-pipéridin-4-yl]-1,4-dihydro-benzo[d][1,3]oxazin-2-one |
| 369 | 6-bromo-1-[1-(2,4,5-trifluoro-benzènesulfonyl)-pipéridin-4-yl]-1,4-dihydro-benzo[d][1,3]oxazin-2-one |
| 370 | 6-bromo-1-[1-(2,6-difluoro-benzènesulfonyl)-pipéridin-4-yl]-1,4-dihydro-benzo[d][1,3]oxazin-2-one |
| 371 | 6-bromo-1-[1-(5-chloro-2,4-difluoro-benzènesulfonyl)-pipéridin-4-yl]-1,4-dihydro-benzo[d][1,3]oxazin-2-one |
| 372 | 6-bromo-1-[1-(2-chloro-benzènesulfonyl)-pipéridin-4-yl]-1,4-dihydro-benzo[d][1,3]oxazin-2-one |
| 373 | 6-bromo-1-[1-(2,3,4-trifluoro-benzènesulfonyl)-pipéridin-4-yl]-1,4-dihydro-benzo[d][1,3]oxazin-2-one |
| 374 | N-{4-[4-(6-bromo-2-oxo-4H-benzo[d][1,3]oxazin-1-yl)-pipéridin-1-sulfonyl]-2-chloro-phényl}-acétamide |
| 375 | 1-[1-(2,3,4-trifluoro-benzènesulfonyl)-pipéridin-4-yl]-1,4-dihydro-benzo[d][1,3]oxazin-2-one |
| 376 | 8-méthyl-1-[1-(2,3,4-trifluoro-benzènesulfonyl)-pipéridin-4-yl]-1,4-dihydro-benzo[d][1,3]oxazin-2-one |
| 377 | 6-chloro-1-[1-(2,3,4-trifluoro-benzènesulfonyl)-pipéridin-4-yl]-1,4-dihydro-benzo[d][1,3]oxazin-2-one |
| 378 | 6-méthyl-1-[1-(2,3,4-trifluoro-benzènesulfonyl)-pipéridin-4-yl]-1,4-dihydro-benzo[d][1,3]oxazin-2-one |
| 379 | N-{2-chloro-4-[4-(6-méthyl-2-oxo-4H-benzo[d][1,3]oxazin-1-yl)-pipéridin-1-sulfonyl]-phényl}-acétamide |
| 380 | 1-[1-(3,4-difluoro-benzènesulfonyl)-pipéridin-4-yl]-1,4-dihydro-benzo[d] [1,3]oxazin-2-one |
| 381 | 1-[1-(3,4-difluoro-benzènesulfonyl)-pipéridin-4-yl]-8-méthyl-1,4-dihydro-benzo[d][1,3]oxazin-2-one |
| 382 | 6-chloro-1-[1-(3,4-difluoro-benzènesulfonyl)-pipéridin-4-yl]-1,4-dihydro-benzo[d][1,3]oxazin-2-one |
| 383 | 1-[1-(3,4-difluoro-benzènesulfonyl)-pipéridin-4-yl]]-6-méthyl-1,4-dihydro-benzo[d][1,3]oxazin-2-one |
| 384 | 6-bromo-1-[1-(3,4-difluoro-benzènesulfonyl)-pipéridin-4-yl]-1,4-dihydro-benzo[d][1,3]oxazin-2-one |
| 385 | N-{2-chloro-4-[4-(8-méthyl-2-oxo-4H-benzo[d][1,3]oxazin-1-yl)-pipéridin-1-sulfonyl]-phényl}-acétamide |
| 386 | 1-[1-(2-chloro-4,5-difluoro-benzènesulfonyl)-pipéridin-4-yl]-1,4-dihydro-benzo[d][1,3]oxazin-2-one |
| 387 | 1-[1-(2-chloro-4,5-difluoro-benzènesulfonyl)-pipéridin-4-yl]-8-méthyl-1,4-dihydro-benzo[d][1,3]oxazin-2-one |
| 388 | 6-chloro-1-[1-(2-chloro-4,5-difluoro-benzènesulfonyl)-pipéridin-4-yl]-1,4-dihydro-benzo[d][1,3]oxazin-2-one |
| 389 | 1-[1-(2-chloro-4,5-difluoro-benzènesulfonyl)-pipéridin-4-yl]-6-méthyl-1,4-dihydro-benzo[d][1,3]oxazin-2-one |
| 390 | 6-bromo-1-[1-(2-chloro-4,5-difluoro-benzènesulfonyl)-pipéridin-4-yl]-1,4-dihydro-benzo[d][1,3]oxazin-2-one |
| 391 | N-{2-chloro-4-[4-(2-oxo-4H-benzo[d][1,3]oxazin-1-yl)-pipéridin-1-sulfonyl]-phényl}-acétamide |
| 392 | 1-[1-(benzo[1,2,5]oxadiazole-4-sulfonyl)-pipéridin-4-yl]-1,4-dihydro-benzo[d][1,3]oxazin-2-one |
| 393 | 1-[1-(benzo[1,2,5]oxadiazole-4-sulfonyl)-pipéridin-4-yl]-8-méthyl-1,4-dihydro-benzo[d][1,3]oxazin-2-one |
| 394 | 1-[1-(benzo[1,2,5]oxadiazole-4-sulfonyl)-pipéridin-4-yl]-6-chloro-1,4-dihydro-benzo[d][1,3]oxazin-2-one |
| 395 | 1-[1-(benzo[1,2,5]oxadiazole-4-sulfonyl)-pipéridin-4-yl]-6-méthyl-1,4-dihydro-benzo[d][1,3]oxazin-2-one |
| 396 | 1-[1-(benzo[1,2,5]oxadiazole-4-sulfonyl)-pipéridin-4-yl]-6-bromo-1,4-dihydro-benzo[d][1,3]oxazin-2-one |
| 397 | N-{2-chloro-4-[4-(6-chloro-2-oxo-4H-benzo[d][1,3]oxazin-1-yl)-pipéridin-1-sulfonyl]-phényl}-acétamide |
| 398 | 1-[1-(benzo[1,2,5]thiadiazole-4-sulfonyl)-pipéridin-4-yl]-1,4-dihydro-benzo[d][1,3]oxazin-2-one |
| 399 | 1-[1-(benzo[1,2,5]thiadiazole-4-sulfonyl)-pipéridin-4-yl]-8-méthyl-1,4-dihydro-benzo[d][1,3]oxazin-2-one |
| 400 | 1-[1-(benzo[1,2,5]thiadiazole-4-sulfonyl)-pipéridin-4-yl]-6-chloro-1,4-dihydro-benzo[d][1,3]oxazin-2-one |
| 401 | 1-[1-(benzo[1,2,5]thiadiazole-4-sulfonyl)-pipéridin-4-yl]-6-méthyl-1,4-dihydro-benzo[d][1,3]oxazin-2-one |
| 402 | 1-[1-(benzo[1,2,5]thiadiazole-4-sulfonyl)-pipéridin-4-yl]-6-bromo-1,4-dihydro-benzo[d][1,3]oxazin-2-one |
| 403 | 1-(1-éthanesulfonyl-pipéridin-4-yl)-8-méthyl-1,4-dihydro-benzo[d][1,3]oxazin-2-one |
| 404 | 1-[1-(2,4-difluoro-benzènesulfonyl)-pipéridin-4-yl]-1,4-dihydro-benzo[d][1,3]oxazin-2-one |
| 405 | 1-[1-(2,4-difluoro-benzènesulfonyl)-pipéridin-4-yl]-8-méthyl-1,4-dihydro-benzo[d][1,3]oxazin-2-one |
| 406 | 6-chloro-1-[1-(2,4-difluoro-benzènesulfonyl)-pipéridin-4-yl]-1,4-dihydro-benzo[d][1,3]oxazin-2-one |
| 407 | 1-[1-(2,4-difluoro-benzènesulfonyl)-pipéridin-4-yl]-6-méthyl-1,4-dihydro-benzo[d][1,3]oxazin-2-one |
| 408 | 6-bromo-1-[1-(2,4-difluoro-benzènesulfonyl)-pipéridin-4-yl]-1,4-dihydro-benzo[d][1,3]oxazin-2-one |
| 409 | 8-méthyle-1-[1-propane-2-sulfonyl)-pipéridin-4-yl]-1,4-dihydro-benzo[d][1,3]oxazin-2-one |
| 410 | 1-[1-(3,4-dichloro-benzènesulfonyl)-pipéridin-4-yl]-1,4-dihydro-benzo[d][1,3]oxazin-2-one |
| 411 | 1-[[1-(3,4-dichloro-benzènesulfonyl)-pipéridin-4-yl]-8-méthyl-1,4-dihydro-benzo[d][1,3]oxazin-2-one |
| 412 | 6-chloro-1-[1-(3,4-dichloro-benzènesulfonyl)-pipéridin-4-yl]-1,4-dihydro-benzo[d][1,3]oxazin-2-one |
| 413 | 1-[1-(3,4-dichloro-benzènesulfonyl)-pipéridin-4-yl]-6-méthyl-1,4-dihydro-benzo[d][1,3]oxazin-2-one |
| 414 | 6-bromo-1-[1-(3,4-dichloro-benzènesulfonyl)-pipéridin-4-yl]-1,4-dihydro-benzo[d][1,3]oxazin-2-one |
| 415 | 8-méthyl-1-[1-(propane-1-sulfonyl)-pipéridin-4-yl]-1,4-dihydro-benzo[d][1,3]oxazin-2-one |
| 416 | 1-[1-(2-chloro-6-méthyl-benzènesulfonyl)-pipéridin-4-yl]-1,4-dihydro-benzo[d][1,3]oxazin-2-one |
| 417 | 1-[1-(2-chloro-6-méthyl-benzènesulfonyl)-pipéridin-4-yl]-8-méthyl-1,4-dihydro-benzo[d][1,3]oxazin-2-one |
| 418 | 6-chloro-1-[1-(2-chloro-6-méthyl-benzènesulfonyl)-pipéridin-4-yl]-1,4-dihydro-benzo[d][1,3]oxazin-2-one |
| 419 | 1-[[1-(2-chloro-6-méthyl-benzènesulfonyl)-pipéridin-4-yl]-6-méthyl-1,4-dihydro-benzo[d][1,3]oxazin-2-one |
| 420 | 1-[1-(2-chloro-6-méthyl-benzènesulfonyl)-pipéridin-4-yl]-6-méthyl-1,4-dihydro-benzo[d][1,3]oxazin-2-one |
| 421 | 8-méthyl-1-[1-(2,3,5,6-tétraméthyl-benzènesulfonyl)-pipéridin-4-yl]-1,4-dihydro-benzo[d][1,3]oxazin-2-one |
| 422 | 1-[1-(2,3,4-trichloro-benzènesulfonyl)-pipéridin-4-yl]-1,4-dihydro-benzo[d][1,3]oxazin-2-one |
| 423 | 8-méthyle-1-[1-(2,3,4-trichloro-benzènesulfonyl)-pipéridin-4-yl]-1,4-dihydro-benzo[d][1,3]oxazin-2-one |
| 424 | 6-chloro-1-[1-(2,3,4-trichloro-benzènesulfonyl)-pipéridin-4-yl]-1,4-dihydro-benzo[d][1,3]oxazin-2-one |
| 425 | 6-méthyl-1-[1-(2,3,4-trichloro-benzènesulfonyl)-pipéridin-4-yl]-1,4-dihydro-benzo[d][1,3]oxazin-2-one |
| 426 | 6-bromo-1-[1-(2,3,4-trichloro-benzènesulfonyl)-pipéridin-4-yl]-1,4-dihydro-benzo[d][1,3]oxazin-2-one |
| 427 | 1-[1-(2,3,5,6-tétraméthyl-benzènesulfonyl)-pipéridin-4-yl]-1,4-dihydro-benzo[d][1,3]oxazin-2-one |
| 428 | 1-[1-(thiophène-3-sulfonyl)-pipéridin-4-yl]-1,4-dihydro-benzo[d][1,3]oxazin-2-one |
| 429 | 8-méthyl-1-[1-(thiophène-3-sulfonyl)-pipéridin-4-yl]-1,4-dihydro-benzo[d] [1,3]oxazin-2-one |
| 430 | 6-chloro-1-[1-(thiophène-3-sulfonyl)-pipéridin-4-yl]-1,4-dihydro-benzo[d][1,3]oxazin-2-one |
| 431 | 6-méthyl-1-[1-(thiophène-3-sulfonyl)-pipéridin-4-yl]-1,4-dihydro-benzo[d][1,3]oxazin-2-one |
| 432 | 6-bromo-1-[1-(thiophène-3-sulfonyl)-pipéridin-4-yl]-1,4-dihydro-benzo[d][1,3]oxazin-2-one |
| 433 | 6-chloro-1-[1-(2,3,5,6-tétraméthyl-benzènesulfonyl)-pipéridin-4-yl]-1,4-dihydro-benzo[d][1,3]oxazin-2-one |
| 434 | 1-[1-(2,4,6-trichloro-benzènesulfonyl)-pipéridin-4-yl]-1,4-dihydro-benzo[d][1,3]oxazin-2-one |
| 435 | 8-méthyl-1-[1-(2,4,6-trichloro-benzènesulfonyl)-pipéridin-4-yl]-1,4-dihydro-benzo[d][1,3]oxazin-2-one |
| 436 | 6-chloro-1-[1-(2,4,6-trichloro-benzènesulfonyl)-pipéridin-4-yl]-1,4-dihydro-benzo[d][1,3]oxazin-2-one |
| 437 | 6-méthyl-1-[1-(2,4,6-trichloro-benzènesulfonyl)-pipéridin-4-yl]-1,4-dihydro-benzo[d][1,3]oxazin-2-one |
| 438 | 6-bromo-1-[1-(2,4,6-trichloro-benzènesulfonyl)-pipéridin-4-yl]-1,4-dihydro-benzo[d][1,3]oxazin-2-one |
| 439 | 6-méthyl-1-[1-(2,3,5,6-tétraméthyl-benzènesulfonyl)-pipéridin-4-yl]-1,4-dihydro-benzo[d][1,3]oxazin-2-one |
| 440 | 1-[1-(2-bromo-4,6-difluoro-benzènesulfonyl)-pipéridin-4-yl]-8-méthyl-1,4-dihydro-benzo[d][1,3]oxazin-2-one |
| 441 | 1-[1-(2-bromo-4,6-difluoro-benzènesulfonyl)-pipéridin-4-yl]-6-chloro-1,4-dihydro-benzo[d][1,3]oxazin-2-one |
| 442 | 1-[1-(2-bromo-4,6-difluoro-benzènesulfonyl)-pipéridin-4-yl]-6-méthyl-1,4-dihydro-benzo[d][1,3]oxazin-2-one |
| 443 | 6-bromo-1-[1-(2-bromo-4,6-difluoro-benzènesulfonyl)-pipéridin-4-yl]-1,4-dihydro-benzo[d][1,3]oxazin-2-one |
| 444 | 6-bromo-1-[1-(2,3,5,6-tétraméthyl-benzènesulfonyl)-pipéridin-4-yl]-1,4-dihydro-benzo[d][1,3]oxazin-2-one |
| 445 | 1-[1-(4-bromo-2-trifluorométhoxy-benzènesulfonyl)-pipéridin-4-yl]-1,4-dihydro-benzo[d][1,3]oxazin-2-one |
| 446 | 1-[1-(4-bromo-2-trifluorométhoxy-benzènesulfonyl)-pipéridin-4-yl]-8-méthyl-1,4-dihydro-benzo[d][1,3]oxazin-2-one |
| 447 | 1-[1-(4-bromo-2-trifluorométhoxy-benzènesulfonyl)-pipéridin-4-yl]-6-chloro-1,4-dihydro-benzo[d][1,3]oxazin-2-one |
| 448 | 1-[1-(4-bromo-2-trifluorométhoxy-benzènesulfonyl)-pipéridin-4-yl]-6-méthyl-1,4-dihydro-benzo[d][1,3]oxazin-2-one |
| 449 | 6-bromo-1-[1-(4-bromo-2-trifluorométhoxy-benzènesulfonyl)-pipéridin-4-yl]-1,4-dihydro-benzo[d][1,3]oxazin-2-one |
| 450 | 1-[1-(4-phénoxy-benzènesulfonyl)-pipéridin-4-yl]-1,4-dihydro-benzo[d][1,3]oxazin-2-one |
| 451 | 1-[1-(3-bromo-benzènesulfonyl)-pipéridin-4-yl]-1,4-dihydro-benzo[d][1,3]oxazin-2-one |
| 452 | 1-[1-(3-bromo-benzènesulfonyl)-pipéridin-4-yl]-8-méthyl-1,4-dihydro-benzo[d][1,3]oxazin-2-one |
| 453 | 1-[1-(3-bromo-benzènesulfonyl)-pipéridin-4-yl]-6-chloro-1,4-dihydro-benzo[d][1,3]oxazin-2-one |
| 454 | 1-[1-(3-bromo-benzènesulfonyl)-pipéridin-4-yl]-6-méthyl-1,4-dihydro-benzo[d][1,3]oxazin-2-one |
| 455 | 6-bromo-1-[1-(3-bromo-benzènesulfonyl)-pipéridin-4-yl]-1,4-dihydro-benzo[d][1,3]oxazin-2-one |
| 456 | 8-méthyl-1-[1-(4-phénoxy-benzènesulfonyl)-pipéridin-4-yl]-1,4-dihydro-benzo[d][1,3]oxazin-2-one |
| 457 | 1-[1-(4-tert-butyl-benzènesulfonyl)-pipéridin-4-yl]-1,4-dihydro-benzo[d][1,3]oxazin-2-one |
| 458 | 1-[1-(4-tert-butyl-benzènesulfonyl)-pipéridin-4-yl]-8-méthyl-1,4-dihydro-benzo[d][1,3]oxazin-2-one |
| 459 | 1-[1-(4-tert-butyl-benzènesulfonyl)-pipéridin-4-yl]-6-chloro-1,4-dihydro-benzo[d][1,3]oxazin-2-one |
| 460 | 1-[1-(4-tert-butyl-benzènesulfonyl)-pipéridin-4-yl]-6-méthyl-1,4-dihydro-benzo[d][1,3]oxazin-2-one |
| 461 | 6-bromo-1-[1-(4-tert-butyl-benzènesulfonyl)-pipéridin-4-yl]-1,4-dihydro-benzo[d][1,3]oxazin-2-one |
| 462 | 6-chloro-1-[1-(4-phénoxy-benzènesulfonyl)-pipéridin-4-yl]-1,4-dihydro-benzo[d][1,3]oxazin-2-one |
| 463 | 1-[1-(2-bromo-4,6-difluoro-benzènesulfonyl)-pipéridin-4-yl]-1,4-dihydro-benzo[d][1,3]oxazin-2-one |
| 464 | 1-[1-(2-méthanesulfonyl-benzènesulfonyl)-pipéridin-4-yl]-8-méthyl-1,4-dihydro-benzo[d][1,3]oxazin-2-one |
| 465 | 6-chloro-1-[1-(2-méthanesulfonyl-benzènesulfonyl)-pipéridin-4-yl]-1,4-dihydro-benzo[d][1,3]oxazin-2-one |
| 466 | 1-[1-(2-méthanesulfonyl-benzènesulfonyl)-pipéridin-4-yl]-6-méthyl-1,4-dihydro-benzo[d][1,3]oxazin-2-one |
| 467 | 6-bromo-1-[1-(2-méthanesulfonyl-benzènesulfonyl)-pipéridin-4-yl]-1,4-dihydro-benzo[d][1,3]oxazin-2-one |
| 468 | 8-méthyl-1-[1-(4-propyl-benzènesulfonyl)-pipéridin-4-yl]-1,4-dihydro-benzo[d][1,3]oxazin-2-one |
| 469 | 6-chloro-1-[1-(4-propyl-benzènesulfonyl)-pipéridin-4-yl]-1,4-dihydro-benzo[d][1,3]oxazin-2-one |
| 470 | 6-méthyl-1-[1-(4-propyl-benzènesulfonyl)-pipéridin-4-yl]-1,4-dihydro-benzo[d][1,3]oxazin-2-one |
| 471 | 6-bromo-1-[1-(4-propyl-benzènesulfonyl)-pipéridin-4-yl]-1,4-dihydro-benzo[d][1,3]oxazin-2-one |
| 472 | 1-[1-(3-chloro-2-méthyl-benzènesulfonyl)-pipéridin-4-yl]-8-méthyl-1,4-dihydro-benzo[d][1,3]oxazin-2-one |
| 473 | 6-chloro-1-[1-(3-chloro-2-méthyl-benzènesulfonyl)-pipéridin-4-yl]-1,4-dihydro-benzo [d][1,3]oxazin-2-one |
| 474 | 1-[1-(3-chloro-2-méthyl-benzènesulfonyl)-pipéridin-4-yl]-6-méthyl-1,4-dihydro-benzo[d][1,3]oxazin-2-one |
| 475 | 6-bromo-1-[1-(3-chloro-2-méthyl-benzènesulfonyl)-pipéridin-4-yl]-1,4-dihydro-benzo[d][1,3]oxazin-2-one |
| 476 | 1-[1-(4-butyl-benzènesulfonyl)-pipéridin-4-yl]-8-méthyl-1,4-dihydro-benzo[d][1,3]oxazin-2-one |
| 477 | 1-[1-(4-butyl-benzènesulfonyl)-pipéridin-4-yl]-6-chloro-1,4-dihydro-benzo[d][1,3]oxazin-2-one |
| 478 | 1-[1-(4-butyl-benzènesulfonyl)-pipéridin-4-yl]-6-méthyl-1,4-dihydro-benzo[d][1,3]oxazin-2-one |
| 479 | 6-bromo-1-[1-(4-butyl-benzènesulfonyl)-pipéridin-4-yl]-1,4-dihydro-benzo[d][1,3]oxazin-2-one |
| 480 | 1-[1-(4-bromo-3-méthyl-benzènesulfonyl)-pipéridin-4-yl]-8-méthyl-1,4-dihydro-benzo[d][1,3]oxazin-2-one |
| 481 | 1-[1-(4-bromo-3-méthyl-benzènesulfonyl)-pipéridin-4-yl]-6-chloro-1,4-dihydro-benzo[d][1,3]oxazin-2-one |
| 482 | 1-[1-(4-bromo-3-méthyl-benzènesulfonyl)-pipéridin-4-yl]-6-méthyl-1,4-dihydro-benzo[d][1,3]oxazin-2-one |
| 483 | 6-bromo-1-[1-(4-bromo-3-méthyl-benzènesulfonyl)-pipéridin-4-yl]-1,4-dihydro-benzo[d][1,3]oxazin-2-one |
| 484 | 1-{1-[4-(1,1-diméthyl-propyl)-benzènesulfonyl]-pipéridin-4-yl}-8-méthyl-1,4-dihydro-benzo[d][1,3]oxazin-2-one |
| 485 | 6-chloro-1-{1-[4-(1,1-diméthyl-propyl)-benzènesulfonyl]-pipéridin-4-yl}-1,4-dihydro-benzo[d][1,3]oxazin-2-one |
| 486 | 1-{1-[4-(1,1-diméthyl-propyl)-benzènesulfonyl]-pipéridin-4-yl}-6-méthyl-1,4-dihydro-benzo[d][1,3]oxazin-2-one |
| 487 | 6-bromo-1-{1-[4-(1,1-diméthyl-propyl)-benzènesulfonyl]-pipéridin-4-yl}-1,4-dihydro-benzo[d][1,3]oxazin-2-one |
| 488 | 1-(1-éthènesulfonyl-pipéridin-4-yl)-8-méthyl-1,4-dihydro-benzo[d][1,3]oxazin-2-one |
| 489 | Acide 3-[4-(8-méthyl-2-oxo-4H-benzo[d][1,3]oxazin-1-yl)-pipéridine-1-sulfonyl] -benzoïque |
| 490 | Acide 3-[4-(6-méthyl-2-oxo-4H-benzo[d][1,3]oxazin-1-yl)-pipéridine-1-sulfonyl]-benzoïque |
| 491 | Acide 3-[4-(6-bromo-2-oxo-4H-benzo[d][1,3]oxazin-1-yl)-pipéridine-1-sulfonyl] -benzoïque |
| 492 | 1-[1-(3-chloro-2-fluoro-benzènesulfonyl)-pipéridin-4-yl]-8-méthyl-1,4-dihydro-benzo[d][1,3]oxazin-2-one |
| 493 | 6-chloro-1-[1-(3-chloro-2-fluoro-benzènesulfonyl)-pipéridin-4-yl]-1,4-dihydro-benzo[d][1,3]oxazin-2-one |
| 494 | 1-[1-(3-chloro-2-fluoro-benzènesulfonyl)-pipéridin-4-yl]-6-méthyl-1,4-dihydro-benzo[d][1,3]oxazin-2-one |
| 495 | 6-bromo-1-[1-(3-chloro-2-fluoro-benzènesulfonyl)-pipéridin-4-yl]-1,4-dihydro-benzo[d][1,3]oxazin-2-one |
| 496 | N-{4-méthyl-5-[4-(8-méthyl-2-oxo-4H-benzo[d][1,3]oxazin-1-yl)-pipéridine-1-sulfonyl]-thiazol-2-yl}-acétamide |
| 497 | N-{5-[4-(6-chloro-2-oxo-4H-benzo[d][1,3]oxazin-1-yl)-pipéridine-1-sulfonyl]-4-méthyl-thiazol-2-yl}-acétamide |
| 498 | N-{4-méthyl-5-[4-(6-méthyl-2-oxo-4H-benzo[d][1,3]oxazin-1-yl)-pipéridme-1-sulfonyl]-thiazol-2-yl} -acétamide |
| 499 | N-{5-[4-(6-bromo-2-oxo-4H-benzo[d][1,3]oxazin-1-yl)-pipéridine-1-sulfonyl]-4-méthyl-thiazol-2-yl}-acétamide |
| 500 | 1-[1-(2-bromo-4-fluoro-benzènesulfonyl)-pipéridin-4-yl]-8-méthyl-1,4-dihydro-benzo[d][1,3]oxazin-2-one |
| 501 | 1-[1-(2-bromo-4-fluoro-benzènesulfonyl)-pipéridin-4-yl]-6-chloro-1,4-dihydro-benzo[d][1,3]oxazin-2-one |
| 502 | 1-[1-(2-bromo-4-fluoro-benzènesulfonyl)-pipéridin-4-yl]-6-méthyl-1,4-dihydro-benzo[d][1,3]oxazin-2-one |
| 503 | 6-brome-1-[1-(2-bromo-4-fluoro-benzènesulfonyl)-pipéridin-4-yl]-1,4-dihydro-benzo[d][1,3]oxazin-2-one |
| 504 | 1-[1-(5-chloro-2-fluoro-benzènesulfonyl)-pipéridin-4-yl]-8-méthyl-1,4-dihydro-benzo[d][1,3]oxazin-2-one |
| 505 | 6-chlore-1-[1-(5-chloro-2-fluoro-benzènesulfonyl)-pipéridin-4-yl]-1,4-dihydro-benzo[d][1,3]oxazin-2-one |
| 506 | 1-[1-(5-chloro-2-fluoro-benzènesulfonyl)-pipéridin-4-yl]-6-méthyl-1,4-dihydro-benzo[d][1,3]oxazin-2-one |
| 507 | 6-bromo-1-[1-(5-chloro-2-fluoro-benzènesulfonyl)-pipéridin-4-yl]-1,4-dihydro-benzo[d][1,3]oxazin-2-one |
| 508 | 1-[1-(4-bromo-3-trifluorométhyl-benzènesulfonyl)-pipéridin-4-yl]-8-méthyl-1,4-dihydro-benzo[d][1,3]oxazin-2-one |
| 509 | 1-[1-(4-bromo-3-trifluorométhyl-benzènesulfonyl)-pipéridin-4-yl]-6-chloro-1,4-dihydro-benzo[d][1,3]oxazin-2-one |
| 510 | 1-[1-(4-bromo-3-trifluorométhyl-benzènesulfonyl)-pipéridin-4-yl]-6-méthyl-1,4-dihydro-benzo[d][1,3]oxazin-2-one |
| 511 | 6-bromo-1-[1-(4-bromo-3-trifluorométhyl-benzènesulfonyl)-pipéridin-4-yl]-1,4-dihydro-benzo[d][1,3]oxazin-2-one |
| 512 | 1-[1-(2-méthanesulfbnyl-benzènesulfonyl)-pipéridin-4-yl]-1,4-dihydro-benzo[d][1,3]oxazin-2-one |
| 513 | 1-[1-(4-propyl-benzènesulfonyl)-pipéridin-4-yl]-1,4-dihydro-benzo[d][1,3]oxazin-2-one |
| 514 | 1-[1-(3-chloro-2-méthyl-benzènesulfonyl)-pipéridin-4-yl]-1,4-dihydro-benzo[d][1,3]oxazin-2-one |
| 515 | 1-[1-(4-butyl-benzènesulfonyl)-pipéridin-4-yl]-1,4-dihydro-benzo[d][1,3]oxazin-2-one |
| 516 | 1-[1-(4-bromo-3-méthyl-benzènesulfonyl)-pipéridin-4-yl]-1,4-dihydro-benzo[d][1,3]oxazin-2-one |
| 517 | 1-{1-[4-(1,1-diméthyl-propyl)-benzènesulfonyl]-pipéridin-4-yl}-1,4-dihydro-benzo[d][1,3]oxazin-2-one |
| 518 | N-{4-méthyl-5-[4-(2-oxo-4H-benzo[d][1,3]oxazin-1-yl)-pipéridine-1-sulfonyl]-thiazol-2-yl}-acétamide |
| 519 | 1-[1-(3-chloro-2-fluoro-benzènesulfonyl)-pipéridin-4-yl]-1,4-dihydro-benzo[d][1,3]oxazin-2-one |
| 520 | 1-[1-(2-bromo-4-fluoro-benzènesulfonyl)-pipéridin-4-yl]-1,4-dihydro-benzo [d] [1,3]oxazin-2-one |
| 521 | 1-[1-(4-bromo-3-trifluorométhyl-benzènesulfonyl)-pipéridin-4-yl]-1,4-dihydro-benzo[d][1,3]oxazin-2-one |
| 522 | 1-[1-(5-chloro-2-fluoro-benzènesulfonyl)-pipéridin-4-yl]-1,4-dihydro-benzo[d][1,3]oxazin-2-one |
| 523 | 1-[1-(isoqumoléme-5-sulfonyl)-pipéridin-4-yl]-1,4-dihydro-benzo[d][1,3]oxazin-2-one |
| 524 | 6-fluoro-1-[1-(2-méthanesulfonyl-benzènesulfonyl)-pipéridin-4-yl]-1,4-dihydro-benzo[d][1,3]oxazin-2-one |
| 525 | 6-fluor-1-[1-(4-propyl-benzènesulfonyl)-pipéridin-4-yl]-1,4-dihydro-benzo[d][1,3]oxazin-2-one |
| 526 | 1-[1-(3-chloro-2-méthyl-benzènesulfonyl)-pipéridin-4-yl]-6-fluoro-1,4-dihydro-benzo[d][1,3]oxazin-2-one |
| 527 | 1-[1-(4-butyl-benzènesulfonyl)-pipéridin-4-yl]-6-fluoro-1,4-dihydro-benzo[d][1,3]oxazin-2-one |
| 528 | 1-[1-(4-bromo-3-méthyl-benzènesulfonyl)-pipéridin-4-yl]-6-fluoro-1,4-dihydro-benzo[d][1,3]oxazin-2-one |
| 529 | 1-{1-[4-(1,1-diméthyl-propyl)-benzènesulfonyl]-pipéridin-4-yl}-6-fluoro-1,4-dihydro-benzo[d][1,3]oxazin-2-one |
| 530 | N-{5-[4-(6-fluoro-2-oxo-4H-benzo[d][1,3]oxazin-1-yl)-pipéridine-1-sulfonyl]-4-méthyl-thiazol-2-yl}-acétamide |
| 531 | 1-[1-(3-chloro-2-fluoro-benzènesulfonyl)-pipéridin-4-yl]-6-fluoro-1,4-dihydro-benzo[d][1,3]oxazin-2-one |
| 532 | 1-[1-(2-bromo-4-fluoro-benzènesulfonyl)-pipéridin-4-yl]-6-fluoro-1,4-dihydro-benzo[d][1,3]oxazin-2-one |
| 533 | 1-[1-(4-bromo-3-trifluorométhyl-benzènesulfonyl)-pipéridin-4-yl]-6-fluoro-1,4-dihydro-benzo[d][1,3]oxazin-2-one |
| 534 | 1-[1-(5-chloro-2-fluoro-benzènesulfonyl)-pipéridin-4-yl]-6-fluoro-1,4-dihydro-benzo[d][1,3]oxazin-2-one |
| 535 | 6-fluor-1[1-(isoquinoléine-5-sulfonyl)-pipéridin-4-yl]-1,4-dihydro-benzo[d][1,3]oxazin-2-one |
| 536 | 6-fluoro-1-[1-(quinoléine-8-sulfonyl)-pipéridin-4-yl]-1,4-dihydro-benzo[d][1,3]oxazin-2-one |
| 537 | 1-[1-(5-chloro-3-méthyl-benzo[b]thiophène-2-sulfonyl)-pipéridin-4-yl]-6-fluoro-1,4-dihydro-benzo[d][1,3]oxazin-2-one |
| 538 | 6-fluor-1-[1-(naphtalène-1-sulfonyl)-pipéridin-4-yl]-1,4-dihydro-benzo[d][1,3]oxazin-2-one |
| 539 | 6-fluoro-1-[1-(naphtalène-2-sulfonyl)-pipéridin-4-yl]-1,4-dihydro-benzo[d][1,3]oxazin-2-one |
| 540 | 1-[1-(benzo[b]thiophène-2-sulfonyl)-pipéridin-4-yl]-6-fluoro-1,4-dihydro-benzo[d][1,3]oxazin-2-one |
| 541 | 1-[1-(benzo[b]thiophène-3-sulfonyl)-pipéridin-4-yl]-6-fluoro-1,4-dihydro-benzo[d][1,3]oxazin-2-one |
| 542 | 8-méthoxy-1-[1-(quinoléine-8-sulfonyl)-pipéridin-4-yl]-1,4-dihydro-benzo[d][1,3]oxazin-2-one |
| 543 | 1-[1-(5-chloro-3-méthyl-benzo[b]thiophène-2-sulfonyl)-pipéridin-4-yl]-8-méthoxy-1,4-dihydro-benzo[d][1,3]oxazin-2-one |
| 544 | 8-méthoxy-1-[1-(naphtalène-1-sulfonyl)-pipéridin-4-yl]-1,4-dihydro-benzo[d][1,3]oxazin-2-one |
| 545 | 8-méthoxy-1-[1-(naphtalène-2-sulfonyl)-pipéridin-4-yl]-1,4-dihydro-benzo[d][1,3]oxazin-2-one |
| 546 | 1-[1-(benzo[b]thiophène-2-sulfonyl)-pipéridin-4-yl]-8-méthoxy-1,4-dihydro-benzo[d][1,3]oxazin-2-one |
| 547 | 1-[1-(benzo[b]thiophène-3-sulfonyl)-pipéridin-4-yl]-8-méthoxy-1,4-dihydro-benzo[d][1,3]oxazin-2-one |
| 548 | 5-chlore-1-[1-(2-méthanesulfonylbenzènesulfonyl)-pipéridin-4-yl]-1,4-dihydro-benzo[d] [1,3]oxazin-2-one |
| 549 | 5-chloro-1-[1-(4-propyl-benzènesulfonyl)-pipéridin-4-yl]-1,4-dihydro-benzo[d] [1,3]oxazin-2-one |
| 550 | 5-chloro-1-[1-(3-chloro-2-méthyl-benzènesulfonyl)-pipéridin-4-yl]-1,4-dihydro-benzo[d][1,3]oxazin-2-one |
| 551 | 1-[1-(4-butyl-benzènesulfonyl)-pipéridin-4-yl]-5-chloro-1,4-dihydro-benzo[d][1,3]oxazin-2-one |
| 552 | 1-[1-(4-bromo-3-méthyl-benzènesulfonyl)-pipéridin-4-yl]-5-chloro-1,4-dihydro-benzo[d][1,3]oxazin-2-one |
| 553 | 5-chloro-1-{1-[4-(1,1-diméthyl-propyl)-benzènesulfonyl]-pipéridin-4-yl}-1,4-dihydro-benzo[d][1,3]oxazin-2-one |
| 554 | N-{5-[4-(5-chloro-2-oxo-4H-benzo[d][1,3]oxazin-1-yl)-pipéridine-1-sulfonyl]-4-méthyl-thiazol-2-yl}-acétamide |
| 555 | 5-chloro-1-[1-(3-chloro-2-fluoro-benzènesulfonyl)-pipéridin-4-yl]-1,4-dihydro-benzo[d][1,3]oxazin-2-one |
| 556 | 1-[1-(2-bromo-4-fluoro-benzènesulfonyl)-pipéridin-4-yl]-5-chloro-1,4-dihydro-benzo[d][1,3]oxazin-2-one |
| 557 | 1-[1-(4-bromo-3-trifluorométhyl-benzènesulfonyl)-pipéridin-4-yl]-5-chloro-1,4-dihydro-benzo[d][1,3]oxazin-2-one |
| 558 | 5-chloro-1-[1-(5-chloro-2-fluoro-benzènesulfonyl)-pipéridin-4-yl]-1,4-dihydro-benzo[d][1,3]oxazin-2-one |
| 559 | 5-chloro-1-[1-(isoquinoléine-5-sulfonyl)-pipéridin-4-yl]-1,4-dihydro-benzo[d][1,3]oxazin-2-one |
| 560 | 1-[1-(2-méthanesulfonyl-benzènesulfonyl)-pipéridin-4-yl-8-méthoxy-1,4-dihydro-benzo[d][1,3]oxazin-2-one |
| 561 | 1-[1-(2-méthanesulfonyl-benzènesulfonyl)-pipéridin-4-yl]-8-méthoxy-1,4-dihydro-benzo[d][1,3]oxazin-2-one |
| 562 | 1-[1-(3-chloro-2-méthyl-benzènesulfonyl)-pipéridin-4-yl]-8-méthoxy-1,4-dihydro-benzo[d][1,3]oxazin-2-one |
| 563 | 1-[1-(4-butyl-benzènesulfonyl)-pipéridin-4-yl]-8-méthoxy-1,4-dihydro-benzo[d][1,3]oxazin-2-one |
| 564 | 1-[1-(4-bromo-3-méthyl-benzènesulfonyl)-pipéridin-4-yl]-8-méthoxy-1,4-dihydro-benzo[d][1,3]oxazin-2-one |
| 565 | 1-{1-[4-(1,1-diméthyl-propyl)-benzènesulfonyl]-pipéridin-4-yl}-8-méthoxy-1,4-dihydro-benzo[d][1,3]oxazin-2-one |
| 566 | N-{5-[4-(8-méthoxy-2-oxo-4H-benzo[d][1,3]oxazin-1-yl)-pipéridine-1-sulfonyl]-4-méthyl-thiazol-2-yl}-acétamide |
| 567 | 1-[1-(3-chloro-2-fluoro-benzènesulfonyl)-pipéridin-4-yl]-8-méthoxy-1,4-dihydro-benzo[d][1,3]oxazin-2-one |
| 568 | 1-[1-(2-bromo-4-fluoro-benzènesulfonyl)-pipéridin-4-yl]-8-méthoxy-1,4-dihydro-benzo[d][1,3]oxazin-2-one |
| 569 | 1-[1-(4-bromo-3-trifluorométhyl-benzènesulfonyl)-pipéridin-4-yl]-8-méthoxy-1,4-dihydro-benzo[d][1,3]oxazin-2-one |
| 570 | 1-[1-(5-chloro-2-fluoro-benzènesulfonyl)-pipéridin-4-yl]-8-méthoxy-1,4-dihydro-benzo[d][1,3]oxazin-2-one |
| 571 | Chlorhydrate de 1-[1-(isoquinoléine-5-sulfonyl)-pipéridin-4-yl]-8-méthoxy-1,4-dihydro-benzo[d][1,3]oxazin-2-one |
| 572 | 1-[1-(4-méthyl-naphtalène-1-sulfonyl)-pipéridin-4-yl]-1,4-dihydro-benzo[d][1,3]oxazin-2-one |
| 573 | 6-chloro-1-[1-(4-méthyl-naphtalène-1-sulfonyl)-pipéridin-4-yl]-1,4-dihydro-benzo[d][1,3]oxazin-2-one |
| 574 | 6-méthyl-1-[1-(4-méthyl-naphtalène-1-sulfonyl)-pipéridin-4-yl]-1,4-dihydro-benzo[d][1,3]oxazin-2-one |
| 575 | 8-méthyl-1-[1-(4-méthyl-naphtalène-1-sulfonyl)-pipéridin-4-yl]-1,4-dihydro-benzo[d][1,3]oxazin-2-one |
| 576 | 6-fluoro-1-[1-(4-méthyl-naphtalène-1-sulfonyl)-pipéridin-4-yl]-1,4-dihydro-benzo[d][1,3]oxazin-2-one |
| 577 | 8-méthoxy-1-[1-(4-méthyl-naphtalène-1-sulfonyl)-pipéridin-4-yl]-1,4-dihydro-benzo[d][1,3]oxazin-2-one |
| 578 | 5-chloro-1-[1-(4-méthyl-naphtalène-1-sulfonyl)-pipéridin-4-yl]-1,4-dihydro-benzo[d][1,3]oxazin-2-one |
| 579 | 5-chloro-1-[1-(naphtalène-1-sulfonyl)-pipéridin-4-yl]-1,4-dihydro-benzo[d][1,3]oxazin-2-one |
| 580 | 5-chloro-1-[1-(naphtalène-2-sulfonyl)-pipéridin-4-yl]-1,4-dihydro-benzo[d][1,3]oxazin-2-one |
| 581 | 5-chloro-1-[1-(quinoléine-8-sulfonyl)-pipéridin-4-yl]-1,4-dihydro-benzo[d][1,3]oxazin-2-one |
| 582 | 5-chloro-1-[1-(5-chloro-3-méthyl-benzo[b]thiophène-2-sulfonyl)-pipéridin-4-yl]-1,4-dihydro-benzo[d][1,3]oxazin-2-one |
| 583 | 1-[1-(benzo[b]thiophène-2-sulfonyl)-pipéridin-4-yl]-5-chloro-1,4-dihydro-benzo[d][1,3]oxazin-2-one |
| 584 | 1-[1-(benzo [b]thiophène-3-sulfonyl)-pipéridin-4-yl]-5-chloro-1,4-dihydro-benzo[d][1,3]oxazin-2-one |
| 585 | 6-bromo-1-[1-(4-méthyl-naphtalène-1-sulfonyl)-pipéridin-4-yl]-1,4-dihydro-benzo[d][1,3]oxazin-2-one |
| 586 | Acide 2-chloro-4-fluoro-5-[4-(8-méthyl-2-oxo-4H-benzo[d][1,3]oxazin-1-yl)-pipéridine-1-sulfonyl]-benzoïque |
| 587 | Acide 2-chloro-5-[4-(6-chloro-2-oxo-4H-benzo[d][1,3]oxazin-1-yl)-pipéridine-1-sulfonyl]-benzoïque |
| 588 | Acide 2-chloro-4-fluoro-5-[4-(6-méthyl-2-oxo-4H-benzo[d][1,3]oxazin-1-yl)-pipéridine-1-sulfonyl]-benzoïque |
| 589 | Acide 2-chloro-4-fluoro-5-[4-(2-oxo-4H-benzo[d][1,3]oxazin-1-yl)-pipéridine-1-sulfonyl]-benzoïque |
| 590 | Acide 2-chloro-4-fluoro-5-[4-(8-méthoxy-2-oxo-4H-benzo[d][1,3]oxazin-1-yl)-pipéridine-1-sulfonyl]-benzoïque |
| 591 | Acide 2-chloro-5-[4-(5-chloro-2-oxo-4H-benzo[d][1,3]oxazin-1-yl)-pipéridine-1-sulfonyl]-benzoïque |
| 592 | Acide 3-[4-(2-oxo-4H-benzo[d][1,3]oxazin-1-yl)-pipéridine-1-sulfonyl]-benzoïque |
| 593 | Acide 3-[4-(8-méthoxy-2-oxo-4H-benzo[d][1,3]oxazin-1-yl)-pipéridine-1-sulfonyl] -benzoïque |
| 594 | Acide 3-[4-(5-chloro-2-oxo-4H-benzo[d][1,3]oxazin-1-yl)-pipéridine-1-sulfonyl]-benzoïque |
| 595 | Chlorhydrate de 1-[1-(isoquinoléine-5-sulfonyl)-pipéridin-4-yl]-8-méthyl-1,4-dihydro-benzo[d][1,3]oxazin-2-one |
| 596 | Chlorhydrate de 6-chloro-1-[1-(isoquinoléine-5-sulfonyl)-pipéridin-4-yl]-1,4-dihydro-benzo[d][1,3]oxazin-2-one |
| 597 | Chlorhydrate de 1-[1-(isoquinoléine-5-sulfonyl)-pipéridin-4-yl]-6-méthyl-1,4-dihydro-benzo[d][1,3]oxazin-2-one |
| 598 | 6,7-difluoro-1-[1-(quinoléine-8-sulfonyl)-pipéridin-4-yl]-1,4-dihydro-benzo[d][1,3]oxazin-2-one |
| 599 | 1-[1-(5-chloro-3-méthyl-benzo[b]thiophène-2-sulfonyl)-pipéridin-4-yl]-6,7-difluoro-1,4-dihydro-benzo[d][1,3]oxazin-2-one |
| 600 | 6,7-difluoro-1-[1-(naphtalène-1-sulfonyl)-pipéridin-4-yl]-1,4-dihydro-benzo[d][1,3]oxazin-2-one |
| 601 | 6,7-difluoro-1-[1-(naphtalène-2-sulfonyl)-pipéridin-4-yl]-1,4-dihydro-benzo[d][1,3]oxazin-2-one |
| 602 | 1-[1-(benzo[b]thiophène-2-sulfonyl)-pipéridin-4-yl]-6,7-difluoro-1,4-dihydro-benzo[d][1,3]oxazin-2-one |
| 603 | 1-[1-(benzo[b]thiophène-3-sulfonyl)-pipéridin-4-yl]-6,7-difluoro-1,4-dihydro-benzo[d][1,3]oxazin-2-one |
| 604 | 1-[1-(5-diméthylamino-naphtalène-1-sulfonyl)-pipéridin-4-yl]-6,7-difluoro-1,4-dihydro-benzo[d][1,3]oxazin-2-one |
| 605 | 1-[1-(biphényl-4-sulfonyl)-pipéridin-4-yl]-6,7-difluoro-1,4-dihydro-benzo[d][1,3]oxazin-2-one |
| 606 | 1-[1-(benzo[1,2,5]thiadiazole-4-sulfonyl)-pipéridin-4-yl]-6,7-difluoro-1,4-dihydro-benzo[d][1,3]oxazin-2-one |
| 607 | 1-[1-(benzo[1,2,5]oxadiazole-4-sulfonyl)-pipéridin-4-yl]-6,7-difluoro-1,4-dihydro-benzo[d][1,3]oxazin-2-one |
| 608 | 1-[1-(7-chloro-benzo[1,2,5]oxadiazole-4-sulfonyl)-pipéridin-4-yl]-6,7-difluoro-1,4-dihydro-benzo[d][1,3]oxazin-2-one |
| 609 | 6,7-difluoro-1-[1-(4-méthyl-naphtalène-1-sulfonyl)-pipéridin-4-yl]-1,4-dihydro-benzo[d][1,3]oxazin-2-one |
| 610 | 1-[1-(4-chloro-naphtalène-1-sulfonyl)-pipéridin-4-yl]-1,4-dihydro-benzo[d][1,3]oxazin-2-one |
| 611 | 1-[1-(4-fluoro-naphtalène-1-sulfonyl)-pipéridin-4-yl]-1,4-dihydro-benzo[d][1,3]oxazin-2-one |
| 612 | 1-[1-(dibenzofran-2-sulfonyl)-pipéridin-4-yl]-1,4-dihydro-benzo[d][1,3]oxazin-2-one |
| 613 | 1-[1-(2,3-dihydro-dibenzofran-5-sulfonyl)-pipéridin-4-yl]-1,4-dihydro-benzo[d][1,3]oxazin-2-one |
| 614 | 1-[1-(biphényl-2-sulfonyl)-pipéridin-4-yl]-1,4-dihydro-benzo [d] [1,3]oxazin-2-one |
| 615 | 1-[1-(5-isoxazol-5-yl-thiophène-2-sulfbnyl)-pipéridin-4-yl]-1,4-dihydro-benzo[d][1,3]oxazin-2-one |
| 616 | 1-[1-(4-chloro-naphtalène-1-sulfbnyl)-pipéridin-4-yl]-8-méthyl-1,4-dihydro-benzo[d][1,3]oxazin-2-one |
| 617 | 1-[1-(4-fluoro-naphtalène-1-sulfonyl)-pipéridin-4-yl]-8-méthyl-1,4-dihydro-benzo[d][1,3]oxazin-2-one |
| 618 | 1-[1-(dibenzofuran-2-sulfonyl)-pipéridin-4-yl]-8-méthyl-1,4-dihydro-benzo[d][1,3]oxazin-2-one |
| 619 | 1-[1-(2,3-dihydro-benzofuran-5-sulfonyl)-pipéridin-4-yl]-8-méthyl-1,4-dihydro-benzo[d][1,3]oxazin-2-one |
| 620 | 1-[1-(biphényl-2-sulfonyl)-pipéridin-4-yl]-8-méthyl-1,4-dihydro-benzo[d][1,3]oxazin-2-one |
| 621 | 1-[1-(5-isoxazol-5-yl-thiophène-2-sulfonyl)-pipéridin-4-yl]-8-méthyl-1,4-dihydro-benzo[d][1,3]oxazin-2-one |
| 622 | 5-chloro-1-[1-(4-chloro-naphtalène-1-sulfonyl)-pipéridin-4-yl]-1,4-dihydro-benzo[d][1,3]oxazin-2-one |
| 623 | 5-chloro-1-[1-(4-fluoro-naphtalène-1-sulfonyl)-pipéridin-4-yl]-1,4-dihydro-benzo[d][1,3]oxazin-2-one |
| 624 | 5-chlore-1-[1-(dibenzofuran-2-sulfonyl)-pipéridin-4-yl]-1,4-dihydro-benzo[d][1,3]oxazin-2-one |
| 625 | 5-chloro-1-[1-(2,3-dihydro-benzofuran-5-sulfonyl)-pipéridin-4-yl]-1,4-dihydro-benzo[d][1,3]oxazin-2-one |
| 626 | 1-[1-(biphényl-2-sulfonyl)-pipéridin-4-yl]-5-chloro-1,4-dihydro-benzo[d][1,3]oxazin-2-one |
| 627 | 5-chloro-1-[1-(5-isoxazol-5-yl-thiophène-2-sulfonyl)-pipéridin-4-yl]-1,4-dihydro-benzo[d][1,3]oxazin-2-one |
| 628 | 1-[1-(4-chloro-naphtalène-1-sulfonyl)-pipéridin-4-yl]-8-méthoxy-1,4-dihydro-benzo[d][1,3]oxazin-2-one |
| 629 | 1-[1-(4-fluoro-naphtalène-1-sulfonyl)-pipéridin-4-yl]-8-méthoxy-1,4-dihydro-benzo[d][1,3]oxazin-2-one |
| 630 | 1-[1-(dibenzofuran-2-sulfonyl)-pipéridin-4-yl]-8-méthoxy-1,4-dihydro-benzo[d][1,3]oxazin-2-one |
| 631 | 1-[1-(2,3-dihydro-dibenzofuran-5-sulfonyl)-pipéridin-4-yl]-8-méthoxy-1,4-dihydro-benzo[d][1,3]oxazin-2-one |
| 632 | 1-[1-(biphényl-2-sulfonyl)-pipéridin-4-yl]-8-méthoxy-1,4-dihydro-benzo[d][1,3]oxazin-2-one |
| 633 | 1-[1-(5-isoxazol-5-yl-thiophène-2-sulfonyl)-pipéridin-4-yl]-8-méthoxy-1,4-dihydro-benzo[d][1,3]oxazin-2-one |
| 634 | 6-chloro-1-[1-(4-chloro-naphtalène-1-sulfonyl)-pipéridin-4-yl]-1,4-dihydro-benzo[d][1,3]oxazin-2-one |
| 635 | 6-chloro-1-[1-(4-fluoro-naphtalène-1-sulfonyl)-pipéridin-4-yl]-1,4-dihydro-benzo[d][1,3]oxazin-2-one |
| 636 | 6-chloro-1-[1-(dibenzofuran-2-sulfonyl)-pipéridin-4-yl]-1,4-dihydro-benzo[d][1,3]oxazin-2-one |
| 637 | 6-chloro-1-[1-(2,3-dihydro-dibenzofuran-5-sulfonyl)-pipéridin-4-yl]-1,4-dihydro-benzo[d][1,3]oxazin-2-one |
| 638 | 1-[1-(biphényl-2-sulfonyl)-pipéridin-4-yl]-6-chloro-1,4-dihydro-benzo[d][1,3]oxazin-2-one |
| 639 | 6-chloro-1-[1-(5-isoxazol-5-yl-thiophène-2-sulfonyl)-pipéridin-4-yl]-1,4-dihydro-benzo[d][1,3]oxazin-2-one |
| 640 | 1-[1-(4-chloro-naphtalène-1-sulfonyl)-pipéridin-4-yl]-6-méthyl-1,4-dihydro-benzo[d][1,3]oxazin-2-one |
| 641 | 1-[1-(4-fluoro-naphtalène-1-sulfonyl)-pipéridin-4-yl]-6-méthyl-1,4-dihydro-benzo[d][1,3]oxazin-2-one |
| 642 | 1-[1-(dibenzokran-2-sulfonyl)-pipéridin-4-yl]-6-méthyl-1,4-dihydro-benzo[d] [1,3]oxazin-2-one |
| 643 | 1-[1-(2,3-dihydro-dibenzofuran-5-sulfonyl)-pipéridin-4-yl]-6-méthyl-1,4-dihydro-benzo[d][1,3]oxazin-2-one |
| 644 | 1-[1-(biphényl-2-sulfoyl)-pipéridin-yl]-6-méthyl-1,4-dihydro-benzo[d][1,3]oxazin-2-one |
| 645 | 1-[1-(5-isoxazol-5-yl-thiophène-2-sulfonyl)-pipéridin-4-yl]-6-méthyl-1,4-dihydro-benzo[d][1,3]oxazin-2-one |
| 646 | 1-[1-(4-chloro-naphtalène-1-sulfonyl)-pipéridin-4-yl]-6,7-difluoro-1,4-dihydro-benzo[d][1,3]oxazin-2-one |
| 647 | 6,7-difluoro-1-[1-(4-fluoro-naphtalène-1-sulfonyl)-pipéridin-4-yl]-1,4-dihydro-benzo[d][1,3]oxazin-2-one |
| 648 | 1-[1-(dibenzokran-2-sulfonyl)-pipéridin-4-yl]-6,7-difluoro-1,4-dihydro-benzo[d][1,3]oxazin-2-one |
| 649 | 1-[1-(2,3-dihydro-benzofuran-5-sulfonyl)-pipéridin-4-yl]-6,7-difluoro-1,4-dihydro-benzo[d][1,3]oxazin-2-one |
| 650 | 1-[1-(biphényl-2-sulfonyl)-pipéridin-4-yl]-6,7-difluoro-1,4-dihydro-benzo[d][1,3]oxazin-2-one |
| 651 | 6,7-difluoro-1-[1-(5-isoxazol-5-yl-thiophène-2-sulfonyl)-pipéridin-4-yl]-1,4-dihydro-benzo[d][1,3]oxazin-2-one |
| 652 | 1-[1-(1,2-diméthyl-1H-imidazole-4-sulfonyl)-pipéridin-4-yl]-1,4-dihydro-benzo[d][1,3]oxazin-2-one |
| 653 | 1-[1-(5-méthyl-benzo[1,2,5]thiadiazole-4-sulfonyl)-pipéridin-4-yl]-1,4-dihydro-benzo[d][1,3]oxazin-2-one |
| 654 | 1-[1-(3,5-diméthyl-isoxazole-4-sulfonyl)-pipéridin-4-yl]-1,4-dihydro-benzo[d][1,3]oxazin-2-one |
| 655 | 1-[1-(1,2-diméthyl-1H-imidazole-4-sulfonyl)-pipéridin-4-yl]-8-méthyl-1,4-dihydro-benzo[d][1,3]oxazin-2-one |
| 656 | 8-méthyl-1-[1-(5-méthyl-benzo[1,2,5]thiadiazole-4-sulfonyl)-pipéridin-4-yl]-1,4-dihydro-benzo[d][1,3]oxazin-2-one |
| 657 | 1-[1-(3,5-diméthyl-isoxazole-4-sulfonyl)-pipéridin-4-yl]-8-méthyl-1,4-dihydro-benzo[d][1,3]oxazin-2-one |
| 658 | 6-chloro-1-[1-(1,2-diméthyl-1H-imidazole-4-sulfonyl)-pipéridin-4-yl]-1,4-dihydro-benzo[d][1,3]oxazin-2-one |
| 659 | 6-chloro-1-[1-(5-méthyl-benzo[1,2,5]thiadiazole-4-sulfonyl)-pipéridin-4-yl]-1,4-dihydro-benzo[d][1,3]oxazin-2-one |
| 660 | 6-chloro-1-[1-(3,5-diméthyl-isoxazole-4-sulfonyl)-pipéridin-4-yl]-1,4-dihydro-benzo[d][1,3]oxazin-2-one |
| 661 | 1-[1-(1,2-diméthyl-1H-imidazole-4-sulfonyl)-pipéridin-4-yl]-8-méthoxy-1,4-dihydro-benzo[d][1,3]oxazin-2-one |
| 662 | 8-méthoxy-1-[1-(5-méthyl-benzo[1,2,5]thiadiazole-4-sulfonyl)-pipéridin-4-yl]-1,4-dihydro-benzo[d][1,3]oxazin-2-one |
| 663 | 1-[1-(3,5-diméthyl-isoxazole-4-sulfonyl)-pipéridin-4-yl]-8-méthoxy-1,4-dihydro-benzo[d][1,3]oxazin-2-one |
| 664 | 5-chloro-1-[1-(1,2-diméthyl-1H-imidazole-4-sulfonyl)-pipéridin-4-yl]-1,4-dihydro-benzo[d][1,3]oxazin-2-one |
| 665 | 5-chloro1-[1-(5-méthyl-benzo[12,5]thiadiazole-4-sulfonyl)-pipéridin-4-yl]-1,4-dihydro-benzo[d][1,3]oxazin-2-one |
| 666 | 5-chloro-1-[1-(3,5-diméthyl-isoxazole-4-sulfonyl)-pipéridin-4-yl]-1,4-dihydro-benzo[d][1,3]oxazin-2-one |
| 667 | 1-[1-(1,2-diméthyl-1H-imidazole-4-sulfonyl)-pipéridin-4-yl]-6-méthyl-1,4-dihydro-benzo[d][1,3]oxazin-2-one |
| 668 | 6-méthyl-1-[1-(5-méthyl-benzo[1,2,5]thiadiazole-4-sulfonyl)-pipéridin-4-yl]-1,4-dihydro-benzo[d][1,3]oxazin-2-one |
| 669 | 1-[1-(3,5-diméthyl-isoxazole-4-sulfonyl)-pipéridin-4-yl]-6-méthyl-1,4-dihydro-benzo[d][1,3]oxazin-2-one |
| 670 | 1-[1-(1,2-diméthyl-1H-imidazole-4-sulfonyl)-pipéridin-4-yl]-6-fluoro-1,4-dihydro-benzo[d][1,3]oxazin-2-one |
| 671 | 6-fluoro-1-[1-(5-méthyl-benzo[1,2,5]thiadiazole-4-sulfonyl)-pipéridin-4-yl]-1,4-dihydro-benzo[d][1,3]oxazin-2-one |
| 672 | 1-[1-(3,5-diméthyl-isoxazole-4-sulfonyl)-pipéridin-4-yl]-6-fluoro-1,4-dihydro-benzo[d][1,3]oxazin-2-one |
| 673 | 1-[1-(1,2-diméthyl-1H-imidazole-4-sulfonyl)-pipéridin-4-yl]-6,7-difluoro-1,4-dihydro-benzo[d][1,3]oxazin-2-one |
| 674 | 6,7-difluoro-1-[1-(5-méthyl-benzo[1,2,5]thiadiazole-4-sulfonyl)-pipéridin-4-yl]-1,4-dihydro-benzo[d][1,3]oxazin-2-one |
| 675 | 1-[1-(3,5-diméthyl-isoxazole-4-sulfonyl)-pipéridin-4-yl]-6,7-difluoro-1,4-dihydro-benzo[d][1,3]oxazin-2-one |
| 676 | 1-[1-(5-chloro-naphtalène-1-sulfonyl)-pipéridin-4-yl]-1,4-dihydro-benzo [d][1,3]oxazin-2-one |
| 677 | 1-[1-(5-chloro-naphtalène-2-sulfonyl)-pipéridin-4-yl]-1,4-dihydro-benzo [d][1,3]oxazin-2-one |
| 678 | N-{5-[4-(2-oxo-4H-benzo[d][1,3]oxazin-1-yl)-pipéridine-1-sulfonyl]-naphtalén-1-yl}-acétamide |
| 679 | 1-[1-(5-chloro-naphtalène-1-sulfonyl)-pipéridin-4-yl]-8-méthyl-1,4-dihydro-benzo [d][1,3]oxazin-2-one |
| 680 | 1-[1-(5-chloro-naphtalène-2-sulfonyl)-pipéridin-4-yl]-8-méthyl-1,4-dihydro-benzo [d][1,3]oxazin-2-one |
| 681 | N-{5-[4-(8-méthyl-2-oxo-4H-benzo[d][1,3]oxazin-1-yl)-pipéridine-1-sulfonyl]-naphtalén-1-yl}-acétamide |
| 682 | 5-chloro-1-[1-(5-chloro-naphtalène-1-sulfonyl)-pipéridin-4-yl]-1,4-dihydro-benzo [d][1,3]oxazin-2-one |
| 683 | 5-chloro-1-[1-(5-chloro-naphtalène-2-sulfonyl)-pipéridin-4-yl]-1,4-dihydro-benzo [d] [1,3]oxazin-2-one |
| 684 | N-{5-[4-(5-chloro-2-oxo-4H-benzo[d][1,3]oxazin-1-yl)-pipéridine-1-sulfonyl]-naphtalén-1-yl}-acétamide |
| 685 | 1-[1-(5-chloro-naphtalène-1-sulfonyl)-pipéridin-4-yl]-8-méthoxy-1,4-dihydro-benzo[d][1,3]oxazin-2-one |
| 686 | 1-[1-(5-chloro-naphtalène-2-sulfonyl)-pipéridin-4-yl]-8-méthoxy-1,4-dihydro-benzo[d][1,3]oxazin-2-one |
| 687 | N-{5-[4-(8-méthoxy-2-oxo-4H-benzo[d][1,3]oxazin-1-yl)-pipéridine-1-sulfonyl]-naphtalén-1-yl} acétamide |
| 688 | Ester méthylique de l'acide 2,5-diméthyl-4-[4-(8-méthyl-2-oxo-4H-benzo[d][1,3]oxazin-1-yl)-pipéridine-1-sulfonyl]-furan-3-carboxylique |
| 689 | 8-méthyl-1-[1-(2-oxo-2,3-dihydro-benzothiazole-6-sulfonyl)-pipéridin-4-yl]-1,4-dihydro-benzo[d][1,3]oxazin-2-one |
| 690 | 1-[1-(4-fluoro-3-méthyl-benzènesulfonyl)-pipéridin-4-yl]-8-méthyl-1,4-dihydro-benzo[d][1,3]oxazin-2-one |
| 691 | 8-méthyl-1-[1-(2-oxo-2,3-dihydro-benzoxazole-6-sulfonyl)-pipéridin-4-yl]-1,4-dihydro-benzo[d][1,3]oxazin-2-one |
| 692 | 1-[1-(4-cyclohexyl-benzènesulfonyl)-pipéridin-4-yl]-8-méthyl-1,4-dihydro-benzo[d][1,3]xazin-2-one |
| 693 | Ester méthylique de l'acide 2,5-diméthyl-4-[4-(2-oxo-4H-benzo[d][1,3]oxazin-1-yl)-pipéridine-1-sulfonyl]-furan-3-carboxylique |
| 694 | 1-[1-(4-fluoro-3-méthyl-benzènesulfonyl)-pipéridin-4-yl]-1,4-dihydro-benzo[d][1,3]oxazin-2-one |
| 695 | 1-[1-(2-oxo-2,3-dihydro-benzoxazole-6-sulfonyl)-pipéridin-4-yl]-1,4-dihydro-benzo[d][1,3]oxazin-2-one |
| 696 | 1-[1-(4-cyclohexyl-benzènesulfonyl)-pipéridin-4-yl]-1,4-dihydro-benzo[d][1,3]oxazin-2-one |
| 697 | Acide 2-fluoro-5-[4-(6-méthyl-2-oxo-4H-benzo[d][1,3]oxazin-1-yl)-pipéridine-1-sulfonyl]-benzoïque |
| 698 | Acide 2-fluoro-5-[4-(2-oxo-4H-benzo[d][1,3]oxazin-1-yl)-pipéridine-1-sulfonyl] -benzoïque |
| 699 | 1-[1-(2-oxo-2,3-dihydro-benzothiazole-6-sulfonyl)-pipéridin-4-yl]-1,4-dihydro-benzo[d][1,3]oxazin-2-one |
| 700 | 1-[1-(5-pyridin-2-yl-thiophène-2-sulfonyl)-pipéridin-4-yl]-1,4-dihydro-benzo[d][1,3]oxazin-2-one |
| 701 | 3-[4-(2-oxo-4H-benzo[d][1,3]oxazin-1-yl)-pipéridine-1-sulfonyl]-benzonitrile |
| 702 | Ester méthylique de l'acide 3-[4-(2-oxo-4H-benzo[d][1,3]oxazin-1-yl)-pipéridine-1-sulfonyl]-thiophène-2-carboxylique |
| 703 | 1-{5-[4-(2-oxo-4H-benzo[d][1,3]oxazin-1-yl)-pipéridine-1-sulfonyl]-naphtalèn-1-yl}-pyrrolidinyl-2,5-dione |
| 704 | 1-[1-(2-chloro-5-trifluorométhyl-benzènesulfonyl)-pipéridin-4-yl]-1,4-dihydro-benzo[d][1,3]oxazin-2-one |
| 705 | 1-[1-(3,4-diméthyl-benzènesulfonyl)-pipéridin-4-yl]-1,4-dihydro-benzo[d][1,3]oxazin-2-one |
| 706 | 8-méthyl-1-[1-(5-pyridin-2-yl-thiophène-2-sulfonyl)-pipéridin-4-yl]-1,4-dihydro-benzo[d][1,3]oxazin-2-one |
| 707 | 3-[4-(8-méthyl-2-oxo-4H-benzo[d][1,3]oxazin-1-yl)-pipéridine-1-sulfonyl]-benzonitrile |
| 708 | Ester méthylique de l'acide 3-[4-(8-méthyl-2-oxo-4H-benzo[d][1,3]oxazin-1-yl)-pipéridine-1-sulfonyl]-thiophène-2-carboxylique |
| 709 | 1-[5-[4-(8-méthyl-2-oxo-4H-benzo[d][1,3]oxazin-1-yl)-pipéridine-1-sulfonyl]-naphtalén-1-yl]-pyrrolidine-2,5-dione |
| 710 | 1-[1-(2-chloro-5-trifluorométhyl-benzènesulfonyl)-pipéridin-4-yl]-8-méthyl-1,4-dihydro-benzo[d][1,3]oxazin-2-one |
| 711 | 1-[1-(3,4-diméthyl-benzènesulfonyl)-pipéridin-4-yl]-8-méthyl-1,4-dihydro-benzo[d][1,3]oxazin-2-one |
| 712 | 5-chloro-1-[1-(5-pyridin-2-yl-thiophène-2-sulfonyl)-pipéridin-4-yl]-1,4-dihydro-benzo[d][1,3]oxazin-2-one |
| 713 | 3-[4-(5-chloro-2-oxo-4H-benzo[d][1,3]oxazin-1-yl)-pipéridine-1-sulfonyl]-benzonitrile |
| 714 | Ester méthylique de l'acide 3-[4-(5-chloro-2-oxo-4H-benzo[d][1,3]oxazin-1-yl)-pipéridine-1-sulfonyl]-thiophène-2-carboxylique |
| 715 | 1-{5-[4-(5-chloro-2-oxo-4H-benzo[d][1,3]oxazin-1-yl)-pipéridine-1-sulfonyl]-naphtalén-1-yl}-pyrrolidine-2,5-dione |
| 716 | 5-chloro-1-[1-(2-chloro-5-trifluorométhyl-benzènesulfonyl)-pipéridin-4-yl]-1,4-dihydro-benzo[d][1,3]oxazin-2-one |
| 717 | 5-chloro-1-[1-(3,4-diméthyl-benzènesulfonyl)-pipéridin-4-yl]-1,4-dihydro-benzo[d][1,3]oxazin-2-one |
| 718 | 6-méthylo-1-[1-(5-pyridin-2-yl-thiophène-2-sulfonyl)-pipéridin-4-yl]-1,4-dihydro-benzo[d][1,3]oxazin-2-one |
| 719 | 3-[4-(6-méthyl-2-oxo-4H-benzo[d][1,3]oxazin-1-yl)-pipéridine-1-sulfonyl]-benzonitrile |
| 720 | Ester méthylique de l'acide 3-[4-(6-méthyl-2-oxo-4H-benzo[d][1,3]oxazin-1-yl)-pipéridine-1-sulfonyl]-thiophène-2-carboxylique |
| 721 | 1-{5-[4-(6-méthyl-2-oxo-4H-benzo[d][1,3]oxazin-1-yl)-pipéridine-1-sulfonyl]-naphtalén-1-yl}-pyrrolidine-2,5-dione |
| 722 | 1-[1-(2-chloro-5-trifluorométhyl-benzènesulfonyl)-pipéridin-4-yl]-6-méthyl-1,4-dihydro-benzo[d][1,3]oxazin-2-one |
| 723 | 1-[1-(3,4-diméthyl-benzènesulfonyl)-pipéridin-4-yl]-6-méthyl-1,4-dihydro-benzo[d][1,3]oxazin-2-one |
| 724 | 6-chloro-1-[1-(5-pyridin-2-yl-thiophène-2-sulfonyl)-pipéridin-4-yl]-1,4-dihydro-benzo[d][1,3]oxazin-2-one |
| 725 | 3-[4-(6-chloro-2-oxo-4H-benzo[d][1,3]oxazin-1-yl)-pipéridine-1-sulfonyl]-benzonitrile |
| 726 | Ester méthylique de l'acide 3-[4-(6-chloro-2-oxo-4H-benzo[d][1,3]oxazin-1-yl)-pipéridine-1-sulfonyl]-thiophène-2-carboxylique |
| 727 | 1-{5-[4-(6-chloro-2-oxo-4H-benzo[d][1,3]oxazin-1-yl)-pipéridine-1-sulfonyl]-naphtalén-1-yl)-pyrrolidine-2,5-dione |
| 728 | 6-chloro-1-[1-(2-chloro-5-trifluorométhyl-benzènesulfonyl)-pipéridin-4-yl]-1,4-dihydro-benzo[d][1,3]oxazin-2-one |
| 729 | 6-chloro-1-[1-(3,4-diméthyl-benzènesulfonyl)-pipéridin-4-yl]-1,4-dihydro-benzo[d][1,3]oxazin-2-one |
| 730 | 1-[1-(5-méthyl-isoxazole-4-sulfonyl)-pipéridin-4-yl]-1,4-dihydro-benzo[d][1,3]oxazin-2-one |
| 731 | 1-[1-(2,2-diméthyl-chroman-6-sulfonyl)-pipéridin-4-yl]-1,4-dihydro-benzo[d][1,3]oxazin-2-one |
| 732 | 1-[1-(4-méthyl-3,4-dihydro-2H-benzo[1,4]oxazine-7-sulfonyl)-pipéridin-4-yl]-1,4-dihydro-benzo[d][1,3]oxazin-2-one |
| 733 | 1-[1-(2,3-dihydro-benzo[1,4]dioxine-6-sulfonyl)-pipéridin-4-yl]-1,4-dihydro-benzo[d][1,3]oxazin-2-one |
| 734 | 1-[1-(1,3,5-triméthyl-1H-pyrazole-4-sulfonyl)-pipéridin-4-yl]-1,4-dihydro-benzo[d][1,3]oxazin-2-one |
| 735 | 1-[1-(3-méthyl-2-oxo-2,3-dihydro-benzooxazole-6-sulfonyl)-pipéridin-4-yl]-1,4-dihydro-benzo[d][1,3]oxazin-2-one |
| 736 | 8-méthyl-1-[1-(5-méthyl-isoxazole-4-sulfonyl)-pipéridin-4-yl]-1,4-dihydro-benzo[d][1,3]oxazin-2-one |
| 737 | 1-[1-(2,2-diméthyl-chroman-6-sulfonyl)-pipéridin-4-yl]-8-méthyl-1,4-dihydro-benzo[d][1,3]oxazin-2-one |
| 738 | 8-méthyl-1-[1-(4-méthyl-3,4-dihydro-2H-benzo[1,4]oxazine-7-sulfonyl)-pipéridin-4-yl]-1,4-dihydro-benzo[d][1,3]oxazin-2-one |
| 739 | 1-[1-(2,3-dihydro-benzo[1,4]dioxine-6-sulfonyl)-pipéridin-4-yl]-8-méthyl-1,4-dihydro-benzo[d][1,3]oxazin-2-one |
| 740 | 8-méthyl-1-[1-(1,3,5-triméthyl-1H-pyrazole-4-sulfonyl)-pipéridin-4-yl]-1,4-dihydro-benzo[d][1,3]oxazin-2-one |
| 741 | 8-méthyl-1-[1-(3-méthyl-2-oxo-2,3-dihydro-benzooxazole-6-sulfonyl)-pipéridin-4-yl]-1,4-dihydro-benzo[d][1,3]oxazin-2-one |
| 742 | 8-méthoxy-1-[1-(1,3,5-triméthyl-1H-pyrazole-4-sulfonyl)-pipéridin-4-yl]-1,4-dihydro-benzo[d][1,3]oxazin-2-one |
| 743 | 8-méthoxy-1-[1-(3-méthyl-2-oxo-2,3-dihydro-benzooxazole-6-sulfonyl)-pipéridin-4-yl]-1,4-dihydro-benzo[d][1,3]oxazin-2-one |
| 744 | 1-[1-(benzo[d]isoxazol-3-ylméthanesulfonyl)-pipéridin-4-yl]-1,4-dihydro-benzo[d][1,3]oxazin-2-one |
| 745 | 1-[1-(2,2,4,6,7-pentaméthyl-2,3-dihydro-benzofuran-5-sulfonyl)-pipéridin-4-yl]-1,4-dihydro-benzo[d][1,3]oxazin-2-one |
| 746 | 6-méthyl-5-[4-(2-oxo-4H-benzo[d][1,3]oxazin-1-yl)-pipéridine-1-sulfonyl]-1 H-pyrimidine-2,4-dione |
| 747 | 1-[1-(3-méthyl-quinoléine-8-sulfonyl)-pipéridin-4-yl]-1,4-dihydro-benzo[d][1,3]oxazin-2-one |
| 748 | 1-[1-(2,2,5,7,8-pentaméthyl-chroman-6-sulfonyl)-pipéridin-4-yl]-1,4-dihydro-benzo[d][1,3]oxazin-2-one |
| 749 | 1,4-diméthyl-6-[4-(2-oxo-4H-benzo[d][1,3]oxazin-1-yl)-pipéridine-1-sulfonyl]-1,4-dihydro-quinoxaline-2,3-dione |
| 750 | 1-[1-(1H-imidazole-4-sulfonyl)-pipéridin-4-yl]-1,4-dihydro-benzo[d][1,3]oxazin-2-one |
| 751 | 1-[1-(2-oxo-1,2,3,4-tétrahydro-quinoléine-6-sulfonyl)-pipéridin-4-yl]-1,4-dihydro-benzo[d][1,3]oxazin-2-one |
| 752 | 7-[4-(2-oxo-4H-benzo[d][1,3]oxazin-1-yl)-pipéridine-1-sulfonyl]-1,5-dihydro-benzo[b][1,4]diazépine-2,4-dione |
| 753 | 8-méthyl-1-[1-(3-méthyl-quinoléine-8-sulfonyl)-pipéridin-4-yl]-1,4-dihydro-benzo[d][1,3]oxazin-2-one |
| 754 | 6-chloro-1-[1-(3-méthyl-quinoléine-8-sulfonyl)-pipéridin-4-yl]-1,4-dihydro-benzo [d][1,3]oxazin-2-one |
| 755 | 5-chloro-1-[1-(3-méthyl-quinoléine-8-sulfonyl)-pipéridin-4-yl]-1,4-dihydro-benzo[d][1,3]oxazin-2-one |
| 756 | 8-méthoxy-1-[1-(3-méthyl-quinoléine-8-sulfonyl)-pipéridin-4-yl]-1,4-dihydro-benzo[d][1,3]oxazin-2-one |
| 757 | 1-[1-(pyridine-2-sulfonyl)-pipéridin-4-yl]-1,4-dihydro-benzo[d][1,3]oxazin-2-one |
| 758 | 1-[1-(6,7-dihydroxy-naphtalène-1-sulfonyl)-pipéridin-4-yl]-1,4-dihydro-benzo[d][1,3]oxazin-2-one |
| 759 | 3-acétoxy-5-[4-(2-oxo-4H-benzo[d][1,3]oxazin-1-yl)-pipéridine-1-sulfonyl]-naphtalén-2-yl ester de l'acide acétique |
| 760 | 1-[1-(1H-benzoimidazole-2-sulfonyl)-pipéridin-4-yl]-1,4-dihydro-benzo[d][1,3]oxazin-2-one |
| 761 | 1-[1-(1H-benzoimidazole-2-sulfonyl)-pipéridin-4-yl]-8-méthoxy-1,4-dihydro-benzo[d][1,3]oxazin-2-one |
| 762 | 1-[1-(1H-benzoimidazole-2-sulfonyl)-pipéridin-4-yl]-5-chloro-1,4-dihydro-benzo[d][1,3]oxazin-2-one |
| 763 | 1-[1-(2,5-diméthoxy-benzènesulfonyl)-pipéridin-4-yl]-6-fluoro-1,4-dihydro-benzo[d][1,3]oxazin-2-one |
| 764 | 1-[1-(2,5-diméthoxy-benzènesulfonyl)-pipéridin-4-yl]-8-méthoxy-1,4-dihydro-benzo[d][1,3]oxazin-2-one |
| 765 | 1-[1-(2,5-diméthoxy-benzènesulfonyl)-pipéridin-4-yl]-6,7-difluoro-1,4-dihydro-benzo[d][1,3]oxazin-2-one |
| 766 | 5-chloro-1-[1-(2,5-diméthoxy-benzènesulfonyl)-pipéridin-4-yl]-1,4-dihydro-benzo[d][1,3]oxazin-2-one |
| 767 | 1-[1-(5-diméthylamino-naphtalène-1-sulfonyl)-pipéridin-4-yl]-8-méthoxy-1,4-dihydro-benzo[d][1,3]oxazin-2-one |
| 768 | 5-chloro-1-[1-(5-diméthylamino-naphtalène-1-sulfonyl)-pipéridin-4-yl]-1,4-dihydro-benzo[d][1,3]oxazin-2-one |
| 769 | 6-chloro-1-[1-(5-chloro-naphtalène-1-sulfonyl)-pipéridin-4-yl]-1,4-dihydro-benzo[d][1,3]oxazin-2-one |
| 770 | 1-[1-(5-chloro-naphtalène-1-sulfonyl)-pipéridin-4-yl]-6-méthyl-1,4-dihydro-benzo[d][1,3]oxazin-2-one |
| 771 | 1-[1-(5-chloro-naphtalène-1-sulfonyl)-pipéridin-4-yl]-6-fluoro-1,4-dihydro-benzo[d][1,3]oxazin-2-one |
| 772 | 6-chloro-1-[1-(5-chloro-naphtalène-2-sulfonyl)-pipéridin-4-yl]-1,4-dihydro-benzo[d][1,3]oxazin-2-one |
| 773 | 1-[1-(5-chloro-naphtalène-2-sulfonyl)-pipéridin-4-yl]-6-méthyl-1,4-dihydro-benzo[d][1,3]oxazin-2-one |
| 774 | 1-[1-(5-chloro-naphtalène-2-sulfonyl)-pipéridin-4-yl]-6-fluoro-1,4-dihydro-benzo[d][1,3]oxazin-2-one |
| 775 | 6-méthyl-1-[1-(3-méthyl-quinoléine-8-sulfonyl)-pipéridin-4-yl]-1,4-dihydro-benzo[d][1,3]oxazin-2-one |
| 776 | 6-fluoro-1-[1-(3-méthyl-quinoléine-8-sulfonyl)-pipéridin-4-yl]-1,4-dihydro-benzo[d][1,3]oxazin-2-one |
| 777 | 8,7-difluoro-1-[1-(3-méthyl-quinoléine-8-sulfonyl)-pipéridin-4-yl]-1,4-dihydro-benzo[d][1,3]oxazin-2-one |
| 778 | 6-chloro-1-[1-(3-méthyl-2-oxo-2,3-dihydro-benzooxazole-6-sulfonyl)-pipéridin-4-yl]-1,4-dihydro-benzo[d][1,3]oxazin-2-one |
| 779 | 6-méthyl-1-[1-(3-méthyl-2-oxo-2,3-dihydro-benzooxazole-6-sulfonyl)-pipéridin-4-yl]-1,4-dihydro-benzo[d][1,3]oxazin-2-one |
| 780 | 6-fluoro-1-[1-(3-méthyl-2-oxo-2,3-dihydro-benzooxazole-6-sulfonyl)-pipéridin-4-yl]-1,4-dihydro-benzo[d][1,3]oxazin-2-one |
| 781 | 6,7-difluoro-1-[1-(3-méthyl-2-oxo-2,3-dihydro-benzooxazole-6-sulfonyl)-pipéridin-4-yl]-1,4-dihydro-benzo[d][1,3]oxazin-2-one |
| 782 | 5-chloro-1-[1-(3-méthyl-2-oxo-2,3-dihydro-benzooxazole-6-sulfonyl)-pipéridin-4-yl]-1,4-dihydro-benzo[d][1,3]oxazin-2-one |
| 783 | 6-chloro-1-[1-(4-méthyl-3,4-dihyâro-2H-benzo[1,4]oxazine-7-sulfonyl)-pipéridin-4-yl]-1,4-dihydro-benzo[d][1,3]oxazin-2-one |
| 784 | 6-méthyl-1-[1-(4-méthyl-3,4-dihydro-2H-benzo[1,4]oxazine-7-sulfonyl)-pipéridin-4-yl]-1,4-dihydro-benzo[d][1,3]oxazin-2-one |
| 785 | 6-fluoro-1-[1-(4-méthyl-3,4-dihydro-2H-benzo[1,4]oxazine-7-sulfonyl)-pipéridin-4-yl]-1,4-dihydro-benzo[d][1,3]oxazin-2-one |
| 786 | 8-méthoxy-1-[1-(4-méthyl-3,4-dihydro-2H-benzo[1,4]oxazine-7-sulfonyl)-pipéridin-4-yl]-1,4-dihydro-benzo[d][1,3]oxazin-2-one |
| 787 | 5-chloro-1-[1-(4-méthyl-3,4-dihydro-2H-benzo[1,4]oxazine-7-sulfonyl)-pipéridin-4-yl]-1,4-dihydro-benzo[d][1,3]oxazin-2-one |

58. Procédé pour la préparation de composés de type sulfonamide dérivés de benzoxazinone de formule générale (I) selon une ou plusieurs des revendications 42 à 57, **caractérisé en ce qu'**il comprend la réaction d'au moins un composé dérivé de pipéridine de formule générale (IIb), dans laquelle R^{1b} à R^{9b} ont la définition selon une ou plusieurs des revendications 42 à 57, et/ou d'un sel, de préférence, un sel de type chlorhydrate de ceux-ci, 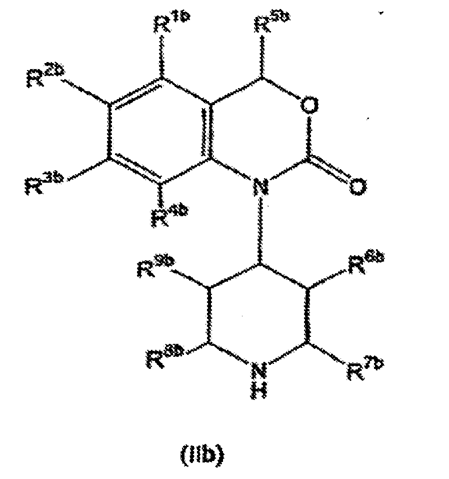 avec au moins un composé de formule générale (IIIb), 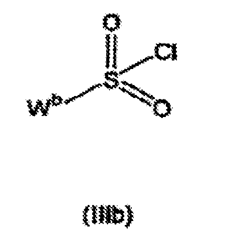 dans laquelle W^{b} a la définition selon une ou plusieurs des revendications 42 à 57, dans un milieu de réaction approprié, facultativement en présence d'au moins une base et/ou d'au moins un agent auxiliaire.

59. Procédé pour la préparation d'un sel physiologiquement acceptable des composés de type sulfonamide dérivés de benzoxazinone selon les revendications 42 à 57, **caractérisé en ce qu'**on fait réagir au moins un composé de formule générale (Ib), comportant au moins un groupe basique, avec au moins un acide, de préférence, un acide inorganique ou organique, de préférence, en présence d'un milieu de réaction approprié.

60. Procédé pour la préparation d'un sel physiologiquement acceptable des composés de type sulfonamide dérivés de benzoxazinone selon les revendications 42 à 57, **caractérisé en ce qu'**on fait réagir au moins un composé de formule générale (Ib), comportant au moins un groupe acide, avec au moins une base, de préférence, en présence d'un milieu de réaction approprié.

61. Médicament comprenant au moins un composé de type sulfonamide dérivé de benzoxazinone selon l'une quelconque des revendications 42 à 57, et facultativement, un ou plusieurs adjuvants pharmaceutiquement acceptables.

62. Médicament selon la revendication 61, pour la prophylaxie et/ou le traitement des troubles de consommation d'aliments, de préférence, pour la régulation de l'appétit, pour le maintien, l'accroissement ou la diminution du poids corporel; pour la prophylaxie et/ou le traitement de l'obésité ; pour la prophylaxie et/ou le traitement de la boulimie; pour la prophylaxie et/ou le traitement de l'anorexie ; pour la prophylaxie et/ou le traitement de la cachéxie ; pour la prophylaxie et/ou le traitement du diabète de type II (diabète sucré non insulinodépendant).

63. Médicament selon la revendication 61, pour la prophylaxie et/ou le traitement de troubles gastro-intestinaux, de préférence, le syndrome du côlon irritable ; pour la prophylaxie et/ou le traitement de troubles du système nerveux central ; pour la prophylaxie et/ou le traitement de l'anxiété ; pour la prophylaxie et/ou le traitement des attaques de panique ; pour la prophylaxie et/ou le traitement de la dépression ; pour la prophylaxie et/ou le traitement des troubles bipolaires ; pour la prophylaxie et/ou le traitement des troubles cognitifs, de préférence, les troubles de la mémoire ; pour améliorer la cognition (pour la stimulation cognitive) ; pour la prophylaxie et/ou le traitement de la démence sénile ; pour la prophylaxie et/ou le traitement d'une psychose ; pour la prophylaxie et/ou le traitement de troubles neurodégénératifs, de préférence, choisis parmi le groupe consistant en la maladie d'Alzheimer, la maladie de Parkinson, la maladie de Huntington et la sclérose en plaques ; pour la prophylaxie et/ou le traitement de la schizophrénie ; ou pour la prophylaxie et/ou le traitement d'un trouble d'hyperactivité (ADHC, trouble d'hyperactivité et de déficit de l'attention).

64. Utilisation d'au moins un composé de type sulfonamide dérivé de benzoxazinone selon l'une quelconque des revendications de 42 à 57, pour la fabrication d'un médicament pour la prophylaxie et/ou le traitement des troubles de consommation d'aliments.

65. Utilisation selon la revendication 64, pour la régulation de l'appétit, pour le maintien, l'accroissement ou la diminution du poids corporel ; pour la prophylaxie et/ou le traitement de l'obésité ; pour la prophylaxie et/ou le traitement de la boulimie ; pour la prophylaxie et/ou le traitement de l'anorexie ; pour la prophylaxie et/ou le traitement de la cachéxie ; ou pour la prophylaxie et/ou le traitement du diabète de type II (diabète sucré non insulinodépendant).

66. Utilisation d'au moins un composé de type sulfonamide dérivé de benzoxazinone selon l'une quelconque des revendications 42 à 57, pour la fabrication d'un médicament pour la prophylaxie et/ou le traitement de troubles gastro-intestinaux, de préférence, le syndrome du côlon irritable ; pour la prophylaxie et/ou le traitement de troubles du système nerveux central ; pour la prophylaxie et/ou le traitement de l'anxiété ; pour la prophylaxie et/ou le traitement des attaques de panique ; pour la prophylaxie et/ou le traitement de la dépression ; pour la prophylaxie et/ou le traitement des troubles bipolaires ; pour la prophylaxie et/ou le traitement des troubles cognitifs, de préférence, les troubles de la mémoire ; pour améliorer la cognition (pour la stimulation cognitive) ; pour la prophylaxie et/ou le traitement de la démence sénile ; pour la prophylaxie et/ou le traitement d'une psychose ; pour la prophylaxie et/ou le traitement de troubles neurodégénératifs, de préférence, choisis parmi le groupe consistant en la maladie d'Alzheimer, la maladie de Parkinson, la maladie de Huntington et la sclérose en plaques ; pour la prophylaxie et/ou le traitement de la schizophrénie ; ou pour la prophylaxie et/ou le traitement d'un trouble d'hyperactivité (ADHC, trouble d'hyperactivité et de déficit de l'attention).
